(19) Europäisches Patentamt
European Patent Office
Office européen des brevets

(11) EP 4 089 079 A1

(12) EUROPEAN PATENT APPLICATION
published in accordance with Art. 153(4) EPC

(43) Date of publication:
**16.11.2022 Bulletin 2022/46**

(21) Application number: **20912440.3**

(22) Date of filing: **31.12.2020**

(51) International Patent Classification (IPC):
***C07D 295/155*** *(2006.01)* ***C07D 417/04*** *(2006.01)*
***C07D 413/04*** *(2006.01)* ***A61K 31/5377*** *(2006.01)*
***A61K 31/5375*** *(2006.01)* ***A61P 19/02*** *(2006.01)*
***A61P 35/00*** *(2006.01)*

(52) Cooperative Patent Classification (CPC):
**A61K 31/40; A61K 31/402; A61K 31/4025; A61K 31/407; A61K 31/4439; A61K 31/506; A61K 31/5375; A61K 31/5377; A61P 1/00; A61P 1/04; A61P 11/00; A61P 11/02; A61P 11/06; A61P 17/00; A61P 17/06;** (Cont.)

(86) International application number:
**PCT/CN2020/142168**

(87) International publication number:
**WO 2021/139599 (15.07.2021 Gazette 2021/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2020 CN 202010008803**
**06.01.2020 CN 202010009133**

(71) Applicant: **Sunshine Lake Pharma Co., Ltd.**
**Dongguan, Guangdong 523000 (CN)**

(72) Inventors:
- **ZHANG, Yingjun**
**Dongguan, Guangdong 523871 (CN)**
- **LIU, Bing**
**Dongguan, Guangdong 523871 (CN)**
- **PAN, Wei**
**Dongguan, Guangdong 523871 (CN)**
- **WANG, Feng**
**Dongguan, Guangdong 523871 (CN)**
- **HE, Wei**
**Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) ROR GAMMA T INHIBITOR, PREPARATION METHOD THEREFOR AND USE THEREOF

(57) The present invention relates to the technical field of drugs, and in particular, to an RORγt inhibitor, a preparation method thereof and uses thereof. The invention also relates to a pharmaceutical composition of the compound, a method for preparing the pharmaceutical composition, and use of the compound or the pharmaceutical composition in treating or preventing cancer, inflammation, or autoimmune diseases mediated by RORγt in mammals, especially humans.

EP 4 089 079 A1

(52) Cooperative Patent Classification (CPC): (Cont.)
**A61P 19/00; A61P 19/02; A61P 29/00;
A61P 35/00; A61P 37/06; A61P 37/08;
C07D 205/04; C07D 207/08; C07D 207/09;
C07D 207/12; C07D 207/273; C07D 207/416;
C07D 231/04; C07D 243/08; C07D 263/22;
C07D 295/155; C07D 401/12; C07D 401/14;
C07D 403/04; C07D 405/12; C07D 413/04;
C07D 413/06; C07D 417/04; C07D 491/107**

**Description**

## CROSS-REFERENCE TO RELATED APPLICATIONS

This application claims the priorities and benefits of Chinese Patent Application No. 202010008803.7, filed with the State Intellectual Property Office of China on January 06, 2020; and Chinese Patent Application No. 202010009133.0, filed with the State Intellectual Property Office of China on January 06, 2020, all of which are incorporated herein by reference in their entireties.

## FIELD

The invention belongs to the technical field of medicines, and in particular relates to a class of small molecule compounds, compositions, preparation methods and uses thereof, wherein the compounds or compositions can be used as Retinoid-related orphan receptor gamma t (RORyt) inhibitors and used for the prevention or treatment of cancer, inflammation or immune-related diseases.

## BACKGROUND

Retinoid-related orphan receptor is a subfamily of transcription factors in the superfamily of steroid hormone nuclear receptors. The retinoid-related orphan receptor family includes ROR$\alpha$, ROR$\beta$, and RORy, which are encoded by different genes (RORA, RORB, and RORC), respectively. The retinoid-related orphan receptor contains four major domains: a *N*-terminal A/B domain, a DNA-binding domain, a hinge domain, and a ligand-binding domain.

Retinoid-related orphan receptor gamma t (RORyt) is one of the two isoforms of retinoid-related orphan receptor gamma (RORy), and it is also known as ROR$\gamma$2. Studies have shown that RORyt is only expressed in lymphoid lineage and embryonic lymphoid tissue inducer cells (Sun et al., Science 288: 2369-2372, 2000; Eberl et al., Nat Immunol. 5: 64-73, 2004). As a characteristic transcription factor of T helper cells (Th17), RORyt plays an important role in the differentiation of Th17 cells and is a key regulator in the differentiation of Th17 cells (Ivanov, II, McKenzie BS, Zhou L, Tadokoro CE, Lepelley A, Lafaille JJ, et al. Cell 2006; 126(6): 1121-33).

Th17 can secrete interleukin 17 (IL-17) and other pro-inflammatory cytokines, which play an important role in autoimmune diseases and the body's defense response. IL-17 is a pro-inflammatory cytokine in the development of inflammation and various autoimmune diseases, and is closely related to a variety of autoimmune and inflammatory diseases, such as rheumatoid arthritis, psoriasis, psoriatic arthritis, spondyloarthritis, asthma, inflammatory bowel disease, systemic lupus erythematosus and multiple sclerosis, etc. (Jetten et al., Nucl. Recept. Signal, 2009, 7: e003; Manel et al., Nat. Immunol., 2008, 9, 641-649). It is also implicated in the occurrence of inflammation-related tumors, where Th17 cells are activated during disease and are responsible for recruiting other inflammatory cell types, such as neutrophils, to mediate pathology in target tissues (Korn et al., Annu. Rev. Immunol., 2009, 27:485-517).

The role of RORyt in the pathogenesis of autoimmune disease or inflammation has been extensively studied and well elucidated (Jetten et al., Adv. Dev.Biol, 2006, 16: 313-355; Meier et al. Immunity, 2007, 26:643-654; Aloisi et al., Nat. Rev. Immunol., 2006, 6:205-217; Jager et al., J. Immunol., 2009, 183:7169-7177; Barnes et al., Nat. Rev. Immunol., 2008, 8: 183-192). Therefore, inhibition of RORyt will effectively inhibit the cell differentiation of Th17, regulate the production and secretion levels of IL-17 and other pro-inflammatory cytokines, thereby regulating the body's immune system, and treating cancer, immune and inflammatory diseases related to the regulation of RORyt.

## SUMMARY OF THE INVENTION

The following only outlines some aspects of the present invention, and is not limited thereto. These and other sections are described more fully later. All references of this specification are incorporated herein by reference in their entirety. When there is a discrepancy between the disclosed content of this specification and the cited documents, the disclosed content of this specification shall prevail.

The invention provides a class of compounds with retinoid-related orphan receptor gamma t (RORyt) inhibitory activity, which are used in the manufacture of a medicament for preventing or treating cancer, inflammation or autoimmune diseases mediated by RORyt, such as cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease, *etc.* The compounds of the present invention can inhibit RORyt well and have excellent physicochemical properties and pharmacokinetic properties.

The present invention also provides methods of preparing these compounds, pharmaceutical compositions comprising these compounds, and methods of using these compounds and compositions to treat the above-mentioned

diseases in mammals, especially humans.

Specifically:

In one aspect, the invention relates to a compound having Formula (I), or a stereoisomer, a geometric isomer, a tautomer, an *N*-oxide, a hydrate, a solvate, a metabolite, an ester, a pharmaceutically acceptable salt or a prodrug thereof,

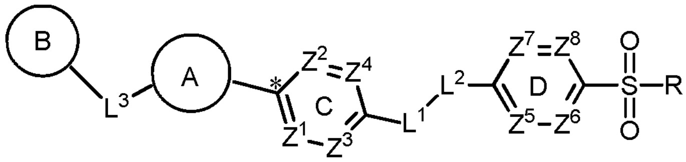

(I),

wherein:

R is $C_{1-6}$ alkyl, $C_{1-6}$ alkoxy, $C_{1-6}$ alkylamino, $C_{3-8}$ cycloalkylamino, $-C_{1-6}$ alkylene-$C_{3-8}$ cycloalkyl, $C_{3-8}$ cycloalkyl, $C_{1-6}$ haloalkyl or $C_{1-6}$ haloalkoxy;

each of $Z^1$, $Z^2$, $Z^3$, $Z^4$, $Z^5$, $Z^6$, $Z^7$ and $Z^8$ is independently $CR^1$ or N;

each $R^1$ is independently hydrogen, deuterium, cyano, fluorine, chlorine, bromine, iodine, $C_{1-6}$ alkyl, $C_{1-6}$ haloalkyl, hydroxy-substituted $C_{1-6}$ alkyl, $-C_{1-6}$ alkylene-$C_{1-6}$ alkoxy, $C_{1-6}$ alkoxy, $C_{3-8}$ cycloalkyl or 3- to 7-membered heterocyclyl;

ring A is 3- to 5-membered heterocyclyl or 7-membered heterocyclyl, and each of the 3- to 5-membered heterocyclyl and 7-membered heterocyclyl is independently and optionally substituted with 1, 2, 3, 4 or 5 $R^2$; wherein * represents the direction in which ring A is connected to ring C;

each $R^2$ is independently oxo, $R^a$, $C_{1-6}$ alkyl, $-C_{1-6}$ alkylene-$R^a$, $-OR^b$, $-C_{1-6}$ alkylene-$OR^b$, $-(C{=}O)-R^a$, $-C_{1-6}$ alkylene-$(C{=}O)-R^a$, $-(C{=}O)-NR^cR^d$ or $-C_{1-6}$ alkylene-$(C{=}O)-NR^cR^d$;

each $R^a$ is independently deuterium, fluorine, chlorine, bromine, iodine, hydroxy, amino, nitro, cyano, $C_{1-6}$ alkyl, $C_{1-6}$ alkoxy, $C_{1-6}$ haloalkyl, $C_{3-8}$ cycloalkyl, $C_{6-10}$ aryl, 5- to 10-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;

each $R^b$, $R^c$ and $R^d$ is independently hydrogen, deuterium, $C_{1-6}$ alkyl, $C_{1-6}$ haloalkyl, $C_{1-6}$ alkoxy, $C_{3-8}$ cycloalkyl, $C_{6-10}$ aryl, 5- to 10-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl; wherein each $R^a$, $R^b$, $R^c$ and $R^d$ is independently and optionally substituted with 1, 2, 3, 4, 5 or 6 $R^g$;

each $R^g$ is independently deuterium, fluorine, chlorine, bromine, iodine, oxo, hydroxy, amino, nitro, cyano, $C_{1-6}$ alkyl, $C_{1-6}$ haloalkyl, $C_{1-6}$ alkoxy, $C_{1-6}$ haloalkoxy, $C_{3-8}$ cycloalkyl or 3- to 7-membered heterocyclyl;

ring B is $C_{6-10}$ aryl, $C_{3-8}$ cycloalkyl, 5- to 12-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3 or 4 $R^e$;

each $R^e$ is independently deuterium, fluorine, chlorine, bromine, iodine, cyano, nitro, hydroxy, amino, $C_{1-6}$ alkyl, $C_{1-6}$ alkoxy, $C_{3-8}$ cycloalkyl, 3- to 7-membered heterocyclyl, $C_{1-6}$ haloalkyl or $C_{1-6}$ haloalkoxy, wherein each of the $C_{1-6}$ alkyl, $C_{1-6}$ alkoxy, $C_{3-8}$ cycloalkyl, 3- to 7-membered heterocyclyl, $C_{1-6}$ haloalkyl and $C_{1-6}$ haloalkoxy is independently and optionally substituted with 1, 2, 3 or 4 substituents selected from cyano, nitro, hydroxy, amino or 3- to 7-membered heterocyclyl;

$L^1$ is $-S(O)_2-NH-$, $-NH-S(O)_2-$, $-S(O)-NH-$, $-NH-S(O)-$, $-C(=O)NH-$ or $-NHC(=O)-$;

$L^2$ is a bond or $-CR^3R^4-$;

$L^3$ is carbonyl, S, -O-, $-NR^5-$ or $-CR^6k^7-$;

$R^3$ is hydrogen, deuterium, hydroxy-substituted $C_{1-6}$ alkyl, $-C_{1-6}$ alkylene-$OC(=O)-R^f$, $-C_{1-6}$ alkylene-$C(=O)-O-R^f$ or $-C_{1-6}$ alkylene-$R^f$;

$R^f$ is $C_{2-6}$ alkyl, amino-substituted $C_{1-6}$ alkyl, $C_{1-6}$ haloalkyl, $-C_{1-6}$ alkylene-$C(=O)-OR^h$ or $-OP(=O)-(OR^m)(OR^n)$; wherein each $R^h$, $R^m$ and $R^n$ is independently hydrogen, deuterium, sodium, potassium, $C_{1-6}$ alkyl or $C_{1-6}$ haloalkyl;

$R^4$ is hydrogen, deuterium or hydroxy-substituted $C_{1-6}$ alkyl;

$R^5$ is hydrogen, deuterium, $C_{1-4}$ alkyl, $C_{1-4}$ haloalkyl, $C_{1-4}$ alkoxy, $C_{3-6}$ cycloalkyl or 3- to 6-membered heterocyclyl;

each of $R^6$ and $R^7$ is independently hydrogen, deuterium, $C_{1-4}$ alkyl, $C_{1-4}$ haloalkyl, $C_{1-4}$ haloalkoxy, $C_{1-4}$ alkoxy, $C_{3-6}$ cycloalkyl or 3- to 6-membered heterocyclyl.

In some embodiments, R is $C_{1-4}$ alkyl, $C_{1-4}$ alkoxy, $C_{1-4}$ alkylamino, $C_{3-6}$ cycloalkylamino, $-C_{1-4}$ alkylene-$C_{3-6}$ cycloalkyl, $C_{3-6}$ cycloalkyl, $C_{1-4}$ haloalkyl or $C_{1-4}$ haloalkoxy.

In some embodiments, each $R^1$ is independently hydrogen, deuterium, cyano, fluorine, chlorine, bromine, iodine, $C_{1-4}$ alkyl, $C_{1-4}$ haloalkyl, hydroxy-substituted $C_{1-4}$ alkyl, -$C_{1-4}$ alkylene-$C_{1-4}$ alkoxy, $C_{1-4}$ alkoxy, $C_{3-6}$ cycloalkyl or 3- to 7-membered heterocyclyl.

In some embodiments, each $R^2$ is independently oxo, $R^a$, $C_{1-4}$ alkyl, -$C_{1-4}$alkylene-$R^a$, -$OR^b$, -$C_{1-4}$alkylene-$OR^b$, -(C=O)-$R^a$, -$C_{1-4}$alkylene-(C=O)-$R^a$, -(C=O)-$NR^cR^d$ or -$C_{1-4}$ alkylene-(C=O)-$NR^cR^d$.

In some embodiments, each $R^a$ is independently deuterium, fluorine, chlorine, bromine, iodine, hydroxy, amino, nitro, cyano, $C_{1-4}$ alkyl, $C_{1-4}$ alkoxy, $C_{1-4}$ haloalkyl, $C_{3-6}$ cycloalkyl, $C_{6-10}$ aryl, 5- to 7-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;

each $R^b$, $R^c$ and $R^d$ is independently hydrogen, deuterium, $C_{1-4}$ alkyl, $C_{1-4}$ haloalkyl, $C_{1-4}$ alkoxy, $C_{3-6}$ cycloalkyl, $C_{6-10}$ aryl, 5- to 7-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl; wherein each $R^a$, $R^b$, $R^c$ and $R^d$ is independently and optionally substituted with 1, 2, 3, 4, 5 or 6 $R^g$.

In some embodiments, each $R^g$ is independently deuterium, fluorine, chlorine, bromine, iodine, oxo, hydroxy, amino, nitro, cyano, $C_{1-4}$ alkyl, $C_{1-4}$ haloalkyl, $C_{1-4}$ alkoxy, $C_{1-4}$ haloalkoxy, $C_{3-6}$ cycloalkyl or 3- to 7-membered heterocyclyl.

In some embodiments, ring B is $C_{6-10}$ aryl, $C_{3-6}$ cycloalkyl, 5- to 7-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3, 4, 5 or 6 $R^e$.

In some embodiments, each $R^e$ is independently deuterium, fluorine, chlorine, bromine, iodine, cyano, nitro, hydroxy, amino, $C_{1-4}$ alkyl, $C_{1-4}$ alkoxy, $C_{3-6}$ cycloalkyl, 3- to 7-membered heterocyclyl, $C_{1-4}$ haloalkyl or $C_{1-4}$ haloalkoxy, wherein each of the $C_{1-4}$ alkyl, $C_{1-4}$ alkoxy, $C_{3-6}$ cycloalkyl, 3- to 7-membered heterocyclyl, $C_{1-4}$ haloalkyl and $C_{1-4}$ haloalkoxy is independently and optionally substituted with 1, 2, 3 or 4 substituents selected from cyano, nitro, hydroxy, amino or 3- to 7-membered heterocyclyl.

In some embodiments, $R^3$ is hydrogen, deuterium, hydroxy-substituted $C_{1-4}$ alkyl, -$C_{1-4}$ alkylene-O-C(=O)-$R^f$, -$C_{1-4}$ alkylene-C(=O)-O-$R^f$ or -$C_{1-4}$alkylene-$R^f$;

$R^f$ is $C_{2-4}$ alkyl, amino-substituted $C_{1-4}$ alkyl, $C_{1-4}$ haloalkyl, -$C_{1-4}$alkylene-C(=O)-$OR^h$ or -O-P(=O)-($OR^m$)($OR^n$); wherein each $R^h$, $R^m$ and $R^n$ is independently hydrogen, deuterium, sodium, potassium, $C_{1-4}$ alkyl or $C_{1-4}$ haloalkyl; $R^4$ is hydrogen, deuterium or hydroxy-substituted $C_{1-4}$ alkyl.

In other embodiments, wherein R is methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, methylamino, ethylamino, dimethylamino, cyclopropylamino, cyclobutylamino, cyclopentylamino, cyclohexylamino, -methylene-cyclopropyl, -methylene-cyclobutyl, -methylene-cyclopentyl, -methylene-cyclohexyl, -ethylene-cyclopropyl, -ethylene-cyclobutyl, -ethylene-cyclopentyl, -ethylene-cyclohexyl, cyclopropyl, cyclobutyl, cyclopentyl or cyclohexyl.

In other embodiments, wherein each $R^1$ is independently hydrogen, deuterium, cyano, fluorine, chlorine, bromine, iodine, methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, hydroxymethyl, 2-hydroxyethyl, -methylenemethoxy, -methyleneethoxy, -methylene-*n*-propoxy, -methyleneisopropoxy, cyclopropyl, cyclobutyl, cyclopentyl or cyclohexyl.

In some embodiments, ring A is

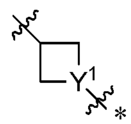, 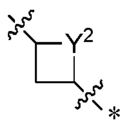, 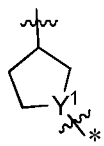, 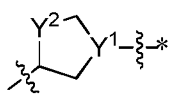,

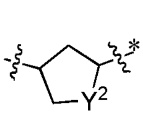, 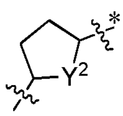, 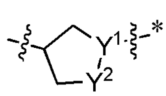, 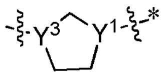, 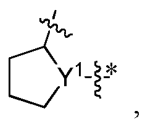, 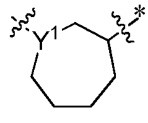, 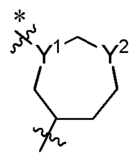,

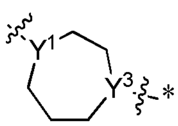 or 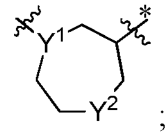 ;

wherein, each $Y^1$ and $Y^3$ is independently N or CH; $Y^2$ is O, S, NH or $CH_2$; * represents the direction in which ring A is connected to ring C; the ring A is optionally substituted with 1, 2, 3, 4 or 5 $R^2$.

In other embodiments, ring A is

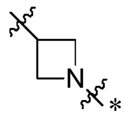 , 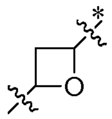 , 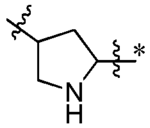 , 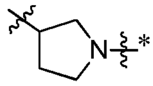 ,

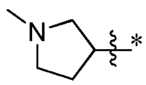 , 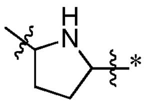 , 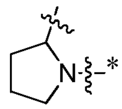 , 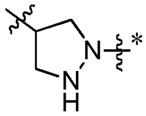 , 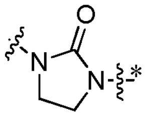 , 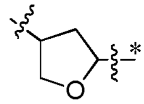 , 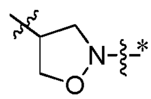 ,

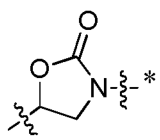 , 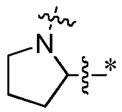 , 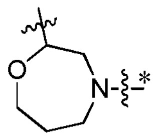 , 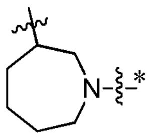 , 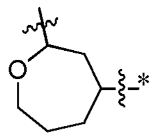 , 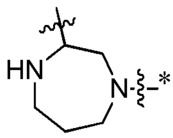 , 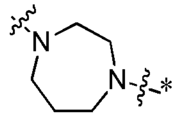

or

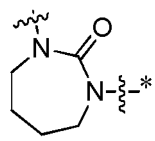 ;

wherein, * represents the direction in which ring A is connected to ring C; the ring A is optionally substituted with 1, 2, 3, 4 or 5 $R^2$.

In other embodiments, each $R^2$ is independently oxo, $R^a$, methyl, ethyl, *n*-propyl, isopropyl, *n*-butyl, isobutyl, sec-butyl, tert-butyl, $-CH_2R^a$, $-CH_2CH_2R^a$, $-CH_2CH_2CH_2R^a$, $-C(CH_3)_2R^a$, $-CH(CH_3)CH_2R^a$, $-OR^b$, $-CH_2OR^b$, $-CH_2CH_2OR^b$, $-CH_2CH_2CH_2OR^b$, $-C(CH_3)_2OR^b$, $-CH(CH_3)CH_2OR^b$, $-(C{=}O)-R^a$, $-CH_2(C{=}O)-R^a$, $-CH_2CH_2(C{=}O)-R^a$, $-CH_2CH_2CH_2(C{=}O)-R^a$, $-C(CH_3)_2(C{=}O)-R^a$, $-CH(CH_3)CH_2(C{=}O)-R^a$, $-(C{=}O)-NR^cR^d$, $-CH_2(C{=}O)-NR^cR^d$, $-CH_2CH_2(C{=}O)-NR^cR^d$, $-CH_2CH_2CH_2(C{=}O)-NR^cR^d$, $-C(CH_3)_2(C{=}O)-NR^cR^d$ or $-CH(CH_3)CH_2(C{=}O)-NR^cR^d$.

In other embodiments, each $R^a$ is independently deuterium, fluorine, chlorine, bromine, iodine, hydroxy, amino, nitro, cyano, methyl, ethyl, *n*-propyl, isopropyl, *n*-butyl, isobutyl, sec-butyl, tert-butyl, methoxy, ethoxy, isopropoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, oxetanyl, azetidinyl, pyrrolidinyl, tetrahydrofuranyl, tetrahydropyranyl, morpholinyl, thiomorpholinyl, piperidinyl, piperazinyl, oxepanyl, cyclopropyl, cyclobutyl, cyclopentyl, cyclohexyl, phenyl, thiazolyl, pyrazolyl, imidazolyl, furanyl, oxazolyl, isoxazolyl, triazolyl, thienyl, pyrrolyl, pyridyl, pyrimidinyl,

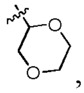 ,

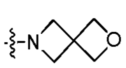 , 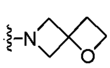 , 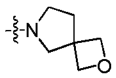 , 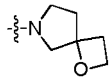 , 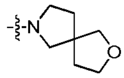 , 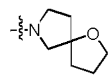 , 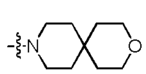 , 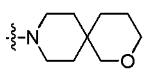 ,

or

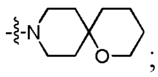;

each $R^b$, $R^c$ and $R^d$ is independently hydrogen, deuterium, methyl, ethyl, *n*-propyl, isopropyl, *n*-butyl, isobutyl, sec-butyl, *tert*-butyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, methoxy, ethoxy, *n*-propoxy, isopropoxy, *n*-butoxy, isobutoxy, *sec*-butoxy, *tert*-butoxy, cyclopropyl, cyclobutyl, cyclopentyl, cyclohexyl, phenyl, oxetanyl, azetidinyl, pyrrolidinyl, tetrahydrofuranyl, tetrahydropyranyl, morpholinyl, thiomorpholinyl, piperidinyl, piperazinyl, oxepanyl, thiazolyl, pyrazolyl, imidazolyl, furanyl, oxazolyl, isoxazolyl, triazolyl, thienyl, pyrrolyl, pyridyl, pyrimidinyl,

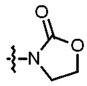, 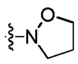, 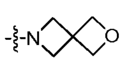, 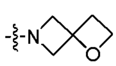, 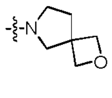, 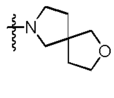, 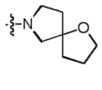, 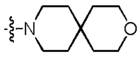,

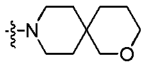 or 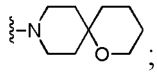;

or, $R^c$ and $R^d$ together with the N atom to which they are attached form

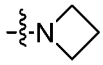, 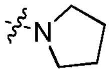, 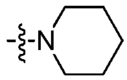 or 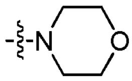;

wherein each $R^a$, $R^b$, $R^c$ and $R^d$ is independently and optionally substituted with 1, 2, 3, 4, 5 or 6 $R^g$.

In other embodiments, each $R^g$ is independently deuterium, fluorine, chlorine, bromine, iodine, oxo, hydroxy, amino, nitro, cyano, methyl, ethyl, *n*-propyl, isopropyl, *n*-butyl, isobutyl, sec-butyl, tert-butyl, methoxy, ethoxy, *n*-propoxy, *iso*-propoxy, *n*-butoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$, $-OCF_2CF_3$, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl or oxepanyl.

In other embodiments, the ring B is phenyl, cyclopropyl, cyclobutyl, cyclopentyl, cyclohexyl, pyridyl, pyrimidinyl, pyrrolyl, pyrazolyl, thiazolyl, imidazolyl, furanyl, oxazolyl, isoxazolyl, thienyl, triazolyl, tetrazolyl, piperazinyl, piperidinyl, morpholinyl, thiomorpholinyl, tetrahydropyranyl, tetrahydrofuranyl, pyrrolidinyl, azetidinyl, oxetanyl,

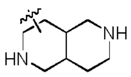, 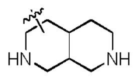,

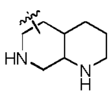, 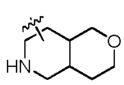, 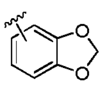, 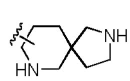, 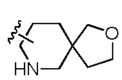, 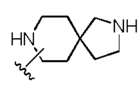 or 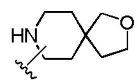;

wherein the ring B is optionally substituted with 1, 2, 3 or 4 $R^e$.

In other embodiments, each $R^e$ is independently deuterium, fluorine, chlorine, bromine, iodine, cyano, nitro, hydroxy, methyl, ethyl, *n*-propyl, isopropyl, methoxy, ethoxy, *n*-propoxy, isopropoxy, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$ or $-OCF_2CF_3$; wherein each of the methyl, ethyl, *n*-propyl, isopropyl, methoxy, ethoxy, *n*-propoxy, isopropoxy, cyclopropyl, cyclobutyl, cyclopentyl, cyclohexyl, oxetanyl,

azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-OCH_2F$, $-OCHF_2$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$ and $-OCF_2CHF_2$ is independently and optionally substituted with 1, 2, 3 or 4 substituents selected from cyano, nitro, hydroxy, amino, piperazinyl, piperidinyl, morpholinyl, thiomorpholinyl, tetrahydropyranyl, tetrahydrofuranyl, pyrrolidinyl, azetidinyl or oxetanyl.

In other embodiments, $R^3$ is hydrogen, deuterium, hydroxymethyl, 2-hydroxyethyl, 1-hydroxyethyl, 3-hydroxy-*n*-propyl, 2-hydroxy-1-methylethyl, $-CH_2OC(=O)R^f$, $-CH_2CH_2OC(=O)R^f$, $-CH_2CH_2CH_2OC(=O)R^f$, $-CH(CH_3)_2OC(=O)R^f$, $-CH_2C(=O)OR^f$, $-CH_2CH_2C(=O)OR^f$, $-CH_2CH_2CH_2C(=O)OR^f$, $-CH(CH_3)_2C(=O)OR^f$, $-CH_2R^f$, $-CH_2CH_2R^f$, $-CH_2CH_2CH_2R^f$ or $-CH(CH_3)_2R^f$;

$R^f$ is ethyl, *n*-propyl, isopropyl, $-CH_2NH_2$, $-CH_2CH_2NH_2$, $-CH_2CH_2CH_2NH_2$, $-CH(NH_2)CH(CH_3)_2$, $-CH_2F$, $-CHF_2$, $-CF_3$, $-CHFCH_2F$, $-CF_2CH_3$, $-CH_2CHF_2$, $-CH_2CF_3$, $-CH_2C(=O)ONa$, $-CH_2CH_2C(=O)-ONa$, $-CH_2CH_2CH_2C(=O)ONa$, $-CH(CH_3)_2C(=O)ONa$ or $-O-P(=O)(ONa)_2$;

$R^4$ is hydrogen, deuterium, hydroxymethyl, 2-hydroxyethyl, 1-hydroxyethyl, 3-hydroxy-*n*-propyl or 2-hydroxy-1-methylethyl.

In other embodiments, $R^5$ is independently hydrogen, deuterium, methyl, ethyl, isopropyl, *n*-propyl, *n*-butyl, isobutyl, sec-butyl, tert-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl;
each of $R^6$ and $R^7$ is independently hydrogen, deuterium, methyl, ethyl, isopropyl, *n*-propyl, *n*-butyl, isobutyl, *sec*-butyl, *tert*-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl.

In another aspect, the present invention relates to a pharmaceutical composition comprising a compound having formula (I), or a stereoisomer, a geometric isomer, a tautomer, an *N*-oxide, a hydrate, a solvate, a metabolite, a pharmaceutically acceptable salt or a prodrug thereof, and a pharmaceutically acceptable excipient, a carrier, an adjuvant or a combination thereof;
wherein the pharmaceutical composition further comprises other active ingredients, and the other active ingredients are drugs for treating cancer, drugs for preventing or treating inflammatory syndrome, drugs for preventing or treating autoimmune diseases, or any combination thereof.

In another aspect, the present invention relates to use of the compound having formula (I), or a pharmaceutical composition thereof in the manufacture of a medicament for preventing or treating cancer, inflammation or autoimmune diseases mediated by RORγt in mammals, including humans.

In some embodiments, the present invention relates to use of the compound having formula (I), or a pharmaceutical composition thereof in the manufacture of a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

In another aspect, the present invention relates to a method of preparing, separating or purifying the compound having formula (I).

The biological test results show that the compounds provided by the present invention have good inhibitory activity on RORγt, and also have good pharmacokinetic characteristics.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

The foregoing merely summarizes certain aspects disclosed herein and is not intended to be limiting in nature. These aspects and other aspects and embodiments are described more fully below.

## DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in

the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

The grammatical articles "a", "an" and "the", as used herein, are intended to include "at least one" or "one or more" unless otherwise indicated herein or clearly contradicted by the context. Thus, the articles are used herein to refer to one or more than one *(i.e.,* at least one) of the grammatical objects of the article. By way of example, "a component" means one or more components, and thus, possibly, more than one component is contemplated and may be employed or used in an implementation of the described embodiments.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In some embodiments, "patient" refers to a human.

The term "comprise" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, *etc.*

"Chiral" refers to molecules which have the property of non-superimposability of the mirror image partner, while the term "achiral" refers to molecules which are superimposable on their mirror image partner.

"Enantiomers" refers to two stereoisomers of a compound which are non-superimposable mirror images of one another.

"Diastereomer" refers to a stereoisomer with two or more centers of chirality and whose molecules are not mirror images of one another. Diastereomers have different physical properties, e.g., melting points, boling points, spectral properties or biological activities. Mixture of diastereomers may separate under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Many organic compounds exist in optically active forms, *i.e.,* they have the ability to rotate the plane of plane-polarized light. In describing an optically active compound, the prefixes D and *L,* or *R* and *S*, are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. A specific stereoisomer may be referred to as an enantiomer, and a mixture of such stereoisomers is called an enantiomeric mixture. A 50:50 mixture of enantiomers is referred to as a racemic mixture or a racemate, which may occur where there has been no stereoselection or stereospecificity in a chemical reaction or process.

Any asymmetric atom (e.g., carbon or the like) of the compound(s) disclosed herein can be present in racemic or enantiomerically enriched, for example the (*R*)-, (*S*)- or (*R,S*)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)- or (*S*)-configuration.

Depending on the choice of the starting materials and procedures, the compounds can be present in the form of one of the possible stereoisomers or as mixtures thereof, such as racemates and diastereoisomer mixtures, depending

on the number of asymmetric carbon atoms. Optically active (*R*)- and (*S*)-isomers may be prepared using chiral synthons or chiral reagents, or resolved using conventional techniques. If the compound contains a double bond, the substituent may be *E* or *Z* configuration. If the compound contains a disubstituted cycloalkyl, the cycloalkyl substituent may have a cis- or trans-configuration.

Any resulting mixtures of stereoisomers can be separated on the basis of the physicochemical differences of the constituents, into the pure or substantially pure geometric isomers, enantiomers, diastereomers, for example, by chromatography and/or fractional crystallization.

Any resulting racemates of final products or intermediates can be resolved into the optical antipodes by methods known to those skilled in the art, *e.g.,* by separation of the diastereomeric salts thereof. Racemic products can also be resolved by chiral chromatography, e.g., high performance liquid chromatography using a chiral adsorbent (HPLC: column: Chiralpak AD-H (4.6mm*250mm, 5μm); mobile phase: *n*-hexane: ethanol=40:60, isocratic elution; flow rate: 1 mL/min). Preferred enantiomers can also be prepared by asymmetric syntheses. See, for example, Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

As described herein, compounds disclosed herein may optionally be substituted with one or more substituents, such as are illustrated generally below, or as exemplified by particular classes, subclasses, and species of the invention.

In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

The term "unsubstituted" means that the specified group has no substituents.

The term "optionally substituted" may be used interchangeably with the term "unsubstituted or substituted", *i.e.,* the structure is unsubstituted or substituted with one or more substituents described in the present invention. The substituents described in the present invention include, but are not limited to, D, oxo (=O), F, Cl, Br, I, CN, $NO_2$, OH, SH, $NH_2$, carboxyl, aldehyde, alkyl, haloalkyl, alkenyl, alkynyl, alkoxy, haloalkoxy, alkylamino, alkylenealkoxy, alkylene-cycloalkyl, cycloalkylamino, carbocyclyl, cycloalkyl, heterocyclyl, aryl, heteroaryl, spiroheterocyclyl, fused heterocyclyl, spirocyclyl, fused cyclyl, and the like.

Furthermore, what need to be explained is that the phrases "each...is independently" and "each of...and...is independently", unless otherwise stated, should be broadly understood, which can mean that the specific options expressed by the same symbol are independent of each other in different groups; or the specific options expressed by the same symbol are independent of each other in same groups.

At each part of the present specification, substitutes of compounds disclosed herein are disclosed in groups or in ranges. It is specifically intended that the invention includes each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl, and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" or "alkyl group" refers to a saturated linear or branched-chain monovalent hydrocarbon group, wherein the alkyl group is optionally substituted with one or more substituents described herein. Unless otherwise stated, the alkyl group contains 1-20 carbon atoms. In one embodiment, the alkyl group contains 1-12 carbon atoms. In another embodiment, the alkyl group contains 3-12 carbon atoms. In another embodiment, the alkyl group contains 1-6 carbon atoms. In still other embodiment, the alkyl group contains 1-4 carbon atoms. In yet other embodiment, the alkyl group contains 1-3 carbon atoms.

Some non-limiting examples of the alkyl group include, methyl (Me, $-CH_3$), ethyl (Et, $-CH_2CH_3$), *n*-propyl (*n*-Pr, $-CH_2CH_2CH_3$), isopropyl (i-Pr, $-CH(CH_3)_2$), *n*-butyl (*n*-Bu, $-CH_2CH_2CH_2CH_3$), isobutyl *(*i-Bu, $-CH_2CH(CH_3)_2$), *sec*-butyl (s-Bu, $-CH(CH_3)CH_2CH_3$), *tert-butyl (*t-Bu, $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, *n*-heptyl and *n*-octyl, *etc.*

The term "alkylene" refers to a saturated divalent hydrocarbon group derived from a straight or branched chain saturated hydrocarbon by the removal of two hydrogen atoms. Unless otherwise specified, the alkylene group contains 1-12 carbon atoms. In some embodiments, the alkylene group contains 1-6 carbon atoms. In other embodiments, the alkylene group contains 1-4 carbon atoms. In still other embodiments, the alkylene group contains 1-3 carbon atoms. In yet other embodiments, the alkylene group contains 1-2 carbon atoms. Alkylene group is exemplified by methylene ($-CH_2-$), ethylene ($-CH_2CH_2-$), isopropylene ($-CH(CH_3)CH_2-$), and the like.

The term "alkoxy" refers to an alkyl group, as previously defined, attached to parent molecular moiety via an oxygen atom. Unless otherwise specified, the alkoxy group contains 1-12 carbon atoms. In one embodiment, the alkoxy group contains 1-6 carbon atoms. In other embodiment, the alkoxy group contains 1-4 carbon atoms. In still other embodiment, the alkoxy group contains 1-3 carbon atoms. The alkoxy group may be optionally substituted with one or more substituents disclosed herein.

Some non-limiting examples of the alkoxy group include, but are not limited to, methoxy (MeO, $-OCH_3$), ethoxy (EtO, $-OCH_2CH_3$), *n*-propoxy (*n*-PrO, *n*-propoxy, $-OCH_2CH_2CH_3$), isopropoxy (*i*-PrO, *i*-propoxy, $-OCH(CH_3)_2$), *n*-butoxy (*n*-BuO, *n*-butoxy, $-OCH_2CH_2CH_2CH_3$), isobutoxy (*i*-BuO, *i*-butoxy, $-OCH_2CH(CH_3)_2$), 2-butoxy (s-BuO, *s*-butoxy, $-OCH(CH_3)CH_2CH_3$), *tert*-butoxy (*t*-BuO, *t*-butoxy, $-OC(CH_3)_3$), *n*-pentoxy (*n*-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-1-butoxy ($-OCH_2CH_2CH(CH_3)_2$), 2-methyl-1-butoxy ($-OCH_2CH(CH_3)CH_2CH_3$), and the like.

The term "alkylamino" embraces "*N*-alkylamino" and "*N,N*-dialkylamino", wherein an amino group is independently substituted with one or two alkyl groups, the alkyl group is as defined herein. In some embodiments, the alkylamino group is lower alkylamino group having one or two $C_{1-6}$ alkyl groups attached to a nitrogen atom. In another embodiment, the alkylamino group is lower alkylamino group having one or two $C_{1-4}$ alkyl groups attached to a nitrogen atom. Some non-limiting examples of suitable alkylamino radical include mono or dialkylamino. Some examples include, but are not limited to, *N*-methylamino, *N*-ethylamino, *N,N*-dimethylamino and *N,N*-diethylamino, and the like.

The term "haloalkyl" or "haloalkoxy" respectively refers to an alkyl or alkoxy group, as the case may be, substituted with one or more halogen atoms, and wherein each of the alkyl or alkoxy is defined as described herein. Examples of such groups include, but are not limited to, difluromethyl, trifluoromethyl, 2,2,2-trifluoroethoxy, 2,2,3,3-tetrafluoropropoxy, difluoromethoxy, trifluoromethoxy, and the like.

The term "hydroxy-substituted alkyl" or "hydroxy-substituted haloalkyl" means that an alkyl or haloalkyl group is substituted with one or more hydroxyl groups, wherein the alkyl or haloalkyl groups have the meaning as described herein. Some non-limiting examples of such groups include hydroxymethyl, 1-hydroxyethyl, 2-hydroxyethyl, 2-hydroxy-1,1,1,3,3,3-hexafluoroisopropyl, and the like. For example, "hydroxy-substituted $C_{1-6}$ alkyl" means that an alkyl group having 1 to 6 carbon atoms is substituted with a hydroxyl group.

The term "amino-substituted alkyl" refers to an alkyl group substituted with one or more amino groups, wherein the alkyl group has the meaning as described herein. Some non-limiting examples of such groups include, $-CH_2NH_2$, $-CH_2CH_2NH_2$, $-CH(NH_2)CH(CH_3)_2$ and the like.

The term "cycloalkyl" refers to a monovalent or multivalent saturated ring having 3 to 12 carbon atoms as a monocyclic, bicyclic, or tricyclic ring system. In some embodiments, the cycloalkyl group contains 7 to 12 carbon atoms. In other embodiments, the cycloalkyl group contains 3 to 8 carbon atoms. In still other embodiments, the cycloalkyl group contains 3 to 6 carbon atoms. The cycloalkyl group may be independently and optionally substituted with one or more substituents disclosed herein. Examples of cycloalkyl groups include, but are not limited to, cyclopropyl, cyclobutyl, cyclopentyl, cyclohexyl and the like.

The term "cycloalkylamino" embraces "*N*-cycloalkylamino" and "*N,N*-cycloalkylamino", wherein an amino group is independently substituted with one or two cycloalkyl groups and wherein the cycloalkyl group is as defined herein. In some embodiments, cycloalkylamino is a cycloalkylamino group having one or two $C_{3-8}$ cycloalkyl groups attached to a nitrogen atom. In another embodiment, cycloalkylamino is a cycloalkylamino group having one or two $C_{3-6}$ cycloalkyl groups attached to a nitrogen atom. Some non-limiting examples of suitable cycloalkylamino radical include mono or dicycloalkylamino. Some examples include, but are not limited to, *N*-cyclopropylamino, *N*-cyclobutylamino, *N*-cyclohexylamino, *N,N*-dicyclopropylamino, and the like.

The terms "cycloalkylalkyl" or "-alkylene-cycloalkyl" are used interchangeably herein to mean that a cycloalkyl group is attached to an alkyl (or alkylene) group through a cycloalkyl group, wherein the cycloalkyl, alkyl or alkylene has the meaning as defined herein. Some non-limiting examples of such group include cyclopropylmethyl, cyclobutylmethyl, cyclopentylmethyl, cyclohexylmethyl, cyclopropylethyl, cyclobutylethyl, cyclopentylethyl, cyclohexylethyl and the like.

The term "carbocyclyl", "carbocycle" or "carbocyclic ring" refers to a monovalent or multivalent, nonaromatic, saturated or partially unsaturated ring having 3 to 12 carbon atoms as a monocyclic, bicyclic or tricyclic ring system. A carbobicyclyl group includes a spiro carbobicyclyl group or a fused carbobicyclyl group. Suitable carbocyclyl groups include, but are not limited to, cycloalkyl, cycloalkenyl and cycloalkynyl. In some embodiments, the carbocyclyl group contains 3 to 10 carbon atoms. In other embodiments, the carbocyclyl group contains 3 to 8 carbon atoms. In still other

embodiments, the carbocyclyl group contains 3 to 6 carbon atoms. Further examples of carbocyclyl groups include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, and the like. The carbocyclyl group may be independently and optionally substituted with one or more substituents disclosed herein.

The terms "heterocyclyl" and "heterocycle" are used interchangeably herein, refer to a saturated or partially unsaturated non-aromatic monovalent or polyvalent monocyclic, bicyclic or tricyclic ring system containing 3 to 12 ring atoms, wherein at least one ring member is selected from nitrogen, sulfur and oxygen; polycyclic heterocyclic groups include spiro heterocyclic groups and fused heterocyclic groups. Wherein, in some embodiments, heterocyclyl is a 3- to 10-membered heterocyclyl; in other embodiments, heterocyclyl is a 3- to 8-membered heterocyclyl; in still other embodiments, heterocyclyl is a 3- to 6-membered heterocyclyl; and in some embodiments, heterocyclyl is a 5- to 6-membered heterocyclyl. Unless otherwise specified, the heterocyclyl group may be carbon or nitrogen linked, and a $-CH_2-$ group can be optionally replaced by a -C(=O)- group. In which, the sulfur can be optionally oxygenized to *S*-oxide and the nitrogen can be optionally oxygenized to *N*-oxide. Examples of the heterocyclyl group include, but are not limited to, oxiranyl, azetidinyl, oxetanyl, thietanyl, pyrrolidinyl (e.g., 2-pyrrolidinyl, 3-pyrrolidinyl), pyrazolidinyl, imidazolidinyl, tetrahydrofuranyl, dihydrofuranyl, tetrahydrothienyl, dihydrothienyl, 1,3-dioxolanyl, dithiolanyl, tetrahydropyranyl, dihydropyranyl, 2*H*-pyranyl, 4*H*-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, diazepanyl, oxepanyl, thiepanyl, oxazepinyl, diazepinyl and thiazepinyl, and the like. Some non-limiting examples of the heterocyclyl group wherein $-CH_2-$ group is replaced by -C(=O)- moiety include 2-oxopyrrolidinyl, oxo-1,3-thiazolidinyl, 2-piperidinonyl, pyridazinonyl, 3,5-dioxopiperidinyl, pyrimidinedione-yl, and the like. Some non-limited examples of heterocyclyl wherein the ring sulfur atom is oxidized is sulfolanyl, 1,1-dioxo-thiomorpholinyl. The heterocyclyl group may be optionally substituted with one or more substituents disclosed herein.

The term "spirocyclyl" refers to a saturated or partially unsaturated, non-aromatic, monovalent or polyvalent bicyclic or tricyclic ring containing 5 to 12 ring atoms, wherein the ring shares a carbon atom with the ring and the ring atoms are all carbon atoms. Unless otherwise specified, the spirocyclyl group may be attached to other groups in the molecule through carbon atom, and a $-CH_2-$ group can be optionally replaced by a -C(=O)- group. Some non-limiting examples of the spirocyclic ring include:

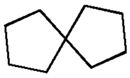, 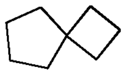, 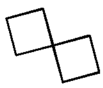, 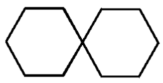.

The terms "spiro heterocyclyl" and "spiro heterocyclic ring" are used interchangeably herein, refer to a saturated or partially unsaturated non-aromatic monovalent or polyvalent bicyclic or tricyclic ring system containing 5 to 12 ring atoms, wherein at least one ring member is selected from nitrogen, sulfur and oxygen, and the ring shares a carbon atom with the ring. Wherein, in some embodiments, spiro heterocyclyl is a 5- to 12-membered spiro heterocyclyl; in other embodiments, spiro heterocyclyl is a 7- to 12-membered spiro heterocyclyl. Unless otherwise specified, the spiro heterocyclyl group may be carbon or nitrogen linked, and a $-CH_2-$ group can be optionally replaced by a -C(=O)- group. In which, the sulfur can be optionally oxygenized to *S*-oxide and the nitrogen can be optionally oxygenized to *N*-oxide. Some non-limiting examples of the spiro heterocyclic ring group include:

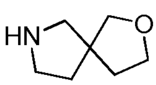, 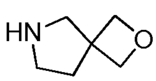, 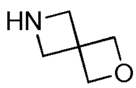, 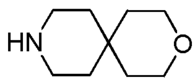, 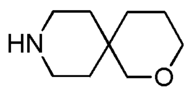, 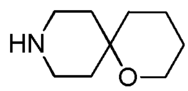,

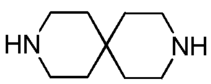, 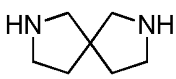, 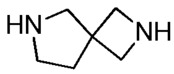, 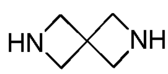, 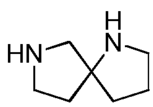, 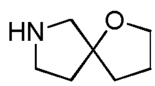,

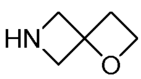, 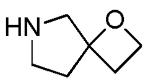, 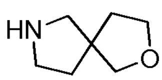, 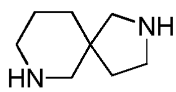, 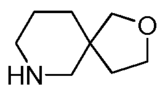, 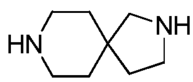,

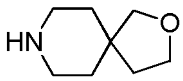.

The term "fused cyclyl" refers to a saturated or partially unsaturated, non-aromatic, monovalent or polyvalent bicyclic or tricyclic ring containing 4 to 12 ring atoms, wherein the ring shares a ring edge (shares a single bond) with the ring, and the ring atoms are all carbon atoms. Unless otherwise specified, the fused cyclyl group may be attached to other groups in the molecule through carbon atoms, and a $-CH_2-$ group can be optionally replaced by a -C(=O)- group. Some non-limiting examples of the fused cyclylic ring include:

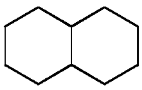, 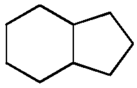, 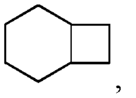, 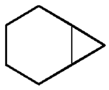, 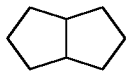, 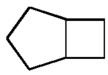.

The terms "fused heterocyclyl", "fused heterocycle" and "fused heterocyclic ring" are used interchangeably herein, refer to a saturated or partially unsaturated non-aromatic monovalent or polyvalent bicyclic or tricyclic ring system containing 4 to 12 ring atoms, wherein at least one ring member is selected from nitrogen, sulfur and oxygen, and the ring shares a ring edge (shares a single bond) with the ring. Wherein, in some embodiments, the fused heterocyclyl group contains 3-9 carbon atoms in the 4-12 ring atoms; in other embodiments, the fused heterocyclyl group contains 3-8 carbon atoms in the 4-12 ring atoms; in still other embodiments, the fused heterocyclyl group contains 3-6 carbon atoms in the 4-12 ring atoms. Unless otherwise specified, the fused heterocyclyl group may be carbon or nitrogen linked, and a $-CH_2-$ group can be optionally replaced by a -C(=O)-group. In which, the sulfur can be optionally oxygenized to *S*-oxide and the nitrogen can be optionally oxygenized to *N*-oxide. Some non-limiting examples of the fused heterocyclic ring include:

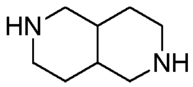, 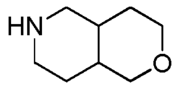, 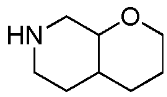, 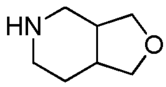, 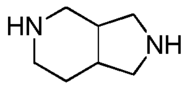, 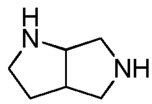,

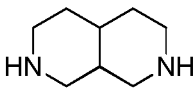, 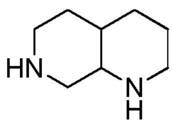, 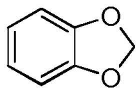.

The term "aryl" refers to monocyclic, bicyclic and tricyclic carbocyclic ring systems having a total of six to fourteen ring members, or six to twelve ring members, or six to ten ring members, in which at least one ring is aromatic and has a single point or multipoint of attachment to the rest of the molecule. The term "aryl" and "aromatic ring" can be used interchangeably herein. In one embodiment, aryl is a carbocyclic ring system consisting of 6-10 ring atoms and containing at least one aromatic ring. Examples of the aryl group may include phenyl, naphthyl and anthracenyl. The aryl group may be independently and optionally substituted with one or more substituents disclosed herein.

The term "heteroaryl" refers to monocyclic, bicyclic and tricyclic ring systems having a total of five to twelve ring members, in which at least one ring is aromatic and at least one ring contains one or more heteroatoms; the heteroaryl group has a single point or multipoint of attachment to the rest of the molecule. The term "heteroaryl" and "heteroaromatic ring" or "heteroaromatic compound" can be used interchangeably herein. Wherein, in some embodiments, heteroaryl is a 5- to 12-membered heteroaryl; in other embodiments, heteroaryl is a 5- to 10-membered heteroaryl; in still other embodiments, heteroaryl is a 5- to 7-membered heteroaryl, and the 5- to 7-membered heteroaryl includes a heteroaryl group consisting of 5, 6 or 7 ring atoms. In some embodiments, heteroaryl is a heteroaryl group consisting of 5-12 ring atoms comprising 1, 2, 3 or 4 heteroatoms independently selected from O, S and N; in other embodiments, heteroaryl is a heteroaryl group consisting of 5-10 ring atoms comprising 1, 2, 3 or 4 heteroatoms independently selected from O, S and N; in still other embodiments, heteroaryl is a heteroaryl group consisting of 5-6 ring atoms comprising 1, 2, 3 or 4 heteroatoms independently selected from O, S and N. The heteroaryl group is optionally substituted with one or more substituents disclosed herein.

Examples of heteroaryl include, but are not limited to, furyl (such as 2-furyl, 3-furyl), imidazolyl (such as *N*-imidazolyl, 2-imidazolyl, 4-imidazolyl, 5-imidazolyl), isoxazolyl (such as 3-isoxazolyl, 4-isoxazolyl, 5-isoxazolyl), oxazolyl (such as 2-oxazolyl, 4-oxazolyl, 5-oxazolyl), oxadiazolyl (such as 1,2,3-oxadiazolyl, 1,2,5-oxadiazolyl, 1,2,4-oxadiazolyl),

oxatriazolyl (such as 1,2,3,4-oxatriazolyl), thiazolyl (such as 2-thiazolyl, 4-thiazolyl, 5-thiazolyl), isothiazolyl, 2-thiadiazolyl (such as 1,3,4-thiadiazolyl, 1,2,3-thiadiazolyl, 1,2,5-thiadiazolyl), thiatriazolyl (such as 1,2,3,4-thiatriazolyl), tetrazolyl (such as 2*H*-1,2,3,4-tetrazolyl, 1*H*-1,2,3,4-tetrazolyl), triazolyl (such as 2*H*-1,2,3-triazolyl, 1*H*-1,2,4-triazolyl, 4*H*-1,2,4-triazolyl), thienyl (such as 2-thienyl, 3-thienyl), 1*H*-pyrazolyl (such as 1*H*-pyrazol-3-yl, 1*H*-pyrazol-4-yl, 1*H*-pyrazol-5-yl), 1,2,3-thiodiazolyl, 1,3,4-thiodiazolyl, 1,2,5-thiodiazolyl, pyrrolyl (such as *N*-pyrrolyl, 2-pyrrolyl, 3-pyrrolyl), pyridyl (such as 2-pyridyl, 3-pyridyl, 4-pyridyl), pyrimidinyl (such as 2-pyrimidinyl, 4-pyrimidinyl, 5-pyrimidinyl), pyridazinyl (such as 3-pyridazinyl, 4-pyridazinyl), 2-pyrazinyl, triazinyl (such as 1,3,5-triazinyl), tetrazinyl (such as 1,2,4,5-tetrazinyl, 1,2,3,5-tetrazinyl); examples of heteroaryl also include, but are in no way limited to, the following bicycles: benzimidazolyl, benzopyrazolyl (such as

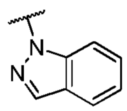, 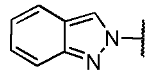),

benzofuranyl, benzothienyl, benzobisoxazolyl, indolyl (such as 2-indolyl), purinyl, quinolinyl (such as 2-quinolinyl, 3-quinolinyl, 4-quinolinyl), isoquinolinyl (such as 1-isoquinolinyl, 3-isoquinolinyl or 4-isoquinolinyl), imidazo[1,2-*a*]pyiidyl, pyrazolo[1,5-*a*]pyridyl, pyrazolo[1,5-*a*]pyrimidinyl, imidazo[1,2-*b*]pyridazinyl, [1,2,4]triazolo[4,3-*b*]pyridazinyl, [1,2,4]triazolo[1,5-a]pyrimidinyl, [1,2,4]triazolo[1,5-a]pyridyl,

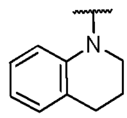, 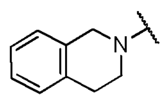,

*etc.*

The term "unsaturated" refers to a moiety having one or more units of unsaturation.

The term "heteroatom" refers to one or more of oxygen, sulfur, nitrogen, phosphorus and silicon, including any oxidized form of nitrogen, sulfur, or phosphorus; the quaternized form of any basic nitrogen; or a substitutable nitrogen of a heterocyclic ring, for example, N (as in 3,4-dihydro-2*H*-pyrrolyl), NH (as in pyrrolidinyl) or NR (as in *N*-substituted pyrrolidinyl).

The term "halogen" or "halogen atom" refers to fluorine atom (F), chlorine atom (Cl), bromine atom (Br) or iodine atom (I).

The term "cyano" or "CN" refers to a cyano structure, and such group can be attached to other groups.

The term "nitro" or "$NO_2$" refers to a nitro structure, and such group can be attached to other groups.

The term "amino" or "$NH_2$" refers to an amino structure, and such group can be attached to other groups.

The terms "carboxy" or "carboxyl", whether used alone or with other terms, such as "carboxyalkyl", refer to $-CO_2H$. The term "carbonyl", whether used alone or with other terms, such as "aminocarbonyl" or "acyloxy", represents -(C=O)-.

The term "j- to k-membered" means that the cyclic group consists of j to k ring atoms, and the ring atoms include carbon atoms and/or heteroatoms such as O, N, S, P, *etc.;* each of j and k is independently any non-zero natural numbers, and k>j; the "j-k" or "j to k" or "j - to k-" includes j, k and any natural numbers between them. For example, "5- to 12-membered", "5- to 10-membered" or "3- to 7-membered" means that the cyclic group consists of 5-12 *(i.e.,* 5, 6, 7, 8, 9, 10, 11 or 12), 5-10 *(i.e.,* 5, 6, 7, 8, 9 or 10), 5-6 *(i.e.,* 5 or 6) or 3-7 *(i.e.,* 3, 4, 5, 6 or 7) ring atoms, and the ring atoms include carbon atoms and/or heteroatoms such as O, N, S, P, *etc.*

A bond drawn from a substituent to the center of one ring within a ring system (as shown in Figure b) represents substitution of the substituent at any substitutable or reasonable position on the ring. For example, formula b represents mono- or poly-substitution of the substituent R' at any substitutable position on the ring, including but not limited to those shown in formula b1 to formula b9.

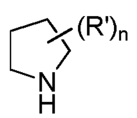
formula b

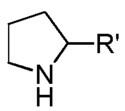
formula b1

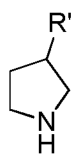
formula b2

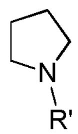
formula b3

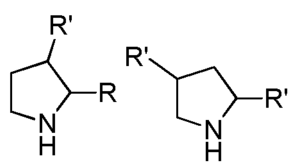
formula b4 formula b5

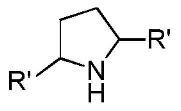
formula b6

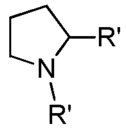
formula b7

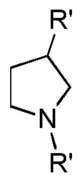
formula b8

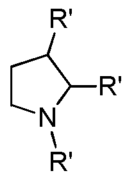
formula b9

As described herein, a linker attached to a ring system (as shown in formula c) means that the linker can be attached to the rest of the molecule at any linkable position on the ring system. Formula c represents that the ring can be connected to the rest of the molecule through any possible attachment position on the ring, including but not limited to those shown in formula c1 to formula c6.

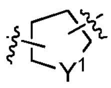
formula c

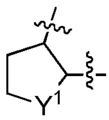
formula c1

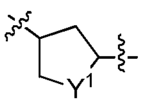
formula c2

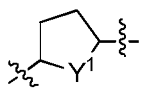
formula c3

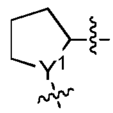
formula c4

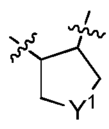
formula c5

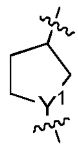
formula c6 .

As described herein, when a group is attached to other parts of the molecule through two sites, unless otherwise stated, each of the two sites is independently and optionally attached to other groups of the molecule, and the groups connected to the two sites can be interchanged; for example, the piperidinyl in formula e can be linked to other parts of the molecule through the E1 end and the E2 end, and when the other parts of the molecule remain unchanged, the E1 end and the E2 end can be interchanged.

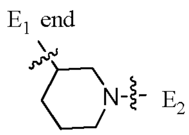
formula d

end

The term "protecting group" or "PG" refers to a substituent that is commonly employed to block or protect a particular functionality while reacting with other functional groups on the compound. For example, an "amino-protecting group" is a substituent attached to an amino group that blocks or protects the amino functionality in the compound. Suitable amino-protecting groups include acetyl, trifluoroacetyl, *t*-butoxy-carbonyl (BOC, Boc), benzyloxycarbonyl (CBZ, Cbz) and 9-fluorenylmethylenoxy-carbonyl (Fmoc). Similarly, a "hydroxy-protecting group" refers to a substituent of a hydroxy group that blocks or protects the hydroxy functionality. Suitable protecting groups include acetyl and silyl. A "carboxy-protecting group" refers to a substituent of the carboxy group that blocks or protects the carboxy functionality. Common carboxy-protecting groups include $-CH_2CH_2SO_2Ph$, cyanoethyl, 2-(trimethylsilyl)ethyl, 2-(trimethylsilyl)ethoxy-methyl, 2-(*p*-toluenesulfonyl) ethyl, 2-(*p*-nitrophenylsulfonyl)-ethyl, 2-(diphenylphosphino)-ethyl, nitroethyl and the like. For a general description of protecting groups and their use, see T. W. Greene, Protective Groups in Organic Synthesis, John Wiley & Sons, New York, 1991; and P. J. Kocienski, Protecting Groups, Thieme, Stuttgart, 2005.

The term "prodrug" refers to a compound that is transformed *in vivo* into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_1$-$C_{24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry,

2008, 51, 2328-2345, all of which are incorporated herein by reference in their entireties.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salt" refers to organic or inorganic salts of a compound disclosed herein. Pharmaceutically acceptable salts are well known in the art. For example, S. M. Berge et al., describe pharmaceutically acceptable salts in detail in J. Pharmaceutical Sciences, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, *p*-toluenesulfonate, undecanoate, valerate, and the like. Salts derived from appropriate bases include alkali metal, alkaline earth metal, ammonium and $N^+(C_{1-4}\ alkyl)_4$ salts. This invention also envisions the quaternization of any basic nitrogen-containing groups of the compounds disclosed herein. Water or oil soluble or dispersable products may be obtained by such quaternization. Representative alkali or alkaline earth metal salts include sodium, lithium, potassium, calcium, magnesium, and the like. Further pharmaceutically acceptable salts include appropriate and nontoxic ammonium, quaternary ammonium, and amine cations formed using counterions, such as halide, hydroxide, carboxylate, sulfate, phosphate, nitrate, $C_{1-8}$ sulfonate or aryl sulfonate.

The term "solvate" refers to an association or complex of one or more solvent molecules and a compound disclosed herein. Examples of solvents that form solvates include, but are not limited to, water, isopropanol, ethanol, methanol, DMSO, ethyl acetate, acetic acid and ethanolamine. The term "hydrate" refers to the complex where the solvent molecule is water.

The term "hydrate" can be used when said solvent is water. In one embodiment, one solvent molecule is associated with one molecule of the compounds disclosed herein, such as a monohydrate. In another embodiment, more than one solvent molecule may be associated with one molecule of the compounds disclosed herein, such as a dihydrate. In still another embodiment, less than one solvent molecule may be associated with one molecule of the compounds disclosed herein, such as a hemihydrate. Furthermore, all the solvates of the invention retain the biological effectiveness of the non-hydrate form of the compounds disclosed herein.

The term "*N*-oxide" refers to one or more than one nitrogen atoms oxidised to form an *N*-oxide, where a compound contains several amine functions. Particular examples of *N*-oxides are the *N*-oxides of a tertiary amine or a nitrogen atom of a nitrogen-containing heterocycle. *N*-oxides can be formed by treatment of the corresponding amine with an oxidizing agent such as hydrogen peroxide or a per-acid (*e.g.,* a peroxycarboxylic acid) (See, Advanced Organic Chemistiy, by Jerry March, 4th Edition, Wiley Interscience, pages). More particularly, N-oxides can be made by the procedure of L. W. Deady (Syn. Comm. 1977, 7, 509-514) in which the amine compound is reacted with *m*-chloroperoxybenzoic acid (MCPBA), for example, in an inert solvent such as dichloromethane.

The term "carrier" includes any solvents, dispersion media, coating agents, surfactants, antioxidants, preservatives (e.g., antibacterial agents, antifungal agents), isotonic agents, salt, drug stabilizers, binders, excipients, dispersants, lubricants, sweetening agents, flavoring agents, coloring agents, or a combination thereof, all of which are well kown to the skilled in the art. (*e.g.,* Remington's Pharmaceutical Sciences, 18th Ed. Mack Printing Company, 1990, pp. 1289-1329, all of which are incorporated herein by reference). Except any conventional carrier is incompatible with the active ingredient, the pharmaceutically acceptable carriers are effectively used in the treatment or pharmaceutical compositions.

As used herein, the "treat", "treating" or "treatment" of any disease or disorder refers to all that can slow, interrupt, arrest, control or stop the progression of the disease or disorder, but does not necessarily mean that all symptoms of the disease or disorder disappear, which also includes prophylactic treatment of said symptoms, especially in patients prone to such disease or disorder. In some embodiments, the "treat", "treating" or "treatment" of any disease or disorder refers to ameliorating the disease or disorder *(i.e.,* slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically,

(e.g., stabilization of a discernible symptom), physiologically, (*e.g.*, stabilization of a physical parameter), or both. In yet another embodiment, "treat", "treating" or "treatment" refers to preventing or delaying the onset or development or progression of the disease or disorder.

The term "therapeutically effective amount" or "therapeutically effective dose" as used herein refers to the amount of a compound of the present invention that is capable of eliciting a biological or medical response (For example, reducing or inhibiting the activity of enzyme or protein, or improving symptoms, alleviating symptoms, slowing or delaying progression of disease, or preventing disease, etc.) in a subject. In a non-limiting embodiment, the term "therapeutically effective amount" refers to an amount effective to: (1) at least partially alleviate, inhibit, prevent and/or ameliorate a disorder or disease (i) mediated by RORγt, or (ii) associated with the activity of RORγt, or (iii) characterized by aberrant activity of RORγt; or (2) reduce or inhibit the activity of RORγt; or (3) reduce or inhibit the expression of RORγt when a compound of the present invention is administered to an individual. In another embodiment, the term "therapeutically effective amount" refers to an effective amount of a compound of the invention capable of at least partially reducing or inhibiting the activity of RORγt; or at least partially reducing or inhibiting the expression of RORγt when administered to a cell, or organ, or non-cellular biological material, or vehicle.

The term "administering" a compound as used herein are to be understood as providing a compound of the present invention or a prodrug of a compound of the present invention to an individual in need thereof. It will be appreciated that one of skill in the art treats a patient currently suffering from this disorder or prophylactically treats a patient suffering from this disorder by administering an effective amount of a compound of the present invention.

The term "composition" as used herein refers to a product comprising the specified ingredients in the specified amounts, as well as any product that results, directly or indirectly, from combination of the specified ingredients in the specified amounts. The meaning of this term in relation to a pharmaceutical composition includes a product comprising the active ingredient (single or multiple) and inert ingredient (single or multiple) that make up the carrier, as well as a mixture, complex or aggregate of any two or more ingredients, or any product that results directly or indirectly from the decomposition of one or more components, or from other types of reactions or interactions of one or more components. Accordingly, the pharmaceutical compositions of the present invention include any composition prepared by admixing a compound of the present invention and a pharmaceutically acceptable carrier.

## DESCRIPTION OF COMPOUNDS OF THE INVENTION

The invention discloses a class of sulfonyl-substituted (hetero) aromatic ring derivatives, pharmaceutically acceptable salts thereof, pharmaceutical preparations and compositions thereof, which can be used as RORγt inhibitors, and have potential use in the treatment of cancer, inflammation or autoimmune diseases mediated by RORγt, such as cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

In one aspect, the present invention provides a compound having Formula (I), or a stereoisomer, an *N*-oxide, a solvate, a metabolite, a pharmaceutically acceptable salt or a prodrug thereof,

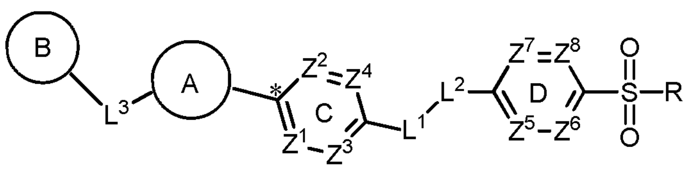

(I),

wherein, ring A, ring B, $L^1$, $L^2$, $L^3$, R, $Z^1$, $Z^2$, $Z^3$, $Z^4$, $Z^5$, $Z^6$, $Z^7$ and $Z^8$ have the meanings described in the present invention; * indicates the direction in which ring A is connected to ring C.

In some embodiments, R is $C_{1-6}$ alkyl, $C_{1-6}$ alkoxy, $C_{1-6}$ alkylamino, $C_{3-8}$ cycloalkylamino, -$C_{1-6}$ alkylene-$C_{3-8}$ cycloalkyl, $C_{3-8}$ cycloalkyl, $C_{1-6}$ haloalkyl or $C_{1-6}$ haloalkoxy.

In other embodiments, R is $C_{1-4}$ alkyl, $C_{1-4}$ alkoxy, $C_{1-4}$ alkylamino, $C_{3-6}$ cycloalkylamino, -$C_{1-4}$ alkylene-$C_{3-6}$ cycloalkyl, $C_{3-6}$ cycloalkyl, $C_{1-4}$ haloalkyl or $C_{1-4}$ haloalkoxy.

In yet other embodiments, wherein R is methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, methylamino, ethylamino, dimethylamino, cyclopropylamino, cyclobutylamino, cyclopentylamino, cyclohexylamino, -methylene-cyclopropyl, -methylene-cyclobutyl, -methylene-cyclopentyl, -methylene-cyclohexyl, -ethylene-cyclopropyl, -ethylene-cyclobutyl, -ethylene-cyclopentyl, -ethylene-cyclohexyl, cyclopropyl, cyclobutyl, cyclopentyl or cyclohexyl.

In some embodiments, $Z^1$ is $CR^1$ or N, wherein $R^1$ has the meaning as described herein.

In some embodiments, $Z^2$ is $CR^1$ or N, wherein $R^1$ has the meaning as described herein.

In some embodiments, $Z^3$ is $CR^1$ or N, wherein $R^1$ has the meaning as described herein.

In some embodiments, $Z^4$ is $CR^1$ or N, wherein $R^1$ has the meaning as described herein.

In some embodiments, $Z^5$ is $CR^1$ or N, wherein $R^1$ has the meaning as described herein.

In some embodiments, $Z^6$ is $CR^1$ or N, wherein $R^1$ has the meaning as described herein.

In some embodiments, $Z^7$ is $CR^1$ or N, wherein $R^1$ has the meaning as described herein.

In some embodiments, $Z^8$ is $CR^1$ or N, wherein $R^1$ has the meaning as described herein.

In some embodiments, each $R^1$ is independently hydrogen, deuterium, cyano, fluorine, chlorine, bromine, iodine, $C_{1-6}$ alkyl, $C_{1-6}$ haloalkyl, hydroxy-substituted $C_{1-6}$ alkyl, -$C_{1-6}$ alkylene-$C_{1-6}$ alkoxy, $C_{1-6}$ alkoxy, $C_{3-8}$ cycloalkyl or 3- to 7-membered heterocyclyl.

In other embodiments, wherein each $R^1$ is independently hydrogen, deuterium, cyano, fluorine, chlorine, bromine, iodine, methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, hydroxymethyl, 2-hydroxyethyl, -methylenemethoxy, -methyleneethoxy, -methylene-*n*-propoxy, -methyleneisopropoxy, cyclopropyl, cyclobutyl, cyclopentyl or cyclohexyl.

In some embodiments, ring A is 3- to 5-membered heterocyclyl or 7-membered heterocyclyl, and each of the 3- to 5-membered heterocyclyl and 7-membered heterocyclyl is independently optionally substituted with 1, 2, 3, 4 or 5 $R^2$; wherein * represents the direction in which ring A is connected to ring C and $R^2$ has the meaning as described in the present invention.

In other embodiments, ring A is

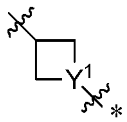, 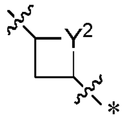, 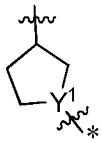, 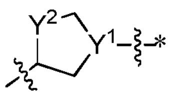,

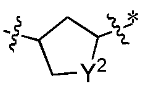, 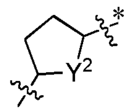, 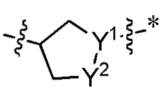, 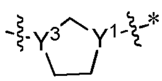, 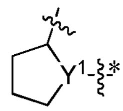, 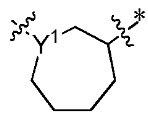, 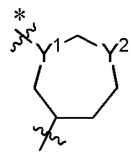,

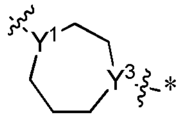 or 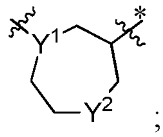;

wherein, each $Y^1$ and $Y^3$ is independently N or CH; $Y^2$ is O, S, NH or $CH_2$; * represents the direction in which ring A is connected to ring C; the ring A is optionally substituted with 1, 2, 3, 4 or 5 $R^2$; $R^2$ has the meaning as described in the present invention.

In other embodiments, ring A is

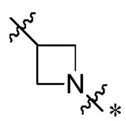, 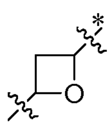, 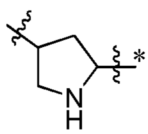, 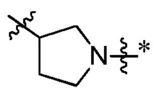,

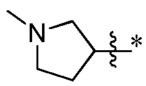, 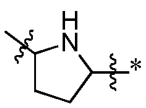, 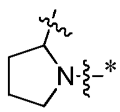, 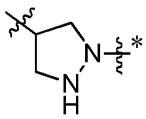, 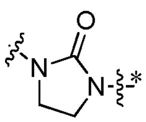, 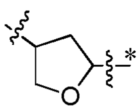, 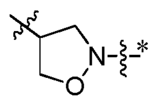,

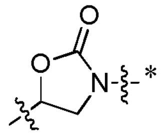, 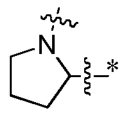, 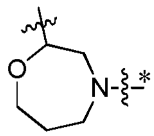, 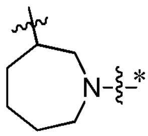, 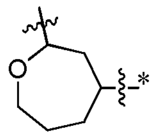, 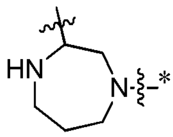, 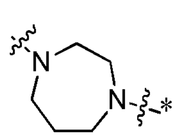,

or

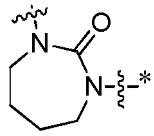;

wherein, * represents the direction in which ring A is connected to ring C; the ring A is optionally substituted with 1, 2, 3, 4 or 5 $R^2$; $R^2$ has the meaning as described in the present invention.

In some embodiments, each $R^2$ is independently oxo, $R^a$, $C_{1-6}$ alkyl, $-C_{1-6}$ alkylene-$R^a$, $-OR^b$, $-C_{1-6}$ alkylene-$OR^b$, $-(C=O)-R^a$, $-C_{1-6}$ alkylene-$(C=O)-R^a$, $-(C=O)-NR^cR^d$ or $-C_{1-6}$ alkylene-$(C=O)-NR^cR^d$, wherein, $R^a$, $R^b$, $R^c$ and $R^d$ have the meanings as described in the present invention.

In other embodiments, each $R^2$ is independently oxo, $R^a$, $C_{1-4}$ alkyl, $-C_{1-4}$alkylene-$R^a$, $-OR^b$, $-C_{1-4}$alkylene-$OR^b$, $-(C=O)-R^a$, $-C_{1-4}$alkylene-$(C=O)-R^a$, $-(C=O)-NR^cR^d$ or $-C_{1-4}$ alkylene-$(C=O)-NR^cR^d$, wherein, $R^a$, $R^b$, $R^c$ and $R^d$ have the meanings as described in the present invention.

In still other embodiments, each $R^2$ is independently oxo, $R^a$, methyl, ethyl, *n*-propyl, isopropyl, *n*-butyl, isobutyl, sec-butyl, tert-butyl, $-CH_2R^a$, $-CH_2CH_2R^a$, $-CH_2CH_2CH_2R^a$, $-C(CH_3)_2R^a$, $-CH(CH_3)CH_2R^a$, $-OR^b$, $-CH_2OR^b$, $-CH_2CH_2OR^b$, $-CH_2CH_2CH_2OR^b$, $-C(CH_3)_2OR^b$, $-CH(CH_3)CH_2OR^b$, $-(C=O)-R^b$, $-CH_2(C=O)-R^b$, $-CH_2CH_2(C=O)-R^b$, $-CH_2CH_2CH_2(C=O)-R^b$, $-C(CH_3)_2(C=O)-R^b$, $-CH(CH_3)CH_2(C=O)-R^b$, $-(C=O)-NR^cR^d$, $-CH_2(C=O)-NR^cR^d$, $-CH_2CH_2(C=O)-NR^cR^d$, $-CH_2CH_2CH_2(C=O)-NR^cR^d$, $-C(CH_3)_2(C=O)-NR^cR^d$ or $-CH(CH_3)CH_2(C=O)-NR^cR^d$, wherein, $R^a$, $R^b$, $R^c$ and $R^d$ have the meanings as described in the present invention.

In some embodiments, each $R^a$ is independently deuterium, fluorine, chlorine, bromine, iodine, hydroxy, amino, nitro, cyano, $C_{1-6}$ alkyl, $C_{1-6}$ alkoxy, $C_{1-6}$ haloalkyl, $C_{3-8}$ cycloalkyl, $C_{6-10}$ aryl, 5- to 10-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;
each $R^b$, $R^c$ and $R^d$ is independently hydrogen, deuterium, $C_{1-6}$ alkyl, $C_{1-6}$ haloalkyl, $C_{1-6}$ alkoxy, $C_{3-8}$ cycloalkyl, $C_{6-10}$ aryl, 5- to 10-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl; wherein each $R^a$, $R^b$, $R^c$ and $R^d$ is independently and optionally substituted with 1, 2, 3, 4, 5 or 6 $R^g$; wherein $R^g$ has the meaning as described in the present invention.

In other embodiments, each $R^a$ is independently deuterium, fluorine, chlorine, bromine, iodine, hydroxy, amino, nitro, cyano, $C_{1-4}$ alkyl, $C_{1-4}$ alkoxy, $C_{1-4}$ haloalkyl, $C_{3-6}$ cycloalkyl, $C_{6-10}$ aryl, 5- to 7-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;
each $R^b$, $R^c$ and $R^d$ is independently hydrogen, deuterium, $C_{1-4}$ alkyl, $C_{1-4}$ haloalkyl, $C_{1-4}$ alkoxy, $C_{3-6}$ cycloalkyl, $C_{6-10}$ aryl, 5- to 7-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl; wherein each $R^a$, $R^b$, $R^c$ and $R^d$ is independently and optionally substituted with 1, 2, 3, 4, 5 or 6 $R^g$; wherein $R^g$ has the meaning as described in the present invention.

In still other embodiments, each $R^a$ is independently deuterium, fluorine, chlorine, bromine, iodine, hydroxy, amino, nitro, cyano, methyl, ethyl, *n*-propyl, isopropyl, *n*-butyl, isobutyl, sec-butyl, tert-butyl, methoxy, ethoxy, isopropoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, oxetanyl, azetidinyl, pyrrolidinyl, tetrahydrofuranyl, tetrahydropyranyl, morpholinyl, thiomorpholinyl, piperidinyl, piperazinyl, oxepanyl, cyclopropyl, cyclobutyl, cyclopentyl, cyclohexyl, phenyl, thiazolyl, pyrazolyl, imidazolyl, furanyl, oxazolyl, isoxazolyl, triazolyl, thienyl, pyrrolyl, pyridyl, pyrimidinyl,

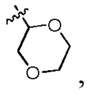, 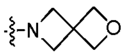, 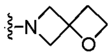, 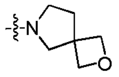, 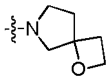, 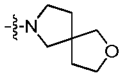, 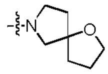, 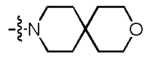,

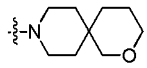 or 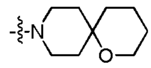;

each $R^b$, $R^c$ and $R^d$ is independently hydrogen, deuterium, methyl, ethyl, *n*-propyl, isopropyl, *n*-butyl, isobutyl, sec-butyl, tert-butyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, methoxy, ethoxy, *n*-propoxy, isopropoxy, *n*-butoxy, isobutoxy, *sec*-butoxy, *tert*-butoxy, cyclopropyl, cyclobutyl, cyclopentyl, cyclohexyl, phenyl, oxetanyl, azetidinyl, pyrrolidinyl, tetrahydrofuranyl, tetrahydropyranyl, morpholinyl, thiomorpholinyl, piperidinyl, piperazinyl, oxepanyl, thiazolyl, pyrazolyl, imidazolyl, furanyl, oxazolyl, isoxazolyl, triazolyl, thienyl, pyrrolyl, pyridyl, pyrimidinyl,

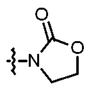, 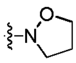, 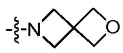, 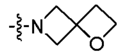, 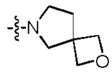, 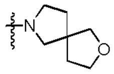, 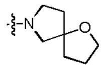, 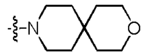,

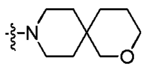 or 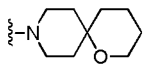;

or, $R^c$ and $R^d$ together with the N atom to which they are attached form

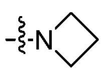, 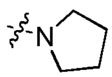, 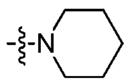 or 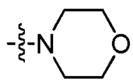;

wherein each $R^a$, $R^b$, $R^c$ and $R^d$ is independently and optionally substituted with 1, 2, 3, 4, 5 or 6 $R^g$; wherein, $R^g$ has the meaning as described in the present invention.

In some embodiments, each $R^g$ is independently deuterium, fluorine, chlorine, bromine, iodine, oxo, hydroxy, amino, nitro, cyano, $C_{1-6}$ alkyl, $C_{1-6}$ haloalkyl, $C_{1-6}$ alkoxy, $C_{1-6}$ haloalkoxy, $C_{3-8}$ cycloalkyl or 3- to 7-membered heterocyclyl.

In other embodiments, each $R^g$ is independently deuterium, fluorine, chlorine, bromine, iodine, oxo, hydroxy, amino, nitro, cyano, $C_{1-4}$ alkyl, $C_{1-4}$ haloalkyl, $C_{1-4}$ alkoxy, $C_{1-4}$ haloalkoxy, $C_{3-6}$ cycloalkyl or 3- to 7-membered heterocyclyl.

In still other embodiments, each $R^g$ is independently hydrogen, deuterium, fluorine, chlorine, bromine, iodine, oxo, hydroxy, amino, nitro, cyano, methyl, ethyl, *n*-propyl, isopropyl, *n*-butyl, isobutyl, sec-butyl, tert-butyl, methoxy, ethoxy, *n*-propoxy, *iso*-propoxy, *n*-butoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$, $-OCF_2CF_3$, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl or oxepanyl.

In some embodiments, ring B is $C_{6-10}$ aryl, $C_{3-8}$ cycloalkyl, 5- to 12-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3 or 4 $R^e$; wherein, $R^e$ has the meaning as described in the present invention.

In other embodiments, ring B is $C_{6-10}$ aryl, $C_{3-6}$ cycloalkyl, 5- to 7-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3, 4, 5 or 6 $R^e$; wherein, $R^e$ has the meaning as described in the present invention.

In still other embodiments, the ring B is phenyl, cyclopropyl, cyclobutyl, cyclopentyl, cyclohexyl, pyridyl, pyrimidinyl, pyrrolyl, pyrazolyl, thiazolyl, imidazolyl, furanyl, oxazolyl, isoxazolyl, thienyl, triazolyl, tetrazolyl, piperazinyl, piperidinyl, morpholinyl, thiomorpholinyl, tetrahydropyranyl, tetrahydrofuranyl, pyrrolidinyl, azetidinyl, oxetanyl,

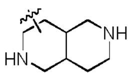, 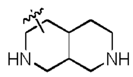,

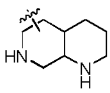, 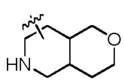, 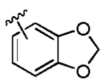, 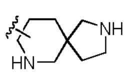, 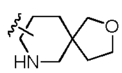, 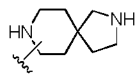 or 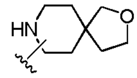;

wherein the ring B is optionally substituted with 1, 2, 3 or 4 $R^e$; $R^e$ has the meaning as described in the present invention.

In some embodiments, each $R^e$ is independently hydrogen, deuterium, fluorine, chlorine, bromine, iodine, cyano, nitro, hydroxy, amino, $C_{1-6}$ alkyl, $C_{1-6}$ alkoxy, $C_{3-8}$ cycloalkyl, 3- to 7-membered heterocyclyl, $C_{1-6}$ haloalkyl or $C_{1-6}$ haloalkoxy, wherein each of the $C_{1-6}$ alkyl, $C_{1-6}$ alkoxy, $C_{3-8}$ cycloalkyl, $C_{1-6}$ haloalkyl and $C_{1-6}$ haloalkoxy is independently and optionally substituted with 1, 2, 3 or 4 substituents selected from cyano, nitro, hydroxy, amino or 3- to 7-membered heterocyclyl.

In other embodiments, each $R^e$ is independently deuterium, fluorine, chlorine, bromine, iodine, cyano, nitro, hydroxy, amino, $C_{1-4}$ alkyl, $C_{1-4}$ alkoxy, $C_{3-6}$ cycloalkyl, 3- to 7-membered heterocyclyl, $C_{1-4}$ haloalkyl or $C_{1-4}$ haloalkoxy, wherein each of the $C_{1-4}$ alkyl, $C_{1-4}$ alkoxy, $C_{3-6}$ cycloalkyl, $C_{1-4}$ haloalkyl and $C_{1-4}$ haloalkoxy is independently and optionally substituted with 1, 2, 3 or 4 substituents selected from cyano, nitro, hydroxy, amino or 3- to 7-membered heterocyclyl.

In still other embodiments, each $R^e$ is independently hydrogen, deuterium, fluorine, chlorine, bromine, iodine, cyano, nitro, hydroxy, methyl, ethyl, *n*-propyl, isopropyl, methoxy, ethoxy, *n*-propoxy, isopropoxy, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$ or $-OCF_2CF_3$; wherein each of the methyl, ethyl, *n*-propyl, isopropyl, methoxy, ethoxy, *n*-propoxy, isopropoxy, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-OCH_2F$, $-OCHF_2$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$ and $-OCF_2CF_3$ is independently and optionally substituted with 1, 2, 3 or 4 substituents selected from cyano, nitro, hydroxy, amino, piperazinyl, piperidinyl, morpholinyl, thiomorpholinyl, tetrahydropyranyl, tetrahydrofuranyl, pyrrolidinyl, azetidinyl or oxetanyl.

In some embodiments, $L^1$ is $-S(O)_2-NH-$, $-NH-S(O)_2-$, -S(O)-NH-, -NH-S(O)-, -C(=O)NH- or -NHC(=O)-; specifically, from left to right, the left end of each linking group in $L^1$ is connected to ring C, for example, $-S(O)_2-NH-$ is attached to ring C through the left sulfur atom.

In some embodiments, $L^2$ is a bond or $-CR^3R^4-$; wherein $R^3$ and $R^4$ have the meanings described herein.

In some embodiments, $L^3$ is -C(=O)-, -S-, -O-, $-NR^5-$ or $-CR^6R^7-$; wherein $R^5$, $R^6$ and $R^7$ have the meanings described herein.

In some embodiments, $R^3$ is hydrogen, deuterium, hydroxy-substituted $C_{1-6}$ alkyl, $-C_{1-6}$ alkylene-O-C(=O)-$R^f$, $-C_{1-6}$ alkylene-C(=O)-O-$R^f$ or $-C_{1-6}$ alkylene-$R^f$; wherein $R^f$ has the meaning as described in the present invention.

In other embodiments, $R^3$ is hydrogen, deuterium, hydroxy-substituted $C_{1-4}$ alkyl, $-C_{1-4}$ alkylene-O-C(=O)-$R^f$, $-C_{1-4}$ alkylene-C(=O)-O-$R^f$ or $-C_{1-4}$ alkylene-$R^f$; wherein $R^f$ has the meaning as described in the present invention.

In still other embodiments, $R^3$ is hydrogen, deuterium, hydroxymethyl, 2-hydroxyethyl, 1-hydroxyethyl, 3-hydroxy-*n*-propyl, 2-hydroxy-1-methylethyl, $-CH_2OC(=O)R^f$, $-CH_2CH_2OC(=O)R^f$, $-CH_2CH_2CH_2OC(=O)R^f$, $-CH(CH_3)_2OC(=O)R^f$, $-CH_2C(=O)OR^f$, $-CH_2CH_2C(=O)OR^f$, $-CH_2CH_2CH_2C(=O)OR^f$, $-CH(CH_3)_2C(=O)OR^f$, $-CH_2R^f$, $-CH_2CH_2R^f$, $-CH_2CH_2CH_2R^f$ or $-CH(CH_3)_2R^f$; wherein $R^f$ has the meaning as described in the present invention.

In some embodiments, $R^f$ is $C_{2-6}$ alkyl, amino-substituted $C_{1-6}$ alkyl, $C_{1-6}$ haloalkyl, $-C_{1-6}$ alkylene-C(=O)-$OR^h$ or -O-P(=O)-($OR^m$)($OR^n$); wherein each $R^h$, $R^m$ and $R^n$ is independently hydrogen, deuterium, sodium, potassium, $C_{1-6}$ alkyl or $C_{1-6}$ haloalkyl.

In other embodiments, $R^f$ is $C_{2-4}$ alkyl, amino-substituted $C_{1-4}$ alkyl, $C_{1-4}$ haloalkyl, $-C_{1-4}$ alkylene-C(=O)-$OR^h$ or -O-P(=O)-($OR^m$)($OR^n$); wherein each $R^h$, $R^m$ and $R^n$ is independently hydrogen, deuterium, sodium, potassium, $C_{1-4}$ alkyl or $C_{1-4}$ haloalkyl.

In still other embodiments, $R^f$ is ethyl, *n*-propyl, isopropyl, $-CH_2NH_2$, $-CH_2CH_2NH_2$, $-CH_2CH_2CH_2NH_2$, $-CH(NH_2)CH(CH_3)_2$, $-CH_2F$, $-CHF_2$, $-CF_3$, $-CHFCH_2F$, $-CF_2CH_3$, $-CH_2CHF_2$, $-CH_2CF_3$, $-CH_2C(=O)ONa$, $-CH_2CH_2C(=O)-ONa$, $-CH_2CH_2CH_2C(=O)ONa$, $-CH(CH_3)_2C(=O)ONa$ or $-O-P(=O)(ONa)_2$.

In some embodiments, $R^4$ is hydrogen, deuterium or hydroxy-substituted $C_{1-6}$ alkyl.

In other embodiments, $R^4$ is hydrogen, deuterium or hydroxy-substituted $C_{1-4}$ alkyl.

In still other embodiments, $R^4$ is hydrogen, deuterium, hydroxymethyl, 2-hydroxyethyl, 1-hydroxyethyl, 3-hydroxy-*n*-propyl or 2-hydroxy-1-methylethyl.

In some embodiments, $R^5$ is hydrogen, deuterium, $C_{1-4}$ alkyl, $C_{1-4}$ haloalkyl, $C_{1-4}$ alkoxy, $C_{3-6}$ cycloalkyl or 3- to 6-membered heterocyclyl;

each of $R^6$ and $R^7$ is independently hydrogen, deuterium, $C_{1-4}$ alkyl, $C_{1-4}$ haloalkyl, $C_{1-4}$ alkoxy, $C_{3-6}$ cycloalkyl or 3- to 6-membered heterocyclyl.

In other embodiments, $R^5$ is independently hydrogen, deuterium, methyl, ethyl, isopropyl, *n*-propyl, *n*-butyl, isobutyl, sec-butyl, tert-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl;

each of $R^6$ and $R^7$ is independently hydrogen, deuterium, methyl, ethyl, isopropyl, *n*-propyl, *n*-butyl, isobutyl, sec-butyl, *tert*-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl.

In some embodiments, the present invention provides a compound having Formula (II) or a stereoisomer, an *N*-oxide, a solvate, a metabolite, a pharmaceutically acceptable salt or a prodrug thereof,

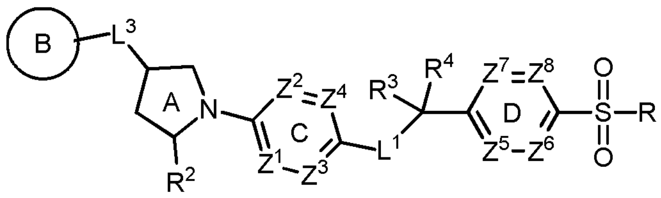

(II)

wherein, ring A, ring B, $L^1$, $L^3$, R, $Z^1$, $Z^2$, $Z^3$, $Z^4$, $Z^5$, $Z^6$, $Z^7$, $Z^8$, $R^2$, $R^3$ and $R^4$ have the meanings described in the present invention.

In some embodiments, the present invention relates to, but is by no means limited to, one of the following compounds, or a stereoisomer, an *N*-oxide, a solvate, a metabolite, a pharmaceutically acceptable salt or a prodrug thereof:

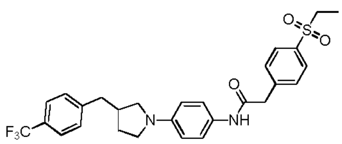

(1),

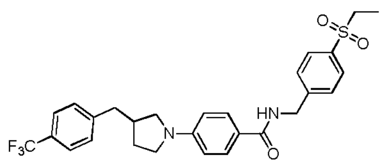

(2),

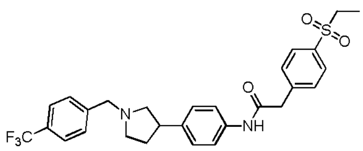

(3),

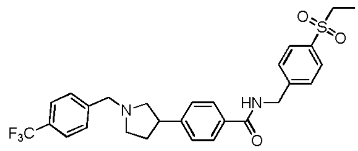

(4),

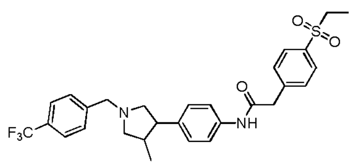

(5),

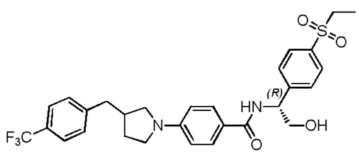

(6),

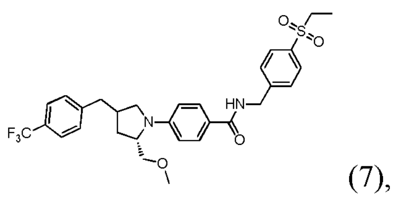

(7),

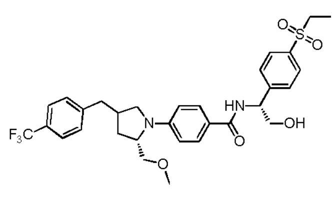

(8),

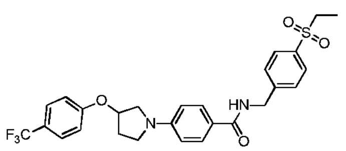

(9),

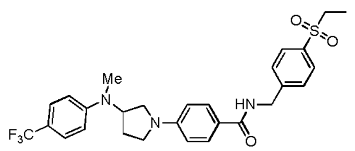

(10),

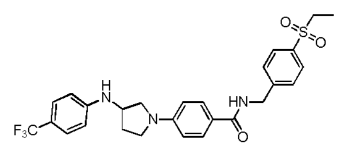

(11),

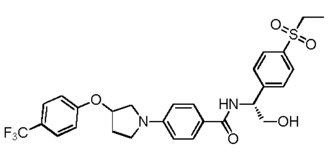 (12), 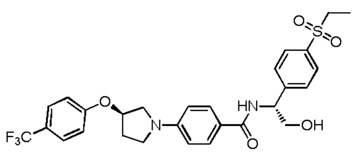 (13), 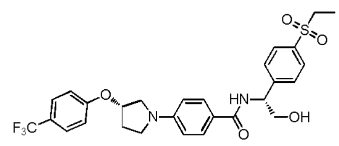 (14),

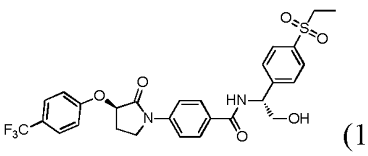 (15), 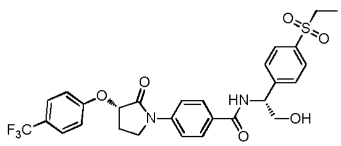 (16), 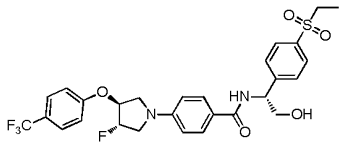 (17),

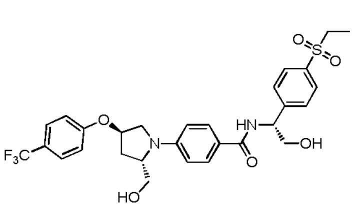 (18), 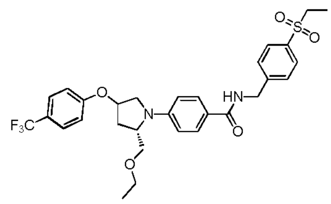 (19), 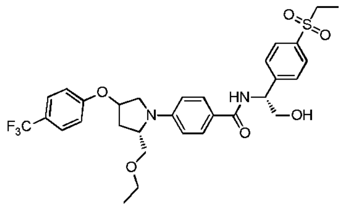 (20),

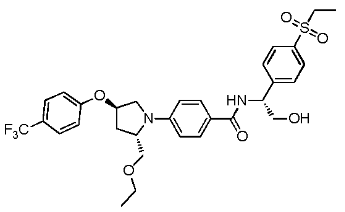 (21), 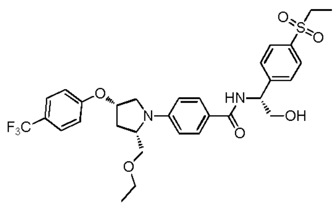 (22), 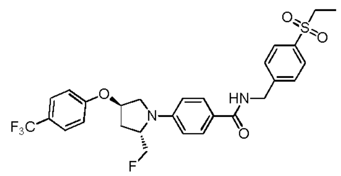 (23),

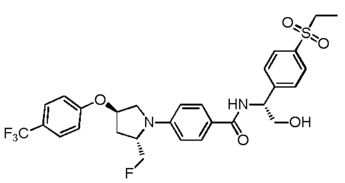 (24), 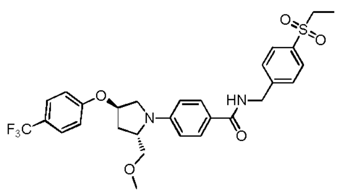 (25), 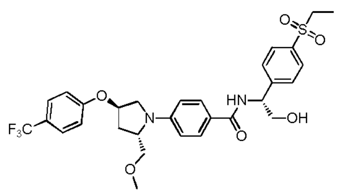 (26),

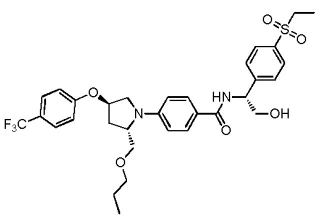 (27), 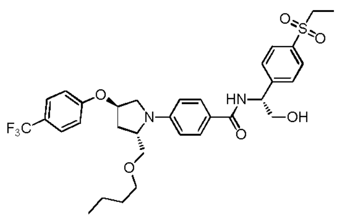 (28), 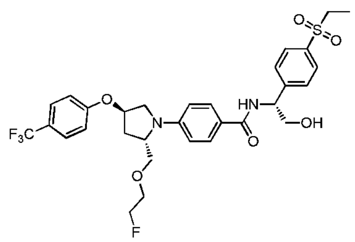 (29),

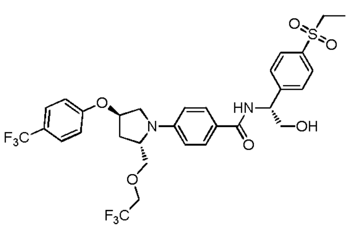 (30), 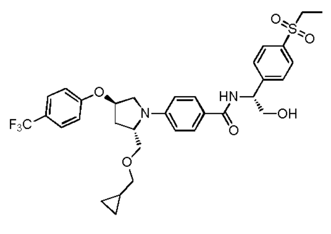 (31), 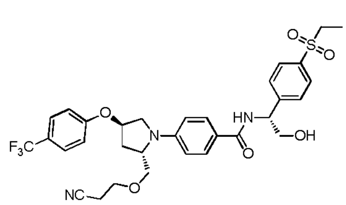 (32),

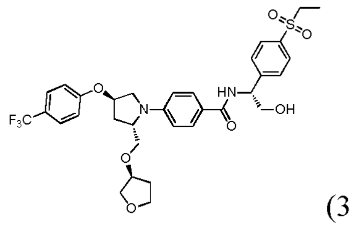 (33), 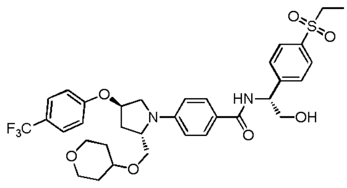 (34), 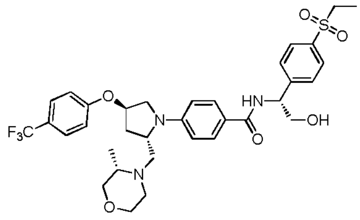 (35),

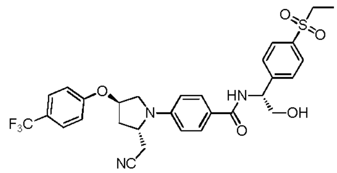 (36), 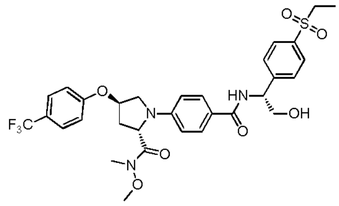 (37), 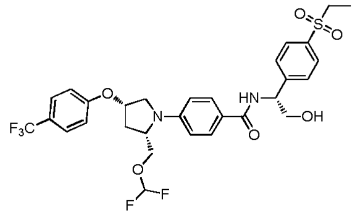 (38),

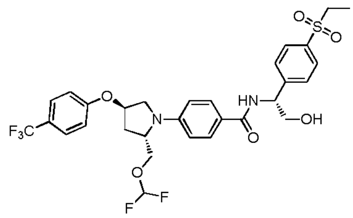 (39), 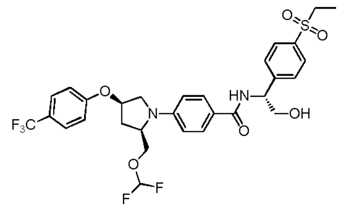 (40), 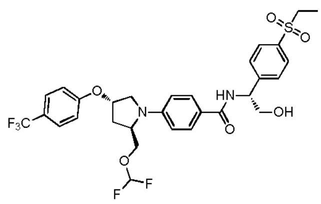 (41),

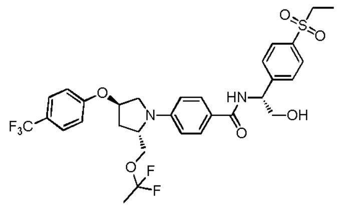 (42), 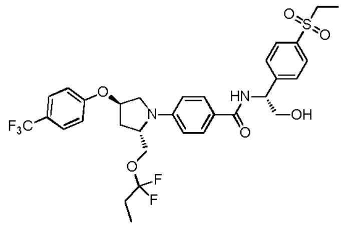 (43), 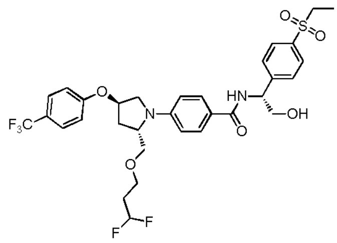 (44),

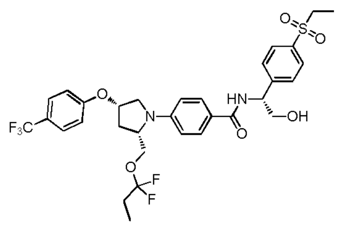 (45), 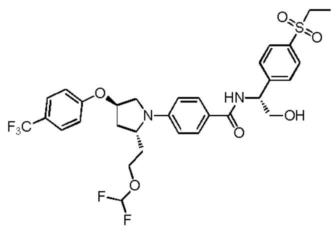 (46), 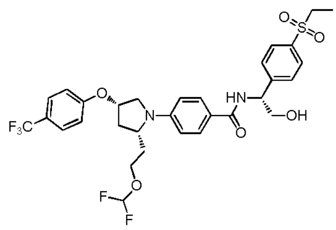 (47),

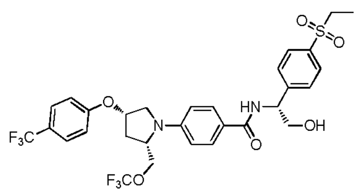 (48), 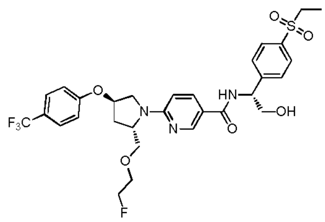 (49), 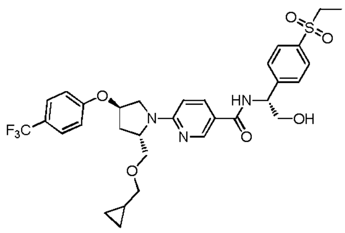 (50),

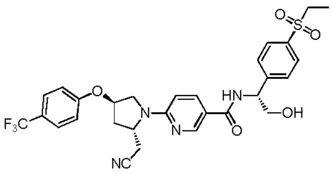 (51), 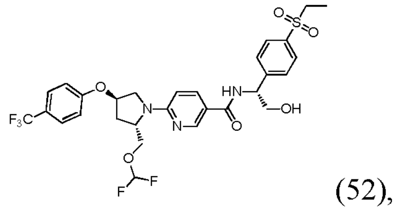 (52), 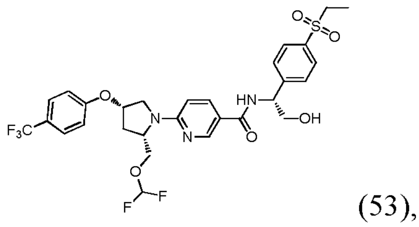 (53),

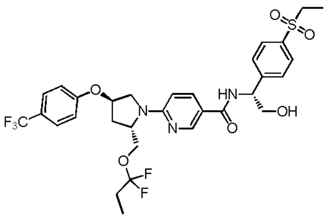 (54), 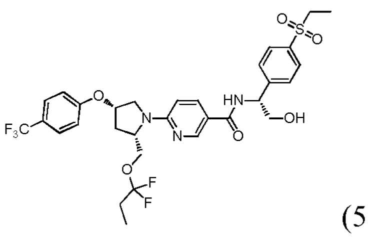 (55), 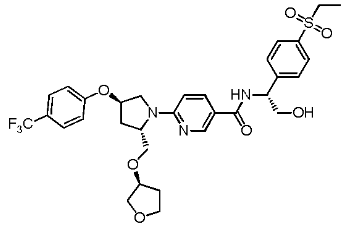 (56),

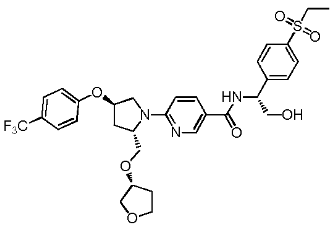 (57), 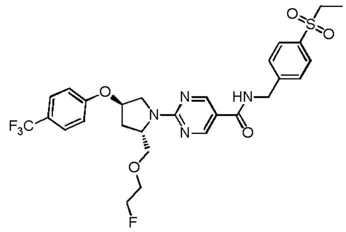 (58), 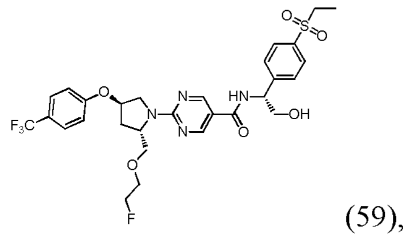 (59),

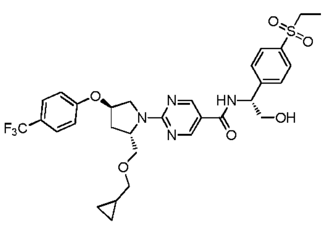 (60), 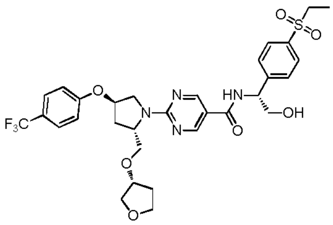 (61), 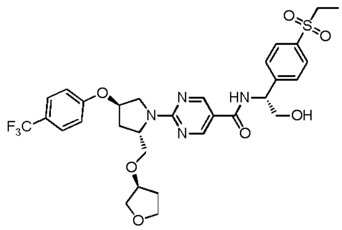 (62),

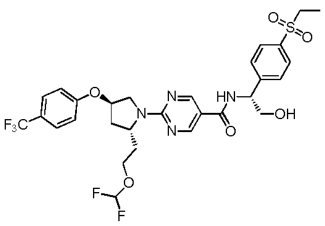 (63), 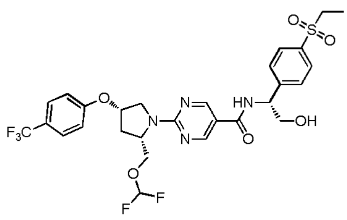 (64), 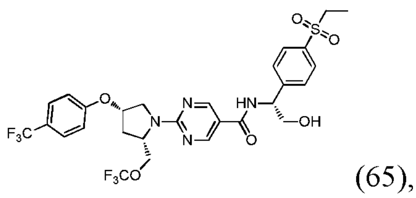 (65),

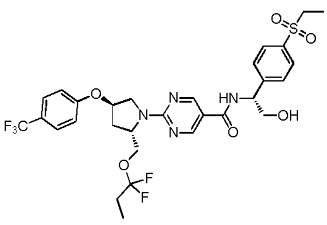 (66), 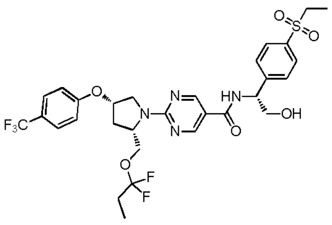 (67), 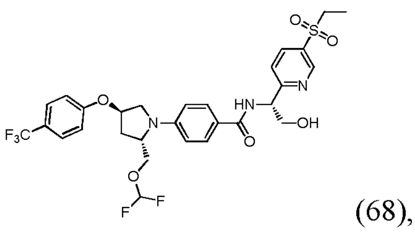 (68),

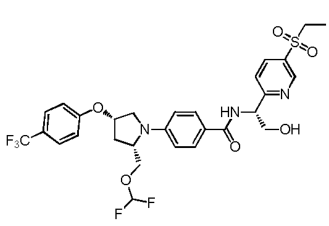 (69), 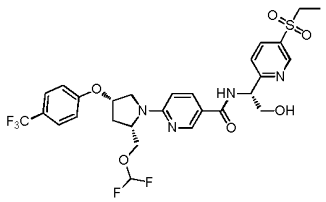 (70), 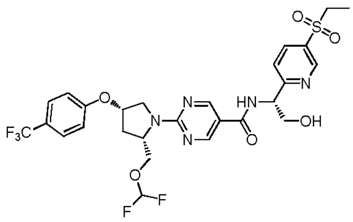 (71),

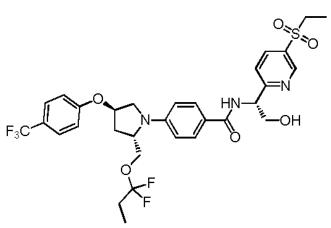 (72), 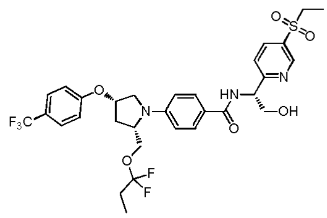 (73), 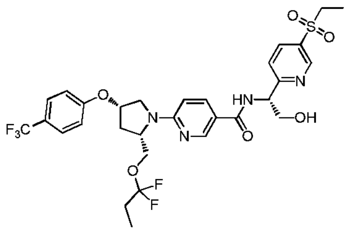 (74),

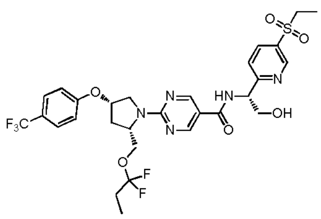 (75), 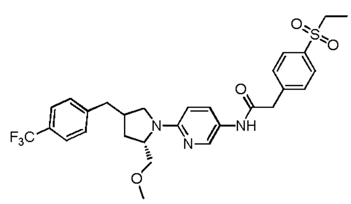 (76), 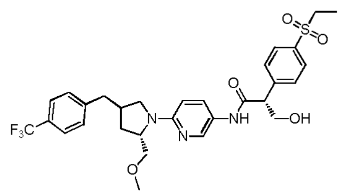 (77),

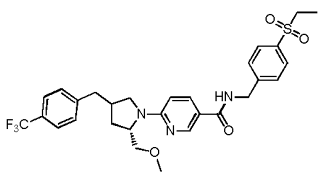 (78), 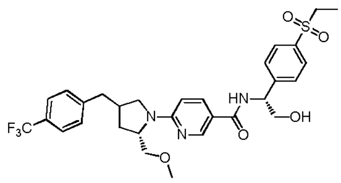 (79), 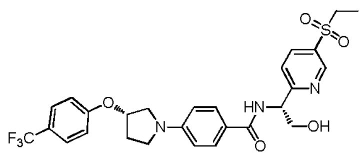 (80),

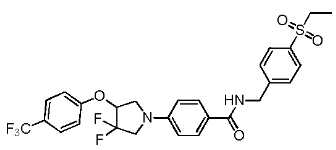 (81), 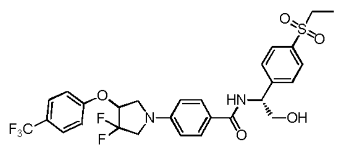 (82), 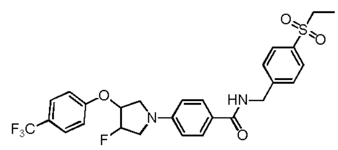 (83),

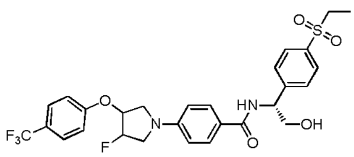 (84), 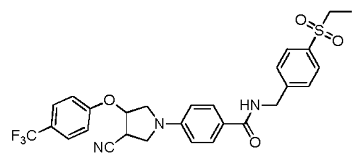 (85),

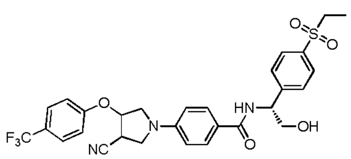 (86), 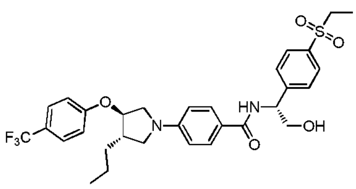 (87),

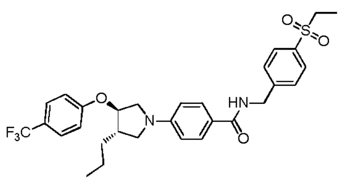 (88),

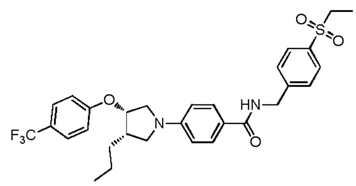 (89),

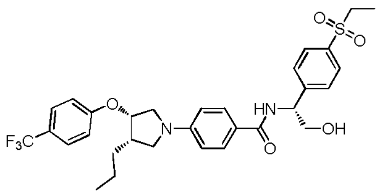 (90),

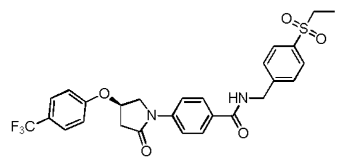 (91),

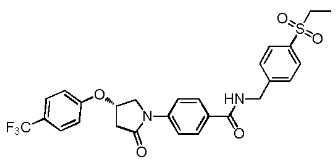 (92),

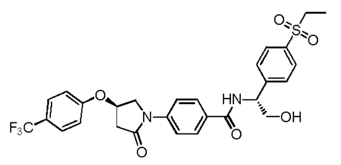 (93),

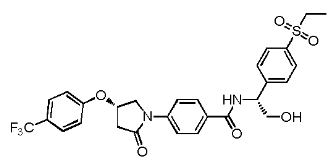 (94),

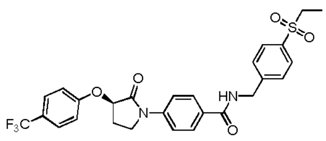 (95),

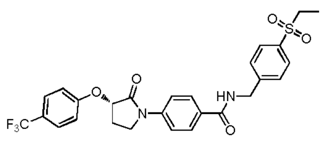 (96),

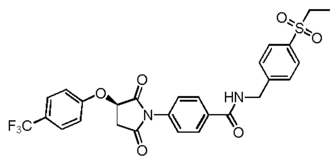 (97),

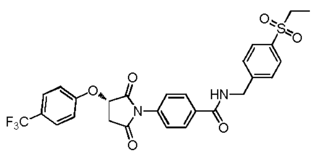 (98),

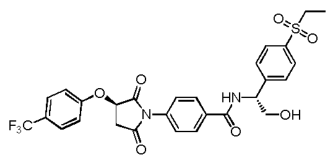 (99),

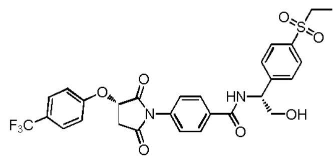 (100),

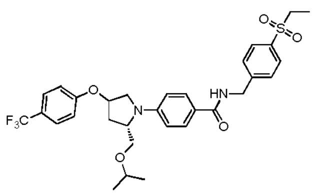 (101),

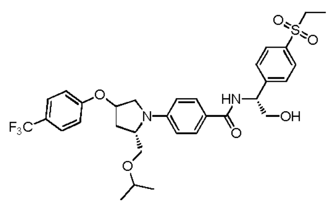 (102),

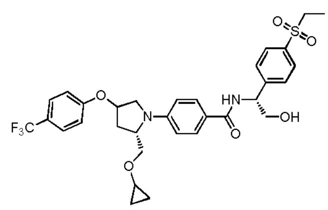 (103),

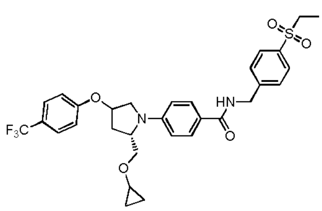 (104),

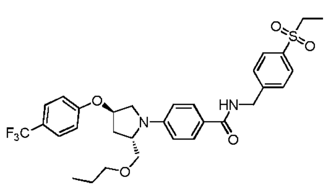 (105),

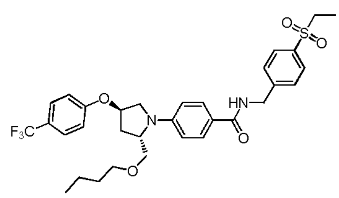 (106),

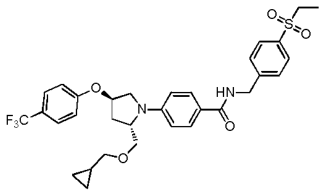 (107), 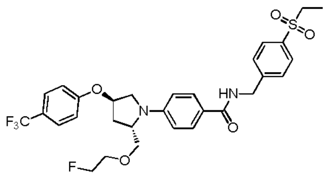 (108), 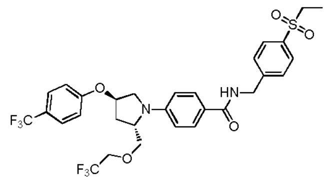 (109),

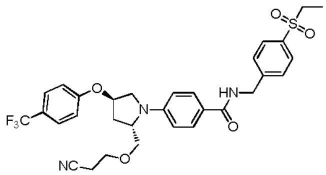 (110), 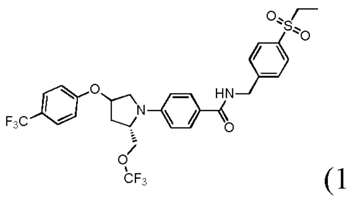 (111), 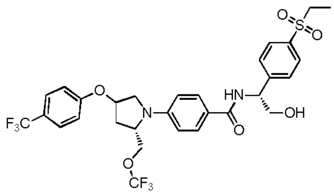 (112),

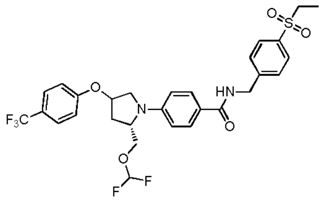 (113), 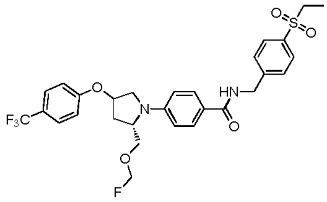 (114), 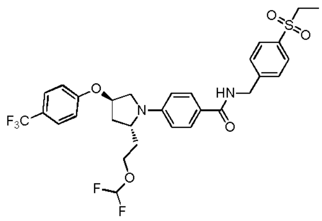 (115),

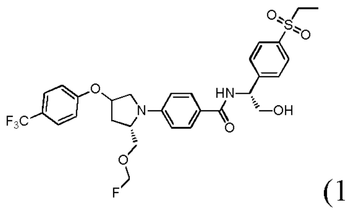 (116), 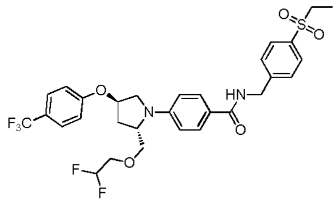 (117), 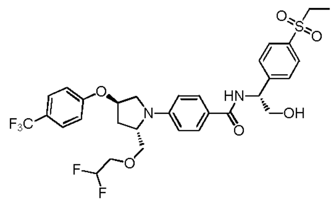 (118),

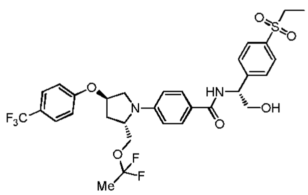 (119), 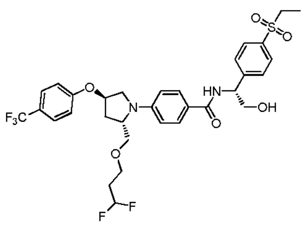 (120), 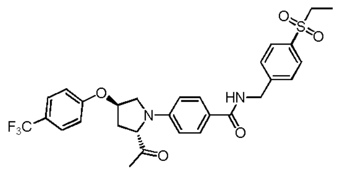 (121),

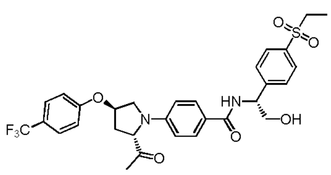 (122), 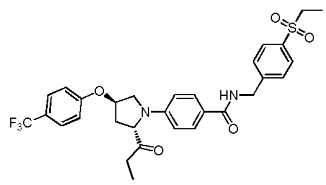 (123), 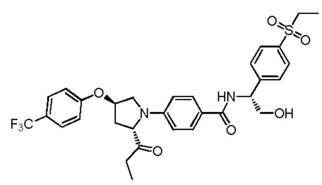 (124),

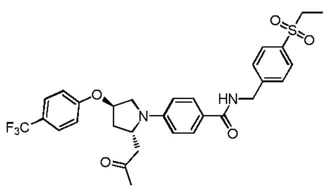 (125), 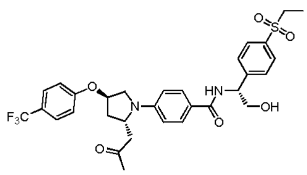 (126), 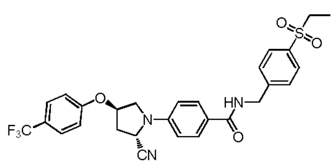 (127),

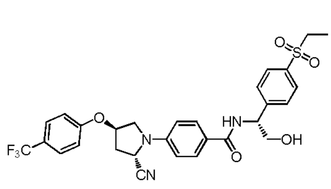 (128), 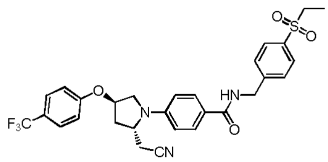 (129), 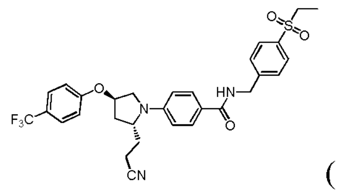 (130),

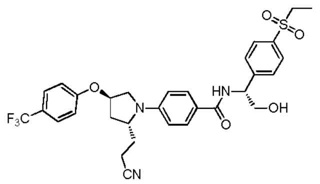 (131), 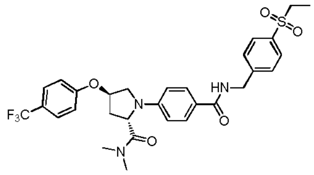 (132), 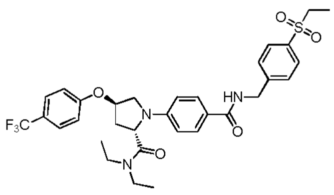 (133),

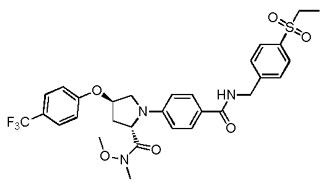 (134), 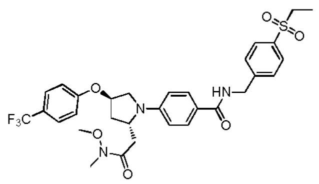 (135), 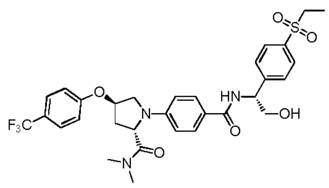 (136),

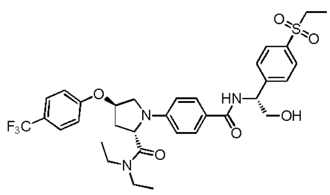 (137), 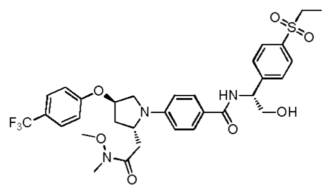 (138), 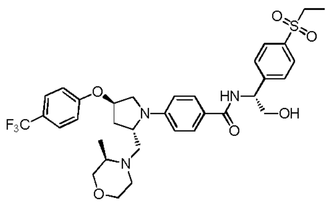 (139),

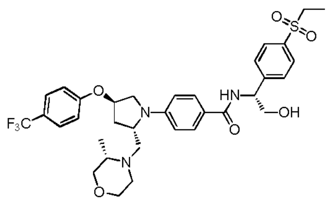 (140), 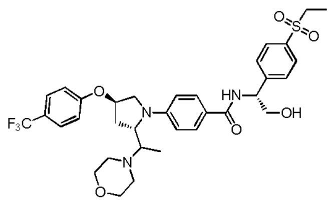 (141), 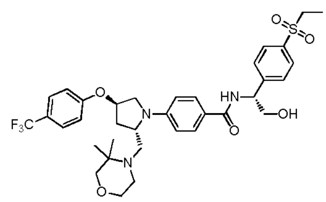 (142),

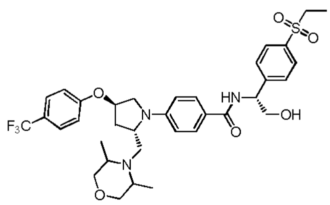 (143), 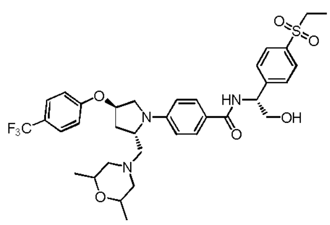 (144), 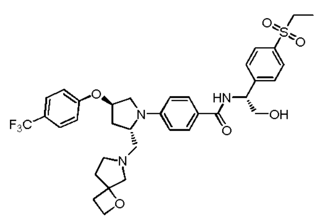 (145),

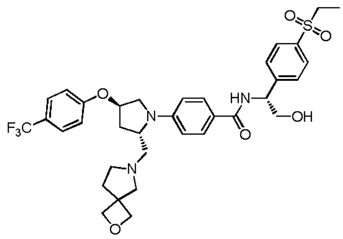 (146), 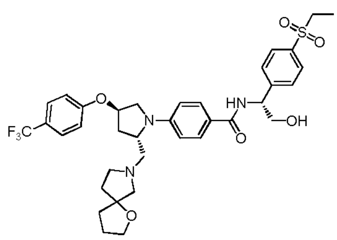 (147), 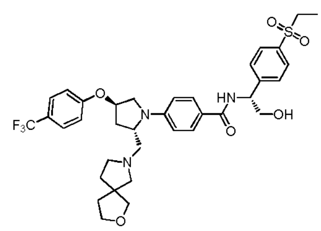 (148),

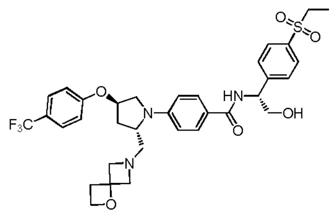 (149), 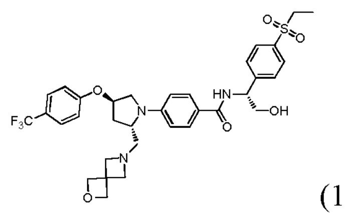 (150), 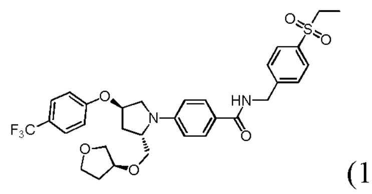 (151),

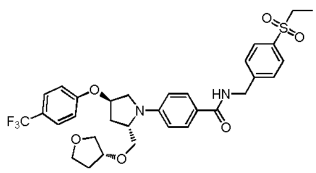 (152), 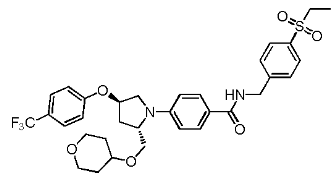 (153), 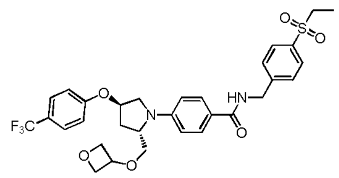 (154),

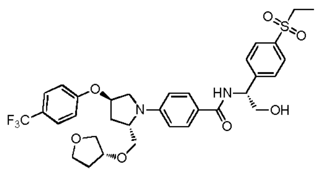 (155), 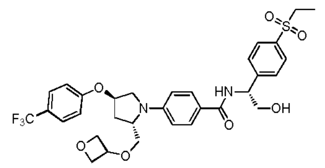 (156), 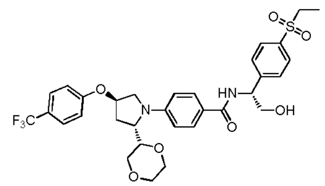 (157),

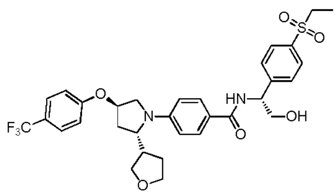 (158), 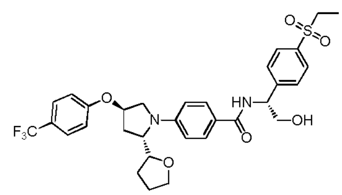 (159), 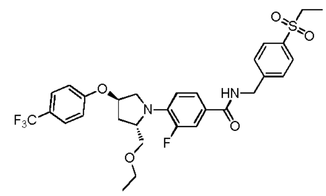 (160),

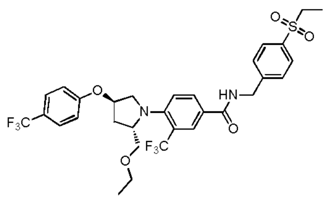 (161), 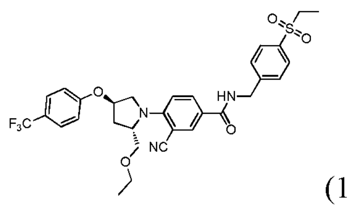 (162), 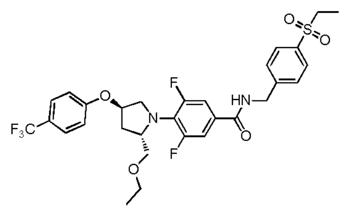 (163),

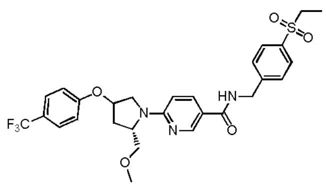 (164), 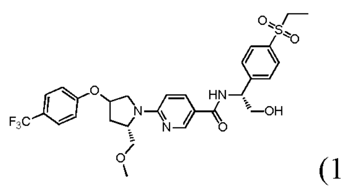 (165), 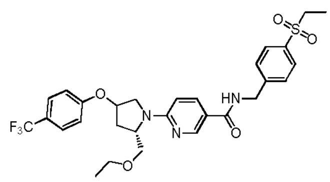 (166),

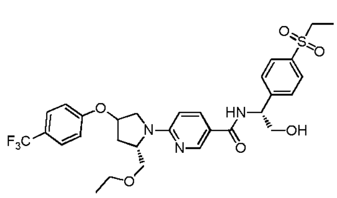 (167), 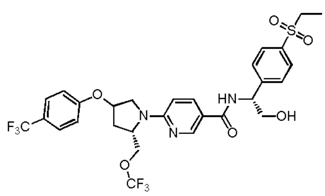 (168), 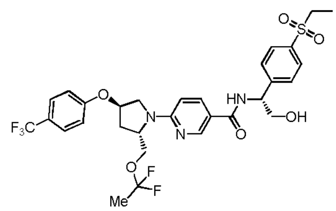 (169),

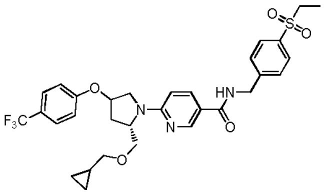 (170), 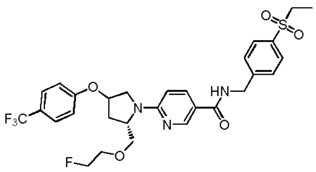 (171), 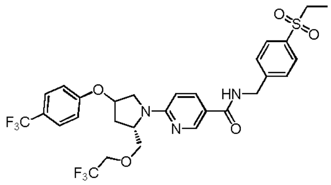 (172),

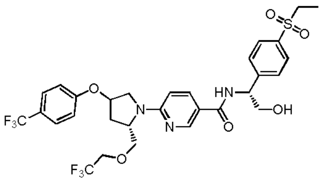 (173), 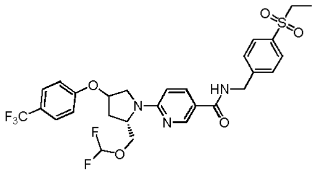 (174), 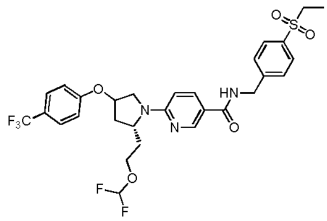 (175),

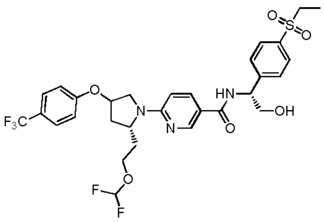 (176), 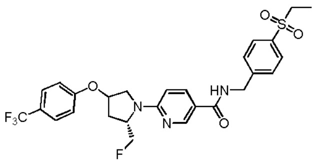 (177), 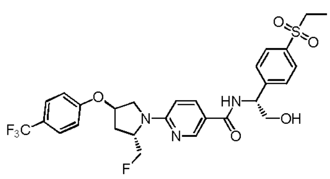 (178),

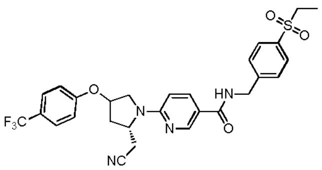 (179), 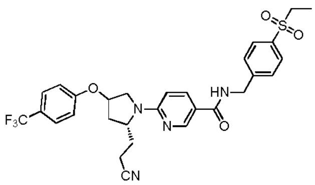 (180), 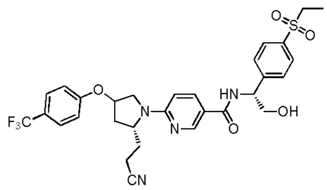 (181),

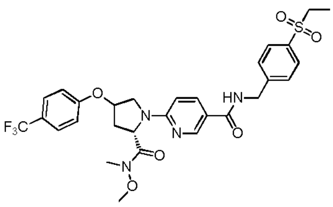 (182), 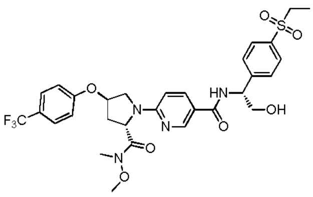 (183), 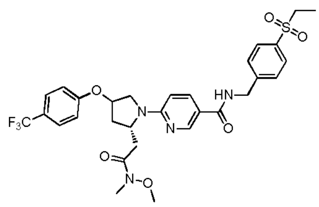 (184),

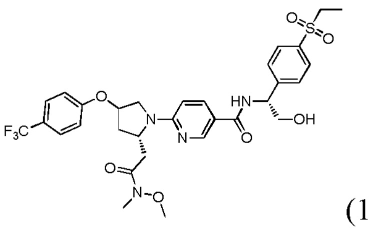 (185), 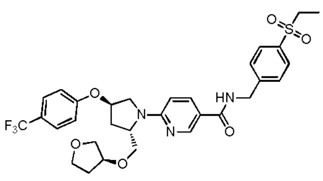 (186), 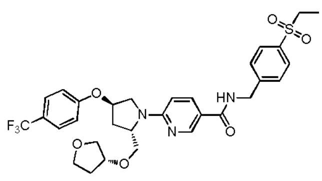 (187),

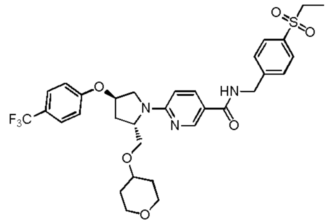 (188), 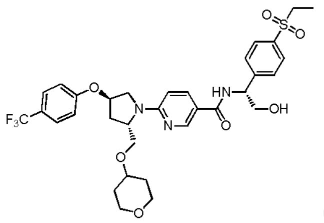 (189), 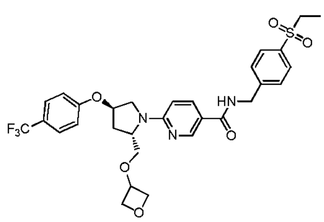 (190),

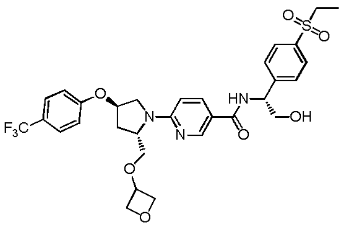 (191), 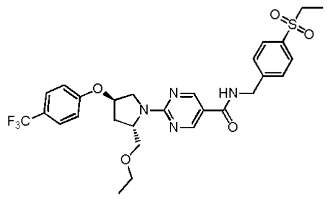 (192), 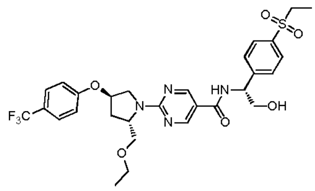 (193),

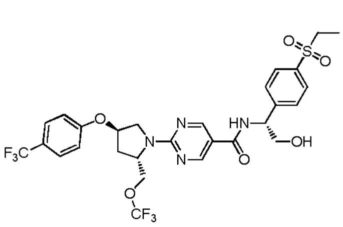 (194), 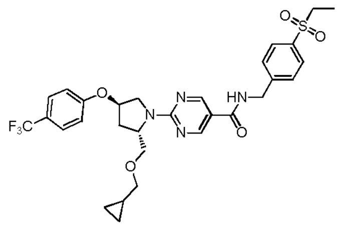 (195),

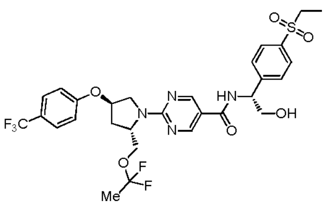 (196), 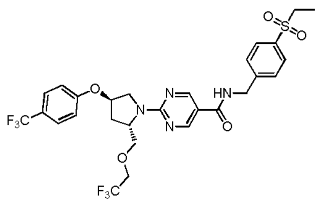 (197), 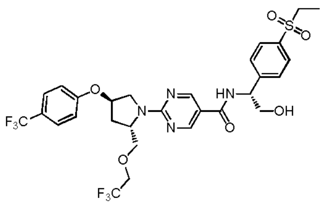 (198),

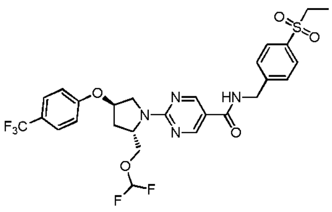 (199), 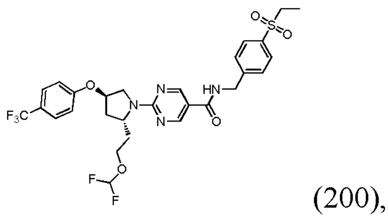 (200), 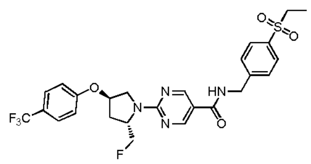 (201),

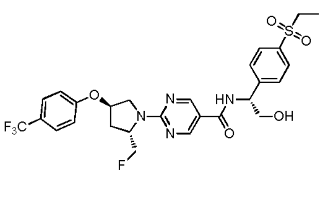 (202), 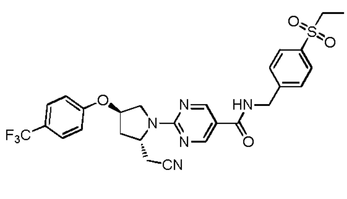 (203), 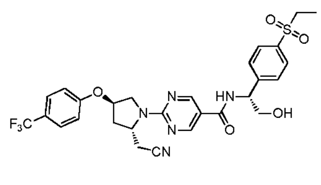 (204),

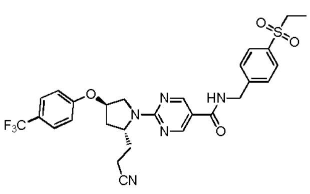 (205), 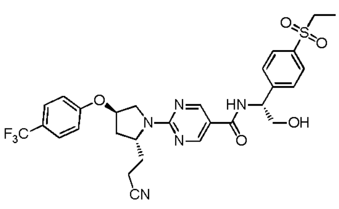 (206), 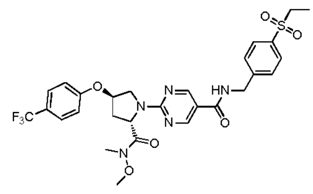 (207),

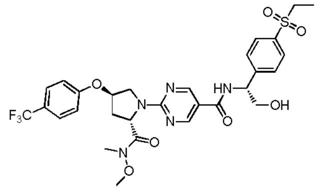 (208),

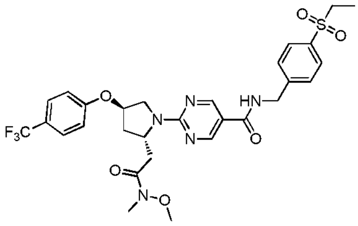 (209),

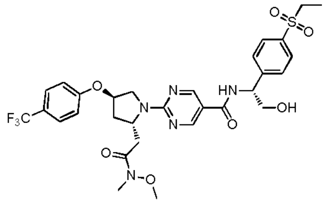 (210),

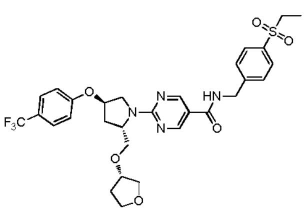 (211),

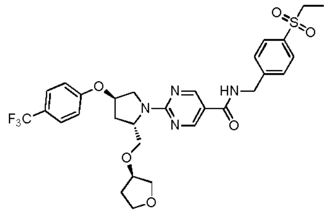 (212),

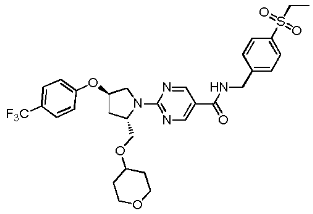 (213),

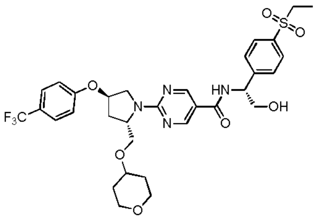 (214),

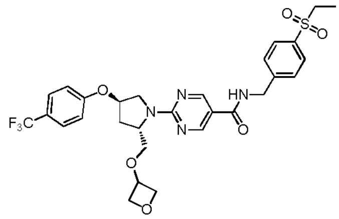 (215),

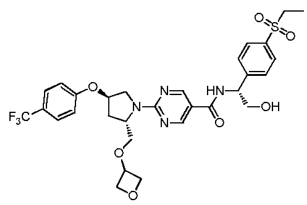 (216),

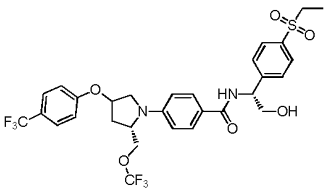 (217),

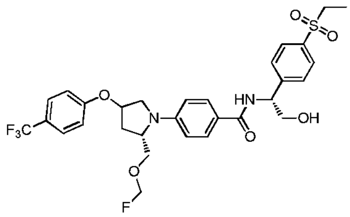 (218),

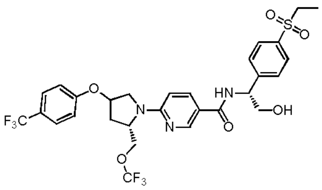 (219),

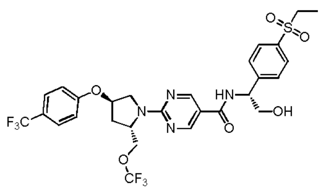 (220),

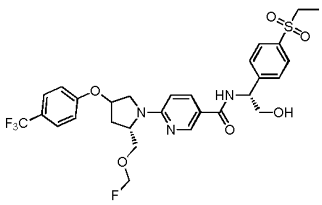 (221),

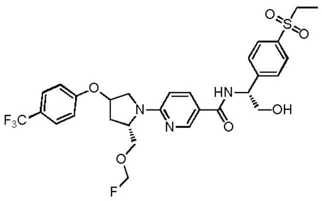 (222),

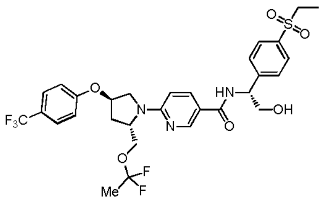 (223),

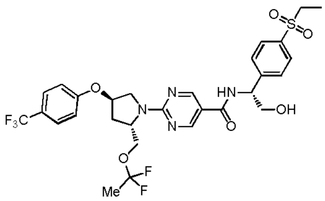 (224),

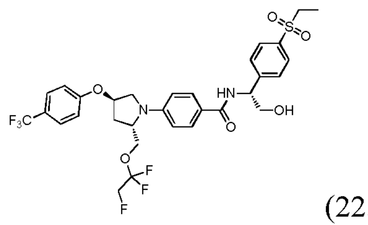
(225),
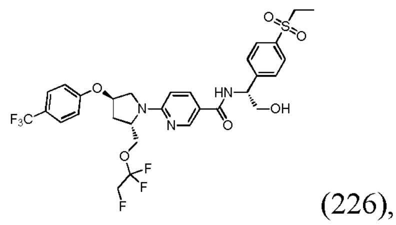
(226),
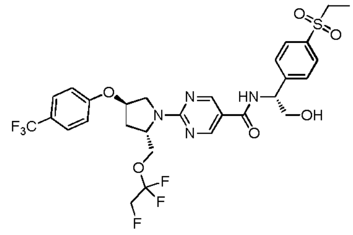
(227),

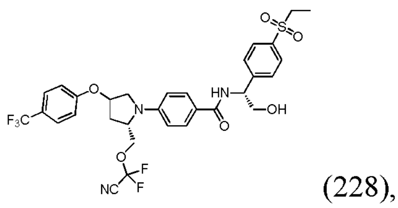
(228),
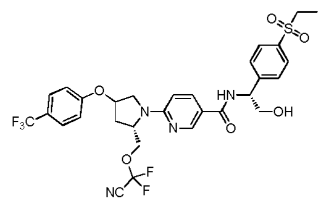
(229),
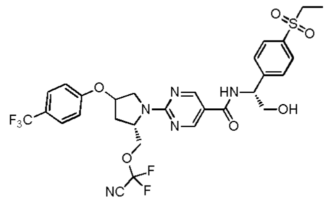
(230),

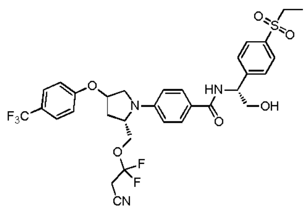
(231),
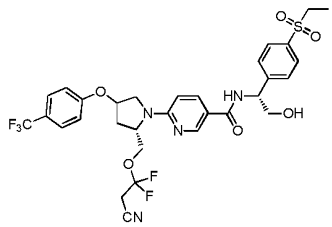
(232),
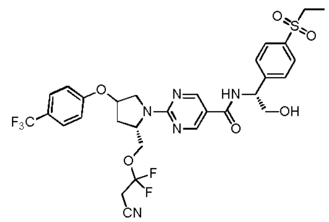
(233),

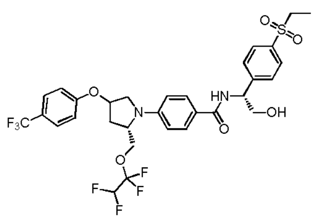
(234),
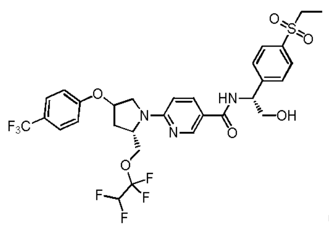
(235),
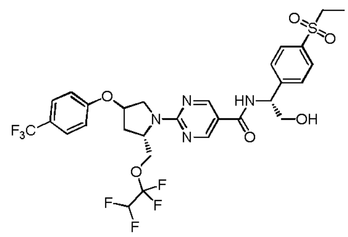
(236),

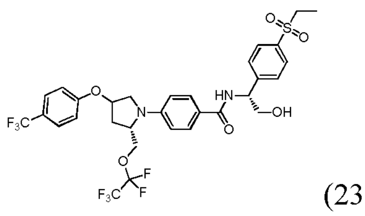
(237),
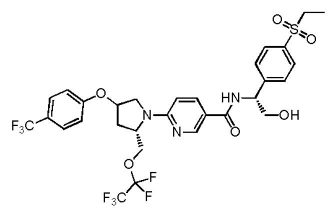
(238),
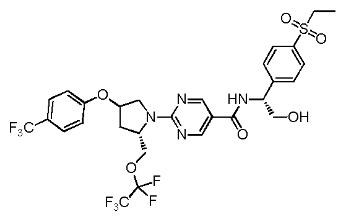
(239),

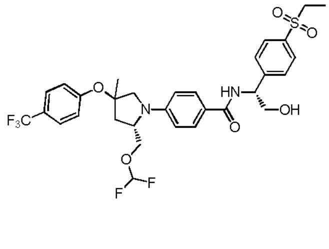
(240),
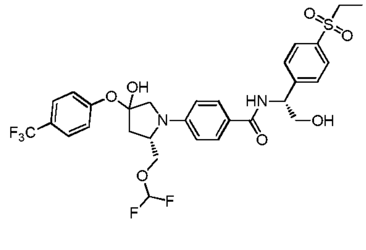
(241),

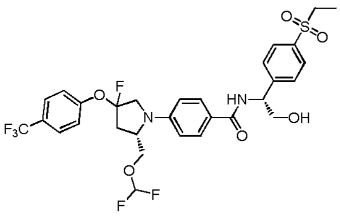 (242), 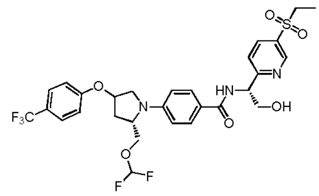 (243), 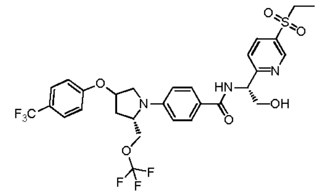 (244),

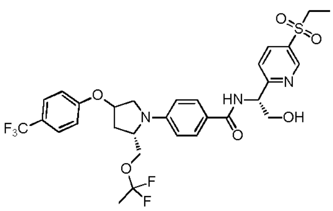 (245), 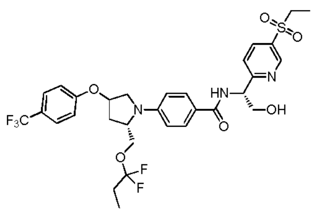 (246), 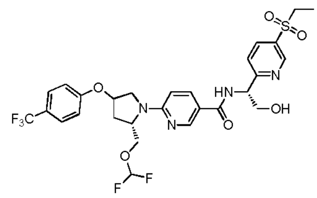 (247),

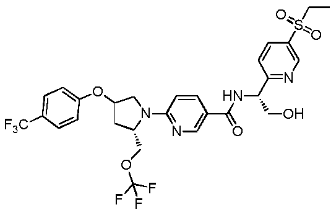 (248), 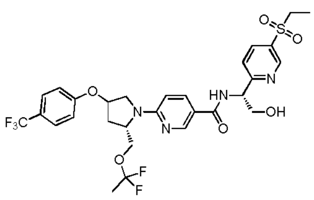 (249), 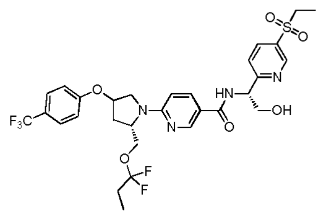 (250),

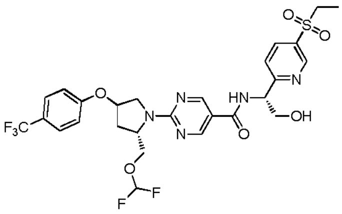 (251), 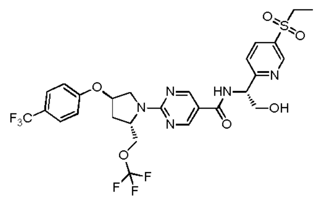 (252), 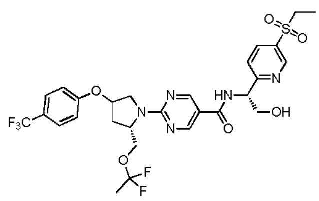 (253),

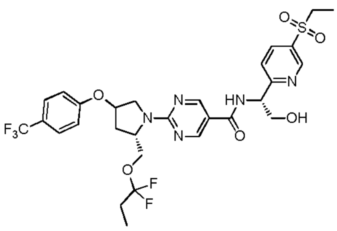 (254), 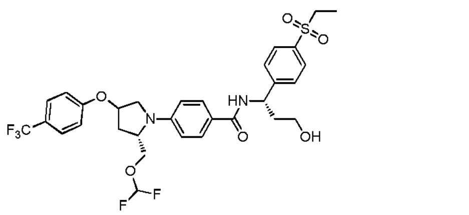 (255),

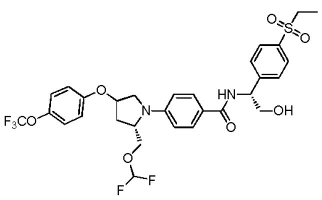 (256), 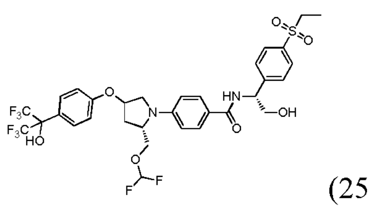 (257), 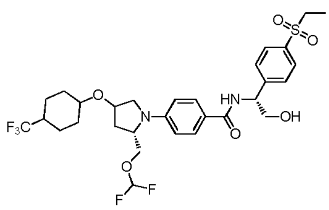 (258),

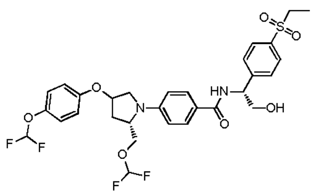 (259), 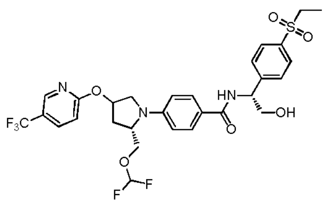 (260), 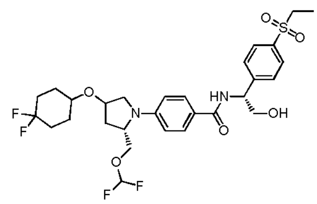 (261),

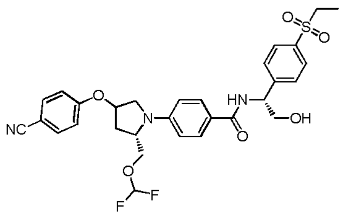 (262), 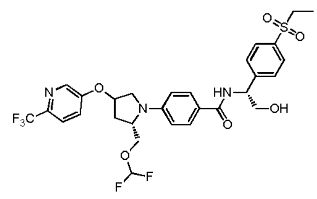 (263), 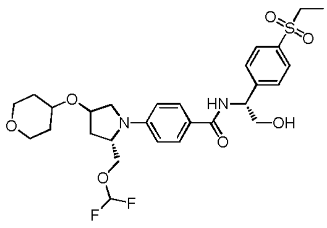 (264),

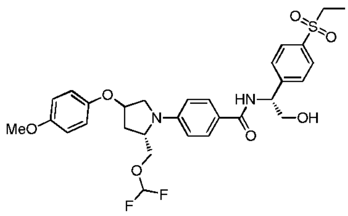 (265), 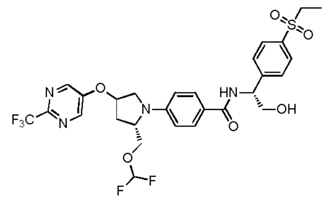 (266), 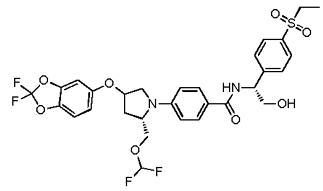 (267),

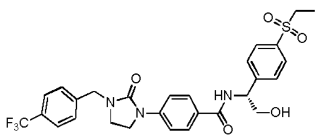 (268), 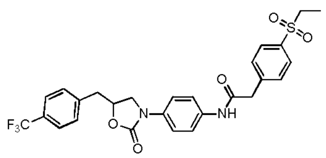 (269), 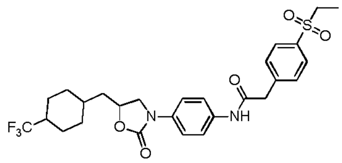 (270),

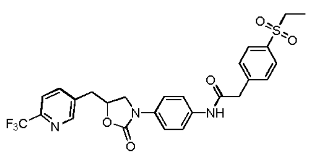 (271), 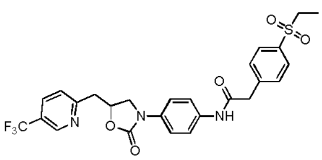 (272), 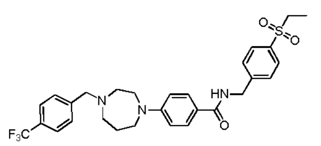 (273),

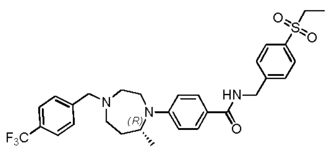 (274), 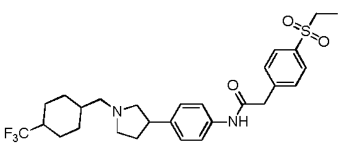 (275), 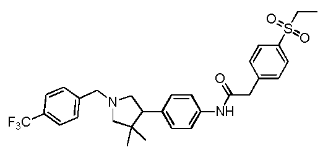 (276),

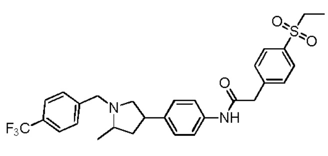 (277), 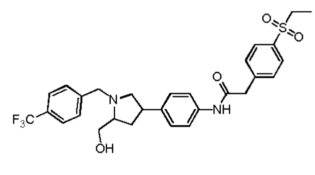 (278), 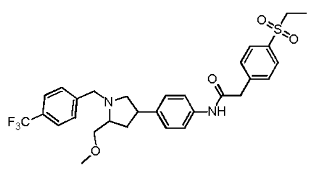 (279),

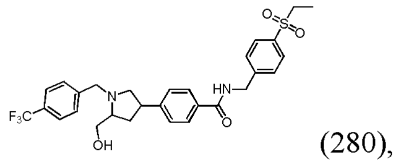 (280),

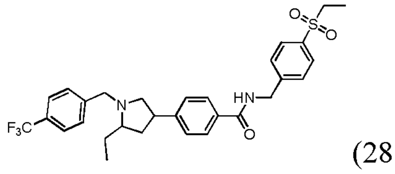 (281),

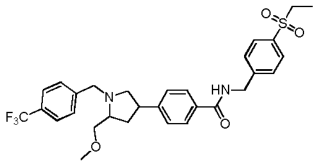 (282),

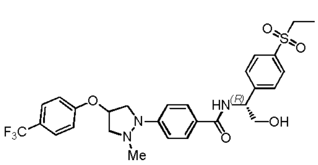 (283),

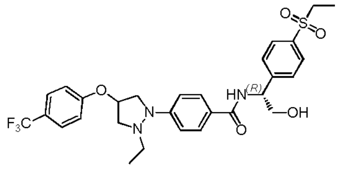 (284),

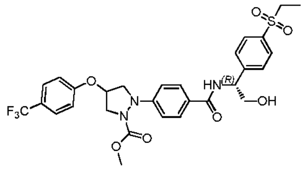 (285),

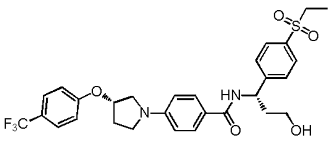 (286),

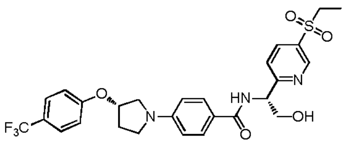 (287),

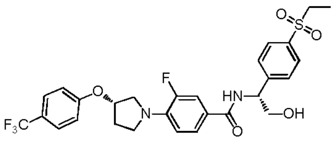 (288),

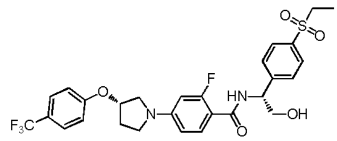 (289),

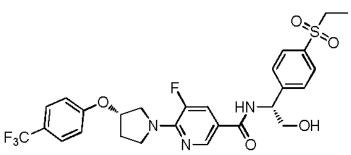 (290),

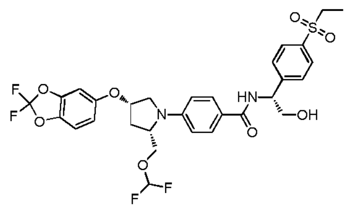 (291),

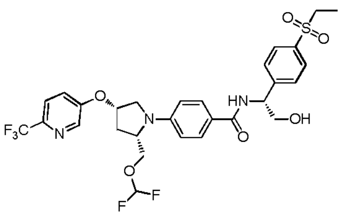 (292),

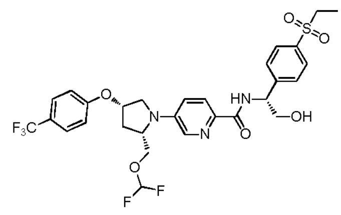 (293),

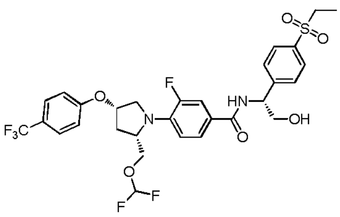 (294),

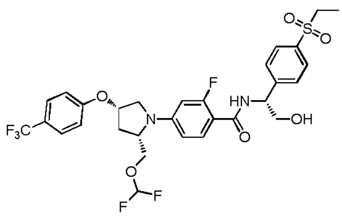 (295),

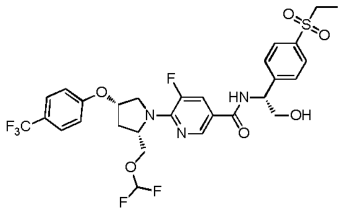

(296),

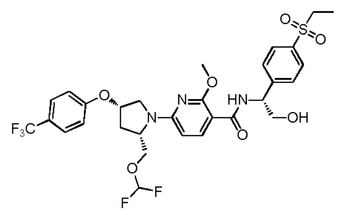

(297),

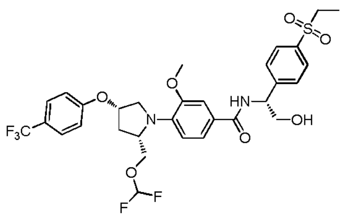

(298),

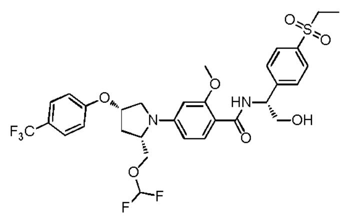

(299),

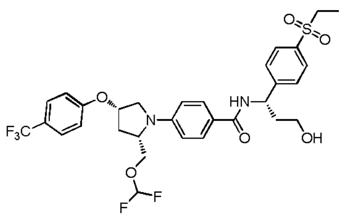

(300),

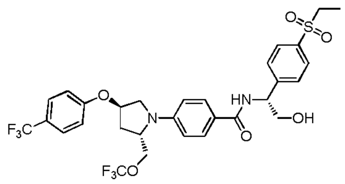

(301),

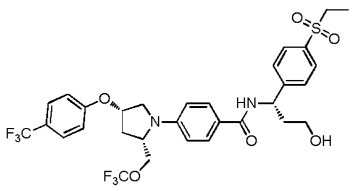

(302),

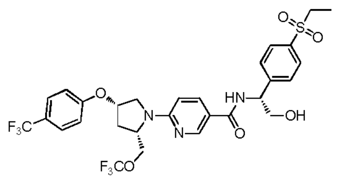

(303),

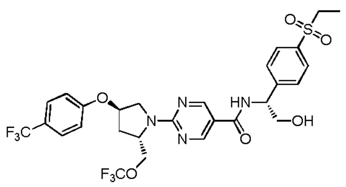

(304),

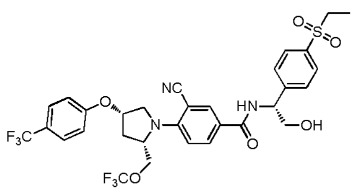

(305),

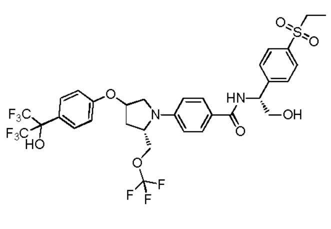

(306),

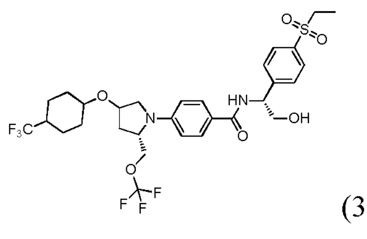

(307),

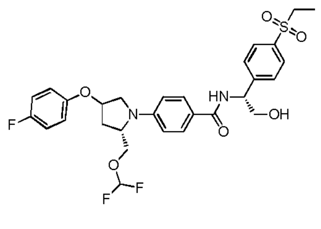

(308),

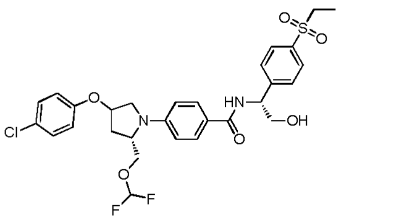

(309),

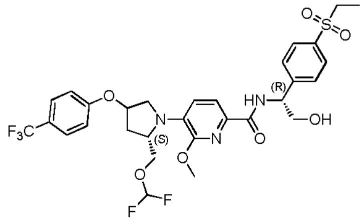

(310),

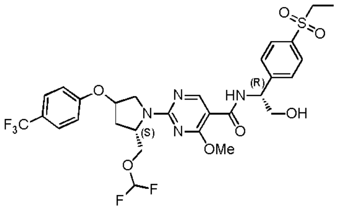

(311),

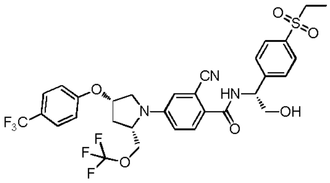

(312),

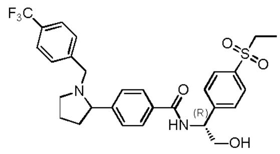

(313),

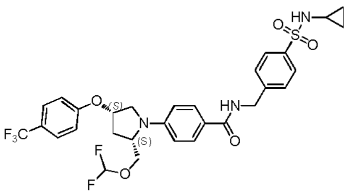

(314),

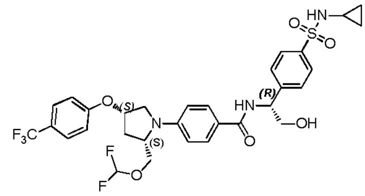

(315),

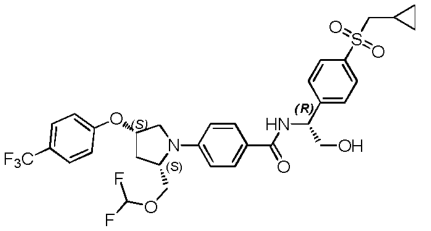

(316),

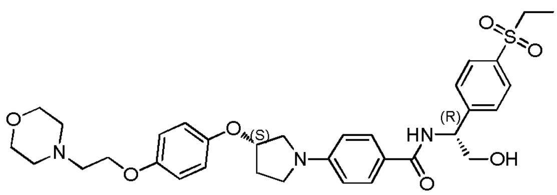

(317),

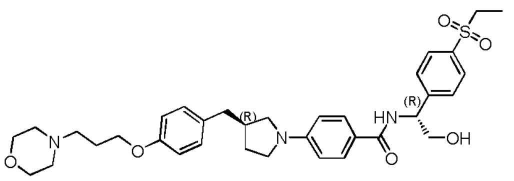

(318),

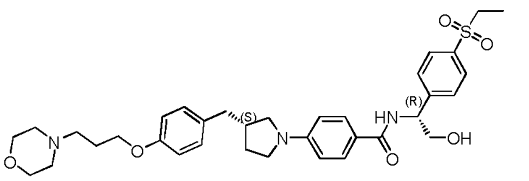

(319),

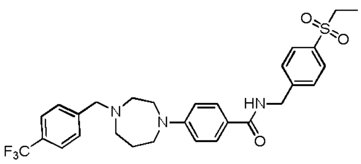

(320),

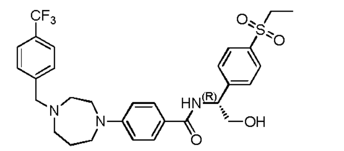

(321),

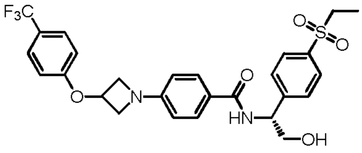

(322),

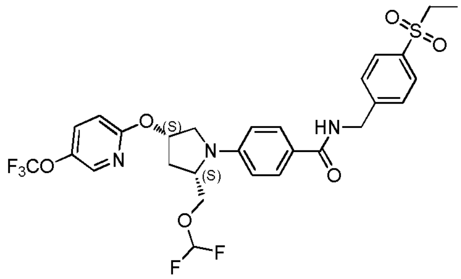

(323),

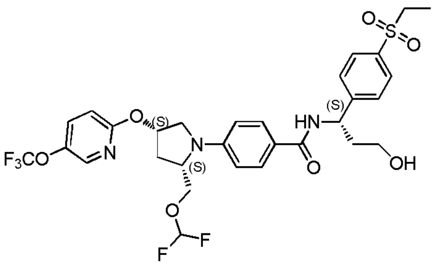

(324),

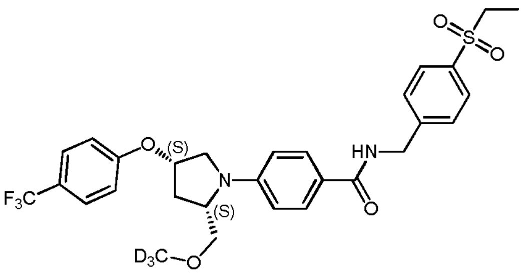

(325),

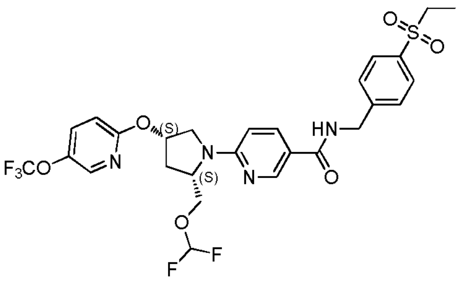

(326),

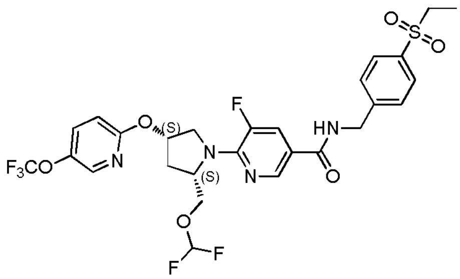

(327),

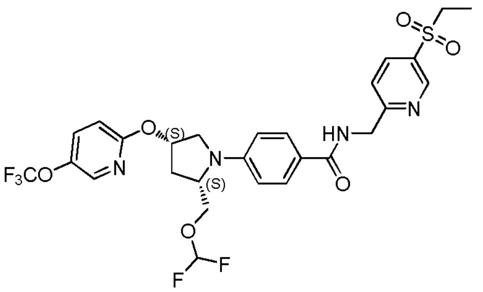

(328),

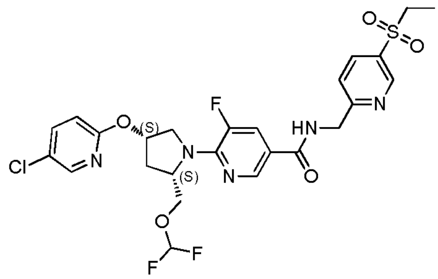

(329),

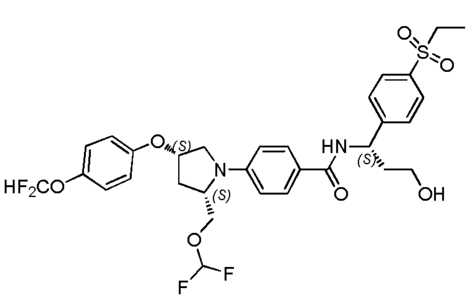

(330),

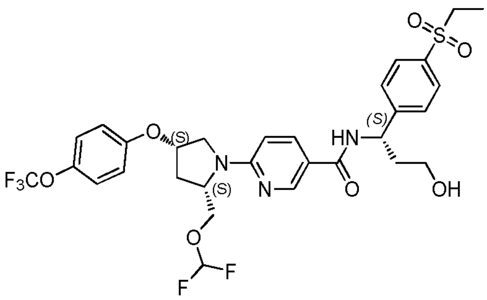

(331),

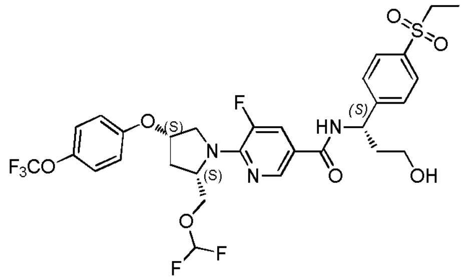

(332),

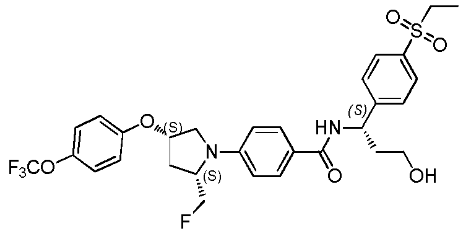

(333),

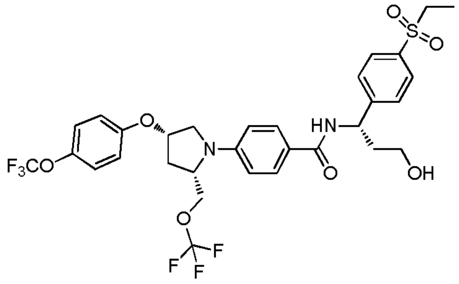

(334),

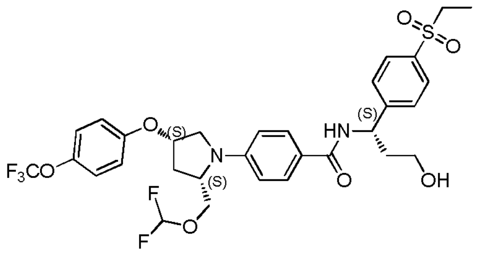

(335),

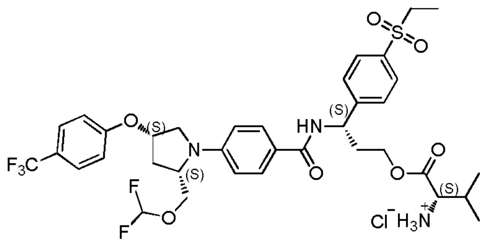

(336),

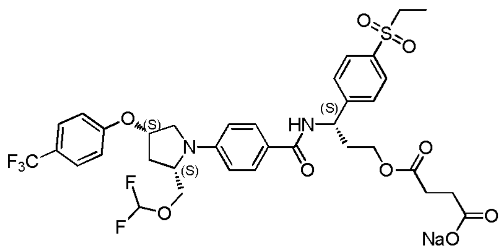

(337),

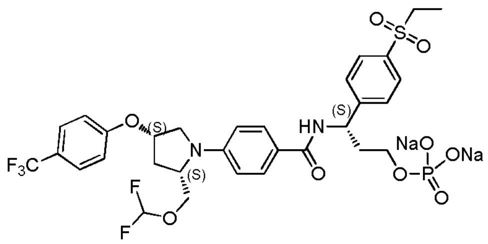

(338),

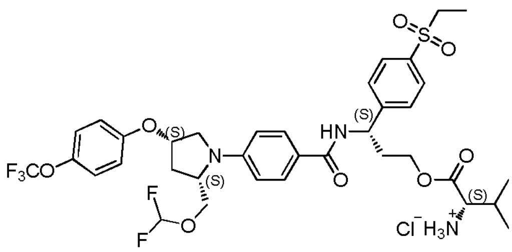

(339),

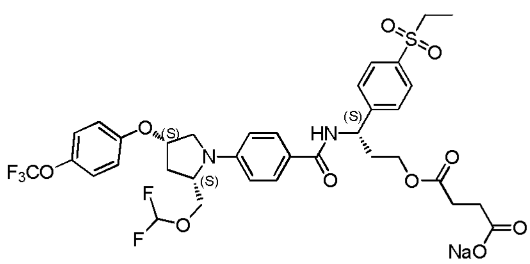

(340),

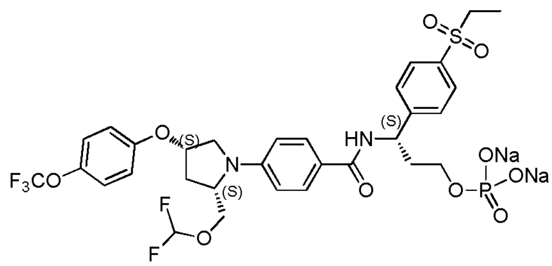

(341)

or

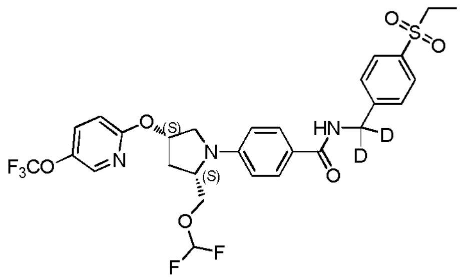

(342).

Unless otherwise specified, a compound having formula (I) or formula (II), or a stereoisomer, a solvate, a metabolite, a pharmaceutically acceptable salt or a prodrug thereof are all embraced within the scope of the invention.

In some embodiments, pharmaceutically acceptable salts of the compounds described herein include, but are not limited to, hydrochloride, sodium salt, potassium salt, and the like.

It is found through research that the compounds of the present invention have excellent affinity activity for RORγ and stability of liver microsomes, and at the same time have good pharmacokinetic properties *in vivo.* Specifically, some of the compounds of the present invention can be metabolized into corresponding alcohol compounds *in vivo,* and the corresponding alcohols have excellent pharmacokinetic properties *in vivo.*

In another aspect, the present invention relates to a pharmaceutical composition comprising a compound having formula (I) or formula (II), or a stereoisomer, a geometric isomer, a tautomer, an N-oxide, a hydrate, a solvate, a metabolite, a pharmaceutically acceptable salt or a prodrug thereof, and a pharmaceutically acceptable excipient, a carrier, an adjuvant or a combination thereof.

In some embodiments, the pharmaceutical composition further comprises other drugs for preventing or treating cancer, inflammatory syndromes or autoimmune diseases, or any combination thereof.

In some embodiments, the pharmaceutical composition can be in the form of a liquid, solid, semi-solid, gel or spray.

In another aspect, the present invention relates to use of the compound having formula (I) or formula (II), or a pharmaceutical composition thereof in the manufacture of a medicament for preventing or treating cancer, inflammation or autoimmune diseases mediated by RORγt in mammals, including humans.

In some embodiments, the present invention relates to use of the compound having formula (I) or formula (II), or a pharmaceutical composition thereof in the manufacture of a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease.

In another aspect, the present invention relates to a method of preparing, separating or purifying the compound having formula (I) or formula (II).

In another aspect, the present invention relates to intermediates for the preparation of compound having formula (I) or formula (II).

The compounds disclosed herein may contain asymmetric or chiral centers, and therefore exist in different stereoisomeric forms. It is intended that all stereoisomeric forms of the compound having formula (I) or formula (II) disclosed herein, including, but not limited to, diastereomers, enantiomers, atropisomers and geometric (or conformational) isomers, as well as mixtures thereof such as racemic mixtures, form part of the present invention.

In a structure disclosed herein, when the stereochemistry of any particular chiral atom is not specified, then all stereoisomers of that structure are contemplated within the present invention and are included in the present invention as compounds disclosed herein. When stereochemistry is indicated by a solid wedge or dashed line representing a particular configuration, then the stereoisomers of that structure are identified and defined.

Compound having formula (I) or formula (II) may exist in different tautomeric forms, and all such tautomers are included within the scope of the present invention.

Compound having formula (I) or formula (II) may exist in the form of salts. In one embodiment, the salt refers to a pharmaceutically acceptable salt. The phrase "pharmaceutically acceptable" refers to that the substance or composition must be chemically and/or toxicologically compatible with the other ingredients comprising a formulation, and/or the mammal being treated therewith. In another embodiment, the salt is not necessarily a pharmaceutically acceptable salt, but can be used for the preparation and/or purification of compound having formula (I) or formula (II) and/or can be used for the separation of intermediates of enantiomers of compound having formula (I) or formula (II).

Pharmaceutically acceptable acid addition salts can be formed by the action of the disclosed compounds with inorganic acids or organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, subsalicylate, tartrate, tosylate and trifluoroacetate salts.

Pharmaceutically acceptable base addition salts can be formed by the action of the disclosed compounds with inorganic or organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, *e.g.,* in "Remington's Pharmaceutical Sciences", 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002), all of which are incorporated herein by reference.

Furthermore, the compounds disclosed herein, including their salts, can also be obtained in the form of their hydrates, or include other solvents such as ethanol, DMSO, and the like, used for their crystallization. The compounds of the present invention may inherently or by design form solvates with pharmaceutically acceptable solvents (including water); therefore, it is intended that the invention embrace both solvated and unsolvated forms of the compounds disclosed herein.

Any formula given herein is also intended to represent isotopically unenriched forms as well as isotopically enriched forms of the compounds. Isotopically enriched compounds have the structure depicted by the general formula given herein, except that one or more atoms are replaced by the atom having a selected atomic mass or mass number. Examples of isotopes that can be incorporated into compounds of the invention include isotopes of hydrogen, carbon, nitrogen, oxygen, phosphorous, sulfur, fluorine, and chlorine, such as $^{2}H$, $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$ and $^{125}I$, respectively.

In another aspect, the compounds of the invention include isotopically enriched compounds as defined herein, for example those into which radioactive isotopes, such as $^{3}H$, $^{14}C$ and $^{18}F$, or those into which non-radioactive isotopes, such as $^{2}H$ and $^{13}C$ are present. Such isotopically enriched compounds are useful in metabolic studies (with $^{14}C$), reaction kinetic studies (with, for example $^{2}H$ or $^{3}H$), detection or imaging techniques, such as positron emission tomography (PE) or single-photon emission computed tomography (SPECT) including drug or substrate tissue distribution assays, or in radioactive treatment of patients. In particular, an $^{18}F$-enriched compound may be particularly desirable for PE or SPECT studies. Isotopically-enriched compound having formula (I) can generally be prepared by conventional techniques known to those skilled in the art or by processes analogous to those described in the accompanying Examples and Preparations using an appropriate isotopically-labeled reagent in place of the non-labeled reagent previously employed.

Further, substitution with heavier isotopes, particularly deuterium *(i.e.,* $^{2}H$ or D), may afford certain therapeutic advantages resulting from greater metabolic stability. For example, increased *in vivo* half-life or reduced dosage requirements or an improvement in therapeutic index. It is understood that deuterium in this context is regarded as a substituent of a compound having formula (I). The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). Pharmaceutically acceptable solvates in accordance with the invention include those wherein the solvent of crystallization may be isotopically substituted, e.g., $D_2O$, acetone-$d_6$, DMSO-$d_6$.

**PHARMACEUTICAL COMPOSITION OF THE COMPOUND OF THE INVENTION AND PREPARATIONS AND ADMINISTRATION**

The present invention provides a pharmaceutical composition comprising the compounds disclosed herein, such as those listed in the Examples; and pharmaceutically acceptable excipient, carrier, adjuvant and a combination thereof.

The present invention provides methods used in the treatment, prevention or improvement of diseases or symptoms thereof, comprising administrating to a patient a safe and effective combination drug containing a compound of the invention and one or more therapeutic active agents. Wherein, the combination drug includes one or more other drugs for preventing or treating inflammatory syndrome, disorder or disease, and the other drugs include but are not limited to:

1) TNF-$\alpha$ inhibitors; 2) non-selective COX-1/COX-2 inhibitors; 3) COX-2 inhibitors; 4) other therapeutic agents for the treatment of inflammatory syndromes and autoimmune diseases, including glucocorticoids, methotrexate, leflunomide, sulfasalazine, azathioprine, cyclosporine, tacrolimus, penicillamine, bucillamine, actarib, mizoribine, clobenzaprine, ciclesonide, hydroxychloroquine, aurothiomalate, auranofin, cyclophosphamide, BAFF/APRIL inhibitors, CTLA-4-immunoglobulin or analogs; 5) leukotriene biosynthesis inhibitors, 5-lipoxygenase inhibitors or 5-lipoxygenase-activated protein (FLAP) antagonists; 6) LTD4 receptor antagonists; 7) PDE4 inhibitors; 8) antihistamine HI receptor antagonists; $\alpha$1 and $\alpha$2-adrenoceptor agonists; 9) anticholinergics; 10) P-adrenergic receptor agonists; 11) insulin-like growth factor type I analogs 12) kinase inhibitors selected from Janus kinase inhibitors (JAK1 and/or JAK2 and/or JAK3 and/or TYK2), p38 MAPK and IKK2; 13) B cell targeting biological drugs such as rituximab; 14 ) selective costimulatory modulators such as abatacept; 15) interleukin inhibitors selected from IL-1 inhibitors such as anakinra, IL-6 inhibitors such as tocilizumab and IL-12/IL-23 inhibitors such as ustekinumab.

The amount of the compound of the pharmaceutical composition disclosed herein refers to an amount which can be effectively detected to inhibit the retinoic acid-related orphan nuclear receptor $\gamma$t of biology sample and patient. The dosage of active ingredient in the compositions of the present invention may vary, however, the amount of active ingredient must be such that an appropriate dosage form can be obtained. The active ingredient can be administered to patients (animals and humans) in need of such treatment in doses that provide optimal drug efficacy. The dose chosen will depend on the desired therapeutic effect, on the route of administration and on the duration of treatment. Dosages will vary from patient to patient, depending on the nature and severity of the disease, the weight of the patient, the patient's specific diet, concomitant medications, and other factors that will be recognized by those skilled in the art. In one embodiment, the dose ranges from about 0.5 mg to 500 mg per patient per day; in another embodiment, the dose ranges from about 0.5 mg to 200 mg per patient per day.

It will also be appreciated that certain of the compounds of present invention can exist in free form for treatment, or where appropriate, as a pharmaceutically acceptable derivative or a prodrug thereof. A pharmaceutically acceptable derivative includes pharmaceutically acceptable prodrugs, salts, esters, salts of such esters, or any other adduct or derivative which upon administration to a patient in need thereof is capable of providing, directly or indirectly, a compound as otherwise described herein, or a metabolite or residue thereof.

The medicament or pharmaceutical compositions of the invention may be prepared and packaged in bulk form wherein a safe and effective amount of a compound having formula (I) or formula (II) disclosed herein can be extracted and then given to the patient, such as with powders or syrups. Typically, patients are administered at dose levels between 0.0001 and 10 mg/kg body weight daily to obtain potent inhibition of the retinoic acid-related orphan nuclear receptor $\gamma$t. Alternatively, the pharmaceutical compositions of the invention may be prepared and packaged in unit dosage form wherein each physically discrete unit contains a safe and effective amount of a compound of Formula (I) disclosed herein. When prepared in unit dosage form, the pharmaceutical compositions disclosed herein may generally contain an effective dose of a compound disclosed herein.

When the pharmaceutical composition of the present invention contains one or more other active ingredients in addition to the compound of the present invention, the compound weight ratio of the compound of the present invention to the second active ingredient may vary and will depend on the effective dose of each ingredient. Typically, an effective dose of each is used. Thus, for example, when a compound of the present invention is mixed with another agent, the weight ratio of the compound of the present invention to the other agent will generally range from about 1000:1 to about 1:1000, such as about 200:1 to about 1:200. Mixtures of compounds of the present invention with other active ingredients are generally also within the above ranges, but in each case an effective dose of each active ingredient should be used.

"Pharmaceutically acceptable excipient" as used herein means a pharmaceutically acceptable material, composition or vehicle involved in giving form or consistency to the pharmaceutical composition. Each excipient must be compatible with the other ingredients of the pharmaceutical composition when commingled, such that interactions which would substantially reduce the efficacy of the compound of the invention when administered to a patient and would result in pharmaceutically unacceptable compositions are avoided. In addition, each excipient must of course be of sufficiently high purity to render it is pharmaceutically acceptable.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound of the present invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: diluents, fillers, binders, disintegrants, lubricants, glidants, granulating agents, coating agents, wetting agents, solvents, co-solvents, suspending agents, emulsifiers, sweetners, flavoring agents, flavor masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

Various carriers used in formulating pharmaceutically acceptable compositions and known techniques for the preparation thereof are disclosed in Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, the contents of each of which are incorporated by reference herein. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention.

The pharmaceutical compositions of the invention are prepared using techniques and methods known to those skilled in the art. Some of the methods commonly used in the art are described in Remington's Pharmaceutical Sciences (Mack Publishing Company).

Therefore, another aspect of the present invention is related to a method for preparing a pharmaceutical composition, the pharmaceutical composition contains the compound disclosed herein and pharmaceutically acceptable excipient, carrier, adjuvant or a combination thereof, the method comprises mixing various ingredients. The pharmaceutical composition containing the compound disclosed herein can be prepared at for example environment temperature and under barometric pressure.

The compound of the invention will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

In one embodiment, the compounds disclosed herein can be prepared to oral. In the other embodiment, the compounds disclosed herein can be prepared to inhalation. In the still other embodiment, the compounds disclosed herein can be prepared to nasal administration. In the yet other embodiment, the compounds disclosed herein can be prepared to transdermal administration. In the still yet other embodiments, the compounds disclosed herein can be prepared to topical administration.

The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxy groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Other useful liquid and semisolid dosage forms include, but are not limited to, those containing the active ingredient(s) provided herein, and a dialkylated mono- or poly-alkylene glycol, including, 1,2-dimethoxymethane, diglyme, triglyme, tetraglyme, polyethylene glycol-350-dimethyl ether, polyethylene glycol-550-dimethyl ether, polyethylene glycol-750-dimethyl ether, wherein 350, 550, and 750 refer to the approximate average molecular weight of the polyethylene glycol. These formulations may further comprise one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium metabisulfite, thiodipropionic acid and its esters, and dithiocarbamates.

Where appropriate, dosage unit formulations for oral administration can be microencapsulated. The formulation can also be prepared to prolong or sustain the release as for example by coating or embedding particulate material in polymers, wax, or the like.

The pharmaceutical compositions provided herein for oral administration may be also provided in the forms of liposomes, micelles, microspheres, or nanosystems. Miccellar dosage forms can be prepared as described in U.S. Pat. No. 6,350,458.

The pharmaceutical compositions provided herein may be provided as non-effervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a source of carbon dioxide.

Coloring and flavoring agents can be used in all of the above dosage forms.

The compounds disclosed herein can also be coupled to soluble polymers as targeted medicament carriers. Such polymers may encompass polyvinylpyrrolidone, pyran copolymer, polyhydroxypropylmethacrylamido-phenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

The pharmaceutical compositions provided herein may be formulated as immediate or modified release dosage forms, including delayed, sustained, pulsed, controlled, targeted, and programmed-release forms.

The pharmaceutical compositions provided herein may be co-formulated with other active ingredients which do not impair the desired therapeutic action, or with substances that supplement the desired action.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (see, Remington: The Science and Practice of Pharmacy, supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

Suitable aqueous vehicles include, but are not limited to, water, saline, physiological saline or phosphate buffered saline (PBS), sodium chloride injection, Ringers injection, isotonic dextrose injection, sterile water injection, dextrose and lactated Ringers injection. Non-aqueous vehicles include, but are not limited to, fixed oils of vegetable origin, castor oil, corn oil, cottonseed oil, olive oil, peanut oil, peppermint oil, safflower oil, sesame oil, soybean oil, hydrogenated vegetable oils, hydrogenated soybean oil, and medium-chain triglycerides of coconut oil, and palm seed oil. Water-miscible vehicles include, but are not limited to, ethanol, 1,3-butanediol, liquid polyethylene glycol (e.g., polyethylene glycol 300 and polyethylene glycol 400), propylene glycol, glycerin, N-methyl-2-pyrrolidone, *N*,*N*-dimethylacetamide, and dimethyl sulfoxide.

Suitable antimicrobial agents or preservatives include, but are not limited to, phenols, cresols, mercurials, benzyl alcohol, chlorobutanol, methyl and propyl p-hydroxybenzoates, thimerosal, benzalkonium chloride (e.g., benzethonium chloride), methyl- and propyl-parabens, and sorbic acid. Suitable isotonic agents include, but are not limited to, sodium chloride, glycerin, and dextrose. Suitable buffering agents include, but are not limited to, phosphate and citrate. Suitable antioxidants are those as described herein, including bisulfite and sodium metabisulfite. Suitable local anesthetics include, but are not limited to, procaine hydrochloride. Suitable suspending and dispersing agents are those as described herein, including sodium carboxymethylcelluose, hydroxypropyl methylcellulose, and polyvinylpyrrolidone. Suitable emulsifying agents include those described herein, including polyoxyethylene sorbitan monolaurate, polyoxyethylene sorbitan monooleate 80, and triethanolamine oleate. Suitable sequestering or chelating agents include, but are not limited to EDTA. Suitable pH adjusting agents include, but are not limited to, sodium hydroxide, hydrochloric acid, citric acid, and lactic acid. Suitable complexing agents include, but are not limited to, cyclodextrins, including α-cyclodextrin, β-cyclodextrin, hydroxypropyl-β-cyclodextrin, sulfobutylether-β-cyclodextrin, and sulfobutylether 7-β-cyclodextrin.

The pharmaceutical compositions provided herein may be formulated for single or multiple dosage administration. The single dosage formulations are packaged in an ampoule, a vial, or a syringe. The multiple dosage parenteral formulations must contain an antimicrobial agent at bacteriostatic or fungistatic concentrations. All parenteral formulations must be sterile, as known and practiced in the art.

In one embodiment, the pharmaceutical compositions are provided as ready-to-use sterile solutions. In another embodiment, the pharmaceutical compositions are provided as sterile dry soluble products, including lyophilized powders and hypodermic tablets, to be reconstituted with a vehicle prior to use. In yet another embodiment, the pharmaceutical compositions are provided as ready-to-use sterile suspensions. In yet another embodiment, the pharmaceutical compositions are provided as sterile dry insoluble products to be reconstituted with a vehicle prior to use. In still another embodiment, the pharmaceutical compositions are provided as ready-to-use sterile emulsions.

The pharmaceutical compositions may be formulated as a suspension, solid, semi-solid, or thixotropic liquid, for administration as an implanted depot. In one embodiment, the pharmaceutical compositions provided herein are dispersed in a solid inner matrix, which is surrounded by an outer polymeric membrane that is insoluble in body fluids but allows the active ingredient in the pharmaceutical compositions diffuse through.

Suitable inner matrixes include polymethylmethacrylate, polybutyl-methacrylate, plasticized or unplasticized polyvinylchloride, plasticized nylon, plasticized polyethylene terephthalate, natural rubber, polyisoprene, polyisobutylene, polybutadiene, polyethylene, ethylene-vinyl acetate copolymers, silicone rubbers, polydimethylsiloxanes, silicone carbonate copolymers, hydrophilic polymers, such as hydrogels of esters of acrylic and methacrylic acid, collagen, cross-linked polyvinyl alcohol, and cross-linked partially hydrolyzed polyvinyl acetate.

Suitable outer polymeric membranes include polyethylene, polypropylene, ethylene/propylene copolymers, ethylene/ethyl acrylate copolymers, ethylene/vinyl acetate copolymers, silicone rubbers, polydimethyl siloxanes, neoprene rubber, chlorinated polyethylene, polyvinylchloride, vinyl chloride copolymers with vinyl acetate, vinylidene chloride,

ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer.

In another aspect, the pharmaceutical composition of the invention is prepared to a dosage form adapted for administration to a patient by inhalation, for example as a dry powder, an aerosol, a suspension, or a solution composition. In one embodiment, the invention is directed to a dosage form adapted for administration to a patient by inhalation as a dry powder. In one embodiment, the invention is directed to a dosage form adapted for administration to a patient by inhalation via a nebulizer. Dry powder compositions for delivery to the lung by inhalation typically comprise a compound disclosed herein or a pharmaceutically acceptable salt thereof as a finely divided powder together with one or more pharmaceutically-acceptable excipients as finely divided powders. Pharmaceutically-acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (e.g., micronised) compound can be defined by a $D_{50}$ value of about 1 to about 10 microns (for example as measured using laser diffraction).

Aerosols may be formed by suspending or dissolving a compound disclosed herein or a pharmaceutically acceptable salt thereof in a liquified propellant. Suitable propellants include halocarbons, hydrocarbons, and other liquified gases. Representative propellants include: trichlorofluoromethane (propellant 11), dichlorofluoromethane (propellant 12), dichlorotetrafluoroethane (propellant 114), tetrafluoroethane (HFA-134a), 1,1-difluoroethane (HFA-152a), difluoromethane (HFA-32), pentafluoroethane (HFA-12), heptafluoropropane (HFA-227a), perfluoropropane, perfluorobutane, perfluoropentane, butane, isobutane, and pentane. Aerosols comprising a compound of formula (I) or a pharmaceutically acceptable salt thereof will typically be administered to a patient via a metered dose inhaler (MDI). Such devices are known to those skilled in the art.

The aerosol may contain additional pharmaceutically-acceptable excipients typically used with MDIs such as surfactants, lubricants, cosolvents and other excipients to improve the physical stability of the formulation, to improve valve performance, to improve solubility, or to improve taste.

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils. Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Topical preparations may be administered by one or more applications per day to the affected area; over skin areas occlusive dressings may advantageously be used. Continuous or prolonged delivery may be achieved by an adhesive reservoir system.

## USE OF THE COMPOUNDS AND PHARMACEUTICAL COMPOSITIONS

The compounds or pharmaceutical compositions disclosed in the present invention can be used in the manufacture of a medicament for treating, preventing, improving, controlling or alleviating cancer, inflammation or autoimmune diseases mediated by RORyt in mammals, including humans, and can also be used in the manufacture of other medicaments for inhibiting RORyt.

In particular, the amount of the compound in the composition of the present invention is effective to detectably inhibit RORyt, and the compound of the present invention can be used as a medicament for preventing or treating cancer, psoriasis, rheumatoid arthritis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease in humans.

The compounds or compositions of the present invention may be used, but in no way limited to, to prevent, treat or ameliorate cancer, psoriasis, rheumatoid arthritis, systemic lupus erythematosus, multiple sclerosis, inflammatory

bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease, or Kawasaki disease in mammals, including humans, using an effective amount of the compounds or compositions of the present invention administered to a patient.

Besides being useful for human treatment, the compounds of the present invention or pharmaceutically compositions are also useful for veterinary treatment of animals such as companion animals, exotic animals and mammals in farm animals. In other embodiments, the animals disclosed herein include horses, dogs, and cats. As used herein, the compounds disclosed herein include the pharmaceutically acceptable derivatives thereof.

**General synthetic** steps:

To describe the invention, the following examples are listed. However, it should be understood that the present invention is not limited to these embodiments, but merely provides a method for practicing the present invention.

Generally, the compounds disclosed herein may be prepared by methods described herein, wherein the substituents are as defined for Formula (I) or Formula (II) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, *e.g.*, by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Tianjin Fuchen Chemical Reagent Factory, Wuhan Xinhuayuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous $CH_2Cl_2$ and $CHCl_3$ were obtained by refluxing the solvent with $CaH_2$. EtOAc, PE, *n*-hexane, *N*,*N*-dimethylacetamide and DMF were treated with anhydrous $Na_2SO_4$ prior to use.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Column chromatography was conducted using a silica gel column. Silica gel (300-400 mesh) was purchased from Qingdao Ocean Chemical Factory.

$^1H$ NMR spectra were recorded by a Bruker 400 MHz spectrometer or Bruker 600 spectrometer, using $CDCl_3$, DMSO-$d_6$, $CD_3OD$ or acetone-$d_6$ (reported in ppm) as solvent, and using TMS (0 ppm) or chloroform (7.26 ppm) as the reference standard. When peak multiplicities were reported, the following abbreviations were used: s (singlet), d (doublet), t (triplet), m (multiplet), br (broadened), dd (doublet of doublets), and dt (doublet of triplets). Coupling constants, when given, were reported in Hertz (Hz).

The measurement conditions for low-resolution mass spectrometry (MS) data are: Agilent 6120 quadrupole HPLC-M (column model: Zorbax SB-C18, 2.1 × 30 mm, 3.5 μm, 6 min, flow rate 0.6 mL / min. Mobile phase: 5%-95% (ACN with 0.1% formic acid) in ($H_2O$ with 0.1% formic acid) using electrospray ionization (ESI) at 210 nm / 254 nm with UV detection.

The purity of the compound was determined by high performance liquid chromatography (HPLC) using an Agilent 1260 HPLC (column model: Agilent zorbax Eclipse Plus C18) and detected by a DAD detector, and finally the area normalization method was used to calculate the compound purity.

The following abbreviations are used throughout the specification:

| | |
|---|---|
| $Ac_2O$ | Acetic Anhydride |
| AcOH | Acetic Acid |
| AgOTf | Silver trifluoromethanesulfonate; |
| $Boc_2O$ | Di-*tert*-butyl dicarbonate; |
| CbzCl | benzyl chloroformate; |
| $CDC1_3$ | deuterated chloroform |

| | |
|---|---|
| CDI | *N*,*N*'-carbonyldiimidazole; |
| DCM | dichloromethane; |
| DAST | diethylaminosulfur trifluoride; |
| DIAD | diisopropyl azodicarboxylate; |
| DMF | *N*,*N*-dimethylformamide; |
| DIPEA | *N*,*N*-diisopropylethylamine; |
| DMAP | 4-dimethylaminopyridine |
| DMSO | dimethylsulfoxide; |
| DMSO-$d_6$ | deuterated dimethyl sulfoxide; |
| deep·$HBF_4$ | 1,3 -bis(dicyclohexylphosphine)propanebis(tetrafluoroborate) |
| EtI | iodoethane |
| EtOAc/EA | ethyl acetate; |
| EtOH | ethyl alcohol; |
| Et3N/TEA | triethylamine; |
| EDCI | 1-ethyl-(3-dimethylaminopropyl)carbodiimide hydrochloride; |
| HATU | 2-(7-aza-1*H*-benzotriazole-1-yl)-1,1,3,3-tetramethyluronium hexafluorophosphate; |
| HOBT | 1-hydroxybenzotriazole; |
| *i*-PrOH | isopropanol; |
| KOAc | potassium acetate; |
| *t*-BuOK | potassium *tert*-butoxide |
| *t*-BuONa | sodium *tert*-butoxide |
| LiOH | lithium hydroxide |
| $LiBH_4$ | lithium borohydride |
| MeCN/ACN | acetonitrile; |
| MeI | iodomethane; |
| MeOH | methanol; |
| MeONa | sodium methoxide; |
| MsCl | methylsulfonyl chloride; |
| $Pd_2(dba)_3$ | tris(dibenzylideneacetone)dipalladium |
| $Pd(dppf)Cl_2$ | [1,1'-bis(diphenylphosphino)ferrocene]dichloropalladium(ii); |
| $Pd(OAc)_2$ | palladium diacetate; |
| Pd(P(*o*-ToL)$_3$)$_2$(OAc)$_2$ | diacetoxybis(tri-o-tolylphosphine)-palladium(ii); |
| PE | petroleum ether; |
| $TMSCF_3$ | (trifluoromethyl)trimethylsilane; |
| TBSOTf | *tert*-butyldimethylsilyl trifluoromethanesulfonate; |
| $Pd(PPh_3)_2Cl_2$ | bis(triphenylphosphine)palladium(ii) chloride; |
| $TMSCF_2Br$ | (bromo(difluoro)methyl)trimethylsilane; |
| Pd/C | palladium on activated carbon |
| TLC thin | layer chromatography; |
| THF | tetrahydrofuran; |
| TFA | trifluoroacetic acid; |
| XantPhos | 4,5-bis(diphenylphosphino)-9,9-dimethylxanthene |
| g | gram |
| h | hour, hours |
| min minute, | minutes |
| mmol | millimole |
| °C Celsius mL (ml) | milliliter |
| RT, rt, r.t. | room temperature |
| rpm | revolution per minute |
| Dess-Martin | reagent 1,1-dihydro-1,1,1-triacetoxy-1,2-benzoiodooxol-3(1*H*)-one |
| Rt | retention time; |
| *t*-Bu-XPhos | 2-di-*tert*-butylphosphino-2',4',6'-triisopropylbiphenyl; |
| Lawesson's | Reagent; |
| N(*n*-Bu)$_4$CN | tetrabutylammonium cyanide; |

Typical synthetic steps for preparing the disclosed compounds of the present invention are shown in the following synthetic schemes. Wherein, X represents a halogen atom; PG represents an amino protecting group; each $T_1$ and $T_2$ is independently selected from a carboxyl group or an amino group, and $T_1$ and $T_2$ cannot be the same group; n is 1 or

3. Unless otherwise specified, rings B, R, $R^2$, $R^3$, $R^4$, $Z^1$, $Z^2$, $Z^3$, $Z^4$, $Z^5$, $Z^6$, $Z^7$ and $Z^8$ have the meanings as described herein.

**Synthesis of important intermediates:**

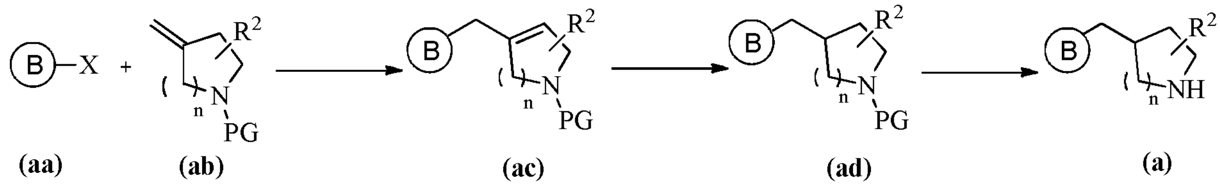

(aa) (ab) (ac) (ad) (a)

Intermediate (a) can be prepared by the following procedure:
Compound (aa) can react with compound (ab) to give compound (ac), compound (ac) can be subjected to catalytic hydrogenation to give compound (ad), and then compound (ad) can be deaminated to give the intermediate compound (a).

Synthetic scheme 1

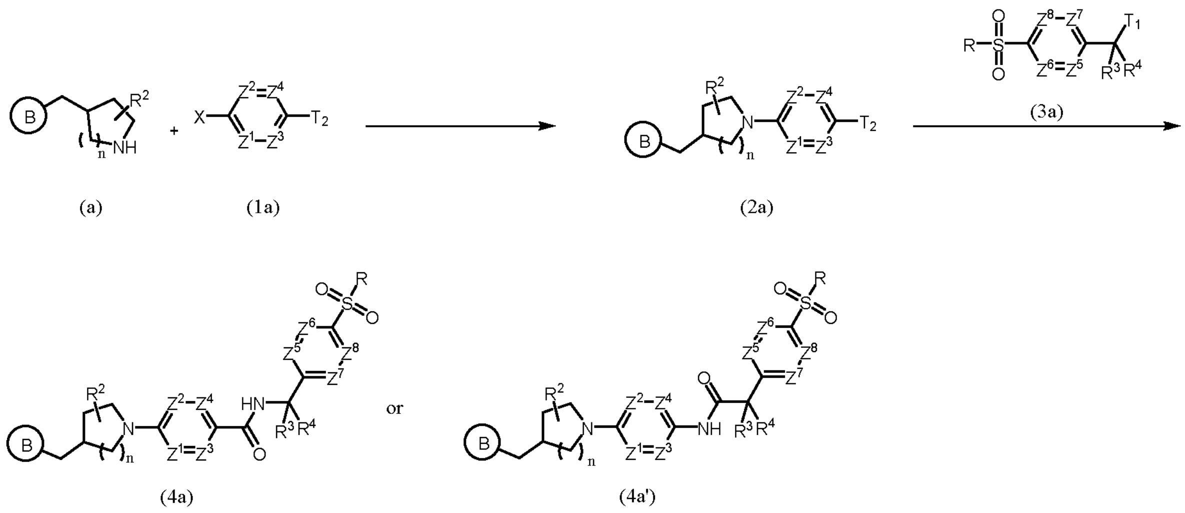

Compound (4a) or (4a') can be prepared by the following procedure:
Compound (a) can be substituted with compound (1a) to give compound (2a), then compound (2a) and compound (3a) can undergo acylation to give compound (4a) or (4a').

Synthetic scheme 2

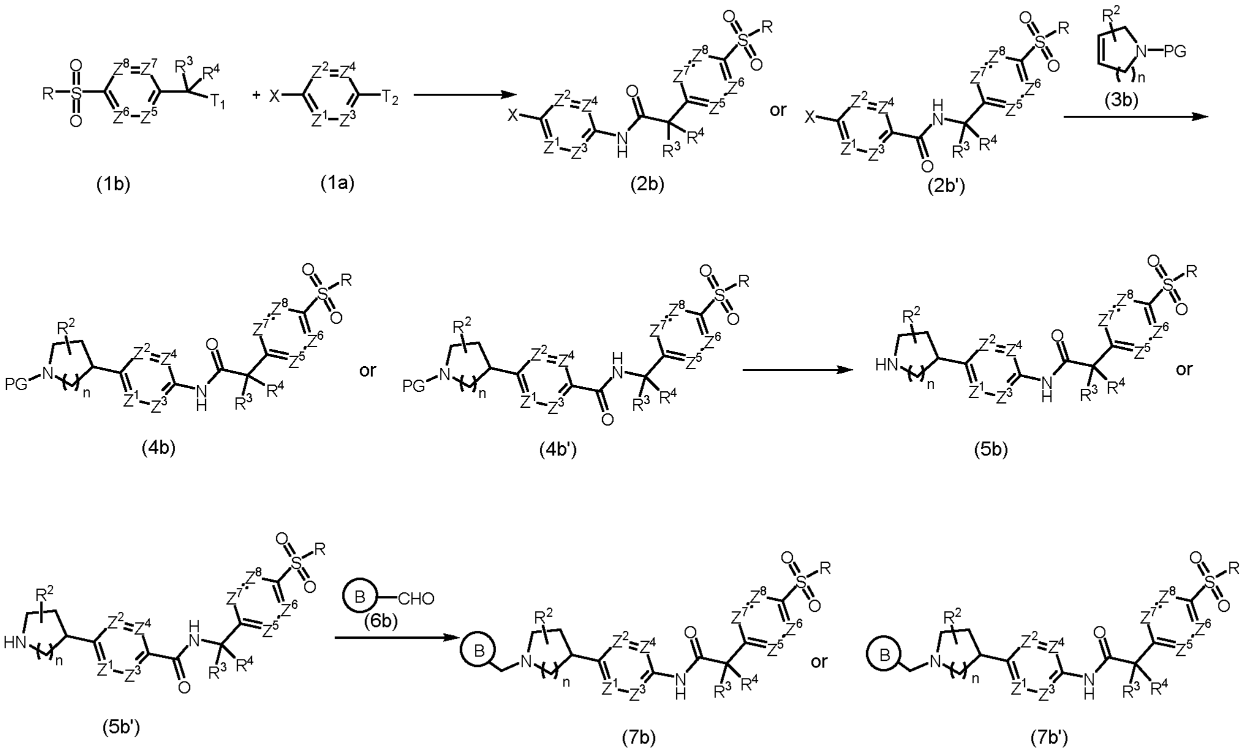


(1b) (1a) (2b) or (2b') (3b)

(4b) or (4b') (5b) or

(5b') (7b) or (7b')

Compound (7b) or (7b') can be prepared by the following procedure:
Compound (1b) and compound (1a) can undergo acylation to give compound (2b) or (2b'), compound (2b) or (2b') and compound (3b) can undergo addition reaction to give compound (4b) or (4b'), compound (4b) or (4b') can be deaminated to give compound (5b) or (5b'), compound (5b) or (5b') can react with compound (6b) to give compound (7b) or (7b').

**Synthetic scheme 3**

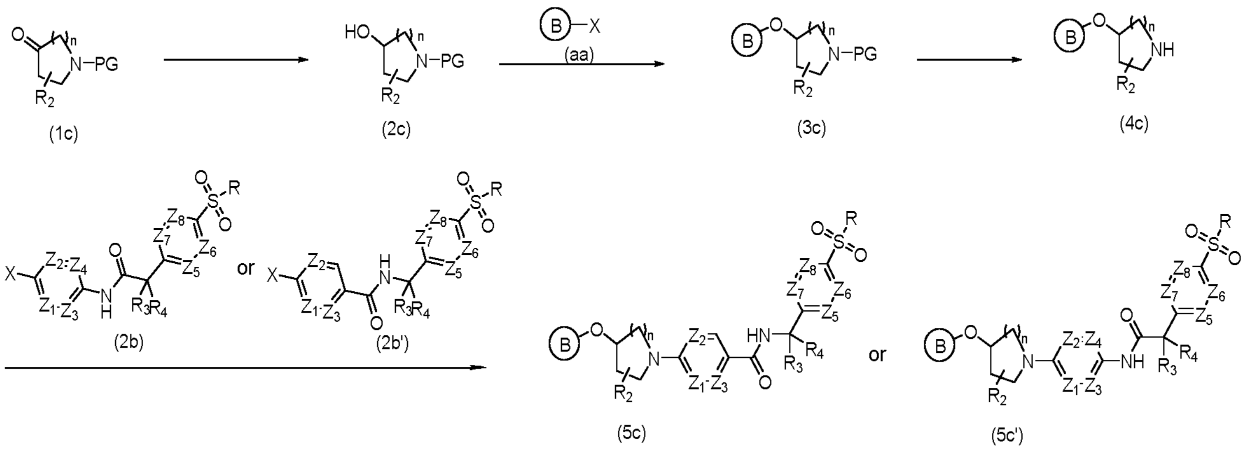


(1c) (2c) (3c) (4c)

(2b) or (2b') (5c) or (5c')

Compound (5c) or (5c') can be prepared by the following procedure:
Compound (1c) can undergo addition reaction to give compound (2c), compound (2c) and compound (aa) can undergo substitution reaction to give compound (3c), compound (3c) can be deaminated to give compound (4c), compound (4c) can be subjected to substitution reaction with compound (2b) or (2b') to give compound (5c) or (5c').

**Synthetic scheme 4**

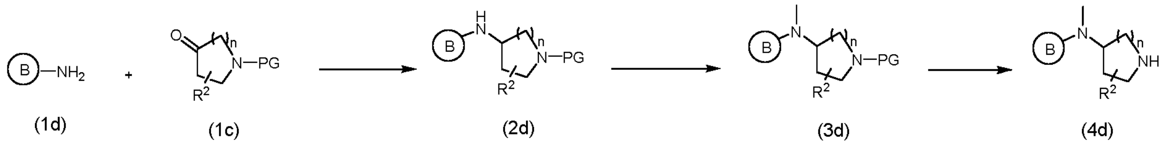

(1d) (1c) (2d) (3d) (4d)

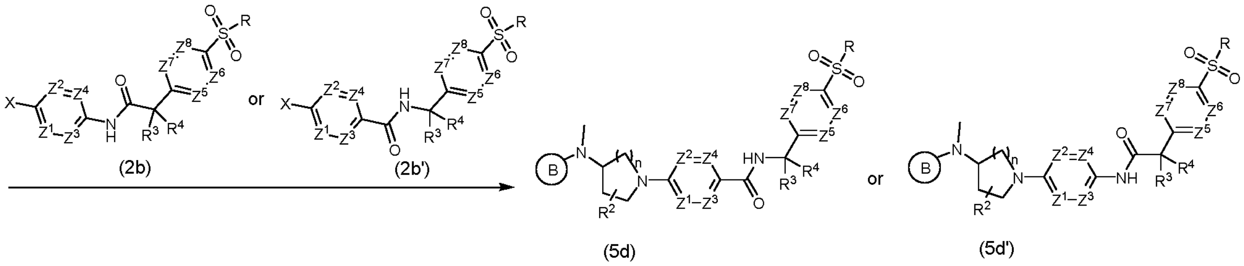

(2b) or (2b') (5d) or (5d')

Compound (5d) or (5d’) can be prepared by the following procedure:
Compound (1d) can react with compound (1c) to give compound (2d), compound (2d) can be methylated to give compound (3d), compound (3d) can be deaminated to give compound (4d), compound (4d) can be subjected to substitution reaction with compound (2b) or (2b’) to give compound (5d) or (5d’).

The compounds, pharmaceutical compositions and uses thereof provided by the present invention will be further described below with reference to the examples.

**Synthesis of intermediate: (*R*)-2-amino-2-(4-(ethylsulfonyl)phenyl) ethanol**

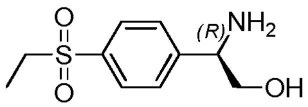

It was prepared by the method disclosed in patent application WO2017132432.

**Examples**

**Example 1 2-(4-(Ethylsulfonyl)phenyl)-*N*-(4-(3-(4-(trifluoromethyl)benzyl)pyrrolidin -1-yl)phenyl)acetamide**

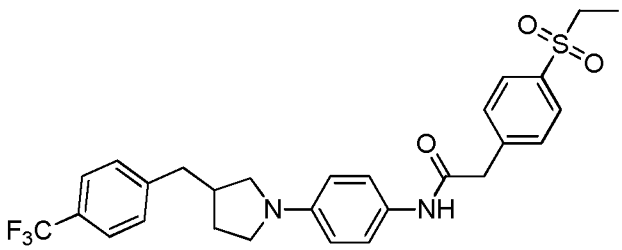

**Step 1: Synthesis of *tert-butyl* 4-(4-(trifluoromethyl)benzyl)-2,3-dihydro-1*H*-pyrrole -1-carboxylate**

Under nitrogen protection, 1-bromo-4-(trifluoromethyl)benzene (1.00 g, 4.40 mmol), *tert-butyl* 3-methylenepyrrolidine-1-carboxylate (1.20 g, 6.50 mmol), $Pd(P(o\text{-}Tol)_3)_2(OAc)_2$ (14 mg, 0.02 mmol) and $Ag_2CO_3$ (1.20 g, 4.40 mmol) were dissolved in DMF (10 mL), then DIPEA(3.00 mL, 18.15 mmol) was added. The mixture was heated to 100 °C and stirred for 24 h. The mixture was cooled to room temperature. The reaction solution was diluted with EtOAc (100 mL), washed successively with $NaHCO_3$ solution (15 mL×2) and saturated NaCl solution (15 mL×3), dried over anhydrous $Na_2SO_4$, concentrated under reduced pressure, and the crude product was separated by silica gel column chromatography (eluent: PE/DCM/EtOAc (v/v/v) = 10/1/1) to give yellow oily liquid (1.03 g, 71%).

MS (ESI, pos.ion) m/z: 272.0 $[M-56+H]^+$.

**Step 2: Synthesis of tert-butyl 3-(4-(trifluoromethyl)benzyl)pyrrolidine-1-carboxylate**

To a solution of *tert-butyl* 4-(4-(trifluoromethyl)benzyl)-2,3-dihydro -1*H*-pyrrole-1-carboxylate (1.03 g, 3.15 mmol) in MeOH (20 mL) was added Pd/C (132 mg, 10%) and the mixture was stirred at room temperature for 2 h under hydrogen protection. The reaction solution was filtered, the filtrate was concentrated under reduced pressure, and the crude product was separated by silica gel column chromatography (eluent: PE/DCM/EtOAc (v/v/v) = 6/1/1) to give yellow oily liquid (143 mg, 14%).
MS (ESI, pos.ion) m/z: 274.2 $[M-56+H]^+$.

**Step 3: Synthesis of 3-(4-(trifluoromethyl)benzyl)pyrrolidine hydrochloride**

*tert*-Butyl 3-(4-(trifluoromethyl)benzyl)pyrrolidine-1-carboxylate (143 mg, 0.43 mmol) was dissolved in a solution of HCl in *i*-PrOH (4 mL, 24.00 mmol, 6 mol/L) and the mixture was stirred at room temperature for 6 h. The mixture was concentrated under reduced pressure to give 3-(4-(trifluoromethyl)benzyl)pyrrolidine hydrochloride.
MS (ESI, pos.ion) m/z: 230.2 $[M+H]^+$.

**Step 4: Synthesis of 1-(4-nitrophenyl)-3-(4-(trifluoromethyl)benzyl)pyrrolidine**

Under nitrogen protection, 3-(4-(trifluoromethyl)benzyl)pyrrolidine hydrochloride, 1-fluoro-4-nitrobenzene (0.10 mL, 0.94 mmol) and DIPEA (0.74 mL, 4.30 mmol) were dissolved in ACN (4 mL). The mixture was heated and stirred at 80°C for 24 h. The reaction solution was cooled to room temperature. Then the mixture was diluted with DCM (50 mL), washed successively with $NaHCO_3$ solution (20 mL) and saturated NaCl solution (10 mL), dried over anhydrous $Na_2SO_4$, and concentrated under reduced pressure. The crude product was separated by silica gel column chromatography (eluent: PE/DCM/EtOAc (v/v/v) = 10/1/1) to give a yellow solid (105 mg, 69%).
MS (ESI, pos.ion) m/z: 351.2 $[M+H]^+$.

**Step 5: Synthesis of 4-(3-(4-(trifluoromethyl)benzyl)pyrrolidin-1-yl)aniline**

To a solution of 1-(4-nitrophenyl)-3-(4-(trifluoromethyl)benzyl)pyrrolidine (105 mg, 0.30 mmol) in MeOH (5 mL) was added Pd/C (20 mg, 10%) under hydrogen protection. The mixture was stirred at room temperature for 12 h. The reaction solution was filtered, and the filtrate was concentrated under reduced pressure to give a dark solid (75 mg, 78%).
MS (ESI, pos.ion) m/z: 321.2 $[M+H]^+$.

**Step 6: Synthesis of 2-(4-(ethylsulfonyl)phenyl)-*N*-(4-(3-(4-(trifluoromethyl)benzyl) pyrrolidin-1-yl) phenyl)acetamide**

Under nitrogen protection, 4-(3-(4-(trifluoromethyl)benzyl)pyrrolidin-1-yl)aniline (75 mg, 0.24 mmol), 2-(4-(ethylsulfonyl)phenyl)acetic acid (106 mg, 0.46 mmol), EDCI (89 mg, 0.46 mmol) and HOBT (64 mg, 0.47 mmol) were dissolved in DCM (4 mL), then TEA (0.14 mL, 1.01 mmol) was added and the mixture was stirred at room temperature for 18 h. The reaction solution was diluted with DCM (80 mL), washed successively with HCl solution (20 mL, 1 mol/L), saturated NaCl solution (20 mL) and saturated $NaHCO_3$ solution (20 mL), dried over anhydrous $Na_2SO_4$, and concentrated under reduced pressure. The crude product was separated by silica gel column chromatography (eluent: PE/DCM/EtOAc (v/v/v) = 6/1/1) to give a colorless solid (75 mg, 60%).
MS (ESI, pos.ion) m/z: 531.1 $[M+H]^+$;
$^1H$ NMR (400 MHz, $CDCl_3$) $\delta$ (ppm): 7.90 (d, *J* = 8.2 Hz, 2H), 7.56 (d, *J* = 8.1 Hz, 4H), 7.31 (d, *J* = 7.9 Hz, 2H), 7.24 (d, *J* = 7.9 Hz, 2H), 6.98 (s, 1H), 6.45 (d, *J* = 8.8 Hz, 2H), 3.77 (s, 2H), 3.37 - 3.32 (m, 1H), 3.28 (s, 1H), 3.10 (q, *J*= 7.5 Hz, 2H), 2.98 (t, *J*= 8.0 Hz, 1H), 2.81 (d, *J*= 7.5 Hz, 2H), 2.64 - 2.56 (m, 1H), 2.15 - 2.05 (m, 1H), 2.01 (dd, *J* = 11.3, 5.8 Hz, 1H), 1.77 (dd, *J* = 10.7, 6.5 Hz, 1H), 1.30 (t, *J* = 7.5 Hz, 3H).

**Example 2 *N*-(4-(ethylsulfonyl)phenyl)-4-(3-(4-(trifluoromethyl)benzyl)pyrrolidin-1-yl) benzamide**

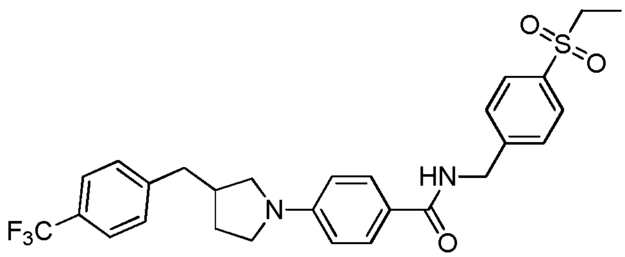

**Step 1: Synthesis of methyl 4-(3-(4-(trifluoromethyl)benzyl)pyrrolidin-1-yl)benzoate**

Under nitrogen protection, 3-(4-(trifluoromethyl)benzyl)pyrrolidine (400 mg, 1.75 mmol), methyl 4-bromobenzoate (750 mg, 3.49 mmol), $Pd_2(dba)_3$ (141 mg, 0.15 mmol), XantPhos (173 mg, 0.30 mmol) and $Cs_2CO_3$ (1.11 g, 3.41 mmol) were dissolved in 1,4-dioxane (12 mL) and the mixture was heated and stirred in a 100°C oil bath for 18 h. The reaction solution was cooled to room temperature, concentrated under reduced pressure, diluted with DCM (50 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 6/1) to give a white powder (400 mg, 64%).

MS (ESI, pos.ion) m/z: 364.2 $[M+H]^+$.

**Step 2: Synthesis of 4-(3-(4-(trifluoromethyl)benzyl)pyrrolidin-1-yl)benzoic acid**

To a solution of methyl 4-(3-(4-(trifluoromethyl)benzyl)pyrrolidin-1-yl)benzoate (200 mg, 0.55 mmol) in MeOH (6 mL) and THF (10 mL) was added a solution of $LiOH{\cdot}H_2O$ (461 mg, 10.99 mmol) in $H_2O$ (3 mL). The mixture was reacted at room temperature at 70°C for 11 h. The reaction solution was cooled to room temperature. HCl solution (1 mol/L) was added to the reaction solution to adjust the pH of the solution to about 5 (a large amount of white solid particles were precipitated during the addition). The resulting mixture was concentrated under reduced pressure, and filtered to obtain the product as a white solid. The filtrate was extracted with EtOAc (30 mL×2), and the organic phases were combined and dried over anhydrous $Na_2SO_4$, concentrated under reduced pressure to give the product as a white solid. The two white solids were combined to give a white solid (192 mg, 100%).

MS (ESI, pos.ion) m/z: 350.2 $[M+H]^+$.

**Step 3: Synthesis of *N*-(4-(ethylsulfonyl)benzyl)-4-(3-(4-(trifluoromethyl)benzyl) pyrrolidin-1-yl) benzamide**

HATU (165 mg, 0.43 mmol), 4-(3-(4-(trifluoromethyl)benzyl)pyrrolidin-1-yl)benzoic acid (100 mg, 0.29 mmol) and (4-(ethylsulfonyl)phenyl)methanamine (88 mg, 0.44 mmol) were dissolved in DCM (5 mL), then TEA (88 mg, 0.87 mmol) was added and the mixture was stirred at room temperature for 4 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: EtOAc/DCM (v/v) = 1/4) to give a white powder (100 mg, 66%).

MS (ESI, pos.ion) m/z: 531.2 $[M+H]^+$;

$^1H$ NMR (400 MHz, $CDCl_3$) δ (ppm): 7.76 (d, *J* = 8.3 Hz, 2H), 7.72 (d, *J* = 8.8 Hz, 2H), 7.57 (d, *J* = 8.0 Hz, 2H), 7.46 (d, *J* = 8.2 Hz, 2H), 7.32 (d, *J* = 8.0 Hz, 2H), 6.77 (t, *J* = 6.0 Hz, 1H), 6.48 (d, *J* = 8.8 Hz, 2H), 4.68 (d, *J* = 6.0 Hz, 2H), 3.48 - 3.39 (m, 2H), 3.36 - 3.30 (m, 1H), 3.10 - 3.02 (m, 3H), 2.83 (d, *J* = 7.5 Hz, 2H), 2.66 - 2.59 (m, 1H), 2.17 - 2.10 (m, 1H), 1.79 - 1.74 (m, 1H), 1.24 (t, *J* = 7.4 Hz, 3H).

$^{13}C$ NMR (151 MHz, $CDCl_3$) δ (ppm): 167.540, 149.99, 145.65, 144.36, 137.06, 128.96, 128.66, 128.45, 128.07, 125.46, 119.89, 110.79, 52.72, 50.62, 47.14, 43.10, 40.18, 39.36, 31.19, 7.39.

**Example 3 2-(4-(Ethylsulfonyl)phenyl)-*N*-(4-(1-(4-(trifluoromethyl)benzyl)pyrrolidin -3-yl)phenyl) acetamide**

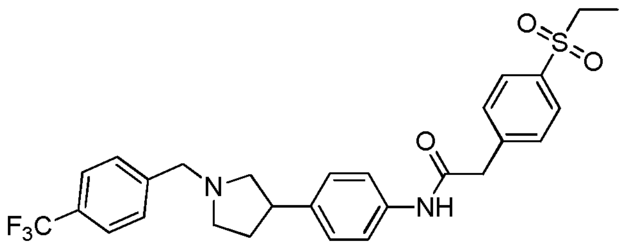

**Step 1: Synthesis of 2-(4-(ethylsulfonyl)phenyl)-*N*-(4-iodophenyl)acetamide**

Under nitrogen protection, 2-(4-(ethylsulfonyl)phenyl)acetic acid (10.20 g, 44.73 mmol), 4-iodoaniline (7.50 g, 34.25 mmol) and HATU (19.80 g, 52.07 mmol) were dissolved in DCM (150 mL), then DIPEA (13 mL, 73.20 mmol) was added, and the mixture was stirred at room temperature for 18 h. The reaction solution was added with DCM (200 mL), and washed with water (50 mL × 4). The organic phase was dried over anhydrous $Na_2SO_4$, concentrated under reduced pressure, and the crude product was separated by silica gel column chromatography (eluent: PE/EtOAc (v/v) = 1/1) to give a white solid (14.20 g, 97%).

MS (ESI, pos.ion) m/z: 430.1 $[M+H]^+$.

**Step 2: Synthesis of benzyl 3-(4-(2-(4-(ethylsulfonyl)phenyl)acetamido)phenyl) -2,3-dihydro-1*H*pyrrole-1-carboxylate**

Under nitrogen protection, 2-(4-(ethylsulfonyl)phenyl)-*N*-(4-iodophenyl)acetamide (500 mg, 1.17 mmol), benzyl 2,5-dihydropyrrole-1-carboxylate (0.41 g, 2.00 mmol), $Pd(P(o\text{-}Tol)_3)_2(OAc)_2$ (150 mg, 0.16 mmol), $Ag_2CO_3$ (320 mg, 1.16 mmol) and DIPEA (0.20 mL, 1.00 mmol)) were dissolved in DMF (40 mL), and the mixture was heated and stirred in a 110°C oil bath for 8 h. The reaction solution was cooled to room temperature and filtered, the filtrate was concentrated under reduced pressure, and the crude product was separated by silica gel column chromatography (eluent: DCM/MeOH (v/v) = 20/1) to give a pale yellow solid (130 mg, 22%).

MS (ESI, pos.ion) m/z: 505.6 $[M+H]^+$.

**Step 3: Synthesis of 2-(4-(ethylsulfonyl)phenyl)-*N*-(4-(pyrrolidin-3-yl)phenyl)acetamide**

To a solution of benzyl 3-(4-(2-(4-(ethylsulfonyl)phenyl)acetamido)phenyl)-2,3 -dihydro-1*H*-pyrrole-1-carboxylate (120 mg, 0.24 mmol) in MeOH (50 mL) was added Pd/C (155 mg, 5%) and the mixture was stirred at room temperature for 6 h under hydrogen protection. The reaction solution was filtered through a celite pad, the filtrate was concentrated under reduced pressure, and the crude product was separated by silica gel column chromatography (eluent: DCM/MeOH (v/v) = 10/1) to give a brown solid (80 mg, 90%).

MS (ESI, pos.ion) m/z: 373.1 $[M+H]^+$.

**Step 4: Synthesis of 2-(4-(ethylsulfonyl)phenyl)-*N*-(4-(1-(4-(trifluoromethyl) benzyl)pyrrolidin-3-yl) phenyl)acetamide**

Under an ice bath, to a solution of 2-(4-(ethylsulfonyl)phenyl)-*N*-(4-(pyrrolidin-3-yl)phenyl)acetamide (100 mg, 0.27 mmol) and 4-(trifluoromethyl)benzaldehyde (0.10 mL, 0.70 mmol) in MeOH (20 mL) was added $NaBH_3CN$ (100 mg, 1.21 mmol). The mixture was stirred at room temperature for 12 h. The reaction solution was concentrated under reduced pressure to remove the organic solvent, and the crude product was separated by silica gel column chromatography (eluent: DCM/MeOH (v/v) = 15/1) to give a pale yellow solid (110 mg, 77%).

MS (ESI, pos.ion) m/z: 531.6 $[M+H]^+$;

$^1H$ NMR (400 MHz, $CDCl_3$) δ (ppm): 8.39 (s, 1H), 7.84 (d, *J* = 8.1 Hz, 2H), 7.64 (s, 4H), 7.58 (d, *J* = 7.9 Hz, 2H), 7.44 (d, *J* = 7.9 Hz, 2H), 7.11 (d, *J* = 8.1 Hz, 2H), 4.19 (s, 2H), 3.82 (s, 2H), 3.64 - 3.53 (m, 2H), 3.53 - 3.33 (m, 2H), 3.22 (s, 2H), 3.09 (ddd, J = 25.8, 14.8, 7.4 Hz, 4H), 2.95 (t, *J*= 9.8 Hz, 1H), 2.64 (s, 2H), 2.36 (s, 1H), 2.06 (d, *J*= 8.6 Hz, 1H), 1.29 (d, *J*= 7.4 Hz, 3H).

**Example 4 *N*-(4-(ethylsulfonyl)benzyl)-4-(1-(4-(trifluoromethyl)benzyl)pyrrolidin -3-yl)benzamide**

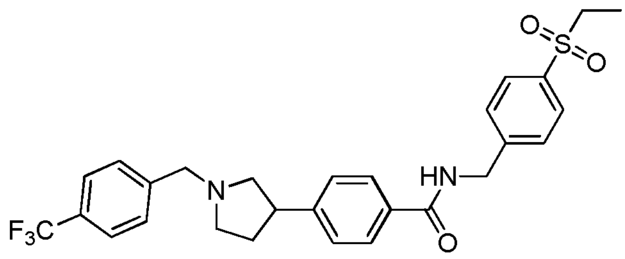

**Step 1: Synthesis of 4-bromo-*N*-(4-(ethylsulfonyl)benzyl)benzamide**

Under nitrogen protection, 4-bromobenzoic acid (1.03 g, 5.12 mmol), (4-(ethylsulfonyl)phenyl)methanamine (1.02 g, 5.12 mmol), EDCI (1.43 g, 7.46 mmol) and HOBT (1.00 g, 7.40 mmol) were dissolved in DCM (10 mL), then TEA (1.4 mL, 10 mmol) was added and the mixture was stirred at room temperature for 4 h. The reaction solution was diluted with DCM (80 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: DCM/EtOAc (v/v) = 10/1) to give a yellow solid (1.42 g, 72%).

MS (ESI, pos.ion) m/z: 382.1 $[M+H]^+$.

**Step 2: Synthesis of benzyl 4-(4-((4-(ethylsulfonyl)benzyl)carbamoyl) phenyl)-2,3-dihydro-1*H*-pyrrole-1-carboxylate**

Under nitrogen protection, 4-bromo-*N*-(4-(ethylsulfonyl)benzyl)benzamide (368 mg, 0.96 mmol), benzyl 2,5-dihydro-1*H*-pyrrole-1-carboxylate (1.72 mL, 9.58 mmol), $Pd(P(o\text{-}Tol)_3)_2(OAc)_2$ (50 mg, 0.05 mmol) and $Ag_2CO_3$ (265 mg, 0.96 mmol) were dissolved in DMF (5 mL), then DIPEA(0.5 mL, 2.82 mmol) was added. The mixture was heated and stirred in a 100°C oil bath for 24 h. The reaction solution was cooled to room temperature, diluted with EtOAc (100 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: DCM/EtOAc (v/v) = 10/1) to give yellow oily liquid (372 mg, 77%).

MS (ESI, pos.ion) m/z: 505.1 $[M+H]^+$.

**Step 3: Synthesis of *N*-(4-(ethylsulfonyl)benzyl)-4-(pyrrolidin-3-yl)benzamide**

To a solution of benzyl 4-(4-((4-(ethylsulfonyl)benzyl)carbamoyl)phenyl) -2,3-dihydro-1*H*-pyrrole-1-carboxylate (372 mg, 0.74 mmol) in MeOH (10 mL) was added Pd/C (265 mg, 10%) and the mixture was stirred at room temperature for 5 h under hydrogen protection. The reaction solution was filtered, dried over anhydrous $Na_2SO_4$, and concentrated under reduced pressure to give dark yellow oil (274 mg, 100%).

MS (ESI, pos.ion) m/z: 373.1 $[M+H]^+$.

**Step 4: Synthesis of *N*-(4-(ethylsulfonyl)benzyl)-4-(1-(4-(trifluoromethyl) benzyl)pyrrolidin-3-yl) benzamide**

4-(Trifluoromethyl)benzaldehyde (145 mg, 0.83 mmol) and *N*-(4-(ethylsulfonyl) benzyl)-4-(pyrrolidin-3-yl)benzamide (154 mg, 0.41 mmol) were dissolved in EtOH (3 mL) and THF (3 mL), then AcOH (0.02 mL, 0.40 mmol) and $NaBH_3CN$ (259 mg, 4.12 mmol) were added successively, and the mixture was stirred at room temperature for 12 h. Saturated $NaHCO_3$ solution (20 mL) was slowly added to the reaction solution to quench the reaction. The organic solvent was removed by concentration under reduced pressure. The residue was extracted with DCM (30 mL×3). The organic phases were combined and dried over anhydrous $Na_2SO_4$ and concentrated under reduced pressure. The crude product was separated by silica gel column chromatography (eluent: DCM/EtOAc (v/v) = 10/1) to give a yellow solid (18 mg, 8%).

MS (ESI, pos.ion) m/z: 531.3 $[M+H]^+$.

**Example 5 2-(4-(Ethylsulfonyl)phenyl)-*N*-(4-(4-methyl-1-(4-(trifluoromethyl)benzyl) pyrrolidin-3-yl)phenyl)acetamide**

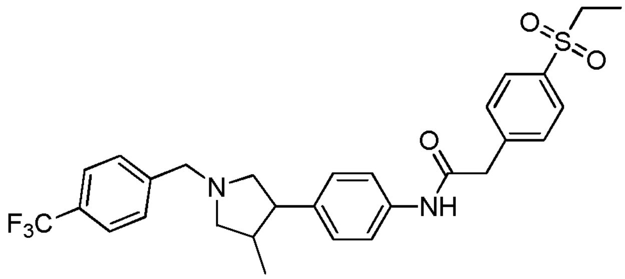

**Step 1: Synthesis of *tert-butyl* 3-(4-(2-(4-(ethylsulfonyl)phenyl)acetamido) phenyl)-4-methyl-2,3-dihydro-1*H*-pyrrole-1-carboxylate**

Under nitrogen protection, 2-(4-(ethylsulfonyl)phenyl)-*N*-(4-iodophenyl)acetamide (9.30 g, 21.68 mmol), tert-butyl 3-methyl-2,5-dihydropyrrole-1-carboxylate (3.01 g, 16.40 mmol), $Pd(P(o\text{-}Tol)_3)_2(OAc)_2$ (1.26 g, 1.34 mmol) and $Ag_2CO_3$ (7.50 g, 27.20 mmol) were dissolved in DMF (35 mL), then DIPEA (12.00 mL, 67.60 mmol) was added and the mixture was heated and reacted in a 100°C oil bath for 14 h. The reaction solution was cooled to room temperature, added with water (200 mL), and extracted with EtOAc (200 mL × 2). The organic phase was washed with water (150 mL×2), dried over anhydrous $Na_2SO_4$, concentrated under reduced pressure, and the crude product was separated by silica gel column chromatography (eluent: PE/EtOAc (v/v) = 1/1) to give grey oil (3.00 g, 38%).
MS (ESI, pos.ion) m/z: 485.1 $[M+H]^+$.

**Step 3: Synthesis of 2-(4-(ethylsulfonyl)phenyl)-*N*-(4-(4-methyl-2,3-dihydro-1*H*-pyrrol-3-yl)phenyl)acetamide**

*tert*-Butyl 3-(4-(2-(4-(ethylsulfonyl)phenyl)acetamido)phenyl)-4-methyl-2,3-dihydro -1*H*-pyrrole-1-carboxylate (3.00 g, 6.19 mmol)was dissolved in a solution of HCl in EtOAc (25 mL, 6 mol/L) and the mixture was stirred at room temperature for 5 h. The reaction solution was added with saturated aqueous $NaHCO_3$ (40 mL). The resulting mixture was concentrated under reduced pressure to remove the organic solvent. The remaining aqueous phase was extracted with EtOAc (50 mL × 2), dried over anhydrous $Na_2SO_4$, concentrated under reduced pressure, and the crude product was separated by silica gel column chromatography (eluent: DCM/MeOH (v/v) = 10/1) to give grey oil (217 mg, 9%).
MS (ESI, pos.ion) m/z: 385.3 $[M+H]^+$.

**Step 4: Synthesis of 2-(4-(ethylsulfonyl)phenyl)-*N*-(4-(4-methylpyrrolidin -3-yl)phenyl)acetamide**

To a solution of 2-(4-(ethylsulfonyl)phenyl)-*N*-(4-(4-methyl-2,3-dihydro -1*H*-pyrrol-3-yl)phenyl)acetamide (217 mg, 0.56 mmol) in a mixed solvent of THF/MeOH (5 mL/5 mL) was added Pd/C (612 mg, 10%) and the mixture was stirred at room temperature for 6 h under hydrogen protection. The reaction solution was filtered through a celite pad, and the filtrate was concentrated under reduced pressure to give a white solid (149 mg, 68%).
MS (ESI, pos.ion) m/z: 387.3 $[M+H]^+$.

**Step 5: Synthesis of 2-(4-(ethylsulfonyl)phenyl)-*N*-(4-(4-methyl-1 -(4-(trifluoromethyl)benzyl)pyrrolidin-3-yl)phenyl)acetamide**

to a solution of 2-(4-(ethylsulfonyl)phenyl)-*N*-(4-(4-methylpyrrolidin-3-yl)phenyl)acetamide (149 mg, 0.39 mmol) and 4-(trifluoromethyl)benzaldehyde (0.10 mL, 0.70 mmol) in EtOH/THF (3 mL/3 mL) was added AcOH (0.01 mL, 0.50 mmol). The mixture was stirred at room temperature for 1 h. Then $NaBH_3CN$ (318 mg, 5.06 mmol) was added. The resulting mixture was stirred at room temperature for 16 h. The reaction solution was added with saturated $Na_2CO_3$ solution (20 mL) to quench the reaction. The resulting mixture was concentrated under reduced pressure to remove the organic solvent, and the aqueous phase was extracted with EtOAc (40 mL×2), the organic phases were combined and dried over anhydrous $Na_2SO_4$, concentrated under reduced pressure, and the crude product was separated by silica gel column chromatography (eluent: PE/EtOAc (v/v) = 1/19) to give a white solid (83 mg, 40%).
MS (ESI, pos.ion) m/z: 545.1 $[M+H]^+$;
$^1H$ NMR (600 MHz, DMSO-$d_6$) $\delta$ (ppm): 10.21 (s, 1H), 7.84 (d, *J* = 8.0 Hz, 2H), 7.69 (d, *J* = 7.7 Hz, 2H), 7.59 (dd, *J* = 17.3, 7.9 Hz, 4H), 7.51 (d, *J* = 8.2 Hz, 2H), 7.21 (d, *J* = 8.2 Hz, 2H), 3.78 (s, 2H), 3.77 - 3.73 (m, 1H), 3.69 (d, *J* = 11.8 Hz, 1H), 3.28 - 3.24 (m, 2H), 2.97 - 2.84 (m, 2H), 2.74 (dd, *J* = 14.8, 7.3 Hz, 1H), 2.65 (s, 1H), 2.29 (s, 1H), 2.19 - 2.11 (m, 1H), 1.10 (t, *J* = 7.3 Hz, 3H), 0.97 (d, *J*= 6.6 Hz, 3H).

**Example 6 *N*-((*R*)-1-(4-(ethylsulfonyl)phenyl)-2-hydroxyethyl)-4-(3-(4-(trifluoromethyl) benzyl)pyrrolidin-1-yl)benzamide**

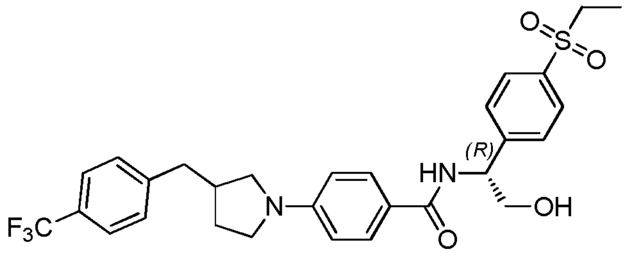

EDCI (96 mg, 0.50 mmol), HOBT (70 mg, 0.52 mmol), DIPEA (40 mg, 0.31 mmol), 4-(3-(4-(trifluoromethyl)benzyl)pyrrolidin-1-yl)benzoic acid (100 mg, 0.29 mmol), (*R*)-2-amino-2-(4-(ethyl)sulfonyl)phenyl)ethanol (66 mg, 0.29 mmol) were successively added to DCM (5 mL) and the mixture was stirred at room temperature for 4 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white powder (100 mg, 62%).

MS (ESI, pos.ion) m/z: 561.2 $[M+H]^+$;

$^1H$ NMR (400 MHz, $CDCl_3$) δ (ppm): 7.76 (d, *J* = 8.0 Hz, 2H), 7.69 (d, *J* = 8.6 Hz, 2H), 7.56 (d, *J* = 8.0 Hz, 2H), 7.49 (d, *J* = 8.1 Hz, 2H), 7.31 (d, *J* = 8.0 Hz, 2H), 7.19 (s, 1H), 6.43 (d, *J* = 8.6 Hz, 2H), 5.18 (s, 1H), 3.87 (d, *J*= 12.2 Hz, 2H), 3.46 - 3.36 (m, 2H), 3.33 - 3.27 (m, 1H), 3.04 (q, *J* = 7.4 Hz, 3H), 2.82 (d, *J* = 7.6 Hz, 2H), 2.65 - 2.57 (m, 1H), 2.15 - 2.11 (m, 1H), 2.05 - 1.92 (m, 1H), 1.80 - 1.71 (m, 1H), 1.22 (t, *J* = 7.4 Hz, 3H).

**Example 7 *N*-(4-(ethylsulfonyl)benzyl)-4-((2S)-2-(methoxymethyl)-4-(4-(trifluoromethyl) benzyl)pyrrolidin-1-yl)benzamide**

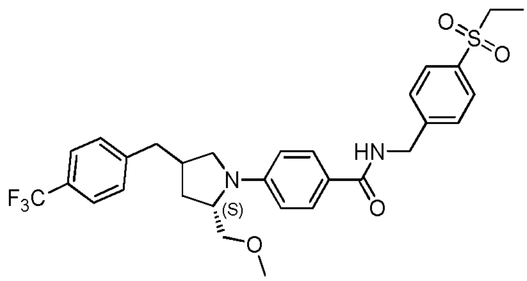

**Step 1: Synthesis of (*S*)-1-*tert*-butyl 2-methyl 4-methylenepyrrolidine-1,2-dicarboxylate**

EDCI (16.11 g, 84.04 mmol), DMAP (1.71 g, 14.00 mmol), (*S*)-1-(*tert*-butoxycarbonyl)-4-methylenepyrrolidine-2-carboxylic acid (10.00 g, 44.00 mmol) were dissolved in DCM/MeOH (100 mL/28 mL) and the mixture was reacted at 35°C for 29 h. The reaction solution was concentrated under reduced pressure, and the crude product was separated by silica gel column chromatography (eluent: PE/EtOAc (v/v) = 4/1) to give colorless liquid (10.21 g, 96%).

MS (ESI, pos.ion) m/z: 242.2 $[M+H]^+$.

**Step 2: Synthesis of (*S*)-1-*tert*-butyl 2-methyl 4-(4-(trifluoromethyl)benzyl) -2,3-dihydro-1*H*-pyrrole-1,2-dicarboxylate**

Under nitrogen protection, $Ag_2CO_3$ (6.09 g, 22.10 mmol), $Pd(P(o\text{-}Tol)_3)_2(OAc)_2$ (220 mg, 0.23 mmol), 1-bromo-4-(trifluoromethyl)benzene (5.00 g, 21.77 mmol), (*S*)-1-*tert*-butyl 2-methyl 4-methylenepyrrolidine-1,2-carboxylate (6.40 g, 26.50 mmol) and DIPEA (15 mL, 86.70 mmol) were dissolved in DMF (60 mL), and the mixture was heated and stirred in a 100°C oil bath for 21 h. The reaction solution was cooled to room temperature and concentrated under reduced pressure. The residue was diluted with EtOAc (200 mL), washed successively with $H_2O$ (30 mL) and saturated NaCl

solution (15 mL×3). The organic phase was dried over anhydrous $Na_2SO_4$, concentrated under reduced pressure, and the crude product was separated by silica gel column chromatography (eluent: PE/EtOAc (v/v) = 4/1) to give pale yellow oily liquid (7.5 g, 89%).

MS (ESI, pos.ion) m/z: 330.1 $[M-56+H]^+$.

**Step 3: Synthesis of (2*S*)-1-*tert*-butyl 2-methyl 4-(4-(trifluoromethyl)benzyl)pyrrolidine-1,2-dicarboxylate**

To a solution of (*S*)-1-*tert*-butyl 2-methyl 4-(4-(trifluoromethyl)benzyl)-2,3-dihydro-1*H*-pyrrole-1,2-dicarboxylate (7.50 g, 19.46 mmol) in MeOH (40 mL) was added Pd/C (1.00 g, 10%) and the mixture was stirred at room temperature for 14 h under hydrogen protection. The reaction solution was filtered, and the filtrate was concentrated under reduced pressure to give yellow transparent liquid (7.50 g, 99%).

MS (ESI, pos.ion) m/z: 332.0 $[M-56+H]^+$.

**Step 4: Synthesis of (2*S*)-*tert*-butyl 2-hydroxymethyl -4-(4-(trifluoromethyl)benzyl)pyrrolidine-1-carboxylate**

To a solution of (2*S*)-1-*tert*-butyl 2-methyl 4-(4-(trifluoromethyl)benzyl)pyrrolidine-1,2-dicarboxylate (7.00 g, 18.07 mmol) in THF (40 mL) was added a solution of $LiBH_4$ in THF (18 mL, 36.00 mmol, 2 mol/L) under an ice bath. After the addition was completed, the mixture was stirred at room temperature for 16 h. Saturated $NH_4Cl$ (30 mL) solution was slowly poured into the reaction solution to quench the reaction, then a large amount of gas was released. The mixture was left standing for layers. The upper organic phase was separated, and the aqueous phase was extracted with EtOAc (40 mL × 3). The combined organic phases were washed with saturated NaCl solution (50 mL), dried over anhydrous $Na_2SO_4$, and concentrated under reduced pressure. The crude product was separated by silica gel column chromatography (eluent: PE/EtOAc (v/v) = 5/1) to give pale yellow oily liquid (5.51 g, 85%).

MS (ESI, pos.ion) m/z: 304.1 $[M-56+H]^+$.

**Step 5: Synthesis of (2*S*)-*tert*-butyl 2-methoxymethyl -4-(4-(trifluoromethyl)benzyl)pyrrolidine-1-carboxylate**

To a solution of (2*S*)-*tert*-butyl 2-(hydroxymethyl)-4-(4-(trifluoromethyl)benzyl)pyrrolidine-1-carboxylate (1.00 g , 2.80 mmol) in NaOH (220 mg, 5.50 mmol) and DMF (10 mL) was added iodomethane (0.85 mL, 14.00 mmol). The mixture was stirred at room temperature for 22 h. The reaction solution was diluted with EtOAc (80 mL), washed successively with $H_2O$ (20 mL) and saturated NaCl solution (20 mL×3), dried over anhydrous $Na_2SO_4$, and concentrated under reduced pressure. The crude product was separated by silica gel column chromatography (eluent: PE/EtOAc (v/v) = 5/1) to give colorless liquid (900 mg, 87%).

MS (ESI, pos.ion) m/z: 318.1 $[M+H]^+$.

**Step 6: Synthesis of (2*S*)-2-methoxymethyl -4-(4-(trifluoromethyl)benzyl)pyrrolidine**

To a solution of DCM/TFA (8 mL/3 mL) was added (2*S*)-*tert*-butyl 2-(methoxymethyl)-4-(4-(trifluoromethyl)benzyl)pyrrolidine-1-carboxylate (900 mg, 2.41 mmol). The mixture was stirred at room temperature for 24 h. The reaction solution was concentrated under reduced pressure. NaOH solution (4 mol/L) was slowly added to the residue to adjust the pH of the system to about 9. The resulting mixture was extracted with DCM (20 mL×3), dried over anhydrous $Na_2SO_4$, and concentrated under reduced pressure. The crude product was separated by silica gel column chromatography (eluent: DCM/MeOH (v/v) = 10/1) to give a yellow solid (200 mg, 30%).

MS (ESI, pos.ion) m/z: 274.1 $[M+H]^+$.

**Step 7: Synthesis of methyl 4-((2*S*)-2-(methoxymethyl)-4-(4-(trifluoromethyl) benzyl)pyrrolidin-1-yl)benzoate**

Under nitrogen protection, (2*S*)-2-(methoxymethyl)-4-(4-(trifluoromethyl) benzyl)pyrrolidine (200 mg, 0.73 mmol), methyl 4-bromobenzoate (320 mg, 1.49 mmol), $Pd_2(dba)_3$ (66 mg, 0.07 mmol), XantPhos (86 mg, 0.15 mmol) and $Cs_2CO_3$ (723 mg, 2.22 mmol) were dissolved in 1,4-dioxane (8 mL) and the mixture was heated and stirred in a 100°C oil bath for 12 h. The reaction solution was cooled to room temperature, then concentrated under reduced pressure. The residue was diluted with DCM (50 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 3/1) to give a yellow solid (120 mg, 40%).

MS (ESI, pos.ion) m/z: 408.2 $[M+H]^+$.

**Step 8: Synthesis of 4-((2*S*)-2-(methoxymethyl)-4-(4-(trifluoromethyl) benzyl)pyrrolidin-1-yl)benzoic acid**

To a solution of methyl 4-((2*S*)-2-(methoxymethyl)-4-(4-(trifluoromethyl)benzyl)pyrrolidin-1-yl)benzoate (120 mg, 0.29 mmol) in MeOH (2 mL) and THF (4 mL) was added a solution of LiOH·$H_2O$ (250 mg, 5.96 mmol) in $H_2O$ (2 mL). The mixture was heated and stirred in a 70°C oil bath for 18 h. The reaction solution was cooled to room temperature. HCl solution (1 mol/L) was added to the reaction solution to adjust the pH of the solution to about 5. The resulting mixture was concentrated under reduced pressure. The residue was extracted with EtOAc (20 mL×2), then the organic phases were combined and dried over anhydrous $Na_2SO_4$, concentrated under reduced pressure to give a white solid (100 mg, 86%).

MS (ESI, pos.ion) m/z: 394.3 $[M+H]^+$.

**Step 9: Synthesis of *N*-(4-(ethylsulfonyl)benzyl)-4-((2*S*)-2-(methoxymethyl) -4-(4-(trifluoromethyl)benzyl)pyrrolidin-1-yl)benzamide**

HATU (119 mg, 0.31 mmol), TEA (80 mg, 0.79 mmol), 4-((2*S*)-2-(methoxymethyl)-4-(4-(trifluoromethyl)benzyl)pyrrolidin-1-yl)benzoic acid (80 mg, 0.20 mmol) and (4-(ethylsulfonyl)phenyl)methanamine (62 mg, 0.31 mmol) were dissolved in DCM (6 mL) and the mixture was stirred at room temperature for 8 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 4/1) to give a white solid (55 mg, 47%).

MS (ESI, pos.ion) m/z: 575.1 $[M+H]^+$.

$^1H$ NMR (400 MHz, $CDCl_3$) δ (ppm): 7.78 (d, *J* = 8.2 Hz, 2H), 7.74 (dd, *J* = 8.6, 6.2 Hz, 2H), 7.59 (dd, *J* = 7.5, 4.5 Hz, 2H), 7.48 (d, *J* = 7.9 Hz, 2H), 7.34 (dd, *J* = 7.5, 4.5 Hz, 2H), 6.82 (d, *J* = 3.7 Hz, 1H), 6.60 (dd, *J* = 8.6, 6.2 Hz, 2H), 4.70 (d, *J* = 5.6 Hz, 2H), 4.08 - 3.96 (m, 1H), 3.64 (dd, *J* = 9.4, 2.9 Hz, 1H), 3.53 - 3.46 (m, 1H), 3.40 (dd, *J* = 9.4, 6.7 Hz, 1H), 3.35 (d, *J* = 4.8 Hz, 2H), 3.29 - 3.16 (m, 1H), 3.09 (q, *J* = 7.4 Hz, 2H), 2.93 - 2.84 (m, 2H), 2.56 - 2.48 (m, 1H), 2.43 - 2.33 (m, 1H), 1.82 (dd, *J* = 11.2, 8.0 Hz, 2H), 1.26 (t, *J* = 7.4 Hz, 3H).

**Example 8 *N*-((R)-1-(4-(ethylsulfonyl)phenyl)-2-hydroxyethyl)-4-((2*S*)-2-(methoxymethyl)-4-(4-(trifluoromethyl)benzyl)pyrrolidin-1-yl)benzamide**

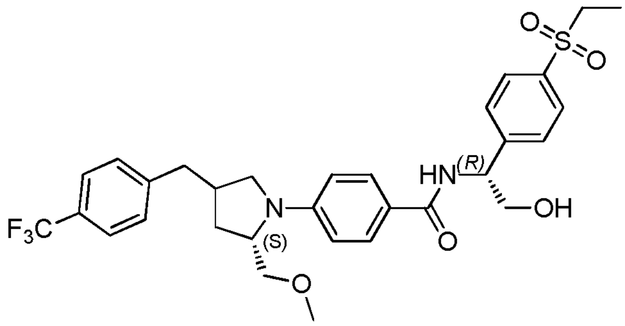

EDCI (75 mg, 0.39 mmol), HOBT (56 mg, 0.39 mmol), 4-((2*S*)-2-(methoxymethyl)-4-(4-(trifluoromethyl)benzyl)pyrrolidin-1-yl)benzoic acid (100 mg, 0.25 mmol) and (*R*)-2-amino-2-(4-(ethylsulfonyl)phenyl)ethanol (90 mg, 0.39 mmol) were dissolved in DCM (6 mL) and the mixture was stirred at room temperature for 14 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 4/1) to give a white solid (101 mg, 66%).

MS (ESI, pos.ion) m/z: 605.3 $[M+H]^+$.

$^1H$ NMR (400 MHz, $CDCl_3$) δ (ppm): 7.82 (d, *J* = 8.0 Hz, 2H), 7.70 (dd, *J* = 8.6, 6.3 Hz, 2H), 7.57 (dd, *J* = 7.8, 4.6 Hz, 2H), 7.53 (d, *J* = 7.2 Hz, 2H), 7.32 (dd, *J* = 7.8, 4.6 Hz, 2H), 7.03 (dd, *J* = 6.8, 3.2 Hz, 1H), 6.58 (dd, *J* = 8.7, 5.7 Hz, 2H), 5.22 (d, *J* = 4.6 Hz, 1H), 4.04 - 3.94 (m, 2H), 3.91 (dd, *J* = 11.3, 4.9 Hz, 1H), 3.61 (dd, *J* = 9.5, 3.1 Hz, 1H), 3.53 - 3.46 (m, 1H), 3.39 (dd, *J* = 9.5, 6.5 Hz, 1H), 3.33 (d, *J* = 4.4 Hz, 3H), 3.27 - 3.14 (m, 1H), 3.07 (q, *J* = 7.4 Hz, 2H), 2.88 - 2.80 (m, 2H), 2.54 - 2.36 (m, 1H), 2.39 - 2.31 (m, 1H), 2.22 - 2.10 (m, 1H), 1.82 - 1.75 (m, 2H), 1.25 (t, *J* = 7.4 Hz, 3H).

**Example 9 *N*-(4-(ethylsulfonyl)benzyl)-4-(3-(4-(trifluoromethyl)phenoxy)pyrrolidin -1-yl)benzamide**

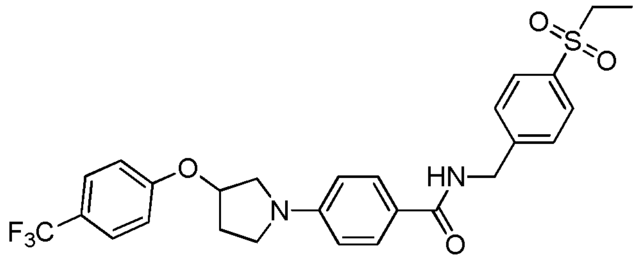

**Step 1: Synthesis of benzyl 3-hydroxypyrrolidine-1-carboxylate**

In an ice-water bath, to a solution of benzyl 3-oxopyrrolidine-1-carboxylate (3.34 g, 15.20 mmol) in MeOH (30 mL) was added $NaBH_4$ (1.00 g, 26.00 mmol), then the mixture was warmed to room temperature and stirred for 12 h. After the reaction, saturated $NaHCO_3$ solution (30 mL) was added to the reaction solution to quench the reaction. MeOH was removed by concentration under reduced pressure. The aqueous phase was extracted with DCM (30 mL×3), and the organic phases were combined and dried over anhydrous $Na_2SO_4$, and concentrated under reduced pressure. The crude product was separated by silica gel column chromatography (eluent: PE/DCM/EtOAc (v/v/v) = 2/1/1) to give yellow liquid (3.30 g, 98%).

MS (ESI, pos.ion) m/z: 222.1 $[M+H]^+$.

**Step 2: Synthesis of benzyl 3-(4-(trifluoromethyl)phenoxy)pyrrolidine-1-carboxylate**

Under nitrogen protection, benzyl 3-hydroxypyrrolidine-1-carboxylate (1.03 g, 4.66 mmol), 1-bromo-4-(trifluoromethyl)benzene (1.53 g, 6.80 mmol), $Cs_2CO_3$ (2.98 g , 9.15 mmol), $Pd(OAc)_2$ (54 mg, 0.24 mmol) and t-Bu-XPhos (199 mg, 0.47 mmol) were dissolved in toluene (20 mL) and the mixture was stirred in a 90°C oil bath for 24 h. The reaction solution was cooled to room temperature, diluted with DCM (80 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/DCM/EtOAc (v/v/v) = 5/1/1) to give yellow oily liquid (688 mg, 40%).

MS (ESI, pos.ion) m/z: 366.1 $[M+H]^+$.

**Step 3: Synthesis of 3-(4-(trifluoromethyl)phenoxy)pyrrolidine**

To a solution of benzyl 3-(4-(trifluoromethyl)phenoxy)pyrrolidine-1-carboxylate (688 mg, 1.88 mmol) in MeOH (10 mL) was added Pd/C (70 mg, 10 %). The mixture was stirred at room temperature for 12 h under hydrogen protection. The reaction solution was filtered, and the filtrate was concentrated under reduced pressure to give tan liquid (429 mg, 98%).

MS (ESI, pos.ion) m/z: 232.1 $[M+H]^+$.

**Step 4: Synthesis of *N*-(4-(ethylsulfonyl)benzyl)-4-(3-(4-(trifluoromethyl) phenoxy)pyrrolidin-1-yl) benzamide**

Under nitrogen protection, 3-(4-(trifluoromethyl)phenoxy)pyrrolidine (123 mg, 0.53 mmol), 4-bromo-*N*-(4-(ethylsulfonyl)benzyl)benzamide (302 mg, 0.79 mmol), $Pd_2(dba)_3$ (93 mg, 0.10 mmol), XantPhos (68 mg, 12 mmol) and *t*-BuONa (100 mg, 1.04 mmol) were dissolved in toluene (5 mL), and the mixture was heated and stirred in a 110°C oil bath for 23 h. The reaction solution was cooled to room temperature, and concentrated under reduced pressure. The residue was diluted with DCM (60 mL), washed successively with $NaHCO_3$ solution (10 mL) and saturated NaCl solution (10 mL), dried over anhydrous $Na_2SO_4$, and concentrated under reduced pressure. The crude product was separated by silica gel column chromatography (eluent: DCM/EtOAc (v/v) = 5/1) to give a yellow solid (120 mg, 42%).

MS (ESI, pos.ion) m/z: 533.1 $[M+H]^+$;

$^1H$ NMR (400 MHz, $CDCl_3$) δ (ppm): 7.85 (d, *J* = 8.2 Hz, 2H), 7.74 (d, *J* = 8.7 Hz, 2H), 7.56 (d, *J*= 8.6 Hz, 2H), 7.53 (d, *J* = 8.1 Hz, 2H), 6.96 (d, *J*= 8.6 Hz, 2H), 6.57 (d, *J*= 8.7 Hz, 2H), 6.46 (t, *J*= 5.8 Hz, 1H), 5.14 (m, 1H), 4.73 (d, *J*= 6.0 Hz, 2H), 3.76 (dd, *J*= 11.2, 4.6 Hz, 1H), 3.56 (dd, *J*= 11.7, 8.2 Hz, 3H), 3.09 (q, *J*= 7.4 Hz, 2H), 2.44 - 2.30 (m, 2H), 1.27 (t, *J*= 7.4 Hz, 3H).

**Example 10 *N*-(4-(ethylsulfonyl)benzyl)-4-(3-(methyl(4-(trifluoromethyl)phenyl) amino)pyrrolidin-1-yl) benzamide**

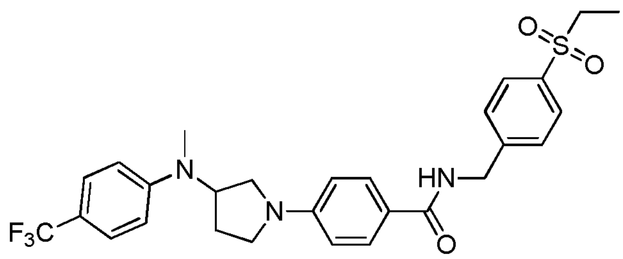

**Step 1: Synthesis of benzyl 3-((4-(trifluoromethyl)phenyl) amino)pyrrolidine-1-carboxylate**

4-(Trifluoromethyl)aniline (2.05 g, 12.73 mmol), benzyl 3-oxopyrrolidine-1-carboxylate (3.32 g, 15.15 mmol) and AcOH (1.40 mL, 24.50 mmol) were dissolved in EtOH (30 mL). The mixture was stirred at room temperature for 2 h, then $NaBH_3CN$ (3.90 g, 62.06 mmol) was added, and the mixture was stirred at room temperature for 12 h. Saturated $NaHCO_3$ solution (30 mL) was added to the reaction solution to quench the reaction. The mixture was concentrated under reduced pressure. The aqueous phase was extracted with DCM (40 mL×3), and the organic phase was washed with saturated NaCl solution (20 mL) and dried over anhydrous $Na_2SO_4$, and concentrated under reduced pressure. The crude product was separated by silica gel column chromatography (eluent: PE/DCM/EtOAc (v/v/v) = 5/1/1) to give a yellow solid (1.11 g, 24%).

MS (ESI, pos.ion) m/z: 365.3 $[M+H]^+$.

**Step 2: Synthesis of benzyl 3-(methyl(4-(trifluoromethyl)phenyl)amino) pyrrolidine-1-carboxylate**

To a suspension of benzyl 3-((4-(trifluoromethyl)phenyl)amino)pyrrolidine -1-carboxylate (504 mg, 1.38 mmol) and NaOH (75 mg, 1.88 mmol) in ACN (8 mL) was added dropwise MeI (0.30 mL, 4.80 mmol), and the mixture was stirred at room temperature for 12 h. The reaction solution was diluted with DCM (60 mL), washed successively with $NaHCO_3$ solution (20 mL) and NaCl solution (20 mL). The aqueous phase was extracted with DCM (20 mL×2). The organic phases were combined, dried over anhydrous $Na_2SO_4$, and concentrated under reduced pressure. The crude product was separated by silica gel column chromatography (eluent: PE/DCM/EtOAc (v/v/v) = 10/1/1) to give yellow transparent liquid (391 mg, 75%).

MS (ESI, pos.ion) m/z: 379.2 $[M+H]^+$.

**Step 3: Synthesis of *N*-methyl-*N*-(4-(trifluoromethyl)phenyl)pyrrolidin-3-amine**

To a solution of benzyl 3-(methyl(4-(trifluoromethyl)phenyl) amino)pyrrolidine-1-carboxylate (198 mg, 0.52 mmol) in MeOH (8 mL) was added Pd/C (50 mg, 10 %). The mixture was stirred at room temperature for 12 h under hydrogen protection. After the reaction, the reaction solution was filtered, and the filtrate was concentrated under reduced pressure to give yellow oil (128 mg, 100%).

MS (ESI, pos.ion) m/z: 245.2 $[M+H]^+$.

**Step 4: Synthesis of *N*-(4-(ethylsulfonyl)benzyl)-4-(3-(methyl(4-(trifluoromethyl) phenyl)amino)pyrrolidin-1-yl)benzamide**

Under nitrogen protection, *N*-methyl-*N*-(4-(trifluoromethyl)phenyl)pyrrolidin-3-amine (126 mg, 0.52 mmol), *N*-(4-(ethylsulfonyl)benzyl)-4-iodobenzamide (342 mg, 0.80 mmol) (refer to Step 1 in Example 4 for the synthesis method), $Pd_2(dba)_3$ (97 mg, 0.11 mmol), XantPhos (70 mg, 0.12 mmol) and *t*-BuONa (100 mg, 1.04 mmol) were dissolved in toluene (6 mL), and the mixture was heated and stirred in a 110°C oil bath for 14 h. The reaction solution was cooled to room temperature, and concentrated under reduced pressure. The residue was diluted with DCM (80 mL), washed successively with $NaHCO_3$ solution (10 mL) and saturated NaCl solution (10 mL), dried over anhydrous $Na_2SO_4$, and concentrated under reduced pressure. The crude product was separated twice by silica gel column chromatography (eluent: DCM/EtOAc (v/v) = 10/1) to give a yellow solid (141 mg, 50%).

MS (ESI, pos.ion) m/z: 546.2 $[M+H]^+$;

$^1$H NMR (400 MHz, $CDCl_3$) δ (ppm): 7.84 (d, *J* = 8.0 Hz, 2H), 7.75 (d, *J* = 8.5 Hz, 2H), 7.52 (d, *J* = 8.0 Hz, 2H), 7.48 (d, *J* = 8.7 Hz, 2H), 6.85 (d, *J* = 8.7 Hz, 2H), 6.58 (d, *J* = 8.6 Hz, 2H), 6.52 (t, *J*= 5.7 Hz, 1H), 4.74 (t, *J* = 7.0 Hz, 2H), 4.70 - 4.62 (m, 1H), 3.67 - 3.53 (m, 2H), 3.42 (dt, *J* = 10.5, 6.9 Hz, 2H), 3.09 (q, *J*= 7.4 Hz, 2H), 2.91 (s, 3H), 2.27 (ddd, *J*= 18.0, 13.6, 6.4 Hz, 2H), 1.27 (t, *J* = 7.2 Hz, 3H).

**Example 11 *N*-(4-(ethylsulfonyl)benzyl)-4-(3-((4-(trifluoromethyl)phenyl)amino) pyrrolidin-1-yl)benzamide**

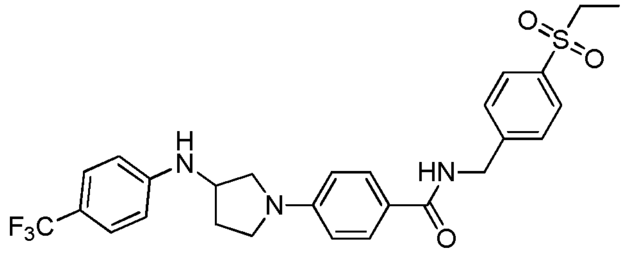

**Step 1: Synthesis of benzyl 3-((tert-butoxycarbonyl)(4-(trifluoromethyl)phenyl)amino) pyrrolidine-1-carboxylate**

To a solution of benzyl 3-((4-(trifluoromethyl)phenyl)amino)pyrrolidine-1-carboxylate (714 mg, 1.96 mmol) and DMAP (470 mg, 3.85 mmol) in DCM (6 mL) was added $Boc_2O$ (0.90 mL, 3.90 mmol) slowly, and the mixture was stirred at room temperature for 5 h. Then the mixture was heated under reflux and reacted for 20 h. The reaction solution was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/DCM/EtOAc (v/v/v) = 5/1/1) to give colorless liquid (539 mg, 59%).

MS (ESI, pos.ion) m/z: 487.2 $[M+23]^+$.

**Step 2: Synthesis of tert-butyl pyrrolidin-3-yl(4-(trifluoromethyl)phenyl)carbamate**

To a solution of benzyl 3-((*tert*-butoxycarbonyl)(4-(trifluoromethyl)phenyl) amino)pyrrolidine-1-carboxylate (485 mg, 1.33 mmol) in MeOH (8 mL) was added Pd/C (50 mg, 10 %). The mixture was stirred at room temperature for 12 h under hydrogen protection. The reaction solution was filtered, and the filtrate was concentrated under reduced pressure to give yellow oil (300 mg, 100%).

MS (ESI, pos.ion) m/z: 331.1 $[M+H]^+$.

**Step 3: Synthesis of *tert*-butyl (1-(4-((4-(ethylsulfonyl)benzyl)carbamoyl)phenyl) pyrrolidin-3-yl)(4-(trifluoromethyl)phenyl)carbamate**

Under nitrogen protection, *tert-butyl* pyrrolidin-3-yl(4-(trifluoromethyl)phenyl)carbamate (132 mg, 0.45 mmol), *N*-(4-(ethylsulfonyl)benzyl)-4-iodobenzamide (642 mg, 1.55 mmol), $Pd_2(dba)_3$ (93 mg, 0.10 mmol), XantPhos (68 mg, 0.12 mmol) and *t*-BuONa (100 mg, 1.04 mmol) were dissolved in toluene (4 mL), and the mixture was heated and stirred in a 100°C oil bath for 24 h. The reaction solution was cooled to room temperature, and concentrated under reduced pressure. The residue was diluted with DCM (60 mL), washed successively with saturated $NaHCO_3$ solution (10 mL) and saturated NaCl solution (10 mL), dried over anhydrous $Na_2SO_4$, and concentrated under reduced pressure to give yellow oil (277 mg, 97%).

MS (ESI, pos.ion) m/z: 632.2 $[M+H]^+$.

**Step 4: Synthesis of *N*-(4-(ethylsulfonyl)benzyl)-4-(3-((4-(trifluoromethyl) phenyl)amino)pyrrolidin-1-yl)benzamide**

*tert*-Butyl (1-(4-((4-(ethylsulfonyl)benzyl)carbamoyl)phenyl)pyrrolidin-3-yl) (4-(trifluoromethyl)phenyl)carbamate (70 mg, 0.11 mmol) was dissolved in a solution of HCl in *i*-PrOH (20 mL, 91.60 mmol, 4.58 mol/L ) and the mixture was stirred at room temperature for 4 h. The reaction solution was concentrated under reduced pressure. The residue was diluted with DCM (60 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 2/1) to give a yellow solid (55 mg, 94%).

MS (ESI, pos.ion) m/z: 532.2 $[M+H]^+$;

$^1H$ NMR (400 MHz, $CDCl_3$) $\delta$ (ppm): 7.84 (d, *J* = 8.2 Hz, 2H), 7.73 (d, *J* = 8.7 Hz, 2H), 7.52 (d, *J* = 8.2 Hz, 2H), 7.43 (d, *J* = 8.5 Hz, 2H), 6.65 (d, *J* = 8.5 Hz, 2H), 6.56 (d, *J*= 8.7 Hz, 2H), 6.49 (t, *J*= 6.0 Hz, 1H), 4.73 (d, *J*= 6.1 Hz, 2H), 4.28 (m, 1H), 4.18 (br, 1H), 3.75 (dd, *J*= 10.1, 5.7 Hz, 1H), 3.54 (m, 1H), 3.51 - 3.42 (m, 1H), 3.30 (dd, *J* = 10.3, 3.3 Hz, 1H), 3.09 (q, *J* = 7.4 Hz, 2H), 2.45 - 2.36 (m, 1H), 2.16 - 2.05 (m, 1H), 1.27 (t, J= 7.4 Hz, 3H).

**Example 12 *N*-((R)-1-(4-(ethylsulfonyl)phenyl)-2-hydroxyethyl)-4-(3-(4-(trifluoromethyl)phenoxy) pyrrolidin-1-yl)benzamide**

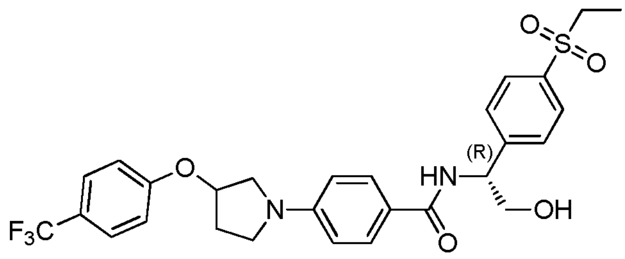

**Step 1: Synthesis of 4-(3-(4-(trifluoromethyl)phenoxy)pyrrolidin-1-yl)benzonitrile**

Under nitrogen protection, to a solution of 3-(4-(trifluoromethyl)phenoxy)pyrrolidine (478 mg, 2.07 mmol) and 4-fluorobenzonitrile (750 mg, 6.19 mmol) in DMSO (10 mL) was added $K_2CO_3$ (1.50 g, 10.90 mmol). The mixture was heated and stirred in an oil bath at 120°C for 24 h. The reaction solution was cooled to room temperature, diluted with EtOAc (80 mL), washed with saturated NaCl solution (10 mL×3), dried over anhydrous $Na_2SO_4$, and concentrated under reduced pressure. The crude product was separated by silica gel column chromatography (eluent: PE/DCM/EtOAc (v/v/v) = 5/1/1) to give a white solid (512 mg, 75%).

MS (ESI, pos.ion) m/z: 333.1 $[M+H]^+$.

**Step 2: Synthesis of 4-(3-(4-(trifluoromethyl)phenoxy)pyrrolidin-1-yl)benzoic acid**

To a solution of 4-(3-(4-(trifluoromethyl)phenoxy)pyrrolidin-1-yl)benzonitrile (512 mg, 1.54 mmol) in EtOH (15 mL) was added MeONa (2.87 g, 52.52 mmol). The mixture was heated and stirred in an oil bath at 100°C for 10 h. The reaction solution was cooled to room temperature, and concentrated under reduced pressure. The residue was added with $H_2O$ (100 mL) to dissolve all solids. The aqueous phase was extracted with DCM (30 mL×3). The organic phases were combined, dried over anhydrous $Na_2SO_4$, and concentrated under reduced pressure to give the by-product amide as a yellow solid. The aqueous phase was neutralized to pH 3 with HCl solution (1 mol/L), then the aqueous phase was extracted with EtOAc (30 mL×3). The organic phases were combined, dried over anhydrous $Na_2SO_4$, and concentrated under reduced pressure to obtain a yellow solid (329 mg, 61%).

MS (ESI, pos.ion) m/z: 352.1 $[M+H]^+$.

**Step 3: Synthesis of *N*-((R)-1-(4-(ethylsulfonyl)phenyl)-2-hydroxyethyl) -4-(3-(4-(trifluoromethyl)phenoxy)pyrrolidin-1-yl)benzamide**

4-(3-(4-(Trifluoromethyl)phenoxy)pyrrolidin-1-yl)benzoic acid (123 mg, 0.35 mmol), (*R*)-2-amino-2-(4-(ethylsulfonyl)phenyl)ethanol (94 mg, 0.41 mmol), EDCI (134 mg, 0.70 mmol) and HOBT (94 mg, 0.70 mmol) were dissolved in DCM (4 mL), then TEA (0.14 mL, 1.00 mmol) was added and the mixture was stirred at room temperature for 20 h. The reaction solution was diluted with DCM (80 mL), washed successively with HCl solution (20 mL, 1 mol/L), saturated NaCl solution (20 mL) and saturated $Na_2CO_3$ solution (20 mL), dried over anhydrous $Na_2SO_4$, and concentrated under reduced pressure. The crude product was separated by silica gel column chromatography (eluent: DCM/EtOAc (v/v) = 1/1) to give a pale yellow solid (122 mg, 62%).

MS (ESI, pos.ion) m/z: 563.1 $[M+H]^+$;

$^1H$ NMR (400 MHz, $CDCl_3$) $\delta$ (ppm): 7.86 (d, *J* = 8.3 Hz, 2H), 7.74 (d, *J* = 8.8 Hz, 2H), 7.57 (d, *J*= 8.1 Hz, 4H), 6.96 (d, *J*= 8.6 Hz, 2H), 6.90 (d, *J*= 6.7 Hz, 1H), 6.56 (d, *J*= 8.8 Hz, 2H), 5.28 (dd, *J* = 10.9, 4.3 Hz, 1H), 5.14 (s, 1H), 4.09 - 3.94 (m, 2H), 3.76 (dd, *J* = 11.2, 4.7 Hz, 1H), 3.63 - 3.50 (m, 3H), 3.09 (q, *J* = 7.4 Hz, 2H), 2.49 - 2.28 (m, 3H), 1.28 (t, *J* = 7.4 Hz, 3H).

**Example 13 *N*-((*R*)-1-(4-(ethylsulfonyl)phenyl)-2-hydroxyethyl)-4-((*R*)-3-(4-(trifluoromethyl)phenoxy)pyrrolidin-1-yl)benzamide**

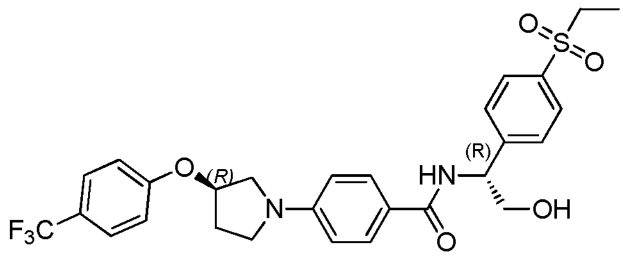

**Step 1: Synthesis of *tert*-butyl (*R*)-3-(4-(trifluoromethyl) phenoxy)pyrrolidine-1-carboxylate**

*tert*-Butyl (*S*)-3hydroxypyrrolidine-1-carboxylate (1.00 g, 5.34 mmol), 4-(trifluoromethyl)phenol (865 mg, 5.34 mmol), $PPh_3$ (2.00 g, 5.83 mmol) were added to THF (20 mL). The mixture was transferred to 0 °C, and DIAD (1.40 mL, 7.11 mmol) was slowly added. After the addition was complete, the mixture was transferred to room temperature and reacted for 21 h. The reaction solution was concentrated under reduced pressure. The residue was diluted with methyl tert-butyl ether (30 mL) and stirred at -20 °C. A large amount of white insoluble solids were precipitated, filtered while cold, and the filter cake was washed with cold methyl tert-butyl ether. The filtrate was concentrated under reduced pressure, and the crude product was separated by silica gel column chromatography (eluent: PE/EtOAc (v/v) = 6/1) to give the product as pale yellow liquid (1.76 g, 100%).
MS (ESI, pos.ion) m/z: 276.2 $[M-56+H]^+$.

**Step 2: Synthesis of (*R*)-3-(4-(trifluoromethyl)phenoxy)pyrrolidine**

To a solution of *tert-butyl* (*R*)-3-(4-(trifluoromethyl)phenoxy)pyrrolidine-1-carboxylate (800 mg, 2.42 mmol) in DCM (8 mL) was added a solution of HCl in methanol (2 mL, 20%). The mixture was stirred at room temperature for 9 h. After the reaction was completed, saturated sodium bicarbonate was added for washing, and the reaction solution was concentrated under reduced pressure to obtain light red liquid (558 mg, 100%).
MS (ESI, pos.ion) m/z: 232.2 $[M+H]^+$.

**Step 3: Synthesis of methyl (*R*)-4-(3-(4-(trifluoromethyl)phenoxy) pyrrolidin-1-yl)benzoate**

Under nitrogen protection, (*R*)-3-(4-(trifluoromethyl)phenoxy)pyrrolidine (600 mg, 2.60 mmol), $Pd_2(dba)_3$ (237 mg, 0.26 mmol), Xantphos (225 mg, 0.39 mmol), $Cs_2CO_3$ (1.70 g, 5.22 mmol), methyl 4-iodobenzoate (840 mg, 3.91 mmol) were successively added to 1,4-dioxane (16 mL) and the mixture was reacted at 100 °C for 19 h. The reaction solution was cooled to room temperature, and concentrated under reduced pressure. The residue was diluted with DCM (80 mL), washed successively with $NaHCO_3$ solution (30 mL) and saturated NaCl solution (30 mL), dried over anhydrous $Na_2SO_4$, and concentrated under reduced pressure. The crude product was separated by silica gel column chromatography (eluent: PE/EtOAc (v/v) = 5/1) to give a yellow solid (800 mg, 84%).
MS (ESI, pos.ion) m/z: 366.2 $[M+H]^+$.

**Step 4: Synthesis of (*R*)-4-(3-(4-(trifluoromethyl)phenoxy)pyrrolidin-1-yl)benzoic acid**

To a solution of methyl (*R*)-4-(3-(4-(trifluoromethyl)phenoxy)pyrrolidin-1-yl)benzoate (300 mg, 0.82 mmol) in MeOH (7 mL) and THF (3 mL) was added a solution of $LiOH \cdot H_2O$ (88 mg, 2.10 mmol) in $H_2O$ (2 mL). The mixture was reacted at room temperature for 7 h. The reaction solution was concentrated under reduced pressure. The remaining solution was added with HCl solution (1 mol/L) to adjust the pH of the solution to about 4. The resulting mixture was extracted with EtOAc (20 mL×2). The organic phases were combined, washed with saturated NaCl (25 mL) solution, dried over anhydrous $Na_2SO_4$, and concentrated under reduced pressure. The crude product was separated by silica gel column chromatography (eluent: DCM/EtOAc (v/v) = 4/1) to give a white solid (251 mg, 87%).
MS (ESI, pos.ion) m/z: 352.1 $[M+H]^+$.

**Step 5: Synthesis of *N*-((R)-1-(4-(ethylsulfonyl)phenyl)-2-hydroxyethyl)-4-((*R*)-3-(4-(trifluoromethyl)phenoxy)pyrrolidin-1-yl)benzamide**

EDCI (81 mg, 0.42 mmol), HOBT (57 mg, 0.42 mmol), (*R*)-2-amino-2-(4-(ethylsulfonyl)phenyl)ethanol (78 mg, 0.34 mmol), (*R*)-4-(3-(4-(trifluoromethyl)phenoxy)pyrrolidin-1-yl)benzoic acid (100 mg , 0.28 mmol) and TEA (43 mg, 0.42 mmol) were successively added to DCM (6 mL) and the mixture was reacted at room temperature for 16 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (133 mg, 83%).

MS (ESI, pos.ion) m/z: 563.1 $[M+H]^+$.

$^1H$ NMR (400 MHz, $CDCl_3$) δ (ppm): 7.85 (d, *J* = 8.3 Hz, 2H), 7.74 (d, *J* = 8.8 Hz, 2H), 7.56 (d, *J* = 8.5 Hz, 2H), 7.55 (d, *J* = 8.5 Hz, 2H), 6.99 - 6.92 (m, 3H), 6.55 (d, *J* = 8.8 Hz, 2H), 5.26 (dd, *J* = 10.8, 4.3 Hz, 1H), 5.13 (s, 1H), 4.05 - 3.93 (m, 2H), 3.76 (dd, *J* = 11.2, 4.7 Hz, 1H), 3.60 - 3.50 (m, 3H), 3.08 (q, *J* = 7.4 Hz, 2H), 2.62 (t, *J* = 5.7 Hz, 1H), 2.41 - 2.28 (m, 2H), 1.27 (t, *J* = 7.4 Hz, 3H).

**Example 14 *N*-((R)-1-(4-(ethylsulfonyl)phenyl)-2-hydroxyethyl)-4-((*S*)-3-(4-(trifluoromethyl)phenoxy)pyrrolidin-1-yl)benzamide**

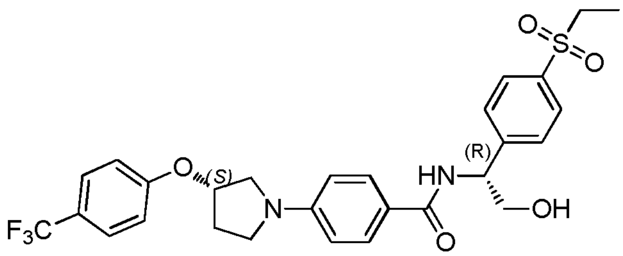

Using *tert-butyl* (*R*)-3-hydroxypyrrolidine-1-carboxylate as raw material, the title compound was prepared as a white solid (125 mg, 78%) by referring to the methods of step 1 to step 5 of Example 13.

MS (ESI, pos.ion) m/z: 563.1 $[M+H]^+$.

$^1H$ NMR (400 MHz, $CDCl_3$) δ (ppm): 7.82 (d, *J* = 8.3 Hz, 2H), 7.74 (d, *J* = 8.7 Hz, 2H), 7.54 (d, *J* = 8.1 Hz, 4H), 7.18 (d, *J* = 7.0 Hz, 1H), 6.94 (d, *J* = 8.6 Hz, 2H), 6.53 (d, *J* = 8.8 Hz, 2H), 5.22 (d, *J* = 5.3 Hz, 1H), 5.12 (s, 1H), 3.96 (dd, *J* = 11.3, 3.9 Hz, 1H), 3.88 (dd, *J* = 11.4, 5.2 Hz, 1H), 3.74 (dd, *J* = 11.2, 4.7 Hz, 1H), 3.58 - 3.48 (m, 3H), 3.07 (q, *J* = 7.4 Hz, 2H), 2.41 - 2.27 (m, 2H), 1.24 (t, *J* = 7.4 Hz, 3H).

**Example 15 *N*-((R)-1-(4-(ethylsulfonyl)phenyl)-2-hydroxyethyl)-4-((*R*)-2-oxo-3-(4-(trifluoromethyl)phenoxy)pyrrolidin-1-yl)benzamide**

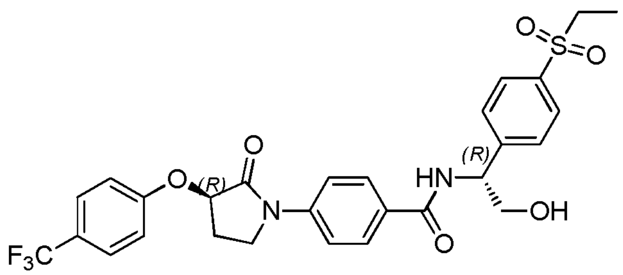

**Step 1: Synthesis of (*S*)-2-oxopyrrolidin-3-yl methanesulfonate**

Under an ice bath, MsCl (1.00 mL, 12.92 mmol) was slowly added dropwise to a solution of (*S*)-3-hydroxypyrrolidin-2-one (1.03 g, 10.20 mmol) and TEA (3.00 mL, 21.58 mmol) in THF (20 mL). After the addition was completed, the mixture was stirred at room temperature for 12 h. The reaction system was directly used for the next reaction.

**Step 2: Synthesis of (*R*)-3-(4-(trifluoromethyl)phenoxy)pyrrolidin-2-one**

(*S*)-2-Oxopyrrolidin-3-ylmethanesulfonate (1.82 g, 10.20 mmol), 4-(trifluoromethyl)phenol (1.65 g, 10.20 mmol) and $K_2CO_3$ (7.00 g, 50.65 mmol) were dissolved in DMF (20 mL). The mixture was heated and stirred in an 80°C oil bath for 22 h. The reaction solution was cooled to room temperature, diluted with EtOAc (80 mL), and washed with $NaHCO_3$ solution (20 mL×5). The organic phase was dried over anhydrous $Na_2SO_4$, and concentrated under reduced pressure. The crude product was separated by silica gel column chromatography (eluent: DCM/EtOAc (v/v) = 10/1) to give yellow oil (315 mg, 13%).

MS (ESI, pos.ion) m/z: 246.2 $[M+H]^+$.

**Step 3: Synthesis of methyl (*R*)-4-(2-oxo-3-(4-(trifluoromethyl)phenoxy)pyrrolidin-1-yl)benzoate**

Under nitrogen protection, (R)-3-(4-(trifluoromethyl)phenoxy)pyrrolidin-2-one (315 mg, 1.28 mmol), methyl 4-bromobenzoate (198 mg, 0.92 mmol), $Pd_2(dba)_3$ (22 mg, 0.02 mmol), XantPhos (22 mg, 0.04 mmol) and $Cs_2CO_3$ (403 mg, 1.23 mmol) were dissolved in 1,4-dioxane (6 mL) and the mixture was heated and stirred in a 100°C oil bath for 6 h. The reaction solution was cooled to room temperature and concentrated under reduced pressure, and the crude product was separated by silica gel column chromatography (eluent: PE/DCM/EtOAc (v/v/v) = 5/1/1) to give a yellow solid (335 mg, 68%).

MS (ESI, pos.ion) m/z: 380.0 $[M+H]^+$.

**Step 4: Synthesis of (*R*)-4-(2-oxo-3-(4-(trifluoromethyl)phenoxy)pyrrolidin-1-yl)benzoic acid**

To a solution of methyl (*R*)-4-(2-oxo-3-(4-(trifluoromethyl)phenoxy) pyrrolidin-1-yl)benzoate (232 mg, 0.61 mmol) in THF/$H_2O$ (10 mL/3 mL) was added LiOH·$H_2O$ (81 mg, 1.93 mmol). The mixture was heated and stirred in a 50°C oil bath for 2 h. The reaction solution was cooled to room temperature, and $H_2O$ (20 mL) was added to the reaction solution, then HCl solution (1 mol/L) was added dropwise to the system to adjust the pH of the system to about 3. The aqueous phase was concentrated under reduced pressure to obtain a yellow solid (223 mg, 100%).

MS (ESI, pos.ion) m/z: 398.3$[M+Na]^+$.

**Step 5: Synthesis of *N*-((*R*)-1-(4-(ethylsulfonyl)phenyl)-2-hydroxyethyl)-4-((*R*)-2-oxo-3-(4(trifluoromethyl)phenoxy)pyrrolidin-1-yl)benzamide**

(*R*)-4-(2-Oxo-3-(4-(trifluoromethyl)phenoxy)pyrrolidin-1-yl)benzoic acid (122 mg, 0.33 mmol), (*R*)-2-amino-2-(4-(ethylsulfonyl)phenyl)ethanol (93 mg, 0.41 mmol), EDCI (195 mg, 1.02 mmol) and HOBT (139 mg, 1.03 mmol) were dissolved in DCM (10 mL), then TEA (0.20 mL, 1.40 mmol) was added and the mixture was stirred at room temperature for 20 h. The reaction solution was added with DCM (80 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: DCM/EtOAc (v/v) = 1/2) to give a yellow solid (130 mg, 68%).

MS (ESI, pos.ion) m/z: 577.3 $[M+H]^+$;

$^1H$ NMR (400 MHz, DMSO-$d_6$) δ (ppm): 8.80 (d, *J* = 7.7 Hz, 1H), 7.98 (d, *J* = 8.7 Hz, 2H), 7.84 (d, *J* = 8.2 Hz, 4H), 7.68 (t, *J* = 8.2 Hz, 4H), 7.27 (d, *J* = 8.6 Hz, 2H), 5.46 (t, *J* = 8.3 Hz, 1H), 5.16 (dd, *J* = 13.4, 6.9 Hz, 1H), 5.06 (t, *J* = 5.7 Hz, 1H), 3.99 - 3.85 (m, 2H), 3.79 - 3.67 (m, 2H), 3.26 (q, *J* = 7.3 Hz, 2H), 2.81 - 2.72 (m, 1H), 2.21 - 2.12 (m, 1H), 1.10 (t, *J* = 7.3 Hz, 3H).

**Example 16 *N*-((*R*)-1-(4-(ethylsulfonyl)phenyl)-2-hydroxyethyl)-4-((S)-2-oxo-3 -(4-(trifluoromethyl)phenoxy)pyrrolidin-1-yl)benzamide**

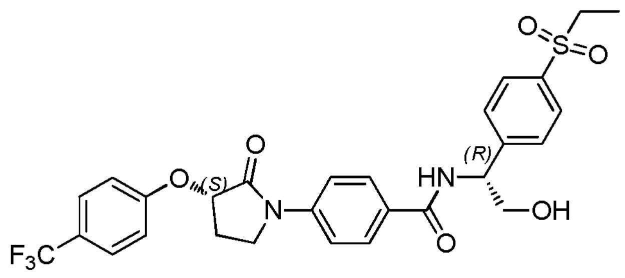

Using (*S*)-3-hydroxypyrrolidin-2-one as raw material, the title compound was prepared as a yellow solid (120 mg, 62%) by referring to the methods of step 1 to step 5 of Example 15.
MS (ESI, pos.ion) m/z: 577.1 $[M+H]^+$;
$^1H$ NMR (400 MHz, DMSO-$d_6$) δ (ppm): 8.81 (d, *J* = 7.8 Hz, 1H), 7.99 (d, *J* = 8.8 Hz, 2H), 7.85 (d, *J* = 8.2 Hz, 4H), 7.69 (t, *J* = 8.8 Hz, 4H), 7.28 (d, *J* = 8.5 Hz, 2H), 5.47 (t, *J* = 8.3 Hz, 1H), 5.19 - 5.13 (m, 1H), 5.06 (t, *J* = 5.7 Hz, 1H), 3.99 - 3.89 (m, 2H), 3.79 - 3.68 (m, 2H), 3.27 (q, *J* = 7.3 Hz, 2H), 2.83 - 2.73 (m, 1H), 2.22 - 2.12 (m, 1H), 1.10 (t, *J* = 7.3 Hz, 3H).

**Example 17 *N*-((*R*)-1-(4-(ethylsulfonyl)phenyl)-2-hydroxyethyl)-4-((3*S*,4*S*)-3-fluoro-4-(4-(trifluoromethyl)phenoxy)pyrrolidin-1-yl)benzamide**

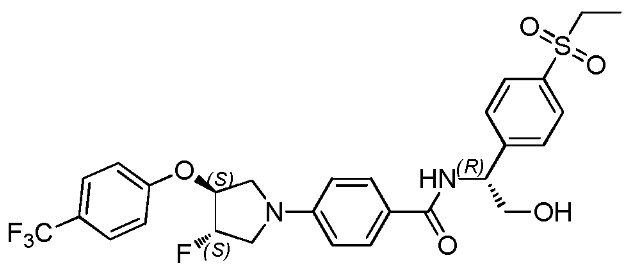

**Step 1: Synthesis of *tert*-butyl (3*S*,4*S*)-3-fluoro-4-(4-(trifluoromethyl) phenoxy)pyrrolidine-1-carboxylate**

Under nitrogen protection, *tert*-butyl (3*S*,4*S*)-3-fluoro-4-hydroxypyrrolidine -1-carboxylate (500 mg, 2.44 mmol), 1-bromo-4-(trifluoromethyl)benzene (822 mg, 3.65 mmol), $Pd(OAc)_2$ (30 mg , 0.13 mmol), *t*-Bu-XPhos (106 mg, 0.25 mmol) and $Cs_2CO_3$ (1.58 g, 4.85 mmol) were dissolved in toluene (16 mL) and the mixture was reacted in a 90°C oil bath for 24 h. The reaction solution was cooled to room temperature, and concentrated under reduced pressure. The residue was diluted with DCM (100 mL), washed successively with $NaHCO_3$ solution (30 mL) and saturated NaCl solution (30 mL), dried over anhydrous $Na_2SO_4$, and concentrated under reduced pressure. The crude product was separated by silica gel column chromatography (eluent: PE/EtOAc (v/v) = 4/1) to give a white solid (300 mg, 35%).
MS (ESI, pos.ion) m/z: 294.7 $[M-56+H]^+$.

**Step 2: Synthesis of (3S,4S)-3-fluoro-4-(4-(trifluoromethyl)phenoxy)pyrrolidine**

To a solution of *tert*-butyl (3*S*,4*S*)-3-fluoro-4-(4-(tiifluoromethyl) phenoxy)pyrrolidine-1-carboxylate (300 mg, 0.86 mmol) in DCM (6 mL) was added a solution of HCl in methanol (2 mL, 20%). The mixture was stirred at room temperature for 12 h. The reaction solution was concentrated under reduced pressure. The residue was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: DCM/MeOH (v/v) = 20/1) to give pale yellow liquid (106 mg, 50%).
MS (ESI, pos.ion) m/z: 250.2 $[M+H]^+$.

**Step 3: Synthesis of ethyl 4-((3*S*,4*S*)-3-fluoro-4-(4-(trifluoromethyl) phenoxy)pyrrolidin-1-yl)benzoate**

Under nitrogen protection, (3*S*,4*S*)-3-fluoro-4-(4-(trifluoromethyl)phenoxy)pyrrolidine (100 mg, 0.40 mmol), ethyl 4-iodobenzoate (166 mg, 0.60 mmol), $Pd_2(dba)_3$ (36 mg, 0.04 mmol), XantPhos (34 mg, 0.06 mmol) and $Cs_2CO_3$

(261 mg, 0.80 mmol) were dissolved in 1,4-dioxane (6 mL), and the mixture was heated and stirred in a 100°C oil bath for 23 h. The reaction solution was cooled to room temperature, and concentrated under reduced pressure. The residue was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 5/1) to give yellow liquid (100 mg, 63%).

MS (ESI, pos.ion) m/z: 398.1 $[M+H]^+$.

**Step 4: Synthesis of 4-((3*S*,4*S*)-3-fluoro-4-(4-(trifluoromethyl)phenoxy)pyrrolidin-1-yl)benzoic acid**

To a solution of ethyl 4-((3*S*,4*S*)-3-fluoro-4-(4-(trifluoromethyl)phenoxy) pyrrolidin-1-yl)benzoate (100 mg, 0.25 mmol) in MeOH (6 mL) was added a solution of $LiOH·H_2O$ (86 mg, 2.05 mmol) in $H_2O$ (1 mL). The mixture was reacted at room temperature for 24 h. The reaction solution was added with HCl solution (1 mol/L) to adjust the pH of the solution to about 5. The resulting mixture was extracted with EtOAc (20 mL × 2), and the organic phases were combined, washed with saturated NaCl (10 mL) solution, dried over anhydrous $Na_2SO_4$, and concentrated under reduced pressure. The crude product was separated by silica gel column chromatography (eluent: PE/EtOAc (v/v) = 3/2) to give a pale yellow solid (50 mg, 54%).

MS (ESI, pos.ion) m/z: 340.2 $[M+H]^+$.

**Step 5: Synthesis of *N*-((*R*)-1-(4-(ethylsulfonyl)phenyl)-2-hydroxyethyl) -4-((3*S*,4*S*)-3-fluoro-4-(4-(trifluoromethyl)phenoxy)pyrrolidin-1-yl)benzamide**

(*R*)-2-Amino-2-(4-(ethylsulfonyl)phenyl)ethanol (37 mg, 0.16 mmol), 4-((3*S*,4*S*)-3-fluoro-4-(4-trifluoromethyl)phenoxy)pyrrolidin-1-yl)benzoic acid (50 mg , 0.16 mmol), EDCI (38 mg, 0.20 mmol), HOBT (27 mg, 0.20 mmol) and TEA (27 mg, 0.27 mmol) were dissolved in DCM (5 mL), and the mixture was stirred at room temperature for 16 h. The reaction solution was added with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (50 mg, 64%).

MS (ESI, pos.ion) m/z: 581.3 $[M+H]^+$.

$^1H$ NMR (400 MHz, $CDCl_3$) δ (ppm): 7.85 (d, *J* = 8.3 Hz, 2H), 7.76 (d, *J* = 8.7 Hz, 2H), 7.59 (d, *J* = 8.7 Hz, 2H), 7.56 (d, *J* = 8.3 Hz, 2H), 7.00 (d, *J* = 8.7 Hz, 2H), 6.97 (s, 1H), 6.57 (d, *J* = 8.7 Hz, 2H), 5.39 (d, *J* = 3.4 Hz, 1H), 5.30 - 5.25 (m, 1H), 5.12 (dd, *J* = 9.8, 4.3 Hz, 1H), 4.03 (dd, *J* = 11.2, 3.5 Hz, 1H), 3.96 (dd, *J* = 11.2, 4.9 Hz, 1H), 3.90 (dd, *J* = 13.0, 3.2 Hz, 1H), 3.87 - 3.76 (m, 1H), 3.78 - 3.67 (m, 1H), 3.64 (d, *J* = 11.3 Hz, 1H), 3.08 (q, *J* = 7.4 Hz, 2H), 1.27 (t, *J* = 7.4 Hz, 3H).

**Example 18 *N*-((*R*)-1-(4-(ethylsulfonyl)phenyl)-2-hydroxyethyl)-4-((2*S*,4*R*)-2-(hydroxymethyl)-4-(4-(trifluoromethyl)phenoxy)pyrrolidin-1-yl)benzamide**

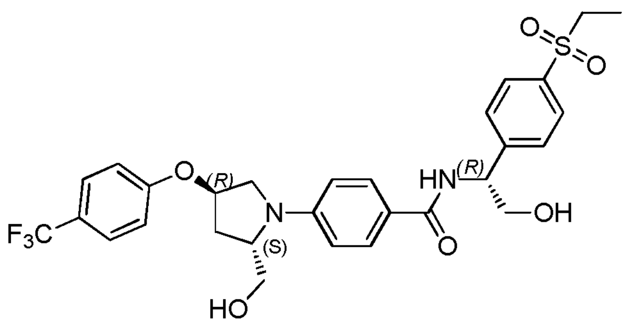

**Step 1: Synthesis of (2*S*,4*R*)-1-*tert*-butyl 2-methyl 4-(4-(trifluoromethyl)phenoxy)pyrrolidine-1,2-dicarboxylate**

(2*S*,4*S*)-1-*tert*-Butyl 2-methyl 4-hydroxypyrrolidine-1,2-dicarboxylate (10.00 g, 40.77 mmol), 4-(trifluoromethyl)phenol (6.60 g, 40.71 mmol), $PPh_3$ (15.40 g, 44.87 mmol) were dissolved in THF (160 mL). DIAD (10.50 mL, 53.30 mmol) was slowly added under the ice bath. After the addition was complete, the mixture was stirred at room temperature for 21 h. The reaction solution was concentrated under reduced pressure. The residue was diluted with methyl tert-butyl ether (50 mL) and stirred at -20 °C. A large amount of white insoluble solids were precipitated. The mixture was filtered

while cold. The filter cake was washed with cold methyl tert-butyl ether, and the filtrate was concentrated under reduced pressure. The crude product was separated by silica gel column chromatography (eluent: PE/EtOAc (v/v) = 6/1) to give a white solid (9.30 g, 58%).

MS (ESI, pos.ion) m/z: 411.9 $[M+Na]^+$.

**Step 2: Synthesis of *tert*-butyl (2*S*,4*R*)-2-(hydroxymethyl)-4-(4-(trifluoromethyl)phenoxy)pyrrolidine-1-carboxylate**

To a solution of (2*S*,4*R*)-1-*tert*-butyl 2-methyl 4-(4-(trifluoromethyl)phenoxy)pyrrolidine-1,2-dicarboxylate (7.00 g, 17.98 mmol) in THF (70 mL) was added $LiBH_4$ (590 mg, 27.09 mmol) under an ice bath. After the addition was completed, the mixture was stirred at room temperature for 14 h. Saturated $NH_4Cl$ (40 mL) solution was added to the reaction solution to quench the reaction. The mixture was left standing for layers, the upper organic phase was separated, the aqueous phase was extracted with EtOAc (30 mL × 2). The combined organic phases were dried over anhydrous $Na_2SO_4$, concentrated under reduced pressure, and the crude product was separated by silica gel column chromatography (eluent: PE/EtOAc (v/v) = 3/1) to give colorless oily liquid (5.90 g, 91%).

MS (ESI, pos.ion) m/z: 383.9 $[M+Na]^+$.

**Step 3: Synthesis of ((2*S*,4*R*)-4-(4-(trifluoromethyl)phenoxy)pyrrolidin-2-yl)methanol**

*tert*-Butyl (2*S*,4*R*)-2-(hydroxymethyl)-4-(4-(trifluoromethyl)phenoxy)pyrrolidine -1-carboxylate (5.90 g, 16.33 mmol) was dissolved in a mixed solution of DCM (40 mL) and HCl in methanol (20 mL, 20%). The mixture was reacted at room temperature for 16 h. The reaction solution was concentrated under reduced pressure to give brown liquid (4.30 g, 100%).

MS (ESI, pos.ion) m/z: 262.2 $[M+H]^+$.

**Step 4: Synthesis of ethyl 4-((2*S*,4*R*)-2-(hydroxymethyl)-4-(4-(trifluoromethyl)phenoxy)pyrrolidin-1-yl)benzoate**

Under nitrogen protection, ((2*S*,4*R*)-4-(4-(trifluoromethyl)phenoxy)pyrrolidin -2-yl)methanol (500 mg, 1.91 mmol), $Pd_2(dba)_3$ (175 mg, 0.19 mmol), XantPhos (167 mg, 0.29 mmol), $Cs_2CO_3$ (1.90 g, 5.83 mmol) and ethyl 4-iodobenzoate (686 mg, 2.48 mmol) were dissolved in 1,4-dioxane (12 mL), and the mixture was reacted in a 100°C oil bath for 10 h. The reaction solution was cooled to room temperature, and concentrated under reduced pressure. The residue was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 3/1) to give a brown solid (248 mg, 32%).

MS (ESI, pos.ion) m/z: 410.3 $[M+H]^+$.

**Step 5: Synthesis of 4-((2*S*,4*R*)-2-(hydroxymethyl)-4-(4-(trifluoromethyl)phenoxy) pyrrolidin-1-yl)benzoic acid**

To a solution of ethyl 4-((2*S*,4*R*)-2-(hydroxymethyl)-4-(4-(trifluoromethyl)phenoxy)pyrrolidin-1-yl)benzoate(150 mg, 0.37 mmol) in THF (6 mL) was added a solution of $LiOH{\cdot}H_2O$ (95 mg, 2.26 mmol) in $H_2O$ (1 mL). The mixture was reacted at room temperature for 6 h. The reaction solution was added with HCl solution (1 mol/L) to adjust the pH of the solution to about 5. The aqueous phase was extracted with EtOAc (20 mL × 2), and the organic phases were combined, washed with saturated NaCl (10 mL) solution, dried over anhydrous $Na_2SO_4$, and concentrated under reduced pressure. The crude product was separated by silica gel column chromatography (eluent: DCM/MeOH (v/v) = 20/1) to give colorless liquid (100 mg, 72%).

MS (ESI, pos.ion) m/z: 382.1 $[M+H]^+$.

**Step 6: Synthesis of *N*-((*R*)-1-(4-(ethylsulfonyl)phenyl)-2-hydroxyethyl) -4-((2*S*,4*R*)-2-(hydroxymethyl)-4-(4-(trifluoromethyl)phenoxy)pyrrolidin-1-yl)benzamide**

4-((2*S*,4*R*)-2-(Hydroxymethyl)-4-(4-(trifluoromethyl)phenoxy)pyrrolidin-1-yl)benzoic acid (100 mg, 0.26 mmol), (*R*)-2-amino-2-(4-(ethylsulfonyl)phenyl)ethanol (72 mg, 0.31 mmol), EDCI (75 mg, 0.39 mmol), HOBT (53 mg, 0.39 mmol) and TEA (53 mg, 0.52 mmol) were dissolved in DCM (5 mL), and the mixture was reacted at room temperature for 10 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (78 mg, 50%).

MS (ESI, pos.ion) m/z: 593.4 $[M+H]^+$.

$^1H$ NMR (400 MHz, $CDCl_3$) δ (ppm): 7.75 (d, *J* = 8.2 Hz, 2H), 7.66 (d, *J* = 8.4 Hz, 2H), 7.52 (d, *J* = 8.4 Hz, 2H), 7.47 (d, *J* = 8.2 Hz, 2H), 7.30 (d, *J* = 7.0 Hz, 1H), 6.94 (d, *J* = 8.5 Hz, 2H), 6.53 (d, *J* = 8.5 Hz, 2H), 5.18 (s, 2H), 4.13 (d, *J* = 3.4 Hz, 1H), 3.91 - 3.80 (m, 4H), 3.57 (d, *J* = 11.1 Hz, 1H), 3.49 - 3.42 (m, 1H), 3.03 (q, *J* = 7.3 Hz, 2H), 2.58 - 2.49 (m, 1H), 2.36 - 2.28 (m, 1H), 1.21 (t, *J* = 7.4 Hz, 3H).

**Example 19 4-((2*S*)-2-(ethoxymethyl)-4-(4-(trifluoromethyl)phenoxy)pyrrolidin-1-yl) -*N*-(4-(ethylsulfonyl)benzyl)benzamide**

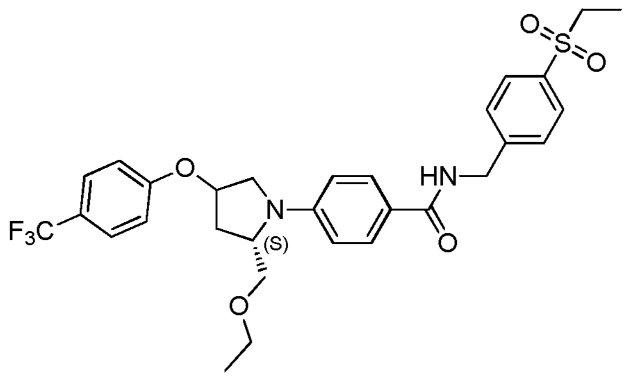

**Step 1: Synthesis of (2*S*)-1-*tert*-butyl 2-methyl 4-(4-(trifluoromethyl)phenoxy)pyrrolidine-1,2-dicarboxylate**

Under nitrogen protection, (2*S*)-1-*tert*-butyl 2-methyl 4-hydroxypyrrolidine-1,2-dicarboxylate (10.03 g, 40.89 mmol), 1-bromo-4-(trifluoromethyl)benzene (13.80 g, 61.30 mmol), $Cs_2CO_3$ (27 g, 82.87 mmol), $Pd(OAc)_2$ (460 mg, 2.05 mmol) and *t*-Bu-XPhos (1.70 g, 4.00 mmol) were dissolved in toluene (100 mL), and the mixture was reacted in a 90°C oil bath for 24 h. The reaction solution was cooled to room temperature, diluted with DCM (120 mL), washed successively with $H_2O$ (50 mL) and saturated NaCl solution (50 mL), dried over anhydrous $Na_2SO_4$, and concentrated under reduced pressure. The crude product was separated by silica gel column chromatography (eluent: PE/DCM/EtOAc (v/v/v) = 5/1/1) to give yellow oily liquid (4.34 g, 27%).

MS (ESI, pos.ion) m/z: 412.2 $[M+Na]^+$.

**Step 2: Synthesis of *tert*-butyl (2*S*)-2-(hydroxymethyl) -4-(4-(trifluoromethyl)phenoxy)pyrrolidine-1-carboxylate**

To a solution of (2*S*)-1-*tert*-butyl 2-methyl 4-(4-(trifluoromethyl)phenoxy)pyrrolidine-1,2-dicarboxylate (4.34 g , 11.10 mmol) in THF (30 mL) was added $LiBH_4$ (500 mg, 22.96 mmol). The mixture was stirred at room temperature for 10 h. Saturated $NaHCO_3$ solution (40 mL) was added to the reaction solution to quench the reaction. The mixture was extracted with DCM (50 mL×3). The organic phase was washed with saturated NaCl solution (40 mL) and dried over anhydrous $Na_2SO_4$, and concentrated under reduced pressure. The crude product was separated by silica gel column chromatography (eluent: PE/DCM/EtOAc (v/v/v) = 3/1/1) to give a white solid (3.16 g, 79%).

MS (ESI, pos.ion) m/z: 384.2 $[M+Na]^+$.

**Step 3: Synthesis of ((2*S*)-4-(4-(trifluoromethyl)phenoxy)pyrrolidin-2-yl)methanol**

*tert*-Butyl (2*S*)-2-(hydroxymethyl)-4-(4-(trifluoromethyl)phenoxy)pyrrolidine -1-carboxylate (231 mg, 0.64 mmol) was dissolved in a solution of HCl in *i*-PrOH (10 mL, 45.80 mmol, 4.58 mol/L ), and the mixture was stirred at room temperature for 10 h. The reaction solution was concentrated under reduced pressure, and saturated $Na_2CO_3$ solution (20 mL) was added to the residue. The aqueous phase was extracted with DCM (40 mL×3), and the organic phases were combined and washed with saturated NaCl solution (30 mL), dried over anhydrous $Na_2SO_4$, and concentrated under reduced pressure to give a pink solid (160 mg, 96%).

MS (ESI, pos.ion) m/z: 262.2 $[M+H]^+$.

**Step 4: Synthesis of 4-((2*S*)-2-(hydroxymethyl)-4-(4-(trifluoromethyl)phenoxy) pyrrolidin-1-yl)benzonitrile**

Under nitrogen protection, ((2*S*)-4-(4-(trifluoromethyl)phenoxy)pyrrolidin -2-yl)methanol (160 mg, 0.61 mmol), 4-iodobenzonitrile (230 mg, 1.26 mmol), $Pd_2(dba)_3$ (115 mg, 0.13 mmol), XantPhos (80 mg, 0.14 mmol) and $Cs_2CO_3$

(500 mg, 1.53 mmol) were dissolved in 1,4-dioxane (4 mL), and the mixture was heated and stirred in a 100°C oil bath for 24 h. The reaction solution was cooled to room temperature, and concentrated under reduced pressure. The residue was dissolved with DCM (80 mL), washed successively with saturated aqueous $NaHCO_3$ solution (40 mL) and saturated NaCl solution (30 mL), dried over anhydrous $Na_2SO_4$, and concentrated under reduced pressure. The crude product was separated by silica gel column chromatography (eluent: PE/DCM/EtOAc (v/v/v) = 2/1/1) to give a tan solid (198 mg, 89%).

MS (ESI, pos.ion) m/z: 363.3 $[M+H]^+$.

**Step 5: Synthesis of 4-((2*S*)-2-(ethoxymethyl)-4-(4-(trifluoromethyl) phenoxy)pyrrolidin-1-yl)benzonitrile**

Under nitrogen protection, to a solution of 4-((2*S*)-2-(hydroxymethyl)-4-(4-(trifluoromethyl)phenoxy)pyrrolidin-1-yl)benzonitrile (250 mg, 0.69 mmol) in DMF (4 mL) was added NaH (55 mg, 1.38 mmol, 60%). After 30 min, EtI (0.10 mL, 1.25 mmol) was added, and the mixture was stirred at room temperature for 12 h. The reaction solution was diluted with EtOAc (60 mL), washed saturated NaCl solution (10 mL×5), dried over anhydrous $Na_2SO_4$, and concentrated under reduced pressure. The crude product was separated by silica gel column chromatography (eluent: PE/DCM/EtOAc (v/v/v) = 5/1/1) to give a yellow solid (188 mg, 70%).

MS (ESI, pos.ion) m/z: 391.5 $[M+H]^+$.

**Step 6: Synthesis of 4-((2*S*)-2-(ethoxymethyl)-4-(4-(trifluoromethyl) phenoxy)pyrrolidin-1-yl)benzoic acid**

Under nitrogen protection, to a solution of 4-((2*S*)-2-(ethoxymethyl)-4-(4-(trifluoromethyl)phenoxy)pyrrolidin-1-yl)benzonitrile (188 mg, 0.48 mmol) in EtOH (5 mL) was added MeONa (453 mg, 8.07 mmol). The mixture was heated and stirred in an oil bath at 100°C for 24 h. The reaction solution was cooled to room temperature, and HCl solution (20 mL, 1 mol/L) was added to the reaction solution to quench the reaction. The aqueous phase was extracted with EtOAc (20 mL×3), and the combined organic phases were washed with saturated NaCl solution (20 mL), dried over anhydrous $Na_2SO_4$, and concentrated under reduced pressure to give a dark yellow solid (195 mg, 99%).

MS (ESI, pos.ion) m/z: 410.5 $[M+H]^+$.

**Step 7: Synthesis of 4-((2*S*)-2-(ethoxymethyl)-4-(4-trifluoromethyl)phenoxy) pyrrolidin-1-yl)-N-(4-(ethylsulfonyl)benzyl)benzamide**

4-((2*S*)-2-(Ethoxymethyl)-4-(4-(trifluoromethyl)phenoxy)pyrrolidin-1-yl)benzoic acid (95 mg, 0.23 mmol), (4-(ethylsulfonyl)phenyl)methanamine (70 mg, 0.35 mmol), EDCI (90 mg, 0.47 mmol) and HOBT (63 mg, 0.47 mmol) were dissolved in DCM (3 mL), then TEA (0.10 mL, 0.72 mmol) was added and the mixture was stirred at room temperature for 20 h. The reaction solution was diluted with DCM (80 mL), washed successively with HCl solution (20 mL, 1 mol/L), saturated NaCl solution (20 mL) and saturated $Na_2CO_3$ solution (20 mL), dried over anhydrous $Na_2SO_4$, and concentrated under reduced pressure. The crude product was separated by silica gel column chromatography (eluent: DCM/EtOAc (v/v) = 1/1) and silica gel plate chromatography (developing solvent: DCM/EtOAc (v/v) = 5/1) to give a yellow solid (34 mg, 25%).

MS (ESI, pos.ion) m/z: 591.2 $[M+H]^+$;

$^1H$ NMR (400 MHz, $CDCl_3$) $\delta$ (ppm): 7.88 (d, *J* = 8.3 Hz, 2H), 7.76 (d, *J* = 8.8 Hz, 2H), 7.61 (d, *J* = 8.6 Hz, 2H), 7.56 (d, *J* = 8.3 Hz, 2H), 6.99 (d, *J* = 8.6 Hz, 2H), 6.70 (d, *J* = 8.9 Hz, 2H), 6.46 (t, *J* = 6.1 Hz, 1H), 5.17 (t, *J* = 4.5 Hz, 1H), 4.76 (d, *J* = 60 Hz, 2H), 4.16 (td, *J*= 8.5, 4.3 Hz, 1H), 3.74 (ddd, *J* = 16.1, 10.4, 4.7 Hz, 3H), 3.59 - 3.43 (m, 3H), 3.12 (q, *J* = 7.4 Hz, 2H), 2.54 (d, *J* = 14.2 Hz, 1H), 2.41 (dd, *J* = 8.4, 5.4 Hz, 1H), 1.29 (t, *J* = 7.4 Hz, 3H), 1.19 (t, *J* = 7.0 Hz, 3H).

**Example 20 4-((2*S*)-2-(ethoxymethyl)-4-(4-trifluoromethyl)phenoxy)pyrrolidin-1-yl) -*N*-((*R*)-1-(4-(ethylsulfonyl)phenyl)-2-hydroxyethyl)benzamide**

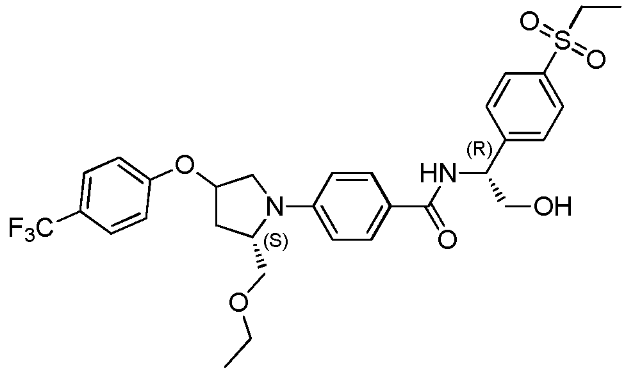

4-((2*S*)-2-(Ethoxymethyl)-4-(4-(trifluoromethyl)phenoxydin-1-yl)benzoic acid (95 mg, 0.23 mmol), (*R*)-2-amino-2-(4-(ethylsulfonyl)phenyl)ethanol (65 mg, 0.28 mmol), EDCI (90 mg, 0.47 mmol) and HOBT (63 mg, 0.47 mmol) were dissolved in DCM (3 mL), then TEA (0.10 mL, 0.72 mmol) was added and the mixture was stirred at room temperature for 20 h. The reaction solution was diluted with DCM (80 mL), washed successively with HCl solution (20 mL, 1 mol/L), saturated $Na_2CO_3$ solution (20 mL) and saturated NaCl solution (20 mL), dried over anhydrous $Na_2SO_4$, and concentrated under reduced pressure. The crude product was separated by silica gel column chromatography (eluent: DCM/EtOAc (v/v) = 1/2) and preparative silica gel plate chromatography (developing solvent: DCM/EtOAc (v/v) = 1/1) to give a yellow solid (75 mg, 52%).

MS (ESI, pos.ion) m/z: 621.3 $[M+H]^+$;

$^1H$ NMR (400 MHz, $CDCl_3$) δ (ppm): 7.86 (d, *J* = 6.7 Hz, 2H), 7.75 (d, *J* = 8.7 Hz, 2H), 7.57 (t, *J* = 6.9 Hz, 4H), 6.97 (d, *J* = 8.6 Hz, 3H), 6.67 (d, *J* = 8.6 Hz, 2H), 5.30 (s, 1H), 5.14 (t, *J* = 4.5 Hz, 1H), 4.12 (dd, *J* = 8.3, 4.2 Hz, 1H), 4.02 (d, *J* = 15.6 Hz, 2H), 3.80 - 3.63 (m, 3H), 3.58 - 3.40 (m, 3H), 3.09 (q, *J* = 7.4 Hz, 2H), 2.51 (d, *J* = 14.2 Hz, 1H), 2.37 (td, *J* = 8.5, 4.4 Hz, 1H), 1.28 (t, *J* = 7.9 Hz, 3H), 1.16 (t, *J* = 7.0 Hz, 3H).

**Example 21 4-((2*S*,4*R*)-2-(ethoxymethyl)-4-(4-trifluoromethyl)phenoxy)pyrrolidin -1-yl)-*N*-((*R*)-1-(4-(ethylsulfonyl)phenyl)-2-hydroxyethyl)benzamide**

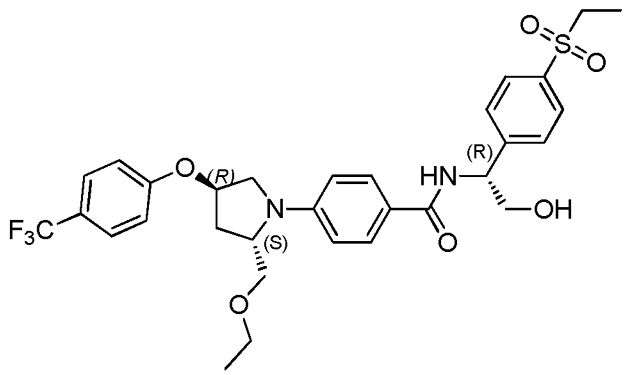

**Step 1: Synthesis of *tert*-butyl (2*S*,4*R*)-2-(ethoxymethyl)-4-(4-(trifluoromethyl) phenoxy)pyrrolidine-1-carboxylate**

Under nitrogen protection, to a solution of iodoethane (1.50 mL, 18.75 mmol) and *tert*-butyl (2*S*,4*R*)-2-(hydroxymethyl)-4-(4-(trifluoromethyl)phenoxy)pyrrolidine-1-carboxylate (600 mg, 1.66 mmol) in THF (10 mL) was added NaH (531 mg, 13.28 mmol, 60%). The mixture was stirred at room temperature for 23 h. The reaction solution was added with HCl solution (1 mol/L) to quench the reaction, and the pH of the solution was adjusted to about 5. The resulting mixture was diluted with EtOAc (30 mL), extracted and separated. The organic phase was washed with saturated NaCl (15 mL) solution, dried over anhydrous $Na_2SO_4$, and concentrated under reduced pressure. The crude product was separated by silica gel column chromatography (eluent: PE/EtOAc (v/v) = 4/1) to give yellow liquid (150 mg, 23%).

MS (ESI, pos.ion) m/z: 334.1 $[M-56+H]^+$.

**Step 2: Synthesis of (2*S*,4*R*)-2-(ethoxymethyl)-4-(4-(trifluoromethyl)phenoxy)pyrrolidine**

To a solution of *tert*-butyl (2*S*,4*R*)-2-(ethoxymethyl)-4-(4-(trifluoromethyl) phenoxy)pyrrolidine-1-carboxylate (150 mg, 0.39 mmol) in DCM (6 mL) was added a solution of HCl in methanol (2 mL, 20%). The mixture was stirred at room temperature for 11 h. After the reaction was completed, saturated sodium bicarbonate was added for washing, and the reaction solution was concentrated under reduced pressure to give brown liquid (111 mg, 100%).
MS (ESI, pos.ion) m/z: 290.2 $[M+H]^+$.

**Step 3: Synthesis of ethyl 4-((2*S*,4*R*)-2-(ethoxymethyl)-4-(4-(trifluoromethyl) phenoxy)pyrrolidin-1-yl)benzoate**

Under nitrogen protection, (2*S*,4*R*)-2-(ethoxymethyl)-4-(4-(trifluoromethyl) phenoxy)pyrrolidine (100 mg, 0.35 mmol), $Pd_2(dba)_3$ (31 mg, 0.03 mmol), XantPhos (30 mg, 0.05 mmol), $Cs_2CO_3$ (225 mg, 0.69 mmol) and ethyl 4-iodobenzoate (89 mg, 0.41 mmol) were dissolved in 1,4-dioxane (6 mL) in turn and the mixture was reacted in a 100°C oil bath for 12 h. The reaction solution was cooled to room temperature, and concentrated under reduced pressure. The residue was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 5/1) to give yellow liquid (70 mg, 48%).
MS (ESI, pos.ion) m/z: 424.0 $[M+H]^+$.

**Step 4: Synthesis of 4-((2*S*,4*R*)-2-(ethoxymethyl)-4-(4-(trifluoromethyl) phenoxy)pyrrolidin-1-yl)benzoic acid**

To a solution of ethyl 4-((2*S*,4*R*)-2-(ethoxymethyl)-4-(4-(trifluoromethyl) phenoxy)pyrrolidin-1-yl)benzoate (80 mg, 0.19 mmol) in MeOH (4 mL) was added a solution of $LiOH \cdot H_2O$ (50 mg, 1.19 mmol) in $H_2O$ (1 mL). The mixture was reacted at room temperature for 13 h. The reaction solution was added with HCl solution (1 mol/L) to adjust the pH of the solution to about 5. The resulting mixture was extracted with EtOAc (30 mL × 2). The organic phases were combined, washed with saturated NaCl (20 mL) solution, dried over anhydrous $Na_2SO_4$, and concentrated under reduced pressure. The crude product was separated by silica gel column chromatography (eluent: DCM/EtOAc (v/v) = 4/1) to give a yellow solid (60 mg, 78%).
MS (ESI, pos.ion) m/z: 410.1 $[M+H]^+$.

**Step 5: Synthesis of 4-((2*S*,4*R*)-2-(ethoxymethyl)-4-(4-(trifluoromethyl)phenoxy) pyrrolidin-1-yl)-*N*-((*R*)-1-(4-(ethylsulfonyl)phenyl)-2-hydroxyethyl)benzamide**

EDCI (56 mg, 0.29 mmol), HOBT (39 mg, 0.29 mmol), TEA (30 mg, 0.30 mmol), (*R*)-2-amino-2-(4-(ethylsulfonyl)phenyl)ethanol (40 mg, 0.17 mmol) and 4-((2*S*,4*R*)-2-(ethoxymethyl)-4-(4-(trifluoromethyl)phenoxy)pyrrolidin-1-yl)benzoic acid (60 mg, 0.15 mmol) were successively added to DCM (4 mL), and the mixture was reacted at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (50 mg, 55%).
MS (ESI, pos.ion) m/z: 621.2 $[M+H]^+$.
$^1H$ NMR (400 MHz, $CDCl_3$) δ (ppm): 7.87 (d, *J* = 8.3 Hz, 2H), 7.76 (d, *J* = 8.7 Hz, 2H), 7.58 (d, *J* = 8.2 Hz, 4H), 6.98 (d, *J* = 8.3 Hz, 3H), 6.64 (d, *J* = 8.7 Hz, 2H), 5.29 - 5.22 (m, 2H), 4.29 - 4.24 (m, 1H), 4.05 (dd, *J* = 11.1, 3.4 Hz, 1H), 3.98 (dd, *J* = 11.3, 4.8 Hz, 1H), 3.93 (dd, *J* = 10.4, 6.3 Hz, 1H), 3.61 - 3.55 (m, 2H), 3.53 - 3.44 (m, 3H), 3.10 (q, *J* = 7.4 Hz, 2H), 2.60 - 2.55 (m, 1H), 2.45 - 2.37 (m, 1H), 1.30 (t, *J* = 7.4 Hz, 3H), 1.20 (t, *J* = 7.0 Hz, 3H).

**Example 22 4-((2*S*,4*S*)-2-(ethoxymethyl)-4-(4-trifluoromethyl)phenoxy)pyrrolidin -1-yl)-*N*-((*R*)-1-(4-(ethylsulfonyl)phenyl)-2-hydroxyethyl)benzamide**

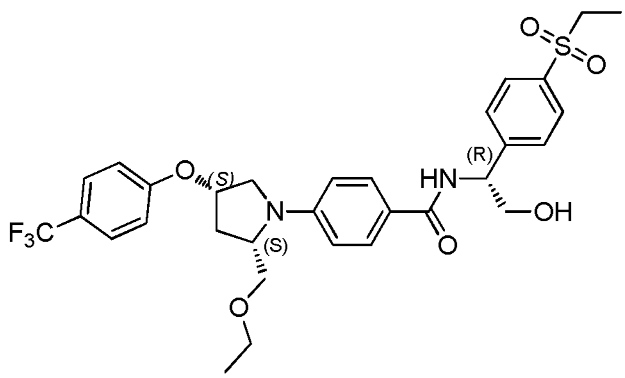

Using (2*S*,4*R*)-1-*tert*-butyl 2-methyl 4-hydroxypyrrolidine-1,2-dicarboxylate (2.00 g, 8.15 mmol) as raw material, the title compound was prepared as a white solid (60 mg, 72%) by referring to the methods of step 1 to step 2 of Example 18 and the methods of step 1 to step 5 of Example 21.

MS (ESI, pos.ion) m/z: 621.4 $[M+H]^+$.

$^1H$ NMR (400 MHz, $CDCl_3$) δ (ppm): 7.83 (d, *J* = 8.1 Hz, 2H), 7.74 (d, *J* = 8.7 Hz, 2H), 7.57 (d, *J* = 8.1 Hz, 2H), 7.56 (d, *J* = 8.5 Hz, 2H),7.03 (d, *J* = 6.6 Hz, 1H), 6.97 (d, *J* = 8.5 Hz, 2H), 6.66 (d, *J* = 8.7 Hz, 2H), 5.24 (d, *J* = 5.7 Hz, 1H), 5.14 (s, 1H), 4.16 - 4.08 (m, 1H), 4.00 (dd, *J* = 11.2, 3.5 Hz, 1H), 3.93 (dd, *J* = 11.2, 4.8 Hz, 1H), 3.76 - 3.64 (m, 3H), 3.55 - 3.40 (m, 3H), 3.07 (q, *J* = 7.4 Hz, 2H), 2.50 (d, *J* = 14.3 Hz, 1H), 2.41 - 2.31 (m, 1H), 1.27 (t, *J* = 7.4 Hz, 3H), 1.16 (t, *J* = 7.0 Hz, 3H).

**Example 23 *N*-(4-(ethylsulfonyl)benzyl)-4-((2*S*,4*R*)-2-(fluoromethyl)-4-(4-(trifluoromethyl)phenoxy)pyrrolidin-1-yl)benzamide**

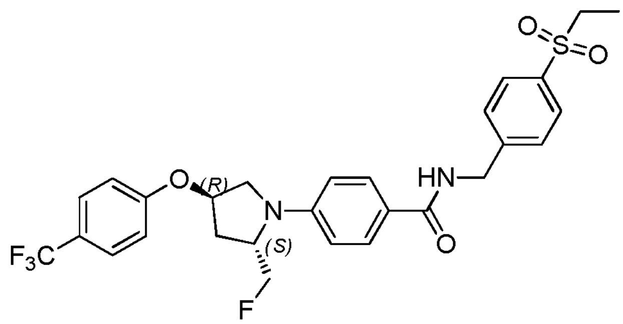

**Step 1: Synthesis of 4-((2*S*,4*R*)-2-(fluoromethyl)-4-(4-(trifluoromethyl) phenoxy)pyrrolidin-1-yl)benzonitrile**

To a solution of 4-((2*S*,4*R*)-2-(hydroxymethyl)-4-(4-(trifluoromethyl) phenoxy)pyrrolidin-1-yl)benzonitrile (210 mg, 0.58 mmol) (the preparation method refers to the methods of step 1 to step 4 of Example 19 using (2*S*,4*R*)-1-*tert*-butyl 2-methyl 4-hydroxypyrrolidine-1,2-dicarboxylate as raw material) in DCM (5 mL) was added DAST (0.30 mL, 2.30 mmol) slowly at room temperature. The mixture was stirred at room temperature for 3 h. Saturated $NaHCO_3$ solution (20 mL) was added to the reaction solution, then the aqueous phase was extracted with DCM (30 mL×3). The combined organic phases were washed with saturated NaCl solution (20 mL) and dried over anhydrous $Na_2SO_4$, and concentrated under reduced pressure. The crude product was separated by silica gel column chromatography (eluent: PE/DCM/EtOAc (v/v/v) = 5/1/1) to give a tan solid (171 mg, 81%).

MS (ESI, pos.ion) m/z: 365.2 $[M+H]^+$.

**Step 2: Synthesis of 4-((2*S*,4*R*)-2-(fluoromethyl)-4-(4-(trifluoromethyl) phenoxy)pyrrolidin-1-yl)benzoic acid**

To a solution of 4-((2*S*,4*R*)-2-(fluoromethyl)-4-(4-(trifluoromethyl) phenoxy)pyrrolidin-1-yl)benzonitrile (135 mg, 0.37 mmol) in EtOH (6 mL) was added MeONa (822 mg, 15.22 mmol). The mixture was heated and stirred in an oil bath at 100°C for 12 h. The reaction solution was cooled to room temperature, and $H_2O$ (20 mL) was added to the reaction solution, then the resulting mixture was extracted with DCM (10 mL×2). HCl solution (1.0 mol/L) was added to the

aqueous phase to adjust the pH to about 3. The mixture was extracted with EtOAc (20 mL×3). The organic phases were combined and dried over anhydrous $Na_2SO_4$, and concentrated under reduced pressure to give a yellow solid (99 mg, 70%).

MS (ESI, pos.ion) m/z: 384.2 $[M+H]^+$.

**Step 3: Synthesis of *N*-(4-(ethylsulfonyl)benzyl)-4-((2*S*, 4*R*)-2-(fluoromethyl)-4-(4-(trifluoromethyl)phenoxy)pyrrolidin-1-yl)benzamide**

4-((2*S*,4*R*)-2-(Fluoromethyl)-4-(4-(trifluoromethyl)phenoxy)pyrrolidin-1-yl)benzoic acid (43 mg, 0.11 mmol), (4-(ethylsulfonyl)phenyl)methanamine (30 mg, 0.15 mmol), EDCI (45 mg, 0.23 mmol) and HOBT (32 mg, 0.24 mmol) were dissolved in DCM (4 mL), then TEA (0.05 mL, 0.40 mmol) was added and the mixture was stirred at room temperature for 15 h. The reaction solution was diluted with DCM (80 mL), washed successively with HCl solution (20 mL, 1 mol/L), saturated NaCl solution (20 mL) and saturated $Na_2CO_3$ solution (20 mL), dried over anhydrous $Na_2SO_4$, and concentrated under reduced pressure. The crude product was separated by silica gel column chromatography (eluent: DCM/EtOAc (v/v) = 5/1) to give a yellow solid (51 mg, 81%).

MS (ESI, pos.ion) m/z: 565.1 $[M+H]^+$;

$^1$H NMR (600 MHz, $CDCl_3$) δ (ppm): 7.88 (d, *J* = 8.3 Hz, 2H), 7.75 (d, *J* = 8.8 Hz, 2H), 7.59 (d, *J* = 8.7 Hz, 2H), 7.55 (d, *J* = 8.2 Hz, 2H), 7.01 (d, *J* = 8.6 Hz, 2H), 6.93 (d, *J* = 8.9 Hz, 2H), 6.51 (t, *J* = 5.9 Hz, 1H), 5.10 (d, *J* = 46.8 Hz, 1H), 4.85 (m, 1H), 4.75 (d, *J* = 6.0 Hz, 2H), 3.95 (dd, *J* = 12.7, 3.5 Hz, 1H), 3.91 - 3.83 (m, 1H), 3.42 - 3.32 (m, 1H), 3.24 - 3.19 (m, 1H), 3.12 (q, *J* = 7.4 Hz, 2H), 2.55 (m, 1H), 2.09 - 1.99 (m, 1H), 1.30 (t, *J* = 7.4 Hz, 3H).

**Example 24 *N*-((*R*)-1-(4-(ethylsulfonyl)phenyl)-2-hydroxyethyl)-4-((2*S*,4*R*)-2-(fluoromethyl)-4-(4-(trifluoromethyl)phenoxy)pyrrolidin-1-yl)benzamide**

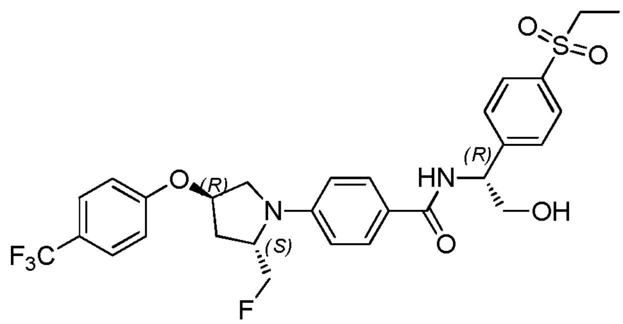

4-((2*S*,4*R*)-2-(Fluoromethyl)-4-(4-(trifluoromethyl)phenoxy)pyrrolidin-1-yl)benzoic acid (56 mg, 0.15 mmol), (*R*)-2-amino-2-(4-(ethylsulfonyl)phenyl)ethanol (43 mg, 0.19 mmol), EDCI (57 mg, 0.30 mmol) and HOBT (42 mg, 0.31 mmol) were dissolved in DCM (4 mL), then TEA (0.06 mL, 0.40 mmol) was added and the mixture was stirred at room temperature for 21 h. The reaction solution was diluted with DCM (80 mL), washed successively with HCl solution (20 mL, 1 mol/L), saturated $Na_2CO_3$ solution (20 mL) and saturated NaCl solution (20 mL), dried over anhydrous $Na_2SO_4$, and concentrated under reduced pressure. The crude product was separated by silica gel column chromatography (eluent: DCM/EtOAc (v/v) = 1/1) to give a pink solid (55 mg, 63%).

MS (ESI, pos.ion) m/z: 595.1 $[M+H]^+$;

$^1$H NMR (600 MHz, $CDCl_3$) δ (ppm): 7.89 (d, *J* = 8.3 Hz, 2H), 7.75 (d, *J* = 8.8 Hz, 2H), 7.58 (d, *J* = 8.4 Hz, 2H), 7.57 (d, *J* = 8.9 Hz, 2H), 6.99 (d, *J* = 8.6 Hz, 2H), 6.92 (d, *J* = 8.9 Hz, 2H), 6.89 (d, 1H), 5.33 - 5.29 (m, 1H), 5.08 (d, *J* = 46.6 Hz, 1H), 4.85 - 4.80 (m, 1H), 4.10 - 4.05 (m, 1H), 4.03 - 3.99 (m, 1H), 3.93 (dd, *J* = 12.7, 3.5 Hz, 1H), 3.88 - 3.83 (m, 1H), 3.35 (ddd, *J* = 31.2, 13.6, 1.8 Hz, 1H), 3.20 (dd, *J* = 12.7, 8.3 Hz, 1H), 3.10 (q, *J* = 7.4 Hz, 2H), 2.56 - 2.50 (m, 1H), 2.02 (m, 1H), 1.29 (t, *J* = 7.4 Hz, 3H).

**Example 25 *N*-(4-(ethylsulfonyl)benzyl)-4-((2*S*,4*R*)-2-(methoxymethyl)-4-(4 -(trifluoromethyl)phenoxy)pyrrolidin-1-yl)benzamide**

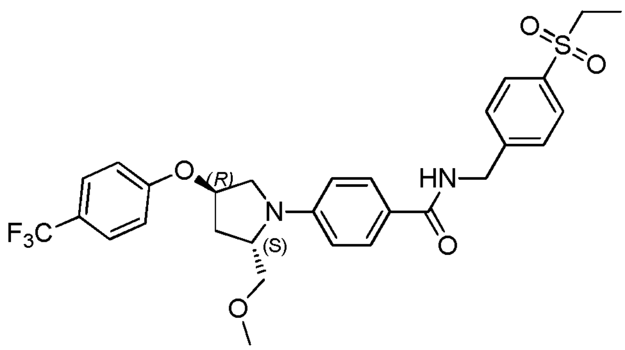

**Step 1: Synthesis of 4-((2*S*,4*R*)-2-(methoxymethyl)-4-(4-(trifluoromethyl) phenoxy)pyrrolidin-1-yl)benzonitrile**

Under nitrogen protection, to a solution of MeI (0.15 mL, 2.40 mmol) and 4-((2*S*,4*R*)-2-(hydroxymethyl)-4-(4-(trifluoromethyl)phenoxy)pyrrolidin-1-yl)benzonitrile (150 mg, 0.41 mmol) in THF (4 mL) was added NaH (50 mg, 1.25 mmol, 60%). The mixture was stirred at room temperature for 17 h. Saturated NaCl solution (20 mL) was added to the reaction solution to quench the reaction. The mixture was extracted with EtOAc (30 mL × 2), dried over anhydrous $Na_2SO_4$, filtered, and concentrated under reduced pressure. The crude product was separated by silica gel column chromatography (eluent: PE/EtOAc (v/v) = 4/1) to give yellow liquid (120 mg, 77%).

MS (ESI, pos.ion) m/z: 377.1 $[M+H]^+$.

**Step 2: Synthesis of 4-((2*S*,4*R*)-2-(methoxymethyl)-4-(4-(trifluoromethyl) phenoxy)pyrrolidin-1-yl)benzoic acid**

To a solution of 4-((2*S*,4*R*)-2-(methoxymethyl)-4-(4-(trifluoromethyl) phenoxy)pyrrolidin-1-yl)benzonitrile (400 mg, 1.06 mmol) in EtOH (10 mL) was added MeONa (5.70 g, 105.52 mmol). The mixture was heated and stirred in an oil bath at 100°C for 24 h. To the reaction solution was added $H_2O$ (15 mL) to quench the reaction. HCl solution (1 mol/L) was added to adjust pH to about 5. The mixture was concentrated under reduced pressure. The residue was diluted with saturated NaCl solution (20 mL), extracted with EtOAc (60 mL × 2), dried over anhydrous $Na_2SO_4$, and concentrated under reduced pressure. The crude product was separated by silica gel column chromatography (eluent:PE/EtOAc (v/v) = 3/1) to give a pale yellow solid (360 mg, 85%).

MS (ESI, pos.ion) m/z: 396.3 $[M+H]^+$.

**Step 3: Synthesis of *N*-(4-(ethylsulfonyl)benzyl)-4-((2*S*, 4*R*)-2-(methoxymethyl)-4-(4-(trifluoromethyl)phenoxy)pyrrolidin-1-yl)benzamide**

(4-(Ethylsulfonyl)phenyl)methanamine (90 mg, 0.45 mmol), 4-((2*S*,4*R*)-2-(methoxymethyl)-4-(4-(trifluoromethyl)phenoxy)pyrrolidin-1-yl)benzoic acid (100 mg , 0.25 mmol), HATU (216 mg, 0.57 mmol) and TEA (0.10 mL, 0.72 mmol) were dissolved in DCM (4 mL). The mixture was stirred at room temperature for 17 h. The reaction solution was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 4/1) to give a white solid (115 mg, 53%).

MS (ESI, pos.ion) m/z: 577.3 $[M+H]^+$.

$^1H$ NMR (400 MHz, $CDCl_3$) δ (ppm): 7.78 - 7.72 (m, 4H), 7.54 (d, *J* = 8.6 Hz, 2H), 7.45 (d, *J* = 8.1 Hz, 2H), 6.95 (d, *J* = 8.6 Hz, 2H), 6.88 (t, *J* = 5.7 Hz, 1H), 6.60 (d, *J* = 8.6 Hz, 2H), 5.23 - 5.16 (m, 1H), 4.67 (d, *J* = 6.0 Hz, 2H), 4.26 - 4.20 (m, 1H), 3.90 (dd, *J* = 10.4, 6.1 Hz, 1H), 3.54 (dd, *J* = 9.6, 4.8 Hz, 1H), 3.47 (dd, *J* = 10.3, 4.4 Hz, 2H), 3.32 (s, 3H), 3.06 (q, *J* = 7.4 Hz, 2H), 2.56 - 2.50 (m, 1H), 2.41 - 2.33 (m, 1H), 1.23 (t, *J* = 7.4 Hz, 3H).

**Example 26 *N*-((*R*)-1-(4-(ethylsulfonyl)phenyl)-2-hydroxyethyl)-4-((2*S*,4*R*)-2-(methoxymethyl)-4-(4-(trifluoromethyl)phenoxy)pyrrolidin-1-yl)benzamide**

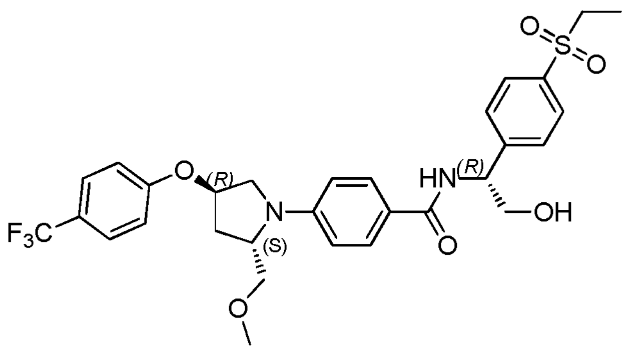

(*R*)-2-Amino-2-(4-(ethylsulfonyl)phenyl)ethanol (132 mg, 0.58 mmol), 4-((2*S*,4*R*)-2-(methoxymethyl)-4-(4-(trifluoromethyl)phenoxy)pyrrolidin-1-yl)benzoic acid (190 mg , 0.48 mmol), EDCI (138 mg, 0.72 mmol) and HOBT (97 mL, 0.72 mmol) were dissolved in DCM (4 mL). The mixture was stirred at room temperature for 17 h. The reaction solution was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (110 mg, 38%).

MS (ESI, pos.ion) m/z: 607.3 $[M+H]^+$.

$^1H$ NMR (400 MHz, $CDCl_3$) $\delta$ (ppm): 7.80 (d, *J* = 8.3 Hz, 2H), 7.72 (d, *J* = 8.8 Hz, 2H), 7.53 (t, *J* = 8.4 Hz, 4H), 7.10 (d, *J* = 6.9 Hz, 1H), 6.95 (d, *J* = 8.6 Hz, 2H), 6.59 (d, *J* = 8.8 Hz, 2H), 5.22 - 5.17 (m, 2H), 4.26 - 4.22 (m, 1H), 3.96 (dd, *J* = 11.5, 3.8 Hz, 1H), 3.89 (dd, *J* = 10.2, 6.0 Hz, 2H), 3.54 (dd, *J* = 9.6, 4.8 Hz, 1H), 3.48 (dd, *J* = 9.9, 3.1 Hz, 2H), 3.32 (s, 3H), 3.06 (q, *J* = 7.4 Hz, 2H), 2.56 - 2.50 (m, 1H), 2.41 - 2.33 (m, 1H), 1.24 (t, *J* = 7.4 Hz, 3H).

**Example 27 *N*-((*R*)-1-(4-(ethylsulfonyl)phenyl)-2-hydroxyethyl)-4-((2*S*,4*R*)-2-(propoxymethyl)-4-(4-(trifluoromethyl)phenoxy)pyrrolidin-1-yl)benzamide**

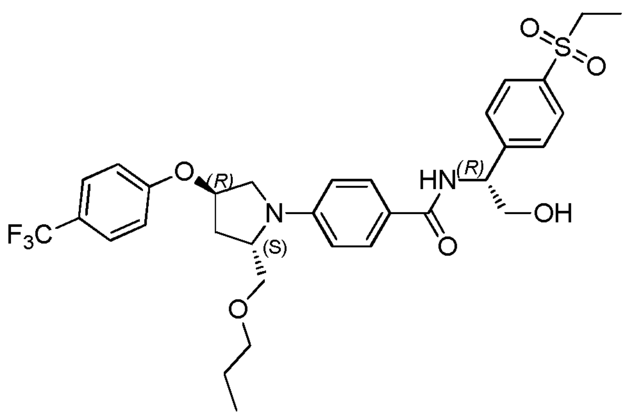

**Step 1: Synthesis of ethyl 4-((2*S*,4*R*)-2-(propoxymethyl)-4-(4-(trifluoromethyl) phenoxy)pyrrolidin-1-yl)benzoate**

Under nitrogen protection, to a solution of 1-bromopropane (0.50 mL, 4.66 mmol) and ethyl 4-((2*S*,4*R*)-2-(hydroxymethyl)-4-(4-(trifluoromethyl)phenoxy)pyrrolidin-1-yl)benzoate (200 mg, 0.49 mmol) in THF (6 mL) was added NaH (195 mg, 4.88 mmol, 60%). The mixture was stirred at room temperature for 17 h. The reaction solution was added with HCl solution (1 mol/L) to quench the reaction, and the pH of the solution was adjusted to about 5. The resulting mixture was diluted with EtOAc (50 mL), extracted and separated, and the organic phase was washed with saturated NaCl (20 mL) solution, dried over anhydrous $Na_2SO_4$, concentrated under reduced pressure. The crude product was separated by silica gel column chromatography (eluent: PE/EtOAc (v/v) = 4/1) to give yellow liquid (160 mg, 72%).

MS (ESI, pos.ion) m/z: 452.3 $[M+H]^+$.

**Step 2: Synthesis of 4-((2*S*,4*R*)-2-(propoxymethyl)-4-(4-(trifluoromethyl) phenoxy)pyrrolidin-1-yl)benzoic acid**

To a solution of ethyl 4-((2*S*,4*R*)-2-(propoxymethyl)-4-(4-(trifluoromethyl) phenoxy)pyrrolidin-1-yl)benzoate (160 mg, 0.35 mmol) in MeOH (6 mL) was added a solution of $LiOH{\cdot}H_2O$ (442 mg, 10.53 mmol) in $H_2O$ (3 mL). The mixture was reacted at room temperature for 23 h. The reaction solution was added with HCl solution (1 mol/L) to adjust the pH of the solution to about 5. The resulting mixture was extracted with EtOAc (30 mL $\times$ 2). The organic phases were combined, washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give a yellow solid (70 mg, 47%).

MS (ESI, pos.ion) m/z: 424.3 $[M+H]^+$.

**Step 3: Synthesis of *N*-((*R*)-1-(4-(ethylsulfonyl)phenyl)-2-hydroxyethyl) -4-((2*S*,4*R*)-2-(propoxymethyl)-4-(4-(trifluoromethyl)phenoxy)pyrrolidin-1-yl)benzamide**

(*R*)-2-Amino-2-(4-(ethylsulfonyl)phenyl)ethanol (50 mg, 0.22 mmol), 4-((2*S*,4*R*)-2-(propoxymethyl)-4-(4-(trifluoromethyl)phenoxy)pyrrolidin-1-yl)benzoic acid (70 mg, 0.17 mmol), HATU (95 mg, 0.25 mmol) and DIPEA(64 mg, 0.50 mmol) were dissolved in DCM (4 mL). The mixture was stirred at room temperature for 16 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (40 mg, 38%).

MS (ESI, pos.ion) m/z: 635.1 $[M+H]^+$.

$^1H$ NMR (400 MHz, $CDCl_3$) $\delta$ (ppm): 7.83 (d, *J* = 7.8 Hz, 2H), 7.73 (d, *J* = 8.5 Hz, 2H), 7.55 (d, *J* = 8.3 Hz, 4H), 7.03 (d, *J* = 6.9 Hz, 1H), 6.96 (d, *J* = 8.5 Hz, 2H), 6.60 (d, *J* = 8.5 Hz, 2H), 5.27 - 5.20 (m, 2H), 4.25 (s, 1H), 4.01 - 3.88 (m, 3H), 3.57 (dd, *J* = 9.4, 4.6 Hz, 1H), 3.55 - 3.45 (m, 2H), 3.40 - 3.29 (m, 2H), 3.07 (q, *J* = 7.3 Hz, 2H), 2.59 - 2.52 (m, 1H), 2.42 - 2.32 (m, 1H), 1.60 - 1.51 (m, 2H), 1.26 (t, *J* = 7.3 Hz, 3H), 0.90 (t, *J* = 7.4 Hz, 3H).

**Example 28 4-((2*S*,4*R*)-2-(butoxymethyl)-4-(4-trifluoromethyl)phenoxy)pyrrolidin -1-yl)-*N*-((*R*)-1-(4-(ethylsulfonyl)phenyl)-2-hydroxyethyl)benzamide**

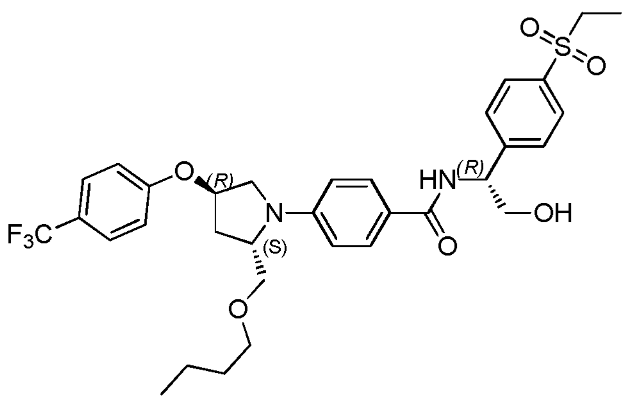

**Step 1: Synthesis of ethyl 4-((2*S*,4*R*)-2-(butoxymethyl)-4-(4-(trifluoromethyl) phenoxy)pyrrolidin-1-yl)benzoate**

Under nitrogen protection, to a solution of 1-bromobutane (0.50 mL, 4.66 mmol) and ethyl 4-((2*S*,4*R*)-2-(hydroxymethyl)-4-(4-(trifluoromethyl)phenoxy)pyrrolidin-1-yl)benzoate (200 mg, 0.49 mmol) in THF (6 mL) was added NaH (98 mg, 2.45 mmol, 60%). The mixture was stirred at room temperature for 17 h. The reaction solution was added with HCl solution (1 mol/L) to quench the reaction, and the pH of the solution was adjusted to about 5. The resulting mixture was diluted with EtOAc (50 mL), extracted and separated. The organic phase was washed with saturated NaCl (20 mL) solution, dried over anhydrous $Na_2SO_4$, and concentrated under reduced pressure. The crude product was separated by silica gel column chromatography (eluent: PE/EtOAc (v/v) = 4/1) to give yellow liquid (160 mg, 70%).

MS (ESI, pos.ion) m/z: 466.1 $[M+H]^+$.

**Step 2: Synthesis of 4-((2*S*,4*R*)-2-(butoxymethyl)-4-(4-(trifluoromethyl) phenoxy)pyrrolidin-1-yl)benzoic acid**

To a solution of ethyl 4-((2*S*,4*R*)-2-(butoxymethyl)-4-(4-(trifluoromethyl) phenoxy)pyrrolidin-1-yl)benzoate (160 mg, 0.34 mmol) in MeOH (6 mL) was added a solution of $LiOH{\cdot}H_2O$ (442 mg, 10.53 mmol) in $H_2O$ (3 mL). The mixture was reacted at room temperature for 23 h. The reaction solution was added with HCl solution (1 mol/L) to adjust the pH of the solution to about 5. The resulting mixture was extracted with EtOAc (30 mL × 2). The organic phases were combined, washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give a yellow solid (70 mg, 47%).

MS (ESI, pos.ion) m/z: 438.3 $[M+H]^+$.

**Step 3: Synthesis of 4-((2*S*,4*R*)-2-(butoxymethyl)-4-(4-(trifluoromethyl)phenoxy) pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (50 mg, 0.22 mmol), 4-((2*S*,4*R*)-2-(butoxymethyl)-4-(4-(trifluoromethyl)phenoxy)pyrrolidin-1-yl)benzoic acid (70 mg, 0.16 mmol), HATU (95 mg, 0.25 mmol) and DIPEA(64 mg, 0.50 mmol) were dissolved in DCM (4 mL). The mixture was stirred at room temperature for 16 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (35 mg, 34%).

MS (ESI, pos.ion) m/z: 649.2 $[M+H]^+$.

$^1H$ NMR (400 MHz, $CDCl_3$) δ (ppm): 7.83 (d, *J* = 8.3 Hz, 2H), 7.73 (d, *J* = 8.7 Hz, 2H), 7.55 (d, *J* = 7.4 Hz, 4H), 7.03 (d, *J* = 7.0 Hz, 1H), 6.95 (d, *J* = 8.6 Hz, 2H), 6.60 (d, *J*= 8.8 Hz, 2H), 5.29 - 5.21 (m, 2H), 4.24 (d, *J*= 3.4 Hz, 1H), 4.00 (dd, *J*= 11.3, 3.8 Hz, 1H), 3.96 - 3.86 (m, 2H), 3.57 (dd, *J* = 9.7, 4.6 Hz, 1H), 3.52 (dd, *J* = 9.7, 4.6 Hz, 1H), 3.48 (dd, *J* = 10.5, 4.1 Hz, 1H), 3.44 - 3.33 (m, 2H), 3.07 (q, *J* = 7.4 Hz, 2H), 2.58 - 2.52 (m, 1H), 2.41 - 2.33 (m, 1H), 1.55 - 1.48 (m, 2H), 1.37 - 1.31 (m, 2H), 1.26 (t, *J*= 7.4 Hz, 6H), 0.90 (t, *J*= 7.4 Hz, 3H).

**Example 29 *N*-((*R*)-1-(4-(ethylsulfonyl)phenyl)-2-hydroxyethyl)-4-((2*S*,4*R*)-2-((2-fluoroethoxy)methyl)-4-(4-(trifluoromethyl)phenoxy)pyrrolidin-1-yl)benzamide**

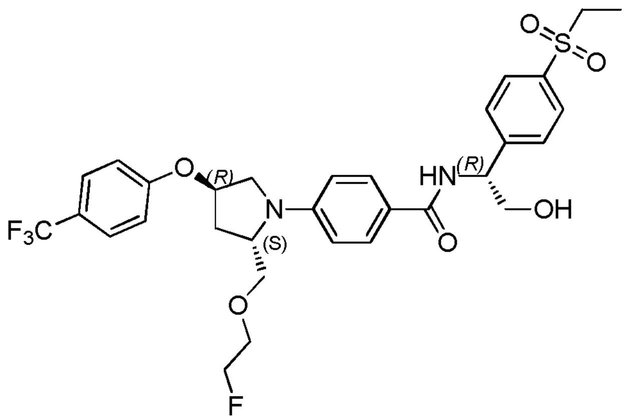

**Step 1: Synthesis of ethyl 4-((2*S*,4*R*)-2-((2-fluoroethoxy)methyl)-4-(4-(trifluoromethyl)phenoxy)pyrrolidin-1-yl)benzoate**

Under nitrogen protection, to a solution of 1-bromo-2-fluoroethane (2.00 mL, 26.77 mmol) and ethyl 4-((2*S*,4*R*)-2-(hydroxymethyl)-4-(4-(trifluoromethyl)phenoxy)pyrrolidin-1-yl) benzoate (450 mg, 1.10 mmol) in THF (8 mL) was added NaH (272 mg, 6.80 mmol, 60%). The mixture was stirred at room temperature for 17 h. The reaction solution was added with HCl solution (1 mol/L) to quench the reaction, and the pH of the solution was adjusted to about 5. The resulting mixture was diluted with EtOAc (50 mL), extracted and separated. The organic phase was washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give yellow liquid (240 mg, 48%).

MS (ESI, pos.ion) m/z: 456.3 $[M+H]^+$.

**Step 2: Synthesis of 4-((2*S*,4*R*)-2-((2-fluoroethoxy)methyl)-4-(4-(trifluoromethyl) phenoxy)pyrrolidin-1-yl)benzoic acid**

To a solution of ethyl 4-((2*S*,4*R*)-2-((2-fluoroethoxy)methyl)-4-(4-(trifluoromethyl) phenoxy)pyrrolidin-1-yl)benzoate (240 mg, 0.53 mmol) in MeOH (6 mL) was added a solution of $LiOH{\cdot}H_2O$ (442 mg, 10.53 mmol) in $H_2O$ (3 mL). The mixture was reacted at room temperature for 14 h. The reaction solution was added with HCl solution (1 mol/L) to adjust the pH of the solution to about 5. The resulting mixture was extracted with EtOAc (30 mL $\times$ 2). The organic phases were combined, washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give a yellow solid (150 mg, 67%).

MS (ESI, pos.ion) m/z: 428.1 $[M+H]^+$.

**Step 3: Synthesis of *N*-((*R*)-1-(4-(ethylsulfonyl)phenyl)-2-hydroxyethyl)-4-((2*S*,4*R*)-2-((2-fluoroethoxy)methyl)-4-(4-(trifluoromethyl)phenoxy)pyrrolidin-1-yl)benzamide**

(*R*)-2-Amino-2-(4-(ethylsulfonyl)phenyl)ethanol (96 mg, 0.42 mmol), 4-((2*S*,4*R*)-2-((2-fluoroethoxy)methyl)-4-(4-(trifluoromethyl)phenoxy)pyrrolidin-1-yl)benzoic acid (150 mg, 0.35 mmol), EDCI (100 mg, 0.52 mmol) and HOBT (71 mL, 0.53 mmol) were dissolved in DCM (4 mL). The mixture was stirred at room temperature for 17 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (90 mg, 40%).

MS (ESI, pos.ion) m/z: 639.1 $[M+H]^+$.

$^1H$ NMR (400 MHz, $CDCl_3$) $\delta$ (ppm): 7.84 (d, *J*= 7.9 Hz, 2H), 7.77 (d, *J*= 8.3 Hz, 2H), 7.59 - 7.54 (m, 4H), 7.15 (d, *J* = 6.5 Hz, 1H), 6.99 (d, *J* = 8.5 Hz, 2H), 6.65 (d, *J* = 8.3 Hz, 2H), 5.30 - 5.23 (m, 2H), 4.58 (s, 1H), 4.46 (s, 1H), 4.29 (d, *J*= 3.6 Hz, 1H), 4.04 - 3.98 (m, 1H), 3.95 (dd, *J* = 9.8, 6.0 Hz, 2H), 3.75 - 3.68 (m, 2H), 3.68 - 3.61 (m, 2H), 3.50 (dd, *J* = 10.3, 3.7 Hz, 1H), 3.09 (q, *J*= 7.4 Hz, 2H), 2.63 - 2.57 (m, 1H), 2.45 - 2.37 (m, 1H), 1.27 (t, *J*= 7.4 Hz, 3H).

**Example 30 *N*-((*R*)-1-(4-(ethylsulfonyl)phenyl)-2-hydroxyethyl)-4-((2*S*,4*R*)-2-((2,2,2-trifluoroethoxy)methyl)-4-(4-(trifluoromethyl)phenoxy)pyrrolidin-1-yl)benzamide**

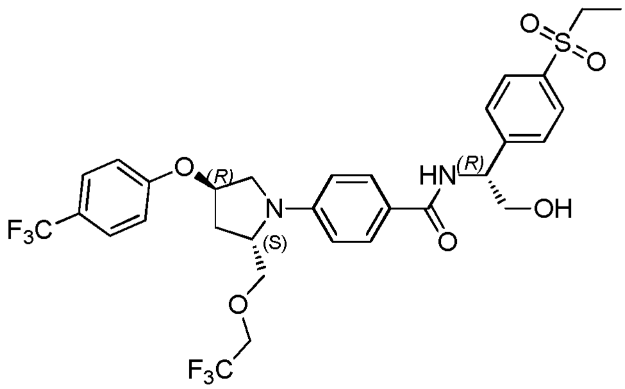

**Step 1: Synthesis of ((2*S*,4*R*)-1-(4-cyanophenyl)-4-(4-(trifluoromethyl) phenoxy)pyrrolidin-2-yl)methyl methanesulfonate**

Under nitrogen protection, to a solution of 4-((2*S*,4*R*)-2-(hydroxymethyl)-4-(4-(trifluoromethyl)phenoxy)pyrrolidin-1-yl)benzonitrile (200 mg, 0.55 mmol) and TEA (0.20 mL, 1.43 mmol) in DCM (6 mL) was added MsCl (0.10 mL, 1.29 mmol). The mixture was stirred at room temperature for 24 h. Saturated NaCl solution (20 mL) was added to the reaction solution to quench the reaction. The mixture was diluted with DCM (30 mL), extracted and separated. The organic phase was dried over anhydrous $Na_2SO_4$, filtered, and concentrated under reduced pressure. The crude product was separated by silica gel column chromatography (eluent: PE/EtOAc (v/v) = 3/1) to give yellow liquid (160 mg, 66%).

MS (ESI, pos.ion) m/z: 441.3 $[M+H]^+$.

**Step 2: Synthesis of 4-((2*S*,4*R*)-2-((2,2,2-trifluoroethoxy)methyl)-4-(4-(trifluoromethyl)phenoxy)pyrrolidin-1-yl)benzonitrile**

To a solution of ((2*S*,4*R*)-1-(4-cyanophenyl)-4-(4-(trifluoromethyl) phenoxy)pyrrolidin-2-yl)methyl methanesulfonate (60 mg, 0.14 mmol) in ACN (4 mL) were added $Cs_2CO_3$ (177 mg, 0.54 mmol) and 3,3,3-trifluoropropyl-1-ol (160 mg, 1.60 mmol). The reaction solution was heated and stirred in an 80°C oil bath for 17 h. The reaction solution was cooled to room temperature and filtered, the filtrate was concentrated under reduced pressure, and the crude product was separated by silica gel column chromatography (eluent: PE/EtOAc (v/v) = 4/1) to give a yellow solid (46 mg, 76%).

MS (ESI, pos.ion) m/z: 445.2 $[M+H]^+$.

**Step 3: Synthesis of 4-((2*S*,4*R*)-2-((2,2,2-trifluoroethoxy)methyl)-4-(4-(trifluoromethyl) phenoxy)pyrrolidin-1-yl)benzoic acid**

To a solution of 4-((2*S*,4*R*)-2-((2,2,2-trifluoroethoxy)methyl)-4-(4-(trifluoromethyl)phenoxy)pyrrolidin-1-yl)benzonitrile (400 mg, 1.06 mmol) in EtOH (5 mL) was added MeONa (1.20 g, 22.21 mmol), the mixture was heated and stirred in an oil bath at 100°C for 18 h. The reaction solution was cooled to room temperature, HCl solution (1 mol/L) was added to the reaction solution to quench the reaction, and the pH of the solution was adjusted to about 5. The resulting mixture was extracted with EtOAc (30 mL × 2). The organic phase was washed with saturated NaCl (20 mL) solution, dried over anhydrous $Na_2SO_4$, and concentrated under reduced pressure. The crude product was separated by silica gel column chromatography (eluent: PE/EtOAc (v/v) = 1/1) to give a pale yellow solid (80 mg, 77%).

MS (ESI, pos.ion) m/z: 464.2 $[M+H]^+$.

**Step 4: Synthesis of *N*-((*R*)-1-(4-(ethylsulfonyl)phenyl)-2-hydroxyethyl)-4-((2*S*,4*R*) -2-((2,2,2-trifluoroethoxy)methyl)-4-(4-(trifluoromethyl)phenoxy)pyrrolidin-1-yl)benzamide**

(*R*)-2-Amino-2-(4-(ethylsulfonyl)phenyl)ethanol (55 mg, 0.24 mmol), 4-((2*S*,4*R*)-2-((2,2,2-trifluoroethoxy)methyl)-4-(4-(trifluoromethyl)phenoxy)pyrrolidin-1-yl)benzoic acid (80 mg , 0.17 mmol), EDCI (49 mg, 0.26 mmol) and HOBT (35 mL, 0.26 mmol) were dissolved in DCM (4 mL). The mixture was stirred at room temperature for 13 h. The reaction solution was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (76 mg, 65%).

MS (ESI, pos.ion) m/z: 675.3 $[M+H]^+$.

$^1H$ NMR (400 MHz, $CDCl_3$) δ (ppm): 7.84 (d, *J* = 8.4 Hz, 2H), 7.76 (d, *J* = 8.7 Hz, 2H), 7.57 (t, *J* = 7.4 Hz, 4H), 7.08 (d, *J* = 7.0 Hz, 1H), 6.97 (d, *J* = 8.4 Hz, 2H), 6.61 (d, *J* = 8.7 Hz, 2H), 5.32 - 5.21 (m, 2H), 4.34 - 4.27 (m, 1H), 4.01 (dd, *J* = 11.3, 3.8 Hz, 1H), 3.97 - 3.90 (m, 2H), 3.88 - 3.72 (m, 4H), 3.52 (dd, *J* = 10.3, 3.7 Hz, 1H), 3.09 (q, *J* = 7.4 Hz, 2H), 2.62 - 2.56 (m, 1H), 2.48 - 2.39 (m, 1H), 1.28 (t, *J* = 7.4 Hz, 3H).

**Example 31 4-((2*S*,4*R*)-2-((cyclopropylmethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

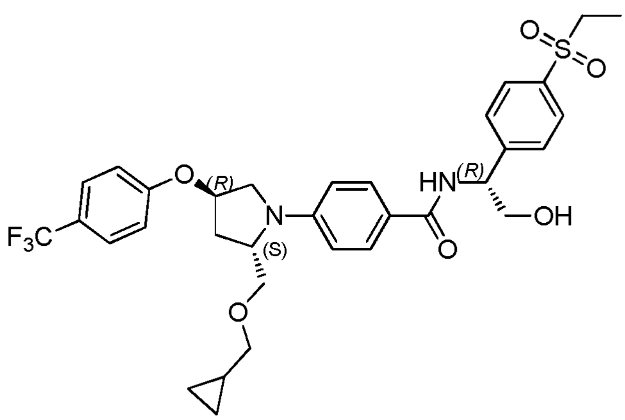

**Step 1: Synthesis of 4-((2*S*,4*R*)-2-((cyclopropylmethoxy)methyl)-4-(4-(trifluoromethyl) phenoxy)pyrrolidin-1-yl)benzonitrile**

Under nitrogen protection, to a solution of 4-((2*S*,4*R*)-2-(hydroxymethyl)-4-(4-(trifluoromethyl)phenoxy)pyrrolidin-1-yl)benzonitrile (300 mg, 0.83 mmol) and (bromomethyl)cyclopropane (0.40 mL, 4.12 mmol) in THF (8 mL) was added NaH (100 mg, 2.50 mmol, 60%). The mixture was stirred at room temperature for 20 h. Saturated NaCl solution (20 mL) was added to the reaction solution to quench the reaction. The mixture was extracted with EtOAc (30 mL × 2), dried over anhydrous $Na_2SO_4$, concentrated under reduced pressure, and the crude product was separated by silica gel column chromatography (eluent: PE/EtOAc (v/v) = 4/1) to give yellow liquid (178 mg, 52%).
MS (ESI, pos.ion) m/z: 416.9 $[M+H]^+$.

**Step** 2: **Synthesis of 4-((2*S*,4*R*)-2-((cyclopropylmethoxy)methyl)-4-(4-(trifluoromethyl) phenoxy)pyrrolidin-1-yl)benzoic acid**

To a solution of 4-((2*S*,4*R*)-2-((cyclopropylmethoxy)methyl)-4-(4-(trifluoromethyl) phenoxy)pyrrolidin-1-yl)benzonitrile (400 mg, 1.06 mmol) in EtOH (8 mL) was added MeONa (2.20 g, 40.73 mmol), the mixture was heated and stirred in an oil bath at 100 °C for 16 h. The reaction solution was cooled to room temperature, then $H_2O$ (15 mL) was added to quench the reaction, and HCl solution (12 mol/L) was added to adjust pH to about 5. The mixture was concentrated under reduced pressure. The residue was diluted with saturated NaCl solution (20 mL), and extracted with EtOAc (60 mL × 2). The organic phases were combined and dried over anhydrous $Na_2SO_4$ and concentrated under reduced pressure. The crude product was separated by silica gel column chromatography (eluent: PE/EtOAc (v/v) = 3/1) to give a pale yellow solid (160 mg, 85%).
MS (ESI, pos.ion) m/z: 436.3 $[M+H]^+$.

**Step 3: Synthesis of 4-((2*S*,4*R*)-2-((cyclopropylmethoxy)methyl)-4-(4-(trifluoromethyl) phenoxy)pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (84 mg, 0.37 mmol), 4-((2*S*,4*R*)-2-((cyclopropylmethoxy)methyl)-4-(4-(trifluoromethyl)phenoxy)pyrrolidin-1-yl)benzoic acid (160 mg , 0.37 mmol), EDCI (105 mg, 0.55 mmol) and HOBT (74 mg, 0.55 mmol) were dissolved in DCM (4 mL). The mixture was stirred at room temperature for 22 h. The reaction solution was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 2/1) to give a white solid (100 mg, 42%).
MS (ESI, pos.ion) m/z: 647.0 $[M+H]^+$.
$^1H$ NMR (400 MHz, $CDCl_3$) $\delta$ (ppm): 7.82 (d, *J* = 8.3 Hz, 2H), 7.73 (d, *J* = 8.8 Hz, 2H), 7.56 (d, *J* = 8.3 Hz, 2H), 7.54 (d, *J* = 8.6 Hz, 2H), 7.04 (d, *J* = 6.9 Hz, 1H), 6.97 (d, *J* = 8.6 Hz, 2H), 6.61 (d, *J*= 8.8 Hz, 2H), 5.27 - 5.20 (m, 2H), 4.28 - 4.23 (m, 1H), 3.99 (dd, *J*= 11.3, 3.9 Hz, 1H), 3.95 - 3.88 (m, 2H), 3.58 (d, *J* = 3.8 Hz, 2H), 3.48 (dd, *J* = 10.4, 4.1 Hz, 1H), 3.25 (d, *J* = 6.8 Hz, 2H), 3.07 (q, *J* = 7.4 Hz, 2H), 2.59 - 2.54 (m, 1H), 2.42 - 2.34 (m, 1H), 1.26 (t, *J* = 7.4 Hz, 3H), 1.03 - 0.96 (m, 1H), 0.53 - 0.48 (m, 2H), 0.19-1.15 (m, 2H).

**Example 32 4-((2*S*,4*R*)-2-((2-cyanoethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

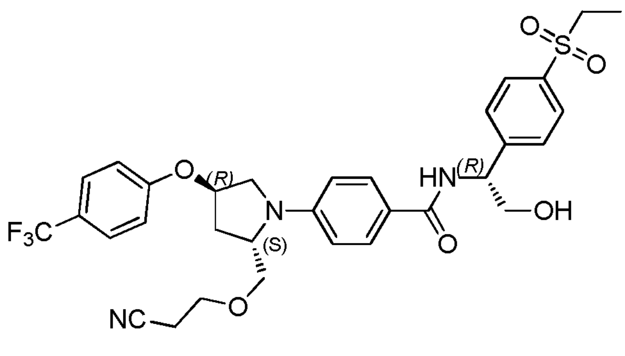

**Step 1: Synthesis of 4-((2*S*,4*R*)-2-((2-cyanoethoxy)methyl)-4-(4-(trifluoromethyl) phenoxy)pyrrolidin-1-yl)benzoic acid**

Under nitrogen protection, to a solution of 3-bromopropionitrile (0.30 mL, 3.62 mmol) and 4-((2*S*,4*R*)-2-(hydroxymethyl)-4-(4-(trifluoromethyl)phenoxy)pyrrolidin-1-yl)benzoic acid (80 mg, 0.21 mmol) in THF (6 mL) was added NaH (60 mg, 1.50 mmol, 60%). The mixture was stirred at room temperature for 24 h. To the reaction solution was added HCl solution (1 mol/L) to quench the reaction, and the pH of the solution was adjusted to about 5. The resulting mixture was diluted with EtOAc (30 mL), extracted and separated. The organic phase was washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (25 mg, 27%).
MS (ESI, pos.ion) m/z: 435.1 $[M+H]^+$.

**Step 2: Synthesis of 4-((2*S*,4*R*)-2-((2-cyanoethoxy)methyl)-4-(4-(trifluoromethyl) phenoxy)pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (15 mg, 0.07 mmol), 4-((2*S*,4*R*)-2-((2-cyanoethoxy)methyl)-4-(4-trifluoromethyl)phenoxy)pyrrolidin-1-yl)benzoic acid (25 mg , 0.06 mmol), EDCI (16 mg, 0.08 mmol), HOBT (11 mg, 0.08 mmol) and TEA (11 mg, 0.11 mmol) were dissolved in DCM (2 mL), and the mixture was stirred at room temperature for 10 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (10 mg, 27%).
MS (ESI, pos.ion) m/z: 646.1 $[M+H]^+$.
$^1H$ NMR (400 MHz, $CDCl_3$) δ (ppm): 7.86 (d, *J* = 7.3 Hz, 2H), 7.75 (d, *J* = 8.8 Hz, 2H), 7.57 (d,, *J* = 7.3 Hz, 2H), 7.54 (d, *J* = 8.8 Hz, 2H), 6.99 (d, *J* = 8.5 Hz, 3H), 6.62 (d, *J* = 8.7 Hz, 2H), 5.31 - 5.26 (m, 2H), 4.31 (s, 1H), 4.02 (s, 1H), 3.97 (dd, *J* = 10.2, 6.1 Hz, 2H), 3.75 (dd, *J* = 9.7, 3.8 Hz, 1H), 3.66 - 3.63 (m, 2H), 3.62 - 3.56 (m, 2H), 3.51 (dd, *J* = 10.4, 3.7 Hz, 1H), 3.09 (q, *J* = 7.4 Hz, 2H), 2.63 - 2.58 (m, 1H), 2.58 - 2.54 (m, 2H), 2.46 - 2.40 (m, 1H), 1.28 (t, *J* = 7.4 Hz, 3H).

**Example 33 *N*-((*R*)-1-(4-(ethylsulfonyl)phenyl)-2-hydroxyethyl)-4-((2*S*,4*R*)-2-((((S) -tetrahydrofuran-3-yl)oxy)methyl)-4-(4-(trifluoromethyl)phenoxy)pyrrolidin-1-yl)benzamide**

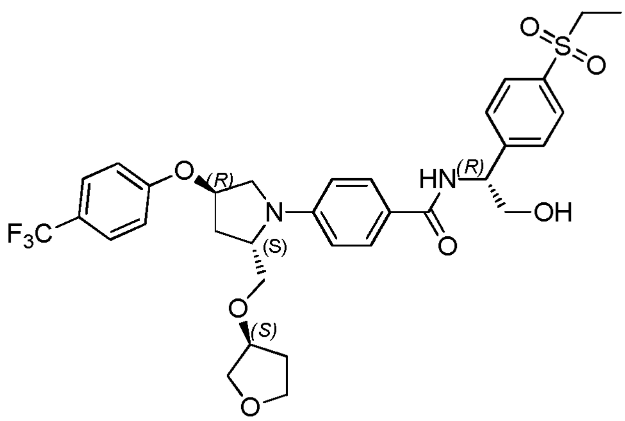

**Step 1: Synthesis of 4-((2*S*,4*R*)-2-((((*S*)-tetrahydrofuran-3-yl)oxy)methyl)-4-(4-(trifluoromethyl)phenoxy)pyrrolidin-1-yl)benzoic acid**

Under nitrogen protection, to a solution of (R)-tetrahydrofuran-3-yl mesylate (420 mg, 2.53 mmol) and ethyl 4-((2*S*,4*R*)-2-(hydroxymethyl)-4-(4-(trifluoromethyl)phenoxy) pyrrolidin-1-yl)benzoate (200 mg, 0.49 mmol) in THF (6 mL) was added NaH (80 mg, 2.00 mmol, 60%). The mixture was stirred at room temperature for 23 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL×3), dried over anhydrous $Na_2SO_4$, and concentrated under reduced pressure. The crude product was separated by silica gel column chromatography (eluent: DCM/MeOH (v/v) = 20/1) to give yellow liquid (50 mg, 23%).

MS (ESI, pos.ion) m/z: 452.1 $[M+H]^+$.

**Step 2: Synthesis of *N*-((*R*)-1-(4-(ethylsulfonyl)phenyl)-2-hydroxyethyl)-4-((2*S*,4*R*)-2-((((*S*) -tetrahydrofuran-3-yl)oxy)methyl)-4-(4-(trifluoromethyl)phenoxy)pyrrolidin-1-yl)benzamide**

(*R*)-2-Amino-2-(4-(ethylsulfonyl)phenyl)ethanol (30 mg, 0.13 mmol), 4-((2*S*,4*R*)-2-((((*S*)-tetrahydrofuran-3-yl)oxy)methyl)-4-(4-trifluoromethyl)phenoxy)pyrrolidin-1-yl) benzoic acid (50 mg , 0.11 mmol), EDCI (31 mg, 0.16 mmol), HOBT (22 mg, 0.16 mmol) and TEA (22 mg, 0.22 mmol) were dissolved in DCM (4 mL), and the mixture was stirred at room temperature for 9 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (25 mg, 34%).

MS (ESI, pos.ion) m/z: 663.4 $[M+H]^+$.

$^1H$ NMR (400 MHz, $CDCl_3$) $\delta$ (ppm): 7.88 (d, *J* = 8.2 Hz, 2H), 7.76 (d, *J* = 8.7 Hz, 2H), 7.59 (d, *J* = 8.2 Hz, 2H), 7.58 (d, *J* = 8.7 Hz, 2H), 7.02 (d, *J* = 6.9 Hz, 1H), 6.97 (d, *J* = 8.6 Hz, 2H), 6.63 (d, *J* = 8.8 Hz, 2H), 5.29 (d, *J* = 6.1 Hz, 1H), 5.26 - 5.19 (m, 1H), 4.29 (d, *J* = 3.3 Hz, 1H), 4.07 - 4.03 (m, 2H), 3.98 (dd, *J* = 11.3, 4.9 Hz, 1H), 3.90 (dd, *J* = 10.4, 6.2 Hz, 1H), 3.84 - 3.73 (m, 4H), 3.67 (dd, *J* = 9.4, 4.4 Hz, 1H), 3.55 - 3.49 (m, 2H), 3.10 (t, *J* = 7.4 Hz, 2H), 2.59 - 2.53 (m, 1H), 2.45 - 2.37 (m, 1H), 1.99 - 1.83 (m, 2H), 1.30 (t, *J*= 7.4 Hz, 3H).

**Example 34 *N*-((*R*)-1-(4-(ethylsulfonyl)phenyl)-2-hydroxyethyl)-4-((2*S*,4*R*) -2-(((tetrahydro-2*H*-pyran-4-yl)oxy)methyl)-4-(4-(trifluoromethyl)phenoxy)pyrrolidin-1yl)be nzamide**

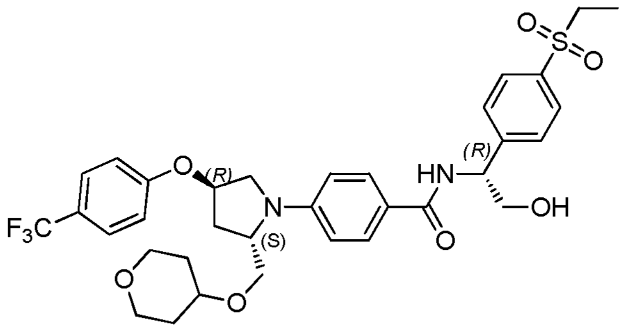

Using tetrahydro-2*H*-pyran-4-yl mesylate (600 mg, 3.33 mmol) as starting material, the title compound was prepared as a white solid (12 mg, 28%) by referring to the synthetic method of Example 33.

MS (ESI, pos.ion) m/z: 667.2 $[M+H]^+$.

$^1H$ NMR (600 MHz, $CDCl_3$) $\delta$ (ppm): 7.86 (d, *J* = 7.5 Hz, 2H), 7.74 (d, *J* = 8.7 Hz, 2H), 7.57 (d, *J* = 7.5 Hz, 2H), 7.56 (d, *J* = 8.7 Hz, 2H), 6.98 (s, 1H), 6.95 (d, *J* = 8.6 Hz, 2H), 6.61 (d, *J* = 8.6 Hz, 2H), 5.28 (s, 1H), 5.25 - 5.21 (m, 1H), 4.28 (d, *J* = 3.5 Hz, 1H), 4.05 - 4.01 (m, 1H), 3.99 - 3.95 (m, 2H), 3.92 - 3.86 (m, 2H), 3.84 - 3.80 (m, 1H), 3.67 - 3.62 (m, 2H), 3.58 (dd, *J*= 9.5, 2.2 Hz, 1H), 3.50 (dd, *J* = 10.4, 3.7 Hz, 1H), 3.45 - 3.38 (m, 3H), 3.39 - 3.36 (m, 1H), 3.08 (q, *J* = 7.3 Hz, 2H), 2.57 - 2.53 (m, 1H), 2.44 - 2.39 (m, 1H), 2.05 - 1.99 (m, 1H), 1.87 (d, *J*= 13.0 Hz, 1H), 1.27 (s, 3H).

**Example 35 *N*-((*R*)-1-(4-(ethylsulfonyl)phenyl)-2-hydroxyethyl)-4-((2*S*,4*R*)-2-(((*S*) -3-methylmorpholinyl)methyl)-4-(4-(trifluoromethyl)phenoxy)pyrrolidin-1-yl)benzamide**

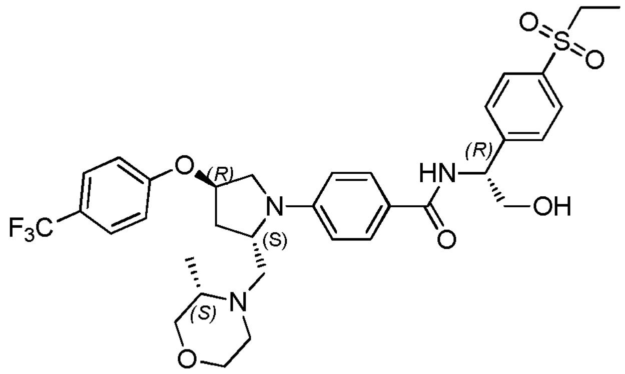

**Step** 1: **Synthesis of *tert*-butyl (2*S*,4*R*)-2-((*S*)-3-methylmorpholine-4-carbonyl) -4-(4-(trifluoromethyl)phenoxy)pyrrolidine-1-carboxylate**

(2*S*,4*R*)-1-(*tert*-butoxycarbonyl)-4-(4-(trifluoromethyl)phenoxy)pyrrolidin-2-carboxyli c acid (800 mg, 2.13 mmol), (3*S*)-3-methylmorpholine (323 mg, 3.20 mmol), HATU (1.22 g, 3.21 mmol) and TEA (0.90 mL, 6.46 mmol) were dissolved in DCM (16 mL), and the mixture was stirred at room temperature for 16 h. The reaction solution was concentrated under reduced pressure, and the crude product was separated by silica gel column chromatography (eluent: PE/EtOAc (v/v) = 3/2) to give colorless liquid (851 mg, 87%).

MS (ESI, pos.ion) m/z: 459.3 $[M+H]^+$.

**Step 2: Synthesis of *tert*-butyl (2*S*,4*R*)-2-(((*S*)-3-methylmorpholinyl)methyl) -4-(4-(trifluoromethyl)phenoxy)pyrrolidine-1-carboxylate**

To a solution of *tert*-butyl (2*S*,4*R*)-2-((*S*)-3-methylmorpholine-4-carbonyl) -4-(4-(trifluoromethyl)phenoxy)pyrrolidine-1-carboxylate (800 mg, 1.75 mmol) in THF (12 mL) was added $BH_3 \cdot SMe_2$ (0.50 mL, 5.00 mmol, 1.0 mol/L) dropwise under an ice bath. After the addition was completed, the mixture was stirred at room temperature for 17 h. MeOH (20 mL) was added to the reaction solution to quench the reaction. The mixture was concentrated under reduced pressure, and the crude product was separated by silica gel column chromatography (eluent: DCM/EtOAc (v/v) = 3/1) to give pale yellow liquid (480 mg, 62%).

MS (ESI, pos.ion) m/z: 445.1 $[M+H]^+$.

**Step 3: Synthesis of (*S*)-3-methyl-4-(((2*S*,4*R*)-4-(4-(trifluoromethyl)phenoxy) pyrrolidin-2-yl)methyl)morpholine**

To a solution of *tert*-butyl (2*S*,4*R*)-2-(((*S*)-3-methylmorpholinyl)methyl) -4-(4-(trifluoromethyl)phenoxy)pyrrolidine-1-carboxylate (400 mg, 0.90 mmol) in DCM (8 mL) was added a solution of HCl in methanol (2 mL, 20%). The mixture was stirred at room temperature for 16 h. The reaction solution was concentrated under reduced pressure to give pale yellow liquid (310 mg, 100%).

MS (ESI, pos.ion) m/z: 345.3 $[M+H]^+$.

**Step 4: Synthesis of ethyl 4-((2*S*,4*R*)-2-(((*S*)-3-methylmorpholinyl)methyl)-4-(4-(trifluoromethyl)phenoxy)pyrrolidin-1-yl)benzoate**

Under nitrogen protection, (*S*)-3-methyl-4-(((2*S*,4*R*)-4-(4-(trifluoromethyl)phenoxy) pyrrolidin-2-yl)methyl)morpholine (300 mg, 0.87 mmol), $Pd_2(dba)_3$ (79 mg, 0.09 mmol), XantPhos (75 mg, 0.13 mmol), $Cs_2CO_3$ (567 mg, 1.74 mmol), ethyl 4-iodobenzoate (312 mg, 1.13 mmol) were dissolved in 1,4-dioxane (8 mL) and the mixture was reacted in a 100°C oil bath for 13 h. The reaction solution was cooled to room temperature, then concentrated under reduced pressure. The residue was diluted with DCM (80 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give brown liquid (346 mg, 81%).

MS (ESI, pos.ion) m/z: 493.4 $[M+H]^+$.

**Step 5: Synthesis of 4-((2*S*,4*R*)-2-(((*S*)-3-methylmorpholinyl)methyl) -4-(4-(trifluoromethyl)phenoxy)pyrrolidin-1-yl)benzoic acid**

To a solution of ethyl 4-((2*S*,4*R*)-2-(((*S*)-3-methylmorpholinyl)methyl) -4-(4-(trifluoromethyl)phenoxy)pyrrolidin-1-yl)benzoate (150 mg, 0.30 mmol) in MeOH (6 mL) was added a solution of $LiOH \cdot H_2O$ (127 mg, 3.03 mmol) in $H_2O$ (2 mL). The mixture was reacted at room temperature for 24 h. To the reaction solution was added HCl solution (1 mol/L) to adjust the pH of the solution to about 5. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 2/1) to give brown liquid (90 mg, 64%).

MS (ESI, pos.ion) m/z: 465.1 $[M+H]^+$.

**Step 6: Synthesis of *N*-((*R*)-1-(4-(ethylsulfonyl)phenyl)-2-hydroxyethyl)-4-((2*S*,4*R*)-2-(((*S*)-3-methylmorpholinyl)methyl)-4-(4-(trifluoromethyl)phenoxy)pyrrolidin-1-yl)benzamide**

(*R*)-2-Amino-2-(4-(ethylsulfonyl)phenyl)ethanol (47 mg, 0.20 mmol), 4-((2*S*,4*R*)-2-(((*S*)-3-methylmorpholinyl)methyl)-4-(4-trifluoromethyl)phenoxy)pyrrolidin-1-yl)ben zoic acid (80 mg, 0.17 mmol), EDCI (50 mg, 0.26 mmol), HOBT (34 mg, 0.25 mmol) and TEA (34 mg, 0.34 mmol) were dissolved in DCM (4 mL), and the mixture was stirred at room temperature for 11 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (76 mg, 65%).

MS (ESI, pos.ion) m/z: 676.4 $[M+H]^+$.

$^1H$ NMR (400 MHz, $CDCl_3$) δ (ppm): 7.82 (d, *J* = 8.2 Hz, 2H), 7.73 (d, *J* = 8.7 Hz, 2H), 7.55 (d, *J* = 8.2 Hz, 2H), 7.54 (d, *J* = 8.7 Hz, 2H), 7.06 (d, *J* = 7.0 Hz, 1H), 6.95 (d, *J*= 8.6 Hz, 2H), 6.60 (d, *J* = 8.7 Hz, 2H), 5.24 (d, *J* = 6.2 Hz, 1H), 5.17 - 5.10 (m, 1H), 4.25 - 4.17 (m, 1H), 3.99 (dd, *J* = 11.3, 3.8 Hz, 1H), 3.95 - 3.86 (m, 2H), 3.78 (d, *J*= 11.3 Hz, 1H), 3.74 (d, *J*= 6.5 Hz, 1H), 3.73 - 3.68 (m, 1H), 3.68 - 3.63 (m, 1H), 3.61 (d, *J* = 9.5 Hz, 1H), 3.53 (dd, *J* = 10.7, 3.5 Hz, 1H), 3.26 (dd, *J* = 10.9, 8.9 Hz, 1H), 3.07 (q, *J* = 7.4 Hz, 2H), 2.89 (d, *J* = 11.6 Hz, 1H), 2.61 (dd, *J* = 12.8, 9.1 Hz, 1H), 2.57 - 2.50 (m, 1H), 2.46 (dd, *J* = 13.2, 3.1 Hz, 1H), 2.42 - 2.37 (m, 2H), 1.25 (t, *J* = 7.4 Hz, 3H), 0.94 (d, *J* = 6.2 Hz, 3H).

**Example 36 4-((2*R*,4*R*)-2-(cyanomethyl)-4-(4-trifluoromethyl)phenoxy)pyrrolidin-1-yl) -*N*-((*R*)-1-(4-(ethylsulfonyl)phenyl)-2-hydroxyethyl)benzamide**

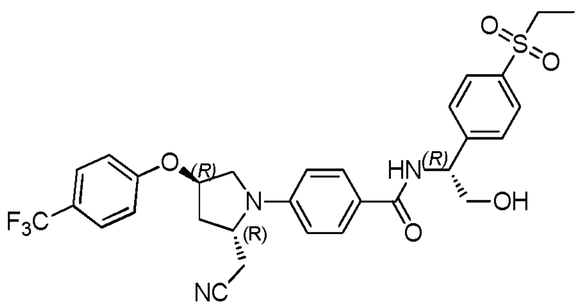

**Step 1: Synthesis of ethyl 4-((2R,4R)-2-(cyanomethyl)-4-(4-(trifluoromethyl)phenoxy) pyrrolidin-1-yl)benzoate**

Ethyl 4-((2*S*,4*R*)-2-(((methylsulfonyl)oxy)methyl)-4-(4-(trifluoromethyl)phenoxy) pyrrolidin-1-yl)benzoate (300 mg, 0.62 mmol) and $N(n\text{-}Bu)_4CN$ (330 mg, 1.23 mmol) were added to ACN (6 mL), and the mixture was heated and stirred in an oil bath at 80°C for 5 h. The reaction solution was cooled to room temperature and concentrated under reduced pressure, and the crude product was separated by silica gel column chromatography (eluent: PE/EtOAc (v/v) = 4/1) to give yellow liquid (200 mg, 78%).

MS (ESI, pos.ion) m/z: 419.2 $[M+H]^+$.

**Step 2: Synthesis of 4-((2R,4R)-2-(cyanomethyl)-4-(4-(trifluoromethyl)phenoxy) pyrrolidin-1-yl)benzoic acid**

To a solution of ethyl 4-((2*R*,4*R*)-2-(cyanomethyl)-4-(4-(trifluoromethyl)phenoxy) pyrrolidin-1-yl)benzoate (150 mg, 0.36 mmol) in MeOH (6 mL) was added a solution of $LiOH \cdot H_2O$ (402 mg, 9.58 mmol) in $H_2O$ (3 mL). The mixture was reacted at room temperature for 21 h. To the reaction solution was added HCl solution (1 mol/L) to adjust the pH of the solution to about 5. The resulting mixture was extracted with EtOAc (30 mL × 2). The organic phases were combined, washed with saturated NaCl (20 mL) solution, dried over anhydrous $Na_2SO_4$, and concentrated under reduced

pressure. The crude product was separated by silica gel column chromatography (eluent: DCM/EtOAc (v/v) = 4/1) to give colorless liquid (95 mg, 68%).

MS (ESI, pos.ion) m/z: 391.2 $[M+H]^+$.

**Step 3: Synthesis of 4-((2R,4R)-2-(cyanomethyl)-4-(4-(trifluoromethyl)phenoxy) pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (68 mg, 0.30 mmol), 4-((2*R*,4*R*)-2-(cyanomethyl)-4-(4-(trifluoromethyl)phenoxy)pyrrolidin-1-yl)benzoic acid (90 mg, 0.23 mmol), HATU (131 mg, 0.34 mmol) and DIPEA (101 mg, 0.78 mmol) were dissolved in DCM (4 mL). The mixture was stirred at room temperature for 13 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (60 mg, 43%).

MS (ESI, pos.ion) m/z: 602.3 $[M+H]^+$.

$^1H$ NMR (400 MHz, $CDCl_3$) $\delta$ (ppm): 7.84 (d, *J*= 6.8 Hz, 2H), 7.77 (d, *J*= 7.7 Hz, 2H), 7.59 - 7.51 (m, 4H), 7.09 (s, 1H), 6.93 (d, *J* = 8.5 Hz, 2H), 6.60 (d, *J* = 7.6 Hz, 2H), 5.24 (s, 2H), 4.42 (s, 1H), 4.07 - 3.91 (m, 3H), 3.63 (d, *J* = 10.5 Hz, 1H), 3.08 (q, *J* = 7.3 Hz, 2H), 2.85 (dd, *J* = 17.0, 6.0 Hz, 1H), 2.70 (d, *J* = 16.6 Hz, 2H), 2.56 - 2.47 (m, 1H), 1.27 (t, *J* = 7.3 Hz, 3H).

**Example 37 (2*S*,4*R*)-1-(4-(((*R*)-1-(4-(ethylsulfonyl)phenyl)-2-hydroxyethyl)carbamoyl) phenyl)-*N*-methoxy-*N*-methyl-4-(4-(trifluoromethyl)phenoxy)pyrrolidine-2-carboxamide**

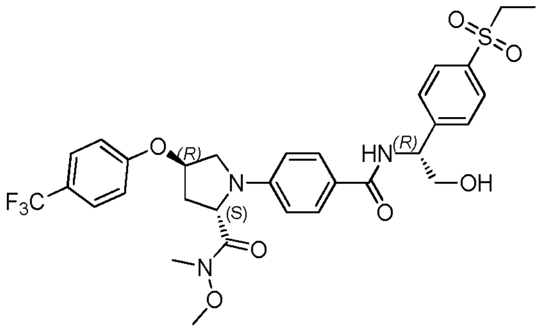

**Step 1: Synthesis of *tert*-butyl (2*S*,4*R*)-2-(methoxy(methyl)carbamoyl)-4-(4-(trifluoromethyl)phenoxy)pyrrolidine-1-carboxylate**

(2*S*,4*R*)-1-(*tert*-Butoxycarbonyl)-4-(4-(trifluoromethyl)phenoxy)pyrrolidin-2-carboxyli c acid (1.00 g, 2.66 mmol), *N*-methoxymethylamine hydrochloride (312 mg, 3.20 mmol), HATU (1.52 g, 4.00 mmol) and DIPEA (1.30 mL, 7.87 mmol) were dissolved in DCM (16 mL), and the mixture was stirred at room temperature for 16 h. The reaction solution was filtered to remove white insolubles. The filtrate was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 2/1) to give colorless liquid (912 mg, 82%).

MS (ESI, pos.ion) m/z: 441.3 $[M+Na]^+$.

**Step 2: Synthesis of (2*S*,4*R*)-*N*-methoxy-*N*-methyl-4-(4-(trifluoromethyl) phenoxy)pyrrolidine-2-carboxamide**

To a solution of *tert*-butyl (2*S*,4*R*)-2-(methoxy(methyl)carbamoyl)-4-(4-(trifluoromethyl)phenoxy)pyrrolidine-1-carboxylate(900 mg, 2.15 mmol) in DCM (16 mL) was added a solution of HCl in methanol (6 mL, 20%). The mixture was stirred at room temperature for 16 h. The reaction solution was concentrated under reduced pressure to give colorless liquid (4.30 g, 100%).

MS (ESI, pos.ion) m/z: 319.1 $[M+H]^+$.

**Step 3: Synthesis of ethyl 4-((2S,4R)-2-(methoxy(methyl)carbamoyl)-4-(4-(trifluoromethyl)phenoxy)pyrrolidin-1-yl)benzoate**

Under nitrogen protection, (2*S*,4*R*)-*N*-methoxy-*N*-methyl-4-(4-(trifluoromethyl)phenoxy)pyrrolidine-2-carboxamide (400 mg, 1.26 mmol), $Pd_2(dba)_3$ (175 mg, 0.13 mmol), XantPhos (109 mg, 0.19 mmol), $Cs_2CO_3$ (818 mg, 2.51 mmol), ethyl 4-iodobenzoate (416 mg, 1.51 mmol) were dissolved in 1,4-dioxane (8 mL) and the mixture was heated and stirred in a 100°C oil bath for 18 h. The reaction solution was cooled to room temperature, and concentrated under reduced pressure. The residue was diluted with DCM (80 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 3/1) to give yellow liquid (150 mg, 26%).

MS (ESI, pos.ion) m/z: 467.3 $[M+H]^+$.

**Step 4: Synthesis of 4-((2*S*,4*R*)-2-(methoxy(methyl)carbamoyl)-4-(4-(trifluoromethyl)phenoxy)pyrrolidin-1-yl)benzoic acid**

To a solution of ethyl 4-((2*S*,4*R*)-2-(methoxy(methyl)carbamoyl)-4-(4-(trifluoromethyl)phenoxy)pyrrolidin-1-yl)benzoate (150 mg, 0.37 mmol) in MeOH (6 mL) was added a solution of LiOH $H_2O$ (89 mg, 2.12 mmol) in $H_2O$ (2 mL). The mixture was reacted at room temperature for 6 h. To the reaction solution was added HCl solution (1 mol/L) to adjust the pH of the solution to about 5. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (30 mg, 32%).

MS (ESI, pos.ion) m/z: 439.1 $[M+H]^+$.

**Step 5: Synthesis of (2*S*,4*R*)-1-(4-(((*R*)-1-(4-(ethylsulfonyl)phenyl)-2-hydroxyethyl)carbamoyl)phenyl)-*N*-methoxy-*N*-methyl-4-(4-(trifluoromethyl)phenoxy)pyrroli dine-2-carboxamide**

(*R*)-2-Amino-2-(4-(ethylsulfonyl)phenyl)ethanol (10 mg, 0.34 mmol), 4-((2*S*,4*R*)-2-(methoxy(methyl)carbamoyl)-4-(4-trifluoromethyl)phenoxy)pyrrolidin-1-yl)benzoic acid (15 mg, 0.03 mmol), EDCI (10 mg, 0.05 mmol), HOBT (7 mg, 0.05 mmol) and TEA (8 mg, 0.08 mmol) were dissolved in DCM (2 mL), and the mixture was stirred at room temperature for 12 h. The reaction solution was diluted with DCM (30 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (6 mg, 27%).

MS (ESI, pos.ion) m/z: 550.4 $[M+H]^+$.

$^1H$ NMR (400 MHz, $CDCl_3$) δ (ppm): 7.85 (d, *J* = 8.1 Hz, 2H), 7.73 (d, *J* = 8.6 Hz, 2H), 7.56 (d, *J* = 8.1 Hz, 2H), 7.54 (d, *J* = 8.8 Hz, 2H), 7.01 (d, *J* = 6.8 Hz, 1H), 6.96 (d, *J*= 8.5 Hz, 2H), 6.53 (d, *J* = 8.6 Hz, 2H), 5.20 (s, 2H), 5.04 (s, 1H), 4.05 (dd, *J* = 10.4, 5.4 Hz, 1H), 3.97 - 3.92 (m, 2H), 3.84 (s, 3H), 3.70 (d, *J*= 9.8 Hz, 1H), 3.58 - 3.52 (m, 1H), 3.23 (s, 3H), 3.08 (q, *J*= 7.4 Hz, 2H), 2.72 - 2.66 (m, 1H), 2.52 - 2.44 (m, 1H), 1.33 (t, *J*= 7.4 Hz, 3H).

**Example 38 4-((2*R*,4*R*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

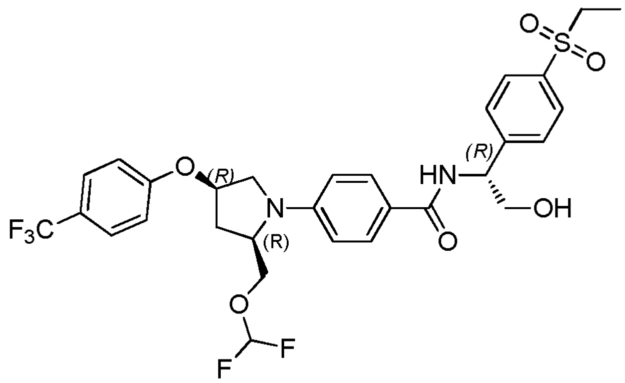

**Step 1: Synthesis of (2*R*,4*R*)-1-*tert*-butyl 2-methyl 4-(4-(trifluoromethyl)phenoxy)pyrrolidine-1,2-dicarboxylate**

(2*R*,4*S*)-1-*tert*-butyl 2-methyl 4-hydroxypyrrolidine-1,2-dicarboxylate (2.00 g, 8.15 mmol), 4-(trifluoromethyl)phenol (1.60 g, 9.87 mmol), $PPh_3$ (3.10 g, 9.03 mmol) were added to THF (30 mL). The mixture was transferred to 0 °C, and DIAD (2.00 mL, 10.16 mmol) was slowly added. After the addition was complete, the mixture was transferred to room temperature and reacted for 24 h. The reaction solution was concentrated under reduced pressure. The residue was diluted with methyl *tert*-butyl ether (25 mL) and stirred at -20 °C. A large amount of white insoluble solids (triphenoxyphosphine) were precipitated, filtered while cold, and the filter cake was washed with cold methyl *tert*-butyl ether. The filtrate was concentrated under reduced pressure, and the crude product was separated by silica gel column chromatography (eluent: PE/EtOAc (v/v) = 4/1) to give a white solid (2.50 g, 79%).

MS (ESI, pos.ion) m/z: 412.1 $[M+Na]^+$.

**Step 2: Synthesis of *tert*-butyl (2*R*,4*R*)-2-(hydroxymethyl)-4-(4-(trifluoromethyl) phenoxy)pyrrolidine-l-carboxylate**

To THF (30 mL) solution was added (2*R*,4*R*)-1-*tert*-butyl 2-methyl 4-(4-(trifluoromethyl)phenoxy)pyrrolidine-1,2-dicarboxylate (3.00 g, 7.70 mmol), then the mixture was transferred to 0 °C, and $LiBH_4$ (336 mg, 15.43 mmol) was added slowly. After the addition was completed, the mixture was stirred at room temperature for 16 h. Saturated $NH_4Cl$ (30 mL) solution was added to the reaction solution to quench the reaction. The mixture was left standing for layers, the upper organic phase was separated, the aqueous phase was extracted with EtOAc (30 mL × 2). The combined organic phases were dried over anhydrous $Na_2SO_4$, concentrated under reduced pressure, and the crude product was separated by silica gel column chromatography (eluent: PE/EtOAc (v/v) = 3/1) to give colorless liquid (2.50 g, 90%).

MS (ESI, pos.ion) m/z: 384.0 $[M+Na]^+$.

**Step 3: Synthesis of *tert*-butyl (2*R*,4*R*)-2-((difluoromethoxy)methyl)-4-(4-(trifluoromethyl)phenoxy)pyrrolidine-1-carboxylate**

To a solution of *tert*-butyl (2*R*,4*R*)-2-(hydroxymethyl)-4-(4-(trifluoromethyl)phenoxy)pyrrolidine-1-carboxylate(500 mg, 1.38 mmol) in DCM (1 mL) were sequentially added $H_2O$ (1 mL), KOAc (678 mg, 6.91 mmol) and $TMSCF_2Br$ (0.50 mL, 3.22 mmol). The mixture was reacted at room temperature for 17 h. The reaction solution was diluted with DCM (30 mL), washed with saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give colorless liquid (420 mg, 74%).

MS (ESI, pos.ion) m/z: 434.3 $[M+Na]^+$.

**Step 4: Synthesis of (2*R*,4*R*)-2-((difluoromethoxy)methyl)-4-(4-(trifluoromethyl)phenoxy)pyrrolidine**

To a solution of *tert*-butyl (2*R*,4*R*)-2-((difluoromethoxy)methyl)-4-(4-(trifluoromethyl)phenoxy)pyrrolidine-1-carboxylate(400 mg, 0.97 mmol) in DCM (8 mL) was added a solution of HCl in methanol (2 mL, 20%). The mixture was stirred at room temperature for 23 h. The reaction solution was concentrated under reduced pressure, and the residue was diluted with DCM (30 mL), washed successively with saturated $NaHCO_3$ solution (15 mL) and saturated NaCl solution (15 mL), dried over anhydrous $Na_2SO_4$, and concentrated under reduced pressure to give colorless liquid (280 mg, 93%).

MS (ESI, pos.ion) m/z: 312.1 $[M+H]^+$.

**Step 5: Synthesis of methyl 4-((2*R*,4*R*)-2-((difluoromethoxy)methyl)-4-(4-(trifluoromethyl)phenoxy)pyrrolidin-1-yl)benzoate**

Under nitrogen protection, (2*R*,4*R*)-2-((difluoromethoxy)methyl)-4-(4-(trifluoromethyl)phenoxy)pyrrolidine (280 mg, 0.90 mmol), $Pd_2(dba)_3$ (82 mg, 0.09 mmol), XantPhos (78 mg, 0.13 mmol), $Cs_2CO_3$ (300 mg, 0.92 mmol) and methyl 4-iodobenzoate (353 mg, 1.35 mmol) were dissolved in 1,4-dioxane (6 mL) in turn and the mixture was reacted in a 100°C oil bath for 22 h. The reaction solution was cooled to room temperature, then concentrated under reduced pressure. The residue was diluted with DCM (50 mL), washed successively with $NaHCO_3$ solution (15 mL) and saturated NaCl solution (15 mL), dried over anhydrous $Na_2SO_4$, concentrated under reduced pressure, and the crude product was separated by silica gel column chromatography (eluent: PE/EtOAc (v/v) = 4/1) to give yellow liquid (300 mg, 75%).
MS (ESI, pos.ion) m/z: 446.0 $[M+H]^+$.

**Step 6: Synthesis of 4-((2*R*,4*R*)-2-((difluoromethoxy)methyl)-4-(4-(trifluoromethyl)phenoxy)pyrrolidin-1-yl)benzoic acid**

To a solution of methyl 4-((2*R*,4*R*)-2-((difluoromethoxy)methyl)-4-(4-(trifluoromethyl)phenoxy)pyrrolidin-1-yl)benzoate (240 mg, 0.54 mmol) in MeOH (6 mL) was added a solution of LiOH $H_2O$ (226 mg, 5.39 mmol) in $H_2O$ (2 mL). The mixture was reacted at room temperature for 17 h. The reaction solution was added with HCl solution (1 mol/L) to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a pale yellow solid (172 mg, 74%).
MS (ESI, pos.ion) m/z: 432.0 $[M+H]^+$.

**Step 7: Synthesis of 4-((2R,4R)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl) benzamide**

EDCI (43 mg, 0.22 mmol), HOBT (30 mg, 0.22 mmol), (*R*)-2-amino-2-(4-(ethylsulfonyl)phenyl)ethanol (44 mg, 0.19 mmol), 4-((2*R*,4*R*)-2-((difluoromethoxy)methyl)-4-(4-(trifluoromethyl)phenoxy)pyrrolidin-1-yl)benzoic acid (65 mg, 0.15 mmol) and TEA (45 mg, 0.44 mmol) were successively added to DCM (4 mL), and the mixture was stirred at room temperature for 17 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (84 mg, 87%).
MS (ESI, pos.ion) m/z: 643.0 $[M+H]^+$.
$^1H$ NMR (400 MHz, $CDCl_3$) δ (ppm): 7.83 (d, *J* = 8.3 Hz, 2H), 7.75 (d, *J* = 8.7 Hz, 2H), 7.58 (d, *J* = 8.6 Hz, 2H), 7.55 (d, *J* = 8.2 Hz, 2H), 7.03 (d, *J* = 6.9 Hz, 1H), 6.97 (d, *J* = 8.6 Hz, 2H), 6.64 (d, *J* = 8.8 Hz, 2H), 6.22 (t, *J* = 74.3 Hz, 1H), 5.24 (dd, *J* = 10.7, 4.4 Hz, 1H), 5.17 (t, *J* = 4.5 Hz, 1H), 4.23 - 4.13 (m, 2H), 4.03 - 3.91 (m, 3H), 3.76 (d, *J* = 11.4 Hz, 1H), 3.70 (dd, *J* = 11.4, 4.7 Hz, 1H), 3.07 (q, *J*= 7.4 Hz, 2H), 2.51 (d, *J*= 14.4 Hz, 1H), 2.43 - 2.34 (m, 1H), 1.26 (t, *J*= 7.4 Hz, 3H).

**Example 39 4-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

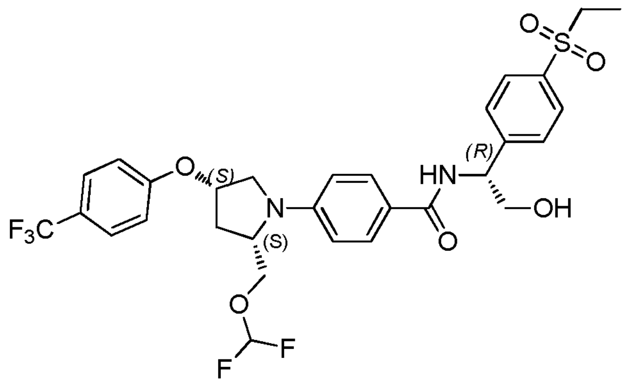

Using (2*S*,4*R*)-1-*tert*-butyl 2-methyl 4-hydroxypyrrolidine-1,2-dicarboxylate as raw material, the title compound was prepared as a white solid (55 mg, 62%) by referring to the methods of step 1 to step 7 of Example 38.

MS (ESI, pos.ion) m/z: 643.1 $[M+H]^+$.

$^1H$ NMR (400 MHz, $CDCl_3$) δ (ppm): 7.78 (d, *J* = 8.3 Hz, 2H), 7.76 (d, *J* = 8.4 Hz, 2H), 7.53 (d, *J* = 7.6 Hz, 4H), 7.50 (s, 1H), 6.93 (d, *J* = 8.3 Hz, 2H), 6.61 (d, *J* = 8.4 Hz, 2H), 6.18 (t, *J* = 74.4 Hz, 1H), 5.18 (s, 1H), 5.13 (s, 1H), 4.20 - 4.05 (m, 2H), 3.96 - 3.86 (m, 2H), 3.85 - 3.77 (m, 1H), 3.74 - 3.62 (m, 2H), 3.05 (q, *J* = 7.2 Hz, 2H), 2.46 (d, *J* = 14.3 Hz, 1H), 2.39 - 2.30 (m, 1H), 1.21 (t, *J* = 7.2 Hz, 3H).

**Example 40 4-((2*S*,4*R*)-2-((difluoromethoxy)methyl)-4-(4-trifluoromethyl) phenoxy)pyrrolidin-1-yl)-*N*-((*R*)-1-(4-(ethylsulfonyl)phenyl)-2-hydroxyethyl)benzamide**

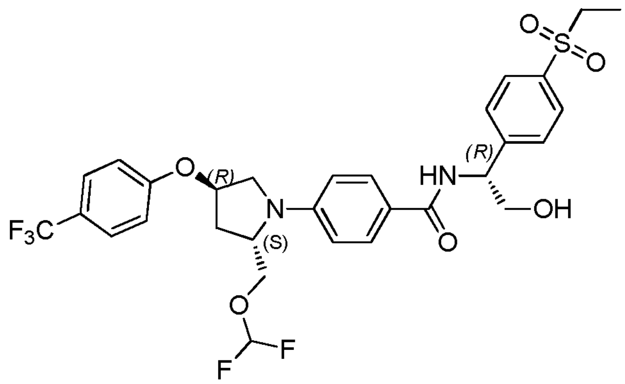

Using (2*S*,4*S*)-1-*tert*-butyl 2-methyl 4-hydroxypyrrolidine-1,2-dicarboxylate as raw material, the title compound was prepared as a white solid (66 mg, 74%) by referring to the methods of step 1 to step 7 of Example 38.

MS (ESI, pos.ion) m/z: 643.1 $[M+H]^+$.

$^1H$ NMR (400 MHz, $CDCl_3$) δ (ppm): 7.82 (d, *J* = 8.3 Hz, 2H), 7.74 (d, *J* = 8.7 Hz, 2H), 7.55 (d, *J* = 8.4 Hz, 2H), 7.53 (d, *J* = 8.2 Hz, 2H), 7.07 (d, *J* = 7.0 Hz, 1H), 6.95 (d, *J* = 8.6 Hz, 2H), 6.61 (d, *J* = 8.8 Hz, 2H), 6.20 (t, *J* = 74.2 Hz, 1H), 5.23 (dd, *J* = 10.8, 4.5 Hz, 1H), 5.20 - 5.14 (m, 1H), 4.37 - 4.30 (m, 1H), 4.02 - 3.96 (m, 3H), 3.92 (dd, *J* = 10.7, 5.8 Hz, 2H), 3.54 (dd, *J* = 10.6, 3.5 Hz, 1H), 3.07 (q, *J* = 7.4 Hz, 2H), 2.57 - 2.43 (m, 2H), 1.25 (t, *J* = 7.4 Hz, 3H).

**Example 41 4-((2*R*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

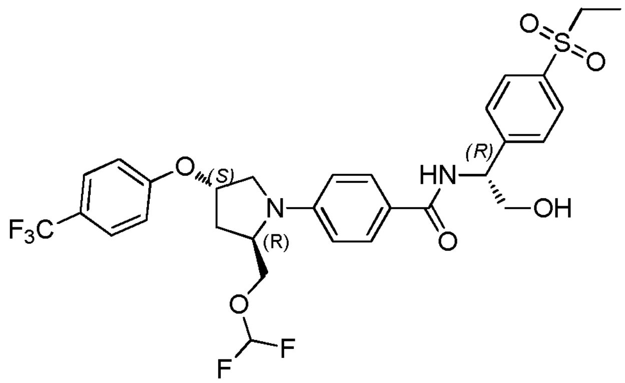

Using (2*R*,4*R*)-1-*tert*-butyl 2-methyl 4-hydroxypyrrolidine-1,2-dicarboxylate (2.00 g, 8.15 mmol) as raw material, the title compound was prepared as a pale yellow solid (48 mg, 81%) by referring to the methods of step 1 to step 7 of Example 38.

MS (ESI, pos.ion) m/z: 643.1 $[M+H]^+$.

$^1H$ NMR (400 MHz, $CDCl_3$) δ (ppm): 7.86 (d, *J* = 8.2 Hz, 2H), 7.77 (d, *J* = 8.7 Hz, 2H), 7.59 (d, *J* = 5.0 Hz, 2H), 7.57 (d, *J* = 4.6 Hz, 2H), 7.04 (d, *J* = 6.8 Hz, 1H), 6.98 (d, *J* = 8.6 Hz, 2H), 6.64 (d, *J* = 8.7 Hz, 2H), 6.23 (t, *J* = 74.1 Hz, 1H), 5.27 (d, *J* = 6.1 Hz, 1H), 5.23 - 5.17 (m, 1H), 4.37 (d, *J*= 4.1 Hz, 1H), 4.06 - 3.92 (m, 5H), 3.57 (dd, *J*= 10.6, 3.3 Hz, 1H), 3.10 (q, *J*= 7.4 Hz, 2H), 2.60 - 2.46 (m, 2H), 1.30 (d, *J*= 7.4 Hz, 3H).

**Example 42 4-((2*S*,4*R*)-2-((1,1-difluoroethoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

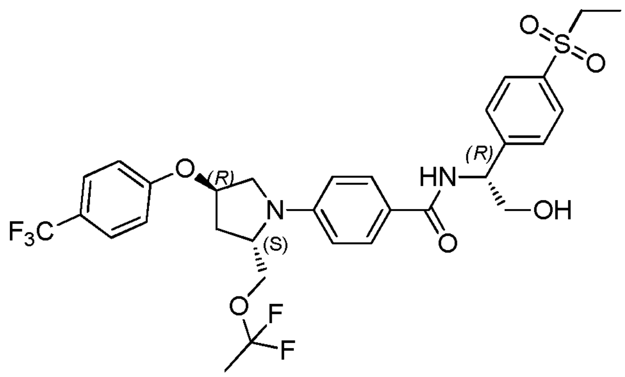

**Step 1: Synthesis of benzyl (2*S*,4*R*)-2-(hydroxymethyl)-4-(4-(trifluoromethyl) phenoxy)pyrrolidine-1-carboxylate**

To a solution of ((2*S*,4*R*)-4-(4-(trifluoromethyl)phenoxy)pyrrolidin-2-yl)methanol (500 mg, 1.91 mmol) and $NaHCO_3$ (643 mg, 7.65 mmol) in THF/$H_2O$ (8 mL/3 mL) was added CbzCl (0.35 mL, 2.49 mmol). The mixture was stirred at room temperature for 16 h. The reaction solution was diluted with saturated $NaHCO_3$ solution (15 mL) and EtOAc (20 mL). The resulting mixture was left standing for layers, the upper organic phase was separated, and the aqueous phase was extracted with EtOAc (20 mL × 2). The combined organic phases were washed with saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give colorless liquid (700 mg, 93%).

MS (ESI, pos.ion) m/z: 396.2 $[M+H]^+$.

**Step 2: Synthesis of benzyl (2*S*,4*R*)-2-(acetoxymethyl)-4-(4-(trifluoromethyl)phenoxy)pyrrolidine-1-carboxylate**

To a solution of benzyl (2*S*,4*R*)-2-(hydroxymethyl)-4-(4-(trifluoromethyl) phenoxy)pyrrolidine-1-carboxylate and DMAP (21 mg, 0.17mmol) in DCM (10 mL) were sequentially added $AC_2O$ (0.25 mL, 2.66 mmol) and TEA (0.50 mL, 3.60 mmol). The mixture was reacted at room temperature for 15 h. The reaction solution was concentrated under reduced pressure, and the crude product was separated by silica gel column chromatography (eluent: PE/EtOAc (v/v)

= 3/1) to give colorless liquid (700 mg, 90%).

MS (ESI, pos.ion) m/z: 438.2 $[M+H]^+$.

**Step 3: Synthesis of benzyl (2*S*,4*R*)-2-((thioacetoxy)methyl)-4-(4-(trifluoromethyl) phenoxy)pyrrolidine-l-carboxylate**

To a solution of benzyl (2*S*,4*R*)-2-(acetoxymethyl)-4-(4-(trifluoromethyl) phenoxy)pyrrolidin-1-yl)carboxylate (700 mg, 1.60 mmol) in toluene (12 mL) was added Lawesson's reagent (8.41 g, 20.79 mmol). The mixture was heated and stirred in an oil bath at 110°C for 17 h. The reaction solution was cooled to room temperature and concentrated under reduced pressure, and the crude product was separated by silica gel column chromatography (eluent: PE/EtOAc (v/v) = 5/1) to give yellow liquid (76 mg, 10%).

MS (ESI, pos.ion) m/z: 476.3 $[M+Na]^+$.

**Step 4: Synthesis of benzyl (2*S*,4*R*)-2-((1,1-difluoroethoxy)methyl)-4-(4-(trifluoromethyl)phenoxy)pyrrolidine-1-carboxylate**

Under nitrogen protection, to a solution of benzyl (2*S*,4*R*)-2-((thioacetoxy)methyl)-4-(4-(trifluoromethyl)phenoxy)pyrrolidine-1-carboxylate (85 mg, 0.19 mmol) in DCM (2 mL) was added DAST (60 mg, 0.37 mmol). The mixture was stirred at room temperature for 7 h. The reaction solution was added with DCM (20 mL), washed successively with $NaHCO_3$ solution (10 mL) and saturated NaCl solution (10 mL), dried over anhydrous $Na_2SO_4$, and concentrated under reduced pressure. The crude product was separated by silica gel column chromatography (eluent: PE/EtOAc (v/v) = 4/1) to give yellow liquid (56 mg, 65%).

MS (ESI, pos.ion) m/z: 482.0 $[M+Na]^+$.

**Step 5: Synthesis of (2*S*,4*R*)-2-((1,1-difluoroethoxy)methyl)-4-(4-(trifluoromethyl)phenoxy)pyrrolidine**

To a solution of benzyl (2S,4R)- 2-((1,1-difluoroethoxy)methyl)-4-(4-(trifluoromethyl) phenoxy)pyrrolidine-1-carboxylate (56 mg, 0.12 mmol) in MeOH (6 mL) was added Pd/C (20 mg, 10%) under hydrogen protection. The mixture was stirred at room temperature for 24 h. The reaction solution was filtered, and the filtrate was concentrated under reduced pressure to give colorless liquid (39 mg, 98%).

MS (ESI, pos.ion) m/z: 326.2 $[M+H]^+$.

**Step 6: Synthesis of methyl 4-((2*S*,4*R*)-2-((1,1-difluoroethoxy)methyl) -4-(4-(trifluoromethyl)phenoxy)pyrrolidin-1-yl)benzoate**

Under nitrogen protection, (2*S*,4*R*)-2-((1,1-difluoroethoxy)methyl)-4-(4-(trifluoromethyl)phenoxy)pyrrolidine (35 mg, 0.11 mmol), $Pd_2(dba)_3$ (10 mg, 0.01 mmol), XantPhos (10 mg, 0.01 mmol), $Cs_2CO_3$ (70 mg, 0.21 mmol) and methyl 4-iodobenzoate (42 mg, 0.16 mmol) were dissolved in 1,4-dioxane (4 mL), and the mixture was reacted in a 100°C oil bath for 16 h. The reaction solution was cooled to room temperature, and concentrated under reduced pressure. The residue was diluted with DCM (40 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give a yellow solid (16 mg, 32%).

MS (ESI, pos.ion) m/z: 460.3 $[M+H]^+$.

**Step 7: Synthesis of 4-((2*S*,4*R*)-2-((1,1-difluoroethoxy)methyl)-4-(4-(trifluoromethyl)phenoxy)pyrrolidin-1-yl)benzoic acid**

To a solution of methyl 4-((2*S*,4*R*)-2-((1,1-difluoroethoxy)methyl)-4-(4-(trifluoromethyl)phenoxy)pyrrolidin-1-yl)benzoate (15 mg, 0.03 mmol) in MeOH (4 mL) was added a solution of $LiOH{\cdot}H_2O$ (30 mg, 0.72 mmol) in $H_2O$ (1 mL). The mixture was reacted at room temperature for 24 h. The reaction solution was diluted with $H_2O$ (10 mL), and HCl solution (1 mol/L) was added to adjust the pH of the solution to about 4. The resulting mixture was extracted with EtOAc (20 mL $\times$ 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (9 mg, 62%).

MS (ESI, pos.ion) m/z: 446.3 $[M+H]^+$.

**Step 8: Synthesis of 4-((2*S*,4*R*)-2-((1,1-difluoroethoxy)methyl)-4-(4-(trifluoromethyl) phenoxy)pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (6 mg, 0.03 mmol), 4-((2*S*,4*R*)-2-((1,1-difluoroethoxy)methyl)-4-(4-trifluoromethyl)phenoxy)pyrrolidin-1-yl)benzoic acid (8 mg , 0.02 mmol), EDCI (6 mg, 0.03 mmol), HOBT (5 mg, 0.04 mmol) and TEA (5 mg, 0.05 mmol) were dissolved in DCM (2 mL), and the mixture was stirred at room temperature for 21 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (7 mg, 59%).

MS (ESI, pos.ion) m/z: 657.5 $[M+H]^+$;

$^1H$ NMR (400 MHz, $CDCl_3$) $\delta$ (ppm): 7.87 (d, *J* = 8.1 Hz, 2H), 7.76 (d, *J* = 8.5 Hz, 2H), 7.57 (d, *J* = 8.0 Hz, 4H), 6.95 (d, *J* = 8.4 Hz, 3H), 6.63 (d, *J* = 8.6 Hz, 2H), 5.30 (s, 1H), 5.16 (s, 1H), 4.31 (s, 1H), 4.07 - 3.96 (m, 3H), 3.93 - 3.84 (m, 2H), 3.57 - 3.51 (m, 1H), 3.09 (q, *J* = 7.5 Hz, 2H), 2.54 - 2.44 (m, 2H), 1.75 (t, *J* = 13.1 Hz, 3H), 1.29 (t, *J* = 7.5 Hz, 3H).

**Example 43 4-((2*S*,4*R*)-2-((1,1-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

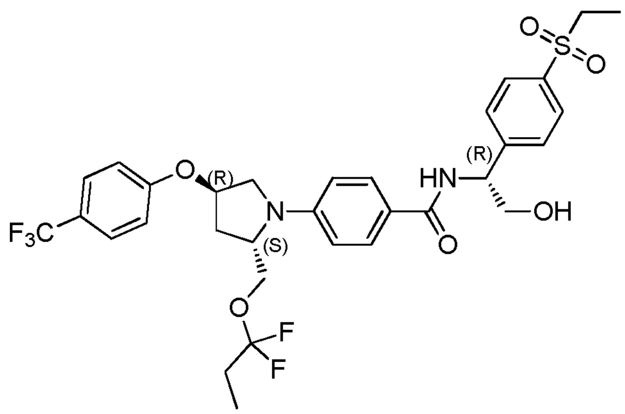

**Step 1: Synthesis of methyl 4-((2S,4R)-2-(((1,1-difluoroallyl)oxy)methyl)-4-(4 -(trifluoromethyl)phenoxy)pyrrolidin-1-yl)benzoate**

Under nitrogen protection, to a solution of 3-bromo-3,3-difluoroprop-1-ene (0.40 mL, 3.91 mmol) and methyl 4-((2*S*,4*R*)-2-(hydroxymethyl)-4-(4-(trifluoromethyl) phenoxy)pyrrolidin-1-yl)benzoate (200 mg, 0.51 mmol) in THF (6 mL) was added NaH (101 mg, 2.53 mmol, 60%). The mixture was stirred at room temperature for 14 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL$\times$2), dried over anhydrous $Na_2SO_4$, and concentrated under reduced pressure. The crude product was separated by silica gel column chromatography (eluent: PE/EtOAc (v/v) = 6/1) to give pale yellow liquid (90 mg, 38%).

MS (ESI, pos.ion) m/z: 472.1 $[M+H]^+$.

**Step 2: Synthesis of methyl 4-((2*S*,4*R*)-2-((1,1-difluoropropoxy)methyl)-4-(4-(trifluoromethyl)phenoxy)pyrrolidin-1-yl)benzoate**

To a solution of methyl 4-((2*S*,4*R*)-2-(((1,1-difluoroallyl)oxy)methyl)-4-(4-(trifluoromethyl)phenoxy)pyrrolidin-1-yl)benzoate (90 mg, 0.19 mmol) in MeOH (6 mL) was added Pd/C (20 mg, 10%) under hydrogen protection. The mixture was stirred at room temperature for 5 h. The reaction solution was filtered, and the filtrate was concentrated under reduced pressure to give colorless liquid (90 mg, 100%).

MS (ESI, pos.ion) m/z: 474.1 $[M+H]^+$.

**Step 3: Synthesis of 4-((2S,4R)-2-((1,1-difluoropropoxy)methyl)-4-(4-(trifluoromethyl)phenoxy)pyrrolidin-1-yl)benzoic acid**

To a solution of methyl 4-((2*S*,4*R*)-2-((1,1-difluoropropoxy)methyl)-4-(4-(trifluoromethyl)phenoxy)pyrrolidin-1-yl)benzoate (90 mg, 0.19 mmol) in MeOH (6 mL) was added a solution of LiOH $H_2O$ (80 mg, 1.91 mmol) in $H_2O$ (2 mL). The mixture was reacted at room temperature for 15 h. The reaction solution was diluted with $H_2O$ (6 mL), and HCl solution (1 mol/L) was added to adjust the pH of the solution to about 5. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give a white solid (75 mg, 86%).

MS (ESI, pos.ion) m/z: 460.1 $[M+H]^+$.

**Step 4: Synthesis of 4-((2*S*,4*R*)-2-((1,1-difluoropropoxy)methyl) -4-(4-(trifluoromethyl)phenoxy)pyrrolidin-1-yl)-*N*-((*R*)-1-(4-(ethylsulfonyl)phenyl)-2-hydroxye thyl)benzamide**

(*R*)-2-Amino-2-(4-(ethylsulfonyl)phenyl)ethanol (17 mg, 0.07 mmol), 4-((2*S*,4*R*)-2-((1,1-difluoropropoxy)methyl)-4-(4-trifluoromethyl)phenoxy)pyrrolidin-1-yl)benzoic acid (30 mg, 0.07 mmol), EDCI (18 mg, 0.09 mmol), HOBT (13 mg, 0.10 mmol) and TEA (10 mg, 0.10 mmol) were dissolved in DCM (4 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (30 mg, 69%).

MS (ESI, pos.ion) m/z: 671.1 $[M+H]^+$;

$^1H$ NMR (400 MHz, $CDCl_3$) δ (ppm): 7.86 (d, *J* = 8.3 Hz, 2H), 7.75 (d, *J* = 8.8 Hz, 2H), 7.57 (d, *J* = 8.1 Hz, 4H), 6.95 (d, *J* = 8.8 Hz, 2H), 6.92 (s, 1H), 6.64 (d, *J* = 8.8 Hz, 2H), 5.28 (d, *J* = 6.3 Hz, 1H), 5.19 - 5.14 (m, 1H), 4.33 (s, 1H), 4.07 - 3.95 (m, 3H), 3.91 (dd, *J* = 10.5, 6.0 Hz, 2H), 3.55 (dd, *J*= 10.5, 3.8 Hz, 1H), 3.09 (q, *J*= 7.4 Hz, 2H), 2.56 - 2.38 (m, 3H), 2.04 - 1.93 (m, 2H), 1.28 (t, *J* = 7.4 Hz, 3H), 1.04 (t, *J* = 7.5 Hz, 3H).

**Example 44 4-((2*S*,4*R*)-2-((3,3-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

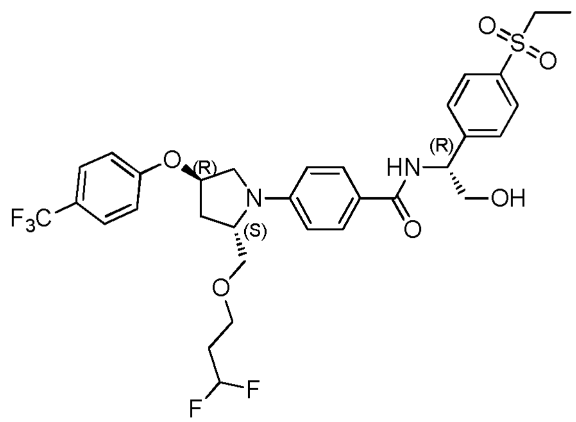

**Step 1: Synthesis of methyl 4-((2*S*,4*R*)-2-(((3,3-difluoroallyl)oxy)methyl)-4-(4-(trifluoromethyl)phenoxy)pyrrolidin-1-yl)benzoate**

Under nitrogen protection, to a solution of 3-bromo-3,3-difluoroprop-1-ene (0.30 mL, 2.95 mmol) and methyl 4-((2*S*,4*R*)-2-(hydroxymethyl)-4-(4-(tiifluoromethyl)phenoxy) pyrrolidin-1-yl)benzoate (150 mg, 0.38 mmol) in THF (6 mL) was added NaH (75 mg, 1.88 mmol, 60%). The mixture was stirred at room temperature for 14 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL×2), dried over anhydrous $Na_2SO_4$, and concentrated under reduced pressure. The crude product was separated by silica gel column chromatography (eluent: PE/EtOAc (v/v) = 6/1) to give pale yellow liquid (25 mg, 14%).

MS (ESI, pos.ion) m/z: 472.1 $[M+H]^+$.

**Step2:Synthesisofmethyl4-((2*S*,4*R*)-2-((3,3-difluoropropoxy)methyl)-4-(4-(trifluoromethyl)phenoxy)pyrrolidin-1-yl)benzoate**

To a solution of methyl 4-((2*S*,4*R*)-2-(((3,3-difluoroallyl)oxy)methyl)-4-(4-(trifluoromethyl)phenoxy)pyrrolidin-1-yl)benzoate (25 mg, 0.05 mmol) in MeOH (6 mL) was added Pd/C (10 mg, 10%) under hydrogen protection. The mixture was stirred at room temperature for 2 h. The reaction solution was filtered, and the filtrate was concentrated under reduced pressure to give colorless liquid (25 mg, 100%).

MS (ESI, pos.ion) m/z: 474.1 $[M+H]^+$.

**Step 3: Synthesis of 4-((2*S*,4*R*)-2-((3,3-difluoropropoxy)methyl)-4-(4-(trifluoromethyl)phenoxy)pyrrolidin-1-yl)benzoic acid**

To a solution of methyl 4-((2*S*,4*R*)-2-((3,3-difluoropropoxy)methyl)-4-(4-(trifluoromethyl)phenoxy)pyrrolidin-1-yl)benzoate (25 mg, 0.05 mmol) in MeOH (4 mL) was added a solution of LiOH $H_2O$ (50 mg, 1.19 mmol) in $H_2O$ (1 mL). The mixture was reacted at room temperature for 24 h. The reaction solution was diluted with $H_2O$ (10 mL), and HCl solution (1 mol/L) was added to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give a white solid (15 mg, 62%).

MS (ESI, pos.ion) m/z: 460.1 $[M+H]^+$.

**Step 4: Synthesis of 4-((2*S*,4*R*)-2-((3,3-difluoropropoxy)methyl)-4-(4-(trifluoromethyl) phenoxy)pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (11 mg, 0.05 mmol), 4-((2*S*,4*R*)-2-((3,3-difluoropropoxy)methyl)-4-(4-trifluoromethyl)phenoxy)pyrrolidin-1-yl)benzoic acid (15 mg, 0.03 mmol), EDCI (12 mg, 0.06 mmol), HOBT (8 mg, 0.06 mmol) and TEA (6 mg, 0.06 mmol) were dissolved in DCM (2 mL), and the mixture was stirred at room temperature for 13 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (15 mg, 69%).

MS (ESI, pos.ion) m/z: 671.1 $[M+H]^+$.

$^1H$ NMR (400 MHz, $CDCl_3$) $\delta$ (ppm): 7.86 (d, *J* = 7.8 Hz, 2H), 7.76 (d, *J* = 8.4 Hz, 2H), 7.58 (d, *J* = 8.1 Hz, 4H), 7.01 (s, 1H), 6.97 (d, *J* = 8.4 Hz, 2H), 6.63 (d, *J* = 8.3 Hz, 2H), 6.06 - 5.75 (m, 1H), 5.28 (s, 1H), 5.21 (s, 1H), 4.29 (s, 1H), 4.07 - 3.95 (m, 2H), 3.90 (dd, *J* = 10.3, 5.9 Hz, 1H), 3.62 - 3.52 (m, 4H), 3.10 (q, *J* = 7.3 Hz, 2H), 2.56 - 2.50 (m, 1H), 2.47 - 2.39 (m, 1H), 2.30 - 2.20 (m, 1H), 2.14 - 2.03 (m, 2H), 1.29 (t, *J*= 7.3 Hz, 3H).

**Example 45 4-((2*S*,4*S*)-2-((1,1-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)benzamide**

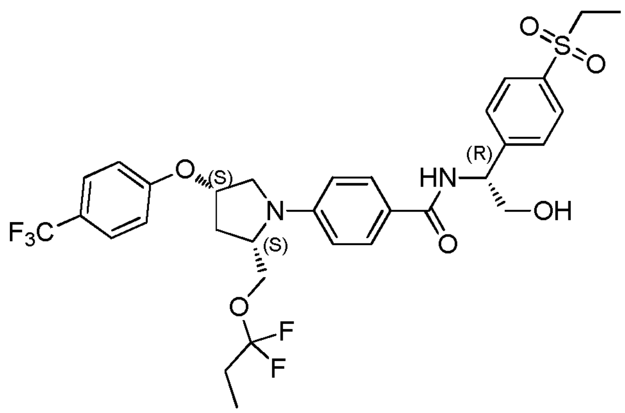

Using (2*S*,4*R*)-1-*tert*-butyl 2-methyl 4-hydroxypyrrolidine-1,2-dicarboxylate as raw material, the title compound was prepared as a white solid (60 mg, 69%) by referring to the methods of step 1 to step 4 of Example 18 and the methods of step 1 to step 4 of Example 43.

MS (ESI, pos.ion) m/z: 671.1 $[M+H]^+$;

$^1H$ NMR (400 MHz, $CDCl_3$) $\delta$ (ppm): 7.84 (d, *J* = 8.2 Hz, 2H), 7.75 (d, *J* = 8.6 Hz, 2H), 7.58 (d, *J* = 9.3 Hz, 2H), 7.56 (d, *J* = 9.3 Hz, 2H), 7.00 (d, *J* = 6.4 Hz, 1H), 6.98 (d, *J*= 8.7 Hz, 2H), 6.65 (d, *J* = 8.6 Hz, 2H), 5.26 (s, 1H), 5.16 (s, 1H), 4.22 - 4.14 (m, 2H), 4.02 (dd, *J* = 11.2, 3.5 Hz, 1H), 3.97 - 3.91 (m, 2H), 3.75 - 3.67 (m, 2H), 3.08 (q, *J* = 7.4 Hz, 2H), 2.49 (d, *J* = 14.3 Hz, 1H), 2.41 - 2.32 (m, 1H), 1.99 - 1.89 (m, 2H), 1.28 (t, *J* = 7.4 Hz, 3H), 1.00 (t, *J*= 7.5 Hz, 3H).

### Example 46 4-((2*R*,4*R*)-2-(2-(difluoromethoxy)ethyl)-4-(4-trifluoromethyl)phenoxy) pyrrolidin-1-yl)-*N*-((*R*)-1-(4-(ethylsulfonyl)phenyl)-2-hydroxyethyl)benzamide

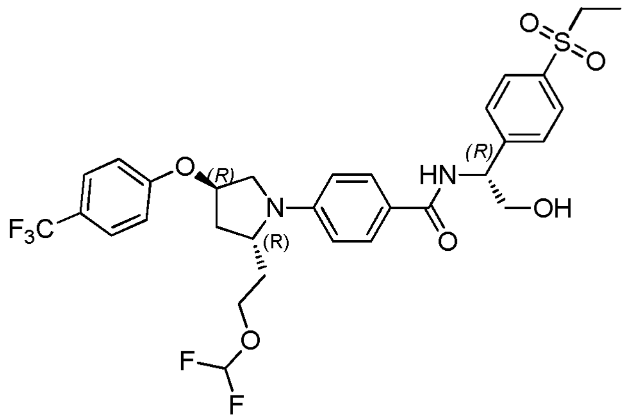

### Step 1: Synthesis of *tert*-butyl (2*S*,4*R*)-2-(((methylsulfonyl)oxy)methyl) -4-(4-(trifluoromethyl)phenoxy)pyrrolidine-1-carboxylate

Under nitrogen protection, to a solution of TEA (0.40 mL, 2.87 mmol) and *tert-butyl* (2*S*,4*R*)-2-(hydroxymethyl)-4-(4-(trifluoromethyl)phenoxy)pyrrolidine-1-carboxylate (345 mg, 0.95 mmol) in DCM (8 mL) was added MsCl (0.15 mL, 1.90 mmol). The mixture was stirred at room temperature for 15 h. To the reaction solution was added saturated NaCl solution (20 mL) to quench the reaction. The resulting mixture was diluted with DCM (50 mL), extracted and separated. The organic phase was dried over anhydrous $Na_2SO_4$, and concentrated under reduced pressure to give pale yellow liquid (419 mg, 100%).

MS (ESI, pos.ion) m/z: 462.3 $[M+Na]^+$.

### Step 2: Synthesis of *tert*-butyl (2*R*,4*R*)-2-(cyanomethyl)-4-(4-(trifluoromethyl) phenoxy)pyrrolidine-1-carboxylate

*tert*-Butyl (2*S*,4*R*)-2-(((methylsulfonyl)oxy)methyl)-4-(4-(trifluoromethyl)phenoxy) pyrrolidin-1-carboxylate (400 mg, 0.91 mmol) and N(*n*-Bu)$_4$CN (366 mg, 1.36 mmol) were added to ACN (8 mL), and the mixture was heated and stirred in an oil bath at 85°C for 4 h. The reaction solution was cooled to room temperature and concentrated under reduced pressure, and the crude product was separated by silica gel column chromatography (eluent: PE/EtOAc (v/v) = 4/1) to give colorless liquid (230 mg, 68%).

MS (ESI, pos.ion) m/z: 315.2 $[M-56+H]^+$.

### Step 3: Synthesis of methyl 2-((2*S*,4*R*)-4-(4-(trifluoromethyl) phenoxy)pyrrolidin-2-yl)acetate

To a solution of *tert*-butyl (2*R*,4*R*)-2-(cyanomethyl)-4-(4-(trifluoromethyl) phenoxy)pyrrolidine-1-carboxylate (1.30 g, 3.51 mmol) in MeOH (4 mL) was added a solution of HCl in MeOH (6 mL, 20%). The mixture was heated and stirred in an oil bath at 80°C for 24 h. The reaction solution was cooled to room temperature and concentrated under reduced pressure. The residue was washed with saturated $NaHCO_3$ solution (30 mL), extracted with EtOAc (40 mL×2). The organic phases were combined and washed with saturated NaCl solution (30 mL), dried over anhydrous $Na_2SO_4$, and concentrated under reduced pressure to give brown liquid (780 mg, 73%).

MS (ESI, pos.ion) m/z: 304.3 $[M+H]^+$.

**Step 4: Synthesis of 2-((2*R*,4*R*)-4-(4-(trifluoromethyl)phenoxy)pyrrolidin-2-yl)ethanol**

To a solution of methyl 2-((2*S*,4*R*)-4-(4-(trifluoromethyl)phenoxy) pyrrolidin-2-yl)acetate (700 mg, 2.31 mmol) in THF (12 mL) was added $LiBH_4$ (200 mg, 9.18 mmol). The mixture was stirred at room temperature for 15 h. Saturated $NH_4Cl$ (15 mL) solution was added to the reaction solution to quench the reaction. The mixture was left standing for layers, the upper organic phase was separated, and the aqueous phase was extracted with EtOAc (15 mL × 2). The combined organic phases were washed with saturated NaCl solution (15 mL), dried over anhydrous $Na_2SO_4$, and concentrated under reduced pressure. The crude product was separated by silica gel column chromatography (eluent: DCM/MeOH (v/v) = 10/1) to give brown liquid (100 mg, 16%).
MS (ESI, pos.ion) m/z: 276.3 $[M+H]^+$.

**Step 5: Synthesis of methyl 4-((2R,4R)-2-(2-hydroxyethyl)-4-(4-(trifluoromethyl)phenoxy)pyrrolidin-1-yl)benzoate**

Under nitrogen protection, 2-((2*R*,4*R*)-4-(4-(trifluoromethyl)phenoxy) pyrrolidin-2-yl)ethanol (100 mg, 0.36 mmol), $Pd_2(dba)_3$ (33 mg, 0.04 mmol), XantPhos (31 mg, 0.05 mmol), $Cs_2CO_3$ (177 mg, 0.54 mmol) and methyl 4-iodobenzoate (123 mg, 0.47 mmol) were dissolved in 1,4-dioxane (8 mL) and the mixture was reacted in a 100°C oil bath for 16 h. The reaction solution was cooled to room temperature, then concentrated under reduced pressure. The residue was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 3/1) to give brown liquid (31 mg, 21%).
MS (ESI, pos.ion) m/z: 410.2 $[M+H]^+$.

**Step 6: Synthesis of methyl 4-((2*R*,4*R*)-2-(2-(difluoromethoxy)ethyl) -4-(4-(trifluoromethyl)phenoxy)pyrrolidin-1-yl)benzoate**

To a solution of methyl 4-((2*R*,4*S*)-2-(2-hydroxyethyl)-4-(4-(trifluoromethyl)phenoxy)pyrrolidin-1-yl)benzoate (30 mg, 0.07 mmol) and KOAc (90 mg, 0.92 mmol) in DCM/$H_2O$ (1 mL/1 mL) was added $TMSCF_2Br$ (0.20 mL, 1.29 mmol). The mixture was stirred at room temperature for 14 h. The reaction solution was added with DCM (20 mL), washed with saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give pale yellow liquid (19 mg, 56%).
MS (ESI, pos.ion) m/z: 460.3 $[M+H]^+$.

**Step 7: Synthesis of 4-((2*R*,4*R*)-2-(2-(difluoromethoxy)ethyl)-4-(4-(trifluoromethyl) phenoxy)pyrrolidin-1-yl)benzoic acid**

To a solution of methyl 4-((2*R*,4*R*)-2-(2-(difluoromethoxy)ethyl)-4-(4-(trifluoromethyl)phenoxy)pyrrolidin-1-yl)benzoate (19 mg, 0.04 mmol) in MeOH (2 mL) was added a solution of LiOH $H_2O$ (34 mg, 0.81 mmol) in $H_2O$ (1 mL). The mixture was reacted at room temperature for 24 h. The reaction solution was diluted with $H_2O$ (10 mL), and HCl solution (1 mol/L) was added to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 3/1) to give a white solid (14 mg, 76%).
MS (ESI, pos.ion) m/z: 446.2 $[M+H]^+$.

**Step 8: Synthesis of 4-((2*R*,4*R*)-2-(2-(difluoromethoxy)ethyl)-4-(4-(trifluoromethyl) phenoxy)pyrrolidin-1-yl)-*N*-((*R*)-1-(4-(ethylsulfonyl)phenyl)-2-hydroxyethyl)benzamide**

(*R*)-2-Amino-2-(4-(ethylsulfonyl)phenyl)ethanol (8 mg, 0.03 mmol), 4-((2*R*,4*R*)-2-(2-(difluoromethoxy)ethyl)-4-(4-trifluoromethyl)phenoxy)pyrrolidin-1-yl)benzoic acid (13 mg, 0.03 mmol), EDCI (11 mg, 0.06 mmol), HOBT (7 mg, 0.05 mmol) and TEA (6 mg, 0.06 mmol) were dissolved in DCM (2 mL), and the mixture was stirred at room temperature for 23 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (13 mg, 68%).
MS (ESI, pos.ion) m/z: 657.1 $[M+H]^+$.
$^1H$ NMR (400 MHz, $CDCl_3$) $\delta$ (ppm): 7.85 (d, *J* = 8.2 Hz, 2H), 7.74 (d, *J* = 8.7 Hz, 2H), 7.56 (d, *J* = 8.5, 2H),

7.55 (d, $J$ = 8.5, 2H), 6.96 (s, 1H), 6.94 (d, $J$ = 8.3 Hz, 2H), 6.61 (d, $J$ = 8.7 Hz, 2H), 6.24 (t, $J$ = 74.4 Hz, 1H), 5.27 (d, $J$ = 6.2 Hz, 1H), 5.10 (s, 1H), 4.23 (d, $J$ = 5.9 Hz, 1H), 4.03 - 3.88 (m, 5H), 3.57 (dd, $J$ = 10.8, 2.0 Hz, 1H), 3.08 (q, $J$ = 7.4 Hz, 2H), 2.59 - 2.48 (m, 2H), 2.34 - 2.27 (m, 2H), 1.27 (t, $J$ = 7.4 Hz, 3H).

**Example 47 4-((2*R*,4*S*)-2-(2-(difluoromethoxy)ethyl)-4-(4-trifluoromethyl)phenoxy) pyrrolidin-1-yl)-*N*-((*R*)-1-(4-(ethylsulfonyl)phenyl)-2-hydroxyethyl)benzamide**

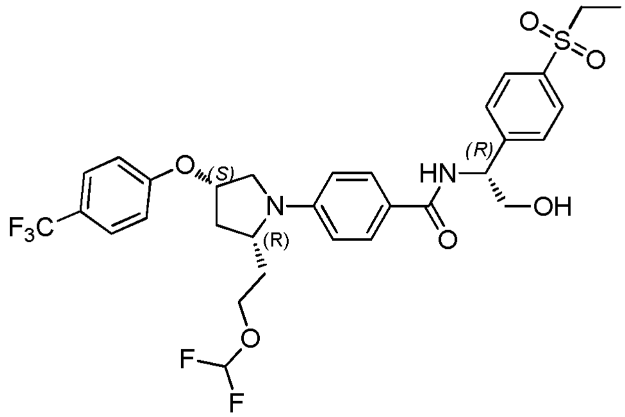

Using (2*S*,4*R*)-1-*tert*-butyl 2-methyl 4-hydroxypyrrolidine-1,2-dicarboxylate as raw material, the title compound was prepared as a white solid (22 mg, 75%) by referring to the methods of step 1 to step 2 of Example 18 and the methods of step 1 to step 8 of Example 46.

MS (ESI, pos.ion) m/z: 657.1 $[M+H]^+$;

$^1H$ NMR (400 MHz, $CDCl_3$) $\delta$ (ppm): 7.86 (d, $J$ = 8.1 Hz, 2H), 7.77 (d, $J$ = 8.6 Hz, 2H), 7.61 (d, $J$ = 9.0 Hz, 2H), 7.58 (d, $J$ = 8.3 Hz, 2H), 7.05 (d, $J$ = 6.8 Hz, 1H), 6.98 (d, $J$ = 8.4 Hz, 2H), 6.61 (d, $J$ = 8.6 Hz, 2H), 6.16 (d, $J$ = 74.5 Hz, 1H), 5.28 (s, 1H), 5.18 (s, 1H), 4.19 (t, $J$ = 7.6 Hz, 1H), 4.06 - 3.89 (m, 4H), 3.74 (d, $J$ = 2.0 Hz, 2H), 3.10 (q, $J$ = 7.4 Hz, 2H), 2.47 - 2.39 (m, 1H), 2.35 (d, $J$ = 14.0 Hz, 1H), 2.31 - 2.22 (m, 1H), 2.07 - 1.98 (m, 1H), 1.29 (t, $J$ = 7.4 Hz, 3H).

**Example 48 *N*-((R)-1-(4-(ethylsulfonyl)phenyl)-2-hydroxyethyl)-4-((2*S*,4*S*)-2-((trifluoromethoxy)methyl)-4-(4-(trifluoromethyl)phenoxy)pyrrolidin-1-yl)benzamide**

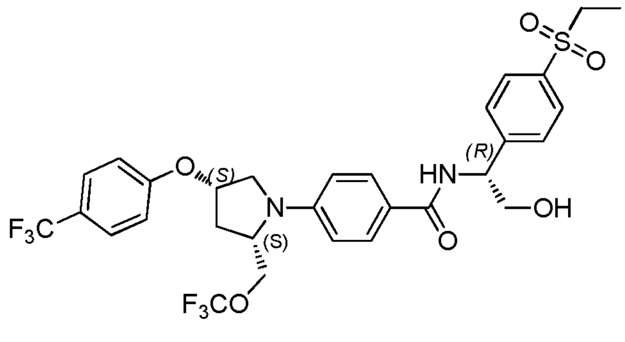

**Step 1: Synthesis of (2*S*,4*S*)-*t*-*tert*-butyl 2-methyl 4-(4-(trifluoromethyl)phenoxy)pyrrolidine-1,2-dicarboxylate**

(2*S*,4*R*)-1-*tert*-Butyl 2-methyl 4-hydroxypyrrolidine-1,2-dicarboxylate (2.00 g, 8.15 mmol), 4-(trifluoromethyl)phenol (1.45 g, 8.94 mmol) and $PPh_3$ (3.08 g, 8.97 mmol) were dissolved in THF (40 mL). Then the mixture was transferred to an ice bath, and DIAD (2.00 mL, 10.16 mmol) was slowly. After the addition was complete, the mixture was stirred at room temperature for 21 h. The reaction solution was concentrated under reduced pressure. The residue was diluted with methyl tert-butyl ether (30 mL) and the system was stirred at -20 °C. A large amount of white insoluble solids were precipitated, filtered while cold, and the filter cake was washed with cold methyl tert-butyl ether. The filtrate

was concentrated under reduced pressure, and the crude product was separated by silica gel column chromatography (eluent: PE/EtOAc (v/v) = 5/1) to give a white solid (2.51 g, 79%).

MS (ESI, pos.ion) m/z: 412.2 $[M+Na]^+$.

**Step 2: Synthesis of *tert*-butyl (2*S*,4*S*)-2-(hydroxymethyl)-4-(4-(trifluoromethyl)phenoxy)pyrrolidine-1-carboxylate**

(2*S*,4*S*)-1-*tert*-Butyl 2-methyl 4-(4-(trifluoromethyl)phenoxy)pyrrolidine -1,2-dicarboxylate (2.50 g, 6.42 mmol) was dissolved in THF (30 mL), then the mixture was transferred to an ice bath, and $LiBH_4$ (286 mg, 13.13 mmol) was added slowly. After the addition was completed, the mixture was stirred at room temperature for 12 h. Saturated $NH_4Cl$ (20 mL) solution was added to the reaction solution to quench the reaction. The mixture was left standing for layers, the upper organic phase was separated, and the aqueous phase was extracted with EtOAc (20 mL × 2). The combined organic phases were dried over anhydrous $Na_2SO_4$, concentrated under reduced pressure, and the crude product was separated by silica gel column chromatography (eluent: PE/EtOAc (v/v) = 3/1) to give colorless oily liquid (1.85 g, 80%).

MS (ESI, pos.ion) m/z: 306.2 $[M-56+H]^+$.

**Step 3: Synthesis of *tert*-butyl (2*S*,4*S*)-2-((trifluoromethoxy)methyl)-4-(4-(trifluoromethyl)phenoxy)pyrrolidine-1-carboxylate**

Under nitrogen protection, *tert*-butyl (2*S*,4*S*)-2-(hydroxymethyl)-4-(4-(trifluoromethyl)phenoxy)pyrrolidine-1-carboxylate(500 mg, 1.38 mmol), AgOTf (1.07 g, 1.15 mmol), selective fluorine reagent (735 mg, 2.07 mmol) and KF (322 mg, 5.54 mmol) were dissolved in EtOAc (6 mL), then 2-fluoropyridine (0.40 mL, 4.65 mmol) and $TMSCF_3$ (0.60 mL, 4.06 mmol) were added in turn. The mixture was stirred at room temperature for 24 h. The reaction solution was filtered and concentrated under reduced pressure, and the crude product was separated by silica gel column chromatography (eluent: PE/EtOAc (v/v) = 4/1) to give colorless liquid (90 mg, 15%).

MS (ESI, pos.ion) m/z: 374.0 $[M-56+H]^+$.

**Step 4: Synthesis of (2*S*,4*S*)-2-((trifluoromethoxy)methyl)-4-(4-(trifluoromethyl)phenoxy)pyrrolidine**

To a solution of *tert*-butyl (2*S*,4*S*)-2-((trifluoromethoxy)methyl)-4-(4-(trifluoromethyl)phenoxy)pyrrolidine-1-carboxylate(80 mg, 0.19 mmol) in DCM (4 mL) was added a solution of HCl in methanol (1 mL, 20%). The mixture was stirred at room temperature for 5 h. After the reaction was completed, saturated sodium bicarbonate was added for washing, and the reaction solution was concentrated under reduced pressure to give brown liquid (60 mg, 98%).

MS (ESI, pos.ion) m/z: 330.2 $[M+H]^+$.

**Step 5: Synthesis of methyl 4-((2*S*,4*S*)-2-((trifluoromethoxy)methyl)-4-(4-(trifluoromethyl)phenoxy)pyrrolidin-1-yl)benzoate**

Under nitrogen protection, (2*S*,4*S*)-2-((trifluoromethoxy)methyl) -4-(4-(trifluoromethyl)phenoxy)pyrrolidine (50 mg, 0.15 mmol), methyl 4-iodobenzoate (59 mg, 0.23 mmol), $Pd_2(dba)_3$ (20 mg, 0.02 mmol), XantPhos (17 mg, 0.03 mmol) and $Cs_2CO_3$ (98 mg, 0.31 mmol) were dissolved in 1,4-dioxane (4 mL) and the mixture was heated and stirred in a 100°C oil bath for 18 h. The reaction solution was cooled to room temperature, and concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give yellow liquid (33 mg, 47%).

MS (ESI, pos.ion) m/z: 464.0 $[M+H]^+$.

**Step 6: Synthesis of 4-((2*S*,4*S*)-2-((trifluoromethoxy)methyl)-4-(4-(trifluoromethyl) phenoxy)pyrrolidin-1-yl)benzoic acid**

To a solution of methyl 4-((2*S*,4*S*)-2-((trifluoromethoxy)methyl)-4-(4-(trifluoromethyl)phenoxy)pyrrolidin-1-yl)benzoate (30 mg, 0.06 mmol) in MeOH (4 mL) was added a solution of LiOH $H_2O$ (54 mg, 1.29 mmol) in $H_2O$ (1 mL). The mixture was reacted at room temperature for 22 h. To the reaction solution was added HCl solution (1 mol/L) to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (25 mg, 86%).

MS (ESI, pos.ion) m/z: 450.1 $[M+H]^+$.

**Step 7: Synthesis of *N*-((*R*)-1-(4-(ethylsulfonyl)phenyl)-2-hydroxyethyl)-4-((2*S*,4*S*) -2-((trifluoromethoxy)methyl)-4-(4-(trifluoromethyl)phenoxy)pyrrolidin-1-yl)benzamide**

(*R*)-2-Amino-2-(4-(ethylsulfonyl)phenyl)ethanol (16 mg, 0.07 mmol), 4-((2*S*,4*S*)-2-((trifluoromethoxy)methyl)-4-(4-trifluoromethyl)phenoxy)pyrrolidin-1-yl)benzoic acid (25 mg , 0.06 mmol), EDCI (21 mg, 0.11 mmol), HOBT (15 mg, 0.11 mmol) and TEA(28 mg, 0.28 mmol) were dissolved in DCM (4 mL), and the mixture was stirred at room temperature for 15 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a pale yellow solid (30 mg, 82%).

MS (ESI, pos.ion) m/z: 661.2 $[M+H]^+$.

$^1H$ NMR (400 MHz, $CDCl_3$) $\delta$ (ppm): 7.80 (d, *J* = 8.3 Hz, 2H), 7.77 (d, *J* = 8.7 Hz, 2H), 7.55 (d, *J* = 8.3 Hz, 2H), 7.54 (d, *J* = 8.5 Hz, 2H), 7.47 (d, *J* = 7.0 Hz, 1H), 6.93 (d, *J* = 8.5 Hz, 2H), 6.61 (d, *J*= 8.7 Hz, 2H), 5.20 (d, *J* = 5.2 Hz, 1H), 5.16 (t, *J* = 4.5 Hz, 1H), 4.46 (s, 1H), 4.25 - 4.17 (m, 2H), 4.08 (d, *J* = 9.0 Hz, 1H), 3.96 - 3.90 (m, 1H), 3.87 - 3.81 (m, 1H), 3.73 (d, *J*= 11.3 Hz, 1H), 3.67 (dd, *J* = 11.4, 4.5 Hz, 1H), 3.06 (q, *J*= 7.4 Hz, 2H), 2.48 (d, *J* = 14.4 Hz, 1H), 2.42 - 2.34 (m, 1H), 1.22 (t, *J* = 7.4 Hz, 3H).

**Example 49 *N*-((*R*)-1-(4-(ethylsulfonyl)phenyl)-2-hydroxyethyl)-6-((2*S*,4*R*)-2-((2-fluoroethoxy)methyl)-4-(4-(trifluoromethyl)phenoxy)pyrrolidin-1-yl)nicotinamide**

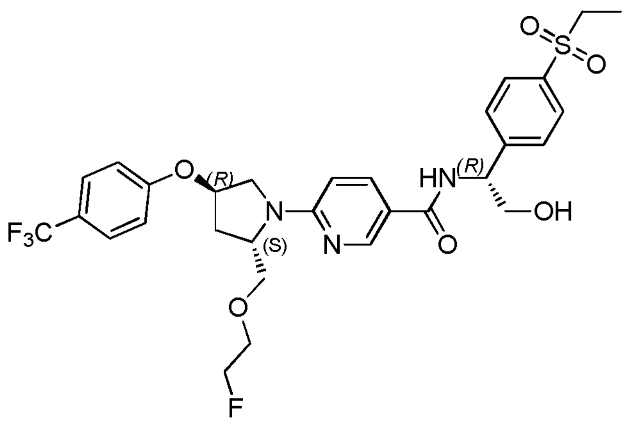

**Step 1: Synthesis of methyl 6-((2*S*,4*R*)-2-(hydroxymethyl)-4-(4-(trifluoromethyl)phenoxy)pyrrolidin-1-yl)nicotinate**

((2*S*,4*R*)-4-(4-(trifluoromethyl)phenoxy)pyrrolidin-2-yl)methanol (1.50 g, 5.74 mmol), methyl 6-fluoronicotinate (2.10 g, 12.24 mmol) and $K_2CO_3$ (2.11 g, 15.19 mmol) were dissolved in DMSO (10 mL). The mixture was heated and stirred in an oil bath at 50°C for 24 h. The reaction solution was cooled to room temperature, then added with EtOAc (100 mL), washed successively with H2O (30 mL × 2) and saturated NaCl solution (30 mL×3), dried over anhydrous $Na_2SO_4$, and concentrated under reduced pressure. The crude product was separated by silica gel column chromatography (eluent: PE/EtOAc (v/v) = 3/1) to give yellow liquid (1.18 g, 52%).

MS (ESI, pos.ion) m/z: 397.1 $[M+H]^+$.

**Step 2: Synthesis of methyl 6-((2*S*,4*R*)-2-((2-fluoroethoxy)methyl) -4-(4-(trifluoromethyl)phenoxy)pyrrolidin-1-yl)nicotinate**

Under nitrogen protection, to a solution of methyl 6-((2*S*,4*R*)-2-(hydroxymethyl)-4-(4-(trifluoromethyl)phenoxy)pyrrolidin-1-yl)nicotinate(400mg, 1.00 mmol) and 1-bromo-2-fluoroethane (1.50 mL, 20.09 mmol) in THF (8 mL) was added NaH (250 mg, 6.25 mmol, 60%). The mixture was stirred at room temperature for 20 h. The reaction solution was added with HCl solution (1 mol/L) to quench the reaction, and the pH of the solution was adjusted to about 5. The resulting mixture was diluted with EtOAc (50 mL), extracted and separated, and the organic phase was washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give yellow liquid (352

mg, 79%).

MS (ESI, pos.ion) m/z: 443.2 $[M+H]^+$.

**Step 3: Synthesis of 6-((2*S*,4*R*)-2-((2-fluoroethoxy)methyl)-4-(4-(trifluoromethyl)phenoxy)pyrrolidin-1-yl)nicotinic acid**

To a solution of methyl 6-((2*S*,4*R*)-2-((2-fluoroethoxy)methyl)-4-(4-**(trifluoromethyl)phenoxy)pyrrolidin-1-yl)nicotinate** (150 mg, 0.33 mmol) in MeOH (4 mL) was added a solution of $LiOH \cdot H_2O$ (275 mg, 6.55 mmol) in $H_2O$ (2 mL). The mixture was reacted at room temperature for 22 h. The reaction solution was added with HCl solution (1 mol/L) to adjust the pH of the solution to about 5. The resulting mixture was extracted with EtOAc (30 mL × 2). The organic phases were combined, washed with saturated NaCl (20 mL) solution, dried over anhydrous $Na_2SO_4$, and concentrated under reduced pressure. The crude product was separated by silica gel column chromatography (eluent: DCM/EtOAc (v/v) = 4/1) to give a yellow solid (120 mg, 85%).

MS (ESI, pos.ion) m/z: 429.3 $[M+H]^+$.

**Step 4: Synthesis of *N*-((*R*)-1-(4-(ethylsulfonyl)phenyl)-2-hydroxyethyl)-6-((2*S*,4*R*) -2-((2-fluoroethoxy)methyl)-4-(4-(trifluoromethyl)phenoxy)pyrrolidin-1-yl)nicotinamide**

(*R*)-2-Amino-2-(4-(ethylsulfonyl)phenyl)ethanol (55 mg, 0.24 mmol), 6-((2*S*,4*R*)-2-((2-fluoroethoxy)methyl)-4-(4-(trifluoromethyl)phenoxy)pyrrolidin-1-yl)nicotinic acid (80 mg , 0.19 mmol), HATU (160 mg, 0.42 mmol) and DIPEA(60 mg, 0.46 mmol) were dissolved in DCM (4 mL). The mixture was stirred at room temperature for 7 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (96 mg, 54%).

MS (ESI, pos.ion) m/z: 640.3 $[M+H]^+$.

$^1H$ NMR (400 MHz, $CDCl_3$) δ (ppm): 8.73 (s, 1H), 7.97 (d, *J* = 8.9 Hz, 1H), 7.83 (d, *J* = 8.2 Hz, 2H), 7.57 (d, *J* = 8.3 Hz, 2H), 7.56 (d, *J* = 8.6 Hz, 2H), 7.45 (d, *J* = 6.6 Hz, 1H), 6.97 (d, *J* = 8.6 Hz, 2H), 6.47 (d, *J* = 8.9 Hz, 1H), 5.32 - 5.24 (m, 2H), 4.61 - 4.56 (m, 2H), 4.49 - 4.42 (m, 1H), 4.05 - 3.92 (m, 3H), 3.87 (dd, *J* = 9.6, 3.6 Hz, 1H), 3.72 - 3.62 (m, 4H), 3.09 (q, *J* = 7.4 Hz, 2H), 2.60-2.55 (m, 1H), 2.44 - 2.36 (m, 1H), 1.27 (t, *J* = 7.4 Hz, 3H).

**Example 50 6-((2*S*,4*R*)-2-((cyclopropylmethoxy)methyl)-4-(4-trifluoromethyl)phenoxy) pyrrolidin-1-yl)-*N*-((*R*)-1-(4-(ethylsulfonyl)phenyl)-2-hydroxyethyl)nicotinamide**

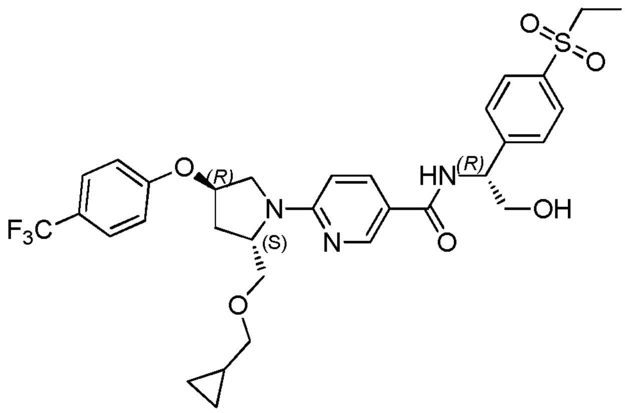

Using (bromomethyl)cyclopropane (1.00 mL, 10.30 mmol) as raw material, the title compound was prepared as a white solid (76 mg, 47%) by referring to the methods of step 2 to step 4 of Example 49.

MS (ESI, pos.ion) m/z: 648.4 $[M+H]^+$;

$^1H$ NMR (400 MHz, $CDCl_3$) δ (ppm): 8.67 (d, *J* = 2.0 Hz, 1H), 7.92 (dd, *J* = 8.9, 2.2 Hz, 1H), 7.83 (d, *J*= 8.3 Hz, 2H), 7.56 (d, *J*= 8.1 Hz, 4H), 7.25 (d, *J*= 7.0 Hz, 1H), 6.97 (d, *J*= 8.6 Hz, 2H), 6.44 (d, *J* = 8.9 Hz, 1H), 5.29 - 5.24 (m, 2H), 4.54 (d, *J* = 3.6 Hz, 1H), 4.00 (dd, *J* = 11.4, 4.0 Hz, 1H), 3.97 - 3.91 (m, 2H), 3.76 - 3.70 (m, 2H), 3.63 (dd, *J* = 9.7, 2.6 Hz, 1H), 3.27 (d, *J* = 6.7 Hz, 2H), 3.09 (q, *J* = 7.4 Hz, 2H), 2.60 - 2.54 (m, 1H), 2.43 - 2.35 (m, 1H), 1.27 (t, *J* = 7.4 Hz, 3H), 1.03 - 0.97 (m, 1H), 0.50 (d, *J* = 8.0 Hz, 2H), 0.18 (d, *J* = 4.6 Hz, 2H).

**Example 51 6-((2*R*,4*R*)-2-(cyanomethyl)-4-(4-trifluoromethyl)phenoxy)pyrrolidin-1-yl) -*N*-((*R*)-1-(4-(ethylsulfonyl)phenyl)-2-hydroxyethyl)nicotinamide**

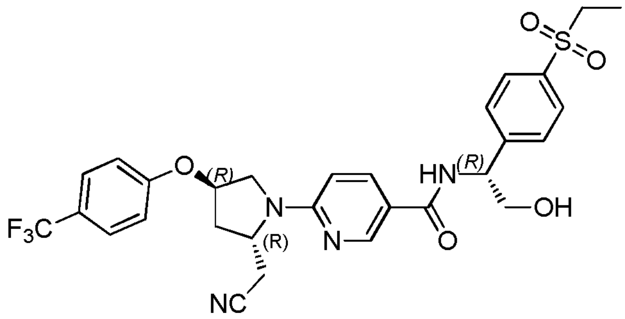

**Step 1: Synthesis of methyl 6-((2*R*,4*R*)-2-(cyanomethyl)-4-(4-(trifluoromethyl) phenoxy)pyrrolidin-1-yl)nicotinate**

Under nitrogen protection, 2-((2*R*,4*R*)-4-(4-(trifluoromethyl)phenoxy) pyrrolidin-2-yl)acetonitrile (60 mg, 0.22 mmol), methyl 6-bromonicotinate (60 mg, 0.28 mmol), $Pd_2(dba)_3$ (20 mg, 0.02 mmol), XantPhos (20 mg, 0.03 mmol) and $Cs_2CO_3$ (100 mg, 0.31 mmol) were dissolved in 1,4-dioxane (4 mL), and the mixture was heated and stirred in a 100°C oil bath for 11 h. The reaction solution was cooled to room temperature, and concentrated under reduced pressure. The residue was diluted with DCM (50 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 3/1) to give yellow liquid (40 mg, 44%).
MS (ESI, pos.ion) m/z: 406.3 $[M+H]^+$.

**Step 2: Synthesis of 6-((2*R*,4*R*)-2-(cyanomethyl)-4-(4-(trifluoromethyl) phenoxy)pyrrolidin-1-yl)nicotinic acid**

To a solution of methyl 6-((2*R*,4*R*)-2-(cyanomethyl)-4-(4-(trifluoromethyl) phenoxy)pyrrolidin-1-yl)nicotinate (40 mg, 0.10 mmol) in MeOH (4 mL) was added a solution of $LiOH{\cdot}H_2O$ (80 mg, 1.91 mmol) in $H_2O$ (1 mL). The mixture was reacted at room temperature for 6 h. The reaction solution was added with HCl solution (1 mol/L) to adjust the pH of the solution to about 5. The resulting mixture was extracted with EtOAc (30 mL $\times$ 2), and the organic phases were combined, washed with saturated NaCl (20 mL) solution, dried over anhydrous $Na_2SO_4$, and concentrated under reduced pressure. The crude product was separated by silica gel column chromatography (eluent: DCM/MeOH (v/v) = 20/1) to give a yellow solid (30 mg, 80%).
MS (ESI, pos.ion) m/z: 392.1 $[M+H]^+$.

**Step 3: Synthesis of 6-((2*R*,4*R*)-2-(cyanomethyl)-4-(4-(trifluoromethyl) phenoxy)pyrrolidin-1-yl)-*N*-((*R*)-1-(4-(ethylsulfonyl)phenyl)-2-hydroxyethyl)nicotinamide**

(*R*)-2-Amino-2-(4-(ethylsulfonyl)phenyl)ethanol (8 mg, 0.03 mmol), 6-((2*R*,4*R*)-2-(cyanomethyl)-4-(4-(trifluoromethyl)phenoxy)pyrrolidin-1-yl)nicotinic acid (10 mg, 0.03 mmol), HATU (20 mg, 0.05 mmol) and DIPEA (9 mg, 0.07 mmol) were dissolved in DCM (4 mL). The mixture was stirred at room temperature for 13 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (5 mg, 32%).
MS (ESI, pos.ion) m/z: 603.3 $[M+H]^+$;
$^1H$ NMR (400 MHz, $CDCl_3$) $\delta$ (ppm): 8.69 (s, 1H), 7.96 (d, *J* = 8.8 Hz, 1H), 7.86 (d, *J* = 8.1 Hz, 2H), 7.61 - 7.52 (m, 4H), 7.12 (s, 1H), 6.94 (d, *J* = 8.5 Hz, 2H), 6.46 (d, *J* = 8.8 Hz, 1H), 5.27 (d, *J* = 21.5 Hz, 2H), 4.64 (s, 1H), 4.06 - 3.94 (m, 3H), 3.70 (d, *J* = 13.9 Hz, 1H), 3.45 (dd, *J* = 16.8, 5.6 Hz, 1H), 3.09 (q, *J*= 7.4 Hz, 2H), 2.81 (d, *J* = 14.7 Hz, 1H), 2.67 - 2.60 (m, 1H), 2.49 - 2.42 (m, 1H), 1.33 (t, *J* = 7.4 Hz, 3H).

**Example 52 6-((2*S*,4*R*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)nicotinamide**

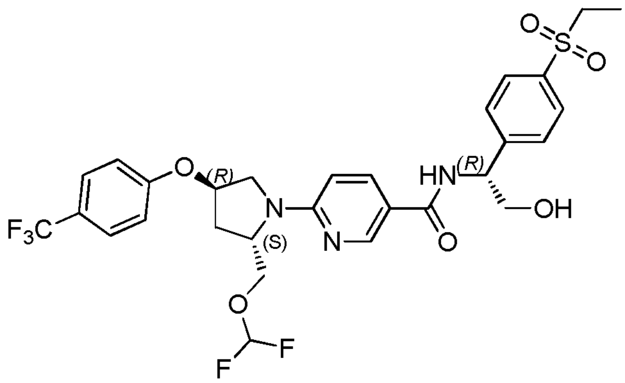

**Step 1: Synthesis of methyl 6-((2*S*,4*R*)-2-((difluoromethoxy)methyl)-4-(4-(trifluoromethyl) phenoxy)pyrrolidin-1-yl)nicotinate**

Under nitrogen protection, methyl 6-((2*S*,4*R*)-2-(hydroxymethyl)-4-(4-(trifluoromethyl)phenoxy)pyrrolidin-1-yl)nicotinate (300 mg, 0.76 mmol) and CuI (144 mg, 0.76 mmol) were added to ACN (6 mL), and 2,2-difluoro-2-(fluorosulfonyl)acetic acid (0.40 mL, 3.87 mmol) was slowly added dropwise while the mixture was stirred at 50°C. After the addition was completed, the mixture was continued stirring for 23 h. The reaction solution was cooled to room temperature, and filtered. The filtrate was added with saturated $NaHCO_3$ solution (20 mL), diluted with EtOAc (50 mL), extracted and separated. The organic phase was washed with saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give colorless liquid (85 mg, 25%).

MS (ESI, pos.ion) m/z: 447.2 $[M+H]^+$.

**Step 2: Synthesis of 6-((2*S*,4*R*)-2-((difluoromethoxy)methyl)-4-(4-(trifluoromethyl) phenoxy)pyrrolidin-1-yl)nicotinic acid**

To a solution of methyl 6-((2*S*,4*R*)-2-((difluoromethoxy)methyl)-4-(4-(trifluoromethyl)phenoxy)pyrrolidin-1-yl)nicotinate (80 mg, 0.17 mmol) in MeOH (4 mL) was added a solution of $LiOH \cdot H_2O$ (145 mg, 3.47 mmol) in $H_2O$ (1 mL). The mixture was reacted at room temperature for 15 h. The reaction solution was added with HCl solution (1 mol/L) to adjust the pH of the solution to about 5. The resulting mixture was extracted with EtOAc (30 mL $\times$ 2), and the organic phases were combined, washed with saturated NaCl (20 mL) solution, dried over anhydrous $Na_2SO_4$, and concentrated under reduced pressure. The crude product was separated by silica gel column chromatography (eluent: DCM/EtOAc (v/v) = 4/1) to give a yellow solid (65 mg, 87%).

MS (ESI, pos.ion) m/z: 433.0 $[M+H]^+$.

**Step 3: Synthesis of 6-((2*S*,4*R*)-2-((difluoromethoxy)methyl)-4-(4-(trifluoromethyl) phenoxy)pyrrolidin-1-yl)-*N*-((*R*)-1-(4-(ethylsulfonyl)phenyl)-2-hydroxyethyl)nicotinamide**

(*R*)-2-Amino-2-(4-(ethylsulfonyl)phenyl)ethanol (45 mg, 0.20 mmol), 6-((2*S*,4*R*)-2-((difluoromethoxy)methyl)-4-(4-(trifluoromethyl)phenoxy)pyrrolidin-1-yl)nicotinic acid (65 mg, 0.15 mmol), HATU (114 mg, 0.30 mmol) and DIPEA (58 mg, 0.45 mmol) were dissolved in DCM (4 mL). The mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (45 mg, 47%).

MS (ESI, pos.ion) m/z: 644.3 $[M+H]^+$.

$^1H$ NMR (400 MHz, $CDCl_3$) $\delta$ (ppm): 8.66 (d, *J* = 2.1 Hz, 1H), 7.92 (dd, *J* = 8.9, 2.3 Hz, 1H), 7.82 (d, *J* = 8.3 Hz, 2H), 7.58 - 7.51 (m, 4H), 7.19 (d, *J* = 7.1 Hz, 1H), 6.94 (d, *J* = 8.6 Hz, 2H), 6.41 (d, *J*= 8.9 Hz, 1H), 6.19 (t, *J* = 74.6 Hz, 1H), 5.28 - 5.24 (m, 1H), 5.20 - 5.15 (m, 1H), 4.68 - 4.62 (m, 1H), 4.29 (dd, *J*= 10.2, 4.5 Hz, 1H), 4.04 (dd, *J*= 10.4, 2.5 Hz, 1H), 4.00 (dd, *J*= 10.4, 2.5 Hz, 1H), 3.95 - 3.89 (m, 2H), 3.69 (dd, *J* = 11.0, 2.8 Hz, 1H), 3.07 (q, *J*= 7.4 Hz, 2H), 2.56 - 2.49 (m, 1H), 2.47-2.41 (m, 1H), 1.25 (t, *J*= 7.4 Hz, 3H).

**Example 53 6-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)nicotinamide**

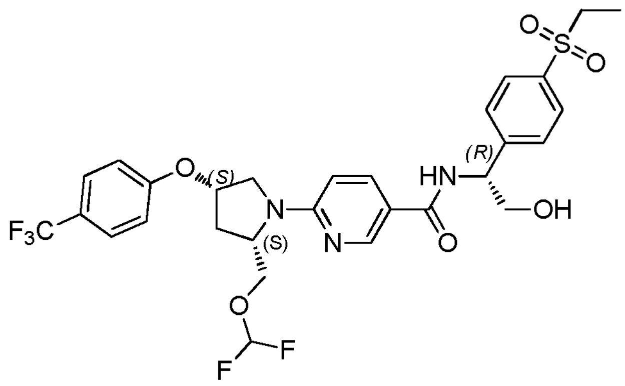

**Step 1: Synthesis of methyl 6-((2*S*,4*S*)-2-((difluoromethoxy)methyl)-4-(4-(trifluoromethyl)phenoxy)pyrrolidin-1-yl)nicotinate**

To a solution of methyl 6-((2*S*,4*S*)-2-(hydroxymethyl)-4-(4-(trifluoromethyl) phenoxy)pyrrolidin-1-yl)nicotinate (130 mg, 0.33 mmol) (it was prepared by the methods of step 1 to step 4 of Example 18 using (2*S*,4*R*)-1-*tert*-butyl 2-methyl 4-hydroxypyrrolidine-1,2-dicarboxylate as raw material) and KOAc (400 mg, 4.08 mmol) in DCM/$H_2O$ (1 mL/1 mL) was added $TMSCF_2Br$ (0.50 mL, 3.22 mmol). The mixture was stirred at room temperature for 24 h. The reaction solution was diluted with DCM (30 mL), washed with saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give pale yellow liquid (60 mg, 41%).
MS (ESI, pos.ion) m/z: 447.1 $[M+H]^+$.

**Step 2: Synthesis of 6-((2*S*,4*S*)-2-((difluoromethoxy)methyl)-4-(4-(trifluoromethyl)phenoxy)pyrrolidin-1-yl)nicotinic acid**

To a solution of methyl 6-((2*S*,4*S*)-2-((difluoromethoxy)methyl) -4-(4-(trifluoromethyl)phenoxy)pyrrolidin-1-yl)nicotinate (60 mg, 0.13 mmol) in MeOH (6 mL) was added a solution of $LiOH \cdot H_2O$ (100 mg, 2.38 mmol) in $H_2O$ (2 mL). The mixture was reacted at room temperature for 15 h. The reaction solution was added with HCl solution (1 mol/L) to adjust the pH of the solution to about 4. The resulting mixture was extracted with EtOAc (20 mL $\times$ 2), and the organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (36 mg, 64%).
MS (ESI, pos.ion) m/z: 433.1 $[M+H]^+$.

**Step 3: Synthesis of 6-((2*S*,4*S*)-2-((difluoromethoxy)methyl)-4-(4-(trifluoromethyl) phenoxy)pyrrolidin-1-yl)-*N*-((*R*)-1-(4-(ethylsulfonyl)phenyl)-2-hydroxyethyl)nicotinamide**

(*R*)-2-Amino-2-(4-(ethylsulfonyl)phenyl)ethanol (22 mg, 0.10 mmol), 6-((2*S*,4*S*)-2-((difluoromethoxy)methyl)-4-(4-trifluoromethyl)phenoxy)pyrrolidin-1-yl)nicotinic acid (35 mg , 0.08 mmol), EDCI (23 mg, 0.12 mmol), HOBT (16 mg, 0.12 mmol) and TEA (12 mg, 0.12 mmol) were dissolved in DCM (4 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (30 mg, 58%).
MS (ESI, pos.ion) m/z: 644.1 $[M+H]^+$;
$^1H$ NMR (400 MHz, $CDCl_3$) $\delta$ (ppm): 8.74 (s, 1H), 7.98 (s, 1H), 7.84 (d, *J* = 6.6 Hz, 2H), 7.58 (d, *J*= 8.0 Hz, 4H), 7.26 - 7.21 (m, 1H), 6.97 (d, *J*= 8.1 Hz, 2H), 6.51 (s, 1H), 6.22 (t, *J*= 74.4 Hz, 1H), 5.30 (s, 1H), 5.16 (s, 1H), 4.54 (s, 1H), 4.31 (d, *J* = 6.8 Hz, 1H), 4.07 - 3.93 (m, 3H), 3.85 (s, 2H), 3.08 (q, *J*= 7.4 Hz, 2H), 2.52 (d, *J*= 13.8 Hz, 1H), 2.42 (s, 1H), 1.27 (t, *J*= 7.4 Hz, 3H).

**Example 54 6-((2*S*,4*R*)-2-((1,1-difluoropropoxy)methyl)-4-(4-trifluoromethyl) phenoxy)pyrrolidin-1-yl)-*N*-((*R*)-1-(4-(ethylsulfonyl)phenyl)-2-hydroxyethyl)nicotinamide**

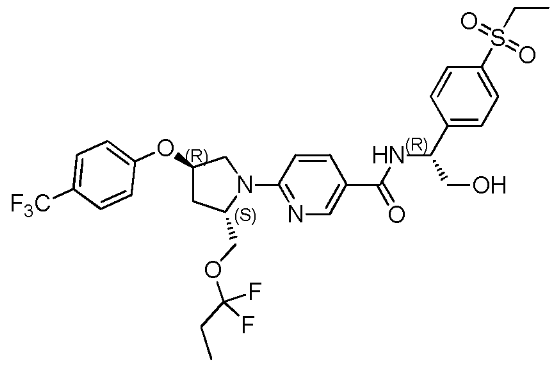

**Step 1: Synthesis of methyl 6-((2*S*,4*R*)-2-(((1,1-difluoroallyl)oxy)methyl)-4-(4-(trifluoromethyl)phenoxy)pyrrolidin-1-yl)nicotinate**

Under nitrogen protection, to a solution of methyl 6-((2*S*,4*R*)-2-(hydroxymethyl)-4-(4-(trifluoromethyl)phenoxy)pyrrolidin-1-yl)nicotinate (280 mg, 0.71 mmol) and 3-bromo-3,3-difluoroprop-1-ene (0.60 mL, 5.90 mmol) in THF (6 mL) was added NaH (136 mg, 3.40 mmol, 60%). The mixture was stirred at room temperature for 16 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL×2), dried over anhydrous $Na_2SO_4$, and concentrated under reduced pressure. The crude product was separated by silica gel column chromatography (eluent: PE/EtOAc (v/v) = 5/1) to give pale yellow liquid (100 mg, 30%).
MS (ESI, pos.ion) m/z: 473.1 $[M+H]^+$.

**Step 2: Synthesis of methyl 6-((2*S*,4*R*)-2-((1,1-difluoropropoxy)methyl)-4-(4-(trifluoromethyl)phenoxy)pyrrolidin-1-yl)nicotinate**

To a solution of methyl 6-((2*S*,4*R*)-2-(((1,1-difluoroallyl)oxy)methyl)-4-(4-**(trifluoromethyl)phenoxy)pyrrolidin-1-yl)nicotinate** (100 mg, 0.21 mmol) in MeOH (6 mL) was added Pd/C (35 mg, 10%) under hydrogen protection. The mixture was stirred at room temperature for 2 h. The reaction solution was filtered, and the filtrate was concentrated under reduced pressure to give colorless liquid (100 mg, 100%).
MS (ESI, pos.ion) m/z: 475.2 $[M+H]^+$.

**Step 3: Synthesis of 6-((2*S*,4*R*)-2-((1,1-difluoropropoxy)methyl) -4-(4-(trifluoromethyl)phenoxy)pyrrolidin-1-yl)nicotinic acid**

To a solution of methyl 6-((2*S*,4*R*)-2-((1,1-difluoropropoxy)methyl) -4-(4-(trifluoromethyl)phenoxy)pyrrolidin-1-yl)nicotinate (100 mg, 0.21 mmol) in MeOH (6 mL) was added a solution of $LiOH \cdot H_2O$ (88 mg, 2.10 mmol) in $H_2O$ (2 mL). The mixture was reacted at room temperature for 17 h. The reaction solution was diluted with $H_2O$ (6 mL), and HCl solution (1 mol/L) was added to adjust the pH of the solution to about 5. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give a white solid (57 mg, 59%).
MS (ESI, pos.ion) m/z: 461.3 $[M+H]^+$.

**Step 4: Synthesis of 6-((2*S*,4*R*)-2-((1,1-difluoropropoxy)methyl)-4-(4-(trifluoromethyl) phenoxy)pyrrolidin-1-yl)-*N*-((*R*)-1-(4-(ethylsulfonyl)phenyl)-2-hydroxyethyl)nicotinamide**

(*R*)-2-Amino-2-(4-(ethylsulfonyl)phenyl)ethanol (30 mg, 0.13 mmol), 6-((2*S*,4*R*)-2-((1,1-difluoropropoxy)methyl)-4-(4-trifluoromethyl)phenoxy)pyrrolidin-1-yl)nicotinic acid (50 mg , 0.11 mmol), EDCI (31 mg, 0.16 mmol), HOBT (22 mg, 0.16 mmol) and TEA (21 mg, 0.21 mmol) were added to DCM (4 mL) in turn, and the mixture was stirred at room temperature for 12 h. The reaction solution was diluted with DCM (40 mL), washed successively with $NaHCO_3$

solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (48 mg, 66%).

MS (ESI, pos.ion) m/z: 672.4 $[M+H]^+$;

$^1H$ NMR (400 MHz, $CDCl_3$) δ (ppm): 8.66 (d, *J* = 1.9 Hz, 1H), 7.92 (dd, *J* = 8.8, 2.2 Hz, 1H), 7.82 (d, *J* = 8.3 Hz, 2H), 7.55 (d, *J* = 6.9 Hz, 4H), 7.12 (d, *J* = 70 Hz, 1H), 6.94 (d, *J* = 8.6 Hz, 2H), 6.41 (d, *J*= 8.8 Hz, 1H), 5.29 - 5.22 (m, 1H), 5.19 - 5.12 (m, 1H), 4.62 (d, *J* = 3.8 Hz, 1H), 4.19 (dd, *J* = 10.2, 4.8 Hz, 1H), 4.04 (dd, *J* = 10.3, 2.6 Hz, 1H), 4.03 - 3.98 (m, 1H), 3.97 - 3.92 (m, 1H), 3.91 - 3.87 (m, 1H), 3.73 (d, *J* = 8.6 Hz, 1H), 3.07 (q, *J* = 7.4 Hz, 2H), 2.55 - 2.48 (m, 1H), 2.46-2.39 (m, 1H), 2.00 - 1.91 (m, 2H), 1.26 (t, *J*= 7.4 Hz, 3H), 1.02 (t, *J*= 7.5 Hz, 3H).

### Example 55 6-((2*S*,4*S*)-2-((1,1-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)nicotinamide

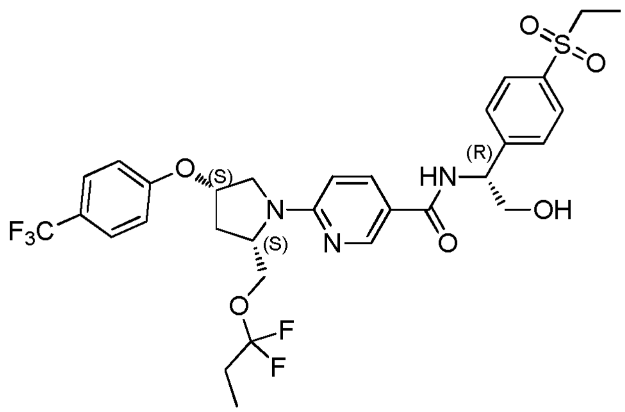

Using methyl 6-((2*S*,4*S*)-2-(hydroxymethyl)-4-(4-(trifluoromethyl)phenoxy) pyrrolidin-1-yl)nicotinate (130 mg, 0.33 mmol) as raw material, the title compound was prepared as a white solid (55 mg, 63%) by referring to the methods of step 1 to step 4 of Example 54.

MS (ESI, pos.ion) m/z: 672.2 $[M+H]^+$;

$^1H$ NMR (400 MHz, $CDCl_3$) δ (ppm): 8.71 (s, 1H), 7.96 (d, *J* = 8.6 Hz, 1H), 7.84 (d, *J* = 7.8 Hz, 2H), 7.59 - 7.53 (m, 4H), 7.19 (s, 1H), 6.97 (d, *J* = 8.5 Hz, 2H), 6.50 (d, *J* = 8.9 Hz, 1H), 5.28 (s, 1H), 5.14 (s, 1H), 4.46 (s, 1H), 4.28 (dd, *J* = 9.5, 4.5 Hz, 1H), 4.02 (s, 1H), 3.97 (t, *J* = 9.2 Hz, 2H), 3.87 (s, 1H), 3.83 (d, *J* = 12.0 Hz, 1H), 3.08 (q, *J* = 7.4 Hz, 2H), 2.50 (d, *J* = 14.2 Hz, 1H), 2.43 -2.35 (m, 1H), 1.97- 1.90 (m, 2H), 1.28 (d, *J* = 7.4 Hz, 3H), 0.98 (t, *J* = 7.5 Hz, 3H).

### Example 56 *N*-((*R*)-1-(4-(ethylsulfonyl)phenyl)-2-hydroxyethyl)-6-((2*S*,4*R*)-2-((((*S*)-tetrahydrofuran-3-yl)oxy)methyl)-4-(4-(trifluoromethyl)phenoxy)pyrrolidin-1-yl)nicotinamide

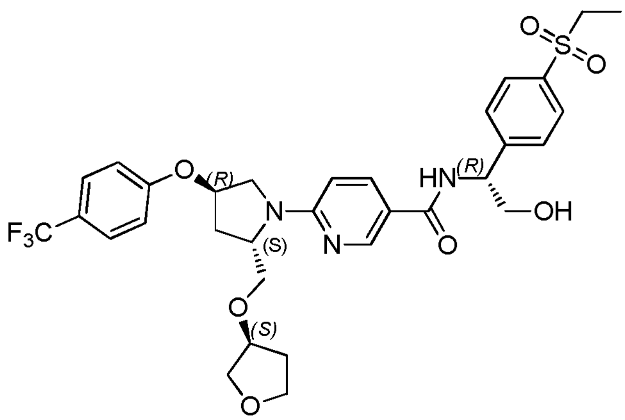

**Step 1: Synthesis of 6-((2*S*,4*R*)-2-((((*S*)-tetrahydrofuran-3-yl)oxy)methyl) -4-(4-(trifluoromethyl)phenoxy)pyrrolidin-1-yl)nicotinic acid**

Under nitrogen protection, to a solution of (*S*)-tetrahydrofuran-3-yl mesylate (754 mg, 4.54 mmol) and methyl 6-((2*S*,4*R*)-2-(hydroxymethyl)-4-(4-(trifluoromethyl)phenoxy) pyrrolidin-1-yl)nicotinate (300 mg, 0.76 mmol) in THF (8 mL) was added NaH (151 mg, 3.78 mmol, 60 wt%). The mixture was stirred at room temperature for 23 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL×3), dried over anhydrous $Na_2SO_4$, and concentrated under reduced pressure. The crude product was separated by silica gel column chromatography (eluent: DCM/MeOH (v/v) = 20/1) to give a yellow solid (70 mg, 20%).
MS (ESI, pos.ion) m/z: 453.3 $[M+H]^+$.

**Step 2: Synthesis of *N*-((*R*)-1-(4-(ethylsulfonyl)phenyl)-2-hydroxyethyl)-6 -((2*S*,4*R*)-2-((((*S*)-tetrahydrofuran-3-yl)oxy)methyl)-4-(4-(trifluoromethyl)phenoxy)pyrrolidin-1-yl)nicotinamide**

(*R*)-2-Amino-2-(4-(ethylsulfonyl)phenyl)ethanol (42 mg, 0.18 mmol), 6-((2*S*,4*R*)-2-((((*S*)-tetrahydrofuran-3-yl)oxy)methyl)-4-(4-trifluoromethyl)phenoxy)pyrrolidin-1-yl) nicotinic acid (70 mg, 0.15 mmol), EDCI (44 mg, 0.23 mmol), HOBT (31 mg, 0.23 mmol) and TEA (30 mg, 0.30 mmol) were added to DCM (5 mL), and the mixture was stirred at room temperature for 6 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (56 mg, 55%).
MS (ESI, pos.ion) m/z: 664.1 $[M+H]^+$;
$^1H$ NMR (400 MHz, $CDCl_3$) δ (ppm): 8.66 (d, *J* = 2.2 Hz, 1H), 7.91 (dd, *J* = 8.9, 2.2 Hz, 1H), 7.84 (d, *J* = 8.2 Hz, 2H), 7.55 (d, *J* = 8.2 Hz, 2H), 7.54 (d, *J* = 8.6 Hz, 2H), 7.14 (d, *J* = 6.9 Hz, 1H), 6.94 (d, *J* = 8.6 Hz, 2H), 6.41 (d, *J* = 8.9 Hz, 1H), 5.27 (dd, *J* = 10.8, 4.6 Hz, 1H), 5.24 - 5.17 (m, 1H), 4.55 (d, *J* = 3.3 Hz, 1H), 4.07 - 3.99 (m, 2H), 3.96 - 3.87 (m, 2H), 3.82 - 3.78 (m, 3H), 3.76 - 3.66 (m, 3H), 3.56 (dd, *J* = 9.4, 2.4 Hz, 1H), 3.08 (q, *J* = 7.4 Hz, 2H), 2.55 - 2.49 (m, 1H), 2.41 - 2.33 (m, 1H), 1.95 - 1.86 (m, 2H), 1.26 (t, *J* = 7.4 Hz, 3H).

**Example 57 *N*-((*R*)-1-(4-(ethylsulfonyl)phenyl)-2-hydroxyethyl)-6-((2*S*,4*R*)-2-((((*R*)-tetrahydrofuran-3-yl)oxy)methyl)-4-(4-(trifluoromethyl)phenoxy)pyrrolidin-1-yl)nico tinamide**

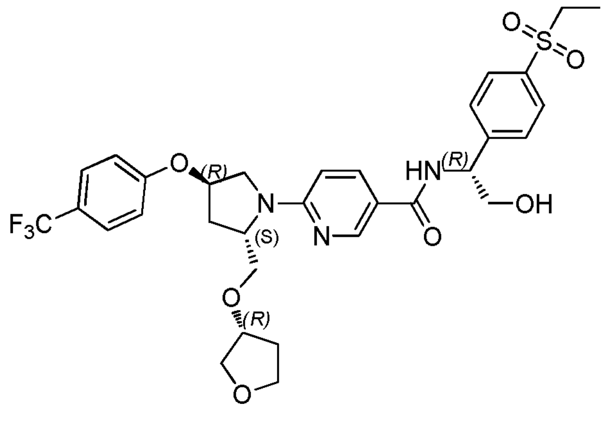

Using (*R*)-tetrahydrofuran-3-yl methanesulfonate (754 mg, 4.54 mmol) as starting material, the title compound was prepared as a white solid (35 mg, 34%) by referring to the synthetic method of Example 56.
MS (ESI, pos.ion) m/z: 664.4 $[M+H]^+$;
$^1H$ NMR (400 MHz, $CDCl_3$) δ (ppm): 8.66 (d, *J* = 2.0 Hz, 1H), 7.91 (dd, *J* = 8.9, 2.3 Hz, 1H), 7.85 (d, *J* = 8.3 Hz, 2H), 7.56 (d, *J* = 8.3 Hz, 2H), 7.55 (d, *J* = 8.6 Hz, 2H), 7.11 (d, *J* = 7.0 Hz, 1H), 6.94 (d, *J* = 8.6 Hz, 2H), 6.42 (d, *J* = 8.9 Hz, 1H), 5.27 (dd, *J* = 11.0, 4.7 Hz, 1H), 5.24 - 5.17 (m, 1H), 4.56 (d, *J*= 3.9 Hz, 1H), 4.06 - 4.00 (m, 2H), 3.97 - 3.88 (m, 2H), 3.88 - 3.72 (m, 3H), 3.70 - 3.59 (m, 4H), 3.08 (q, *J* = 7.4 Hz, 2H), 2.55 - 2.49 (m, 1H), 2.41 - 2.33 (m, 1H), 1.99 - 1.94 (m, 2H), 1.27 (q, *J* = 7.4 Hz, 3H).

**Example 58 *N*-(4-(ethylsulfonyl)benzyl)-2-((2*S*,4*R*)-2-((2-fluoroethoxy)methyl)-4-(4-(trifluoromethyl)phenoxy)pyrrolidin-1-yl)pyrimidine-5-carboxamide**

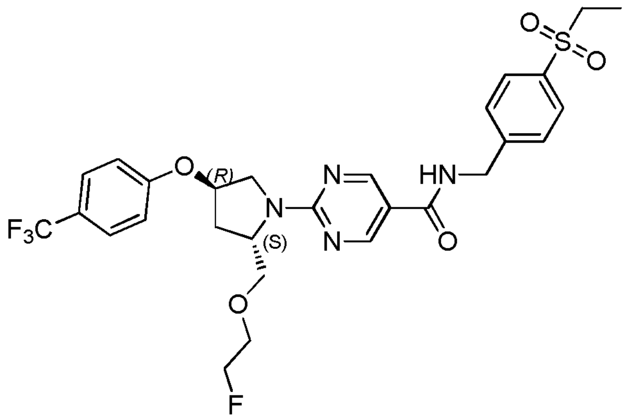

**Step 1: Synthesis of methyl 2-((2*S*,4*R*)-2-(hydroxymethyl)-4-(4-(trifluoromethyl) phenoxy)pyrrolidin-1-yl)pyrimidine-5-carboxylate**

((2*S*,4*R*)-4-(4-(trifluoromethyl)phenoxy)pyrrolidin-2-yl)methanol (550 mg, 2.11 mmol), $K_2CO_3$ (1.16 g, 8.39 mmol) and ethyl 2-chloropyrimidine-5-carboxylate (785 mg, 4.21 mmol) were dissolved in DMSO (10 mL). The mixture was stirred at room temperature for 24 h. The reaction solution was diluted with EtOAc (60 mL), washed successively with $H_2O$ (15 mL × 2) and saturated NaCl solution (15 mL×3), dried over anhydrous $Na_2SO_4$, and concentrated under reduced pressure. The crude product was separated by silica gel column chromatography (eluent: PE/EtOAc (v/v) = 3/1) to give yellow liquid (800 mg, 93%).
MS (ESI, pos.ion) m/z: 411.9 $[M+H]^+$.

**Step 2: Synthesis of ethyl 2-((2*S*,4*R*)-2-((2-fluoroethoxy)methyl)-4-(4-(trifluoromethyl)phenoxy)pyrrolidin-1-yl)pyrimidine-5-carboxylate**

Under nitrogen protection, to a solution of ethyl 2-((2*S*,4*R*)-2-(hydroxymethyl)-4-(4-(trifluoromethyl)phenoxy)pyrrolidin-1-yl)pyrimidine-5-carboxy late (400 mg, 0.97 mmol) and 1-bromo-2-fluoroethane (2.00 mL, 26.77 mmol) in THF (6 mL) was added NaH (250 mg, 6.25 mmol, 60%). The mixture was stirred at room temperature for 12 h. The reaction solution was added with HCl solution (1 mol/L) to quench the reaction, and the pH of the solution was adjusted to about 5. The resulting mixture was diluted with EtOAc (50 mL) and separated, and the organic phase was washed with saturated NaCl (20 mL) solution, dried over anhydrous $Na_2SO_4$, and concentrated under reduced pressure. The crude product was separated by silica gel column chromatography (eluent: PE/EtOAc (v/v) = 3/1) to give yellow liquid (400 mg, 90%).
MS (ESI, pos.ion) m/z: 458.3 $[M+H]^+$.

**Step 3: Synthesis of 2-((2*S*,4*R*)-2-((2-fluoroethoxy)methyl)-4-(4-(trifluoromethyl)phenoxy)pyrrolidin-1-yl)pyrimidine-5-carboxylic acid**

To a solution of ethyl 2-((2*S*,4*R*)-2-((2-fluoroethoxy)methyl)-4-(4-(trifluoromethyl)phenoxy)pyrrolidin-1-yl)pyrimidine-5-carboxylate (240 mg, 0.53 mmol) in THF (6 mL) was added a solution of $LiOH{\cdot}H_2O$ (360 mg, 8.58 mmol) in $H_2O$ (3 mL). The mixture was reacted at room temperature for 22 h. The reaction solution was added with HCl solution (1 mol/L) to adjust the pH of the solution to about 5. The resulting mixture was extracted with EtOAc (30 mL × 2), and the organic phases were combined, washed with saturated NaCl (20 mL) solution, dried over anhydrous $Na_2SO_4$, and concentrated under reduced pressure. The crude product was separated by silica gel column chromatography (eluent: DCM/EtOAc (v/v) = 4/1) to give a yellow solid (120 mg, 64%).
MS (ESI, pos.ion) m/z: 4289.9 $[M+H]^+$.

**Step 4: Synthesis of *N*-(4-(ethylsulfonyl)benzyl)-2-((2*S*,4*R*)-2-((2-fluoroethoxy) methyl)-4-(4-(trifluoromethyl)phenoxy)pyrrolidin-1-yl)pyrimidine-5-carboxamide**

(4-(Ethylsulfonyl)phenyl)methanamine (30 mg, 0.15 mmol), 2-((2*S*,4*R*)-2-((2-fluoroethoxy)methyl)-4-(4-(trifluoromethyl)phenoxy)pyrrolidin-1-yl)pyrimidine-5 -carboxylic acid (40 mg, 0.09 mmol), HATU (60 mg, 0.16 mmol) and TEA (30 mg, 0.30 mmol) were dissolved in DCM (4 mL). The mixture was stirred at room temperature for 20 h. The reaction solution was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (36 mg, 63%).
MS (ESI, pos.ion) m/z: 611.3 $[M+H]^+$.
$^1H$ NMR (400 MHz, $CDCl_3$) δ (ppm): 8.80 (s, 2H), 7.69 (d, *J* = 8.2 Hz, 2H), 7.53 (d, *J* = 8.6 Hz, 2H), 7.41 (d, *J* = 8.2 Hz, 2H), 7.01 (t, *J* = 5.9 Hz, 1H), 6.94 (d, *J* = 8.6 Hz, 2H), 5.22 - 5.15 (m, 1H), 4.68 (d, *J* = 5.9 Hz, 2H), 4.63 - 4.55 (m, 2H), 4.45 (t, *J* = 3.6 Hz, 1H), 4.05 (dd, *J* = 12.4, 2.8 Hz, 1H), 4.00 - 3.91 (m, 2H), 3.74 - 3.69 (m, 2H), 3.63 (q, *J* = 4.3 Hz, 1H), 3.06 (q, *J* = 7.4 Hz, 2H), 2.63 - 2.55 (m, 1H), 2.44 - 2.37 (m, 1H), 1.23 (t, *J*= 7.4 Hz, 3H).

**Example 59 *N*-((*R*)-1-(4-(ethylsulfonyl)phenyl)-2-hydroxyethyl)-2-((2*S*,4*R*)-2-((2-fluoroethoxy)methyl)-4-(4-(trifluoromethyl)phenoxy)pyrrolidin-1-yl)pyrimidine -5-carboxamide**

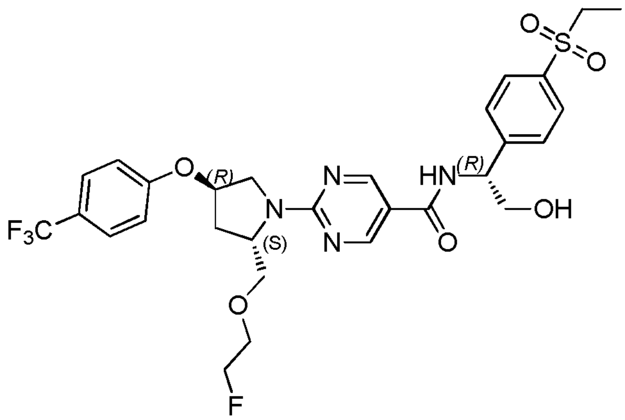

(*R*)-2-Amino-2-(4-(ethylsulfonyl)phenyl)ethanol (55 mg, 0.24 mmol), 2-((2*S*,4*R*)-2-((2-fluoroethoxy)methyl)-4-(4-(trifluoromethyl)phenoxy)pyrrolidin-1-yl)pyrimidine-5 -carboxylic acid (80 mg, 0.19 mmol), EDCI (54 mg, 0.28 mmol) and HOBT (38 mL, 0.28 mmol) were dissolved in DCM (4 mL). The mixture was stirred at room temperature for 18 h. The reaction solution was diluted with DCM (50 mL), washed successively with saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (85 mg, 71%).
MS (ESI, pos.ion) m/z: 641.1 $[M+H]^+$;
$^1H$ NMR (400 MHz, $CDCl_3$) δ (ppm): 8.76 (s, 2H), 7.79 (d, *J* = 7.8 Hz, 2H), 7.53 (d, *J* = 8.3 Hz, 4H), 7.26 (s, 1H), 6.93 (d, *J* = 8.5 Hz, 2H), 5.26 - 5.22 (m, 1H), 5.20 - 5.15 (m, 1H), 4.59 - 4.53 (m, 2H), 4.44 (t, *J* = 3.5 Hz, 1H), 4.04 (dd, *J* = 12.5, 2.6 Hz, 1H), 4.00 - 3.88 (m, 4H), 3.74 - 3.67 (m, 2H), 3.63 (dd, *J* = 8.1, 4.0 Hz, 1H), 3.07 (q, *J* = 7.4 Hz, 2H), 2.63 - 2.54 (m, 1H), 2.43 - 2.36 (m, 1H), 1.24 (t, *J* = 7.3 Hz, 3H).

**Example 60 2-((2*S*,4*R*)-2-((cyclopropylmethoxy)methyl)-4-(4-trifluoromethyl)phenoxy) pyrrolidin-1-yl)-*N*-((*R*)-1-(4-(ethylsulfonyl)phenyl)-2-hydroxyethyl)pyrimidine-5-carboxamide**

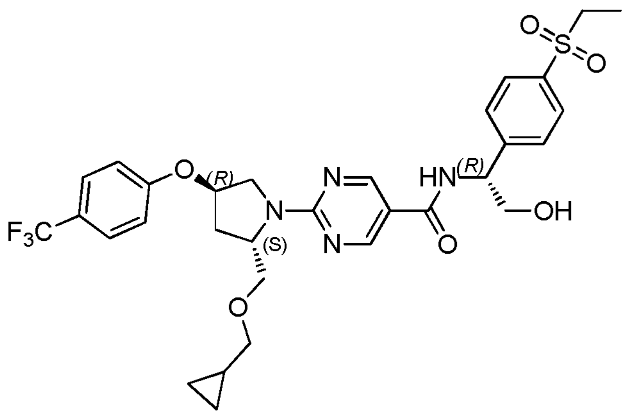

**Step 1: Synthesis of 2-((2*S*,4*R*)-2-((cyclopropylmethoxy)methyl)-4-(4-(trifluoromethyl)phenoxy)pyrrolidin-1-yl)pyrimidine-5-carboxylic acid**

Under nitrogen protection, to a solution of ethyl 2-((2*S*,4*R*)-2-(hydroxymethyl)-4-(4-(trifluoromethyl)phenoxy)pyrrolidin-1-yl)pyrimidine-5-carboxy late (300 mg, 0.73 mmol) and (bromomethyl)cyclopropane (1 mL, 10.30 mmol) in THF (8 mL) was added NaH (145 mg, 3.63 mmol, 60%). The mixture was stirred at room temperature for 16 h. To the reaction solution was added HCl solution (1 mol/L) to quench the reaction, and the pH of the solution was adjusted to about 5. The resulting mixture was diluted with saturated NaCl solution (15 mL) and extracted with EtOAc (30 mL×3). The organic phase was dried over anhydrous $Na_2SO_4$, and concentrated under reduced pressure. The crude product was separated by silica gel column chromatography (eluent: DCM/EtOAc (v/v) = 1/1) to give a yellow solid (180 mg, 56%).
MS (ESI, pos.ion) m/z: 438.2 $[M+H]^+$.

**Step 2: Synthesis of 2-((2*S*,4*R*)-2-((cyclopropylmethoxy)methyl)-4-(4-(trifluoromethyl)phenoxy)pyrrolidin-1-yl)-*N*-((*R*)-1-(4-(ethylsulfonyl)phenyl)-2-hydroxyethyl) pyrimidine-5-carboxamide**

(*R*)-2-Amino-2-(4-(ethylsulfonyl)phenyl)ethanol (122 mg, 0.53 mmol), 2-((2*S*,4*R*)-2-((cyclopropylmethoxy)methyl)-4-(4-(trifluoromethyl)phenoxy)pyrrolidin-1-yl)pyrimid ine-5-carboxylic acid (180 mg, 0.41 mmol), HATU (180 mg, 0.47 mmol) and DIPEA (159 mg, 1.23 mmol) were dissolved in DCM (6 mL). The mixture was stirred at room temperature for 16 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (166 mg, 62%).
MS (ESI, pos.ion) m/z: 649.4 $[M+H]^+$.
$^1H$ NMR (400 MHz, $CDCl_3$) δ (ppm): 8.76 (s, 2H), 7.77 (d, *J* = 8.3 Hz, 2H), 7.52 (d, *J* = 7.3 Hz, 4H), 7.33 (d, *J* = 7.1 Hz, 1H), 6.94 (d, *J* = 8.6 Hz, 2H), 5.26 - 5.14 (m, 2H), 4.59 - 4.53 (m, 1H), 4.02 (dd, *J* = 12.4, 3.1 Hz, 1H), 3.95 (dd, *J* = 10.9, 4.5 Hz, 2H), 3.89 (dd, *J* = 11.5, 5.4 Hz, 1H), 3.80 (dd, *J* = 9.7, 4.4 Hz, 1H), 3.65 (dd, *J* = 9.7, 2.4 Hz, 1H), 3.25 (dd, *J* = 6.7, 1.7 Hz, 2H), 3.06 (q, *J* = 7.4 Hz, 2H), 2.60 - 2.53 (m, 1H), 2.41 - 2.35 (m, 1H), 1.23 (t, *J* = 7.4 Hz, 3H), 1.02 - 0.94 (m, 1H), 0.50 - 0.43 (q, *J* = 4.6 Hz, 2H), 0.15 (q, *J* = 4.6 Hz, 2H).

**Example 61 *N*-((*R*)-1-(4-(ethylsulfonyl)phenyl)-2-hydroxyethyl)-2-((2*S*,4*R*)-2-((((*R*)-tetrahydrofuran-3-yl)oxy)methyl)-4-(4-(trifluoromethyl)phenoxy)pyrrolidin-1-yl)pyrimidine-5 -carboxamide**

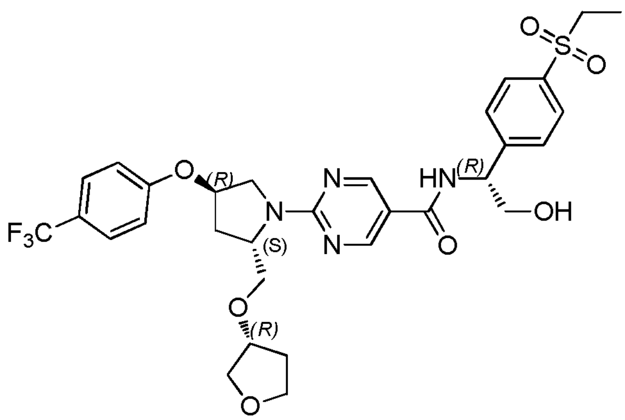

**Step 1: Synthesis of 2-((2*S*,4*R*)-2-((((*R*)-tetrahydrofuran-3-yl)oxy)methyl)-4-(4-(trifluoromethyl)phenoxy)pyrrolidin-1-yl)pyrimidine-5-carboxylic acid**

Under nitrogen protection, to a solution of (*R*)-tetrahydrofuran-3-yl mesylate (808 mg, 4.86 mmol) and ethyl 2-((2*S*,4*R*)-2-(hydroxymethyl)-4-(4-(trifluoromethyl)phenoxy) pyrrolidin-1-yl)pyrimidine-5-carboxylate (250 mg, 0.61 mmol) in THF (6 mL) was added NaH (120 mg, 3.00 mmol, 60%). The mixture was stirred at room temperature for 23 h. To the reaction solution was added HCl solution (1 mol/L) to quench the reaction, and the pH of the solution was adjusted to about 5. The resulting mixture was diluted with saturated NaCl solution (20 mL), extracted with EtOAc (30 mL×3), dried over anhydrous $Na_2SO_4$, and concentrated under reduced pressure. The crude product was separated by silica gel column chromatography (eluent: DCM/MeOH (v/v) = 20/1) to give a yellow solid (50 mg, 17%).
MS (ESI, pos.ion) m/z: 454.3 $[M+H]^+$.

**Step 2: Synthesis of *N*-((*R*)-1-(4-(ethylsulfonyl)phenyl)-2-hydroxyethyl) -2-((2*S*,4*R*)-2-((((*R*)-tetrahydrofuran-3-yl)oxy)methyl)-4-(4-(trifluoromethyl)phenoxy)pyrrolid in-1-yl)pyrimidine-5-carboxamide**

(*R*)-2-Amino-2-(4-(ethylsulfonyl)phenyl)ethanol (40 mg, 0.17 mmol), 2-((2*S*,4*R*)-2-((((*R*)-tetrahydrofuran-3-yl)oxy)methyl)-4-(4-(trifluoromethyl)phenoxy)pyrrolidin-1-y l)pyrimidine-5-carboxylic acid (50 mg, 0.11 mmol), HATU (84 mg, 0.22 mmol) and DIPEA (45 mg, 0.35 mmol) were dissolved in DCM (5 mL). The mixture was stirred at room temperature for 16 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (25 mg, 34%).
MS (ESI, pos.ion) m/z: 665.3 $[M+H]^+$;
$^1H$ NMR (400 MHz, $CDCl_3$) δ (ppm): 8.77 (s, 2H), 7.84 (d, *J* = 8.3 Hz, 2H), 7.55 (d, *J* = 8.3 Hz, 2H), 7.54 (d, *J*= 8.6 Hz, 2H),7.14 (d, *J*= 6.9 Hz, 1H), 6.92 (d, *J*= 8.6 Hz, 2H), 5.26 (d, *J* = 6.0 Hz, 1H), 5.13 (s, 1H), 4.57 (d, *J*= 2.9 Hz, 1H), 4.08 - 3.98 (m, 3H), 3.97 - 3.89 (m, 2H), 3.86 (d, *J* = 8.0 Hz, 1H), 3.84 - 3.76 (m, 2H), 3.72 - 3.67 (m, 1H), 3.67 - 3.61 (m, 2H), 3.08 (q, *J* = 7.4 Hz, 2H), 2.57 - 2.50 (m, 1H), 2.43 - 2.37 (m, 1H), 1.97 (dd, *J* = 6.9, 4.1 Hz, 2H), 1.27 (d, *J* = 7.4 Hz, 3H).

**Example 62 *N*-((*R*)-1-(4-(ethylsulfonyl)phenyl)-2-hydroxyethyl)-2-((2*S*,4*R*)-2-((((*S*)-tetrahydrofuran-3-yl)oxy)methyl)-4-(4-(trifluoromethyl)phenoxy)pyrrolidin-1-yl)pyrimidine-5 -carboxamide**

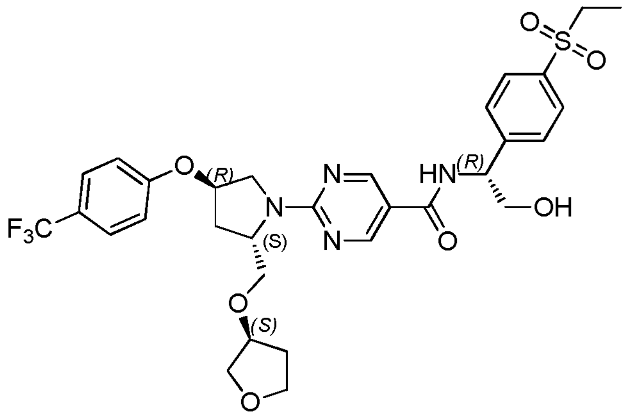

Using (*S*)-tetrahydrofuran-3-yl methanesulfonate (808 mg, 4.86 mmol) as starting material, the title compound was prepared as a white solid (42 mg, 29%) by referring to the synthetic method of Example 61.
MS (ESI, pos.ion) m/z: 665.3 $[M+H]^+$;
$^1H$ NMR (400 MHz, $CDCl_3$) δ (ppm): 8.78 (s, 2H), 7.86 (d, *J* = 8.3 Hz, 2H), 7.55 (t, *J* = 8.2 Hz, 4H), 7.03 (d, *J* = 6.9 Hz, 1H), 6.93 (d, *J* = 8.6 Hz, 2H), 5.31 -524 (m, 1H), 5.14 (d, *J* = 3.7 Hz, 1H), 4.57 (d, *J* = 2.7 Hz, 1H), 4.09 - 4.01 (m, 3H), 4.00 - 3.86 (m, 3H), 3.84 - 3.72 (m, 4H), 3.61 (dd, *J*= 9.5, 2.1 Hz, 1H), 3.09 (q, *J*= 7.4 Hz, 2H), 2.58 - 2.51 (m, 1H), 2.40 (ddd, *J*= 13.4, 8.6, 4.4 Hz, 1H), 1.95 - 1.87 (m, 1H), 1.84- 1.76 (m, 1H), 1.28 (d, *J* = 7.4 Hz, 3H).

**Example 63 2-((2*R*,4*R*)-2-(2-(difluoromethoxy)ethyl)-4-(4-trifluoromethyl)phenoxy) pyrrolidin-1-yl)-*N*-((*R*)-1-(4-(ethylsulfonyl)phenyl)-2-hydroxyethyl)pyrimidine-5-carboxamide**

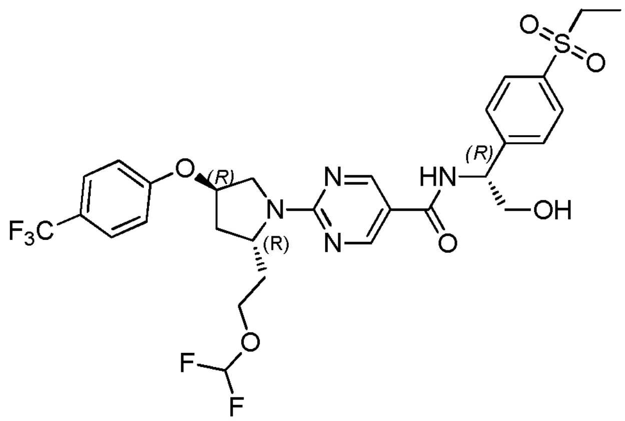

**Step 1: Synthesis of ethyl 2-((2*R*,4*R*)-2-(2-hydroxyethyl)-4-(4-(trifluoromethyl)phenoxy) pyrrolidin-1-yl)pyrimidine-5-carboxylate**

2-((2*R*,4*R*)-4-(4-(trifluoromethyl)phenoxy)pyrrolidin-2-yl)ethanol (50 mg, 0.18 mmol), ethyl 2-chloropyrimidine-5-carboxylate (50 mg, 0.27 mmol) and $K_2CO_3$ (75 mg, 0.54 mmol) were added to acetone (4 mL). The mixture was stirred at room temperature for 12 h. The reaction solution was filtered and concentrated under reduced pressure, and the crude product was separated by silica gel column chromatography (eluent: DCM/EtOAc (v/v) = 3/1) to give pale yellow liquid (50 mg, 65%).
MS (ESI, pos.ion) m/z: 426.3 $[M+H]^+$.

**Step 2: Synthesis of ethyl 2-((2*R*,4*R*)-2-(2-(difluoromethoxy)ethyl)-4-(4-(trifluoromethyl) phenoxy)pyrrolidin-1-yl)pyrimidine-5-carboxylate**

To a solution of ethyl 2-((2*R*,4*R*)-2-(2-hydroxyethyl)-4-(4-(trifluoromethyl) phenoxy)pyrrolidin-1-yl)pyrimidine-5-carboxylate (50 mg, 0.12 mmol) and KOAc (250 mg, 2.54 mmol) in $DCM/H_2O$ (1 mL/1 mL) was added $TMSCF_2Br$

(0.50 mL, 3.22 mmol). The mixture was stirred at room temperature for 24 h. The reaction solution was diluted with DCM (20 mL), washed with saturated NaCl solution (15 mL), dried over anhydrous $Na_2SO_4$, and concentrated under reduced pressure. The crude product was separated by silica gel column chromatography (eluent: DCM/EtOAc (v/v) = 5/1) to give pale yellow liquid (30 mg, 54%).

MS (ESI, pos.ion) m/z: 476.1 $[M+H]^+$.

**Step 3: Synthesis of 2-((2*R*,4*R*)-2-(2-(difluoromethoxy)ethyl)-4-(4-(trifluoromethyl) phenoxy)pyrrolidin-1-yl)pyrimidine-5-carboxylic acid**

To a solution of ethyl 2-((2*R*,4*R*)-2-(2-(difluoromethoxy)ethyl)-4-(4-(trifluoromethyl)phenoxy)pyrrolidin-1-yl)pyrimidine-5-carboxylate (30 mg, 0.06 mmol) in MeOH (6 mL) was added a solution of $LiOH{\cdot}H_2O$ (100 mg, 2.38 mmol) in $H_2O$ (2 mL). The mixture was reacted at room temperature for 6 h. The reaction solution was diluted with $H_2O$ (10 mL), and HCl solution (1 mol/L) was added to adjust the pH of the solution to about 4. The resulting mixture was extracted with EtOAc (20 mL $\times$ 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (19 mg, 67%).

MS (ESI, pos.ion) m/z: 448.1 $[M+H]^+$.

**Step 4: Synthesis of 2-((2*R*,4*R*)-2-(2-(difluoromethoxy)ethyl)-4-(4-(trifluoromethyl)phenoxy)pyrrolidin-1-yl)-*N*-((*R*)-1-(4-(ethylsulfonyl)phenyl)-2-hydroxyethyl) pyrimidine-5-carboxamide**

(*R*)-2-Amino-2-(4-(ethylsulfonyl)phenyl)ethanol (11 mg, 0.05 mmol), 2-((2*R*,4*R*)-2-(2-(difluoromethoxy)ethyl)-4-(4-trifluoromethyl)phenoxy)pyrrolidin-1-yl)pyrimidine-5-carboxylic acid (19 mg , 0.04 mmol), EDCI (16 mg, 0.08 mmol), HOBT (11 mg, 0.08 mmol) and TEA (8 mg, 0.08 mmol) were dissolved in DCM (2 mL), and the mixture was stirred at room temperature for 7 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (20 mg, 72%).

MS (ESI, pos.ion) m/z: 659.2 $[M+H]^+$.

$^1H$ NMR (400 MHz, $CDCl_3$) $\delta$ (ppm): 8.77 (s, 2H), 7.81 (d, *J* = 7.3 Hz, 2H), 7.74 (d, *J* = 6.9 Hz, 1H), 7.53 (d, *J* = 8.0 Hz, 2H), 7.50 (d, *J* = 8.9 Hz, 2H), 6.89 (d, *J* = 8.0 Hz, 2H), 6.18 (t, *J* = 74.7 Hz, 1H), 5.20 (s, 1H), 5.02 (s, 1H), 4.47 (d, *J*= 5.9 Hz, 1H), 4.17 (d, *J*= 12.7 Hz, 1H), 3.95 - 3.90 (m, 3H), 3.86 - 3.78 (m, 2H), 3.06 (q, *J* = 7.4 Hz, 2H), 2.52 - 2.42 (m, 2H), 2.30 - 2.22 (m, 1H), 1.91 - 1.81 (m, 1H), 1.24 (t, *J* = 7.4 Hz, 3H).

**Example 64 2-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)pyrimidine-5-carboxamide**

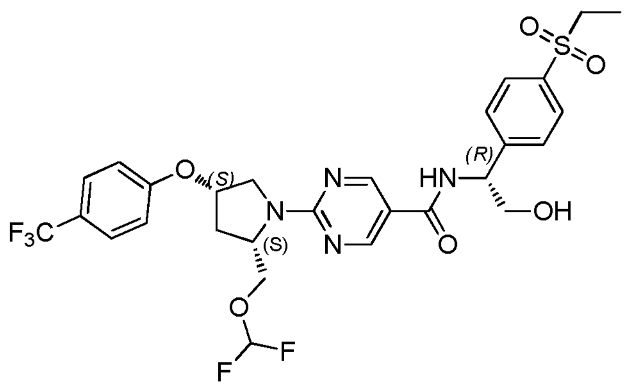

**Step 1: Synthesis of ethyl 2-((2*S*,4*S*)-2-((difluoromethoxy)methyl)-4-(4-(trifluoromethyl)phenoxy)pyrrolidin-1-yl)pyrimidine-5-carboxylate**

To a solution of ethyl 2-((2*S*,4*S*)-2-(hydroxymethyl)-4-(4-(trifluoromethyl) phenoxy)pyrrolidin-1-yl)pyrimidine-5-carboxylate (200 mg, 0.49 mmol) (it was prepared by referring to the synthesis method of (2*S*,4*R*)-4-(4-(trifluoromethyl)phenoxy)pyrrolidin-2-yl)methanol in step 1 of Example 58) and KOAc (600 mg, 6.11 mmol) in DCM/$H_2O$ (1 mL/1 mL) was added $TMSCF_2Br$ (0.50 mL, 3.22 mmol). The mixture was stirred at room temperature for 24 h. The reaction solution was diluted with DCM (30 mL), washed with saturated NaCl solution (15 mL), dried over anhydrous $Na_2SO_4$, and concentrated under reduced pressure. The crude product was separated by silica gel column chromatography (eluent: PE/EtOAc (v/v) = 3/1) to give yellow liquid (100 mg, 45%).

MS (ESI, pos.ion) m/z: 462.1 $[M+H]^+$.

**Step 2: Synthesis of 2-((2*S*,4*S*)-2-((difluoromethoxy)methyl)-4-(4-(trifluoromethyl)phenoxy)pyrrolidin-1-yl)pyrimidine-5-carboxylic acid**

To a solution of ethyl 2-((2*S*,4*S*)-2-((difluoromethoxy)methyl)-4-(4-(trifluoromethyl)phenoxy)pyrrolidin-1-yl)pyrimidine-5-carboxylate (100 mg, 0.22 mmol) in MeOH (6 mL) was added a solution of $LiOH\cdot H_2O$ (100 mg, 2.38 mmol) in $H_2O$ (2 mL). The mixture was reacted at room temperature for 12 h. The reaction solution was added with HCl solution (1 mol/L) to adjust the pH of the solution to about 4. The resulting mixture was extracted with EtOAc (20 mL $\times$ 2), and the organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (84 mg, 89%).

MS (ESI, pos.ion) m/z: 434.0 $[M+H]^+$.

**Step 3: Synthesis of 2-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl) pyrimidine-5-carboxamide**

(*R*)-2-Amino-2-(4-(ethylsulfonyl)phenyl)ethanol (50 mg, 0.22 mmol), 2-((2*S*,4*S*)-2-((difluoromethoxy)methyl)-4-(4-trifluoromethyl)phenoxy)pyrrolidin-1-yl)pyrimidine-5 -carboxylic acid (80 mg, 0.18 mmol), EDCI (53 mg, 0.28 mmol), HOBT (38 mg, 0.28 mmol) and TEA (28 mg, 0.28 mmol) were dissolved in DCM (6 mL), and the mixture was stirred at room temperature for 15 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (96 mg, 81%).

MS (ESI, pos.ion) m/z: 645.1 $[M+H]^+$.

$^1H$ NMR (400 MHz, $CDCl_3$) $\delta$ (ppm): 8.82 (s, 2H), 7.81 (d, *J* = 7.6 Hz, 2H), 7.56 (t, *J* = 8.4 Hz, 4H), 7.23 (s, 1H), 6.96 (d, *J* = 8.5 Hz, 2H), 6.22 (t, *J* = 74.6 Hz, 1H), 5.26 (s, 1H), 5.12 (s, 1H), 4.60 (s, 1H), 4.36 (dd, *J* = 8.9, 3.7 Hz, 1H), 4.10 - 3.88 (m, 5H), 3.08 (q, *J* = 7.4 Hz, 2H), 2.56 (d, *J* = 14.3 Hz, 1H), 2.45 -2.36 (m, 1H), 1.27 (t, *J* = 7.4 Hz, 3H).

**Example 65 *N*-((*R*)-1-(4-(ethylsulfonyl)phenyl)-2-hydroxyethyl)-2-((2*S*,4*S*) -2-((trifluoromethoxy)methyl)-4-(4-(trifluoromethyl)phenoxy)pyrrolidin-1-yl)pyrimidine-5-ca rboxamide**

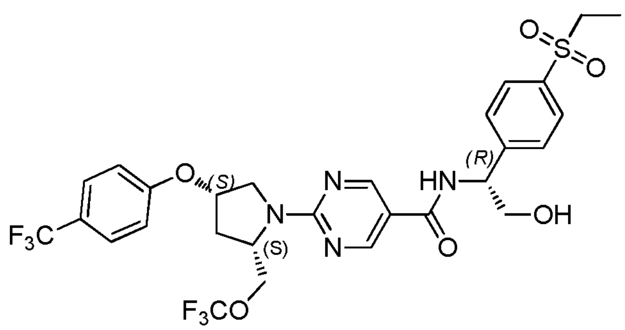

**Step 1: Synthesis of ethyl 2-((2*S*,4*S*)-2-((trifluoromethoxy)methyl)-4-(4-(trifluoromethyl)phenoxy)pyrrolidin-1-yl)pyrimidine-5-carboxylate**

To a solution of (2*S*,4*S*)-2-((trifluoromethoxy)methyl)-4-(4-(trifluoromethyl)phenoxy)pyrrolidine (40 mg, 0.12 mmol) and ethyl 2-chloropyrimidine-5-carboxylate (35 mg, 0.19 mmol) in acetone (4 mL) was added $K_2CO_3$ (100 mg, 0.72 mmol). The mixture was reacted at room temperature for 8 h. The reaction solution was filtered and concentrated under reduced pressure, and the crude product was separated by silica gel column chromatography (eluent: PE/EtOAc

(v/v) = 3/1) to give pale yellow liquid (33 mg, 57%).

MS (ESI, pos.ion) m/z: 480.1 $[M+H]^+$.

**Step 2: Synthesis of 2-((2*S*,4*S*)-2-((trifluoromethoxy)methyl)-4-(4-(trifluoromethyl)phenoxy)pyrrolidin-1-yl)pyrimidine-5-carboxylic acid**

To a solution of ethyl 2-((2*S*,4*S*)-2-((trifluoromethoxy)methyl) -4-(4-(trifluoromethyl)phenoxy)pyrrolidin-1-yl)pyrimidine-5-carboxylate (33 mg, 0.07 mmol) in MeOH (4 mL) was added a solution of $LiOH \cdot H_2O$ (60 mg, 1.43 mmol) in $H_2O$ (1 mL). The mixture was reacted at room temperature for 11 h. The reaction solution was added with HCl solution (1 mol/L) to adjust the pH of the solution to about 4. The resulting mixture was extracted with EtOAc (20 mL $\times$ 2), and the organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (23 mg, 74%).

MS (ESI, pos.ion) m/z: 452.2 $[M+H]^+$.

**Step 3: Synthesis of *N*-((*R*)-1-(4-(ethylsulfonyl)phenyl)-2-hydroxyethyl)-2-((2*S*,4*S*)-2-((trifluoromethoxy)methyl)-4-(4-(trifluoromethyl)phenoxy)pyrrolidin-1-yl)pyrimidi ne-5-carboxamide**

(*R*)-2-Amino-2-(4-(ethylsulfonyl)phenyl)ethanol (14 mg, 0.06mmol), 2-((2*S*,4*S*)-2-((trifluoromethoxy)methyl)-4-(4-trifluoromethyl)phenoxy)pyrrolidin-1-yl)pyrimidine-5-carboxylic acid (22 mg, 0.05 mmol), EDCI (18 mg, 0.09 mmol), HOBT (13 mg, 0.10 mmol) and TEA (14 mg, 0.14 mmol) were dissolved in DCM (4 mL), and the mixture was stirred at room temperature for 17 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a pale yellow solid (17 mg, 53%).

MS (ESI, pos.ion) m/z: 663.0 $[M+H]^+$;

$^1$H NMR (400 MHz, $CDCl_3$) $\delta$ (ppm): 8.81 (s, 2H), 7.82 (d, *J* = 8.2 Hz, 2H), 7.54 (d, *J* = 7.7 Hz, 4H), 6.93 (d, *J* = 8.5 Hz, 2H), 5.20 (t, *J* = 4.6 Hz, 1H), 5.12 (s, 1H), 4.65 - 4.58 (m, 1H), 4.46 (dd, *J* = 8.7, 4.1 Hz, 1H), 4.09 (t, *J* = 9.2 Hz, 1H), 3.99 (dd, *J* = 13.3, 4.6 Hz, 1H), 3.92 (d, *J* = 13.9 Hz, 2H), 3.82 (dd, *J* = 11.6, 5.8 Hz, 1H), 3.07 (q, *J* = 7.4 Hz, 2H), 2.56 (s, 1H), 2.45 - 2.36 (m, 1H), 1.23 (t, *J* = 7.4 Hz, 3H).

**Example 66 2-((2*S*,4*R*)-2-((1,1-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)pyrimidine-5-carboxamide**

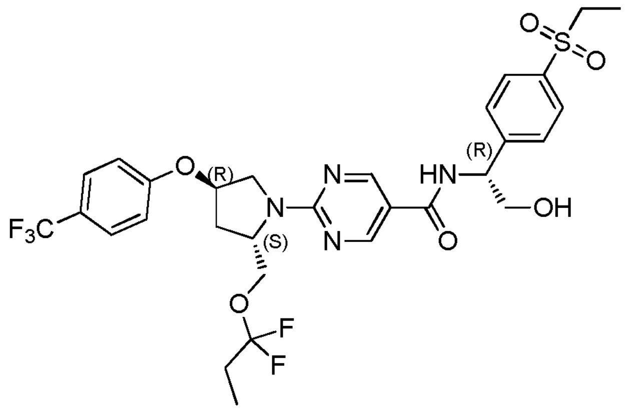

**Step 1: Synthesis of ethyl 2-((2*S*,4*R*)-2-(((1,1-difluoroallyl)oxy)methyl) -4-(4-(trifluoromethyl)phenoxy)pyrrolidin-1-yl)pyrimidine-5-carboxylate**

Under nitrogen protection, to a solution of ethyl 2-((2*S*,4*R*)-2-(hydroxymethyl)-4-(4-(trifluoromethyl)phenoxy)pyrrolidin-1-yl)pyrimidine-5-carboxy late (150 mg, 0.36 mmol) and 3-bromo-3,3-difluoroprop-1-ene (0.30 mL, 2.95 mmol) in THF (6 mL) was added NaH (77 mg, 1.93 mmol, 60%). The mixture was stirred at room temperature for 12 h. To the reaction solution was added HCl solution (1 mol/L) to quench the reaction, and the pH of the solution was adjusted

to about 5. The resulting mixture was diluted with saturated NaCl solution (20 mL), extracted with EtOAc (30 mL×2), dried over anhydrous $Na_2SO_4$, and concentrated under reduced pressure. The crude product was separated by silica gel column chromatography (eluent: PE/EtOAc (v/v) = 5/1) to give pale yellow liquid (60 mg, 34%).

MS (ESI, pos.ion) m/z: 488.1 $[M+H]^+$.

**Step 2: Synthesis of ethyl 2-((2*S*,4*R*)-2-((1,1-difluoropropoxy)methyl)-4-(4-(trifluoromethyl)phenoxy)pyrrolidin-1-yl)pyrimidine-5-carboxylate**

To a solution of ethyl 2-((2*S*,4*R*)-2-(((1,1-difluoroallyl)oxy)methyl)-4-(4-(trifluoromethyl)phenoxy)pyrrolidin-1-yl)pyrimidine-5-carboxylate (50 mg, 0.10 mmol) in MeOH (6 mL) was added Pd/C (20 mg, 10%) under hydrogen protection. The mixture was stirred at room temperature for 2 h. The reaction solution was filtered, and the filtrate was concentrated under reduced pressure to give colorless liquid (50 mg, 100%).

MS (ESI, pos.ion) m/z: 490.2 $[M+H]^+$.

**Step 3: Synthesis of 2-((2*S*,4*R*)-2-((1,1-difluoropropoxy)methyl)-4-(4-(trifluoromethyl)phenoxy)pyrrolidin-1-yl)pyrimidine-5-carboxylic acid**

To a solution of ethyl 2-((2*S*,4*R*)-2-((1,1-difluoropropoxy)methyl)-4-(4-(trifluoromethyl)phenoxy)pyrrolidin-1-yl)pyrimidine-5-carboxylate (50 mg, 0.11 mmol) in MeOH (6 mL) was added a solution of $LiOH \cdot H_2O$ (50 mg, 1.19 mmol) in $H_2O$ (2 mL). The mixture was reacted at room temperature for 22 h. The reaction solution was diluted with $H_2O$ (10 mL), and HCl solution (1 mol/L) was added to adjust the pH of the solution to about 5. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 1/1) to give a white solid (33 mg, 68%).

MS (ESI, pos.ion) m/z: 462.1 $[M+H]^+$.

**Step 4: Synthesis of 4-((2*S*,4*R*)-2-((1,1-difluoropropoxy)methyl)-4-(4-(trifluoromethyl)phenoxy)pyrrolidin-1-yl)-*N*-((*R*)-1-(4-(ethylsulfonyl)phenyl)-2-hydroxyethyl) pyrimidine-5-carboxamide**

(*R*)-2-Amino-2-(4-(ethylsulfonyl)phenyl)ethanol (17 mg, 0.07 mmol), 2-((2*S*,4*R*)-2-((1,1-difluoropropoxy)methyl)-4-(4-trifluoromethyl)phenoxy)pyrrolidin-1-yl)pyrimidi ne-5-carboxylic acid (30 mg, 0.07 mmol), EDCI (18 mg, 0.09 mmol), HOBT (13 mg, 0.10 mmol) and TEA (13 mg, 0.13 mmol) were dissolved in DCM (4 mL), and the mixture was stirred at room temperature for 12 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (35 mg, 80%).

MS (ESI, pos.ion) m/z: 673.4 $[M+H]^+$.

$^1H$ NMR (400 MHz, $CDCl_3$) δ (ppm): 8.79 (s, 2H), 7.81 (d, *J* = 8.2 Hz, 2H), 7.56 - 7.49 (m, 4H), 6.90 (d, *J* = 8.5 Hz, 2H), 5.24 - 5.18 (m, 1H), 5.08 (s, 1H), 4.59 (s, 1H), 4.31 (dd, *J* = 10.2, 4.4 Hz, 1H), 4.09 (d, *J* = 12.6 Hz, 1H), 4.02 (d, *J* = 8.5 Hz, 1H), 3.94 - 3.79 (m, 3H), 3.07 (q, *J* = 7.4 Hz, 2H), 2.47 - 2.42 (m, 2H), 1.98 - 1.86 (m, 2H), 1.23 (t, *J*= 7.4 Hz, 3H), 0.98 (t, *J*= 7.4 Hz, 3H).

**Example 67 2-((2*S*,4*S*)-2-((1,1-difluoropropoxy)methyl)-4-(4-trifluoromethyl)phenoxy) pyrrolidin-1-yl)-*N*-((*R*)-1-(4-(ethylsulfonyl)phenyl)-2-hydroxyethyl)pyrimidine-5-carboxamide**

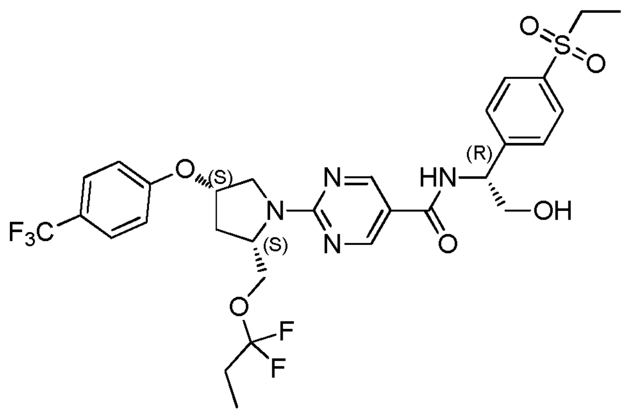

Using ethyl 2-((2*S*,4*S*)-2-(hydroxymethyl)-4-(4-(trifluoromethyl)phenoxy) pyrrolidin-1-yl)pyrimidine-5-carboxylate (200 mg, 0.49 mmol) as raw material, the title compound was prepared as a white solid (88 mg, 75%) by referring to the synthetic method of Example 66.

MS (ESI, pos.ion) m/z: 673.2 $[M+H]^+$;

$^1$H NMR (400 MHz, $CDCl_3$) $\delta$ (ppm): 8.81 (s, 2H), 7.87 (d, *J* = 8.2 Hz, 2H), 7.57 (d, *J* = 8.3 Hz, 4H), 6.97 (d, *J*= 8.5 Hz, 3H), 5.30 - 5.26 (m, 1H), 5.12 (s, 1H), 4.59 (s, 1H), 4.35 (dd, *J*= 9.2, 4.1 Hz, 1H), 4.10 - 4.03 (m, 2H), 4.02 - 3.96 (m, 2H), 3.93 (d, *J* = 13.1 Hz, 1H), 3.10 (q, *J* = 7.4 Hz, 2H), 2.56 (d, *J* = 14.4 Hz, 1H), 2.44 - 2.35 (m, 1H), 2.00 - 1.90 (m, 2H), 1.28 (t, *J* = 7.4 Hz, 3H), 0.98 (t, *J* = 7.5 Hz, 3H).

**Example 68 4-((2*S*,4*R*)-2-((difluoromethoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)benzamide**

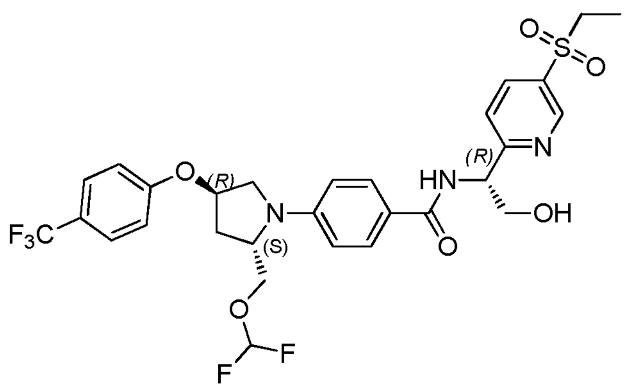

(*R*)-2-Amino-2-(5-(ethylsulfonyl)pyridin-2-yl)ethanol (48 mg, 0.21 mmol), 4-((2*S*,4*R*)-2-((difluoromethoxy)methyl)-4-(4-trifluoromethyl)phenoxy)pyrrolidin-1-yl)benzoic acid (60 mg, 0.14 mmol), EDCI (39 mg, 0.20 mmol), HOBT (28 mg, 0.21 mmol) and TEA (30 mg, 0.30 mmol) were dissolved in DCM (4 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (52 mg, 58%).

MS (ESI, pos.ion) m/z: 644.1 $[M+H]^+$.

$^1$H NMR (400 MHz, $CDCl_3$) $\delta$ (ppm): 8.73 (s, 1H), 7.97 (d, *J* = 8.0 Hz, 1H), 7.93 (dd, *J* = 8.1, 1.9 Hz, 1H), 7.74 (d, *J* = 8.7 Hz, 2H), 7.56 (d, *J* = 8.6 Hz, 2H), 7.14 (d, *J* = 6.9 Hz, 1H), 6.95 (d, *J*= 8.6 Hz, 2H), 6.61 (d, *J* = 8.7 Hz, 2H), 6.21 (t, *J* = 74.1 Hz, 1H), 5.27 (d, *J* = 6.3 Hz, 1H), 5.21 - 5.14 (m, 1H), 4.38 - 4.30 (m, 1H), 4.06 (dd, *J*= 11.2, 3.5 Hz, 1H), 4.01 - 3.89 (m, 4H), 3.54 (dd, *J* = 10.7, 3.5 Hz, 1H), 3.36 (q, *J* = 7.4 Hz, 2H), 2.57 - 2.43 (m, 2H), 1.29 (t, *J* = 7.4 Hz, 3H).

**Example 69 4-((2*S*,4*S*)-2-((difluoromethoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)benzamide**

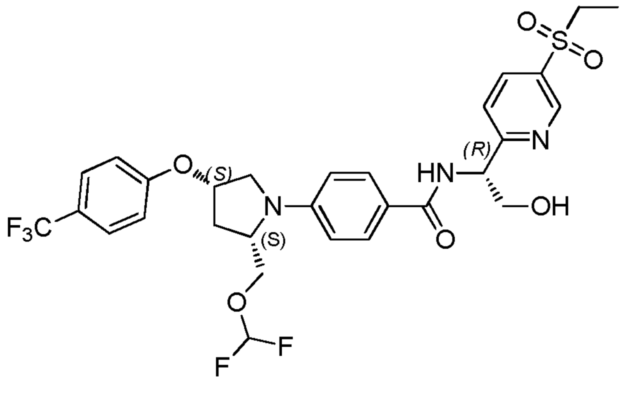

(*R*)-2-Amino-2-(5-(ethylsulfonyl)pyridin-2-yl)ethanol (48 mg, 0.21 mmol), 4-((2*S*,4*S*)-2-((difluoromethoxy)methyl)-4-(4-trifluoromethyl)phenoxy)pyrrolidin-1-yl)benzoic acid (60 mg, 0.14 mmol), EDCI (40 mg, 0.21 mmol), HOBT (30 mg, 0.22 mmol) and TEA (30 mg, 0.30 mmol) were dissolved in DCM (4 mL), and the mixture was stirred at room temperature for 23 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (76 mg, 85%).

MS (ESI, pos.ion) m/z: 644.0 $[M+H]^+$;

$^1H$ NMR (400 MHz, $CDCl_3$) δ (ppm): 8.70 (s, 1H), 7.92 (s, 2H), 7.74 (d, *J* = 8.4 Hz, 2H), 7.58 (d, *J*= 8.6 Hz, 2H), 7.25 (d, *J* = 7.1 Hz, 1H), 6.97 (d, *J* = 8.5 Hz, 2H), 6.62 (d, *J* = 8.5 Hz, 2H), 6.22 (t, *J*= 74.3 Hz, 1H), 5.24 (s, 1H), 5.17 (s, 1H), 4.24 - 4.14 (m, 2H), 4.06 - 3.89 (m, 3H), 3.75 (d, *J* = 11.3 Hz, 1H), 3.69 (dd, *J* = 11.4, 4.4 Hz, 1H), 3.34 (q, *J* = 7.4 Hz, 2H), 2.50 (d, *J* = 14.3 Hz, 1H), 2.43 - 2.34 (m, 1H), 1.26 (d, *J* = 7.4 Hz, 3H).

**Example 70 6-((2*S*,4*S*)-2-((difluoromethoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)nicotinamide**

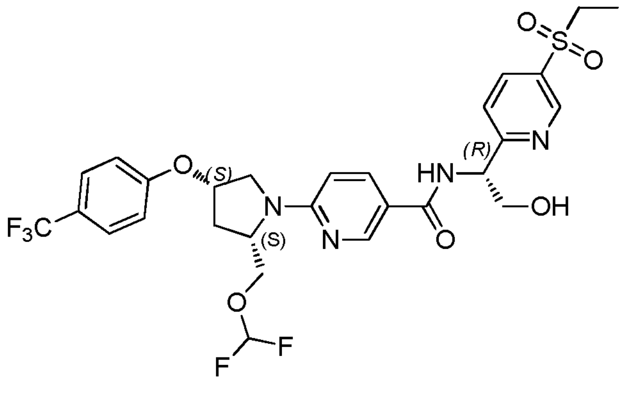

(*R*)-2-Amino-2-(5-(ethylsulfonyl)pyridin-2-yl)ethanol (31 mg, 0.13 mmol), 6-((2*S*,4*S*)-2-((difluoromethoxy)methyl)-4-(4-trifluoromethyl)phenoxy)pyrrolidin-1-yl)nicotinic acid (40 mg, 0.09 mmol), EDCI (26 mg, 0.14 mmol), HOBT (18 mg, 0.13 mmol) and TEA (28 mg, 0.28 mmol) were dissolved in DCM (4 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (48 mg, 80%).

MS (ESI, pos.ion) m/z: 645.3 $[M+H]^+$;

$^1H$ NMR (400 MHz, $CDCl_3$) δ (ppm): 8.71 (s, 1H), 8.66 (s, 1H), 7.96 (s, 2H), 7.92 (dd, *J*= 8.8, 2.0 Hz, 1H), 7.57 (d, *J* = 8.6 Hz, 2H), 7.29 (d, *J* = 7.0 Hz, 1H), 6.97 (d, *J* = 8.6 Hz, 2H), 6.44 (d, *J* = 8.8 Hz, 1H), 6.22 (t, *J* = 74.6 Hz, 1H), 5.31 - 5.26 (m, 1H), 5.16 (t, *J* = 4.2 Hz, 1H), 4.55 - 4.48 (m, 1H), 4.31 (dd, *J* = 9.4, 4.4 Hz, 1H), 4.05 (dd, *J* = 11.2, 3.6 Hz, 1H), 3.99 - 3.93 (m, 2H), 3.89 - 3.78 (m, 2H), 3.35 (q, *J* = 7.5 Hz, 2H), 2.53 (d, *J* = 14.3 Hz, 1H), 2.43 - 2.34 (m, 1H), 1.27 (t, *J* = 7.5 Hz, 3H).

**Example 71 2-((2*S*,4*S*)-2-((difluoromethoxy)methyl)-4-(4-trifluoromethyl) phenoxy)pyrrolidin-1-yl)-*N*-((*R*)-1-(5-(ethylsulfonyl)pyridin-2-yl)-2-hydroxyethyl)pyrimidine-5-carboxamide**

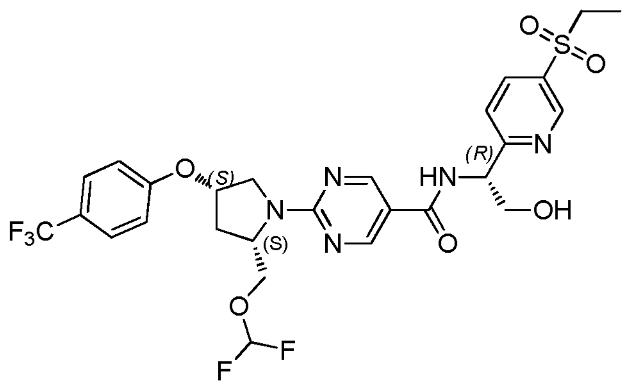

(*R*)-2-Amino-2-(5-(ethylsulfonyl)pyridin-2-yl)ethanol (56 mg, 0.24 mmol), 2-((2*S*,4*S*)-2-((difluoromethoxy)methyl)-4-(4-trifluoromethyl)phenoxy)pyrrolidin-1-yl)pyrimidine-5 -carboxylic acid (70 mg, 0.16 mmol), EDCI (46 mg, 0.24 mmol), HOBT (32 mg, 0.24 mmol) and TEA (35 mg, 0.35 mmol) were dissolved in DCM (4 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (88 mg, 84%).

MS (ESI, pos.ion) m/z: 646.4 $[M+H]^+$;

$^1H$ NMR (400 MHz, $CDCl_3$) δ (ppm): 8.80 (s, 2H), 8.73 (s, 1H), 8.02 - 7.94 (m, 2H), 7.53 (d, *J* = 8.6 Hz, 2H), 6.93 (d, *J* = 8.6 Hz, 2H), 6.19 (t, *J* = 74.7 Hz, 1H), 5.23 (t, *J* = 4.7 Hz, 1H), 5.10 (s, 1H), 4.60 - 4.53 (m, 1H), 4.32 (dd, *J* = 9.1, 4.0 Hz, 1H), 4.03 - 3.84 (m, 5H), 3.34 (q, *J* = 7.4 Hz, 2H), 2.53 (d, *J* = 14.5 Hz, 1H), 2.42 - 2.35 (m, 1H), 1.26 (t, *J* = 7.4 Hz, 3H).

**Example 72 4-((2*S*,4*R*)-2-((1,1-difluoropropoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)benzamide**

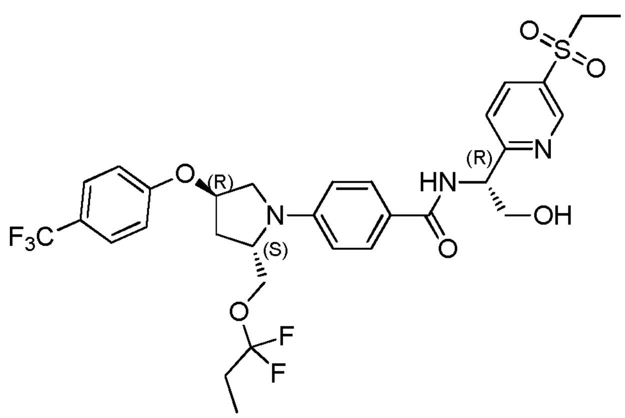

(*R*)-2-Amino-2-(5-(ethylsulfonyl)pyridin-2-yl)ethanol (26 mg, 0.11 mmol), 4-((2*S*,4*R*)-2-((1,1-difluoropropoxy)methyl)-4-(4-trifluoromethyl)phenoxy)pyrrolidin-1-yl)benzoic acid (35 mg, 0.08 mmol), EDCI (21 mg, 0.11 mmol), HOBT (15 mg, 0.11 mmol) and TEA (11 mg, 0.11 mmol) were dissolved in DCM (4 mL), and the mixture was stirred at room temperature for 17 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (25 mg, 49%).

MS (ESI, pos.ion) m/z: 672.2 [M+H]+.

$^1H$ NMR (400 MHz, $CDCl_3$) δ (ppm): 8.71 (s, 1H), 7.95 (d, *J* = 8.0 Hz, 1H), 7.92 (d, *J* = 8.1 Hz, 1H), 7.73 (d, *J* = 8.6 Hz, 2H), 7.56 (d, *J* = 8.6 Hz, 2H), 7.19 (d, *J* = 6.8 Hz, 1H), 6.95 (d, *J* = 8.6 Hz, 2H), 6.61 (d, *J* = 8.6 Hz, 2H), 5.26 (d, *J* = 3.1 Hz, 1H), 5.19 - 5.12 (m, 1H), 4.32 (s, 1H), 4.06-3.98 (m, 2H), 3.95 - 3.88 (m, 3H), 3.53 (dd, *J* = 10.5, 3.6 Hz, 1H), 3.35 (q, *J* = 7.4 Hz, 2H), 2.56 - 2.40 (m, 2H), 2.03 - 1.92 (m, 2H), 1.28 (t, *J*= 7.4 Hz, 3H), 1.03 (t, *J*= 7.5 Hz, 3H).

**Example 73 4-((2*S*,4*S*)-2-((1,1-difluoropropoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)benzamide**

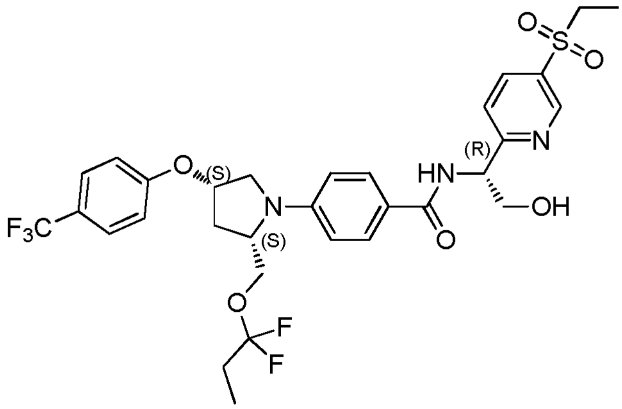

(*R*)-2-Amino-2-(5-(ethylsulfonyl)pyridin-2-yl)ethanol (15 mg, 0.07 mmol), 4-((2*S*,4*S*)-2-((1,1-difluoropropoxy)methyl)-4-(4-trifluoromethyl)phenoxy)pyrrolidin-1-yl)benzoic acid (20 mg, 0.04 mmol), EDCI (16 mg, 0.08 mmol), HOBT (11 mg, 0.08 mmol) and TEA (15 mg, 0.15 mmol) were dissolved in DCM (2 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (16 mg, 55%).

MS (ESI, pos.ion) m/z: 672.4 $[M+H]^+$.

$^1H$ NMR (600 MHz, CDCl3) δ (ppm): 8.73 (s, 1H), 7.98 (s, 1H), 7.95 (s, 1H), 7.74 (d, *J* = 8.0 Hz, 2H), 7.58 (d, *J* = 8.3 Hz, 2H), 7.12 (s, 1H), 6.98 (d, *J* = 8.3 Hz, 2H), 6.65 (d, *J* = 8.0 Hz, 2H), 5.27 (s, 1H), 5.16 (s, 1H), 4.21 - 4.14 (m, 2H), 4.06 (s, 1H), 3.99 - 3.92 (m, 2H), 3.76 - 3.68 (m, 2H), 3.36 (d, *J* = 6.8 Hz, 2H), 2.49 (d, *J* = 14.2 Hz, 1H), 2.40 - 2.35 (m, 1H), 1.95 (d, *J* = 7.6 Hz, 2H), 1.29 (t, *J*= 7.0 Hz, 3H), 1.00 (t, *J*= 7.4 Hz, 3H).

**Example 74 6-((2*S*,4*S*)-2-((1,1-difluoropropoxy)methyl)-4-(4-trifluoromethyl)phenoxy) pyrrolidin-1-yl)-*N*-((*R*)-1-(5-(ethylsulfonyl)pyridin-2-yl)-2-hydroxyethyl)nicotinamide**

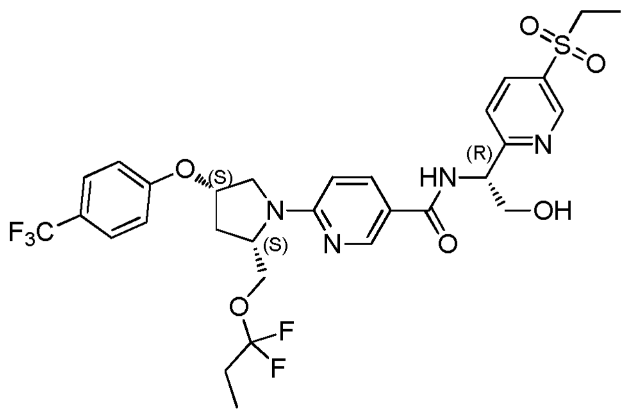

(*R*)-2-Amino-2-(5-(ethylsulfonyl)pyridin-2-yl)ethanol (15 mg, 0.07 mmol), 6-((2*S*,4*S*)-2-((1,1-difluoropropoxy)methyl)-4-(4-trifluoromethyl)phenoxy)pyrrolidin-1-yl)nicotinic acid (20 mg, 0.04 mmol), EDCI (16 mg, 0.08 mmol), HOBT (11 mg, 0.08 mmol) and TEA (10 mg, 0.10 mmol) were dissolved in DCM (3 mL), and the mixture was stirred at room temperature for 22 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (20 mg, 68%).

MS (ESI, pos.ion) m/z: 673.4 $[M+H]^+$.

$^1H$ NMR (600 MHz, $CDCl_3$) $\delta$ (ppm): 8.73 (d, *J* = 1.3 Hz, 1H), 8.66 (d, *J* = 2.0 Hz, 1H), 8.00 (d, *J* = 8.0 Hz, 1H), 7.96 (dd, *J* = 8.1, 1.9 Hz, 1H), 7.92 (dd, *J* = 8.9, 2.4 Hz, 1H), 7.57 (d, *J* = 8.6 Hz, 2H), 7.18 (d, *J* = 6.9 Hz, 1H), 6.97 (d, *J* = 8.6 Hz, 2H), 6.48 (d, *J* = 8.8 Hz, 1H), 5.29 (d, *J* = 4.4 Hz, 1H), 5.14 (t, *J*= 4.9 Hz, 1H), 4.46 (s, 1H), 4.28 (dd, *J*= 9.5, 4.6 Hz, 1H), 4.08 (dd, *J*= 11.2, 3.8 Hz, 1H), 3.99 - 3.95 (m, 2H), 3.90 (dd, *J* = 12.2, 4.8 Hz, 1H), 3.83 (d, *J* = 12.1 Hz, 1H), 3.38 (q, *J* = 7.5 Hz, 2H), 2.51 (d, *J* = 14.3 Hz, 1H), 2.40 - 2.36 (m, 1H), 1.96 - 1.89 (m, 2H), 1.29 (t, *J* = 7.5 Hz, 3H), 0.98 (t, *J* = 7.5 Hz, 3H).

Example 75 **2-((2*S*,4*S*)-2-((1,1-difluoropropoxy)methyl)-4-(4-trifluoromethyl) phenoxy)pyrrolidin-1-yl)-*N*((R)-1-(5-(ethylsulfonyl)pyridin-2-yl)-2-hydroxyethyl)pyrimidine-5-carboxamide**

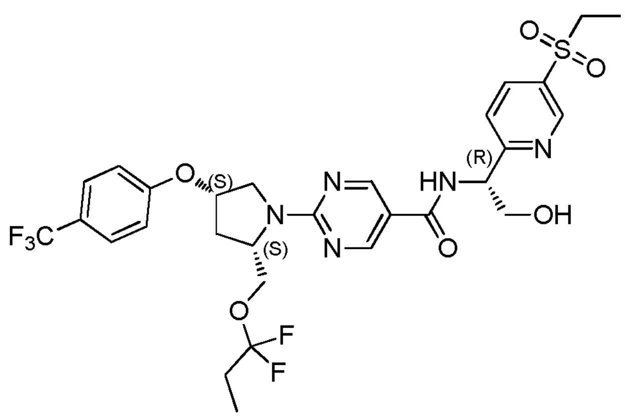

(*R*)-2-Amino-2-(5-(ethylsulfonyl)pyridin-2-yl)ethanol (22 mg, 0.10 mmol), 2-((2*S*,4*S*)-2-((1,1-difluoropropoxy)methyl)-4-(4-trifluoromethyl)phenoxy)pyrrolidin-1-yl)pyrimidi ne-5-carboxylic acid (30 mg, 0.07 mmol), EDCI(24mg, 0.13 mmol), HOBT (17 mg, 0.13 mmol) and TEA (30 mg, 0.30 mmol) were dissolved in DCM (3 mL), and the mixture was stirred at room temperature for 18 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (25 mg, 57%).

MS (ESI, pos.ion) m/z: 674.4 $[M+H]^+$.

$^1H$ NMR (400 MHz, $CDCl_3$) δ (ppm): 8.88 (s, 2H), 8.73 (s, 1H), 7.99 (s, 2H), 7.56 (d, *J* = 8.2 Hz, 3H), 6.96 (d, *J*= 8.2 Hz, 2H), 5.30 (s, 1H), 5.12 (s, 1H), 4.61 (s, 1H), 4.33 (d, *J*= 7.5 Hz, 1H), 4.11 - 3.88 (m, 5H), 3.36 (t, *J* = 7.4 Hz, 2H), 2.55 (d, *J* = 13.6 Hz, 1H), 2.41 (s, 1H), 1.93 (dd, *J* = 18.2, 10.6 Hz, 2H), 1.29 (t, *J* = 7.4 Hz, 3H), 0.96 (t, *J* = 7.5 Hz, 3H).

Example 76 **N-(4-(ethylsulfonyl)benzyl)-4-(4-(4-(trifluoromethyl)benzyl)-1,4-diazepan -1-yl)benzamide**

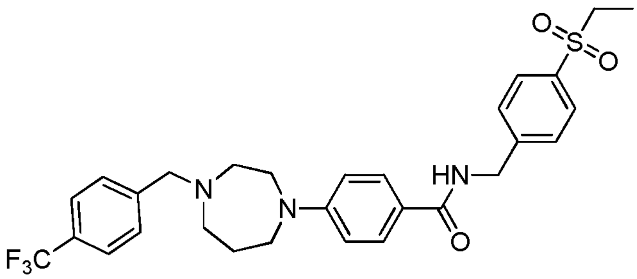

Step 1: **Synthesis of *tert*-butyl 1,4-diazepan-1-carboxylate**

To a solution of 1,4-diazepane (2.52 g, 25.20 mmol) in THF (70 mL) were added TEA (5.20 mL, 37.00 mmol) and $Boc_2O$ (4.60 mL, 20.02 mmol) sequentially under an ice bath. After the addition was completed, the mixture was stirred at room temperature for 19 h. The reaction solution was concentrated under reduced pressure, and the crude product was separated by silica gel column chromatography (eluent: PE/EtOAc (v/v) = 1/1) to give yellow oily liquid (1.00 g, 20%).

MS (ESI, pos.ion) m/z: 201.2 $[M+H]^+$.

Step 2: **Synthesis of *tert*-butyl 4-(4-(trifluoromethyl)benzyl)-1,4-diazepan-1-carboxylate**

Under nitrogen protection, to a solution of tert-butyl 1,4-diazepan-1-carboxylate (1.99 g, 9.94 mmol) and 4-(trifluoromethyl)benzaldehyde (621 mg, 3.57 mmol) in DCM (10 mL) was added AcOH (220 mg, 3.66 mmol).After the mixture was stirred at room temperature for 24 h, $NaBH_3CN$ (448 mg, 7.13 mmol) was added, and the mixture was continued to stir at room temperature for 26 h. Saturated $NaHCO_3$ solution (10 mL) was added to the reaction system to quench the reaction. The reaction solution was diluted with DCM (100 mL), washed successively with saturated $NaHCO_3$ solution (30 mL) and saturated NaCl solution (30 mL). The combined aqueous phases were extracted with DCM (40 mL×2). The combined organic phases were dried over anhydrous $Na_2SO_4$, and concentrated under reduced pressure. The crude product was separated by silica gel column chromatography (eluent: PE/EtOAc (v/v) = 5/1) to give colorless oily liquid (1.00 g, 78%).

MS (ESI, pos.ion) m/z: 359.2 $[M+H]^+$.

**Step 3: Synthesis of 1-(4-(trifluoromethyl)benzyl)-1,4-diazepane**

*tert*-Butyl 4-(4-(trifluoromethyl)benzyl)-1,4-diazepan-1-carboxylate (200 mg, 0.56 mmol) was dissolved in a solution of HCl in methanol (7 mL, 20%) and the mixture was stirred at room temperature for 11 h. Saturated $Na_2CO_3$ solution was added dropwise to the system until no bubbles were generated. The mixture was concentrated under reduced pressure to remove ethanol. The residue was diluted with EtOAc (100 mL) and separated for layers. The organic phase was washed with saturated NaCl solution (40 mL), dried over anhydrous $Na_2SO_4$, and concentrated under reduced pressure to give yellow oily liquid (50 mg, 35%).

MS (ESI, pos.ion) m/z: 259.2 $[M+H]^+$.

Step 4: Synthesis of **4-(4-(4-(trifluoromethyl)benzyl)-1,4-diazepan-1-yl)benzonitrile**

To a solution of 1-(4-(trifluoromethyl)benzyl)-1,4-diazepane (104 mg, 0.40 mmol) and 4-fluorobenzonitrile (100 mg, 0.83 mmol) in DMSO (4 mL) was added $K_2CO_3$ (270 mg, 1.95 mmol). The mixture was heated and stirred in an oil bath at 100°C for 24 h. The reaction solution was cooled to room temperature, diluted with EtOAc (120 mL), washed with saturated NaCl solution (30 mL×5), dried over anhydrous $Na_2SO_4$, and concentrated under reduced pressure. The crude product was separated by silica gel column chromatography (eluent: PE/EtOAc (v/v) = 5/1) to give yellow oily liquid (50 mg, 35%).

MS (ESI, pos.ion) m/z: 360.2 $[M+H]^+$.

Step 5: Synthesis of **4-(4-(4-(trifluoromethyl)benzyl)-1,4-diazepan-1-yl)benzoic** acid

To a solution of 4-(4-(4-(trifluoromethyl)benzyl)-1,4-diazepan-1-yl)benzonitrile (80 mg, 0.22 mmol) in EtOH (4 mL) was added MeONa (434 mg, 8.03 mmol). The mixture was heated and stirred in an oil bath at 100°C for 19 h. The reaction solution was cooled to room temperature, then a small amount of water was added to dissolve the solid. HCl solution (1 mol/L) was added dropwise to the reaction solution to adjust the pH of the solution to about 3. The resulting mixture was concentrated under reduced pressure to remove ethanol. The aqueous phase was extracted with DCM (30 mL×3). The organic phases were combined and dried over anhydrous $Na_2SO_4$, concentrated under reduced pressure to give a yellow solid (84 mg, 100%).

MS (ESI, pos.ion) m/z: 379.2 $[M+H]^+$.

Step 6: Synthesis of ***N*-(4-(ethylsulfonyl)benzyl)-4-(4-(4-(trifluoromethyl)benzyl) -1,4-diazepan-1-yl)benzamide**

4-(4-(4-(Trifluoromethyl)benzyl)-1,4-diazepan-1-yl)benzoic acid (80 mg, 0.21 mmol), (4-(ethylsulfonyl)phenyl)methanamine (130 mg, 0.65 mmol), EDCI (90 mg, 0.47 mmol) and HOBT (58 mg, 0.43 mmol) were dissolved in DCM (7 mL), then TEA (48 mg, 0.47 mmol) was added and the mixture was stirred at room temperature for 25 h. The reaction solution was diluted with DCM (100 mL), washed successively with $NaHCO_3$ solution (30 mL) and saturated NaCl solution (30 mL), dried over anhydrous $Na_2SO_4$, and concentrated under reduced pressure. The crude product was separated by silica gel column chromatography (eluent: PE/EtOAc (v/v) = 1/1) to give pale yellow oily liquid (58 mg, 49%).

MS (ESI, pos.ion) m/z: 560.2 $[M+H]^+$;

$^1H$ NMR (400 MHz, $CDCl_3$) δ (ppm): 7.74 (t, *J* = 9.4 Hz, 4H), 7.55 (d, *J* = 8.0 Hz, 2H),7.45 (m, 4H), 6.86 (t, *J* = 5.9 Hz, 1H), 6.65 (d, *J* = 8.8 Hz, 2H), 4.68 (d, *J* = 6.0 Hz, 2H), 3.69 (s, 2H), 3.62 - 3.52 (m, 4H), 3.06 (q, *J*= 7.4 Hz, 2H), 2.80 - 2.73 (m, 2H), 2.65 - 2.58 (m, 2H), 1.97 (dd, *J* = 10.7, 5.5 Hz, 2H), 1.25 (t, 3H).

**Example 77 *N*-((*S*)-1-(4-(ethylsulfonyl)phenyl)-3-hydroxypropyl)-4-((*S*)-3-(4-(trifluoromethyl)phenoxy)pyrrolidin-1-yl)benzamide**

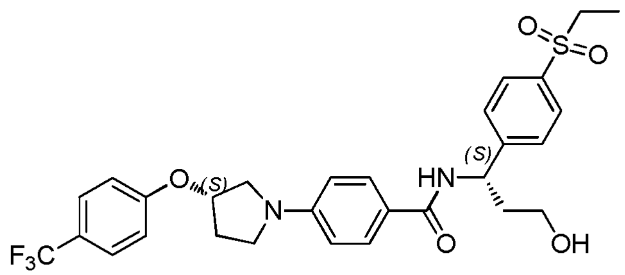

**Step 1: Synthesis of *tert-butyl* (*S*)-3-(4-(trifluoromethyl)phenoxy) pyrrolidine-1-carboxylate**

*tert*-Butyl (*R*)-3-hydroxypyrrolidine-1-carboxylate (1.00 g, 5.34 mmol), 4-(trifluoromethyl)phenol (865 mg, 5.34 mmol), $PPh_3$ (2.00 g, 5.83 mmol) were added to THF (20 mL). The mixture was transferred to 0 °C, and DIAD (1.40 mL, 7.11 mmol) was slowly added. After the addition was complete, the mixture was transferred to room temperature and reacted for 21 h. The reaction solution was concentrated under reduced pressure. The residue was diluted with methyl tert-butyl ether (30 mL) and stirred at -20 °C. A large amount of white insoluble solids were precipitated, and filtered while cold. The filter cake was washed with cold methyl tert-butyl ether, and the filtrate was concentrated under reduced pressure. The crude product was separated by silica gel column chromatography (eluent: PE/EtOAc (v/v) = 6/1) to give pale yellow liquid (1.76 g, 100%).

MS (ESI, pos.ion) m/z: 276.2 $[M-56+H]^+$.

**Step 2: Synthesis of (*S*)-3-(4-(trifluoromethyl)phenoxy)pyrrolidine**

To a solution of *tert-butyl* (*S*)-3-(4-(trifluoromethyl)phenoxy)pyrrolidine-1-carboxylate (800 mg, 2.42 mmol) in DCM (8 mL) was added a solution of HCl in methanol (2 mL, 20%). The mixture was reacted at room temperature for

9 h. After the reaction was completed, saturated sodium bicarbonate solution was added for washing, and the reaction solution was concentrated under reduced pressure to obtain light red liquid (558 mg, 100%).

MS (ESI, pos.ion) m/z: 232.2 $[M+H]^+$.

Step 3: Synthesis of methyl **(*S*)-4-(3-(4-(trifluoromethyl)phenoxy)pyrrolidin-1-yl)benzoate**

Under nitrogen protection, (*S*)-3-(4-(trifluoromethyl)phenoxy)pyrrolidine (600 mg, 2.60 mmol), $Pd_2(dba)_3$ (237 mg, 0.26 mmol), XantPhos (225 mg, 0.39 mmol), $Cs_2CO_3$ (1.26 g, 3.87 mmol), methyl 4-iodobenzoate (884 mg, 3.37 mmol) were successively added to 1,4-dioxane (16 mL), and the mixture was heated to 100°C and reacted for 15 h. The reaction solution was cooled to room temperature and filtered, and the filtrate was concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give a yellow solid (850 mg, 89%).

MS (ESI, pos.ion) m/z: 366.3 $[M+H]^+$.

**Step 4: Synthesis of (*S*)-4-(3-(4-(trifluoromethyl)phenoxy)pyrrolidin-1-yl)benzoic acid**

To a solution of methyl (*S*)-4-(3-(4-(trifluoromethyl)phenoxy)pyrrolidin-1-yl)benzoate (850 mg, 2.33 mmol) in MeOH (6 mL) and THF (6 mL) was added a solution of $LiOH \cdot H_2O$ (2.00 g, 47.70 mmol) in $H_2O$ (6 mL). The mixture was reacted at room temperature for 19 h. The reaction solution was concentrated under reduced pressure, and the remaining solution was added with HCl solution (3 mol/L) to adjust the pH of the solution to about 4. The resulting mixture was extracted with EtOAc (30 mL×2), the organic phases were combined, washed with saturated NaCl (25 mL) solution, dried over anhydrous $Na_2SO_4$, concentrated under reduced pressure, and the crude product was separated by silica gel column chromatography (eluent: PE/EtOAc (v/v) = 2/1) to give a white solid (550 mg, 67%).

MS (ESI, pos.ion) m/z: 352.1 $[M+H]^+$.

**Step 5: Synthesis of *N*-((*S*)-1-(4-(ethylsulfonyl)phenyl)-3-hydroxypropyl)-4-((*S*)-3-(4-(trifluoromethyl)phenoxy)pyrrolidin-1-yl)benzamide**

EDCI (81 mg, 0.42 mmol), HOBT (60 mg, 0.44 mmol), (*R*)-3-amino-3-(4-(ethylsulfonyl)phenyl)propan-1-ol (103 mg, 0.42 mmol), (*S*)-4-(3-(4-(trifluoromethyl)phenoxy)pyrrolidin-1-yl)benzoic acid (100 mg , 0.28 mmol) and TEA (60 mg, 0.59 mmol) were successively added to DCM (6 mL) and the mixture was reacted at room temperature for 12 h. The reaction solution was diluted with DCM (30 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (125 mg, 76%).

MS (ESI, pos.ion) m/z: 577.2 $[M+H]^+$;

$^1H$ NMR (400 MHz, DMSO-$d_6$) δ (ppm): 8.52 (d, *J* = 7.8 Hz, 1H), 7.83 (d, *J* = 8.3 Hz, 2H), 7.76 (d, *J* = 8.7 Hz, 2H), 7.66 (d, *J* = 8.7 Hz, 2H), 7.62 (d, *J* = 8.2 Hz, 2H), 7.17 (d, *J* = 8.6 Hz, 2H), 6.59 (d, *J* = 8.7 Hz, 2H), 5.30 (s, 1H), 5.21 (dd, *J* = 14.2, 8.1 Hz, 1H), 4.65 (t, *J* = 4.8 Hz, 1H), 3.74 (dd, *J*= 11.5, 4.6 Hz, 1H), 3.46 (d, *J* = 10.5 Hz, 4H), 3.25 (q, *J*= 7.3 Hz, 2H), 2.40 - 2.33 (m, 1H), 2.27 - 2.19 (m, 1H), 2.09 - 2.01 (m, 1H), 1.94 - 1.84 (m, 1H), 1.09 (t, *J* = 7.3 Hz, 3H).

**Example 78 *N*-((*R*)-1-(5-(ethylsulfonyl)pyridin-2-yl)-2-hydroxyethyl)-4-((*S*)-3-(4-(trifluoromethyl)phenoxy)pyrrolidin-1-yl)benzamide**

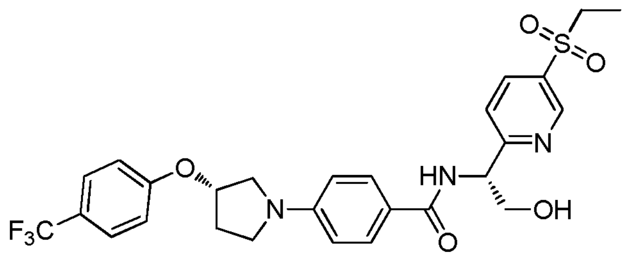

EDCI (94 mg, 0.49 mmol), HOBT (66 mg, 0.49 mmol), (*R*)-2-amino-2-(5-(ethylsulfonyl)pyridin-2-yl)ethanol (113 mg, 0.49 mmol), (*S*)-4-(3-(4-(trifluoromethyl)phenoxy)pyrrolidin-1-yl)benzoic acid (115 mg, 0.33 mmol) and TEA (100

mg, 0.99 mmol) were successively added to DCM (8 mL) and the mixture was reacted at room temperature for 9 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (130 mg, 70%).

MS (ESI, pos.ion) m/z: 564.1 $[M+H]^+$.

$^1H$ NMR (400 MHz, $CDCl_3$) δ (ppm): 8.71 (s, 1H), 7.96 (d, *J* = 8.1 Hz, 1H), 7.91 (d, *J* = 9.4 Hz, 1H), 7.72 (d, *J* = 8.6 Hz, 2H), 7.56 (d, *J* = 8.6 Hz, 2H), 7.15 (d, *J* = 7.0 Hz, 1H), 6.96 (d, *J* = 8.6 Hz, 2H), 6.53 (d, *J*= 8.7 Hz, 2H), 5.25 (d, *J*= 6.2 Hz, 1H), 5.13 (s, 1H), 4.07- 4.01 (m, 1H), 3.97 - 3.89 (m, 1H), 3.75 (dd, *J* = 11.2, 4.7 Hz, 1H), 3.60 - 3.50 (m, 3H), 3.35 (q, *J* = 7.4 Hz, 2H), 3.19 (s, 1H), 2.43 - 2.29 (m, 2H), 1.28 (t, *J*= 7.4 Hz, 3H).

### Example 79 *N*-((*R*)-1-(4-(ethylsulfonyl)phenyl)-2-hydroxyethyl)-3-fluoro-4-((*S*)-3-(4-(trifluoromethyl)phenoxy)pyrrolidin-1-yl)benzamide

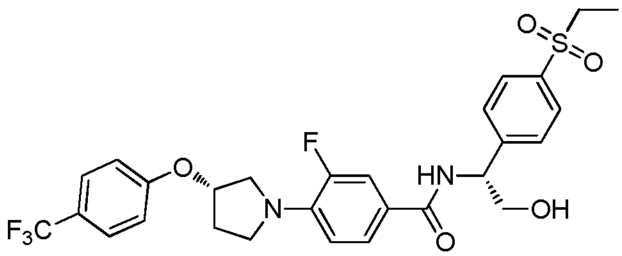

### Step 1: Synthesis of methyl (*S*)-3-fluoro-4-(3-(4-(trifluoromethyl) phenoxy)pyrrolidin-1-yl)benzoate

Under nitrogen protection, (*S*)-3-(4-(trifluoromethyl)phenoxy)pyrrolidine (300 mg, 1.30 mmol), $Pd_2(dba)_3$ (118 mg, 0.13 mmol), XantPhos (112 mg, 0.19 mmol), $Cs_2CO_3$ (640 mg, 1.96 mmol), methyl 3-fluoro-4-iodo-benzoate (472 mg, 1.69 mmol) were successively added to 1,4-dioxane (8 mL) and the mixture was heated to 100°C and reacted for 21 h. The reaction solution was cooled to room temperature, and concentrated under reduced pressure. The residue was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give a yellow solid (400 mg, 80%).

MS (ESI, pos.ion) m/z: 384.1 $[M+H]^+$.

### Step 2: Synthesis of (*S*)-3-fluoro-4-(3-(4-(trifluoromethyl)phenoxy)pyrrolidin-1-yl)benzoic acid

To a solution of methyl (*S*)-3-fluoro-4-(3-(4-(trifluoromethyl)phenoxy) pyrrolidin-1-yl)benzoate (400 mg, 1.04 mmol) in MeOH (5 mL) and THF (3 mL) was added a solution of $LiOH{\cdot}H_2O$ (875 mg, 20.85 mmol) in $H_2O$ (4 mL). The mixture was reacted at room temperature for 15 h. The reaction solution was added with HCl solution (1 mol/L) to adjust the pH of the solution to about 4. The resulting mixture was extracted with EtOAc (30 mL × 2), and the organic phases were combined, washed with saturated NaCl (25 mL) solution, dried over anhydrous $Na_2SO_4$, concentrated under reduced pressure, and the crude product was separated by silica gel column chromatography (eluent: PE/EtOAc (v/v) = 2/1) to give a white solid (340 mg, 88%).

MS (ESI, pos.ion) m/z: 370.2 $[M+H]^+$.

### Step 3: Synthesis of *N*-((*R*)-1-(4-(ethylsulfonyl)phenyl)-2-hydroxyethyl)-3-fluoro -4-((*S*)-3-(4-(trifluoromethyl)phenoxy)pyrrolidin-1-yl)benzamide

EDCI (79 mg, 0.41 mmol), HOBT (55 mg, 0.41 mmol), (*R*)-2-amino-2-(4-(ethylsulfonyl)phenyl)ethanol (74 mg, 0.32 mmol), (*S*)-3-fluoro-4-(3-(4-(trifluoromethyl)phenoxy)pyrrolidin-1-yl)benzoic acid (100 mg , 0.27 mmol) and TEA (60 mg, 0.59 mmol) were successively added to DCM (6 mL) and the mixture was reacted at room temperature for 22 h. The reaction solution was concentrated under reduced pressure, and the crude product was separated by silica gel column chromatography (eluent: DCM/MeOH (v/v) = 20/1) to give a white solid (130 mg, 83%).

MS (ESI, pos.ion) m/z: 581.0 $[M+H]^+$;

$^1H$ NMR (400 MHz, $CDCl_3$) δ (ppm): 7.83 (d, *J*= 8.3 Hz, 2H), 7.58 - 7.52 (m, 5H), 7.50 (d, *J* = 3.4 Hz, 1H), 7.09 (d, *J* = 6.9 Hz, 1H), 6.95 (d, *J* = 8.6 Hz, 2H), 6.61 (t, *J* = 8.6 Hz, 1H), 5.24 (dd, *J*= 10.8, 4.5 Hz, 1H), 5.06 (s, 1H), 4.03 -

3.94 (m, 3H), 3.68 (t, *J*= 8.7 Hz, 2H), 3.60 (t, *J*= 8.7 Hz, 1H), 3.08 (q, *J* = 7.4 Hz, 2H), 2.80 (s, 1H), 2.36 - 2.22 (m, 2H), 1.26 (t, *J* = 7.4 Hz, 3H).

**Example 80 *N*-((*R*)-1-(4-(ethylsulfonyl)phenyl)-2-hydroxyethyl)-2-fluoro-4-((*S*)-3 -(4-(trifluoromethyl)phenoxy)pyrrolidin-1-yl)benzamide**

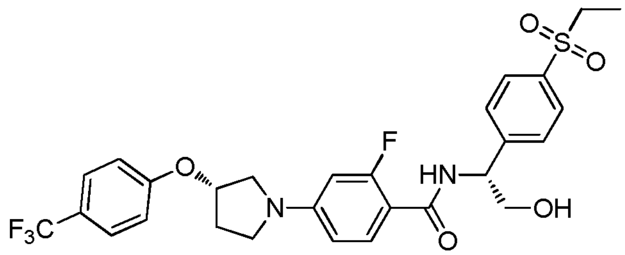

**Step 1: Synthesis of methyl (*S*)-2-fluoro-4-(3-(4-(trifluoromethyl)phenoxy) pyrrolidin-1-yl)benzoate**

Under nitrogen protection, (*S*)-3-(4-(trifluoromethyl)phenoxy)pyrrolidine (300 mg, 1.30 mmol), $Pd_2(dba)_3$ (120 mg, 0.13 mmol), XantPhos (112 mg, 0.19 mmol), $Cs_2CO_3$ (634 mg, 1.95 mmol), methyl 2-fluoro-4-iodo-benzoate (438 mg, 1.56 mmol) were successively added to 1,4-dioxane (8 mL) and the mixture was heated to 100°C and reacted for 12 h. The reaction solution was cooled to room temperature, and concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give a pale yellow solid (328 mg, 66%).
MS (ESI, pos.ion) m/z: 384.1 $[M+H]^+$.

**Step 2: Synthesis of (*S*)-2-fluoro-4-(3-(4-(trifluoromethyl)phenoxy)pyrrolidin-1-yl)benzoic acid**

To a solution of methyl (*S*)-2-fluoro-4-(3-(4-(trifluoromethyl)phenoxy) pyrrolidin-1-yl)benzoate (320 mg, 0.83 mmol) in MeOH (4 mL) and THF (4 mL) was added a solution of $LiOH \cdot H_2O$ (700 mg, 16.68 mmol) in $H_2O$ (4 mL). The mixture was reacted at room temperature for 19 h. The reaction solution was concentrated under reduced pressure, and the residue was added with HCl solution (1 mol/L) to adjust the pH to 4. The resulting mixture was extracted with EtOAc (30 mL × 2), and the combined organic phases were washed with saturated NaCl solution (25 mL), dried over anhydrous $Na_2SO_4$, and concentrated under reduced pressure to give a yellow solid (280 mg, 91%).
MS (ESI, pos.ion) m/z: 370.0 $[M+H]^+$.

**Step 3: Synthesis of *N*-((*R*)-1-(4-(ethylsulfonyl)phenyl)-2-hydroxyethyl)-2-fluoro -4-((*S*)-3-(4-(trifluoromethyl)phenoxy)pyrrolidin-1-yl)benzamide**

EDCI (62 mg, 0.32 mmol), HOBT (43 mg, 0.32 mmol), (*R*)-2-amino-2-(4-(ethylsulfonyl)phenyl)ethanol (60 mg, 0.26 mmol), (*S*)-2-fluoro-4-(3-(4-(trifluoromethyl)phenoxy)pyrrolidin-1-yl)benzoic acid (80 mg, 0.22 mmol) and TEA (43 mg, 0.42 mmol) were successively added to DCM (6 mL) and the mixture was reacted at room temperature for 17 h. The reaction solution was concentrated under reduced pressure, and the crude product was separated by silica gel column chromatography (eluent: DCM/MeOH (v/v) = 20/1) to give a white solid (100 mg, 80%).
MS (ESI, pos.ion) m/z: 581.1 $[M+H]^+$;
$^1H$ NMR (400 MHz, $CDCl_3$) $\delta$ (ppm): 7.95 (d, *J* = 9.0 Hz, 1H), 7.90 (d, *J*= 8.0, 2H), 7.60 (dd, *J* = 8.0, 6.8 Hz, 4H), 7.50 (dd, *J* = 14.7, 6.9 Hz, 1H), 6.98 (d, *J* = 8.6 Hz, 2H), 6.42 (dd, *J* = 8.9, 2.0 Hz, 1H), 6.24 (dd, *J* = 15.7, 1.8 Hz, 1H), 5.36 (d, *J* = 4.5 Hz, 1H), 5.16 (s, 1H), 4.09 - 3.94 (m, 2H), 3.76 (dd, *J* = 11.3, 4.6 Hz, 1H), 3.63 - 3.51 (m, 3H), 3.11 (q, *J* = 7.4 Hz, 2H), 2.55 - 2.31 (m, 3H), 1.29 (d, *J* = 7.5 Hz, 3H).

**Example 81 *N*-((*R*)-1-(4-(ethylsulfonyl)phenyl)-2-hydroxyethyl)-5-fluoro-6-((*S*)-3-(4-(trifluoromethyl)phenoxy)pyrrolidin-1-yl)nicotinamide**

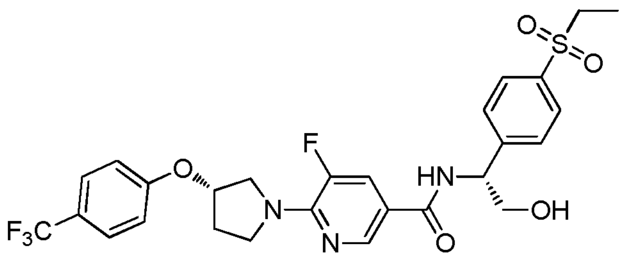

**Step 1: Synthesis of methyl (*S*)-5-fluoro-6-(3-(4-(trifluoromethyl)phenoxy) pyrrolidin-1-yl)nicotinate**

Under nitrogen protection, (*S*)-3-(4-(trifluoromethyl)phenoxy)pyrrolidine (150 mg, 0.65 mmol), $Pd_2(dba)_3$ (59 mg, 0.06 mmol), XantPhos (56 mg, 0.10 mmol), $Cs_2CO_3$ (320 mg, 0.98 mmol), methyl 6-bromo-5-fluoronicotinate (180 mg, 0.77 mmol) were successively added to 1,4-dioxane (6 mL) and the mixture was heated to 100°C and reacted for 16 h. The reaction solution was cooled to room temperature, and concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 4/1) to give a pale yellow solid (210 mg, 84%).

MS (ESI, pos.ion) m/z: 385.1 $[M+H]^+$.

**Step 2: Synthesis of (*S*)-5-fluoro-6-(3-(4-(trifluoromethyl)phenoxy)pyrrolidin -1-yl)nicotinic acid**

To a solution of methyl (*S*)-5-fluoro-6-(3-(4-(trifluoromethyl)phenoxy) pyrrolidin-1-yl)nicotinate (210 mg, 0.55 mmol) in MeOH (3 mL) and THF (3 mL) was added a solution of $LiOH{\cdot}H_2O$ (458 mg, 10.92 mmol) in $H_2O$ (3 mL). The mixture was reacted at room temperature for 16 h. The reaction solution was added with HCl solution (1 mol/L) to adjust the pH to 4. The resulting mixture was extracted with EtOAc (30 mL × 2), and the combined organic phases were washed with saturated NaCl solution (25 mL), dried over anhydrous $Na_2SO_4$, and concentrated under reduced pressure to give a pale yellow solid (190 mg, 94%).

MS (ESI, pos.ion) m/z: 371.2 $[M+H]^+$.

**Step 3: Synthesis of *N*-((R)-1-(4-(ethylsulfonyl)phenyl)-2-hydroxyethyl) -5-fluoro-6-((*S*)-3-(4-(trifluoromethyl)phenoxy)pyrrolidin-1-yl)nicotinamide**

EDCI (77 mg, 0.40 mmol), HOBT (54 mg, 0.40 mmol), (*R*)-2-amino-2-(4-(ethylsulfonyl)phenyl)ethanol (73 mg, 0.32 mmol), (*S*)-5-fluoro-6-(3-(4-(trifluoromethyl)phenoxy)pyrrolidin-1-yl)nicotinic acid (100 mg , 0.27 mmol) and TEA (54 mg, 0.53 mmol) were successively added to DCM (6 mL), and the mixture was reacted at room temperature for 22 h. The reaction solution was concentrated under reduced pressure, and the crude product was separated by silica gel column chromatography (eluent: DCM/MeOH (v/v) = 20/1) to give a white solid (138 mg, 88%).

MS (ESI, pos.ion) m/z: 582.0 $[M+H]^+$.

$^1H$ NMR (400 MHz, DMSO-$d_6$) δ (ppm): 8.64 (d, *J* = 7.8 Hz, 1H), 8.48 (s, 1H), 7.90 (d, *J* = 14.5 Hz, 1H), 7.83 (d, *J* = 8.2 Hz, 2H), 7.65 (t, *J* = 8.9 Hz, 4H), 7.18 (d, *J* = 8.5 Hz, 2H), 5.26 (s, 1H), 5.12 (dd, *J* = 13.6, 7.0 Hz, 1H), 5.06 (t, *J* = 5.8 Hz, 1H), 3.99 (d, *J* = 9.2 Hz, 1H), 3.90 - 3.80 m, 2H), 3.74 - 3.66 (m, 3H), 3.26 (q, *J* = 7.3 Hz, 2H), 2.34 - 2.16 (m, 2H), 1.09 (t, *J* = 7.4 Hz, 3H).

**Example 82 4-((2*S*,4*S*)-4-((2,2-difluorobenzo[*d*][1,3]dioxol-5-yl)oxy)-2-((difluoromethoxy) methyl)pyrrolidin-1-yl)-*N*-((*R*)-1-(4-(ethylsulfonyl)phenyl)-2-hydroxyethyl)benzamide**

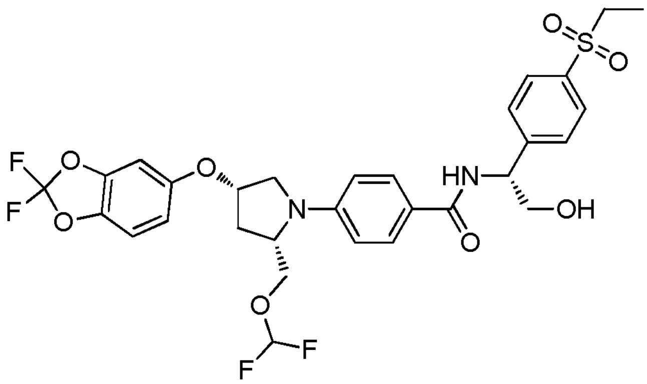

**Step 1: Synthesis of (2*S*,4*S*)-2-methyl 1-*tert*-butyl 4-((2,2-difluorobenzo [*d*][1,3] dioxol-5-yl)oxy)pyrrolidine-1,2-dicarboxylate**

(2*S*,4*R*)-1-*tert*-Butyl 2-methyl 4-hydroxypyrrolidine-1,2-dicarboxylate (2.00 g, 7.75 mmol), 2,2-difluorobenzo[*d*][1,3]dioxol-5-ol (1.50 g, 7.75 mmol), $PPh_3$ (2.35 g, 8.96 mmol) were added to THF (20 mL). The mixture was transferred to 0 °C, and DIAD (2.00 mL, 10.16 mmol) was slowly added. After the addition was complete, the mixture was transferred to room temperature and stirred for 24 h. The reaction solution was concentrated under reduced pressure. The residue was diluted with methyl tert-butyl ether (50 mL) and stirred at -20 °C. A large amount of white insoluble solids (triphenoxyphosphine) were precipitated, filtered while cold, and the filter cake was washed with cold methyl tert-butyl ether. The filtrate was concentrated under reduced pressure, and the crude product was separated by silica gel column chromatography (eluent: PE/EtOAc (v/v) = 4/1) to give yellow liquid (3.00 g, 96%).
MS (ESI, pos.ion) m/z: 346.2 $[M-56+H]^+$.

**Step 2: Synthesis of *tert*-butyl (2*S*,4*S*)-4-((2,2-difluorobenzo[d][1,3]dioxol-5-yl) oxy)-2-hydroxymethyl)pyrrolidine-l-dicarboxylate**

To THF (30 mL) solution was added (2*S*,4*S*)-2-methyl 4-*tert*-butyl 4-((2,2-difluorobenzo[*d*][1,3]dioxol-5-yl)oxy)pyrrolidin-1,2-dicarboxylate (3.30 g, 5.47 mmol), then the mixture was transferred to 0 °C, and $LiBH_4$ (325 mg, 14.92 mmol) was added slowly. After the addition was completed, the mixture was stirred at room temperature for 15 h. Saturated $NH_4Cl$ (20 mL) solution was added to the reaction solution to quench the reaction. The mixture was left standing for layers, the upper organic phase was separated, and the aqueous phase was extracted with EtOAc (20 mL × 2). The combined organic phases were dried over anhydrous $Na_2SO_4$, concentrated under reduced pressure, and the crude product was separated by silica gel column chromatography (eluent: PE/EtOAc (v/v) = 3/1) to give yellow liquid (2.60 g, 93%).
MS (ESI, pos.ion) m/z: 318.2 $[M-56+H]^+$.

**Step 3: Synthesis of *tert-butyl* (2*S*,4*S*)-4-((2,2-difluorobenzo[*d*][1,3]dioxol-5-yl)oxy) -2-((difluoromethoxy)methyl)pyrrolidine-1-dicarboxylate**

To a solution of *tert*-butyl (2*S*,4*S*)-4-((2,2-difluorobenzo[*d*][1,3]dioxol-5-yl) oxy)-2-)hydroxymethyl)pyrrolidine-1-carboxylate (2.50 g, 6.70 mmol) in DCM (12 mL) were sequentially added $H_2O$ (12 mL), KOAc (8.0 g, 80.30 mmol), $TMSCF_2Br$ (8.50 mL, 55.00 mmol). The mixture was stirred at room temperature for 19 h. The reaction solution was diluted with DCM (50 mL), washed with saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give colorless liquid (2.25 g, 79%).
MS (ESI, pos.ion) m/z: 446.1 $[M+Na]^+$.

**Step 4: Synthesis of (2*S*,4*S*)-4-((2,2-difluorobenzo[*d*][1,3]dioxol-5-yl) oxy)-2-(difluoromethoxy)methyl)pyrrolidine**

To a solution of *tert*-butyl (2*S*,4*S*)-4-((2,2-difluorobenzo[*d*][1,3]dioxol-5-yl) oxy)-2-((difluoromethoxy)methyl)pyrrolidine-1-carboxylate (2.20 g, 5.20 mmol) in DCM (10 mL) was added a solution of HCl in 1,4-dioxane (3.0 mL, 4 mol/L). The mixture was stirred at room temperature for 13 h. The reaction solution was concentrated under reduced pressure, and the residue was diluted with DCM (40 mL), washed successively with saturated $NaHCO_3$ solution (20 mL) and

saturated NaCl solution (20 mL), dried over anhydrous $Na_2SO_4$, and concentrated under reduced pressure to give yellow liquid (1.52 g, 90%).

MS (ESI, pos.ion) m/z: 324.1 $[M+H]^+$.

**Step 5: Synthesis of methyl 4-((2*S*,4*S*)-4-((2,2-difluorobenzo[*d*][1,3]dioxol-5-yl)oxy) -2-((difluoromethoxy)methyl)pyrrolidin-1-yl)benzoate**

Under nitrogen protection, (2*S*,4*S*)-4-((2,2-difluorobenzo[*d*][1,3]dioxol -5-yl)oxy)-2-(difluoromethoxy)methyl)pyrrolidine (1.50 g, 4.64 mmol), $Pd_2(dba)_3$ (425 mg, 0.46 mmol), XantPhos (403 mg, 0.70 mmol), $Cs_2CO_3$ (2.27 g, 6.97 mmol), methyl 4-iodobenzoate (1.58 g, 6.03 mmol) were successively added to 1,4-dioxane (20 mL) and the mixture was heated in a 100°C oil bath for 23 h. The reaction solution was cooled to room temperature, then concentrated under reduced pressure. The residue was diluted with DCM (60 mL), washed successively with $NaHCO_3$ solution (20 mL) and saturated NaCl solution (20 mL), dried over anhydrous $Na_2SO_4$, and concentrated under reduced pressure. The crude product was separated by silica gel column chromatography (eluent: PE/EtOAc (v/v) = 4/1) to give yellow liquid (1.62 g, 76%).

MS (ESI, pos.ion) m/z: 458.1$[M+H]^+$.

**Step 6: Synthesis of 4-((2*S*,4*S*)-4-((2,2-difluorobenzo[*d*][1,3]dioxol-5-yl)oxy) -2-((difluoromethoxy)methyl)pyrrolidin-1-yl)benzoic acid**

To a solution of methyl 4-((2*S*,4*S*)-4-((2,2-difluorobenzo[*d*][1,3]dioxol -5-yl)oxy)-2-((difluoromethoxy)methyl)pyrrolidin-1-yl)benzoate (1.60 g, 3.50 mmol) in MeOH (8 mL) and THF (8 mL) was added $LiOH \cdot H_2O$ (3.00 g, 71.50 mmol) in $H_2O$ (8 mL). The mixture was reacted at room temperature for 15 h. The reaction solution was concentrated under reduced pressure, and the remaining solution was added with HCl solution (3 mol/L) to adjust the pH of the solution to about 4. The resulting mixture was extracted with EtOAc (20 mL×2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 5/1) to give a pale yellow solid (1.42 g, 91%).

MS (ESI, pos.ion) m/z: 444.1 $[M+H]^+$.

**Step 7: Synthesis of 4-((2*S*,4*S*)-4-((2,2-difluorobenzo[*d*][1,3]dioxol-5-yl)oxy)-2-((difluoromethoxy)methyl)pyrrolidin-1-yl)-*N*-((*R*)-1-(4-(ethylsulfonyl)phenyl)-2-hydroxyethyl) benzamide**

EDCI (86 mg, 0.45 mmol), HOBT (60 mg, 0.44 mmol), (*R*)-2-amino-2-(4-(ethylsulfonyl)phenyl)ethanol (75 mg, 0.33 mmol), 4-((2*S*,4*S*)-4-((2,2-difluorobenzo[*d*][1,3]dioxol-5-yl)oxy)-2-((difluoromethoxy)methyl)pyrrolidin-1-yl)benzoic acid (130 mg, 0.29 mmol) and TEA (63 mg, 0.62 mmol) were successively added to DCM (6 mL), and the mixture was reacted at room temperature for 24 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 3/2) to give a pale yellow solid (135 mg, 70%).

MS (ESI, pos.ion) m/z: 655.1 $[M+H]^+$;

$^1H$ NMR (400 MHz, $CDCl_3$) $\delta$ (ppm): 7.87 - 7.80 (m, 2H), 7.75 (d, *J*= 8.0 Hz, 2H), 7.54 (d, *J* = 6.9 Hz, 2H), 7.06 (d, *J*= 6.7 Hz, 1H), 6.98 (d, *J*= 8.7 Hz, 1H), 6.69 (d, *J*= 2.4 Hz, 1H), 6.63 (d, *J* = 7.7 Hz, 2H), 6.57 (dd, *J* = 8.7, 2.4 Hz, 1H), 6.23 (t, *J* = 74.3 Hz, 1H), 5.25 (s, 1H), 5.03 (t, *J* = 4.6 Hz, 1H), 4.23 - 4.13 (m, 2H), 4053 - 3.90 (m, 3H), 3.73 (d, *J* = 11.4 Hz, 1H), 3.64 (dd, *J* = 11.3, 4.5 Hz, 1H), 3.07 (q, *J* = 7.3 Hz, 2H), 2.49 (d, *J* = 14.4 Hz, 1H), 2.37 - 2.30 (m, 1H), 1.26 (t, *J* = 7.3 Hz, 3H).

**Example 83 4-((2*S*,4*S*)-2-((difluoromethoxy)methyl)-4-((6-trifluoromethyl)pyridin -3-yl)oxy)pyrrolidin-1-yl)-*N*-((*R*)-1-(4-(ethylsulfonyl)phenyl)-2-hydroxyethyl)benzamide**

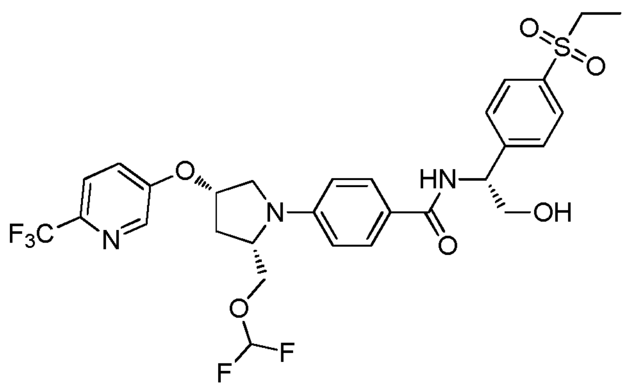

**Step 1: Synthesis of (2*S*,4*S*)-1-*tert*-butyl 2-methyl 4-((6-(trifluoromethyl)pyridin-3-yl)oxy)pyrrolidine-1,2-dicarboxylate**

Under nitrogen protection, to a solution of (2*S*,4*R*)-1-*tert*-butyl 2-methyl 4-hydroxypyrrolidine-1,2-dicarboxylate (3.10 g, 11.63 mmol) in THF (35 mL) were added 6-(trifluoromethyl)pyridin-3-ol (1.91 g, 11.72 mmol) and $PPh_3$ (3.4 g, 12.81 mmol). The mixture was cooled to 0°C, and DIAD (3.10 mL, 15.10 mmol) was slowly added dropwise for 30 min. After the addition was completed, the mixture was transferred to room temperature and stirred for 13 h. The reaction solution was concentrated under reduced pressure, and diluted with methyl tert-butyl ether (80 mL). The mixture was cooled to -15°C, and a white solid was precipitated. The resulting mixture was filtered while cold, and the filtrate was concentrated under reduced pressure. The residue was diluted with DCM (80 mL), washed with $NaHCO_3$ solution (100 mL), dried over anhydrous $Na_2SO_4$, and concentrated under reduced pressure. The crude product was separated by silica gel column chromatography (eluent: PE/EtOAc (v/v) = 4/1) to give colorless transparent liquid (4.20 g, 90%).
MS (ESI, pos.ion) m/z: 335.1 $[M-56+H]^+$.

**Step 2: Synthesis of *tert-butyl* (2*S*,4*S*)-2-(hydroxymethyl)-4-((6-(trifluoromethyl) pyridin-3-yl)oxy)pyrrolidin-1-carboxylate**

Under nitrogen protection, (2*S*,4*S*)-1-*tert*-butyl 2-methyl 4-((6-(trifluoromethyl)pyridin-3-yl)oxy)pyrrolidin-1,2-dicarboxylate (4.20 g, 10.80 mmol) was dissolved in THF (100 mL), then the mixture was transferred to -10 °C, and $LiBH_4$ (400 mg, 18.0 mmol) was added in portions. After the addition was completed, the mixture was stirred at room temperature for 8 h. Saturated $NH_4Cl$ (50 mL) solution was added to the reaction solution to quench the reaction. The mixture was extracted with EtOAc (20 mL × 3). The combined organic phases were washed with saturated NaCl solution (40 mL), dried over anhydrous $Na_2SO_4$, and concentrated under reduced pressure. The crude product was separated by silica gel column chromatography (eluent: PE/EtOAc (v/v) = 1/1) to give colorless transparent liquid (3.21 g, 82%).

**Step 3: Synthesis of *tert*-butyl (2*S*,4*S*)-2-((difluoromethoxy)methyl)-4-((6-(trifluoromethyl)pyridin-3-yl)oxy)pyrrolidine-1-carboxylate**

To a solution of tert-butyl (2*S*,4*S*)-2-(hydroxymethyl)-4-((6-(trifluoromethyl) pyridin-3-yl)oxy)pyrrolidin-1-carboxylate (3.20 g, 8.83 mmol) in DCM (3 mL) was added $H_2O$ (3 mL). After the mixture was stirred for 5 minutes, KOAc (10.80 g, 109.0 mmol) and $TMSCF_2Br$ (10.50 mL, 67.52 mmol) were added, and the mixture was stirred at room temperature for 16 h. $NaHCO_3$ solution (20 mL) was added to the reaction solution, and the resulting mixture was extracted with DCM (20 mL×2). The organic phases were combined, dried over anhydrous $Na_2SO_4$, and concentrated under reduced pressure. The crude product was separated by silica gel column chromatography (eluent: PE/EtOAc (v/v) = 5/1) to give colorless transparent liquid (2.10 g, 58%).
MS (ESI, pos.ion) m/z: 413.1 $[M+H]^+$.

**Step 4: Synthesis of (2*S*,4*S*)-2-((difluoromethoxy)methyl)-4-((6-(trifluoromethyl)pyridin-3-yl)oxy)pyrrolidine**

To a solution of *tert-Butyl* (2*S*,4*S*)-2-((difluoromethoxy)methyl)-4-((6-(trifluoromethyl)pyridin-3-yl)oxy)pyrrolidine-1-carboxylate (2.10 g, 5.10 mmol) in MeOH (10 mL) was slowly dropwise added a solution of HCl in methanol (25 mL, 20%). The mixture was reacted at room temperature for 5 h. The reaction solution was concentrated under reduced pressure. The residue was added with saturated $NaHCO_3$ solution (50 mL), and extracted with DCM (20 mL × 2). The organic phases were combined and dried over anhydrous $Na_2SO_4$, concentrated under reduced pressure to give colorless

liquid (1.50 g, 94%).

**Step 5: Synthesis of methyl 4-((2*S*,4*S*)-2-((difluoromethoxy)methyl) -4-((6-(trifluoromethyl)pyridin-3-yl)oxy)pyrrolidin-1-yl)benzoate**

Under nitrogen protection, to a solution of methyl 4-iodobenzoate (1.28 g, 4.88 mmol) and (2*S*,4*S*)-2-((difluoromethoxy)methyl)-4-((6-(trifluoromethyl)pyridin-3-yl)oxy)pyrrolidine (900 mg, 2.90 mmol) in toluene (20 mL) were added $Cs_2CO_3$ (1.50 g, 4.60 mmol), Xantphos (100 mg, 0.17 mmol) and $Pd_2(dba)_3$ (150 mg, 0.16 mmol). The mixture was heated to 95°C and stirred for 10 h. The reaction solution was cooled to room temperature and filtered. The filtrate was concentrated, and the mixed solution was diluted with $NaHCO_3$ solution (30 mL) and DCM (30 mL). The aqueous phase was extracted with DCM (20 mL). The organic phases were combined, dried over anhydrous $Na_2SO_4$, and concentrated under reduced pressure. The crude product was separated by silica gel column chromatography (eluent: PE/EtOAc (v/v) = 4/1) to give a yellow solid (200 mg, 16%).
MS (ESI, pos.ion) m/z: 447.1 $[M+H]^+$.

**Step 6: Synthesis of 4-((2*S*,4*S*)-2-((difluoromethoxy)methyl)-4-((6-(trifluoromethyl)pyridin-3-yl)oxy)pyrrolidin-1-yl)benzoic acid**

To a solution of methyl 4-((2*S*,4*S*)-2-((difluoromethoxy)methyl)-4-((6-(trifluoromethyl)pyridin-3-yl)oxy)pyrrolidin-1-yl)benzoate (200 mg, 0.45 mmol) in MeOH (5 mL) and $H_2O$ (3 mL) was added $LiOH{\cdot}H_2O$ (150 mg, 3.50 mmol). The mixture was reacted at room temperature for 8 h. The reaction solution was concentrated under reduced pressure, and the remaining solution was added with HCl solution (5 mL, 1 mol/L) to adjust the pH of the solution to about 5. The resulting mixture was extracted with DCM (20 mL×3). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 10/1) to give a white solid (190 mg, 98%).
MS (ESI, pos.ion) m/z: 433.1 $[M+H]^+$.

**Step 7: Synthesis of 4-((2*S*,4*S*)-2-((difluoromethoxy)methyl)-4-(6-trifluoromethyl)pyridin-3-yl)oxy)pyrrolidin-1-yl)-*N*-((*R*)-1-(4-(ethylsulfonyl)phenyl)-2-hydrox yethyl)benzamide**

4-((2*S*,4*S*)-2-((Difluoromethoxy)methyl)-4-((6-(trifluoromethyl)pyridin-3-yl)oxy)pyrr olidin-1-yl)benzoic acid (90 mg, 0.21 mmol), (2*R*)-2-amino-2-(4-(ethylsulfonyl)phenyl)ethanol (70 mg, 0.31 mmol), HATU (100 mg, 0.26 mmol) and DIPEA (0.1 mL, 0.56 mmol) were dissolved in DCM (20 mL). The mixture was reacted at room temperature for 15 h. The reaction solution was diluted with DCM (20 mL), washed successively with HCl solution (15 mL, 1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a pale yellow solid (94 mg, 70%).
MS (ESI, pos.ion) m/z: 644.3 $[M+H]^+$;
$^1H$ NMR (400 MHz, $CDCl_3$) δ (ppm): 8.38 (d, *J* = 2.4 Hz, 1H), 7.83 (d, *J* = 8.2 Hz, 2H), 7.76 (d, *J* = 8.7 Hz, 2H), 7.66 (d, *J* = 8.7 Hz, 1H), 7.54 (d, *J* = 8.2 Hz, 2H), 7.33 (dd, *J* = 8.7, 2.3 Hz, 1H), 7.05 (d, *J* = 6.9 Hz, 1H), 6.64 (d, *J* = 8.7 Hz, 2H), 6.22 (t, *J* = 74.1 Hz, 1H), 5.29 - 5.18 (m, 2H), 4.28 - 4.13 (m, 2H), 4.06 - 3.88 (m, 3H), 3.80 - 3.71 (m, 2H), 3.07 (q, *J*= 7.4 Hz, 2H), 2.57 - 2.38 (m, 2H), 1.26 (t, *J* = 7.4 Hz, 3H).

**Example 84 5-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)picolinamide**

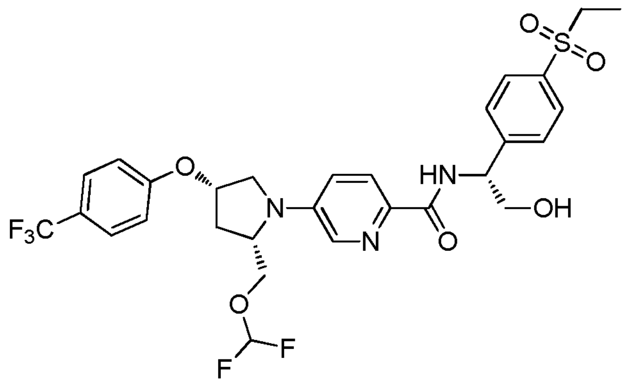

**Step 1: Synthesis of methyl 5-((2*S*,4*S*)-2-((difluoromethoxy)methyl)-4-(4-(trifluoromethyl)phenoxy)pyrrolidin-1-yl)picolinate**

Under nitrogen protection, (2*S*,4*S*)-2-((difluoromethoxy)methyl) -4-(4-(trifluoromethyl)phenoxy)pyrrolidine (500 mg, 1.61 mmol), $Pd_2(dba)_3$ (151 mg, 0.16 mmol), XantPhos (140 mg, 0.24 mmol), $Cs_2CO_3$ (793 mg, 2.43 mmol) and methyl 5-bromopicolinate (451 mg, 2.08 mmol) were dissolved in 1,4-dioxane (12 mL) in turn and the mixture was reacted in a 100°C oil bath for 18 h. The reaction solution was cooled to room temperature, then concentrated under reduced pressure. The residue was diluted with DCM (60 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give yellow liquid (400 mg, 56%).

MS (ESI, pos.ion) m/z: 447.2 $[M+H]^+$.

**Step 2: Synthesis of 2-((2*S*,4*S*)-2-((difluoromethoxy)methyl)-4-(4-(trifluoromethyl) phenoxy)pyrrolidin-1-yl)picolinic acid**

To a solution of methyl 5-((2*S*,4*S*)-2-((difluoromethoxy)methyl)-4-(4-(trifluoromethyl)phenoxy)pyrrolidin-1-yl)picolinate (400 mg, 0.90 mmol) in MeOH (4 mL) and THF (4 mL) was added a solution of $LiOH{\cdot}H_2O$ (752 mg, 17.92 mmol) in $H_2O$ (4 mL). The mixture was stirred at room temperature for 12 h. The reaction solution was added with HCl solution (2 mol/L) to adjust the pH of the solution to about 5. The resulting mixture was extracted with EtOAc (25 mL × 2), and the organic phases were combined, washed with saturated NaCl (20 mL) solution, dried over anhydrous $Na_2SO_4$, and concentrated under reduced pressure. The crude product was separated by silica gel column chromatography (eluent: DCM/MeOH (v/v) = 10/1) to give yellow liquid (350 mg, 90%).

MS (ESI, pos.ion) m/z: 433.1 $[M+H]^+$.

**Step 3: Synthesis of 5-((2*S*,4*S*)-2-((difluoromethoxy)methyl)-4-(4-(trifluoromethyl) phenoxy)pyrrolidin-1-yl)-*N*-((*R*)-1-(4-(ethylsulfonyl)phenyl)-2-hydroxyethyl)pyridineamide**

EDCI (66 mg, 0.34 mmol), HOBT (46 mg, 0.34 mmol), (*R*)-2-amino-2-(4-(ethylsulfonyl)phenyl)ethanol (63 mg, 0.27 mmol), 5-((2*S*,4*S*)-2-((difluoromethoxy)methyl)-4-(4-(trifluoromethyl)phenoxy)pyrrolidin-1-yl)picolinic acid (100 mg, 0.23 mmol) and TEA (46 mg, 0.45 mmol) were successively added to DCM (4 mL) and the mixture was stirred at room temperature for 20 h. The reaction solution was diluted with DCM (30 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a pale yellow solid (65 mg, 44%).

MS (ESI, pos.ion) m/z: 644.2 $[M+H]^+$;

$^1H$ NMR (400 MHz, $CDCl_3$) δ (ppm): 8.54 (d, *J* = 7.6 Hz, 1H), 8.01 (d, *J* = 8.8 Hz, 1H), 7.98 (d, *J* = 2.6 Hz, 1H), 7.86 (d, *J* = 8.3 Hz, 2H), 7.59 (dd, *J* = 8.2, 5.0 Hz, 4H), 7.00 (d, *J* = 2.8 Hz, 1H), 6.97 (d, *J*= 8.6 Hz, 2H), 6.22 (t, *J* = 74.0 Hz, 1H), 5.28 (dd, *J* = 11.9, 4.9 Hz, 1H), 5.19 (t, *J* = 4.6 Hz, 1H), 4.28 - 4.22 (m, 1H), 4.17 (dd, *J* = 10.1, 4.9 Hz, 1H), 4.06 - 3.95 (m, 3H), 3.79 (d, *J* = 11.4 Hz, 1H), 3.71 (dd, *J*= 11.4, 4.7 Hz, 1H), 3.08 (q, *J*= 7.4 Hz, 2H), 2.51 (d, *J*= 14.5 Hz, 1H), 2.44 - 2.36 (m, 1H), 1.26 (t, *J* = 7.4 Hz, 4H).

**Example 85 4-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)-3-fluorobenzamide**

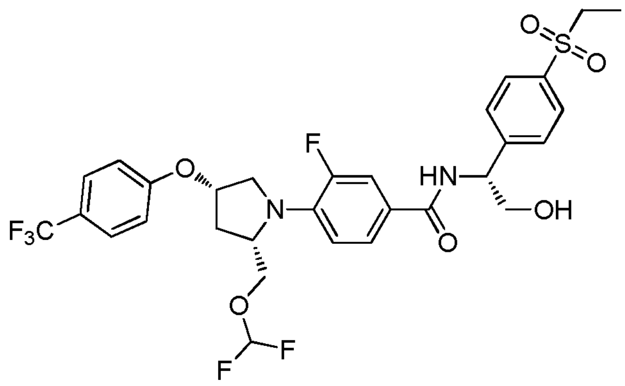

**Step 1: Synthesis of *tert-butyl* (2*S*,4*S*)-2-((difluoromethoxy)methyl)-4-(4-(trifluoromethyl) phenoxy)pyrrolidine-1-carboxylate**

To a solution of tert-butyl (2*S*,4*S*)-2-(hydroxymethyl)-4-(4-(trifluoromethyl) phenoxy)pyrrolidin-1-carboxylate (1.75 g, 4.84 mmol) in DCM (3 mL) was dropwise added $H_2O$ (3 mL). After the mixture was stirred for 5 minutes, KOAc (4.60 g, 46.40 mmol) and $TMSCF_2Br$ (5.9 mL, 38 mmol) were added, and the mixture was continued to stir at room temperature for 19 h. To the reaction solution was added $H_2O$ (45 mL) and the resulting mixture was extracted with DCM (25 mL$\times$2). The combined organic phases were washed with saturated NaCl solution (35 mL), dried over anhydrous $Na_2SO_4$, and concentrated under reduced pressure. The crude product was separated by silica gel column chromatography (eluent: PE/EtOAc (v/v) = 5/1) to give colorless transparent oil (1.65 g, 83%).

MS (ESI, pos.ion) m/z: 434.1 $[M+Na]^+$.

**Step 2: Synthesis of (2*S*,4*S*)-2-((difluoromethoxy)methyl)-4-(4-(trifluoromethyl) phenoxy)pyrrolidine**

To a solution of *tert-Butyl* (2*S*,4*S*)-2-((difluoromethoxy)methyl)-4-(4-(trifluoromethyl)phenoxy)pyrrolidine-1-carboxylate (1.65 g, 4.01 mmol) in MeOH (10 mL) was dropwise added a solution of HCl in methanol (25 mL, 20%). The mixture was stirred at room temperature for 6 h. The reaction solution was concentrated under reduced pressure. The resulting mixture was added with $NaHCO_3$ solution (50 mL), and extracted with DCM (20 mL $\times$ 2). The organic phase was dried over anhydrous $Na_2SO_4$, concentrated under reduced pressure to give a pale yellow solid (1.20 g, 96%).

**Step 3: Synthesis of methyl 4-((2*S*,4*S*)-2-((difluoromethoxy)methyl)-4-(4-(trifluoromethyl)phenoxy)pyrrolidin-1-yl)-3-fluorobenzoate**

Under nitrogen protection, to a solution of (2*S*,4*S*)-2-((difluoromethoxy)methyl) -4-((4-(trifluoromethyl)phenoxy)pyrrolidine (200 mg, 0.64 mmol) in toluene (10 mL) were added $Cs_2CO_3$ (500 mg, 1.50 mmol), XantPhos (50 mg, 0.08 mmol) and $Pd_2(dba)_3$ (90 mg, 0.09 mmol). The mixture was heated to 100°C and stirred for 26 h. The reaction solution was cooled to room temperature and filtered. The filtrate was concentrated, and the mixed solution was diluted with $NaHCO_3$ solution (30 mL) and DCM (30 mL). The aqueous phase was extracted with DCM (20 mL). The organic phases were combined, dried over anhydrous $Na_2SO_4$, and concentrated under reduced pressure. The crude product was separated by silica gel column chromatography (eluent: PE/EtOAc (v/v) = 4/1) to give pale yellow liquid (190 mg, 64%).

MS (ESI, pos.ion) m/z: 464.2 $[M+H]^+$.

**Step 4: Synthesis of 4-((2*S*,4*S*)-2-((difluoromethoxy)methyl)-4-(4-(trifluoromethyl) phenoxy)pyrrolidin-1-yl)-3-fluorobenzoic acid**

To a solution of methyl 4-((2*S*,4*S*)-2-((difluoromethoxy)methyl)-4-(4-(trifluoromethyl)phenoxy)pyrrolidin-1-yl)-3-fluorobenzoate (190 mg, 0.41 mmol) in MeOH (15 mL) was added a solution of $LiOH{\cdot}H_2O$ (180 mg, 4.20 mmol) in $H_2O$ (3 mL). The mixture was reacted at room temperature for 15 h. The reaction solution was added with HCl solution (15 mL, 1 mol/L) to adjust the pH of the solution to about 5. The resulting mixture was concentrated under reduced

pressure and extracted with DCM (20 mL×3). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 1/1) to give pale yellow liquid (160 mg, 87%).

MS (ESI, pos.ion) m/z: 450.1 $[M+H]^+$.

**Step 5: Synthesis of 4-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-3-fluorobenzamide**

(*R*)-2-Amino-2-(4-(ethylsulfonyl)phenyl)ethanol (100 mg, 0.44 mmol), 4-((2*S*,4*S*)-2-((difluoromethoxy)methyl)-4-(4-(trifluoromethyl)phenoxy)pyrrolidin-1-yl)-3-fluorobe nzoic acid (160 mg, 0.36 mmol), HATU (170 mg, 0.43 mmol) and DIPEA(0.1 mL, 0.56 mmol) were dissolved in DCM (20 mL). The mixture was stirred at room temperature for 25 h. The reaction solution was diluted with DCM (10 mL), and the resulting mixture was washed successively with HCl solution (20 mL, 0.1 mol/L), saturated $NaHCO_3$ solution (20 mL) and saturated NaCl solution (15 mL), dried over anhydrous $Na_2SO_4$, filtered and concentrated under reduced pressure. The crude product was separated by silica gel column chromatography (eluent: DCM/EtOAc (v/v) = 1/1) to give a white solid (60 mg, 26%).

MS (ESI, pos.ion) m/z: 661.1 $[M+H]^+$;

$^1H$ NMR (400 MHz, $CDCl_3$) δ (ppm): 7.83 (d, *J* = 8.2 Hz, 2H), 7.66 - 7.47 (m, 6H), 7.09 (d, *J* = 6.8 Hz, 1H), 6.97 (d, *J*= 8.5 Hz, 2H), 6.71 (t, *J*= 8.8 Hz, 1H), 6.17 (t, *J*= 74.4 Hz, 1H), 5.25 (d, *J* = 5.9 Hz, 1H), 5.10 (s, 1H), 4.47 (s, 1H), 4.10 - 3.82 (m, 6H), 3.08 (q, *J* = 7.4 Hz, 2H), 2.43 (s, 2H), 1.30 (t, *J* = 7.4 Hz, 3H).

**Example 86 4-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)-2-fluorobenzamide**

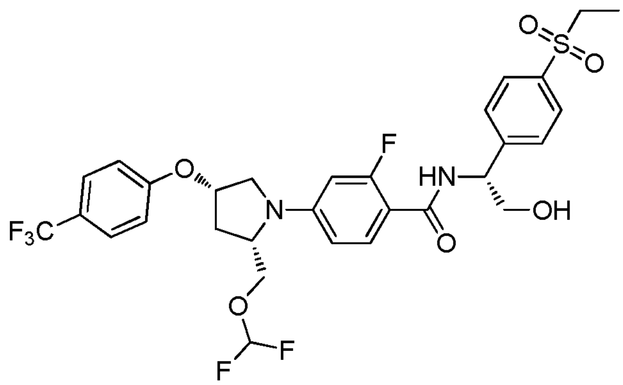

**Step 1: Synthesis of methyl 4-((2*S*,4*S*)-2-((difluoromethoxy)methyl)-4-(4-(trifluoromethyl) phenoxy)pyrrolidin-1-yl)-2-fluorobenzoate**

Under nitrogen protection, to a solution of methyl 2-fluoro-4-iodo-benzoate (410 mg, 1.46 mmol) and (2*S*,4*S*)-2-((difluoromethoxy)methyl)-4-((4-(trifluoromethyl)phenoxy)pyrrolidine (250 mg, 0.80 mmol) in toluene (10 mL) were added $Cs_2CO_3$ (500 mg, 1.53 mmol), XantPhos (50 mg, 0.08 mmol) and $Pd_2(dba)_3$ (90 mg, 0.09 mmol). The mixture was heated to 100°C and stirred for 18 h. The reaction solution was cooled to room temperature and filtered. The filtrate was concentrated, added with $NaHCO_3$ solution (30 mL), and extracted with DCM (30 mL). The organic phase was dried over anhydrous $Na_2SO_4$, and concentrated under reduced pressure. The crude product was separated by silica gel column chromatography (eluent: PE/EtOAc (v/v) = 4/1) to give yellow liquid (210 mg, 56%).

MS (ESI, pos.ion) m/z: 464.0 $[M+H]^+$.

**Step 4: Synthesis of 4-((2*S*,4*S*)-2-((difluoromethoxy)methyl)-4-(4-(trifluoromethyl) phenoxy)pyrrolidin-1-yl)-2-fluorobenzoic acid**

To a solution of methyl 4-((2*S*,4*S*)-2-((difluoromethoxy)methyl)-4-(4-(trifluoromethyl)phenoxy)pyrrolidin-1-yl)-2-fluorobenzoate (210 mg, 0.45 mmol) in MeOH (5 mL) was added a solution of $LiOH·H_2O$ (200 mg, 4.67 mmol) in $H_2O$ (2 mL). The mixture was reacted at room temperature for 10 h. The reaction solution was added with HCl solution (15 mL, 1 mol/L) to adjust the pH of the solution to about 5. The resulting mixture was concentrated under reduced pressure

and extracted with DCM (20 mL×3). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give pale yellow liquid (120 mg, 59%).

MS (ESI, pos.ion) m/z: 450.1 $[M+H]^+$.

**Step 5: Synthesis of 4-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-2-fluorobenzamide**

4-((2*S*,4*S*)-2-(Difluoromethoxymethyl)-4-(4-(trifluoromethyl)phenoxy)pyrrolidin-1-yl) -2-fluorobenzoic acid (160 mg, 0.36 mmol), (R)-2-amino-2-(4-(ethylsulfonyl)phenyl)ethanol (80 mg, 0.35 mmol), HATU (140 mg, 0.36 mmol) and DIPEA (0.1 mL, 0.56 mmol) were dissolved in DCM (20 mL). The mixture was reacted at room temperature for 25 h. The reaction solution was diluted with DCM (10 mL), and the resulting mixture was washed successively with HCl solution (20 mL, 0.1 mol/L), saturated $NaHCO_3$ solution (20 mL) and saturated NaCl solution (15 mL), dried over anhydrous $Na_2SO_4$ and concentrated under reduced pressure. The crude product was separated by silica gel column chromatography (eluent: DCM/EtOAc (v/v) = 1/1) to give a white solid (130 mg, 55%).

MS (ESI, pos.ion) m/z: 661.2 $[M+H]^+$;

$^1H$ NMR (400 MHz, $CDCl_3$) δ (ppm): 7.94 (t, *J* = 9.1 Hz, 1H), 7.88 (d, *J*= 8.3 Hz, 2H), 7.59 (d, *J* = 6.9 Hz, 4H), 7.49 (dd, *J* = 14.5, 7.0 Hz, 1H), 6.97 (d, *J* = 8.6 Hz, 2H), 6.50 (dd, *J* = 8.9, 2.0 Hz, 1H), 6.34 (d, *J* = 15.7 Hz, 1H), 6.23 (t, *J* = 74.4 Hz, 1H), 5.34 (d, *J* = 4.4 Hz, 1H), 5.17 (t, *J* = 4.3 Hz, 1H), 4.22 - 4.12 (m, 2H), 4.00 (dd, *J* = 30.3, 10.1 Hz, 3H), 3.79 - 3.66 (m, 2H), 3.09 (q, *J* = 7.4 Hz, 2H), 2.52 (d, *J* = 14.4 Hz, 1H), 2.44 - 2.36 (m, 1H), 1.28 (t, *J* = 7.4 Hz, 3H).

**Example 87 6-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)-5-fluoronicotinamide**

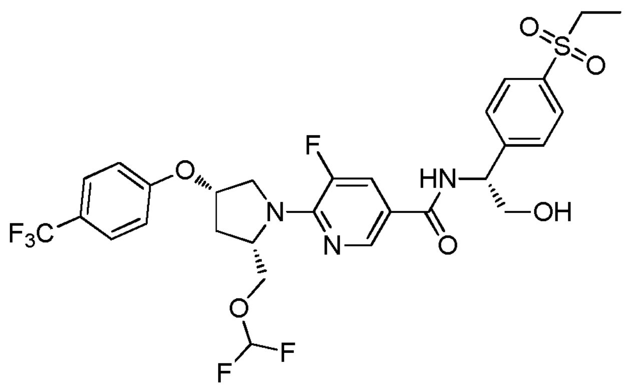

**Step 1: Synthesis of 6-((2*S*,4*S*)-2-((difluoromethoxy)methyl)-4-(4-(trifluoromethyl) phenoxy)pyrrolidin-1-yl)-5-fluoronicotinic acid**

Under nitrogen protection, to a solution of methyl 6-bromo-5-fluoronicotinic acid (70 mg, 0.32 mmol) and (2*S*,4*S*)-2-((difluoromethoxy)methyl)-4-((4-(trifluoromethyl)phenoxy) pyrrolidine (95 mg, 0.31 mmol) in toluene (10 mL) were added $Cs_2CO_3$ (150 mg, 0.46 mmol), XantPhos (30 mg, 0.05 mmol) and $Pd_2(dba)_3$ (50 mg, 0.05 mmol). The mixture was heated to 100°C and stirred for 15 h. The reaction solution was cooled to room temperature and filtered, the filtrate was concentrated under reduced pressure, and the crude product was separated by silica gel column chromatography (eluent: DCM/EtOAc (v/v) = 1/1) to give pale yellow liquid (100 mg, 73%).

MS (ESI, pos.ion) m/z: 451.1 $[M+H]^+$.

**Step 2: Synthesis of 6-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-5-fluoronicotinamide**

6-((2*S*,4*S*)-2-((Difluoromethoxy)methyl)-4-(4-(trifluoromethyl)phenoxy)pyrrolidin-1-y l)-5-fluoronicotinic acid (100 mg, 0.22 mmol), (*R*)-2-amino-2-(4-(ethylsulfonyl)phenyl)ethanol (70 mg, 0.31 mmol), HATU (120 mg, 0.31 mmol) and DIPEA (0.1 mL, 0.56 mmol) were dissolved in DCM (20 mL). The mixture was reacted at room temperature for 14 h. The reaction solution was diluted with DCM (20 mL), washed successively with HCl solution (15 mL,0.1 mol/L),

saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 15/1) to give a white solid(53 mg, 36%).

MS (ESI, pos.ion) m/z: 662.1 $[M+H]^+$;

$^1H$ NMR (400 MHz, $CDCl_3$) $\delta$ (ppm): 8.45 (s, 1H), 7.87 (d, *J* = 8.2 Hz, 2H), 7.69 (d, *J* = 13.7 Hz, 1H), 7.61 - 7.53 (m, 4H), 6.98 (t, *J* = 7.2 Hz, 3H), 6.20 (t, *J* = 74.6 Hz, 1H), 5.29 (d, *J* = 6.4 Hz, 1H), 5.11 (s, 1H), 4.78 (s, 1H), 4.27 (dd, *J* = 9.4, 4.2 Hz, 1H), 4.13 (s, 2H), 4.05 (d, *J* = 3.4 Hz, 1H), 3.99 (dd, *J* = 12.2, 6.5 Hz, 2H), 3.10 (q, *J* = 7.4 Hz, 2H), 2.51 (d, *J* = 14.3 Hz, 1H), 2.37 (dd, *J* = 12.7, 7.0 Hz, 1H), 1.28 (t, *J* = 7.5 Hz, 3H).

**Example 88 6-((2*S*,4*S*)-2-((difluoromethoxy)methyl)-4-(4-trifluoromethyl)phenoxy) pyrrolidin-1-yl)-*N*-((R)-1-(4-(ethylsulfonyl)phenyl)-2-hydroxyethyl)-2-methoxynicotinamide**

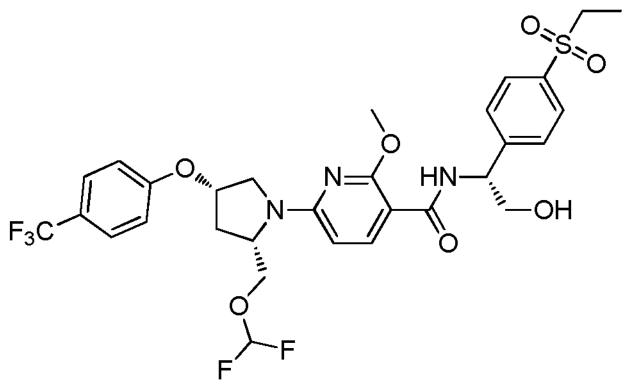

**Step 1: Synthesis of methyl 6-((2*S*,4*S*)-2-((difluoromethoxy)methyl)-4-(4-(trifluoromethyl)phenoxy)pyrrolidin-1-yl)-2-methoxynicotinate**

Under nitrogen protection, to a solution of methyl 6-bromo-2-methoxynicotinate (190 mg, 0.77 mmol) and (2*S*,4*S*)-2-((difluoromethoxy)methyl)-4-((4-(trifluoromethyl)phenoxy) pyrrolidine (150 mg, 0.48 mmol) in toluene (10 mL) were added $Cs_2CO_3$ (250 mg, 0.77 mmol), XantPhos (30 mg, 0.05 mmol) and $Pd_2(dba)_3$ (50 mg, 0.05 mmol). The mixture was heated to 100°C and stirred for 20 h. The reaction solution was cooled to room temperature and filtered. The filtrate was concentrated, added with $NaHCO_3$ solution (30 mL), and extracted with DCM (30 mL×2). The combined organic phases were dried over anhydrous $Na_2SO_4$, and concentrated under reduced pressure. The crude product was separated by silica gel column chromatography (eluent: PE/EtOAc (v/v) = 4/1) to give yellow liquid (130 mg, 57%).

MS (ESI, pos.ion) m/z: 477.0 $[M+H]^+$.

**Step 2: Synthesis of 6-((2*S*,4*S*)-2-((difluoromethoxy)methyl)-4-(4-(trifluoromethyl) phenoxy)pyrrolidin-1-yl)-2-methoxynicotinic acid**

To a solution of methyl 6-((2*S*,4*S*)-2-((difluoromethoxy)methyl)-4-(4-(trifluoromethyl)phenoxy)pyrrolidin-1-yl)-2-methoxynicotinate (130 mg, 0.27 mmol) in MeOH (5 mL) was added a solution of $LiOH{\cdot}H_2O$ (190 mg, 4.41 mmol) in $H_2O$ (1 mL). The mixture was reacted at room temperature for 18 h. The reaction solution was added with HCl solution (5 mL, 1 mol/L) to adjust the pH of the solution to about 5. The resulting mixture was concentrated under reduced pressure and extracted with DCM (20 mL×3). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give pale yellow liquid (110 mg, 87%).

MS (ESI, pos.ion) m/z: 463.3 $[M+H]^+$.

**Step 3: Synthesis of 6-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-2-methoxynicotinamide**

6-((2*S*,4*S*)-2-((Difluoromethoxy)methyl)-4-(4-(trifluoromethyl)phenoxy)pyrrolidin-1-y l)-2-methoxynicotinic acid (110 mg, 0.24 mmol), (*R*)-2-amino-2-(4-(ethylsulfonyl)phenyl)ethanol (70 mg, 0.31 mmol), HATU (130 mg, 0.33 mmol) and DIPEA (0.1 mL, 0.56 mmol) were dissolved in DCM (20 mL). The mixture was reacted at room temperature for 20

h. The reaction solution was diluted with DCM (20 mL), washed successively with HCl solution (15 mL, 0.1 mol/L), saturated $NaHCO_3$ solution (15 mL) and saturated NaCl solution (15 mL). The organic phase was dried over anhydrous $Na_2SO_4$, and concentrated under reduced pressure. The crude product was separated by silica gel column chromatography (eluent: DCM/EtOAc (v/v) = 1/1) to give a pale yellow solid (100 mg, 62%).

MS (ESI, pos.ion) m/z: 674.3 $[M+H]^+$;

$^1H$ NMR (400 MHz, $CDCl_3$) $\delta$ (ppm): 8.59 (d, *J* = 6.7 Hz, 1H), 8.25 (d, *J* = 8.4 Hz, 1H), 7.90 (t, *J* = 12.3 Hz, 2H), 7.65 - 7.51 (m, 4H), 6.97 (d, *J* = 8.6 Hz, 2H), 6.22 (t, *J* = 74.2 Hz, 1H), 6.08 (d, *J* = 8.4 Hz, 1H), 5.31 (d, *J* = 11.1 Hz, 1H), 5.17 (s, 1H), 4.58 (s, 1H), 4.50 (d, *J* = 8.9 Hz, 1H), 4.08 (s, 3H), 3.99 (d, *J* = 12.4 Hz, 2H), 3.91 (t, *J* = 9.6 Hz, 1H), 3.78 (d, *J* = 11.7 Hz, 2H), 3.09 (q, *J* = 7.4 Hz, 2H), 2.55 (d, *J* = 14.4 Hz, 1H), 2.41 - 2.32 (m, 1H), 1.28 (t, *J* = 7.4 Hz, 3H).

**Example 89 4-((2*S*,4*S*)-2-((difluoromethoxy)methyl)-4-(4-trifluoromethyl)phenoxy) pyrrolidin-1-yl)-*N*-((*R*)-1-(4-(ethylsulfonyl)phenyl)-2-hydroxyethyl)-3-methoxybenzamide**

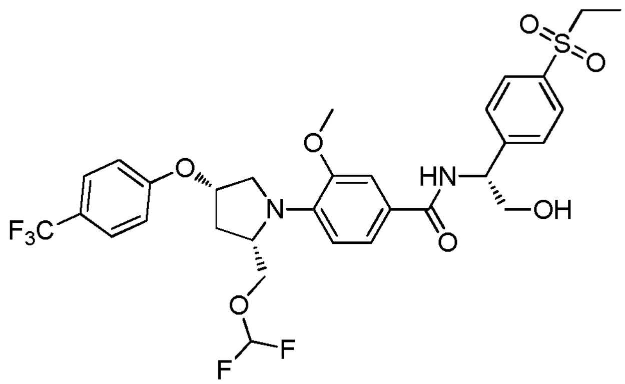

**Step 1: Synthesis of methyl 4-((2*S*,4*S*)-2-((difluoromethoxy)methyl)-4-(4-(trifluoromethyl)phenoxy)pyrrolidin-1-yl)-3-methoxybenzoate**

Under nitrogen protection, to a solution of methyl 4-iodo-3-methoxybenzoate (100 mg, 0.34 mmol) and (2*S*,4*S*)-2-((difluoromethoxy)methyl)-4-(4-(trifluoromethyl)phenoxy)pyrrolidine (95 mg, 0.31 mmol) in toluene (10 mL) were added $Cs_2CO_3$ (150 mg, 0.46 mmol), XantPhos (30 mg, 0.05 mmol) and $Pd_2(dba)_3$ (50 mg, 0.05 mmol). The mixture was heated to 105°C and stirred for 16 h. The reaction solution was cooled to room temperature and filtered. The filtrate was concentrated, added with $NaHCO_3$ solution (30 mL), and extracted with DCM (20 mL$\times$2). The combined organic phases were dried over anhydrous $Na_2SO_4$, and concentrated under reduced pressure. The crude product was separated by silica gel column chromatography (eluent: DCM/EtOAc (v/v) = 1/1) to give pale yellow liquid (120 mg, 83%).

MS (ESI, pos.ion) m/z: 476.2 $[M+H]^+$.

**Step 2: Synthesis of 4-((2*S*,4*S*)-2-((difluoromethoxy)methyl)-4-(4-(trifluoromethyl) phenoxy)pyrrolidin-1-yl)-3-methoxybenzoic acid**

To a solution of methyl 4-((2*S*,4*S*)-2-((difluoromethoxy)methyl)-4-(4-(trifluoromethyl)phenoxy)pyrrolidin-1-yl)-3-methoxybenzoate (120 mg, 0.25 mmol) in MeOH (5 mL) was added a solution of $LiOH \cdot H_2O$ (160 mg, 3.74 mmol) in $H_2O$ (1 mL). The mixture was reacted at room temperature for 13 h. The reaction solution was added with HCl solution (4 mL, 1 mol/L) to adjust the pH of the solution to about 5. The resulting mixture was concentrated under reduced pressure and extracted with DCM (15 mL$\times$2). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give pale yellow liquid (110 mg, 95%).

MS (ESI, pos.ion) m/z: 462.2 $[M+H]^+$.

**Step 3: Synthesis of 4-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-3-methoxybenzamide**

4-((2*S*,4*S*)-2-((Difluoromethoxy)methyl)-4-(4-(trifluoromethyl)phenoxy)pyrrolidin-1-y l)-3-methoxybenzoic acid (110 mg, 0.24 mmol), (*R*)-2-amino-2-(4-(ethylsulfonyl)phenyl)ethanol (71 mg, 0.31 mmol), HATU (120 mg, 0.31 mmol)

and DIPEA (0.1 mL, 0.56 mmol) were dissolved in DCM (20 mL). The mixture was reacted at room temperature for 17 h. The reaction solution was diluted with DCM (20 mL), washed successively with HCl solution (15 mL,0.1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 15/1) to give a pale yellow solid (103 mg, 64%).

MS (ESI, pos.ion) m/z: 673.2 $[M+H]^+$;

$^1H$ NMR (400 MHz, $CDCl_3$) δ (ppm): 7.89 (d, *J* = 8.2 Hz, 2H), 7.60 - 7.54 (m, 4H), 7.44 (s, 1H), 7.32 (d, *J* = 8.4 Hz, 1H), 6.98 (d, *J* = 8.3 Hz, 3H), 6.72 (d, *J* = 8.3 Hz, 1H), 6.12 (t, *J* = 74.6 Hz, 1H), 5.30 (d, *J* = 6.2 Hz, 1H), 5.06 (s, 1H), 4.71 - 4.62 (m, 1H), 4.12 - 3.96 (m, 3H), 3.87 (s, 3H), 3.83 - 3.76 (m, 1H), 3.71 (dd, *J* = 10.5, 6.0 Hz, 1H), 3.10 (q, *J* = 7.4 Hz, 2H), 2.56 (dt, *J* = 14.3, 7.3 Hz, 1H), 2.30 (d, *J* = 13.7 Hz, 1H), 2.20 (s, 1H), 1.29 (t, *J* = 7.4 Hz, 3H).

**Example 90 4-((2*S*,4*S*)-2-((difluoromethoxy)methyl)-4-(4-trifluoromethyl)phenoxy) pyrrolidin-1-yl)-*N*((*R*)-1-(4-(ethylsulfonyl)phenyl)-2-hydroxyethyl)-2-methoxybenzamide**

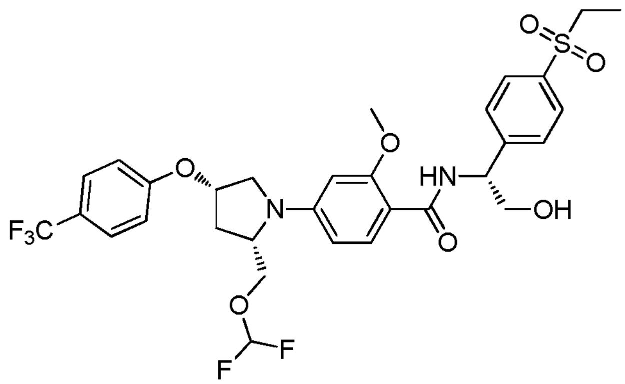

**Step 1: Synthesis of methyl 4-((2*S*,4*S*)-2-((difluoromethoxy)methyl)-4-(4-(trifluoromethyl)phenoxy)pyrrolidin-1-yl)-2-methoxybenzoate**

Under nitrogen protection, to a solution of methyl 4-bromo-2-methoxybenzoate (120 mg, 0.49 mmol) and (2*S*,4*S*)-2-((difluoromethoxy)methyl)-4-(4-(trifluoromethyl) phenoxy)pyrrolidine (150 mg, 0.48 mmol) in toluene (10 mL) were added $Cs_2CO_3$ (250 mg, 0.77 mmol), XantPhos (30 mg, 0.05 mmol) and $Pd_2(dba)_3$ (50 mg, 0.05 mmol). The mixture was heated to 105°C and stirred for 16 h. The reaction solution was cooled to room temperature and filtered. The filtrate was concentrated. The reaction solution was slowly added with $NaHCO_3$ solution (30 mL), and extracted with DCM (20 mL×2). The combined organic phases were dried over anhydrous $Na_2SO_4$, and concentrated under reduced pressure. The crude product was separated by silica gel column chromatography (eluent: DCM/EtOAc (v/v) = 1/1) to give pale yellow liquid (140 mg, 61%).

MS (ESI, pos.ion) m/z: 476.2 $[M+H]^+$.

**Step 2: Synthesis of 4-((2*S*,4*S*)-2-((difluoromethoxy)methyl)-4-(4-(trifluoromethyl) phenoxy)pyrrolidin-1-yl)-2-methoxybenzoic acid**

To a solution of methyl 4-((2*S*,4*S*)-2-((difluoromethoxy)methyl)-4-(4-(trifluoromethyl)phenoxy)pyrrolidin-1-yl)-2-methoxybenzoate (140 mg, 0.29 mmol) in MeOH (10 mL) was added a solution of $LiOH·H_2O$ (160 mg, 3.74 mmol) in $H_2O$ (2 mL). The mixture was reacted at room temperature for 10 h. The reaction solution was added with HCl solution (4 mL, 1 mol/L) to adjust the pH of the solution to about 5. The resulting mixture was concentrated under reduced pressure and extracted with DCM (20 mL×3). The organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 1/1) to give pale yellow liquid (130 mg, 96%).

MS (ESI, pos.ion) m/z: 462.1 $[M+H]^+$.

**Step 3: Synthesis of 4-((2*S*,4*S*)-2-((difluoromethoxy)methyl)-4-(4-(trifluoromethyl)phenoxy)pyrrolidin-1-yl)-*N*-((*R*)-1-(4-(ethylsulfonyl)phenyl)-2-hydroxyethyl)-2-methoxybenzamide**

4-((2*S*,4*S*)-2-((Difluoromethoxy)methyl)-4-(4-(trifluoromethyl)phenoxy)pyrrolidin-1-y l)-2-methoxybenzoic acid (130 mg, 0.28 mmol), (*R*)-2-amino-2-(4-(ethylsulfonyl)phenyl)ethanol (71 mg, 0.31 mmol), HATU (130 mg, 0.33 mmol) and DIPEA (0.1 mL, 0.56 mmol) were dissolved in DCM (20 mL). The mixture was reacted at room temperature for 12 h. The reaction solution was diluted with DCM (20 mL), washed successively with HCl solution (15 mL,0.1 mol/L), saturated $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 15/1) to give a pale yellow solid (103 mg, 54%).
MS (ESI, pos.ion) m/z: 673.2 $[M+H]^+$;
$^1H$ NMR (400 MHz, $CDCl_3$) $\delta$ (ppm): 8.65 (d, *J* = 6.8 Hz, 1H), 8.05 (d, *J* = 8.8 Hz, 1H), 7.88 (d, *J* = 8.3 Hz, 2H), 7.58 (dd, *J* = 8.1, 5.5 Hz, 4H), 6.97 (d, *J* = 8.6 Hz, 2H), 6.32 (dd, *J* = 8.8, 1.7 Hz, 1H), 6.24 (t, *J* = 74.6 Hz, 1H), 6.21 (s, 1H), 5.32 (dt, *J* = 9.1, 4.4 Hz, 1H), 5.17 (t, *J* = 4.3 Hz, 1H), 4.27 - 4.17 (m, 2H), 4.05 - 3.89 (m, 6H), 3.74 (dt, *J* = 11.3, 7.9 Hz, 2H), 3.09 (q, *J* = 7.4 Hz, 2H), 2.52 (d, *J* = 14.4 Hz, 1H), 2.44 - 2.36 (m, 1H), 1.28 (t, *J* = 7.4 Hz, 3H).

**Example 91 4-((2*S*,4*S*)-2-((difluoromethoxy)methyl)-4-(4-trifluoromethyl)phenoxy) pyrrolidin-1-yl)-*N*((*S*)-1-(4-(ethylsulfonyl)phenyl)-3-hydroxypropyl)benzamide**

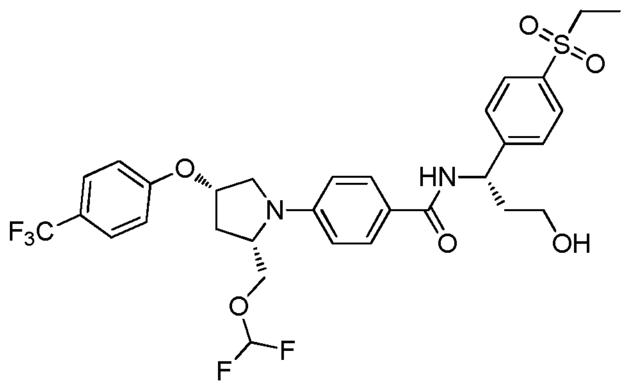

**Step 1: Synthesis of methyl 4-((2*S*,4*S*)-2-((difluoromethoxy)methyl)-4-(4-(trifluoromethyl)phenoxy)pyrrolidin-1-yl)benzoate**

To a solution of methyl 4-((2*S*,4*S*)-2-(hydroxymethyl)-4-(4-**(trifluoromethyl)phenoxy)pyrrolidin-1-yl)benzoate** (150 mg, 0.38 mmol) and KOAc (446 mg, 4.54 mmol) in DCM/$H_2O$ (1 mL/1 mL) was added $TMSCF_2Br$ (0.50 mL, 3.22 mmol). The mixture was reacted at room temperature for 11 h. The reaction solution was diluted with DCM (30 mL), washed with saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give yellow liquid (90 mg, 53%).
MS (ESI, pos.ion) m/z: 446.3 $[M+H]^+$.

**Step 2: Synthesis of 4-((2*S*,4*S*)-2-((difluoromethoxy)methyl)-4-(4-(trifluoromethyl) phenoxy)pyrrolidin-1-yl)benzoic acid**

To a solution of methyl 4-((2*S*,4*S*)-2-((difluoromethoxy)methyl)-4-(4-(trifluoromethyl)phenoxy)pyrrolidin-1-yl)benzoate (90 mg, 0.20 mmol) in MeOH (6 mL) was added a solution of $LiOH{\cdot}H_2O$ (88 mg, 2.10 mmol) in $H_2O$ (2 mL). The mixture was reacted at room temperature for 20 h. The reaction solution was added with HCl solution (1 mol/L) to adjust the pH of the solution to about 4. The resulting mixture was extracted with EtOAc (20 mL $\times$ 2), and the organic phases were combined, washed with saturated NaCl (15 mL) solution, dried over anhydrous $Na_2SO_4$, and concentrated under reduced pressure. The crude product was separated by silica gel column chromatography (eluent: DCM/EtOAc (v/v) = 4/1) to give a white solid (60 mg, 69%).
MS (ESI, pos.ion) m/z: 432.0 $[M+H]^+$.

**Step 3: Synthesis of 4-((2*S*,4*S*)-2-((difluoromethoxy)methyl)-4-(4-(trifluoromethyl) phenoxy)pyrrolidin-1-yl)-*N*-((*S*)-1-(4-(ethylsulfonyl)phenyl)-3-hydroxypropyl)benzamide**

(*S*)-3-Amino-3-(4-(ethylsulfonyl)phenyl)propan-1-ol (29 mg, 0.12 mmol), 4-((2*S*,4*S*)-2-((difluoromethoxy)methyl)-4-(4-trifluoromethyl)phenoxy)pyrrolidin-1-yl)benzoic acid (40 mg , 0.09 mmol), EDCI (26 mg, 0.14 mmol), HOBT (18 mg, 0.13 mmol) and TEA (28 mg, 0.28 mmol) were dissolved in DCM (3 mL), and the mixture was stirred at room temperature for 18 h. The reaction solution was concentrated under reduced pressure, and the crude product was separated by silica gel column chromatography (eluent: DCM/MeOH (v/v) = 20/1) to give a white solid (49 mg, 80%).

MS (ESI, pos.ion) m/z: 657.3 $[M+H]^+$.

$^1H$ NMR (400 MHz, $CDCl_3$) $\delta$ (ppm): 7.86 (d, *J* = 8.2 Hz, 2H), 7.77 (d, *J* = 8.6 Hz, 2H), 7.61 (d, *J* = 8.6 Hz, 2H), 7.56 (d, *J* = 8.2 Hz, 2H), 7.45 (d, *J* = 7.2 Hz, 1H), 6.99 (d, *J* = 8.5 Hz, 2H), 6.66 (d, *J* = 8.6 Hz, 2H), 6.24 (t, *J* = 74.3 Hz, 1H), 5.50 - 5.43 (m, 1H), 5.19 (s, 1H), 4.25 - 4.16 (m, 2H), 3.98 (t, *J*= 9.1 Hz, 1H), 3.82 - 3.65 (m, 4H), 3.11 (q, *J* = 7.4 Hz, 2H), 2.96 (s, 1H), 2.53 (d, *J* = 14.3 Hz, 1H), 2.46 - 2.36 (m, 1H), 2.31 - 2.21 (m, 1H), 2.01 - 1.91 (m, 1H), 1.29 (t, *J* = 7.4 Hz, 3H).

**Example 92 *N*-((*R*)-1-(4-(ethylsulfonyl)phenyl)-2-hydroxyethyl)-4-((2*S*,4*R*)-2-((trifluoromethoxy)methyl)-4-(4-(trifluoromethyl)phenoxy)pyrrolidin-1-yl)benzamide**

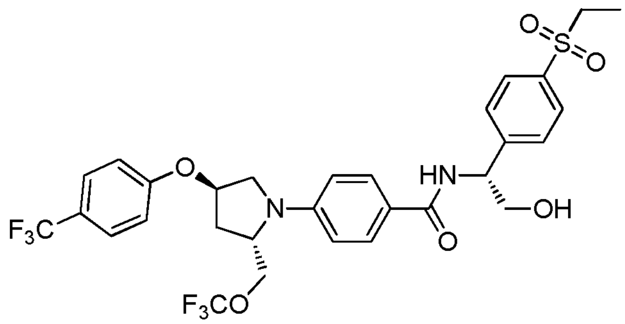

**Step 1: Synthesis of benzyl (2*S*,4*S*)-2-(hydroxymethyl)-4-(4-(trifluoromethyl)phenoxy) pyrrolidine-1-carboxylate**

To a solution of ((2*S*,4*S*)-4-(4-(trifluoromethyl)phenoxy)pyrrolidin-2-yl)methanol (500 mg, 1.91 mmol) and $NaHCO_3$ (643 mg, 7.65 mmol) in THF/$H_2O$ (8 mL/3 mL) was added CbzCl (0.35 mL, 2.49 mmol). The mixture was reacted at room temperature for 16 h. The reaction solution was diluted with saturated $NaHCO_3$ solution (15 mL) and EtOAc (20 mL). The resulting mixture was left standing for layers, the upper organic phase was separated, and the aqueous phase was extracted with EtOAc (20 mL $\times$ 2). The combined organic phases were washed with saturated NaCl solution (15 mL), dried over anhydrous $Na_2SO_4$, filtered, and concentrated under reduced pressure. The crude product was separated by silica gel column chromatography (eluent: PE/EtOAc (v/v) = 4/1) to give colorless liquid (700 mg, 93%).

MS (ESI, pos.ion) m/z = 396.2 $[M+H]^+$.

**Step 2: Synthesis of benzyl (2*S*,4*R*)-2-((trifluoromethoxy)methyl)-4-(4-(trifluoromethyl)phenoxy)pyrrolidine-1-carboxylate**

Under nitrogen protection, AgOTf (1.54 g, 5.99 mmol), selective fluorine reagent (1.07, 3.02 mmol), KF (464 mg, 7.99 mmol) and benzyl (2*S*,4*S*)-2-(hydroxymethyl)-4-(4-(trifluoromethyl)phenoxy)pyrrolidine-1-carboxylate (790 mg, 2.00 mmol) were successively added to EtOAc (6 mL), then 2-fluoropyridine (0.50 mL, 5.80 mmol) and $TMSCF_3$ (0.90 mL, 6.10 mmol) were added, and the mixture was reacted at room temperature for 48 h. The reaction solution was filtered and concentrated under reduced pressure, and the crude product was separated by silica gel column chromatography (eluent: PE/EtOAc (v/v) = 4/1) to give colorless liquid (330 mg, 35%).

MS (ESI, pos.ion) m/z: 464.2 $[M+H]^+$.

**Step 3: Synthesis of (2*S*,4*R*)-2-((trifluoromethoxy)methyl)-4-(4-(trifluoromethyl) phenoxy)pyrrolidine**

To a solution of benzyl (2*S*,4*R*)-2-((trifluoromethoxy)methyl)-4-(4-(trifluoromethyl)phenoxy)pyrrolidine-1-carboxylate (300 mg, 0.65 mmol) in MeOH (8.0 mL) was added Pd/C (30 mg, 10%) under hydrogen protection. The mixture was reacted at room temperature for 14 h. The reaction solution was concentrated under reduced pressure to give brown liquid (196 mg, 92%).

MS (ESI, pos.ion) m/z: 330.0 $[M+H]^+$.

### Step 4: Synthesis of methyl 4-((2*S*,4*R*)-2-((trifluoromethoxy)methyl)-4-(4-(trifluoromethyl)phenoxy)pyrrolidin-1-yl)benzoate

Under nitrogen protection, (2*S*,4*R*)-2-((difluoromethoxy)methyl)-4-(4-(trifluoromethyl)phenoxy)pyrrolidine (100 mg, 0.30 mmol), $Pd_2(dba)_3$ (27 mg, 0.03 mmol), XantPhos (26 mg, 0.04 mmol), $Cs_2CO_3$ (200 mg, 0.61 mmol) and methyl 4-iodobenzoate (120 mg, 0.46 mmol) were added to 1,4-dioxane (4 mL) in turn and the mixture was heated at 100°C and reacted for 20 h. The reaction solution was cooled to room temperature, then concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give yellow liquid (100 mg, 71%).

MS (ESI, pos.ion) m/z: 464.0 $[M+H]^+$.

### Step 5: Synthesis of 4-((2*S*,4*R*)-2-((trifluoromethoxy)methyl)-4-(4-(trifluoromethyl)phenoxy)pyrrolidin-1-yl)benzoic acid

To a solution of methyl 4-((2*S*,4*R*)-2-((trifluoromethoxy)methyl)-4-(4-(trifluoromethyl)phenoxy)pyrrolidin-1-yl)benzoate (100 mg, 0.22 mmol) in MeOH (2.0 mL) was added a solution of $LiOH{\cdot}H_2O$ (271 mg, 6.46 mmol) in $H_2O$ (1.0 mL). The mixture was reacted at room temperature for 18 h. To the reaction solution was added HCl solution (1 mol/L) to adjust the pH of the solution to about 4. The resulting mixture was extracted with EtOAc (20 mL $\times$ 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (37 mg, 38%).

MS (ESI, pos.ion) m/z: 450.2 $[M+H]^+$.

### Step 6: Synthesis of *N*-((*R*)-1-(4-(ethylsulfonyl)phenyl)-2-hydroxyethyl)-4-((2*S*,4*R*) -2-((trifluoromethoxy)methyl)-4-(4-(trifluoromethyl)phenoxy)pyrrolidin-1-yl)benzamide

EDCI (24 mg, 0.13 mmol), HOBT (17 mg, 0.13 mmol), (*R*)-2-amino-2-(4-(ethylsulfonyl)phenyl)ethanol (25 mg, 0.11 mmol), 4-((2*S*,4*R*)-2-((trifluoromethoxy)methyl)-4-(4-(trifluoromethyl)phenoxy)pyrrolidin-1-yl)benzoic acid (37 mg , 0.08 mmol) and TEA (25 mg, 0.25 mmol) were successively added to DCM (4 mL) and the mixture was reacted at room temperature for 18 h. The reaction solution was concentrated under reduced pressure, and the crude product was separated by silica gel column chromatography (eluent: DCM/MeOH (v/v) = 20/1) to give a white solid (5 mg, 82%).

MS (ESI, pos.ion) m/z: 661.0 $[M+H]^+$;

$^1H$ NMR (400 MHz, $CDCl_3$) $\delta$ (ppm): 7.83 (d, *J* = 8.2 Hz, 2H), 7.76 (d, *J* = 8.7 Hz, 2H), 7.56 (d, *J* = 8.3 Hz, 2H), 7.55 (d, *J* = 8.2 Hz, 2H), 7.02 (d, *J* = 6.6 Hz, 1H), 6.95 (d, *J* = 8.6 Hz, 2H), 6.62 (d, *J* = 8.7 Hz, 2H), 5.28 - 5.22 (m, 1H), 5.20 - 5.14 (m, 1H), 4.39 (s, 1H), 4.14 - 4.07 (m, 2H), 4.04 - 3.98 (m, 1H), 3.94 (dd, *J* = 10.7, 5.6 Hz, 2H), 3.56 (dd, *J* = 10.7, 3.3 Hz, 1H), 3.08 (q, *J* = 7.4 Hz, 2H), 2.71 - 2.60 (m, 1H), 2.53 (t, *J* = 5.9 Hz, 2H), 1.26 (t, *J* = 7.4 Hz, 3H).

### Example 93 *N*-((*S*)-1-(4-(ethylsulfonyl)phenyl)-3-hydroxypropyl)-4-((2*S*,4*S*)-2-((trifluoromethoxy)methyl)-4-(4-(trifluoromethyl)phenoxy)pyrrolidin-1-yl)benzamide

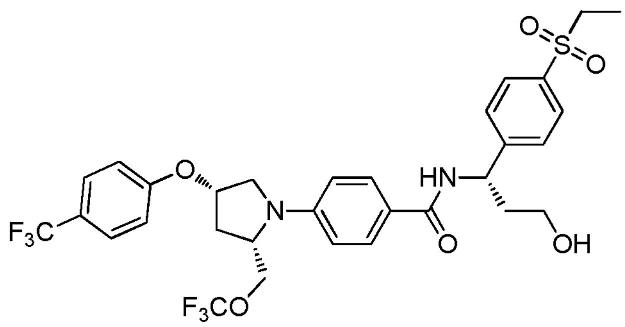

(*S*)-3-Amino-3-(4-(ethylsulfonyl)phenyl)propan-1-ol (40 mg, 0.16 mmol), 4-((2*S*,4*S*)-2-((trifluoromethoxy)methyl)-4-(4-trifluoromethyl)phenoxy)pyrrolidin-1-yl)benzoic acid (55 mg , 0.12 mmol), EDCI (35 mg, 0.18 mmol), HOBT (24 mg, 0.18 mmol) and TEA (38 mg, 0.38 mmol) were dissolved in DCM (4 mL), and the mixture was reacted at room temperature for 12 h. The reaction solution was diluted with DCM (40 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/MeOH (v/v) = 20/1) to give a white solid (70 mg, 85%).

MS (ESI, pos.ion) m/z: 675.1 $[M+H]^+$.

$^1H$ NMR (400 MHz, $CDCl_3$) δ (ppm): 7.85 (d, *J* = 8.3 Hz, 2H), 7.76 (d, *J* = 8.7 Hz, 2H), 7.59 (d, *J* = 8.6 Hz, 2H), 7.54 (d, *J* = 8.3 Hz, 2H), 7.41 (d, *J*= 7.3 Hz, 1H), 6.97 (d, *J* = 8.6 Hz, 2H), 6.63 (d, *J* = 8.8 Hz, 2H), 5.48 - 5.43 (m, 1H), 5.19 (t, *J* = 4.5 Hz, 1H), 4.29 - 4.19 (m, 2H), 4.14 - 4.18 (m, 1H), 3.82 - 3.74 (m, 2H), 3.73 - 3.63 (m, 2H), 3.09 (q, *J* = 7.4 Hz, 2H), 2.53 (d, *J* = 14.4 Hz, 1H), 2.46 - 2.37 (m, 1H), 2.29 - 2.19 (m, 1H), 1.99 - 1.90 (m, 1H), 1.28 (t, *J* = 7.4 Hz, 3H).

**Example 94 *N*-((*R*)-1-(4-(ethylsulfonyl)phenyl)-2-hydroxyethyl)-6-((2*S*,4*S*)-2-((trifluoromethoxy)methyl)-4-(4-(trifluoromethyl)phenoxy)pyrrolidin-1-yl)nicotinamide**

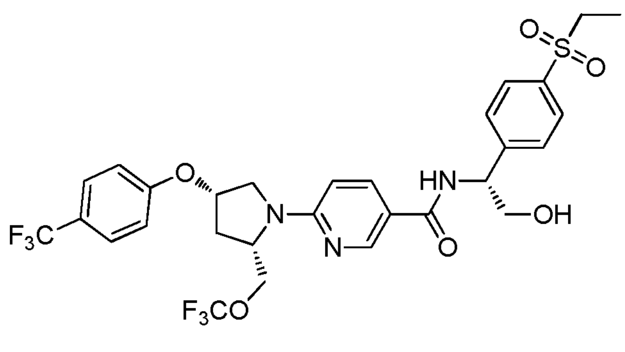

**Step 1: Synthesis of methyl 6-(2*S*,4*S*)-2-((trifluoromethoxy)methyl)-4-(4-(trifluoromethyl)phenoxy)pyrrolidin-1-yl)nicotinate**

Under nitrogen protection, AgOTf (1.75 g, 6.81 mmol), selective fluorine reagent (1.20, 3.39 mmol), KF (528 mg, 9.09 mmol) and methyl 6-(2*S*,4*S*)-2-(hydroxymethyl)-4-(4-(trifluoromethyl)phenoxy)pyrrolidine-1-yl)nicotinate (900 mg, 2.27 mmol) were successively added to EtOAc (10 mL), then 2-fluoropyridine (0.7 mL, 8.10 mmol) and $TMSCF_3$ (1.0 mL, 6.80 mmol) were added, and the mixture was reacted at room temperature for 27 h. The reaction solution was filtered and concentrated under reduced pressure, and the crude product was separated by silica gel column chromatography (eluent: PE/EtOAc (v/v) = 4/1) to give colorless liquid (430 mg, 41%).

MS (ESI, pos.ion) m/z: 465.1 $[M+H]^+$.

**Step 2: Synthesis of 6-((2*S*,4*S*)-2-((trifluoromethoxy)methyl)-4-(4-(trifluoromethyl)phenoxy)pyrrolidin-1-yl)nicotinic acid**

To a solution of methyl 6-(2*S*,4*S*)-2-((trifluoromethoxy)methyl)-4-(4-(trifluoromethyl)phenoxy)pyrrolidin-1-yl)nicotinate (400 mg, 0.86 mmol) in MeOH (8 mL) was added a solution of $LiOH{\cdot}H_2O$ (722 mg, 17.21 mmol) in $H_2O$ (2

mL). The mixture was reacted at room temperature for 17 h. To the reaction solution was added HCl solution (1 mol/L) to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (280 mg, 72%).

MS (ESI, pos.ion) m/z: 451.1 $[M+H]^+$.

**Step 3: Synthesis of 4-((2*S*,4*S*)-2-((trifluoromethoxy)methyl)-4-(4-(trifluoromethyl)phenoxy)pyrrolidin-1-yl)-*N*((*R*)-1-(4-(ethylsulfonyl)phenyl)-2-hydroxyethyl) benzamide**

(*R*)-2-Amino-2-(4-(ethylsulfonyl)phenyl)ethanol (66 mg, 0.29 mmol), 6-((2*S*,4*S*)-2-((trifluoromethoxy)methyl)-4-(4-(trifluoromethyl)phenoxy)pyrrolidin-1-yl)nicotinic acid (100 mg , 0.22 mmol), EDCI (63 mg, 0.33 mmol), HOBT (45 mg, 0.33 mmol) and TEA (70 mg, 0.69 mmol) were dissolved in DCM (6 mL), and the mixture was reacted at room temperature for 9 h. The reaction solution was concentrated under reduced pressure, and the crude product was separated by silica gel column chromatography (eluent: DCM/MeOH (v/v) = 20/1) to give a white solid (121 mg, 82%).

MS (ESI, pos.ion) m/z: 662.1 $[M+H]^+$;

$^1H$ NMR (400 MHz, $CD_3OD$) δ (ppm): 8.52 (d, *J* = 2.1 Hz, 1H), 7.82 (dd, *J* = 8.9, 2.4 Hz, 1H), 7.66 (d, *J* = 8.3 Hz, 2H), 7.43 (d, *J* = 8.3 Hz, 2H), 7.39 (d, *J* = 8.7 Hz, 2H), 6.81 (d, *J* = 8.6 Hz, 2H), 6.32 (d, *J* = 8.9 Hz, 1H), 5.04 (t, *J* = 4.4 Hz, 2H), 4.46 - 4.37 (m, 1H), 4.26 (dd, *J* = 9.0, 4.4 Hz, 1H), 3.92 (t, *J* = 9.2 Hz, 1H), 3.76 - 3.69 (m, 2H), 3.69 - 3.61 (m, 2H), 2.94 (q, *J* = 7.4 Hz, 2H), 2.34 (d, *J* = 14.3 Hz, 1H), 2.29 - 2.21 (m, 1H), 1.07 (t, *J* = 7.4 Hz, 3H).

**Example 95 *N*-((*R*)-1-(4-(ethylsulfonyl)phenyl)-2-hydroxyethyl)-2-((2*S*,4*R*)-2-((trifluoromethoxy)methyl)-4-(4-(trifluoromethyl)phenoxy)pyrrolidin-1-yl)pyrimidine-5-carbo xamide**

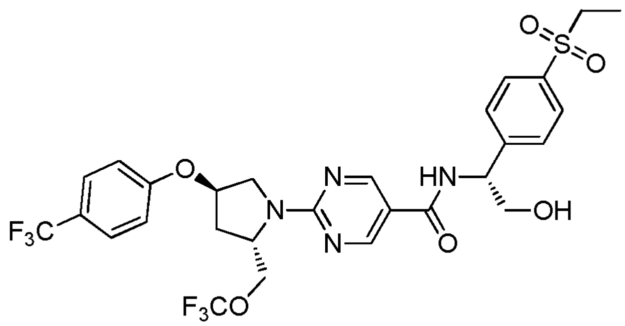

**Step 1: Synthesis of ethyl ((2*S*,4*R*)-2-((trifluoromethoxy)methyl)-4-(4-(trifluoromethyl)phenoxy)pyrrolidin-1-yl)pyrimidine-5-carboxylate**

(2*S*,4*R*)-2-((trifluoromethoxy)methyl)-4-(4-(trifluoromethyl)phenoxy)pyrrolidine (80 mg, 0.24 mmol), $K_2CO_3$ (201 mg, 1.45 mmol) and ethyl 2-chloropyrimidine-5-carboxylate (68 mg, 0.36 mmol) were added to acetone (4 mL) sequentially. The mixture was reacted at room temperature for 18 h. The reaction solution was filtered and concentrated under reduced pressure, and the crude product was separated by silica gel column chromatography (eluent: PE/EtOAc (v/v) = 4/1) to give pale yellow liquid (96 mg, 82%).

MS (ESI, pos.ion) m/z: 480.0 $[M+H]^+$.

**Step 2: Synthesis of 2-((2*S*,4*R*)-2-((trifluoromethoxy)methyl)-4-(4-(trifluoromethyl)phenoxy)pyrrolidin-1-yl)pyrimidine-5-carboxylic acid**

To a solution of ethyl ((2*S*,4*R*)-2-((trifluoromethoxy)methyl)-4-(4-(trifluoromethyl)phenoxy)pyrrolidin-1-yl)pyrimidine-5-carboxylate (96 mg, 0.20 mmol) in MeOH (4 mL) was added a solution of $LiOH{\cdot}H_2O$ (168 mg, 4.00 mmol) in $H_2O$ (1.0 mL). The mixture was reacted at room temperature for 23 h. To the reaction solution was added HCl solution (1 mol/L) to adjust the pH of the solution to about 4. The resulting mixture was extracted with EtOAc (20 mL × 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (52 mg, 57%).

MS (ESI, pos.ion) m/z: 452.0 $[M+H]^+$.

**Step 3: Synthesis of *N*-((*R*)-1-(4-(ethylsulfonyl)phenyl)-2-hydroxyethyl)-2-((2*S*,4*R*)-2-((trifluoromethoxy)methyl)-4-(4-(trifluoromethyl)phenoxy)pyrrolidin-1-yl)pyrimidi ne-5-carboxamide**

EDCI (33 mg, 0.17 mmol), HOBT (23 mg, 0.17 mmol), (*R*)-2-amino-2-(4-(ethylsulfonyl)phenyl)ethanol (34 mg, 0.15 mmol), 2-((2*S*,4*R*)-2-((trifluoromethoxy)methyl)-4-(4-(trifluoromethyl)phenoxy)pyrrolidin-1-yl)pyrimidine -5-carboxylic acid (22 mg, 0.05 mmol) and TEA (34 mg, 0.34 mmol) were successively added to DCM (4 mL) and the mixture was reacted at room temperature for 19 h. The reaction solution was concentrated under reduced pressure, and the crude product was separated by silica gel column chromatography (eluent: DCM/MeOH (v/v) = 20/1) to give a white solid (62 mg, 81%).

MS (ESI, pos.ion) m/z: 663.1 $[M+H]^+$.

$^1H$ NMR (400 MHz, $CD_3OD$) $\delta$ (ppm): 8.82 (s, 2H), 7.83 (d, $J$ = 8.3 Hz, 2H), 7.57 (d, $J$ = 8.3 Hz, 2H), 7.53 (d, $J$ = 8.6 Hz, 2H), 6.93 (d, $J$ = 8.5 Hz, 2H), 5.21 (t, $J$ = 5.2 Hz, 1H), 5.12 (d, $J$ = 2.1 Hz, 1H), 4.63 (s, 1H), 4.55 (dd, $J$ = 9.8, 4.2 Hz, 1H), 4.22 - 4.14 (m, 2H), 3.92 - 3.86 (m, 2H), 3.82 (dd, $J$ = 11.6, 6.5 Hz, 1H), 3.10 (q, $J$ = 7.4 Hz, 2H), 2.52 (dd, $J$ = 7.2, 4.4 Hz, 2H), 1.24 (t, $J$ = 7.4 Hz, 3H).

**Example 96 2-cyano-*N*-((*R*)-1-(4-(ethylsulfonyl)phenyl)-2-hydroxyethyl)-4-((2*S*,4*S*) -2-((trifluoromethoxy)methyl)-4-(4-(trifluoromethyl)phenoxy)pyrrolidin-1-yl)benzamide**

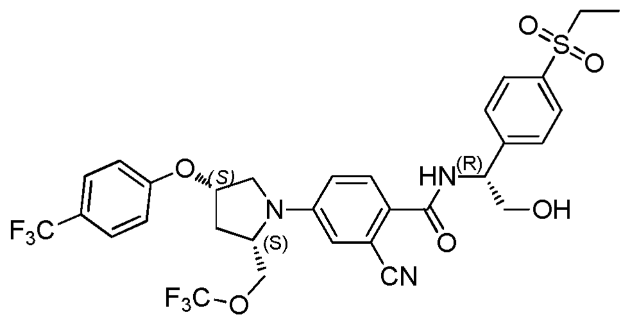

**Step 1: Synthesis of methyl 2-cyano-4-((2*S*,4*S*)-2-(hydroxymethyl)-4-(4-(trifluoromethyl)phenoxy)pyrrolidin-1-yl)benzoate**

To a solution of ((2*S*,4*S*)-4-(4-(trifluoromethyl)phenoxy)pyrrolidin-2-yl)methanol (500 mg, 1.90 mmol) and methyl 2-cyano-4-fluorobenzoate (351 mg, 1.90 mmol) in DMSO (9 mL) was added $K_2CO_3$ (1.10 g, 6.14 mmol). The reaction solution was stirred at 55°C. The reaction solution was cooled to room temperature, and concentrated under reduced pressure. The residue was diluted with DCM (60 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 1/1) to give colorless liquid (240 mg, 30%).

MS (ESI, pos.ion) m/z: 421.1 $[M+H]^+$.

**Step 2: Synthesis of methyl 2-cyano-4-((2*S*,4*S*)-2-((trifluoromethoxy)methyl)-4-(4-(trifluoromethyl)phenoxy)pyrrolidin-1-yl)benzoate**

Under anhydrous and anaerobic conditions, methyl 2-cyano-4-((2*S*,4*S*)-2-(hydroxymethyl)-4-(4-(trifluoromethyl)phenoxy)pyrrolidin-1-yl)benzoate (240 mg, 0.57 mmol), AgOTf (300 mg, 1.2 mmol), selective fluorine reagent (320 mg, 0.86 mmol) and KF (100 mg, 1.72 mmol) were dissolved in DCM (15 mL), then 2-fluoropyridine (0.15 mL, 1.70 mmol) was added, and finally $TMSCF_3$ (0.2 mL, 1 mmol) was added dropwise. The dropwise addition was completed in 10 min, and the mixture was stirred at room temperature for 9 h. The reaction solution was filtered, and to the filtrate was added $NaHCO_3$ solution (20 mL). The resulting mixture was extracted with DCM (30 mL×2). The organic phases were combined, dried over anhydrous $Na_2SO_4$, and concentrated under reduced pressure. The crude product was separated by silica gel column chromatography (eluent: PE/EtOAc (v/v) = 3/1) to give colorless liquid (50 mg, 18%).

MS (ESI, pos.ion) m/z: 489.1 $[M+H]^+$.

**Step 3: Synthesis of 2-cyano-4-((2*S*,4*S*)-2-((trifluoromethoxy)methyl)-4-(4-(trifluoromethyl)phenoxy)pyrrolidin-1-yl)benzoic acid**

To a solution of methyl 2-cyano-4-((2*S*,4*S*)-2-((trifluoromethoxy)methyl)-4-(4-(trifluoromethyl)phenoxy)pyrrolidin-1-yl)benzoate (50 mg, 0.10 mmol) in MeOH (5 mL) and $H_2O$ (1 mL) were LiOH (150 mg, 3.5 mmol). The mixture was reacted at room temperature. The reaction solution was added with HCl solution (2.0 mol/L) to adjust the pH of the solution to about 5. The resulting mixture was extracted with EtOAc (25 mL $\times$ 2), and the organic phases were combined, washed with saturated NaCl (20 mL) solution, dried over anhydrous $Na_2SO_4$, concentrated under reduced pressure, and the crude product was separated by silica gel column chromatography (eluent: DCM/MeOH (v/v) = 10/1) to give a brown solid (40 mg, 82%).

MS (ESI, pos.ion) m/z: 475.1 $[M+H]^+$.

**Step 4: Synthesis of 2-cyano-*N*-((*R*)-1-(4-(ethylsulfonyl)phenyl)-2-hydroxyethyl)-4-((2*S*,4*S*)-2-((trifluoromethoxy)methyl)-4-(4-(trifluoromethyl)phenoxy)pyrroli din-1-yl)benzamide**

(2*R*)-2-amino-2-(4-(ethylsulfonyl)phenyl)ethanol (30 mg, 0.13 mmol), 2-cyano-4-((2*S*,4*S*)-2-((trifluoromethoxy)methyl)-4-(4-(trifluoromethyl)phenoxy)pyrrolidin-1-yl)be nzoic acid (40 mg , 0.084 mmol), HATU (50 mg, 0.13 mmol) and DIPEA (0.1 mL, 0.56 mmol) were dissolved in DCM (5 mL). The mixture was stirred at room temperature for 16 h. The reaction solution was diluted with DCM (30 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: DCM/EtOAc (v/v) = 1/1) to give a white solid (10 mg, 17%).

MS (ESI, pos.ion) m/z: 686.1 $[M+H]^+$;

$^1$H NMR (400 MHz, $CDCl_3$) $\delta$ (ppm): 7.99 (d, *J* = 1.8 Hz, 1H), 7.83 (t, *J* = 10.2 Hz, 3H), 7.58 (dd, *J* = 13.1, 8.5 Hz, 4H), 7.31 (t, *J* = 6.8 Hz, 1H), 6.99 (d, *J* = 8.5 Hz, 2H), 6.80 (d, *J* = 9.2 Hz, 1H), 5.27 (d, *J* = 5.2 Hz, 1H), 5.12 (s, 1H), 4.67 (s, 1H), 4.12 (d, *J* = 11.5 Hz, 1H), 3.97 (ddd, *J* = 14.8, 11.3, 4.6 Hz, 4H), 3.82 (dd, *J* = 11.3, 7.3 Hz, 1H), 3.11 (q, *J* = 7.4 Hz, 2H), 2.49 (d, *J* = 4.3 Hz, 2H), 1.28 (t, *J* = 7.4 Hz, 3H).

**Example 97 3-cyano-*N*-((*R*)-1-(4-(ethylsulfonyl)phenyl)-2-hydroxyethyl)-4-((2*S*,4*S*)-2-((trifluoromethoxy)methyl)-4-(4-(trifluoromethyl)phenoxy)pyrrolidin-1-yl)benzamide**

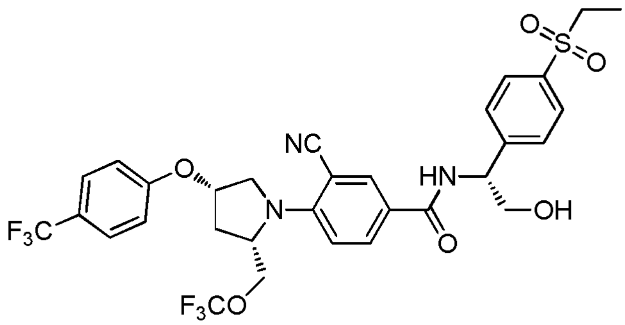

(2*R*)-2-Amino-2-(4-(ethylsulfonyl)phenyl)ethanol (70 mg, 0.31 mmol), 3-cyano-4-((2*S*,4*S*)-2-((trifluoromethoxy)methyl)-4-(4-(trifluoromethyl)phenoxy)pyrrolidin-1-yl)be nzoic acid (130 mg , 0.27 mmol) (taking methyl 3-cyano-4-fluorobenzoate as raw material, it was prepared by referring to the methods of step 1 to step 3 of Example 96), HATU (130 mg, 0.33 mmol) and DIPEA (0.1 mL, 0.56 mmol) were dissolved in DCM (15 mL).The reaction solution was diluted with DCM (20 mL), washed successively with HCl solution (15 mL,0.1 mol/L), saturated $NaHCO_3$ solution (15 mL) and saturated NaCl solution (15 mL). The organic phase was dried over anhydrous $Na_2SO_4$, and concentrated under reduced pressure. The crude product was separated by silica gel column chromatography (eluent: DCM/MeOH (v/v) = 15/1) to give a white solid (30 mg, 16%).

MS (ESI, pos.ion) m/z: 686.1 $[M+H]^+$;

$^1$H NMR (400 MHz, $CDCl_3$) $\delta$ (ppm): 8.48 (d, *J* = 1.9 Hz, 1H), 7.97 (d, *J* = 8.8 Hz, 1H), 7.85 (d, *J* = 8.3 Hz, 2H), 7.58 (dd, *J* = 8.1, 4.9 Hz, 3H), 7.33 (d, *J* = 6.7 Hz, 1H), 6.98 (d, *J* = 8.6 Hz, 2H), 6.63 (d, *J* = 8.9 Hz, 1H), 5.33 (s, 1H), 5.19 - 5.07 (m, 1H), 4.50 (dd, *J* = 12.5, 7.1 Hz, 1H), 4.39 (d, *J* = 12.6 Hz, 1H), 4.16 - 4.02 (m, 2H), 4.01 - 3.91 (m, 2H), 3.76 (dd, *J* = 11.0, 4.6 Hz, 1H), 3.09 (q, *J* = 7.4 Hz, 2H), 2.84 - 2.74 (m, 1H), 2.19 - 2.08 (m, 1H), 1.28 (t, *J* = 7.4 Hz, 3H).

**Example 98 4-((2*S*,4*S*)-2-((difluoromethoxy)methyl)-4-(4-trifluoromethyl)phenoxy) pyrrolidin-1-yl)-*N*-((*S*)-1-(4-(ethylsulfonyl)phenyl)-2-hydroxyethyl)benzamide**

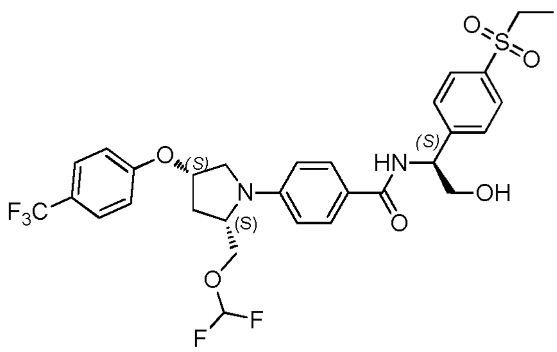

**Step 1: Synthesis of methyl 4-((2*S*,4*S*)-2-((difluoromethoxy)methyl)-4-(4-(trifluoromethyl)phenoxy)pyrrolidin-1-yl)benzoate**

Under nitrogen protection, (2*S*,4*S*)-2-((difluoromethoxy)methyl)-4-(4-(trifluoromethyl)phenoxy)pyrrolidine (200 mg, 0.64 mmol), $Pd_2(dba)_3$ (58 mg, 0.06 mmol), XantPhos (55 mg, 0.10 mmol), $Cs_2CO_3$ (418 mg, 1.28 mmol) and methyl 4-iodobenzoate (252 mg, 0.96 mmol) were added to 1,4-dioxane (6 mL) in turn and the mixture was reacted in a 100°C oil bath for 24 h. The reaction solution was cooled to room temperature, then concentrated under reduced pressure. The residue was diluted with DCM (50 mL), washed successively with $NaHCO_3$ solution (15 mL) and saturated NaCl solution (15 mL), dried over anhydrous $Na_2SO_4$, and concentrated under reduced pressure. The crude product was separated by silica gel column chromatography (eluent: PE/EtOAc (v/v) = 5/1) to give yellow liquid (220 mg, 77%).

MS (ESI, pos.ion) m/z: 446.2 $[M+H]^+$.

**Step 2: Synthesis of 4-((2*S*,4*S*)-2-((difluoromethoxy)methyl)-4-(4-(trifluoromethyl)phenoxy)pyrrolidin-1-yl)benzoic acid**

To a solution of methyl 4-((2*S*,2*S*)-2-((difluoromethoxy)methyl)-4-(4-**(trifluoromethyl)phenoxy)pyrrolidin-1-yl)benzoate**(220 mg, 0.49 mmol) in MeOH (3 mL) and THF (3 mL) was added a solution of $LiOH{\cdot}H_2O$ (414 mg, 9.87 mmol) in $H_2O$ (2 mL). The mixture was stirred at room temperature for 16 h. The reaction solution was concentrated under reduced pressure, and to the residue was added HCl solution (1 mol/L) to adjust the pH of the solution to about 4. The resulting mixture was extracted with EtOAc (20 mL $\times$ 2). The organic phases were combined, washed with saturated NaCl (15 mL) solution, dried over anhydrous $Na_2SO_4$, and concentrated under reduced pressure. The crude product was separated by silica gel column chromatography (eluent: DCM/MeOH (v/v) = 20/1) to give a white solid (170 mg, 80%).

MS (ESI, pos.ion) m/z: 432.2 $[M+H]^+$.

**Step 3: Synthesis of 4-((2*S*,4*S*)-2-((difluoromethoxy)methyl)-4-(4-(trifluoromethyl)phenoxy)pyrrolidin-1-yl)-*N*-((*S*)-1-(4-(ethylsulfonyl)phenyl)-2-hydroxyethyl) benzamide**

HATU (230 mg, 0.60 mmol), (*S*)-2-amino-2-(4-(ethylsulfonyl)phenyl)ethanol (160 mg, 0.60 mmol), 4-((2*S*,4*S*)-2-((difluoromethoxy)methyl)-4-(4-(trifluoromethyl)phenoxy) pyrrolidin-1-yl)benzoic acid (200 mg , 0.46 mmol) and TEA (140 mg, 1.38 mmol) were successively added to DCM (6 mL) and the mixture was stirred at room temperature for 22 h. The reaction solution was diluted with DCM (30 mL), washed successively with saturated HCl solution (15 ml, 0.5 mol/L) and saturated NaCl solution (15 ml), dried over anhydrous $Na_2SO_4$, and concentrated under reduced pressure. The crude product was separated by silica gel column chromatography (eluent: DCM/EtOAc (v/v) = 1/1) to give a white solid (230 mg, 77%).

MS (ESI, pos.ion) m/z: 642.9 $[M+H]^+$.

$^1H$ NMR (400 MHz, $CDCl_3$) $\delta$ (ppm): 7.87 - 7.81 (m, 2H), 7.76 (d, *J* = 8.2 Hz, 2H), 7.61 - 7.51 (m, 4H), 7.04 (d, *J* = 6.7 Hz, 1H), 6.97 (d, *J* = 8.6 Hz, 2H), 6.64 (d, *J* = 7.4 Hz, 2H), 6.22 (t, *J* = 74.3 Hz, 1H), 5.26 (s, 1H), 5.17 (t, *J* = 4.4 Hz, 1H), 4.24 - 4.15 (m, 2H), 4.05 - 3.93 (m, 3H), 3.76 (d, *J* = 11.4 Hz, 1H), 3.70 (dd, *J* = 11.4, 4.6 Hz, 1H), 3.11 - 3.04 (m, 2H), 2.51 (d, *J* = 14.4 Hz, 1H), 2.43 - 2.34 (m, 1H), 1.26 (t, *J* = 7.4 Hz, 3H).

**Example 99 *N*-((*R*)-1-(4-(ethylsulfonyl)phenyl)-2-hydroxyethyl)-4-(1-(4-(trifluoromethyl) benzyl)pyrrolidin-2-yl)benzamide**

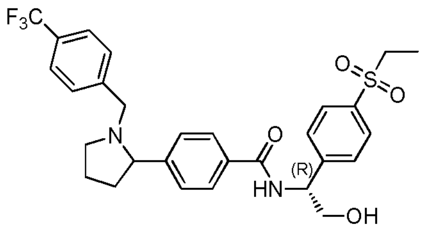

**Step 1: Synthesis of methyl 4-(4-(*tert*-butoxycarbonylamino)butyryl)benzoate**

To a solution of 4-iodobenzoate (3.00 g, 11.00 mmol) in THF (20 mL) was added *i*-PrMgBr (11 mL, 11.00 mmol) at -50°C. The mixture continued to stir for half an hour. Then the mixture was returned to room temperature, and the mixture continued to stir for half an hour at room temperature. After cooling to -50°C, tert-butyl 2-oxopyrrolidone-1-carbonate (2.50 g, 13.00 mmol) was added, and the mixture continued to stir for half an hour before returning to room temperature. The mixture was reacted for 5 hours, and the reaction was quenched by adding water. The resulting mixture was extracted with EtOAc (30 ml × 2). The organic phases were combined, dried over anhydrous $Na_2SO_4$, filtered, concentrated and separated by silica gel column chromatography (eluent: PE/EtOAc (v/v) = 10/1) to give colorless oily liquid (2.00 g, 54%).
MS (ESI, pos.ion) m/z: 322 $[M+H]^+$.

**Step 2: Synthesis of methyl 4-(3,4-dihydro-2H-pyrrol-5-yl)benzoate**

To a solution of methyl 4-(4-(*tert*-butoxycarbonyl)amino)butyryl)benzoate (2.00 g, 6.20 mmol) in DCM (10 mL) was added trifluoroacetate (1.6 mL, 24.00 mmol). The mixture was stirred at room temperature. The mixture was reacted for 2 h. The reaction was quenched by adding water. The resulting mixture was extracted with EtOAc (30 mL × 2). The organic phases were combined, dried over anhydrous $Na_2SO_4$, filtered, concentrated and separated by silica gel column chromatography (eluent: PE/EtOAc (v/v) = 10/1) to give a white solid (1.10 g, 87%).
MS (ESI, pos.ion) m/z: 204 $[M+H]^+$.

**Step 3: Synthesis of methyl 4-(pyrrolidin-2-yl)benzoate**

Methyl 4-(3,4-dihydro-2*H*-pyrrol-5-yl)benzoate (2.23 g, 11.00 mmol) was dissolved in THF (12 mL) and MeOH (5 mL), and $NaBH_4$ (0.11 g, 2.90 mmol) was added slowly at room temperature. The mixture was reacted for 12 h. The reaction was quenched by adding water. The resulting mixture was extracted with EtOAc (30 mL × 2). The organic phases were combined, dried over anhydrous $Na_2SO_4$, filtered and concentrated to give colorless liquid (1.12 g, 49%).
MS (ESI, pos.ion) m/z: 206 $[M+H]^+$.

**Step 4: Synthesis of *tert*-butyl 2-(4-(methoxycarbonyl)phenyl)pyrrolidine-1-carboxylate**

To a solution of methyl 4-(pyrrolidin-2-yl)benzoate (2.25 g, 11.00 mmol) in DCM (10 mL) were added TEA (3.6 mL, 28.00 mmol) and $Boc_2O$ (2.87 g, 13.10 mmol). The mixture was stirred at room temperature. The mixture was reacted for 5 h. The reaction was quenched by adding water. The resulting mixture was extracted with EtOAc (30 mL × 2). The organic phases were combined, dried over anhydrous $Na_2SO_4$, filtered, concentrated and separated by silica gel column chromatography (eluent: PE/EtOAc (v/v) = 10/1) to give a white solid (1.05 g, 31%).
MS (ESI, pos.ion) m/z: 306 $[M+H]^+$.

**Step 5: Synthesis of 4-(1-(*tert*-butoxycarbonyl)pyrrolidin-2-yl)benzoic acid**

*tert*-Butyl 2-(4-(methoxycarbonyl)phenyl)pyrrolidine-1-carboxylate (1.05 g, 3.44 mmol) was dissolved in MeOH (10 mL) and $H_2O$ (5 mL) and the mixture was stirred at room temperature. The mixture was reacted for 12 h, and dilute hydrochloric acid was added to adjust the reaction system to acidity. The resulting mixture was extracted with EtOAc (30 mL × 2). The organic phases were combined, dried over anhydrous $Na_2SO_4$, filtered and concentrated to give a

white solid (0.88 g, 88%).

MS (ESI, pos.ion) m/z: 292 $[M+H]^+$.

**Step 6: Synthesis of *tert*-butyl 2-(4-(((*R*)-1-(4-(ethylsulfonyl)phenyl)-2-hydroxyethyl) carbamoyl)phenyl)pyrrolidine-1-carboxylate**

4-(1-(*tert*-Butoxycarbonyl)pyrrolidin-2-yl)benzoic acid (210 mg, 0.72 mmol), (*R*)-3-amino-3-(4-(ethylsulfonyl)phenyl)ethanol (206 mg, 0.86 mmol) and HATU (332 mg, 0.86 mmol) were dissolved in DCM (12 mL), then $Et_3N$ (0.22 mL, 1.70 mmol) was added and the mixture was stirred at room temperature. The mixture was reacted for 12 h. The reaction was quenched by adding water. The resulting mixture was extracted with EtOAc (10 mL × 2). The organic phases were combined, dried over anhydrous $Na_2SO_4$, filtered, concentrated and separated by silica gel column chromatography (eluent: PE/EtOAc (v/v) = 1/1) to give a white solid (220 mg, 60%).

MS (ESI, pos.ion) m/z: 503 $[M+H]^+$.

**Step 7: Synthesis of *N*-((*R*)-1-(4-(ethylsulfonyl)phenyl)-2-hydroxyethyl) -4-(pyrrolidin-2-yl)benzamide**

To a solution of *tert*-butyl 2-(4-(((*R*)-1-(4-(ethylsulfonyl)phenyl)-2-hydroxyethyl) carbamoyl)phenyl)pyrrolidine-1-carboxylate (150 mg, 0.29 mmol) in DCM (10 mL) was added $CF_3COOH$ (0.10 mL, 1.30 mmol) slowly. The mixture was stirred at room temperature. The mixture was reacted for 2 h, then saturated sodium carbonate solution was added to adjust pH to neutrality, and the resulting mixture was extracted with DCM (10 mL × 2). The organic phases were combined, dried over anhydrous $Na_2SO_4$, filtered and concentrated under reduced pressure to give colorless liquid (120 mg, 99%).

**Step 8: Synthesis of *N*-((*R*)-1-(4-(ethylsulfonyl)phenyl)-2-hydroxyethyl)-4-(1-(4-(trifluoromethyl)benzyl)pyrrolidin-2-yl)benzamide**

To a solution of *N*-((*R*)-1-(4-(ethylsulfonyl)phenyl)-2-hydroxyethyl) -4-(pyrrolidin-2-yl)benzamide (120 mg, 0.29 mmol) in MeOH (10 mL) were added 4-(trifluoromethyl)benzaldehyde (61 mg, 0.35 mmol) and $NaCNBH_3$ (7 mg, 0.11 mmol). The mixture was stirred at room temperature. The mixture was reacted for 12 h. The reaction was quenched by adding water. The resulting mixture was extracted with DCM (10 mL × 2). The organic phases were combined, dried over anhydrous $Na_2SO_4$, filtered, concentrated and separated by silica gel column chromatography (eluent: DCM/EtOAc (v/v) = 3/1) to give a white solid (84 mg, 51%).

MS (ESI, pos.ion) m/z: 570 $[M+H]^+$;

$^1H$ NMR (400 MHz, $CDCl_3$) δ (ppm): 7.82 (d, *J* = 8.1 Hz, 4H), 7.55 (t, *J* = 7.7 Hz, 6H), 7.39 (d, *J* = 7.8 Hz, 2H), 7.34 (d, *J* = 6.9 Hz, 1H), 5.26 (d, *J* = 6.2 Hz, 1H), 3.96 (ddd, *J* = 16.3, 11.3, 4.0 Hz, 2H), 3.81 (d, *J* = 13.4 Hz, 1H), 3.49 (t, *J* = 8.1 Hz, 1H), 3.19 (d, *J* = 13.5 Hz, 1H), 3.07 (dd, *J* = 15.0, 7.5 Hz, 3H), 2.31 - 2.16 (m, 2H), 2.01 - 1.65 (m, 3H), 1.25 (t, *J* = 7.5 Hz, 3H).

**Example 100 (*R*)-*N*(1-(4-(ethylsulfonyl)phenyl)-2-hydroxyethyl)-4-(3-(4-(trifluoromethyl) phenoxy)acridin-1-yl)benzamide**

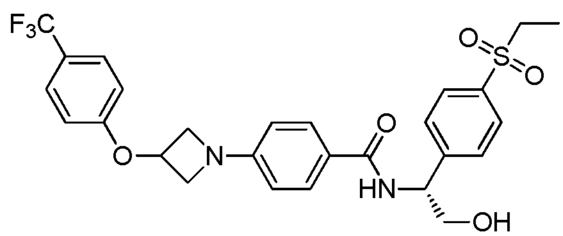

**Step 1: Synthesis of *tert*-butyl 3-(4-(trifluoromethyl)phenoxy)acridin-1-carboxylate**

To a solution of 4-(trifluoromethyl)phenol (2.00 g, 12.3 mmol) in anhydrous THF (45 mL) were added tert-butyl 3-hydroxyacridine-1-carboxylate (2.11 g, 12.2 mmol) and $PPh_3$ (3.80 g, 14.3 mmol). The mixture was cooled to 0°C and then DIAD (4.10 mL, 20.0 mmol) was added dropwise over 30 minutes. The reaction solution was stirred at room temperature for 18 h. The reaction solution was concentrated under reduced pressure, and methyl tert-butyl ether (80

mL) was added. The mixture was cooled to -15 °C and stirred, and a white solid was precipitated. The resulting mixture was filtered, and the filtrate was concentrated. The crude product was diluted with DCM (80 mL) and washed with $NaHCO_3$ solution (100 mL). The organic phase was dried over anhydrous $Na_2SO_4$, concentrated under reduced pressure, and the crude product was separated by silica gel column chromatography (eluent: PE/EtOAc (v/v) = 4/1) to give colorless liquid (780 mg, 20%).
MS (ESI, pos.ion) m/z: 549.1[M+H]$^+$.

**Step 2: Synthesis of 3-(4-(trifluoromethyl)phenoxy)acridine**

To a solution of *tert*-butyl 3-(4-(trifluoromethyl)phenoxy)acridin-1-carboxylate (780 mg, 2.46 mmol) in DCM (15 mL) was added TFA (6.6 mL, 89 mmol). The reaction solution was stirred at room temperature for 8 h. The reaction solution was diluted with DCM (20 mL), washed successively with saturated $NaHCO_3$ solution (40 mL) and saturated NaCl solution (40 mL), dried over anhydrous $Na_2SO_4$, and concentrated under reduced pressure. The crude product was separated by silica gel column chromatography (eluent: DCM/MeOH (v/v) = 10/1) to give colorless liquid (400 mg, 75%).
MS (ESI, pos.ion) m/z: 218.1 [M+H]$^+$.

**Step 3: Synthesis of methyl 4-(3-(4-(trifluoromethyl)phenoxy)acridin-1-yl)benzoate**

Under nitrogen protection, to a solution of methyl 4-iodo-benzoate (800 mg, 3.05 mmol) and 3-(4-(trifluoromethyl)phenoxy)acridine (400 mg, 1.84 mmol) in toluene (50 mL) were added $Cs_2CO_3$ (900 mg, 2.76 mmol), XantPhos (60 mg, 0.10 mmol) and $Pd_2(dba)_3$ (95 mg, 0.10 mmol). The mixture was heated to 95°C and stirred for 14 h. The reaction solution was cooled to room temperature and filtered. The filtrate was concentrated, and the mixed solution was diluted with $NaHCO_3$ solution (30 mL) and DCM (30 mL). The aqueous phase was extracted with DCM (20 mL). The organic phases were combined, dried over anhydrous $Na_2SO_4$, and concentrated under reduced pressure. The crude product was separated by silica gel column chromatography (eluent: PE/EtOAc (v/v) = 4/1) to give a yellow solid (250 mg, 39%).
MS (ESI, pos.ion) m/z: 352.1 [M+H]$^+$.

**Step 4: Synthesis of 4-(3-(4-(trifluoromethyl)phenoxy)acridin-1-yl)benzoic acid**

To a solution of methyl 4-(3-(4-(trifluoromethyl)phenoxy)acridin-1-yl)benzoate (250 mg, 0.71 mmol) in THF (15 mL, 184 mmol) were added $H_2O$ (3 mL) and LiOH (230 mg, 5.37 mmol). The reaction solution was stirred and reacted at room temperature for 16 h. HCl solution (1.0 mol/L) was slowly dropwise added to the reaction solution to adjust the pH of the solution to about 5. The resulting mixture was extracted with EtOAc (10 mL $\times$ 3), and the organic phases were combined, washed with saturated NaCl solution (15 mL), dried over anhydrous $Na_2SO_4$, filtered and concentrated under reduced pressure to give a crude product was a white solid (200 mg, 83%).
MS (ESI, pos.ion) m/z: 338.1 [M+H]$^+$.

**Step 5: Synthesis of (*R*)-*N*-(1-(4-(ethylsulfonyl)phenyl)-2-hydroxyethyl)-4-(3-(4-(trifluoromethyl)phenoxy)acridin-1-yl)benzamide**

(2*R*)-2-Amino-2-(4-(ethylsulfonyl)phenyl)ethanol (80 mg, 0.35 mmol), 4-(3-(4-(trifluoromethyl)phenoxy)acridin-1-yl)benzoic acid (60 mg, 0.18 mmol), HATU (100 mg, 0.26 mmol) and DIPEA (0.2 mL, 1.2 mmol) were dissolved in DCM (20 mL). The mixture was stirred and reacted at room temperature for 17 h. After the reaction was completed, the reaction solution was diluted with DCM (40 mL), washed successively with HCl solution (15 mL,0.1 mol/L), saturated $NaHCO_3$ solution (20 mL) and saturated NaCl solution (15 mL). The organic phase was dried over anhydrous $Na_2SO_4$, filtered and concentrated under reduced pressure. The crude product was separated by silica gel column chromatography (eluent: DCM/EtOAc (v/v) = 1/1) to give a white solid (53 mg, 54%).
MS (ESI, pos.ion) m/z: 549.1[M+H]$^+$;
$^1$H NMR (400 MHz, $CDCl_3$) $\delta$ (ppm): 8.52 (d, *J* = 7.6 Hz, 1H), 7.91 (dd, *J* = 24.0, 8.1 Hz, 4H), 7.64 (dd, *J* = 18.1, 8.0 Hz, 4H), 5.55 (d, *J* = 5.9 Hz, 1H), 3.07 (q, *J* = 7.4 Hz, 2H), 2.84 (s, 2H), 1.24 (t, *J* = 7.3 Hz, 3H).

**Example 101 *N*-((*R*)-1-(4-((*N*-cyclopropylamino)sulfonyl)phenyl)-2-hydroxyethyl) -4-((2*S*,4*S*)-2-((difluoromethoxy)methyl)-4-(4-(trifluoromethyl)phenoxy)pyrrolidin-1-yl)benza** mide

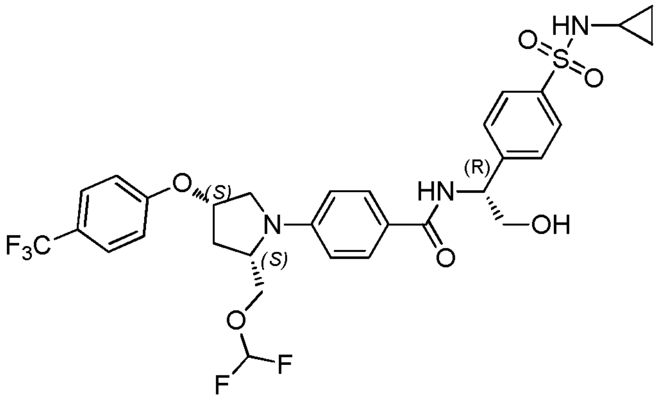

**Step 1: Synthesis of (*R*)-4-phenyl-2-oxazolone**

To a solution of (2*R*)-2-amino-2-phenylethanol (1.58 g, 11.5 mmol) in DCM (15 mL) were added TEA (3.30 mL, 22.6 mmol) dropwise and triphosgene (3.20 g, 12.1 mmol) in turn. The reaction solution was stirred and reacted at room temperature for 1 h. After the reaction was completed, the reaction solution was diluted with DCM (50 mL), washed successively with HCl solution (15 mL,0.1 mol/L), saturated $NaHCO_3$ solution (15 mL) and saturated NaCl solution (15 mL). The organic phase was dried over anhydrous $Na_2SO_4$, filtered and concentrated under reduced pressure to give a white solid (1.20 g, 64%).

MS (ESI, pos.ion) m/z: 164.1 $[M+H]^+$.

**Step 2: Synthesis of (*R*)-4-(2-oxooxazolidin-4-yl)-1-phenylsulfonyl chloride**

To a solution of (*R*)-4-phenyl-2-oxazolone (1.20 g, 7.35 mmol) in DCM (5 mL) was added chlorosulfonic acid (6.0 mL, 91 mmol), and the reaction solution was stirred at room temperature for 5 h. The reaction solution was dropped into ice water (70 mL) to quench the reaction. The aqueous phase was extracted with EtOAc (20 mL×3), and the organic phase was washed with saturated NaCl solution (15 mL), dried over anhydrous $Na_2SO_4$, filtered and concentrated under reduced pressure to give colorless liquid (1.20 g, 62%).

MS (ESI, pos.ion) m/z: 262.1 $[M+H]^+$.

**Step 3: Synthesis of (*R*)-*N*-cyclopropyl-4-(2-oxooxazolidin-4-yl)benzenesulfonamide**

To a solution of cyclopropylamine (1.1 mL, 16 mmol) in DCM (15 mL) was slowly added (*R*)-4-(2-oxooxazolidin-4-yl)-1-phenylsulfonyl chloride (1.20 g, 4.59 mmol), and the reaction solution was stirred at room temperature for 8 h. After the reaction was completed, the reaction solution was concentrated under reduced pressure to give a pale yellow solid (800 mg, 62%).

MS (ESI, pos.ion) m/z: 283.1 $[M+H]^+$.

**Step 4: Synthesis of (*R*)-4-(1-amino-2-hydroxyethyl)-*N*-cyclopropylbenzenesulfonamide**

To a solution of (*R*)-*N*-cyclopropyl-4-(2-oxooxazolidin-4-yl)benzenesulfonamide (200 mg, 0.71 mmol) in MeOH (16 mL) were added $H_2O$ (3 mL) and KOH (220 mg, 1.76 mmol) in turn, and the reaction solution was heated to 80°C and stirred for 8 h. The reaction solution was concentrated under reduced pressure, and the crude product was separated by silica gel column chromatography (eluent: DCM/MeOH (v/v) = 10/1) to give a yellow solid (100 mg, 55%).

MS (ESI, pos.ion) m/z: 257.1 $[M+H]^+$.

**Step 5: Synthesis of *N*-((*R*)-1-(4-((*N*-cyclopropylamino)sulfonyl)phenyl)-2-hydroxyethyl)-4-((2*S*,4*S*)-2-((difluoromethoxy)methyl)-4-(4-(trifluoromethyl)phenoxy)pyrrolid in-1-yl)benzamide**

4-((2*S*,4*S*)-2-((Difluoromethoxy)methyl)-4-(4-(trifluoromethyl)phenoxy)pyrrolidin-1-y l)benzoic acid (211 mg, 0.49 mmol), (*R*)-4-(1-amino-2-hydroxyethyl) -*N*-cyclopropylbenzenesulfonamide (100 mg, 0.39 mmol), HATU (200 mg, 0.51 mmol) and DIPEA (0.15 mL, 0.91 mmol) were dissolved in DCM (25 mL). The reaction solution was stirred at room temperature for 17 h. The reaction solution was diluted with DCM (10 mL), washed successively with HCl solution (10 mL, 1.0 mol/L), saturated $NaHCO_3$ solution (10 mL) and saturated NaCl solution (5 mL). The organic phase was dried over anhydrous $Na_2SO_4$, and concentrated under reduced pressure. The crude product was separated by silica gel

column chromatography (eluent: DCM/EtOAc (v/v) = 1/1) to give a white solid (123 mg, 47%).

MS (ESI, pos.ion) m/z: 670.3 $[M+H]^+$.

$^1H$ NMR (400 MHz, $CD_3OD$) δ (ppm): 7.84 (t, *J* = 7.8 Hz, 4H), 7.62 (dd, *J* = 8.4, 3.0 Hz, 4H), 7.13 (d, *J* = 8.6 Hz, 2H), 6.75 (d, *J* = 8.8 Hz, 2H), 6.40 (t, *J* = 75.1 Hz, 1H), 5.30 (s, 1H), 5.24 (t, *J* = 6.2 Hz, 1H), 4.24 (dd, *J* = 8.9, 4.3 Hz, 1H), 4.15 (dd, *J* = 9.8, 4.2 Hz, 1H), 3.96 (t, *J* = 9.6 Hz, 1H), 3.90 (d, *J* = 6.3 Hz, 2H), 3.74 (s, 2H), 2.46 (s, 2H), 2.16 - 2.07 (m, 1H), 0.56 - 0.43 (m, 4H).

**Example 102 *N*-((*R*)-1-(4-((cyclopropylmethyl)sulfonyl)phenyl)-2-hydroxyethyl) -4-((2*S*,4*S*)-2-((difluoromethoxy)methyl)-4-(4-(trifluoromethyl)phenoxy)pyrrolidin-1-yl)benza mide**

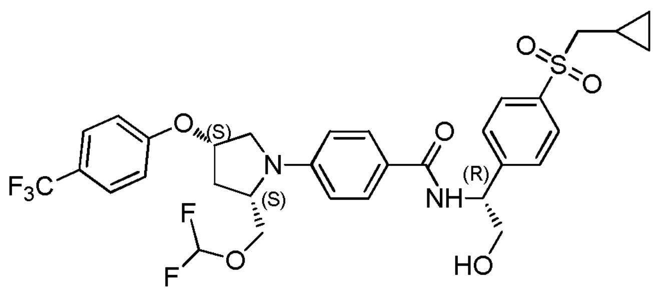

**Step 1: Synthesis of (*R*)-4-(4-iodophenyl)oxazolidin-2-one**

Under nitrogen protection, (R)-4-phenyloxazolidin-2-one (1.40 g, 8.58 mmol) and TfOH (0.10 mL, 1.1 mmol) were dissolved in ACN (50 mL). The mixture was stirred at room temperature and 1,3-diiodo-5,5-dimethylhydantoin (3.20 g, 8.25 mmol) was added. The reaction solution was heated to 80°C and stirred for 10 h. After the reaction was completed, the stirring was stopped. The reaction solution was cooled to room temperature, then $Na_2SO_3$ solution (50 mL) was added. The mixed solution was diluted with DCM (50 mL). The aqueous phase was extracted with DCM (20 mL). The organic phases were combined, dried over anhydrous $Na_2SO_4$, filtered and concentrated under reduced pressure. The crude product was separated by silica gel column chromatography (eluent: PE/EtOAc (v/v) = 3/1) to give a pale yellow solid (900 mg, 36%).

MS (ESI, pos.ion) m/z: 290.1 $[M+H]^+$.

**Step 2: Synthesis of (*R*)-4-(4-((cyclopropylmethyl)sulfonyl)phenyl)oxazolidin-2-one**

Under nitrogen protection, sodium cyclopropylmethylsulfinate (760 mg, 5.35 mmol), (*R*)-4-(4-iodophenyl)oxazolidin-2-one (700 mg, 2.42 mmol), CuI (90 mg, 0.45 mmol) and (2*S*,4*R*)-*N*-(2,6-dimethylphenyl)-4-hydroxypyrrolidine-2-carboxamide (110 mg, 0.47 mmol) was dissolved in DMSO (5 mL), then $K_3PO_4$ (650 mg, 3.55 mmol) was added. The reaction solution was heated to 50°C and stirred for 16 h. The reaction solution was diluted with $H_2O$ (30 mL) and DCM (40 mL). The organic phase was washed successively with HCl solution (25 mL,0.1 mol/L), saturated $NaHCO_3$ solution (25 mL) and saturated NaCl solution (25 mL). The organic phase was dried over anhydrous $Na_2SO_4$, filtered and concentrated under reduced pressure to give a yellow solid (390 mg, 57%).

MS (ESI, pos.ion) m/z: 282.1 $[M+H]^+$.

**Step 3: Synthesis of (*R*)-2-amino-2-(4-((cyclopropylmethyl)sulfonyl)phenyl)ethanol**

To a solution of (R)-4-(4-((cyclopropylmethyl)sulfonyl)phenyl)oxazolidin-2-one (391 mg, 1.39 mmol) in MeOH (15 mL) was added $H_2O$ (2 mL), then KOH (950 mg, 7.62 mmol) was slowly added. The reaction solution was heated to 80°C and stirred for 13 h. The reaction solution was concentrated under reduced pressure, and the crude product was separated by silica gel column chromatography (eluent: DCM/MeOH (v/v) = 10/1) to give a yellow solid (280 mg, 79%).

MS (ESI, pos.ion) m/z: 256.1 $[M+H]^+$.

**Step 4: Synthesis of *N*-((*R*)-1-(4-((cyclopropylmethyl)sulfonyl)phenyl) -2-hydroxyethyl)-4-((2*S*,4*S*)-2-((difluoromethoxy)methyl)-4-(4-(trifluoromethyl)phenoxy)pyrr olidin-1-yl)benzamide**

4-((2*S*,4*S*)-2-(Difluoromethoxymethyl)-4-(4-(trifluoromethyl)phenoxy)pyrrolidin-1-yl) benzoic acid (111 mg,

0.26 mmol), (*R*)-2-amino-2-(4-((cyclopropylmethyl)sulfonyl)phenyl)ethanol (60 mg, 0.24 mmol), HATU (120 mg, 0.31 mmol) and DIPEA (0.05 mL, 0.30 mmol) were dissolved in DCM (25 mL). The reaction solution was stirred at room temperature for 15 h. The reaction solution was diluted with DCM (20 mL), washed successively with HCl solution (20 mL, 1.0 mol/L), saturated $NaHCO_3$ solution (20 mL) and saturated NaCl solution (25 mL). The organic phase was dried over anhydrous $Na_2SO_4$, and concentrated under reduced pressure. The crude product was separated by silica gel column chromatography (eluent: DCM/EtOAc (v/v) = 1/1) to give a white solid (93 mg, 59%).

MS (ESI, pos.ion) m/z: 669.3 $[M+H]^+$;

$^1H$ NMR (400 MHz, $CDCl_3$) δ (ppm): 7.86 (d, *J* = 7.6 Hz, 2H), 7.76 (d, *J* = 8.5 Hz, 2H), 7.56 (dd, *J* = 16.3, 8.0 Hz, 4H), 7.09 (d, *J* = 7.0 Hz, 1H), 6.97 (d, *J* = 8.2 Hz, 2H), 6.64 (d, *J* = 8.3 Hz, 2H), 6.22 (t, *J* = 74.3 Hz, 1H), 5.26 (s, 1H), 5.17 (s, 1H), 4.17 (d, *J* = 8.7 Hz, 2H), 4.07 - 3.85 (m, 3H), 3.73 (dd, *J* = 17.3, 7.1 Hz, 2H), 2.97 (d, *J* = 7.0 Hz, 2H), 2.51 (d, *J* = 14.2 Hz, 1H), 2.39 (d, *J* = 5.6 Hz, 1H), 0.95 (s, 1H), 0.56 (d, *J* = 7.3 Hz, 2H), 0.17 (d, *J* = 3.5 Hz, 2H).

**Example** 103 ***N*-(4-((*N*-cyclopropylamino)sulfonyl)benzyl)-4-((2*S*,4*S*)-2-((difluoromethoxy) methyl)-4-(4-(trifluoromethyl)phenoxy)pyrrolidin-1-yl)benzamide**

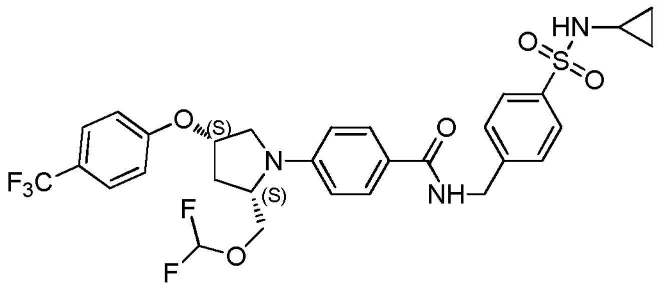

**Step 1: Synthesis of 4-(aminomethyl)-*N*-cyclopropylbenzenesulfonamide**

To a solution of benzylamine (1.00 mL, 9.16 mmol) in DCM (2 mL) were added TFA (1 mL, 10.84 mmol) dropwise and chlorosulfonic acid (10 mL, 152 mmol) in turn. The reaction solution was stirred and reacted at room temperature for 3 h. The stirring was stopped, and the reaction solution was dropped into ice water (80 mL) to quench the reaction. The mixture was used directly in the next step without further treatment. The above mixture was added dropwise to a solution of cyclopropylamine (1.7 mL, 7.29 mmol) in saturated $NaHCO_3$ (80 mL) for 30 min, and the reaction solution was stirred at room temperature for 8 h. The reaction solution was extracted with EtOAc (50 mL). The organic phase was dried over anhydrous $Na_2SO_4$, filtered and concentrated under reduced pressure. The crude product was separated by silica gel column chromatography (eluent: DCM/MeOH (v/v) = 10/1) to give a pale yellow solid (708 mg, 43%).

MS (ESI, pos.ion) m/z: 227.1 $[M+H]^+$.

**Step 2: Synthesis of *N*-(4-((*N*-cyclopropylamino)sulfonyl)benzyl)-4-((2*S*,4*S*)-2-((difluoromethoxy)methyl)-4-(4-(trifluoromethyl)phenoxy)pyrrolidin-1-yl)benzamide**

4-((2*S*,4*S*)-2-((Difluoromethoxy)methyl)-4-(4-(trifluoromethyl)phenoxy)pyrrolidin-1-y l)benzoic acid (161 mg, 0.37 mmol), 4-(aminomethyl)-*N*-cyclopropylbenzenesulfonamide (60 mg, 0.26 mmol), HATU (130 mg, 0.33 mmol) and DIPEA (0.1 mL, 0.56 mmol) were dissolved in DCM (15 mL). The reaction solution was stirred at room temperature for 16 h. The reaction solution was diluted with DCM (10 mL), and the resulting mixture was washed successively with HCl solution (20 mL, 0.1 mol/L), saturated $NaHCO_3$ solution (20 mL) and saturated NaCl solution (15 mL), dried over anhydrous $Na_2SO_4$, filtered and concentrated under reduced pressure. The crude product was separated by silica gel column chromatography (eluent: DCM/EtOAc (v/v) = 1/1) to give a white solid (83 mg, 49%).

MS (ESI, pos.ion) m/z: 640.1 $[M+H]^+$;

$^1H$ NMR (400 MHz, $CDCl_3$) δ (ppm): 7.85 (d, *J* = 8.1 Hz, 2H), 7.75 (d, *J* = 8.6 Hz, 2H), 7.59 (d, *J* = 8.5 Hz, 2H), 7.49 (d, *J* = 8.1 Hz, 2H), 6.97 (d, *J* = 8.6 Hz, 2H), 6.66 (d, *J* = 8.7 Hz, 2H), 6.47 (s, 1H), 6.22 (t, *J* = 74.2 Hz, 2H), 5.17 (s, 1H), 4.84 (s, 1H), 4.72 (d, *J* = 5.8 Hz, 2H), 4.19 (t, *J* = 7.8 Hz, 2H), 3.96 (t, *J* = 9.3 Hz, 1H), 3.72 (dt, *J* = 11.0, 7.7 Hz, 2H), 2.52 (d, *J* = 14.4 Hz, 1H), 2.39 (d, *J* = 6.3 Hz, 1H), 2.23 (s, 1H), 0.88 (s, 1H), 0.61 (d, *J* = 4.6 Hz, 4H).

**Example 104 *N*-((*R*)-1-(4-(ethylsulfonyl)phenyl)-2-hydroxyethyl)-4-((*S*)-3-(4-(2-morpholinoethoxy)phenoxy)pyrrolidin-1-yl)benzamide**

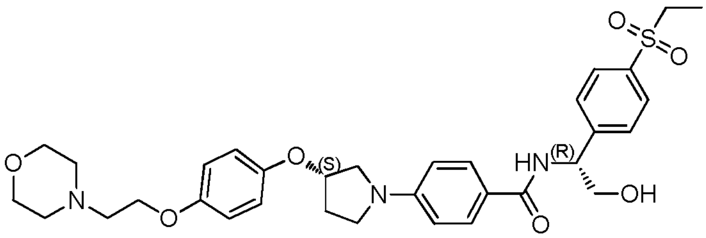

**Step 1: Synthesis of 4-(2-(4-(benzyloxy)phenoxy)ethyl)morpholine**

4-(2-Chloroethyl)morpholine hydrochloride (2.00 g, 10.42 mmol), 4-(benzyloxy)phenol (2.13 g, 10.43 mmol) and $K_2CO_3$ (3.00 g, 21.71 mmol) were added to DMF (20 mL). The mixture was reacted at 105°C for 24 h. The reaction solution was filtered and the filtrate was concentrated under reduced pressure, and the crude product was separated by silica gel column chromatography (eluent: DCM/EtOAc (v/v) = 1/2) to give light brown liquid (5.88 g, 58%).
MS (ESI, pos.ion) m/z: 314.2 $[M+H]^+$.

**Step 2: Synthesis of 4-(2-morpholinoethoxy)phenol**

To a solution of 4-(2-(4-(benzyloxy)phenoxy)ethyl)morpholine (1.80 g, 5.74 mmol) in MeOH (20 mL) was added Pd/C (180 mg, 10%) under hydrogen protection. The mixture was reacted at room temperature for 16 h. The reaction solution was filtered, and the filtrate was concentrated under reduced pressure to give a light brown solid (1.28 g, 100%).
MS (ESI, pos.ion) m/z: 224.2 $[M+H]^+$.

**Step 3: Synthesis of *tert*-butyl (*S*)-3-(4-(2-morpholinoethoxy)phenoxy) pyrrolidine-1-carboxylate**

*tert*-Butyl (*R*)-3-hydroxypyrrolidine-1-carboxylate (1.10 g, 5.87 mmol), 4-(2-morpholinoethoxy)phenol (1.28 g, 5.73 mmol), $PPh_3$ (1.65 g, 6.29 mmol) were added to THF (20 mL). The mixture was transferred to 0 °C, and DIAD (1.50 mL, 7.62 mmol) was slowly added. After the addition was complete, the mixture was transferred to room temperature and reacted for 22 h. The reaction solution was concentrated under reduced pressure. The residue was diluted with methyl *tert*-butyl ether (30 mL) and stirred at -20 °C. A large amount of white insoluble solids (triphenoxyphosphine) were precipitated, filtered while cold, and the filter cake was washed with cold methyl *tert*-butyl ether. The filtrate was concentrated under reduced pressure, and the crude product was separated by silica gel column chromatography (eluent: DCM/EtOAc (v/v) = 1/2) to give yellow liquid (1.90 g, 84%).
MS (ESI, pos.ion) m/z: 393.1 $[M+H]^+$.

**Step 4: Synthesis of (*S*)-4-(2-(4-((pyrrolidin-3-yl)oxy)phenoxy)ethyl)morpholine**

To a solution of *tert*-butyl (*S*)-3-(4-(2-morpholinoethoxy)phenoxy)pyrrolidine -1-carboxylate (1.80 g, 4.59 mmol) in DCM (6 mL) was added a solution of HCl in 1,4-dioxane (6.0 mL, 4 mol/L). The mixture was reacted at room temperature for 22 h. The reaction solution was concentrated under reduced pressure, and to the the residue was added DCM (20 mL) and a little triethylamine to dissolve the residue. The resulting mixture was concentrated under reduced pressure, and the crude product was separated by silica gel column chromatography (eluent: DCM/MeOH (v/v) = 4/1) to give pale yellow liquid (1.10 g, 82%).
MS (ESI, pos.ion) m/z: 293.2 $[M+H]^+$.

**Step 5: Synthesis of ethyl (*S*)-4-(3-(4-(2-morpholinoethoxy)phenoxy) pyrrolidin-1-yl)benzoate**

Under nitrogen protection, (*S*)-4-(2-(4-((pyrrolidin-3-yl)oxy)phenoxy)ethyl)morpholine (1.00 g, 3.42 mmol), $Pd_2(dba)_3$ (300 mg, 0.33 mmol), Ruphos (240 mg, 0.51 mmol), $Cs_2CO_3$ (900 mg, 2.76 mmol) and ethyl 4-iodobenzoate (1.13 g, 4.10 mmol) were successively added to 1,4-dioxane (16 mL) and the mixture was reacted in a 100°C oil bath for 24 h. The reaction solution was cooled to room temperature and filtered through a celite pad, the filtrate was concentrated under reduced pressure, and the crude product was separated by silica gel column chromatography (eluent:

DCM/MeOH (v/v) = 30/1) to give a pale brown solid (660 mg, 44%).
MS (ESI, pos.ion) m/z: 441.3 $[M+H]^+$.

**Step 6: Synthesis of (*S*)-4-(3-(4-(2-morpholinoethoxy)phenoxy)pyrrolidin-1-yl)benzoate hydrochloride**

To a solution of ethyl (*S*)-4-(3-(4-(2-morpholinoethoxy)phenoxy)pyrrolidin-1-yl) benzoate (660 mg, 1.50 mmol) in MeOH (3 mL) and THF (3 mL) was added a solution of $LiOH{\cdot}H_2O$ (330 mg, 8.30 mmol) in $H_2O$ (3 mL). The mixture was stirred at room temperature for 15 h. The reaction solution was concentrated under reduced pressure, and HCl solution (1 mol/L) was added to the remaining solution to adjust the pH of the solution to about 4. A large amount of insoluble solid was precipitated, which was filtered and dried *in vacuo* to obtain a pale yellow solid (450 mg, 67%).
MS (ESI, pos.ion) m/z: 413.2 $[M+H]^+$.

**Step 7: Synthesis of *N*-((*R*)-1-(4-(ethylsulfonyl)phenyl)-2-hydroxyethyl)-4-((*S*)-3-(4-(2-morpholinoethoxy)phenoxy)pyrrolidin-1-yl)benzamide**

HATU (254 mg, 0.67 mmol), (R)-2-amino-2-(4-(ethylsulfonyl)phenyl)ethanol (112 mg, 0.49 mmol), (*S*)-4-(3 -(4-(2-morpholinoethoxy)phenoxy)pyrrolidin-1-yl)benzoate hydrochloride (200 mg , 0.45 mmol) and TEA (0.2 mL, 1.40 mmol) were successively added to DCM (4 mL) and the mixture was stirred at room temperature for 16 h. The reaction solution was concentrated under reduced pressure. The residue was dissolved with DCM (40 mL), washed successively with $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/MeOH (v/v) = 10/1) to give a pale yellow solid (100 mg, 36%).
MS (ESI, pos.ion) m/z: 624.4 $[M+H]^+$.
$^1H$ NMR (600 MHz, $CDCl_3$) δ (ppm): 7.83 (d, *J* = 8.2 Hz, 2H), 7.72 (d, *J* = 8.6 Hz, 2H), 7.54 (d, *J* = 8.2 Hz, 2H), 6.99 (d, *J* = 6.8 Hz, 1H), 6.83 (q, *J* = 9.3 Hz, 4H), 6.53 (d, *J*= 8.6 Hz, 2H), 5.25 (s, 1H), 4.98 (s, 1H), 4.06 (t, *J* = 5.6 Hz, 2H), 3.99 (dd, *J* = 11.2, 3.7 Hz, 1H), 3.93 (dd, *J* = 11.3, 4.9 Hz, 1H), 3.75 - 3.70 (m, 4H), 3.65 (dd, *J* = 10.9, 4.5 Hz, 1H), 3.60 - 3.45 (m, 3H), 3.07 (q, *J* = 7.4 Hz, 2H), 2.78 (t, *J*= 5.6 Hz, 2H), 2.57 (s, 4H), 2.39 - 2.32 (s, 1H), 2.29 - 2.19 (m, 1H), 1.26 (t, *J* = 7.4 Hz, 3H).

**Example 105 *N*-((*R*)-1-(4-(ethylsulfonyl)phenyl)-2-hydroxyethyl)-4-((*R*)-3-(4-(3-morpholinopropoxy)benzyl)pyrrolidin-1-yl)benzamide / *N*-((*R*)-1-(4-(ethylsulfonyl)phenyl)-2-hydroxyethyl)-4-((*S*)-3-(4-(3-morpholinopropoxy)benzyl)pyrrolidin-1-yl)benzamide**

105-1:

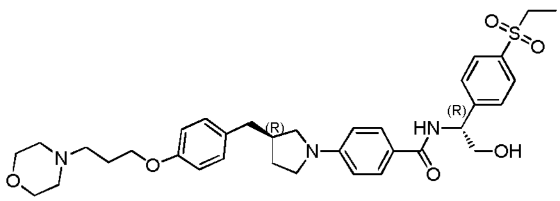

105-2:

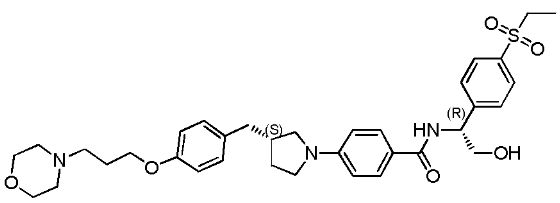

**Step 1: Synthesis of 4-(3-(4-bromophenoxy)propyl)morpholine**

3-Morpholinopropan-1-ol (2.00 g, 13.50 mmol), 4-bromophenol (2.38 g, 13.50 mmol), $PPh_3$ (3.89 g, 14.83 mmol) were added to THF (20 mL). The mixture was transferred to 0 °C, and DIAD (3.50 mL, 17.78 mmol) was slowly added. After the addition was complete, the mixture was transferred to room temperature and reacted for 16 h. The

reaction solution was concentrated under reduced pressure. The residue was diluted with methyl *tert*-butyl ether (30 mL) and stirred at -20 °C. A large amount of white insoluble solids (triphenoxyphosphine) were precipitated, filtered while cold, and the filter cake was washed with cold methyl *tert*-butyl ether. The filtrate was concentrated under reduced pressure, and the crude product was separated by silica gel column chromatography (eluent: DCM/MeOH (v/v) = 20/1) to give a white solid (1.70 g, 42%).

MS (ESI, pos.ion) m/z: 302.0 $[M+H]^+$.

**Step 2: Synthesis of *tert-butyl* 4-(4-(3-morpholinopropoxy)benzyl)-2,3-dihydro -1*H*-pyrrole-1-carboxylate**

4-(3-(4-Bromophenoxy)propyl)morpholine (1.70 g, 5.66 mmol), *tert-butyl* 3-methylenepyrrolidine-1-carboxylate (2.14 g, 11.33 mmol), $Ag_2CO_3$ (1.56 g, 5.66 mmol), DIPEA (3.0 mL, 17.34 mmol) and trans-bis-MU(M)-bis[2-(di-o-tolylphosphino)benzyl]acetate dipalladium(II) (265 mg, 0.28 mmol) were added to DMF (20 mL) under nitrogen protection, and the mixture was reacted at 100°C for 24 h. The reaction solution was filtered and concentrated under reduced pressure. The residue was diluted with EtOAc (100 mL), washed successively with $H_2O$ solution (30 mL) and saturated NaCl solution (30 mL), dried over anhydrous $Na_2SO_4$, and concentrated under reduced pressure. The crude product was separated by silica gel column chromatography (eluent: DCM/MeOH (v/v) = 20/1) to give brown liquid (1.66 g, 73%).

MS (ESI, pos.ion) m/z: 403.2 $[M+H]^+$.

**Step 3: Synthesis of *tert-butyl* 3-(4-(3-morpholinopropoxy)benzyl) pyrrolidine-1-carboxylate**

To a solution of *tert-butyl* 4-(4-(3-morpholinopropoxy)benzyl)-2,3-dihydro -1*H*-pyrrole-1-carboxylate (1.60 g, 3.98 mmol) in MeOH (10 mL) was added Pd/C (160 mg, 10%) and the mixture was stirred at room temperature for 24 h under hydrogen protection. The reaction solution was filtered, and the filtrate was concentrated under reduced pressure to give yellow liquid (1.61 g, 100%).

MS (ESI, pos.ion) m/z: 405.3 $[M+H]^+$.

**Step 4: Synthesis of 4-(3-(4-((pyrrolidin-3-yl)methyl)phenoxy)propyl)morpholine**

To a solution of *tert-butyl* 3-(4-(3-morpholinopropoxy)benzyl)pyrrolidine -1-carboxylate (1.60 g, 3.96 mmol) in DCM (10 mL) was added a solution of HCl in 1,4-dioxane (5.0 mL, 4 mol/L). The mixture was reacted at room temperature for 2 h. The reaction solution was concentrated under reduced pressure, and the residue was dissolved in DCM and an appropriate amount of TEA. The resulting mixture was concentrated under reduced pressure, and the crude product was separated by silica gel column chromatography (eluent: DCM/MeOH (v/v) = 4/1) to give light brown liquid (900 mg, 75%).

MS (ESI, pos.ion) m/z: 305.3 $[M+H]^+$.

**Step 5: Synthesis of ethyl 4-(3-(4-(3-morpholinopropoxy)benzyl)pyrrolidin-1-yl)benzoate**

Under nitrogen protection, 4-(3-(4-((pyrrolidin-3-yl)methyl)phenoxy)propyl) morpholine (900 mg, 2.96 mmol), $Pd_2(dba)_3$ (270 mg, 0.29 mmol), Ruphos (206 mg, 0.44 mmol), $Cs_2CO_3$ (963 mg, 2.96 mmol), ethyl 4-iodobenzoate (0.60 mL,3.57 mmol) were successively added to 1,4-dioxane (12 mL) and the mixture was reacted in a 100°C oil bath for 24 h. The reaction solution was cooled to room temperature and filtered through a celite pad, the filtrate was concentrated under reduced pressure, and the crude product was separated by silica gel column chromatography (eluent: DCM/MeOH (v/v) = 30/1) to give a pale brown solid (680 mg, 51%).

MS (ESI, pos.ion) m/z: 453.3 $[M+H]^+$.

**Step 6: Synthesis of 4-(3-(4-(3-morpholinopropoxy)benzyl)pyrrolidin-1-yl)benzoate hydrochloride**

To a solution of ethyl 4-(3-(4-(3-morpholinopropoxy)benzyl)pyrrolidin-1-yl)benzoate (680 mg, 1.50 mmol) in MeOH (3 mL) and THF (3 mL) was added a solution of $LiOH{\cdot}H_2O$ (330 mg, 8.30 mmol) in $H_2O$ (3 mL). The mixture was stirred at room temperature for 15 h. The reaction solution was concentrated under reduced pressure, and HCl solution (1 mol/L) was added to the remaining solution to adjust the pH of the solution to about 4. A large amount of insoluble solid was precipitated, which was filtered and dried *in vacuo* to obtain a pale yellow solid (500 mg, 72%).

MS (ESI, pos.ion) m/z: 462.1 $[M+H]^+$.

**Step 7: Synthesis of *N*-((*R*)-1-(4-(ethylsulfonyl)phenyl)-2-hydroxyethyl)-4-(3-(4-(3-morpholinopropoxy)benzyl)pyrrolidin-1-yl)benzamide**

HATU (250 mg, 0.66 mmol), (R)-2-amino-2-(4-(ethylsulfonyl)phenyl)ethanol (110 mg, 0.48 mmol), **4-(3-(4-(3-morpholinopropoxy)benzyl)pyrrolidin-1-yl)benzoate** hydrochloride (200 mg, 0.43 mmol) and TEA (0.25 mL, 1.80 mmol) were successively added to DCM (6 mL) and the mixture was stirred at room temperature for 20 h. The reaction solution was concentrated under reduced pressure. The residue was dissolved with DCM (40 mL), washed successively with $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/MeOH (v/v) = 10/1) to give a white solid (150 mg, 54%).
MS (ESI, pos.ion) m/z: 636.5 $[M+H]^+$.

**Step 8: Synthesis of *N*((*R*)-1-(4-(ethylsulfonyl)phenyl)-2-hydroxyethyl) -4-((*R*)-3-(3-morpholino)benzyl)pyrrolidin-1-yl)benzamide and *N*-((*R*)-1-(4-(ethylsulfonyl) phenyl)-2-hydroxyethyl)-4-((*S*)-3-(3-morpholino)benzyl)pyrrolidin-1-yl)benzamide**

*N*-((*R*)-1-(4-(Ethylsulfonyl)phenyl)-2-hydroxyethyl)-4-(3-(4-(3-morpholino)benzyl)py rrolidin-1-yl)benzamide (150 mg, 0.24 mmol) was resolved by chiral preparation to give diastereomer 105-1: *N*-((*R*)-1-(4-(ethylsulfonyl)phenyl)-2-hydroxyethyl)-4-((*R*)-3-(4-(3-morpholino)benzyl)pyrrolidin-1-yl)benzamide (40 mg, 27%) and 105-2: *N*-((*R*)-1-(4-(ethylsulfonyl)phenyl)-2-hydroxyethyl)-4-((*S*)-3-(4-(3-morpholino)benzyl)pyrrolidin-1-yl)benzamide (55 mg, 37%).
MS (ESI, pos.ion) m/z: 636.2 $[M+H]^+$;
105-1:
$^1$H NMR (400 MHz, $CDCl_3$) δ (ppm): 7.86 (d, *J* = 8.3 Hz, 2H), 7.70 (d, *J* = 8.7 Hz, 2H), 7.56 (d, *J* = 8.2 Hz, 2H), 7.10 (d, *J* = 8.4 Hz, 2H), 6.90 (d, *J* = 6.9 Hz, 1H), 6.84 (d, *J* = 8.5 Hz, 2H), 6.48 (d, *J* = 8.8 Hz, 2H), 5.28 (s, 1H), 4.06 - 3.93 (m, 4H), 3.75 - 3.68 (m, 4H), 3.47 - 3.36 (m, 2H), 3.32 (dd, *J* = 16.8, 8.3 Hz, 1H), 3.12 - 3.00 (m, 3H), 2.70 (d, *J* = 5.8 Hz, 2H), 2.62 - 2.55 (m, 1H), 2.55 - 2.50 (m, 2H), 2.47 (s, 3H), 2.16 - 2.07 (m, 1H), 2.00 - 1.92 (m, 2H), 1.81 - 1.73 (m, 2H), 1.27 (t, *J* = 7.4 Hz, 4H).
105-2:
$^1$H NMR (400 MHz, $CDCl_3$) δ (ppm): 7.83 (d, *J* = 8.1 Hz, 2H), 7.70 (d, *J* = 8.5 Hz, 2H), 7.55 (d, *J* = 8.0 Hz, 2H), 7.09 (d, *J* = 8.2 Hz, 2H), 6.97 (d, *J* = 6.6 Hz, 1H), 6.84 (d, *J* = 8.2 Hz, 2H), 6.47 (d, *J* = 8.5 Hz, 2H), 5.25 (s, 1H), 4.00 (t, *J* = 6.2 Hz, 3H), 3.93 (dd, *J* = 11.1, 4.8 Hz, 1H), 3.71 (s, 4H), 3.47 - 3.36 (m, 2H), 3.31 (dd, *J* = 16.5, 8.1 Hz, 1H), 3.05 (dt, *J* = 23.2, 8.2 Hz, 3H), 2.69 (d, *J* = 7.1 Hz, 2H), 2.61 - 2.50 (m, 3H), 2.47 (s, 3H), 2.11 (d, *J* = 6.0 Hz, 1H), 2.00- 1.93 (m, 2H), 1.77 (dd, *J* = 18.6, 10.1 Hz, 2H), 1.25 (d, *J* = 7.3 Hz, 4H).

**Example 106 *N*-((*R*)-1-(4-(ethylsulfonyl)phenyl)-2-hydroxyethyl)-4-((*R*)-3-(4-(2-morpholinoethoxy)benzyl)pyrrolidin-1-yl)benzamide / *N*-((*R*)-1-(4-(ethylsulfonyl)phenyl) -2-hydroxyethyl)-4-((*S*)-3-(4-(2-morpholinoethoxy)benzyl)pyrrolidin-1-yl)benzamide**

106-1:

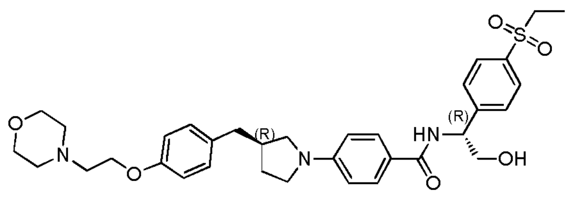

106-2:

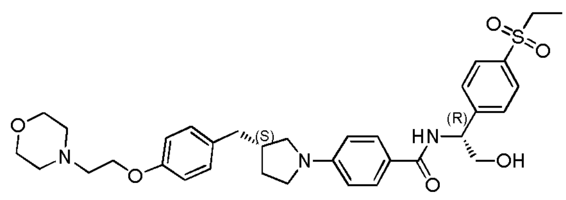

**Step 1: Synthesis of 4-(2-(4-bromophenoxy)ethyl)morpholine**

4-(2-Chloroethyl)morpholine hydrochloride (2.00 g, 10.43 mmol), 4-bromophenol (1.90 g, 10.76 mmol) and $K_2CO_3$ (3.00 g, 21.70 mmol) were added to ACN (30 mL). The mixture was reacted at 80°C for 24 h. The reaction solution was filtered and the filtrate was concentrated under reduced pressure, and the crude product was separated by silica gel column chromatography (eluent: DCM/EtOAc (v/v) = 2/1) to give pale yellow liquid (2.60 g, 87%).
MS (ESI, pos.ion) m/z: 288.1 $[M+H]^+$.

**Step 2: Synthesis of *tert*-butyl 4-(4-(2-morpholinoethoxy)benzyl)-2,3-dihydro-1*H*-pyrrole-1-carboxylate**

4-(2-(4-Bromophenoxy)ethyl)morpholine (2.50 g, 8.74 mmol), *tert-butyl* 3-methylenepyrrolidine-1-carboxylate (3.30 g, 17.47 mmol), $Ag_2CO_3$ (2.40 g, 8.70 mmol), DIPEA (4.5 mL, 26.01 mmol) and trans-bis-MU(M)-bis[2-(di-o-tolylphosphino)benzyl]acetate dipalladium(II) (440 mg, 0.47 mmol) were added to DMF (20 mL) under nitrogen protection, and the mixture was reacted at 100°C for 16 h. The reaction solution was filtered and concentrated under reduced pressure. The residue was diluted with EtOAc (100 mL), washed successively with $H_2O$ solution (30 mL) and saturated NaCl solution (30 mL), dried over anhydrous $Na_2SO_4$, and concentrated under reduced pressure. The crude product was separated by silica gel column chromatography (eluent: DCM/MeOH (v/v) = 20/1) to give brown liquid (2.08 g, 61%).
MS (ESI, pos.ion) m/z: 389.1 $[M+H]^+$.

**Step 3: Synthesis of *tert*-butyl 2-(4-(3-morpholinoethoxy)benzyl) pyrrolidine-1-carboxylate**

To a solution of *tert-butyl* 4-(4-(2-morpholinoethoxy)benzyl)-2,3-dihydro-1*H*-pyrrole-1-carboxylate (2.00 g, 5.15 mmol) in MeOH (10 mL) was added Pd/C (200 mg, 10%) and the mixture was stirred at room temperature for 24 h under hydrogen protection. The reaction solution was filtered, and the filtrate was concentrated under reduced pressure to give yellow liquid (2.01 g, 100%).
MS (ESI, pos.ion) m/z: 391.3 $[M+H]^+$.

**Step 4: Synthesis of 4-(2-(4-((pyrrolidin-3-yl)methyl)phenoxy)ethyl)morpholine**

To a solution of *tert-butyl* 3-(4-(2-morpholinoethoxy)benzyl)pyrrolidine-1-carboxylate (2.00 g, 5.12 mmol) in DCM (6 mL) was added a solution of HCl in 1,4-dioxane (6.0 mL, 4 mol/L). The mixture was reacted at room temperature for 24 h. The reaction solution was concentrated under reduced pressure, and the residue was dissolved in DCM and an appropriate amount of TEA. The resulting mixture was concentrated under reduced pressure, and the crude product was separated by silica gel column chromatography (eluent: DCM/MeOH (v/v) = 4/1) to give light brown liquid (1.40 g, 94%).
MS (ESI, pos.ion) m/z: 291.2 $[M+H]^+$.

**Step 5: Synthesis of ethyl 4-(3-(4-(2-morpholinoethoxy)benzyl)pyrrolidin-1-yl)benzoate**

Under nitrogen protection, 4-(2-(4-((pyrrolidin-3-yl)methyl)phenoxy)ethyl)morpholine (1.50 g, 5.17 mmol), $Pd_2(dba)_3$ (378 mg, 0.41 mmol), Ruphos (290 mg, 0.62 mmol), $Cs_2CO_3$ (1.18 g, 3.62 mmol) and ethyl 4-iodobenzoate (1.43 g, 5.18 mmol) were successively added to 1,4-dioxane (16 mL) and the mixture was reacted in a 100°C oil bath for 24 h. The reaction solution was cooled to room temperature and filtered through a celite pad, the filtrate was concentrated under reduced pressure, and the crude product was separated by silica gel column chromatography (eluent: DCM/MeOH (v/v) = 30/1) to give a pale brown solid (1.25 g, 55%).
MS (ESI, pos.ion) m/z: 439.3 $[M+H]^+$.

**Step 6: Synthesis of 4-(3-(4-(2-morpholinoethoxy)benzyl)pyrrolidin-1-yl)benzoic acid**

To a solution of ethyl 4-(3-(4-(3-morpholinoethoxy)benzyl)pyrrolidin-1-yl)benzoate (1.20 g, 2.74 mmol) in MeOH (4 mL) and THF (3 mL) was added a solution of $LiOH{\cdot}H_2O$ (500 mg, 12.57 mmol) in $H_2O$ (3 mL). The mixture was stirred at room temperature for 16 h. The reaction solution was concentrated under reduced pressure, and HCl solution (1 mol/L) was added to the remaining solution to adjust the pH of the solution to about 4. A large amount of insoluble solid was precipitated, which was filtered and dried *in vacuo* to obtain a pale yellow solid (900 mg, 74%).
MS (ESI, pos.ion) m/z: 448.1 $[M+H]^+$.

**Step 7: Synthesis of *N*((R*)*-1-(4-(ethylsulfonyl)phenyl)-2-hydroxyethyl)-4-(3-4-(2-morpholinoethoxy)benzyl)pyrrolidin-1-yl)benzamide**

HATU (380 mg, 1.00 mmol), (R)-2-amino-2-(4-(ethylsulfonyl)phenyl)ethanol (170 mg, 0.74 mmol), 4-(3-(4-(2-morpholinoethoxy)benzyl)pyrrolidin-1-yl)benzoic acid (300 mg, 0.67 mmol) and TEA (0.3 mL, 2.20 mmol) were successively added to DCM (8 mL) and the mixture was stirred at room temperature for 20 h. The reaction solution was concentrated under reduced pressure. The residue was dissolved with DCM (40 mL), washed successively with $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/MeOH (v/v) = 10/1) to give a white solid (200 mg, 48%).
MS (ESI, pos.ion) m/z: 622.3 $[M+H]^+$.

**Step 8: Synthesis of *N*((*R*)-1-(4-(ethylsulfonyl)phenyl)-2-hydroxyethyl)-4-((*R*)-3-(4-(2-morpholinoethoxy)benzyl)pyrrolidin-1-yl)benzamide / *N*-((*R*)-1-(4-(ethylsulfonyl) phenyl)-2-hydroxyethyl)-4-((*S*)-3-(4-(2-morpholinoethoxy)benzyl)pyrrolidin-l-yl)benzamide**

*N*-((*R*)-1-(4-(Ethylsulfonyl)phenyl)-2-hydroxyethyl)-4-(3-(4-(2-morpholinoethoxy)ben zyl)pyrrolidin-1-yl)benzamide (200 mg, 0.32 mmol) was resolved by chiral preparation to give diastereomer 106-1: *N*-((*R*)-1-(4-(ethylsulfonyl)phenyl)-2-hydroxyethyl)-4-((*R*)-3-(4-(2-morpholinoethoxy)benzyl)pyrrolidin-1-yl)benzamide (35 mg, 18%) and 106-2: *N*-((*R*)-1-(4-(ethylsulfonyl)phenyl)-2-hydroxyethyl)-4-((*S*)-3-(4-(2-morpholinoethoxy)benzyl)pyrrol idin-1-yl)benzamide (35 mg, 18%).
MS (ESI, pos.ion) m/z: 622.3 $[M+H]^+$.
106-1:
$^1H$ NMR (400 MHz, $CDCl_3$) δ (ppm): 7.85 (d, *J* = 8.1 Hz, 2H), 7.70 (d, *J* = 8.6 Hz, 2H), 7.56 (d, *J* = 8.1 Hz, 2H), 7.10 (d, *J* = 8.3 Hz, 2H), 6.93 (d, *J* = 6.8 Hz, 1H), 6.85 (d, *J* = 8.3 Hz, 2H), 6.47 (d, *J* = 8.6 Hz, 2H), 5.26 (s, 1H), 4.08 (s, 2H), 4.03 - 3.93 (m, 2H), 3.76 - 3.68 (m, 4H), 3.47 - 3.37 (m, 2H), 3.32 (dd, *J* = 16.5, 8.1 Hz, 1H), 3.11 - 2.97 (m, 3H), 2.80 (t, *J* = 5.5 Hz, 2H), 2.70 (d, *J* = 7.0 Hz, 2H), 2.58 (s, 5H), 1.27 (t, *J* = 7.2 Hz, 5H).
106-2:
$^1H$ NMR (400 MHz, $CDCl_3$) δ (ppm): 7.84 (d, *J* = 8.2 Hz, 2H), 7.70 (d, *J* = 8.6 Hz, 2H), 7.55 (d, *J* = 8.2 Hz, 2H), 7.10 (d, *J* = 8.4 Hz, 2H), 6.94 (d, *J* = 6.9 Hz, 1H), 6.85 (d, *J* = 8.4 Hz, 2H), 6.47 (d, *J* = 8.7 Hz, 2H), 5.26 (s, 1H), 4.10 (t, *J* = 5.6 Hz, 2H), 4.03 - 3.93 (m, 2H), 3.77 - 3.68 (m, 4H), 3.45 - 3.35 (m, 2H), 3.32 (dd, *J* = 16.6, 8.2 Hz, 1H), 3.11 - 3.00 (m, 3H), 2.80 (t, *J* = 5.6 Hz, 2H), 2.70 (d, *J* = 7.3 Hz, 2H), 2.58 (d, *J* = 4.2 Hz, 5H), 1.27 (t, *J* = 7.2 Hz, 5H).

**Example 107 4-((2*S*,4*S*)-2-((difluoromethoxy)methyl)-4-((5-trifluoromethoxy)pyridin -2-yl)oxy)pyrrolidin-1-yl)-*N*-(4-(ethylsulfonyl)benzyl)benzamide**

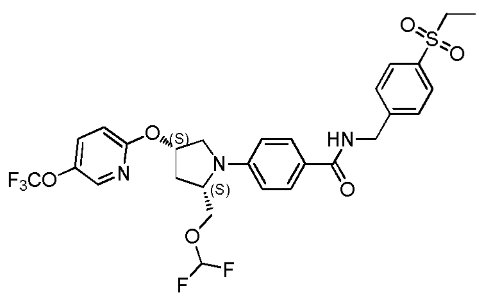

**Step 1: Synthesis of (2*S*,4*R*)-1-*tert*-butyl 2-methyl 4-(((benzyloxy)carbonyl)oxy)pyrrolidine-1,2-dicarboxylate**

To a solution of (2*S*,4*R*)-1-*tert*-butyl 2-methyl 4-hydroxypyrrolidine-1,2-dicarboxylate (20.00 g, 81.54 mmol) in DMAP (31.46 g, 244.60 mmol) and DCM (200 mL) was slowly added dropwise CbzCl (18.0 mL, 126.09 mmol) under an ice bath. The mixture was stirred at room temperature for 24 h. The reaction solution was filtered, and the filtrate was washed successively with saturated $NaHCO_3$ solution (100 mL) and saturated NaCl solution (100 mL), dried over anhydrous $Na_2SO_4$, concentrated under reduced pressure, and the concentrated solution was separated by silica gel column chromatography (eluent: PE/EtOAc (v/v) = 4/1) to give a white solid (30 g, 96%).
MS (ESI, pos.ion) m/z: 324.2 $[M-56+H]^+$.

**Step 2: Synthesis of *tert-butyl* (2*S*,4*R*)-4-(((benzyloxy)carbonyl)oxy)-2-(hydroxymethyl)pyrrolidine-1-carboxylate**

To THF (80 mL) solution was added (2*S*,4*R*)-1-*tert*-butyl 2-methyl 4-(((benzyloxy)carbonyl)oxy)pyrrolidine-1,2-dicarboxylate (28.30 g, 74.59 mmol), then the mixture was transferred to 0 °C, and a solution of $BH_3$ in THF solution (370 mL, 1 mol/L) was added. After the addition was completed, the mixture was reacted at 45 °C for 11 h. Under vigorous stirring, saturated $NH_4Cl$ (200 mL) solution was added to the reaction solution to quench the reaction. The mixture was left standing for layers, the organic phase was concentrated under reduced pressure, the aqueous phase was extracted with EtOAc (100 mL × 2). The combined organic phases were dissolved, washed with saturated NaCl solution (100 mL), dried over anhydrous $Na_2SO_4$, concentrated under reduced pressure, and the concentrated solution was separated by silica gel column chromatography (eluent: PE/EtOAc (v/v) = 3/2) to give colorless liquid (19.17 g, 73%).
MS (ESI, pos.ion) m/z: 296.2 $[M-56+H]^+$.

**Step 3: Synthesis of *tert-butyl* (2*S*,4*R*)-4-(((benzyloxy)carbonyl)oxy)-2-((difluoromethoxy)methyl)pyrrolidine-1-carboxylate**

To a solution of *tert*-butyl (2*S*,4*R*)-4-(((benzyloxy)carbonyl)oxy)-2-(hydroxymethyl) pyrrolidine-1-carboxylate (25.00 g, 71.15 mmol) in DCM (40 mL) were sequentially added $H_2O$ (40.0 mL), KOAc (28.00 g, 285.31 mmol) and $TMSCF_2Br$ (23.0 mL, 147.90 mmol). The mixture was stirred at room temperature for 16 h. The reaction solution was diluted with DCM (100 mL) and $H_2O$ (100 mL), extracted and separated. The organic phase was washed with saturated NaCl solution (50 mL), dried over anhydrous $Na_2SO_4$, concentrated under reduced pressure, and the concentrated solution was separated by silica gel column chromatography (eluent: PE/EtOAc (v/v) = 4/1) to give colorless liquid (20 g, 70%).
MS (ESI, pos.ion) m/z: 346.1 $[M-56+H]^+$.

**Step 4: Synthesis of benzyl ((3*R*,5*S*)-5-((difluoromethoxy)methyl) pyrrolidin-3-yl)carbonate**

To a solution of *tert*-butyl (2*S*,4*R*)-4-(((benzyloxy)carbonyl)oxy)-2-((difluoromethoxy)methyl)pyrrolidine-1-carboxylate (20.00 g, 49.83 mmol) in DCM (20 mL) was added a solution of HCl in 1,4-dioxane (30 mL, 4 mol/L). The mixture was stirred at room temperature for 24 h. The reaction solution was concentrated under reduced pressure, and the concentrated solution was diluted with EtOAc (80 mL), washed successively with saturated $Na_2CO_3$ solution (30 mL) and saturated NaCl solution (30 mL), dried over anhydrous $Na_2SO_4$, and concentrated under reduced pressure to give brown liquid (15.00 g, 100%).
MS (ESI, pos.ion) m/z: 302.3 $[M+H]^+$.

**Step 5: Synthesis of methyl 4-((2*S*,4*R*)-4-(((benzyloxy)carbonyl)oxy)-2-((difluoromethoxy)methyl)pyrrolidin-1-yl)benzoate**

Under nitrogen protection, benzyl ((3*R*,5*S*)-5-((difluoromethoxy)methyl)pyrrolidin -3-yl)carbonate (12.00 g, 39.83 mmol), $Pd_2(dba)_3$ (1.83 g, 2.00 mmol), Ruphos (1.40 g, 3.00 mmol), $Cs_2CO_3$ (15.00 g, 46.04 mmol) and methyl 4-iodobenzoate (10.44 g, 39.84 mmol) were successively added to 1,4-dioxane (140 mL) and the mixture was reacted at 100 °C for 20 h. The reaction solution was cooled to room temperature, filtered through a celite pad, the filtrate was concentrated under reduced pressure, and the concentrated solution was separated by silica gel column chromatography (eluent: PE/EtOAc (v/v) = 4/1) to give pale yellow liquid (14.84 g, 86%).
MS (ESI, pos.ion) m/z: 436.2 $[M+H]^+$.

**Step 6: Synthesis of methyl 4-((2*S*,4*R*)-2-((difluoromethoxy)methyl) -4-hydroxypyrrolidin-1-yl)benzoate**

To a solution of methyl 4-((2*S*,4*R*)-4-(((benzyloxy)carbonyl)oxy) -2-((difluoromethoxy)methyl)pyrrolidin-1-yl)benzoate (15.00 g, 34.45 mmol) in MeOH (60 mL) was added Pd/C (1.00 g, 10%). The mixture was degassed and refilled with hydrogen, and was reacted at room temperature for 24 h. The reaction solution was filtered through a celite pad, and concentrated under reduced pressure, and the concentrated solution was separated by silica gel column chromatography (eluent: PE/EtOAc (v/v) = 3/2) to give pale yellow liquid (10.0 g, 96%).
MS (ESI, pos.ion) m/z: 302.2 $[M+H]^+$.

**Step 7: Synthesis of methyl 4-((2*S*,4*R*)-2-((difluoromethoxy)methyl)-4-((methylsulfonyl)oxy)pyrrolidin-1-yl)benzoate**

Methyl 4-((2*S*,4*R*)-2-((difluoromethoxy)methyl)-4-hydroxypyrrolidin-1-yl)benzoate (1.00 g, 3.32 mmol), MsCl (0.40 mL, 5.16 mmol), DMAP (40 mg, 0.33 mmol) and TEA (1.40 mL, 10.07 mmol) were added to DCM (12 mL). The mixture was stirred at room temperature for 11 h. The reaction solution was concentrated under reduced pressure, and the crude product was separated by silica gel column chromatography (eluent: PE/EtOAc (v/v) = 3/2) to give pale yellow liquid (1.20 g, 95%).

MS (ESI, pos.ion) m/z: 380.1 $[M+H]^+$.

**Step 8: Synthesis of methyl 4-((2*S*,4*S*)-2-((difluoromethoxy)methyl)-4-((5-(trifluoromethoxy)pyridin-2-yl)oxy)pyrrolidin-1-yl)benzoate**

Methyl 4-((2*S*,4*R*)-2-((difluoromethoxy)methyl)-4-((methylsulfonyl)oxy) pyrrolidin-1-yl)benzoate (1.20 g, 3.16 mmol), 5-(trifluoromethoxy)pyridin-2-ol (623 mg, 3.48 mmol) and $K_2CO_3$ (900 mg, 6.32 mmol) were added to DMF (8 mL) and the mixture was reacted at 70 °C for 16 h. The reaction solution was cooled to room temperature, diluted with EtOAc (40 mL), washed with $H_2O$ (15 mL × 2) and saturated NaCl solution (15 mL), dried over anhydrous $Na_2SO_4$, concentrated under reduced pressure, and the crude product was separated by silica gel column chromatography (eluent: PE/EtOAc (v/v) = 4/1) to give pale yellow liquid (1.20 g, 82%).

MS (ESI, pos.ion) m/z: 463.3 $[M+H]^+$.

**Step 9: Synthesis of 4-((2*S*,4*S*)-2-((difluoromethoxy)methyl)-4-((5-(trifluoromethoxy) pyridin-2-yl)oxy)pyrrolidin-1-yl)benzoic acid**

To a solution of methyl 4-((2*S*,4*S*)-2-((difluoromethoxy)methyl)-4-((5-(trifluoromethoxy)pyridin-2-yl)oxy)pyrrolidin-1-yl)benzoate (1.20 g, 2.60 mmol) in MeOH (4 mL) and THF (4 mL) was added a solution of $LiOH.H_2O$ (653 mg, 15.56 mmol) in $H_2O$ (4 mL). The mixture was reacted at 50°C for 24 h. The reaction solution was concentrated under reduced pressure, and the remaining solution was added with HCl solution (1 mol/L) to adjust the pH of the solution to about 4. The resulting mixture was extracted with EtOAc (50 mL), washed with saturated NaCl (20 mL) solution, dried over anhydrous $Na_2SO_4$, concentrated under reduced pressure, and the crude product was separated by silica gel column chromatography (eluent: PE/EtOAc (v/v) = 3/2) to give a white solid (1.00 g, 86%).

MS (ESI, pos.ion) m/z: 449.2 $[M+H]^+$.

**Step 10: Synthesis of 4-((2*S*,4*S*)-2-((difluoromethoxy)methyl)-4-((5-trifluoromethoxy) pyridin-2-yl)oxy)pyrrolidin-1-yl)-*N*-(4-(ethylsulfonyl)benzyl)benzamide**

HATU (170 mg, 0.45 mmol), (4-(ethylsulfonyl)phenyl)methanamine (80 mg, 0.40 mmol), 4-((2*S*,4*S*)-2-((difluoromethoxy)methyl)-4-((5-(trifluoromethoxy)pyridin-2-yl)oxy) pyrrolidin-1-yl)benzoic acid (150 mg , 0.33 mmol) and TEA (101 mg, 1.00 mmol) were successively added to DCM (6 mL) and the mixture was reacted at room temperature for 20 h. The reaction solution was concentrated under reduced pressure, the residue was diluted with DCM (50 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the crude product was separated by silica gel column chromatography (eluent: DCM/EtOAc (v/v) = 4/1) to give a white solid (100 mg, 47%).

MS (ESI, pos.ion) m/z: 630.2 $[M+H]^+$;

$^1H$ NMR (400 MHz, $CDCl_3$) δ (ppm): 8.11 (d, *J* = 2.2 Hz, 1H), 7.82 (d, *J* = 8.3 Hz, 2H), 7.75 (d, *J* = 8.8 Hz, 2H), 7.50 (d, *J* = 8.3 Hz, 3H), 6.77 (d, *J* = 9.0 Hz, 1H), 6.66 (d, *J*= 8.8 Hz, 2H), 6.60 (t, *J* = 5.7 Hz, 1H), 6.24 (t, *J* = 74.4 Hz, 1H), 5.74 (s, 1H), 4.71 (d, *J* = 5.9 Hz, 2H), 4.23 - 4.15 (m, 2H), 3.98 (t, *J* = 9.3 Hz, 1H), 3.72 (d, *J* = 2.7 Hz, 2H), 3.08 (q, *J* = 7.3 Hz, 2H), 2.49 - 2.36 (m, 2H), 1.26 (t, *J* = 7.4 Hz, 3H).

**Example 108 4-((2*S*,4*S*)-2-((difluoromethoxy)methyl)-4-((5-trifluoromethoxy)pyridin-2-yl) oxy)pyrrolidin-1-yl)-*N*-((*S*)-1-(4-(ethylsulfonyl)phenyl)-3-hydroxypropyl)benzamide**

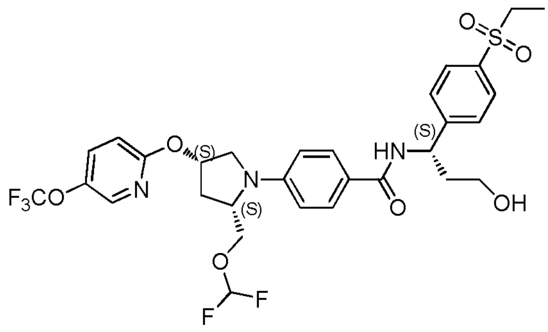

**Step 1: Synthesis of *tert-butyl* (*S*)-(1-(4-(ethylsulfonyl)phenyl) -3-hydroxypropyl)carbamate**

To a solution of methyl (*S*)-3-(((*tert*-butoxy)carbonyl)amino)-3-(4-(ethylsulfonyl)phenyl)propionate (2.00 g, 5.39 mmol) in THF (12 mL) was added $LiBH_4$ (176 mg, 8.08 mmol) under nitrogen protection. The mixture was reacted at room temperature for 14 h. The reaction solution was slowly poured into saturated $NH_4Cl$ solution (20 mL) to quench the reaction. The resulting mixture was concentrated under reduced pressure, and the concentrated solution was diluted with EtOAc (50 mL), washed with saturated NaCl solution (20 mL), dried over anhydrous $Na_2SO_4$ and concentrated under reduced pressure. The concentrated solution was separated by silica gel column chromatography (eluent: DCM/EtOAc (v/v) = 3/2) to give a white solid (1.80 g, 97%).

MS (ESI, pos.ion) m/z: 288.2 $[M-56+H]^+$.

**Step 2: Synthesis of (*S*)-3-amino-3-(4-(ethylsulfonyl)phenyl)propan-1-ol hydrochloride**

To a solution of *tert*-butyl (*S*)-(1-(4-(ethylsulfonyl)phenyl)-3-hydroxypropyl)carbamate (300 mg, 0.87 mmol) in DCM (4 mL) was added a solution of HCl in 1,4-dioxane (1.0 mL, 4 mmol/L). The mixture was reacted at room temperature for 6 h. The resulting mixture was concentrated under reduced pressure to give a white solid (244 mg, 100%).

MS (ESI, pos.ion) m/z: 244.2 $[M+H]^+$.

**Step 3: Synthesis of 4-((2*S*,4*S*)-2-((difluoromethoxy)methyl)-4-(5-(trifluoromethoxy)pyridin-2-yl)oxy)pyrrolidin-1-yl)-*N*-((*S*)-1-(4-(ethylsulfonyl)phenyl)-3-hydr oxypropyl)benzamide**

HATU (275 mg, 0.72 mmol), **(*S*)-3-amino-3-(4-(ethylsulfonyl)phenyl)propan-1-ol** hydrochloride (171 mg, 0.610 mmol), 4-((2*S*,4*S*)-2-((difluoromethoxy)methyl) -4-((5-(trifluoromethoxy)pyridin-2-yl)oxy)pyrrolidin-1-yl)benzoic acid (250 mg , 0.56 mmol) and TEA (170 mg, 1.68 mmol) were successively added to DCM (6 mL) and the mixture was reacted at room temperature for 24 h. The reaction solution was concentrated under reduced pressure, the concentrated solution was diluted with DCM (50 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/EtOAc (v/v) = 1/1) to give a white solid (130 mg, 35%).

MS (ESI, pos.ion) m/z: 674.2 $[M+H]^+$;

$^1H$ NMR (400 MHz, $CDCl_3$) δ (ppm): 8.10 (d, *J* = 2.7 Hz, 1H), 7.85 (d, *J* = 8.3 Hz, 2H), 7.74 (d, *J* = 8.8 Hz, 2H), 7.55 (d, *J* = 8.3 Hz, 2H), 7.50 (dd, *J* = 9.0, 2.1 Hz, 1H), 7.33 (d, *J* = 7.3 Hz, 1H), 6.77 (d, *J* = 9.0 Hz, 1H), 6.64 (d, *J* = 8.8 Hz, 2H), 6.24 (t, *J* = 74.3 Hz, 1H), 5.74 (d, *J* = 3.6 Hz, 1H), 5.48 - 5.43 (m, 1H), 4.22 - 4.11 (m, 2H), 3.97 (t, *J*= 9.4 Hz, 1H), 3.77 (s, 1H), 3.71 - 3.64 (m, 3H), 3.09 (q, J = 7.4 Hz, 2H), 2.89 (s, 1H), 2.47 (d, *J* = 14.4 Hz, 1H), 2.44 - 2.35 (m, 1H), 2.29 - 2.19 (m, 1H), 1.99- 1.89 (m, 1H), 1.27 (t, *J* = 7.4 Hz, 3H).

**Example 109 *N*-(4-(ethylsulfonyl)benzyl)-4-((2*S*,4*S*)-2-(3,3,3-$d_3$-methoxymethyl)-4-(4-(trifluoromethyl)phenoxy)pyrrolidin-1-yl)benzamide**

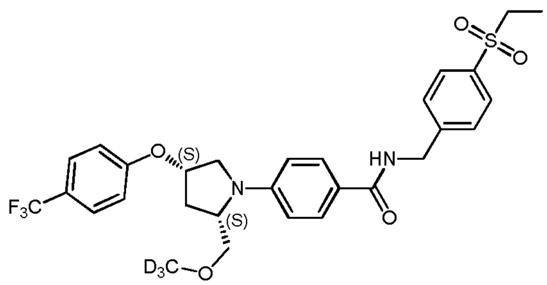

**Step 1: Synthesis of ethyl 4-((2*S*,4*S*)-2-(hydroxymthyl)-4-(4-(trifluoromethyl) phenoxy)pyrrolidin-1-yl)benzoate**

4-((2*S*,4*S*)-2-((Difluoromethoxy)methyl)-4-(4-(trifluoromethyl)phenoxy)pyrrolidin-l-y 1)benzoic acid (2.02 g, 4.68 mmol) was dissolved in a solution of HCl in ethanol (10 mL, 4.0 M). The reaction solution was reacted at room temperature for 8 h. After the reaction was completed, the reaction solution was concentrated under reduced pressure, then $NaHCO_3$ solution (50 mL) was added. The resulting mixture was extracted with DCM (30 mL). The organic phase was dried over anhydrous $Na_2SO_4$, filtered and concentrated under reduced pressure. The crude product was separated by silica gel column chromatography (eluent: PE/EtOAc (v/v) = 1/1) to give a white solid (980 mg, 51%).

MS (ESI, pos.ion) m/z: 410.2 $[M+H]^+$.

**Step 2: Synthesis of ethyl 4-((2*S*,4*S*)-2-(((methylsulfonyl)oxy)methyl)-4-(4-trifluoromethyl)phenoxy)pyrrolidin-1-yl)benzoate**

Ethyl 4-((2*S*,4*S*)-2-(hydroxymethyl)-4-(4-(trifluoromethyl)phenoxy)pyrrolidin-1-yl) benzoate (500 mg, 1.22 mmol ) and TEA (0.40 mL, 2.7 mmol) were dissolved in DCM (10 mL, 156 mmol), then MsCl (0.2 mL, 3 mmol) was added dropwise. The reaction solution was stirred at room temperature for 10 h. After the reaction was completed, the mixture was concentrated under reduced pressure, and the crude product was separated by silica gel column chromatography (eluent: DCM/EtOAc (v/v) = 5/1) to give pale yellow liquid (500 mg, 84%).

MS (ESI, pos.ion) m/z: 488.1 $[M+H]^+$.

**Step 3: Synthesis of ethyl 4-((2*S*,4*S*)-2-(3,3,3-*d*$_3$-methoxymethyl)-4-(4-(trifluoromethyl)phenoxy)pyrrolidin-1-yl)benzoate**

Ethyl 4-((2*S*,4*S*)-2-(((methylsulfonyl)oxy)methyl)-4-(4-(trifluoromethyl)phenoxy) pyrrolidin-1-yl)benzoate (800 mg, 1.64 mmol) was dissolved in $CD_3OD$ (0.2 mL, 5 mmol). Then NaH (35 mg, 0.88 mmol) was added, and the reaction solution was stirred at room temperature. After the reaction was completed, the stirring was stopped, the reaction solution was concentrated under reduced pressure, and the residue was diluted with DCM (20 mL), then HCl solution (15 mL, 0.1 mol/L) was added. The aqueous phase was extracted with DCM (30 mL$\times$2), and the combined organic phases were washed with saturated NaCl solution (15 mL), dried over anhydrous $Na_2SO_4$, filtered and concentrated under reduced pressure. The crude product was separated and purified by silica gel column chromatography (eluent: PE/EtOAc (v/v) = 1/1) to give a white solid (500 mg, 75%).

MS (ESI, pos.ion) m/z: 427.2 $[M+H]^+$.

**Step 4: Synthesis of 4-((2*S*,4*S*)-2-(3,3,3-*d*$_3$-methoxy)methyl)-4-(4-(trifluoromethyl) phenoxy)pyrrolidin-1-yl)benzoic acid**

To a solution of ethyl 4-((2*S*,4*S*)-2-(3,3,3-*d*$_3$-methoxymethyl)-4-(4-(trifluoromethyl)phenoxy)pyrrolidin-1-yl)benzoate (500 mg, 1.18 mmol) in MeOH (10 mL) and $H_2O$ (2 mL) were added LiOH (150 mg, 3.5 mmol). The mixture was reacted at room temperature. The reaction solution was added with HCl solution (2.0 mol/L) to adjust the pH of the solution to about 5. The resulting mixture was extracted with EtOAc (25 mL $\times$ 2), and the organic phases were combined, washed with saturated NaCl (20 mL) solution, dried over anhydrous $Na_2SO_4$, concentrated under reduced pressure, and the crude product was separated by silica gel column chromatography (eluent: DCM/MeOH (v/v) = 10/1) to give a brown solid (400 mg, 82%).

MS (ESI, pos.ion) m/z: 399.1 $[M+H]^+$.

**Step 5: Synthesis of *N*-(4-(ethylsulfonyl)benzyl)-4-((2*S*, 4*S*)-2-(3,3,3-$d_3$-methoxymethyl)-4-(4-(trifluoromethyl)phenoxy)pyrrolidin-1-yl)benzamide**

4-((2*S*,4*S*)-2-(3,3,3-$d_3$-methoxymethyl)-4-(4-(trifluoromethyl)phenoxy)pyrrolidin-1-yl) benzoic acid (100 mg, 0.25 mmol), (4-(ethylsulfonyl)phenyl)methanamine (76 mg, 0.38 mmol), HATU (128 mg, 0.33 mmol) and DIPEA (0.1 mL, 0.56 mmol) were dissolved in DCM (15 mL). The mixture was stirred at room temperature for 16 h. The reaction solution was diluted with DCM (10 mL), and the resulting mixture was washed successively with HCl solution (20 mL, 0.1 mol/L), saturated $NaHCO_3$ solution (20 mL) and saturated NaCl solution (15 mL), dried over anhydrous $Na_2SO_4$, filtered and concentrated under reduced pressure. The crude product was separated by silica gel column chromatography (eluent: DCM/EtOAc (v/v) = 1/1) to give a white solid (44 mg, 30%).

MS (ESI, pos.ion) m/z: 580.2 $[M+H]^+$;

$^1H$ NMR (600 MHz, $CDCl_3$) $\delta$ (ppm): 7.85 (d, *J* = 8.2 Hz, 2H), 7.74 (d, *J* = 8.9 Hz, 2H), 7.62 (d, *J* = 8.8 Hz, 2H), 7.56 (t, *J* = 8.2 Hz, 2H), 6.96 (d, *J* = 8.6 Hz, 2H), 6.64 (d, *J* = 8.6 Hz, 2H), 5.58 (dd, *J* = 13.5, 6.4 Hz, 1H), 5.13 (t, *J* = 4.8 Hz, 1H), 4.15 - 4.08 (m, 1H), 3.74 - 3.63 (m, 2H), 3.46 (d, *J* = 9.1 Hz, 1H), 3.11 - 3.05 (m, 4H), 2.46 (d, *J*= 14.2 Hz, 1H), 2.38 - 2.33 (m, 1H), 1.26 (t, *J*= 7.4 Hz, 3H).

**Example 110 6-((2*S*,4*S*)-2-((difluoromethoxy)methyl)-4-((5-trifluoromethoxy)pyridin-2-yl) oxy)pyrrolidin-1-yl)-*N*-(4-(ethylsulfonyl)benzyl)nicotinamide**

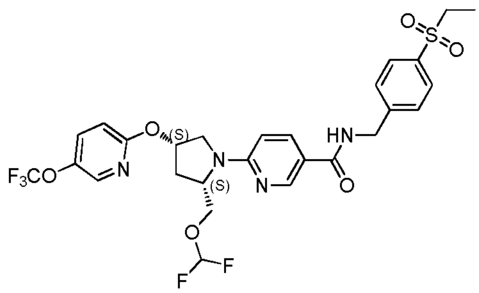

**Step 1: Synthesis of (2*S*,4*R*)-1-benzyl 2-methyl 4-((methylsulfonyl)oxy)pyrrolidine-1,2-dicarboxylate**

To a solution of (2*S*,4*R*)-1-benzyl 2-methyl 4-hydroxypyrrolidine-1,2-dicarboxylate (10.00 g, 34.73 mmol) in DCM (120 mL) were added MsCl (4.0 mL, 51.64 mmol), TEA (9.65 mL, 69.43 mmol) and DMAP (424 mg, 3.47 mmol) in turn. The mixture was stirred at room temperature for 21 h. The reaction solution was washed successively with $H_2O$ (80 mL $\times$ 2) and saturated NaCl solution (80 mL), dried over anhydrous $Na_2SO_4$, and concentrated under reduced pressure to give brown liquid (12.4 g, 100%).

MS (ESI, pos.ion) m/z: 358.6 $[M+H]^+$.

**Step 2: Synthesis of benzyl (2*S*,4*R*)-2-(hydroxymethyl)-4-((methylsulfonyl) oxy)pyrrolidine-1-carboxylate**

To a solution of (2*S*,4*R*)-1-benzyl 2-methyl 4-((methylsulfonyl)oxy)pyrrolidine-1,2-dicarboxylate (12.0 g, 33.58 mmol) in THF (100 mL) was added $LiBH_4$ (1.21 g, 55.56 mmol), and the mixture was reacted for 6 h at room temperature. Under vigorous stirring, the reaction solution was slowly poured into cooled saturated $NH_4Cl$ solution (100 mL) to quench the reaction. The mixture was concentrated under reduced pressure, the remaining aqueous phase was extracted with EtOAc (80 mL $\times$ 2), washed with saturated NaCl solution (60 mL), dried over anhydrous $Na_2SO_4$, concentrated under reduced pressure to give pale yellow liquid (11.1 g, 100%).

MS (ESI, pos.ion) m/z: 330.0 $[M+H]^+$.

**Step 3: Synthesis of benzyl (2*S*,4*R*)-2-((difluoromethoxy)methyl)-4-((methylsulfonyl)oxy)pyrrolidin-1-carboxylate**

To a solution of benzyl (2*S*,4*R*)-2-(hydroxymethyl)-4-((methylsulfonyl)oxy) pyrrolidine-1-carboxylate (11.00 g, 33.39 mmol) in DCM (30 mL) was added a solution of KOAc (16.40 g, 167.11 mmol) in $H_2O$ (30 mL), then $TMSCF_2Br$ (16.0 mL, 102.89 mmol) was added under an ice-water bath, and the mixture was heated to room temperature and stirred for 14 h. The reaction solution was added with DCM (100 mL) and $H_2O$ (100 mL). The organic phase was separated

and washed with saturated NaCl solution (60 mL), dried over anhydrous $Na_2SO_4$, concentrated under reduced pressure, and the crude product was separated by silica gel column chromatography (eluent: PE/EtOAc (v/v) = 1/1) to give pale yellow liquid (9.0 g, 71%).

MS (ESI, pos.ion) m/z: 380.1 $[M+H]^+$.

**Step 4: Synthesis of benzyl (2*S*,4*S*)-2-((difluoromethoxy)methyl)-4-((5-(trifluoromethoxy)pyridin-2-yl)oxy)pyrrolidin-1-carboxylate**

Benzyl (2*S*,4*R*)-2-((difluoromethoxy)methyl)-4-((methylsulfonyl)oxy)pyrrolidin -1-carboxylate (4.20 g, 11.07 mmol), 5-(trifluoromethoxy)pyridin-2-ol (1.80 g, 10.05 mmol) and $K_2CO_3$ (2.80 g, 20.26 mmol) were added to DMF (20 mL) and the mixture was reacted at 60 °C for 16 h. The reaction solution was diluted with EtOAc (100 mL), washed with $H_2O$ (50 mL × 2) and saturated NaCl solution (40 mL), dried over anhydrous $Na_2SO_4$, concentrated under reduced pressure, and the crude product was separated by silica gel column chromatography (eluent: PE/EtOAc (v/v) = 4/1) to give pale yellow liquid (3.57 g, 77%).

MS (ESI, pos.ion) m/z: 463.1 $[M+H]^+$.

**Step 5: Synthesis of 2-(((3*S*,5*S*)-5-((difluoromethoxy)methyl)pyrrolidin-3-yl) oxy)-5-(trifluoromethoxy)pyridine**

To a solution of benzyl (2*S*,4*S*)-2-((difluoromethoxy)methyl)-4-((5-(trifluoromethoxy)pyridin-2-yl)oxy)pyrrolidine-1-carboxylate (3.57 g, 7.72 mmol) in MeOH (30 mL) was added Pd/C (400 mg, 10%) under hydrogen protection. The mixture was reacted at room temperature for 15 h. The reaction solution was filtered through a celite pad, and the filtrate was concentrated under reduced pressure to give pale yellow liquid (2.38 g, 94%).

MS (ESI, pos.ion) m/z: 329.2 $[M+H]^+$.

**Step 6: Synthesis of methyl 6-((2*S*,4*S*)-2-((difluoromethoxy)methyl)-4-((5-(trifluoromethoxy)pyridin-2-yl)oxy)pyrrolidin-1-yl)nicotinate**

Under nitrogen protection, 2-((3*S*,5*S*)-5-((difluoromethoxy)methyl)pyrrolidin -3-yl)oxy)-5-(trifluoromethoxy)pyridine (200 mg, 0.61 mmol), $K_2CO_3$ (170 mg, 1.23 mmol) and methyl 6-fluoronicotinate (122 mg, 0.78 mmol) were added to DMF (4 mL) in turn and the mixture was reacted at 80 °C for 22 h. The reaction solution was diluted with EtOAc (50 mL), washed successively with $H_2O$ (20 mL × 2) and saturated NaCl solution (20 mL), dried over anhydrous $Na_2SO_4$, concentrated under reduced pressure, and the crude product was separated by silica gel column chromatography (eluent: PE/EtOAc (v/v) = 4/1) to give pale yellow liquid (200 mg, 71%).

MS (ESI, pos.ion) m/z: 464.0 $[M+H]^+$.

**Step 7: Synthesis of 6-((2*S*,4*S*)-2-((difluoromethoxy)methyl)-4-((5-(trifluoromethoxy)pyridin-2-yl)oxy)pyrrolidin-1-yl)nicotinic acid**

To a solution of methyl 6-((2*S*,4*S*)-2-((difluoromethoxy)methyl)-4-((5-(trifluoromethoxy)pyridin-2-yl)oxy)pyrrolidin-1-yl)nicotinate (200 mg, 0.43 mmol) in MeOH (2 mL) and THF (2 mL) was added a solution of LiOH (103 mg, 4.30 mmol) in $H_2O$ (1 mL). The mixture was stirred at room temperature for 16 h. The reaction solution was concentrated under reduced pressure, and the remaining solution was added with HCl solution (1 mol/L) to adjust the pH of the solution to about 4. The resulting mixture was extracted with EtOAc (20 mL), washed with saturated NaCl (10 mL) solution, dried over anhydrous $Na_2SO_4$, concentrated under reduced pressure, and the crude product was separated by silica gel column chromatography (eluent: PE/EtOAc (v/v) = 3/2) to give a white solid (149 mg, 77%).

MS (ESI, pos.ion) m/z: 450.1 $[M+H]^+$.

**Step 8: Synthesis of 6-((2*S*,4*S*)-2-((difluoromethoxy)methyl)-4-((5-trifluoromethoxy)pyridin-2-yl)oxy)pyrrolidin-1-yl)-*N*-(4-(ethylsulfonyl)benzyl)nicotinamide**

HATU (163 mg, 0.43 mmol), (4-(ethylsulfonyl)phenyl)methanamine hydrochloride (85 mg, 0.36 mmol), 6-((2*S*,4*S*)-2-((difluoromethoxy)methyl)-4-((5-(trifluoromethoxy)pyridin -2-yl)oxy)pyrrolidin-1-yl)nicotinic acid (149 mg , 0.33 mmol) and TEA (100 mg, 0.99 mmol) were successively added to DCM (6 mL) and the mixture was reacted at room temperature for 16 h. The reaction solution was concentrated under reduced pressure, the residue was diluted with DCM (40 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the crude product was separated by silica gel column chromatography (eluent: PE/EtOAc (v/v) = 1/1) to give a white solid (150 mg, 72%).

MS (ESI, pos.ion) m/z: 631.9 $[M+H]^+$.

$^1H$ NMR (400 MHz, $CDCl_3$) δ (ppm): 8.66 (d, *J* = 1.9 Hz, 1H), 8.09 (d, *J* = 2.0 Hz, 1H), 7.96 (dd, *J* = 8.8, 2.2 Hz, 1H), 7.75 (d, *J* = 8.2 Hz, 2H), 7.50 - 7.45 (m, 3H), 6.83 (t, *J* = 5.7 Hz, 1H), 6.77 (d, *J* = 9.0 Hz, 1H), 6.44 (d, *J* = 9.0 Hz, 1H), 6.24 (t, *J* = 74.9 Hz, 1H), 5.72 (s, 1H), 4.70 (d, *J* = 5.8 Hz, 2H), 4.53 (s, 1H), 4.32 (dd, *J* = 9.3, 4.2 Hz, 1H), 3.99 (t, *J* = 9.3 Hz, 1H), 3.86 (dd, *J* = 12.2, 4.9 Hz, 1H), 3.76 (d, *J* = 12.2 Hz, 1H), 3.07 (q, *J* = 7.4 Hz, 2H), 2.49 (d, *J* = 14.2 Hz, 1H), 2.44 - 2.34 (m, 1H), 1.24 (t, *J* = 7.4 Hz, 3H).

**Example 111 6-((2*S*,4*S*)-2-((difluoromethoxy)methyl)-4-((5-trifluoromethoxy)pyridin-2-yl) oxy)pyrrolidin-1-yl)-*N*-(4-(ethylsulfonyl)benzyl)-5-fluoronicotinamide**

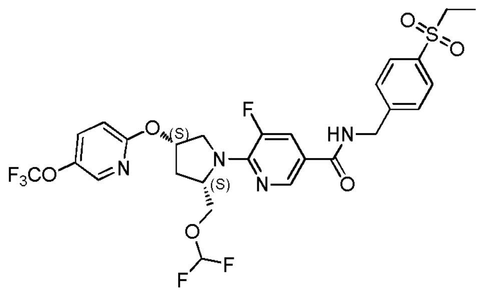

Using methyl 6-chloro-5-fluoronicotinate as raw material, the title compound was prepared as a white solid (105 mg, 76%) by referring to the methods of step 1 to step 8 of Example 110.

MS (ESI, pos.ion) m/z: 649.1 $[M+H]^+$.

$^1H$ NMR (400 MHz, $CDCl_3$) δ (ppm): 8.42 (s, 1H), 8.09 (d, *J* = 2.3 Hz, 1H), 7.78 (d, *J* = 8.2 Hz, 2H), 7.72 (dd, *J* = 13.9, 1.6 Hz, 1H), 7.50 (s, 1H), 7.47 (d, *J* = 8.2 Hz, 2H), 6.77 (d, *J* = 9.0 Hz, 1H), 6.73 (d, *J* = 5.8 Hz, 1H), 6.21 (t, *J* = 74.7 Hz, 1H), 5.68 (s, 1H), 4.75 (s, 1H), 4.71 (d, *J* = 5.9 Hz, 2H), 4.25 (dd, *J*= 9.3, 4.2 Hz, 1H), 4.17 - 4.10 (m, 1H), 4.06 (d, *J* = 13.1 Hz, 1H), 4.00 (t, *J* = 9.3 Hz, 1H), 3.09 (q, *J* = 7.4 Hz, 2H), 2.45 (d, *J* = 14.1 Hz, 1H), 2.41 - 2.33 (m, 1H), 1.26 (t, *J* = 7.4 Hz, 3H).

**Example 112 4-((2*S*,4*S*)-2-((difluoromethoxy)methyl)-4-((5-trifluoromethoxy)pyridin -2-yl)oxy)pyrrolidin-1-yl)-*N*-((5-(ethylsulfonyl)pyridin-2-yl)methyl)benzamide**

HATU (190 mg, 0.50 mmol), (5-(ethylsulfonyl)pyridin-2-yl)methanamine hydrochloride (103 mg, 0.44 mmol), 4-((2*S*,4*S*)-2-((difluoromethoxy)methyl) -4-((5-(trifluoromethoxy)pyridin-2-yl)oxy)pyrrolidin-1-yl)benzoic acid (150 mg , 0.33 mmol) and TEA (101 mg, 1.00 mmol) were successively added to DCM (6 mL) and the mixture was reacted at room temperature for 23 h. The reaction solution was concentrated under reduced pressure, the residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/EtOAc (v/v) = 3/1) to give a white solid (160 mg, 76%).

MS (ESI, pos.ion) m/z: 631.1 $[M+H]^+$;

$^1H$ NMR (400 MHz, $CDCl_3$) δ (ppm): 9.03 (d, *J* = 1.6 Hz, 1H), 8.14 (dd, *J* = 8.2, 2.1 Hz, 1H), 8.11 (d, *J* = 2.3 Hz, 1H), 7.79 (d, *J* = 8.7 Hz, 2H), 7.55 (d, *J* = 8.2 Hz, 1H), 7.52 - 7.47 (m, 1H), 7.21 (t, *J* = 4.9 Hz, 1H), 6.77 (d, *J* = 9.0 Hz, 1H), 6.67 (d, *J* = 8.8 Hz, 2H), 6.24 (t, *J* = 74.4 Hz, 1H), 5.75 (s, 1H), 4.85 (d, *J*= 5.2 Hz, 2H), 4.23 - 4.16 (m, 2H), 3.98 (t, *J*= 9.4 Hz, 1H), 3.72 (d, *J*= 2.7 Hz, 2H), 3.15 (q, *J* = 7.4 Hz, 2H), 2.48 (d, *J* = 14.4 Hz, 1H), 2.45 - 2.36 (m, 1H), 1.31 (t, *J* = 7.4 Hz, 3H).

**Example 113 6-((2*S*,4*S*)-4-((5-chloropyridin-2-yl)oxy)-2-((difluoromethoxy)methyl) pyrrolidin-1-yl)-*N*-((5-(ethylsulfonyl)pyridin-2-yl)methyl)-5-fluoronicotinamide**

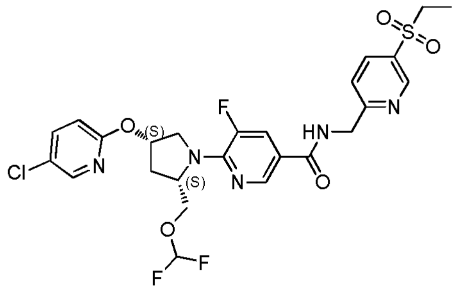

**Step 1: Synthesis of benzyl (2*S*,4*S*)-4-((5-chloropyridin-2-yl)oxy)-2-((difluoromethoxy) methyl)pyrrolidin-1-carboxylate**

Benzyl (2*S*,4*R*)-2-((difluoromethoxy)methyl)-4-((methylsulfonyl)oxy)pyrrolidin -1-carboxylate (2.00 g, 5.27 mmol), 5-chloropyridin-2-ol (820 mg, 6.33 mmol) and $K_2CO_3$ (1.46 g, 10.56 mmol) were added to DMF (20 mL) and the mixture was reacted at 60 °C for 7 h. The reaction solution was diluted with EtOAc (60 mL), washed with $H_2O$ (30 mL × 2) and saturated NaCl solution (30 mL), dried over anhydrous $Na_2SO_4$, concentrated under reduced pressure, and the crude product was separated by silica gel column chromatography (eluent: PE/EtOAc (v/v) = 4/1) to give pale yellow liquid (1.06 g, 49%).

MS (ESI, pos.ion) m/z: 413.3 $[M+H]^+$.

**Step 2: Synthesis of 5-chloro-2-(((3*S*,5*S*)-5-((difluoromethoxy)methyl) pyrrolidin-3-yl)oxy)pyridine**

To a solution of benzyl (2*S*,4*R*)-4-((5-chloropyridin-2-yl)oxy)-2-((difluoromethoxy)methyl)pyrrolidine-1-carboxylate (1.00 g, 2.42 mmol) in DCM (20 mL) was added trimethylsilane (0.75 mL, 5.30 mmol). The mixture was stirred at room temperature for 2 h, then TEA (0.88 mL, 6.33 mmol) was added. The reaction solution was stirred for 15 min. The reaction solution was concentrated under reduced pressure, diluted with EtOAc (50 mL), washed with saturated $NaHCO_3$ solution (20 mL) and saturated NaCl solution (20 mL), dried over anhydrous $Na_2SO_4$, and concentrated under reduced pressure to give light brown liquid (620 mg, 92%).

MS (ESI, pos.ion) m/z: 279.3 $[M+H]^+$.

**Step 3: Synthesis of methyl 6-((2*S*,4*S*)-4-((5-chloropyridin-2-yl)oxy)-2-((difluoromethoxy)methyl)pyrrolidin-1-yl)-5-fluoronicotinate**

5-Chloro-2-((3*S*,5*S*)-5-((difluoromethoxy)methyl)pyrrolidin-3-yl)oxy)pyridine (600 mg, 2.15 mmol), $K_2CO_3$ (500 mg, 3.62 mmol) and methyl 6-chloro-5-fluoronicotinate (490 mg, 2.58 mmol) were added to DMF (8 mL) in turn and the mixture was reacted at 100 °C for 24 h. The reaction solution was diluted with EtOAc (50 mL), washed successively with $H_2O$ (20 mL × 2) and saturated NaCl solution (20 mL), dried over anhydrous $Na_2SO_4$, concentrated under reduced pressure, and the crude product was separated by silica gel column chromatography (eluent: PE/EtOAc (v/v) = 4/1) to give pale yellow liquid (700 mg, 75%).

MS (ESI, pos.ion) m/z: 432.5 $[M+H]^+$.

**Step 4: Synthesis of 6-((2*S*,4*S*)-4-((5-chloropyridin-2-yl)oxy)-2-((difluoromethoxy) methyl)pyrrolidin-1-yl)5-fluoronicotinic acid**

To a solution of methyl 6-((2*S*,4*S*)-4-((5-chloropyridin-2-yl)oxy)-2-((difluoromethoxy)methyl)pyrrolidin-1-yl)5-fluoronicotinate (700 mg, 1.62 mmol) in MeOH (3 mL) and THF (3 mL) were added a solution of LiOH (310 mg, 12.94 mmol) in $H_2O$ (2 mL). The mixture was stirred at 50°C for 16 h. The reaction solution was concentrated under reduced pressure, the remaining solution was added with HCl solution (1 mol/L) to adjust the pH of the solution to about 4. The resulting mixture was extracted with EtOAc (30 mL), washed with saturated NaCl (15 mL) solution, dried over anhydrous $Na_2SO_4$, concentrated under reduced pressure, and the crude product was separated by silica gel column chromatography (eluent: DCM/MeOH (v/v) = 20/1) to give pale yellow liquid (600 mg, 89%).

MS (ESI, pos.ion) m/z: 418.5 $[M+H]^+$.

**Step 5: Synthesis of 6-((2*S*,4*S*)-4-((5-chloropyridin-2-yl)oxy)-2-((difluoromethoxy) methyl)pyrrolidin-1-yl)-*N*-((5-(ethylsulfonyl)pyridin-2-yl)methyl)-5-fluoronicotinamide**

HATU (410 mg, 1.08 mmol), (5-(ethylsulfonyl)pyridin-2-yl)methanamine hydrochloride (204 mg, 0.86 mmol), 6-((2*S*,4*S*)-4-((5-chloropyridin-2-yl)oxy)-2-((difluoromethoxy)methyl)pyrrolidin-1-yl)-5-fluoronicotinic acid (300 mg, 0.72 mmol) and TEA (218 mg, 2.15 mmol) were successively added to DCM (8 mL) and the mixture was stirred at room temperature for 18 h. The reaction solution was concentrated under reduced pressure, the residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/MeOH (v/v) = 20/1) to give a pale yellow solid (330 mg, 77%).
MS (ESI, pos.ion) m/z: 600.1 $[M+H]^+$;
$^1H$ NMR (400 MHz, $CDCl_3$) δ (ppm): 9.03 (d, *J* = 1.4 Hz, 1H), 8.46 (s, 1H), 8.16 (dd, *J* = 8.2, 2.1 Hz, 1H), 8.10 (d, *J* = 2.3 Hz, 1H), 7.72 (dd, *J* = 13.9, 1.6 Hz, 1H), 7.55 (dd, *J* = 13.1, 5.4 Hz, 2H), 7.34 (s, 1H), 6.70 (d, *J* = 8.8 Hz, 1H), 6.21 (t, *J* = 74.7 Hz, 1H), 5.66 (s, 1H), 4.84 (d, *J* = 5.0 Hz, 2H), 4.75 (s, 1H), 4.25 (dd, *J* = 9.2, 4.1 Hz, 1H), 4.15 - 4.04 (m, 2H), 4.00 (t, *J* = 9.3 Hz, 1H), 3.16 (q, *J* = 7.4 Hz, 2H), 2.44 (d, *J* = 14.0 Hz, 1H), 2.41 - 2.31 (m, 1H), 1.32 (t, *J* = 7.4 Hz, 3H).

**Example 114 4-((2*S*,4*S*)-2-((difluoromethoxy)methyl)-4-(4-(difluoromethoxy)phenoxy) pyrrolidin-1-yl)-*N*-((*S*)-1-(4-(ethylsulfonyl)phenyl)-3-hydroxypropyl)benzamide**

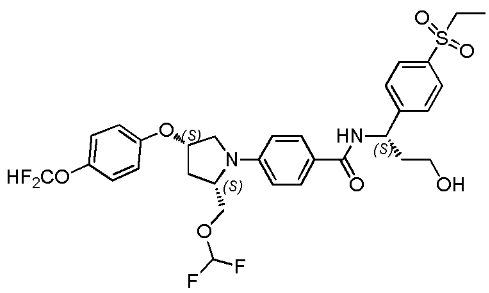

**Step 1: Synthesis of methyl 4-((2*S*,4*S*)-2-((difluoromethoxy)methyl)-4-(4-(difluoromethoxy)phenoxy)pyrrolidin-1-yl)benzoate**

Methyl 4-((2*S*,4*R*)-2-((difluoromethoxy)methyl)-4-hydroxypyrrolidin-1-yl)benzoate (5.30 g, 17.59 mmol), 4-(difluoromethoxy)phenol (3.10 g, 19.36 mmol), $PPh_3$ (5.08 g, 19.37 mmol) were added to THF (40 mL). The mixture was transferred to 0 °C, and DEAD (3.8 mL, 24.13 mmol) was slowly added. After the addition was complete, the mixture was transferred to room temperature and stirred for 21 h. The reaction solution was concentrated under reduced pressure. The residue was diluted with MTBE (20 mL) and stirred at -15 °C. A white insoluble solid was precipitated, and filtered while cold. The filtrate was concentrated under reduced pressure, and the crude product was separated by silica gel column chromatography (eluent: PE/EtOAc (v/v) = 4/1) to give pale yellow liquid (4.30 g, 55%).
MS (ESI, pos.ion) m/z: 444.1 $[M+H]^+$.

**Step 2: Synthesis of 4-((2*S*,4*S*)-2-((difluoromethoxy)methyl)-4-(4-(difluoromethoxy) phenoxy)pyrrolidin-1-yl)benzoic acid**

To a solution of methyl 4-((2*S*,4*S*)-2-((difluoromethoxy)methyl)-4-(4-(difluoromethoxy)phenoxy)pyrrolidin-1-yl)benzoate (4.30 g, 9.70 mmol) in MeOH (15 mL) and THF (15 mL) was added a solution of $LiOH \cdot H_2O$ (4.00 g, 95.33 mmol) in $H_2O$ (10 mL). The mixture was reacted at 50°C for 16 h. The reaction solution was concentrated under reduced pressure, and the remaining solution was added with $H_2O$ (30 mL) and EtOAc (50 mL). Concentrated HCl solution (12 mol/L) was added under an ice bath to adjust the pH of the solution to about 4. The resulting mixture was extracted and separated for layers. The organic phase was washed with saturated NaCl (20 mL) solution, dried over anhydrous $Na_2SO_4$, concentrated under reduced pressure, and the crude product was separated by silica gel column chromatography (eluent: PE/EtOAc (v/v) = 3/2) to give a white solid (3.3 g, 79%).
MS (ESI, pos.ion) m/z: 430.1 $[M+H]^+$.

**Step 3: Synthesis of 4-((2*S*,4*S*)-2-((difluoromethoxy)methyl)-4-(4-(difluoromethoxy) phenoxy)pyrrolidin-1-yl)-*N*-((*S*)-1-(4-(ethylsulfonyl)phenyl)-3-hydroxypropyl)benzamide**

HATU (350 mg, 0.92 mmol), **(*S*)-3-amino-3-(4-(ethylsulfonyl)phenyl)propan-1-ol** hydrochloride (220 mg, 0.79 mmol), 4-((2*S*,4*S*)-2-((difluoromethoxy)methyl) -4-((4-(trifluoromethoxy)phenoxy)pyrrolidin-1-yl)benzoic acid (280 mg , 0.65 mmol) and $Et_3N$ (200 mg, 1.98 mmol) were successively added to DCM (8 mL) and the mixture was reacted at room temperature for 12 h. The reaction solution was concentrated under reduced pressure. The residue was diluted with DCM (40 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a white solid (350 mg, 82%).

MS (ESI, pos.ion) m/z: 655.2 $[M+H]^+$;

$^1H$ NMR (400 MHz, $CDCl_3$) δ (ppm): 8.56 (d, *J* = 7.8 Hz, 1H), 7.83 (d, *J* = 8.3 Hz, 2H), 7.78 (d, *J* = 8.7 Hz, 2H), 7.62 (d, *J* = 8.3 Hz, 2H), 7.16 (d, *J* = 9.0 Hz, 2H), 7.12 (t, *J* = 74.4 Hz, 1H), 7.04 (d, J = 9.1 Hz, 2H), 6.72 (t, *J* = 74.4 Hz, 1H), 6.70 (d, *J* = 9.0 Hz, 2H), 5.21 (q, *J* = 8.6 Hz, 2H), 4.63 (t, *J*= 4.8 Hz, 1H), 4.19 (dd, *J*= 12.9, 8.1 Hz, 1H), 4.07 (dd, *J*= 9.7, 4.6 Hz, 1H), 3.89 (t, *J*= 9.2 Hz, 1H), 3.64 (s, 2H), 3.51 - 3.39 (m, 2H), 3.26 (q, *J*= 7.3 Hz, 2H), 2.46 - 2.36 (m, 1H), 2.23 (d, *J* = 14.3 Hz, 1H), 2.08 - 2.01 (m, 1H), 1.94 - 1.87 (m, 1H), 1.09 (t, *J* = 7.3 Hz, 3H).

**Example 115 6-((2*S*,4*S*)-2-((difluoromethoxy)methyl)-4-(4-trifluoromethoxy)phenoxy) pyrrolidin-1-yl)-*N*-((*S*)-1-(4-(ethylsulfonyl)phenyl)-3-hydroxypropyl)nicotinamide**

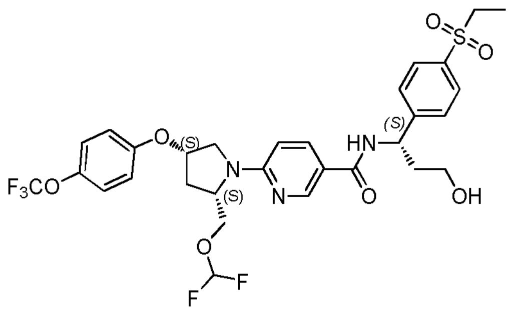

**Step 1: Synthesis of benzyl (2*S*,4*S*)-2-((difluoromethoxy)methyl)-4-(4-(trifluoromethoxy)phenoxy)pyrrolidine-1-carboxylate**

Benzyl (2*S*,4*R*)-2-((difluoromethoxy)methyl)-4-((methylsulfonyl)oxy)pyrrolidin-1-carboxylate (1.50 g, 3.95 mmol), 4-(trifluoromethoxy)phenol (1.06 g, 5.95 mmol) and *t*-BuOK (665 mg, 5.93 mmol) were added to ACN (16 mL) and the mixture was reacted at 70 °C for 21 h. The reaction solution was filtered and concentrated under reduced pressure, and the crude product was separated by silica gel column chromatography (eluent: PE/EtOAc (v/v) = 4/1) to give pale yellow liquid (1.30 g, 71%).

MS (ESI, pos.ion) m/z: 462.1 $[M+H]^+$.

**Step 2: Synthesis of (2*S*,4*S*)-2-((difluoromethoxy)methyl)-4-(4-(trifluoromethoxy)phenoxy)pyrrolidine**

To a solution of benzyl (2*S*,4*S*)-2-((difluoromethoxy)methyl)-4-(4-(trifluoromethoxy)phenoxy)pyrrolidine-1-carboxylate (1.30 g, 2.82 mmol) in MeOH (8 mL) was added Pd/C (100 mg, 10%) under hydrogen protection. The mixture was reacted at room temperature for 15 h. The reaction solution was filtered through a celite pad, and the filtrate was concentrated under reduced pressure to give pale yellow liquid (800 mg, 87%).

MS (ESI, pos.ion) m/z: 328.1 $[M+H]^+$.

**Step 3: Synthesis of methyl 6-((2*S*,4*S*)-2-((difluoromethoxy)methyl)-4-(4-(trifluoromethoxy)phenoxy)pyrrolidin-1-yl)nicotinate**

(2*S*,4*S*)-2-((difluoromethoxy)methyl)-4-(4-(trifluoromethoxy)phenoxy)pyrrolidine (800 mg, 2.45 mmol), $K_2CO_3$ (675 mg, 4.88 mmol) and methyl 6-fluoronicotinate (500 mg, 3.22 mmol) were added to DMSO (6 mL) in turn and the

mixture was reacted at 80 °C for 15 h. The reaction solution was diluted with EtOAc (50 mL), washed successively with $H_2O$ (20 mL × 2) and saturated NaCl solution (20 mL), dried over anhydrous $Na_2SO_4$, concentrated under reduced pressure, and the crude product was separated by silica gel column chromatography (eluent: PE/EtOAc (v/v) = 3/1) to give pale yellow liquid (900 mg, 80%).

MS (ESI, pos.ion) m/z: 463.1 $[M+H]^+$.

**Step 4: Synthesis of 6-((2*S*,4*S*)-2-((difluoromethoxy)methyl)-4-(4-(trifluoromethoxy) phenoxy)pyrrolidin-1-yl)nicotinic acid**

To a solution of methyl 6-((2*S*,4*S*)-2-((difluoromethoxy)methyl)-4-(4-(trifluoromethoxy)phenoxy)pyrrolidin-1-yl)nicotinate (900 mg, 1.95 mmol) in MeOH (3 mL) and THF (3 mL) was added a solution of $LiOH.H_2O$ (400 mg, 9.53 mmol) in $H_2O$ (2 mL). The mixture was stirred at room temperature for 16 h. The reaction solution was concentrated under reduced pressure, and the remaining solution was added with HCl solution (1 mol/L) to adjust the pH of the solution to about 4. The resulting mixture was extracted with EtOAc (40 mL), washed with saturated NaCl (20 mL) solution, dried over anhydrous $Na_2SO_4$, concentrated under reduced pressure, and the crude product was separated by silica gel column chromatography (eluent: DCM/EtOAc (v/v) = 4/1) to give pale yellow liquid (800 mg, 92%).

MS (ESI, pos.ion) m/z: 449.1 $[M+H]^+$.

**Step 5: Synthesis of 6-((2*S*,4*S*)-2-((difluoromethoxy)methyl)-4-(4-(trifluoromethoxy) phenoxy)pyrrolidin-1-yl)-*N*-((*S*)-1-(4-(ethylsulfonyl)phenyl)-3-hydroxypropyl)nicotinamide**

HATU (890 mg, 2.34 mmol), **(*S*)-3-amino-3-(4-(ethylsulfonyl)phenyl)propan-1-ol** hydrochloride (561 mg, 2.01 mmol), 6-((2*S*,4*S*)-2-((difluoromethoxy)methyl)-4-(4-(trifluoromethoxyphenoxy)pyrrolidin-1-yl)nicotinic acid (750 mg , 1.67 mmol) and TEA (507 mg, 5.01 mmol) were successively added to DCM (10 mL) and the mixture was reacted at room temperature for 16 h. The reaction solution was concentrated under reduced pressure. The residue was diluted with DCM (40 mL), washed successively with HCl solution (20 mL, 1 mol/L), saturated $NaHCO_3$ solution (20 mL) and saturated NaCl solution (20 mL), dried over anhydrous $Na_2SO_4$, concentrated under reduced pressure, and the concentrated solution was separated by silica gel column chromatography (eluent: DCM/MeOH (v/v) = 20/1) to give a white solid (895 mg, 79%).

MS (ESI, pos.ion) m/z: 674.1 $[M+H]^+$.

$^1H$ NMR (400 MHz, DMSO-$d_6$) δ (ppm): 8.69 (s, 1H), 8.67 (s, 1H), 7.99 (dd, *J*= 8.8, 1.8 Hz, 1H), 7.84 (d, *J*= 8.2 Hz, 2H), 7.63 (d, *J*= 8.2 Hz, 2H), 7.32 (d, *J*= 8.7 Hz, 2H), 7.09 (d, *J*= 9.0 Hz, 2H), 6.68 (t, *J* = 75.7 Hz, 1H), 6.63 (d, *J* = 8.9 Hz, 1H), 5.29 - 5.16 (m, 2H), 4.63 (t, *J* = 4.8 Hz, 1H), 4.47 (d, *J*= 3.8 Hz, 1H), 4.27 (dd, *J*= 8.8, 4.1 Hz, 1H), 3.87 (t, *J*= 9.2 Hz, 1H), 3.81 (dd, *J* = 12.0, 4.4 Hz, 1H), 3.68 (d, *J* = 12.1 Hz, 1H), 3.48 - 3.38 (m, 2H), 3.26 (q, *J* = 7.3 Hz, 2H), 2.48 - 2.37 (m, 1H), 2.26 (d, *J* = 14.1 Hz, 1H), 2.09 - 2.00 (m, 1H), 1.94 - 1.84 (m, 1H), 1.09 (t, *J* = 7.3 Hz, 3H).

**Example 116 6-((2*S*,4*S*)-2-((difluoromethoxy)methyl)-4-(4-trifluoromethoxy)phenoxy) pyrrolidin-1-yl)-*N*-((*S*)-1-(4-(ethylsulfonyl)phenyl)-3-hydroxypropyl)-5-fluoronicotinamide**

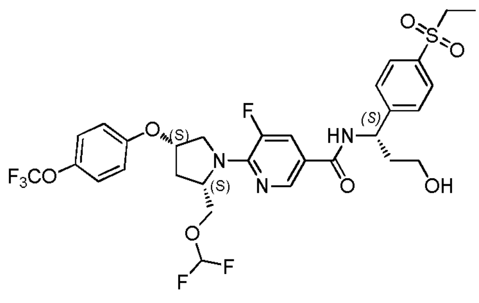

**Step 1: Synthesis of methyl 6-((2*S*,4*S*)-2-((difluoromethoxy)methyl)-4-(4-(trifluoromethoxy)phenoxy)pyrrolidin-1-yl)-5-fluoronicotinate**

(2*S*,4*S*)-2-((difluoromethoxy)methyl)-4-(4-(trifluoromethoxy)phenoxy)pyrrolidine (530 mg, 1.62 mmol), $K_2CO_3$ (450 mg, 3.26 mmol) and methyl 6-chloro-5-fluoronicotinate (370 mg, 1.95 mmol) were added to DMSO (6 mL) in turn and the mixture was reacted at 80 °C for 14 h. The reaction solution was diluted with EtOAc (50 mL), washed successively

with $H_2O$ (20 mL × 2) and saturated NaCl solution (20 mL), dried over anhydrous $Na_2SO_4$, concentrated under reduced pressure, and the concentrated solution was separated by silica gel column chromatography (eluent: PE/EtOAc (v/v) = 3/1) to give pale yellow liquid (537 mg, 69%).

MS (ESI, pos.ion) m/z: 481.2 $[M+H]^+$.

**Step 2: Synthesis of 6-((2*S*,4*S*)-2-((difluoromethoxy)methyl)-4-(4-(trifluoromethoxy)phenoxy)pyrrolidin-1-yl)-5-fluoronicotinic acid**

To a solution of methyl 6-((2*S*,2*S*)-2-((difluoromethoxy)methyl)-4-(4-(trifluoromethoxy)phenoxy)pyrrolidin-1-yl)-5-fluoronicotinate (200 mg, 0.42 mmol) in MeOH (2 mL) and THF (2 mL) was added a solution of $LiOH.H_2O$ (87 mg, 2.07 mmol) in $H_2O$ (1 mL). The mixture was stirred at room temperature for 18 h. The reaction solution was concentrated under reduced pressure, and the remaining solution was added with HCl solution (1 mol/L) to adjust the pH of the solution to about 4. The resulting mixture was extracted with EtOAc (40 mL), washed with saturated NaCl (20 mL) solution, dried over anhydrous $Na_2SO_4$, concentrated under reduced pressure, and the concentrated solution was separated by silica gel column chromatography (eluent: PE/EtOAc (v/v) = 1/1) to give pale yellow liquid (136 mg, 70%).

MS (ESI, pos.ion) m/z: 467.1 $[M+H]^+$.

**Step 3: Synthesis of 6-((2*S*,4*S*)-2-((difluoromethoxy)methyl)-4-(4-(trifluoromethoxy)phenoxy)pyrrolidin-1-yl)-*N*-((*S*)-1-(4-(ethylsulfonyl)phenyl)-3-hydroxyprop yl)-5-fluoronicotinamide**

HATU (166 mg, 0.44 mmol), **(*S*)-3-amino-3-(4-(ethylsulfonyl)phenyl)propan-1-ol** hydrochloride (100 mg, 0.36 mmol), 6-((2*S*,4*S*)-2-((difluoromethoxy)methyl)-4-(4-(trifluoromethoxyphenoxy)pyrrolidin-1-yl)-5-fluoronicotinic acid (136 mg, 0.29 mmol) and TEA (118 mg, 1.17 mmol) were successively added to DCM (6 mL) and the mixture was reacted at room temperature for 20 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (150 mg, 74%).

MS (ESI, pos.ion) m/z: 692.2 $[M+H]^+$;

$^1H$ NMR (400 MHz, $CDCl_3$) δ (ppm): 8.38 (s, 1H), 8.30 (d, *J* = 6.6 Hz, 1H), 7.79 (d, *J* = 8.1 Hz, 2H), 7.64 (d, *J* = 14.1 Hz, 1H), 7.51 (d, *J* = 8.1 Hz, 2H), 7.11 (d, *J* = 8.6 Hz, 2H), 6.84 (d, *J* = 8.9 Hz, 2H), 6.15 (t, *J* = 74.7 Hz, 1H), 5.29 (d, *J* = 4.3 Hz, 1H), 4.98 (s, 1H), 4.68 (s, 1H), 4.17 (dd, *J*= 9.1, 4.1 Hz, 1H), 4.02 (s, 2H), 3.94 (t, *J*= 9.3 Hz, 1H), 3.69 - 3.53 (m, 2H), 3.05 (q, *J*= 7.3 Hz, 2H), 2.42 (d, *J* = 14.2 Hz, 1H), 2.30 - 2.24 (m, 1H), 2.16 - 2.06 (m, 1H), 2.00 - 1.88 (m, 1H), 1.21 (t, *J* = 7.3 Hz, 3H).

**Example 117 *N*-((*S*)-1-(4-(ethylsulfonyl)phenyl)-3-hydroxypropyl)-4-((2*S*,4*S*)-2-(fluoromethyl)-4-(4-(trifluoromethoxy)phenoxy)pyrrolidin-1-yl)benzamide**

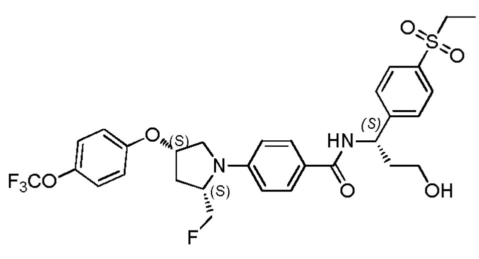

**Step 1: Synthesis of benzyl (2*S*,4*S*)-2-(hydroxymethyl)-4-(4-(trifluoromethoxy) phenoxy)pyrrolidine-1-carboxylate**

Benzyl (2*S*,4*R*)-2-(hydroxymethyl)-4-((methylsulfonyl)oxy)pyrrolidin-1-carboxylate (3.80 g, 11.54 mmol), 4-(trifluoromethoxy)phenol (4.11 g, 23.08 mmol) and $K_2CO_3$ (3.20 g, 23.15 mmol) were added to DMSO (20 mL) and the mixture was reacted at 80 °C for 18 h. The reaction solution was filtered, diluted with DCM (60 mL), washed successively with $H_2O$ (40 mL), NaOH aqueous solution (50 mL, 1 mol/L) and saturated NaCl solution (40 mL), dried over anhydrous $Na_2SO_4$, and concentrated under reduced pressure. The crude product was separated by silica gel column chromatography (eluent: PE/EtOAc (v/v) = 1/1) to give pale yellow liquid (3.43 g, 72%).

MS (ESI, pos.ion) m/z: 412.1 $[M+H]^+$.

**Step 2: Synthesis of benzyl (2*S*,4*S*)-2-(((methylsulfonyl)oxy)methyl)-4-(4-(trifluoromethoxy)phenoxy)pyrrolidine-1-carboxylate**

To a solution of benzyl (2*S*,4*R*)-2-(hydroxymethyl)-4-(4-(trifluoromethoxy) phenoxy)pyrrolidine-1-carboxylate (1.00 g, 2.43 mmol) in DCM (8 mL) were added MsCl (0.30 mL, 3.88 mmol), TEA (0.70 mL, 5.04 mmol) and DMAP (30 mg, 0.25 mmol) in turn. The mixture was reacted at room temperature for 5 h. The reaction solution was concentrated under reduced pressure, and the residue was diluted with DCM, washed successively with HCl solution (0.1 mol/L) and saturated NaCl solution, dried over anhydrous $Na_2SO_4$, and concentrated under reduced pressure to give yellow liquid (1.19 g, 100%).

MS (ESI, pos.ion) m/z: 490.1 $[M+H]^+$.

**Step 3: Synthesis of benzyl (2*S*,4*S*)-2-(fluoromethyl)-4-(4-(trifluoromethoxy) phenoxy)pyrrolidine-1-carboxylate**

To a solution of benzyl (2*S*,4*S*)-2-(((methylsulfonyl)oxy)methyl)-4-(4-(trifluoromethoxy)phenoxy)pyrrolidine-1-carboxylate (1.19 g, 2.43 mmol) in THF (5 mL) was added $Bu_4NF$ (5.0 mL, 1 mol/L). The mixture was stirred at 75°C for 12 h. The reaction solution was concentrated under reduced pressure. The residue was diluted with EtOAc, washed successively with HCl solution (0.5 mol/L), saturated $NaHCO_3$ solution and saturated NaCl solution, dried over anhydrous $Na_2SO_4$, concentrated under reduced pressure, and the concentrated solution was separated by silica gel column chromatography (eluent: PE/EtOAc (v/v) = 4/1) to give a white solid (350 mg, 35%).

MS (ESI, pos.ion) m/z: 414.2 $[M+H]^+$.

**Step 4: Synthesis of (2*S*,4*S*)-2-(fluoromethyl)-4-(4-(trifluoromethoxy)phenoxy)pyrrolidine**

To a solution of benzyl (2*S*,4*S*)-2-(fluoromethyl)-4-(4-(trifluoromethoxy) phenoxy)pyrrolidine-1-carboxylate (350 mg, 0.85 mmol) in MeOH (8 mL) was added Pd/C (50 mg, 10%) under hydrogen protection. The mixture was reacted at room temperature for 16 h. The reaction solution was filtered through a celite pad. The filtrate was concentrated under reduced pressure, and separated by silica gel column chromatography (eluent: DCM/MeOH (v/v) = 20/1) to give pale yellow liquid (100 mg, 42%).

MS (ESI, pos.ion) m/z: 280.2 $[M+H]^+$.

**Step 5: Synthesis of methyl 4-((2*S*,4*S*)-2-(fluoromethyl)-4-(4-(trifluoromethoxy) phenoxy)pyrrolidin-1-yl)benzoate**

Under nitrogen protection, (2*S*,4*S*)-2-(fluoromethyl)-4-(4-(trifluoromethoxy) phenoxy)pyrrolidine (100 mg, 0.36 mmol), $Cs_2CO_3$ (175 mg, 0.54 mmol), methyl 4-iodobenzoate (112 mg, 0.43 mmol), $Pd_2(dba)_3$ (32 mg, 0.03 mmol) and Ruphos (25 mg, 0.05 mmol) were added to 1,4-dioxane (4 mL) in turn and the mixture was reacted at 100°C for 16 h. The reaction solution was filtered through a celite pad. The filtrate was concentrated under reduced pressure, and separated by silica gel column chromatography (eluent: PE/EtOAc (v/v) = 4/1) to give a pale yellow solid (110 mg, 74%).

MS (ESI, pos.ion) m/z: 414.1 $[M+H]^+$.

**Step 6: Synthesis of 4-((2*S*,4*S*)-2-(fluoromethyl)-4-(4-(trifluoromethoxy) phenoxy)pyrrolidin-1-yl)benzoic acid**

To a solution of methyl 4-((2*S*,4*S*)-2-(fluoromethyl)-4-(4-(trifluoromethoxy) phenoxy)pyrrolidin-1-yl)benzoate (110 mg, 0.27 mmol) in MeOH (2 mL) and THF (2 mL) was added a solution of $LiOH.H_2O$ (60 mg, 1.43 mmol) in $H_2O$ (1 mL). The mixture was stirred at room temperature for 17 h. The reaction solution was concentrated under reduced pressure, and the remaining liquid was added with concentrated HCl solution (1 mol/L) to adjust the pH to 4. The resulting mixture was extracted with EtOAc (40 mL), washed with saturated NaCl solution (20 mL), dried over anhydrous $Na_2SO_4$, and concentrated under reduced pressure to give a pale yellow solid (100 mg, 94%).

MS (ESI, pos.ion) m/z: 400.2 $[M+H]^+$.

**Step 7: Synthesis of *N*-((*S*)-1-(4-(ethylsulfonyl)phenyl)-3-hydroxypropyl) -4-((2*S*,4*S*)-2-(fluoromethyl)-4-(4-(trifluoromethoxy)phenoxy)pyrrolidin-1-yl)benzamide**

HATU (142 mg, 0.37 mmol), (*S*)-3-amino-3-(4-(ethylsulfonyl)phenyl)propan-1-ol hydrochloride (84 mg, 0.30 mmol), **4-((2*S*,4*S*)-2-(fluoromethyl)-4-(4-(trifluoromethoxy)** phenoxy)pyrrolidin-1-yl)benzoic acid (100 mg, 0.25 mmol) and TEA (80 mg, 0.79 mmol) were successively added to DCM (4 mL) and the mixture was reacted at room temperature

for 20 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (100 mg, 64%).

MS (ESI, pos.ion) m/z: 625.2 $[M+H]^+$;

$^1H$ NMR (400 MHz, $CDCl_3$) δ (ppm): 7.85 (d, *J* = 8.3 Hz, 2H), 7.74 (d, *J* = 8.7 Hz, 2H), 7.54 (d, *J* = 8.2 Hz, 2H), 7.36 (d, *J* = 7.2 Hz, 1H), 7.18 (d, *J* = 8.7 Hz, 2H), 6.89 (d, *J* = 9.1 Hz, 2H), 6.67 (d, *J* = 8.8 Hz, 2H), 5.48 - 5.43 (m, 1H), 5.08 (t, *J* = 4.3 Hz, 1H), 4.73 (dd, *J* = 8.9, 5.4 Hz, 0.5 H), 4.63 - 4.53 (m, 1H), 4.45 (t, *J* = 8.4 Hz, 0.5H), 4.32 - 4.22 (m, 1H), 3.81 - 3.71 (m, 2H), 3.70 - 3.61 (m, 2H), 3.09 (q, *J* = 7.4 Hz, 2H), 2.85 (s, 1H), 2.44 (d, *J* = 14.1 Hz, 1H), 2.42 - 2.33 (m, 1H), 2.29-2.19 (m, 1H), 1.99- 1.89 (m, 1H), 1.28 (t, *J* = 7.4 Hz, 3H).

**Example 118 *N*-((*S*)-1-(4-(ethylsulfonyl)phenyl)-3-hydroxypropyl)-4-((2*S*,4*S*)-2-((trifluoromethoxy)methyl)-4-(4-(trifluoromethoxy)phenoxy)pyrrolidin-1-yl)benzamide**

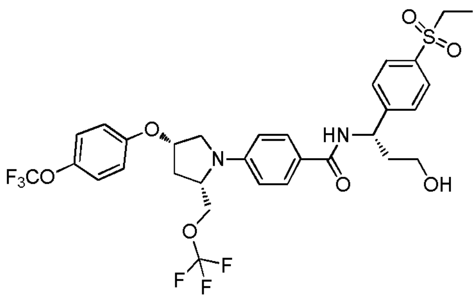

**Step 1: Synthesis of benzyl (2*S*,4*S*)-2-((trifluoromethoxy)methyl)-4-(4-(trifluoromethoxy)phenoxy)pyrrolidine-1-carboxylate**

To a solution of benzyl (2*S*,4*S*)-2-(hydroxymethyl)-4-(4-(trifluoromethoxy)phenoxy)pyrrolidine-1-carboxylate (2.30 g, 5.59 mmol) in DCM (30 mL) were added AgOTf (4.48 g, 17.44 mmol), selective fluorine reagent (3.09 g, 8.72 mmol) and KF (1.35 g, 23.24 mmol). Under nitrogen protection, 2-fluoropyridine (1.50 mL, 17.43 mmol) and $TMSCF_3$ (2.60 mL, 17.59 mmol) were added dropwise. The mixture was reacted at room temperature for 17 h. The reaction solution was filtered and concentrated under reduced pressure, and the crude product was separated by silica gel column chromatography (eluent: PE/EtOAc (v/v) = 4/1) to give colorless liquid (1.40 g, 52%).

MS (ESI, pos.ion) m/z: 480.2 $[M+H]^+$.

**Step 2: Synthesis of (2*S*,4*S*)-2-((trifluoromethoxy)methyl)-4-(4-(trifluoromethoxy) phenoxy)pyrrolidine**

To a solution of benzyl (2*S*,4*S*)-2-((trifluoromethoxy)methyl)-4-(4-(trifluoromethoxy)phenoxy)pyrrolidine-1-carboxylate (1.30 g, 2.71 mmol) in MeOH (12 mL) was added Pd/C (100 mg, 10%) under hydrogen protection. The mixture was reacted at room temperature for 18 h. The reaction solution was filtered through a celite pad. The filtrate was concentrated under reduced pressure, and separated by silica gel column chromatography (eluent: DCM/EtOAc (v/v) = 1/1) to give pale yellow liquid (542 mg, 58%).

MS (ESI, pos.ion) m/z: 346.1 $[M+H]^+$.

**Step 3: Synthesis of methyl 4-((2*S*,4*S*)-2-((trifluoromethoxy)methyl) -4-(4-(trifluoromethoxy)phenoxy)pyrrolidin-1-yl)benzoate**

Under nitrogen protection, (2*S*,4*S*)-2-((trifluoromethoxy)methyl)-4-(4-(trifluoromethoxy)phenoxy)pyrrolidine (540 mg, 1.56 mmol), $Cs_2CO_3$ (764 mg, 2.34 mmol), methyl 4-iodobenzoate (491 mg, 1.87 mmol), $Pd_2(dba)_3$ (143 mg, 0.16 mmol) and Ruphos (110 mg, 0.24 mmol) were added to 1,4-dioxane (12 mL) in turn and the mixture was reacted at 100°C for 16 h. The reaction solution was filtered through a celite pad. The filtrate was concentrated under reduced pressure, and separated by silica gel column chromatography (eluent: PE/EtOAc (v/v) = 4/1) to give pale yellow liquid (600 mg, 88%).

MS (ESI, pos.ion) m/z: 480.1 $[M+H]^+$.

**Step 4: Synthesis of 4-((2*S*,4*S*)-2-((trifluoromethoxy)methyl)-4-(4-(trifluoromethoxy)phenoxy)pyrrolidin-1-yl)benzoic acid**

To a solution of methyl 4-((2*S*,4*S*)-2-((trifluoromethoxy)methyl)-4-(4-(trifluoromethoxy)phenoxy)pyrrolidin-1-yl)benzoate (600 mg, 1.25 mmol) in MeOH (3 mL) and THF (3 mL) was added a solution of $LiOH.H_2O$ (262 mg, 6.24 mmol) in $H_2O$ (2 mL). The mixture was stirred at room temperature for 17 h. The reaction solution was concentrated under reduced pressure, and the remaining solution was added with HCl solution (1 mol/L) to adjust the pH of the solution to about 4. The resulting mixture was extracted with EtOAc (40 mL), washed with saturated NaCl (20 mL) solution, dried over anhydrous $Na_2SO_4$, concentrated under reduced pressure, and the crude product was separated by silica gel column chromatography (eluent: PE/EtOAc (v/v) = 4/1) to give a pale yellow solid (440 mg, 75%).

MS (ESI, pos.ion) m/z: 466.2 $[M+H]^+$.

**Step 5: Synthesis of *N*-((*S*)-1-(4-(ethylsulfonyl)phenyl)-3-hydroxypropyl)-4-((2*S*,4*S*) -2-((trifluoromethoxy)methyl)-4-(4-(trifluoromethoxy)phenoxy)pyrrolidin-1-yl)benzamide**

HATU (245 mg, 0.64 mmol), (*S*)-3-amino-3-(4-(ethylsulfonyl)phenyl)propan-1-ol hydrochloride (144 mg, 0.51 mmol), 4-((2*S*,4*S*)-2-((trifluoromethoxy)methyl)-4-(4-(trifluoromethoxy)phenoxy)pyrrolidin-1-yl)benzoic acid (200 mg , 0.43 mmol) and TEA (240 mg, 2.37 mmol) were successively added to DCM (6 mL) and the mixture was reacted at room temperature for 16 h. The reaction solution was concentrated under reduced pressure. The residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the concentrated solution was separated by silica gel column chromatography (eluent: DCM/EtOAc (v/v) = 1/1) to give a white solid (240 mg, 81%).

MS (ESI, pos.ion) m/z: 691.2 $[M+H]^+$;

$^1H$ NMR (400 MHz, $CDCl_3$) δ (ppm): 7.84 (d, *J* = 8.2 Hz, 2H), 7.76 (d, *J* = 8.6 Hz, 2H), 7.54 (d, *J* = 8.1 Hz, 2H), 7.44 (d, *J* = 7.1 Hz, 1H), 7.18 (d, *J* = 8.6 Hz, 2H), 6.89 (d, *J* = 9.0 Hz, 2H), 6.63 (d, *J* = 8.6 Hz, 2H), 5.45 (s, 1H), 5.10 (s, 1H), 4.23 (d, *J* = 7.8 Hz, 2H), 4.13 (t, *J* = 10.5 Hz, 1H), 3.82 - 3.73 (m, 2H), 3.70 - 3.62 (m, 2H), 3.09 (q, *J* = 7.3 Hz, 2H), 2.92 (s, 1H), 2.51 (d, *J* = 14.3 Hz, 1H), 2.43 -2.33 (m, 1H), 2.30 - 2.18 (m, 1H), 1.98- 1.90 (m, 1H), 1.27 (t, *J* = 7.4 Hz, 3H).

$^{13}C$ NMR (101 MHz, $CDCl_3$) δ (ppm): 167.06, 155.13, 148.87, 148.60, 143.38, 137.33, 129.03, 128.57, 127.39, 122.75, 121.85, 116.16, 111.52, 76.46, 66.61, 59.33, 56.72, 54.41, 51.86, 50.62, 37.49, 33.92, 7.33.

**Example 119 4-((2*S*,4*S*)-2-((difluoromethoxy)methyl)-4-(4-(trifluoromethoxy)phenoxy) pyrrolidin-1-yl)-*N*-((*S*)-1-(4-(ethylsulfonyl)phenyl)-3-hydroxypropyl)benzamide**

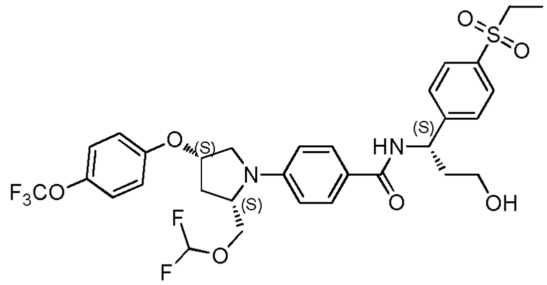

4-((2*S*,4*S*)-2-((Difluoromethoxy)methyl)-4-(4-(trifluoromethoxy)phenoxy)pyrrolidin-1 -yl)benzoic acid (300 mg, 0.67 mmol) (it was prepared with appropriate raw material referring to the synthetic methods of step 1 to step 4 of Example 115), (3*S*)-3-amino-3-(4-(ethylsulfonyl)phenyl)propan-1-ol (206 mg, 0.74 mmol) and HATU (0.26 g, 0.66 mmol) were dissolved in DCM (25 mL), then TEA (0.28 mL, 2.20 mmol) was added. The mixture was stirred at room temperature for 24 h. The reaction was quenched by adding water. The resulting mixture was extracted with DCM (5 mL × 3). The organic phases were combined, dried over anhydrous $Na_2SO_4$, filtered, concentrated and separated by silica gel column chromatography (eluent: PE/EtOAc (v/v) = 2/1) to give a white solid (350 mg, 78%).

MS (ESI, pos.ion) m/z: 673.9 $[M+H]^+$;

$^1H$ NMR (400 MHz, $CDCl_3$) δ (ppm): 7.87 (d, *J* = 8.3 Hz, 2H), 7.77 (d, *J* = 8.7 Hz, 2H), 7.57 (d, *J* = 8.2 Hz, 2H), 7.39 (d, *J* = 7.4 Hz, 1H), 7.20 (d, *J* = 8.7 Hz, 2H), 6.91 (d, *J* = 9.1 Hz, 2H), 6.67 (d, *J* = 8.8 Hz, 2H), 6.25 (t, *J* = 74.4 Hz, 1H), 5.48 (td, *J* = 7.9, 3.9 Hz, 1H), 5.11 (t, *J* = 4.7 Hz, 1H), 4.30 - 4.13 (m, 2H), 3.99 (t, *J* = 9.4 Hz, 1H), 3.90 - 3.62 (m,

4H), 3.12 (q, $J$ = 7.4 Hz, 2H), 2.52 (d, $J$ = 14.3 Hz, 1H), 2.37 (ddd, $J$ = 14.1, 9.1, 5.0 Hz, 1H), 2.35 - 2.17 (m, 1H), 1.97 (dd, $J$ = 17.4, 5.8 Hz, 1H), 1.30 (t, $J$ = 7.4 Hz, 3H).

**Example 120 (*S*)-1-((*S*)-3-(4-((2*S*,4*S*)-2-((difluoromethoxy)methyl)-4-(4-(trifluoromethyl) phenoxy)pyrrolidin-1-yl)benzoylamino)-3-(4-(ethylsulfonyl)phenyl)propoxy)-3-methyl-1-oxob utane-2-amine hydrochloride**

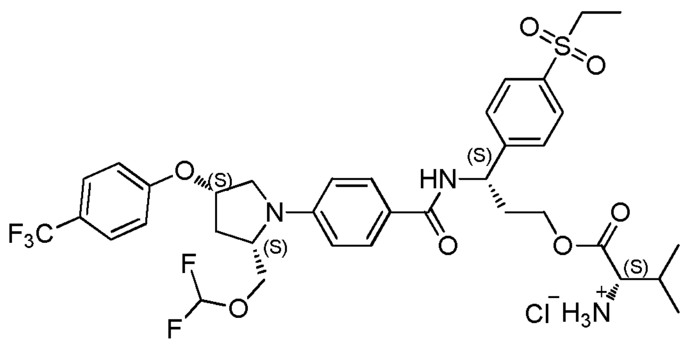

**Step 1: Synthesis of (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl 2-((*tert*-butoxycarbonyl)amino)-3-methylbutyrate**

4-((2*S*,4*S*)-2-((difluoromethoxy)methyl)-4-(4-(trifluoromethyl)phenoxy)pyrrolidin-1-y l)-*N*-((*S*)-1-(4-(ethylsulfonyl)phenyl)-3-hydroxypropyl)benzamide (500 mg, 0.77 mmol), DMAP (18 mg, 0.15 mmol) and EDCI (218 mg, 1.14 mmol) were dissolved in DCM (10 mL), then *L-Boc*-valine (330 mg, 1.52 mmol) was added. The mixture was reacted at room temperature for 23 h. The reaction was quenched by adding saturated sodium chloride. The resulting mixture was extracted with DCM (30 ml × 2). The organic phases were combined, dried over anhydrous $Na_2SO_4$, filtered, concentrated and separated by silica gel column chromatography (eluent: PE/EtOAc (v/v) = 1/1) to give a white solid (0.52 g, 80%).

MS (ESI, pos.ion) m/z: 856.3 $[M+H]^+$.

**Step 2: Synthesis of (*S*)-1-((*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propoxy)-3-methyl-1-oxobutane-2-amine hydrochloride**

To a solution of (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl 2-((*tert*-butoxycarbonyl)amino)-3-methylbutyrate (420 mg, 0.49 mmol) in DCM (10 mL) was added HCl (0.25 mL, 1.0 mmol, 4.0 mol/L). The mixture was reacted at room temperature for 2 h. The resulting mixture was concentrated under reduced pressure to give a white solid (0.32 g, 86%).

MS (ESI, pos.ion) m/z: 756.3 $[M+H]^+$;

$^1H$ NMR (400 MHz, $CDCl_3$) δ (ppm): 8.69 (m, 3H), 8.07 - 7.47 (m, 9H), 6.97 (s, 2H), 6.61 (m, 2H), 6.22 (t, $J$ = 74.5 Hz, 1H), 5.40 (m, 1H), 5.13 (m, 1H), 4.33 (m, 1H), 4.12 (s, 3H), 3.89 (d, $J$ = 23.8 Hz, 2H), 3.69 (m, 2H), 3.07 (m, 2H), 2.39 (d, $J$ = 45.4 Hz, 1H), 1.22 (d, $J$ = 9.1 Hz, 5H), 0.99 (s, 6H).

**Example 121 4-((*S*)-3-(4-((2*S*,4*S*)-2-((difluoromethoxy)methyl)-4-(4-(trifluoromethyl) phenoxy)pyrrolidin-1-yl)benzoylamino)-3-(4-(ethylsulfonyl)phenyl)propoxy)-4-oxobutyrate** sodium

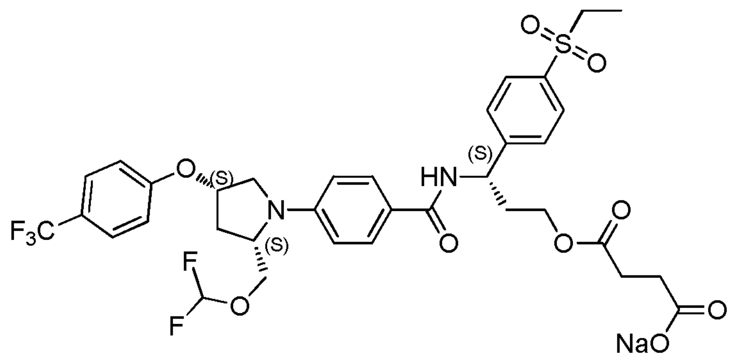

**Step 1: Synthesis of 4-((*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propoxy)-4-oxobutyric acid**

4-((2*S*,4*S*)-2-((difluoromethoxy)methyl)-4-(4-(trifluoromethyl)phenoxy)pyrrolidin-1-y l)-*N*-((*S*)-1-(4-(ethylsulfonyl)phenyl)-3-hydroxypropyl)benzamide (300 mg, 0.46 mmol) and DMAP (140 mg, 1.15 mmol) were dissolved in DCM (10 mL), then succinic anhydride (91 mg, 0.91 mmol) was added. The mixture was reacted at room temperature for 15 h. The reaction was quenched by adding dilute hydrochloric acid solution. The resulting mixture was extracted with DCM (30 mL × 2). The organic phases were combined, dried over anhydrous $Na_2SO_4$, filtered, concentrated and separated by silica gel column chromatography (eluent: DCM/EtOAc (v/v) = 2/1) to give a white solid (0.32 g, 93%).

MS (ESI, pos.ion) m/z: 757.3 $[M+H]^+$.

**Step 2: Synthesis of 4-((*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propoxy)-4-oxobutyrate sodium**

To a solution of 4-((*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propoxy)-4-ox obutyric acid (243 mg, 0.32 mmol) in MeOH (10 mL) was added sodium hydroxide (12 mg, 0.30 mmol). The mixture was reacted at room temperature for 2 h. The resulting mixture was concentrated under reduced pressure to give a white solid (0.25 g, 99%).

MS (ESI, pos.ion) m/z: 757.3 $[M+H]^+$;

$^1H$ NMR (400 MHz, DMSO-$d_6$) δ (ppm): 8.63 (d, *J* = 7.7 Hz, 1H), 7.81 (dd, *J* = 16.2, 8.4 Hz, 4H), 7.63 (d, *J* = 8.1 Hz, 2H), 7.32 (d, *J* = 8.6 Hz, 2H), 7.09 (d, *J* = 9.0 Hz, 2H), 6.90 - 6.49 (m, 3H), 5.22 (d, *J* = 13.5 Hz, 2H), 4.19 (d, *J* = 4.7 Hz, 1H), 4.06 (dd, *J* = 9.7, 4.4 Hz, 1H), 3.88 (t, *J* = 9.2 Hz, 2H), 3.25 (dd, *J* = 14.6, 7.3 Hz, 3H), 2.38 (t, *J* = 7.1 Hz, 2H), 2.23 (d, *J* = 13.7 Hz, 2H), 2.16 (t, *J* = 7.1 Hz, 2H), 2.06 (dd, *J* = 13.9, 8.4 Hz, 2H), 1.90 (dd, *J* = 13.2, 6.6 Hz, 2H), 1.09 (t, *J* = 7.3 Hz, 3H).

**Example 122 sodium (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl phosphate**

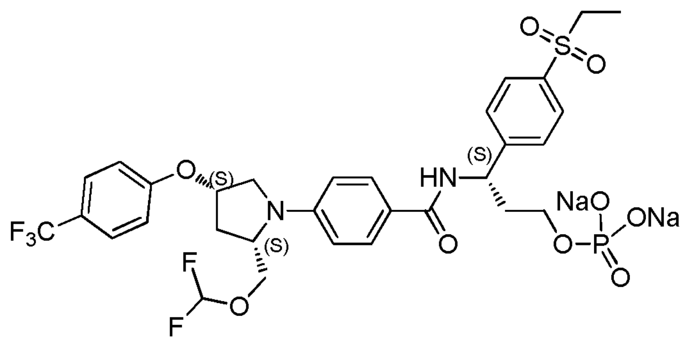

**Step 1: Synthesis of dibenzyl (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl)ph osphate**

4-((2*S*,4*S*)-2-((difluoromethoxy)methyl)-4-(4-(trifluoromethyl)phenoxy)pyrrolidin-1-y l)-*N*-((*S*)-1-(4-(ethylsulfonyl)phenyl)-3-hydroxypropyl)benzamide (200 mg, 0.30 mmol) and tetrazolium (64 mg, 0.91 mmol) were dissolved in DCM (10 mL), then dibenzyl diisopropyl phosphoramide (0.20 mL, 0.60 mmol) was added. The mixture was reacted at room temperature for 3 h. $H_2O_2$ (0.18 g, 1.60 mmol) was added and the reaction was continued for 2 h. The reaction was quenched by adding saturated sodium sulfite solution. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 1/1) to give a white solid (0.12 g, 43%).
MS (ESI, pos.ion) m/z: 917.2 $[M+H]^+$.

**Step 2: Synthesis of (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl dihydrogen phosphate**

To a solution of dibenzyl ((*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl) phosphate (2.24 g, 2.44 mmol) in MeOH (10 mL) was added PdOH/C (200 mg). The reaction mixture was degassed and refilled with $H_2$ for three times. The mixture was reacted at room temperature for 24 h, filtered and concentrated to give a white solid (1.2 g, 67%).
MS (ESI, pos.ion) m/z: 737.2 $[M+H]^+$.

**Step 3: Synthesis of sodium (*S*)-3-(4-((2*S*,4*S*)-2-((difluoromethoxy)methyl)-4-(4-(trifluoromethyl)phenoxy)pyrrolidin-1-yl)benzoylamino)-3-(4-(ethylsulfonyl)phenyl)propyl phosphate**

To a solution of (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl dihydrogen phosphate (402 mg, 0.55 mmol) in MeOH (10 mL) was added sodium hydroxide (43 mg, 1.08 mmol). The mixture was reacted at room temperature for 2 h. The resulting mixture was directly concentrated under reduced pressure to give a dark solid (350 mg, 82%).
MS (ESI, pos.ion) m/z: 737.2 $[M+H]^+$;
$^1H$ NMR (400 MHz, $CD_3OD$) δ (ppm): 7.87 (dd, *J*= 7.9, 6.0 Hz, 4H), 7.71 (d, *J*= 8.2 Hz, 2H), 7.24 (d, *J* = 8.6 Hz, 2H), 7.04 (d, *J* = 9.0 Hz, 2H), 6.74 (d, *J* = 8.7 Hz, 2H), 6.41 (t, *J* = 75.2 Hz, 1H), 5.46 - 5.28 (m, 1H), 5.20 (s, 1H), 4.21 (s, 1H), 4.15 (dd, *J* = 9.7, 3.9 Hz, 1H), 4.03 - 3.87 (m, 3H), 3.71 (s, 2H), 3.18 (q, *J* = 7.3 Hz, 2H), 2.43 (s, 2H), 2.34 - 2.16 (m, 2H), 1.20 (t, *J* = 7.4 Hz, 3H).

**Example 123 (*S*)-1-((*S*)-3-(4-((2*S*,4*S*)-2-((difluoromethoxy)methyl)-4-(4-(trifluoromethoxy) phenoxy)pyrrolidin-1-yl)benzoylamino)-3-(4-(ethylsulfonyl)phenyl)propoxy)-3-methyl-1-oxob utane-2-amine hydrochloride**

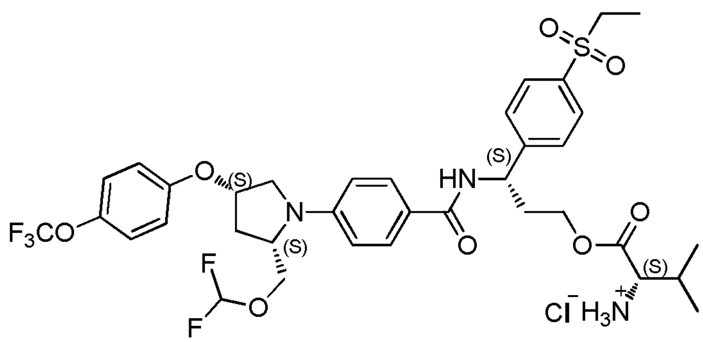

**Step 1: Synthesis of (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl 2-((*tert*-butoxycarbonyl)amino)-3-methylbutyrate**

4-((2*S*,4*S*)-2-((difluoromethoxy)methyl)-4-(4-(trifluoromethoxy)phenoxy)pyrrolidin-1-yl)-*N*-((*S*)-1-(4-(ethylsulfonyl)phenyl)-3-hydroxypropyl)benzamide (500 mg, 0.77 mmol), DMAP (18 mg, 0.15 mmol) and EDCI (218 mg, 1.14 mmol) were dissolved in DCM (10 mL), then L-Boc-valine (330 mg, 1.52 mmol) was added. The mixture was reacted at room temperature for 23 h. The reaction was quenched by adding saturated sodium chloride. The resulting mixture was

extracted with DCM (30 mL × 2). The organic phases were combined, dried over anhydrous $Na_2SO_4$, filtered, concentrated and separated by silica gel column chromatography (eluent: PE/EtOAc (v/v) = 1/1) to give a white solid (0.52 g, 80%).

MS (ESI, pos.ion) m/z: 872.3 $[M+H]^+$.

**Step 2: Synthesis of (*S*)-1-((*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propoxy )-3-methyl-1-oxobutane-2-amine hydrochloride**

To a solution of (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl 2-((*tert*-butoxycarbonyl)amino)-3-methylbutyrate (420 mg, 0.49 mmol) in DCM (10 mL) was added HCl (0.25 mL, 1.0 mmol, 4.0 mol/L). The mixture was reacted at room temperature for 2 h. The resulting mixture was concentrated under reduced pressure to give a white solid (0.32 g, 86%).

MS (ESI, pos.ion) m/z: 772.2 $[M+H]^+$;

$^1H$ NMR (400 MHz, $CDCl_3$) δ (ppm): 8.69 (m, 3H), 8.07 - 7.47 (m, 9H), 6.97 (s, 2H), 6.61 (m, 2H), 6.22 (t, *J* = 74.5 Hz, 1H), 5.40 (m, 1H), 5.13 (m, 1H), 4.33 (m, 1H), 4.12 (s, 3H), 3.89 (d, *J* = 23.8 Hz, 2H), 3.69 (m, 2H), 3.07 (m, 2H), 2.39 (d, *J* = 45.4 Hz, 1H), 1.22 (d, *J* = 9.1 Hz, 5H), 0.99 (s, 6H).

**Example 124 sodium 4-((*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propoxy )-4-oxobutyrate**

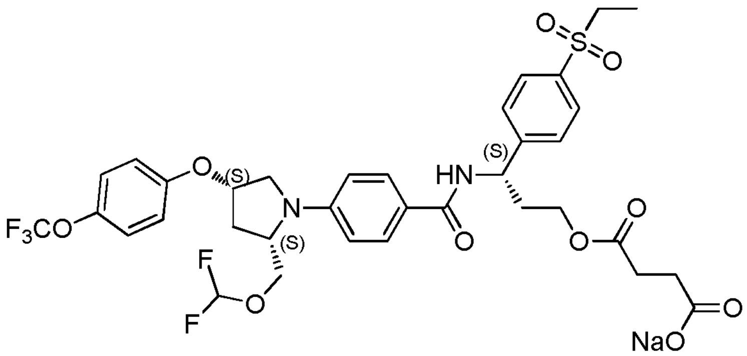

**Step 1: Synthesis of 4-((*S*)-3-(4-((2*S*,4*S*)-2-((difluoromethoxy)methyl) -4-(4-(trifluoromethoxy)phenoxy)pyrrolidin-1-yl)benzoylamino)-3-(4-(ethylsulfonyl)phenyl)pr opoxy)-4-oxobutyric acid**

4-((2*S*,4*S*)-2-((difluoromethoxy)methyl)-4-(4-(trifluoromethoxy)phenoxy)pyrrolidin-1-yl)-*N*-((*S*)-1-(4-(ethylsulfonyl)phenyl)-3-hydroxypropyl)benzamide (300 mg, 0.46 mmol) and DMAP (140 mg, 1.15 mmol) were dissolved in DCM (10 mL), then succinic anhydride (91 mg, 0.91 mmol) was added. The mixture was reacted at room temperature for 15 h. The reaction was quenched by adding dilute hydrochloric acid solution. The resulting mixture was extracted with DCM (30 mL × 2). The organic phases were combined, dried over anhydrous $Na_2SO_4$, filtered, concentrated and separated by silica gel column chromatography (eluent: DCM/EtOAc (v/v) = 2/1) to give a white solid (0.32 g, 93%).

MS (ESI, pos.ion) m/z: 773.3 $[M+H]^+$.

**Step 2: Synthesis of 4-((*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propoxy )-4-oxobutyrate sodium**

To a solution of 4-((*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propoxy)-4-oxobutyric acid (243 mg, 0.32 mmol) in MeOH (10 mL) was added sodium hydroxide (12 mg, 0.30 mmol). The mixture was reacted at room temperature for 2 h, and concentrate under reduced pressure to give a white solid (0.25 g, 99%).

MS (ESI, pos.ion) m/z: 773.3 $[M+H]^+$;

$^1H$ NMR (400 MHz, DMSO-$d_6$) δ (ppm): 8.63 (d, *J* = 7.7 Hz, 1H), 7.81 (dd, *J* = 16.2, 8.4 Hz, 4H), 7.63 (d, *J* = 8.1 Hz, 2H), 7.32 (d, *J* = 8.6 Hz, 2H), 7.09 (d, *J* = 9.0 Hz, 2H), 6.90 - 6.49 (m, 3H), 5.22 (d, *J* = 13.5 Hz, 2H), 4.19 (d, *J* = 4.7 Hz, 1H), 4.06 (dd, *J* = 9.7, 4.4 Hz, 1H), 3.88 (t, *J* = 9.2 Hz, 2H), 3.25 (dd, *J* = 14.6, 7.3 Hz, 3H), 2.38 (t, *J* = 7.1 Hz, 2H), 2.23 (d, *J* = 13.7 Hz, 2H), 2.16 (t, *J* = 7.1 Hz, 2H), 2.06 (dd, *J* = 13.9, 8.4 Hz, 2H), 1.90 (dd, *J* = 13.2, 6.6 Hz, 2H), 1.09 (t, *J* = 7.3 Hz, 3H).

**Example 125 sodium (*S*)-3-(4-((2*S*,4*S*)-2-((difluoromethoxy)methyl)-4-(4-(trifluoromethoxy)phenoxy)pyrrolidin-1-yl)benzamino)-3-(4-(ethylsulfonyl)phenyl)propyl phosphate**

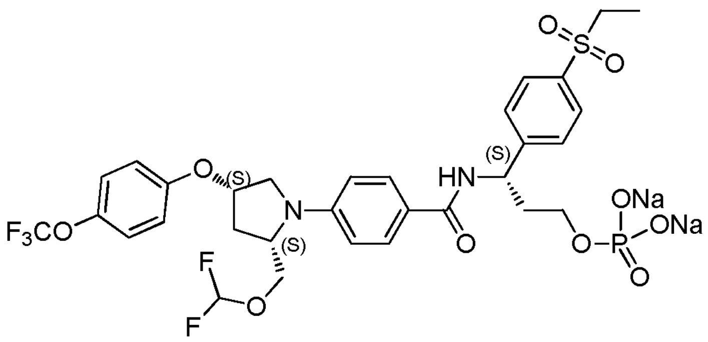

**Step 1: Synthesis of dibenzyl (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl) phosphate**

4-((2*S*,4*S*)-2-((difluoromethoxy)methyl)-4-(4-(trifluoromethyl)phenoxy)pyrrolidin-1-y l)-*N*-((*S*)-1-(4-(ethylsulfonyl)phenyl)-3-hydroxypropyl)benzamide (200 mg, 0.30 mmol) and tetrazolium (64 mg, 0.91 mmol) were dissolved in DCM (10 mL), then dibenzyl diisopropyl phosphoramide (0.20 mL, 0.60 mmol) was added. The mixture was reacted at room temperature for 3 h. $H_2O_2$ (0.18 g, 1.60 mmol, 30 mass%) was added and the reaction was continued for 2 h. The reaction was quenched by adding saturated sodium sulfite solution. The resulting mixture was extracted with DCM (30 mL × 2). The organic phases were combined, dried over anhydrous $Na_2SO_4$, filtered, concentrated and separated by silica gel column chromatography (eluent: PE/EtOAc (v/v) = 1/1) to give a white solid (0.12 g, 43%).

MS (ESI, pos.ion) m/z: 933.2 $[M+H]^+$.

**Step 2: Synthesis of (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl dihydrogen phosphate**

To a solution of dibenzyl ((*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl) phosphate (2.24 g, 2.44 mmol) in MeOH (10 mL) was added PdOH/C (200 mg). The reaction mixture was degassed and refilled with $H_2$ for three times. The mixture was reacted at room temperature for 24 h, filtered and concentrated to give a white solid (1.2 g, 67%).

MS (ESI, pos.ion) m/z: 753.2 $[M+H]^+$.

**Step 3: Synthesis of sodium (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl phosphate**

To a solution of (*S*)-3-(4-((2*S*,4*S*)-2-((difluoromethoxy)methyl)-4-(4-(trifluoromethoxy)phenoxy)pyrrolidin-1-yl)benzoylamino)-3-(4-(ethylsulfonyl)phenyl)propyl dihydrogen phosphate (402 mg, 0.55 mmol) in MeOH (10 mL) was added sodium hydroxide (43 mg, 1.08 mmol). The mixture was reacted at room temperature for 2 h. The resulting mixture was directly concentrated under reduced pressure to give a dark solid (350 mg, 82%).

MS (ESI, pos.ion) m/z: 753.2 $[M+H]^+$;

$^1H$ NMR (400 MHz, $CD_3OD$) δ (ppm): 7.87 (dd, *J*= 7.9, 6.0 Hz, 4H), 7.71 (d, *J*= 8.2 Hz, 2H), 7.24 (d, *J* = 8.6 Hz, 2H), 7.04 (d, *J* = 9.0 Hz, 2H), 6.74 (d, *J* = 8.7 Hz, 2H), 6.41 (t, *J* = 75.2 Hz, 1H), 5.46 - 5.28 (m, 1H), 5.20 (s, 1H), 4.21 (s, 1H), 4.15 (dd, *J* = 9.7, 3.9 Hz, 1H), 4.03 - 3.87 (m, 3H), 3.71 (s, 2H), 3.18 (q, *J* = 7.3 Hz, 2H), 2.43 (s, 2H), 2.34 - 2.16 (m, 2H), 1.20 (t, *J* = 7.4 Hz, 3H).

**Example 126 *N*-(Dideuterium(4-(ethylsulfonyl)phenyl)methyl)-4-((2*S*,4*S*)-2-((difluoromethoxy)methyl)-4-((5-(trifluoromethoxy)pyridin-2-yl)oxy)pyrrolidin-1-yl)benzam ide**

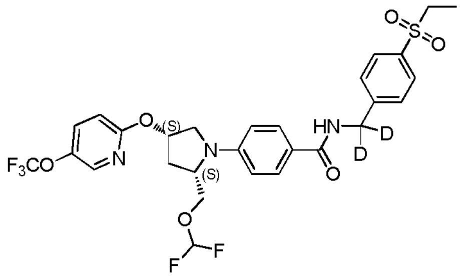

**Step 1: Synthesis of 4-(ethylsulfonyl) benzonitrile**

Sodium ethylsulfinate (1.52 g, 13.09 mmol), 4-iodobenzonitrile (1.00 g, 4.37 mmol), CuI (83 mg, 0.44 mmol), $K_3PO_4$ (1.02 g, 4.81 mmol), (2*S*,4*R*)-4-hydroxy-*N*-*m*-methylpyrrolidine -2-carboxamide (108 mg, 0.44 mmol) were successively added to anhydrous DMSO (10 mL) under nitrogen protection, and the mixture was reacted at 100 °C for 22 h. The reaction solution was diluted with EtOAc (60 mL), and the insoluble solids were removed by filtration. The filtrate was washed successively with $H_2O$ (30 mL × 2) and saturated NaCl solution (30 mL), dried over anhydrous $Na_2SO_4$, concentrated under reduced pressure. The crude product was separated by silica gel column chromatography (eluent: PE/EtOAc (v/v) = 3/1) to give a white solid (700 mg, 82%).
MS (ESI, pos.ion) m/z: 196.1 $[M+H]^+$.

**Step 2: Synthesis of dideuterium (4-(ethylsulfonyl)phenyl)methanamine**

To a solution of 4-(ethylsulfonyl)benzonitrile (280 mg, 1.43 mmol) in THF (8 mL) was added $LiAlD_4$ (120 mg, 2.86 mmol). The mixture was reacted at 45 °C for 18 h. NaOH solution (10 mL, 1 M) was added to the reaction solution to quench the reaction, and the reaction solution was concentrated under reduced pressure, extracted with EtOAc (40 mL), washed with saturated NaCl solution (20 mL), dried over anhydrous $Na_2SO_4$, concentrated under reduced pressure, and the crude product was separated by silica gel column chromatography (eluent: DCM/MeOH (v/v) = 10/1) to give yellow liquid (58 mg, 20%).
MS (ESI, pos.ion) m/z: 202.2 $[M+H]^+$.

**Step 3: Synthesis of *N*-(Dideuterium(4-(ethylsulfonyl)phenyl)methyl)-4-((2*S*,4*S*) -2-((difluoromethoxy)methyl)-4-((5-(trifluoromethoxy)pyridin-2-yl)oxy)pyrrolidin-1-yl)benza mide**

HATU (128 mg, 0.33 mmol), dideuterium(4-(ethylsulfonyl)phenyl)methanamine (58 mg, 0.29 mmol), 4-((2*S*,4*S*)-2-((difluoromethoxy)methyl)-4-((5-(trifluoromethoxy)pyridin-2-yl) oxy)pyrrolidin-1-yl)benzoic acid (100 mg, 0.22 mmol) and TEA (45 mg, 0.44 mmol) were successively added to DCM (4 mL) and the mixture was reacted at room temperature for 23 h. The reaction solution was concentrated under reduced pressure, the residue was diluted with DCM (40 mL), washed successively with HCl solution (15 mL, 1 mol/L), saturated $NaHCO_3$ solution (15 mL) and saturated NaCl solution (15 mL), dried over anhydrous $Na_2SO_4$, concentrated under reduced pressure, and the crude product was separated by silica gel column chromatography (eluent: DCM/EtOAc (v/v) = 4/1) to give a white solid (50 mg, 35%).
MS (ESI, pos.ion) m/z: 632.1 $[M+H]^+$.
$^1H$ NMR (400 MHz, $CDCl_3$) δ (ppm): 8.11 (s, 1H), 7.80 (d, *J* = 6.9 Hz, 2H), 7.75 (d, *J* = 8.6 Hz, 2H), 7.50 (d, *J* = 7.6 Hz, 3H), 6.77 (d, *J* = 8.9 Hz, 1H), 6.65 (d, *J* = 8.5 Hz, 3H), 6.24 (t, *J* = 74.4 Hz, 1H), 5.75 (s, 1H), 4.24 - 4.12 (m, 2H), 3.98 (t, *J* = 9.3 Hz, 1H), 3.72 (d, *J* = 2.2 Hz, 2H), 3.08 (q, *J* = 7.0 Hz, 2H), 2.49 - 2.36 (m, 2H), 1.25 (t, *J* = 7.0 Hz, 3H).

**Biological activity test**

**Biological Example 1 Fluorescence resonance energy transfer (FRET) test**

**1) Test method**

(1) Preparation of RORγ test buffer and 10 mM of DTT

100 mL of 1x Basic test buffer (HEPES (pH 7.4), 100 mM NaCl, 0.01% BSA) was prepared, and 154.25 mg of DTT was added. The mixture was well mixed.

(2) Preparation of compound with gradient concentration

a. Standard compound was prepared: Standard compound was diluted with 100% DMSO to 2.5 mM, then diluted 3-fold with 11 serial dilutions to a final concentration of 42.34 nM;
b. Test compound was prepared referring to the standard compound.

(3) Preparation of 1× protein solution mixture

a. Preparation of the desired amount of 2x B-RORγ LBD/SA-APC protein mixture: The concentration of B-RORγ LBD was 40 nM, and the concentration of SA-APC was 20 nM. They were mixed by gently inversion, and incubated at room temperature for 15 minutes. Then 400 nM of biotin was added. The mixture was mixed by gently inversion, and incubated at room temperature for 10 minutes;
b. Preparation of the desired amount of 2x Biotin-SRCI/SA-eu protein mixture: The concentration of Bioin-SRC1 was 40 nM, and the concentration of SA-eu was 20 nM. They were mixed by gently inversion, and incubated at room temperature for 15 minutes. Then 200 nM of biotin was added. The mixture was mixed by gently inversion, and incubated at room temperature for 10 minutes;
c. The protein mixtures prepared in step a and step b were mixed at a ratio of 1:1, and incubated at room temperature for 5 minutes;
d. 25 μL of the mixture from step c was added to the 384-well plate containing the test compound;
e. The mixture was centrifuged at 1000 rpm for one minute;
f. The mixture was incubated for 1 hour at room temperature.

(4) Data collection and calculation

After incubation at room temperature for 1 hour, the fluorescence values at 665 nm and 615 nm were measured with an EnVision plate reader, and the inhibition rate was calculated. The final $IC_{50}$ values obtained are shown in Table 1;

$$\text{Inhibition rate (\%)} = [(\text{X-Min})/(\text{Max-Min})] \times 100\%$$

X is the fluorescence value ratio of "665/615" of the test compound; Min is the average value of "665/615" of the DMSO blank control; Max is the average value of "665/615" of the 10 μM SRC.

**2) Experimental results**

Table 1 Evaluation of the compound of the present invention on the inhibitory activity of RORγ at the molecular level

| Example No. | $IC_{50}$ (nM) | Example No. | $IC_{50}$ (nM) | Example No. | $IC_{50}$ (nM) | Example No. | $IC_{50}$ (nM) |
|---|---|---|---|---|---|---|---|
| Example 1 | 12 | Example 32 | 19 | Example 64 | 3 | Example 92 | 37 |
| Example 2 | 14 | Example 33 | 132 | Example 65 | 3.2 | Example 94 | 8 |
| Example 5 | 47 | Example 34 | 133 | Example 67 | 8 | Example 95 | 45 |
| Example 6 | 9 | Example 35 | 135 | Example 68 | 75.3 | Example 96 | 64 |
| Example 7 | 8.5 | Example 39 | 3 | Example 69 | 4 | Example 98 | 17 |
| Example 8 | 7.7 | Example 40 | 25 | Example 70 | 7.6 | Example 107 | 78 |
| Example 9 | 11.9 | Example 42 | 17.8 | Example 71 | 9.3 | Example 108 | 6 |
| Example 10 | 23 | Example 44 | 51 | Example 73 | 13 | Example 109 | 6.4 |
| Example 11 | 33.8 | Example 45 | 17 | Example 74 | 11 | Example 110 | 11 |
| Example 12 | 24 | Example 46 | 13.2 | Example 75 | 12.1 | Example 111 | 5 |

(continued)

| Example No. | $IC_{50}$ (nM) | Example No. | $IC_{50}$ (nM) | Example No. | $IC_{50}$ (nM) | Example No. | $IC_{50}$ (nM) |
|---|---|---|---|---|---|---|---|
| Example 14 | 20 | Example 47 | 4.6 | Example 79 | 13 | Example 112 | 8 |
| Example 19 | 18.6 | Example 48 | 6.4 | Example 81 | 78 | Example 113 | 6.2 |
| Example 20 | 14.1 | Example 50 | 52 | Example 82 | 4 | Example 114 | 2 |
| Example 24 | 32.8 | Example 52 | 49.9 | Example 83 | 6 | Example | 13 |
| | | | | | | 115 | |
| Example 25 | 20 | Example 53 | 3 | Example 85 | 4.5 | Example 116 | 10 |
| Example 26 | 10 | Example 55 | 9 | Example 86 | 6 | Example 117 | 14 |
| Example 27 | 20.8 | Example 58 | 57.3 | Example 87 | 3 | Example 118 | 8 |
| Example 28 | 13.3 | Example 59 | 28 | Example 88 | 30 | Example 119 | 9.2 |
| Example 29 | 10.4 | Example 60 | 33.8 | Example 89 | 26 | Example 125 | 140 |
| Example 30 | 17.1 | Example 63 | 48 | Example 90 | 30 | Example 126 | 6 |
| Example 31 | 17 | / | / | / | / | / | / |

Conclusion: The experimental results show that the compounds of the present invention have good inhibitory activity on RORγ.

**Biological example 2 Pharmacokinetic evaluation I**

**1) Test method:**

ICR mice were weighed after an overnight fast for 15 h, and then randomly divided into groups according to body weight. The test compounds were formulated in a vehicle of 10% DMSO + 10% kolliphor HS15 + 80% Saline. For the test group administered intravenously (indicated by *i.v.*), the test animals were administered a dose of 1 mg/kg; for the test group administered by oral administration (indicated by *p.o.*), the test animals were administered a dose of 5 mg/kg. Then, at time points 0, 0.083 (intravenous injection group only), 0.25, 0.5, 1.0, 2.0, 5.0, 7.0 and 24 h, venous blood (approximately 0.2 mL) was collected and placed in an EDTAK2 anticoagulation tube, then centrifuged at 11,000 rpm for 2 min. Plasma was collected and stored at -20°C or -70°C until LC/MS/MS analysis. The drug concentration in plasma was measured at each time point, and the pharmacokinetic parameters were calculated according to the drug concentration-time curve.

**2) Experimental results:**

The pharmacokinetic properties of the compounds of the present invention were tested by the above experiments. The experimental results show that the compounds of the present invention have good pharmacokinetic characteristics in ICR mice. The specific experimental results are shown in Table 2 and Table 3.

Table 2 Pharmacokinetic parameters of some compounds of the present invention in ICR mice

| Example No. | Drug-delivery way | $T_{1/2}$ (h) | Cmax (μM) | $AUC_{last}$ (h*μM) |
|---|---|---|---|---|
| Example 39 | *p.o.* | 3.9 | 1.5 | 11.4 |
| Example 48 | *p.o.* | 11.4 | 1.7 | 20.9 |
| Example 91 | *p.o.* | 7.6 | 1.9 | 21.0 |
| Example 119 | *p.o.* | 6.5 | 2.38 | 28.5 |

Table 3 Oral bioavailability of some compounds of the present invention in ICR mice

| Example No. | Example 39 | Example 48 | Example 91 | Example 119 |
|---|---|---|---|---|
| Oral bioavailability (F) | 35% | 50% | 42% | 71% |

**Biological example 3 Pharmacokinetic evaluation II**

**1) Test method:**

Beagle dogs were weighed after an overnight fast for 15 h, and then randomly divided into groups according to body weight. The test compounds were formulated in a vehicle of 10% DMSO + 10% kolliphor HS15 + 80% Saline (vehicle 1) or gastric-coated capsules (vehicle 2). For the test group administered intravenously (indicated by *i.v.*), the test animals were administered 1 mg/kg of the test compound; for the test group administered by gavage or oral administration (indicated by *p.o.),* the test animals were administered 5 mg/kg of the test compound. Then, at time points 0, 0.083 (intravenous injection group only), 0.25, 0.5, 1.0, 2.0, 5.0, 7.0 and 24 h, venous blood (approximately 0.2 mL) was collected and placed in an EDTAK2 anticoagulation tube, then centrifuged at 11,000 rpm for 2 min. Plasma was collected and stored at -20°C or -70°C until LC/MS/MS analysis (because the test compound was converted into metabolites *in vivo,* the corresponding metabolites could be analyzed by LC/MS/MS). The metabolite concentration in plasma was measured at each time point, and the pharmacokinetic parameters were calculated according to the metabolite concentration-time curve.

**2) Experimental results:**

The pharmacokinetic properties of the compounds of the present invention were tested by the above experiments. The experimental results show that the compounds of the present invention have good pharmacokinetic characteristics in beagle dogs after oral administration. In particular, the compounds of Examples 120-124 and 125 of the present invention were metabolized to corresponding alcohols *in vivo* after gavage or oral administration, and the metabolized alcohol compounds all showed good pharmacokinetics in beagle dogs. The specific experimental results are shown in Table 4.

Table 4 Pharmacokinetic parameters of some example compounds of the present invention in beagle dogs

| Example No. | Vehicle | Drug-delivery way | $T_{1/2}$ (h) | Cmax (μM) | $AUC_{last}$ (h*μM) |
|---|---|---|---|---|---|
| Example 120 | Vehicle 1 | *p.o.* | 12.8 | 1.5 | 21.4 |
| Example 121 | Vehicle 1 | *p.o.* | 10.6 | 1.1 | 5.7 |
| Example 122 | Vehicle 2 | *p.o.* | 7.9 | 1.1 | 17.83 |
| Example 123 | Vehicle 2 | *p.o.* | 29.4 | 1.0 | 10.1 |
| Example 125 | Vehicle 2 | *p.o.* | 15.8 | 2.6 | 39.9 |

**Biological example 4 Stability of the compounds of the present invention in human and rat liver microsomes**

**1) Test method:**

Human or rat liver microsomes were incubated in double wells in polypropylene tubes. A typical incubation mixture included human or rat liver microsomes (final concentration: 0.5 mg protein/mL), target compound (final concentration: 1.5 μM), and K-phosphate buffered solution (containing 1.0 nM EDTA, 100 mM, pH=7.4) in a total volume of 30 μL. Compounds were dissolved in DMSO and diluted with K-phosphate buffered solution to give a final working solution concentration of 30 μM. After 10 min of pre-incubation, 25 μL of test compound or positive control solution was transferred to the microsomal solution. The mixture was mixed well and 30 μL of the mixture was immediately pipetted, ice-cold acetonitrile was added, then 15 μL of NADPH was added as $T_0$. 15 μL of NADPH (final concentration: 2 mM) was added to 30 μL of the mixture for the enzymatic reaction, and the entire experiment was carried out in an incubation tube at 37°C. At various time points (0, 20 and 60 min), the reaction was stopped by adding 150 μL of acetonitrile (with internal standard). The protein was removed by centrifugation at 4000 rpm for 10 min, and the supernatant was collected and analyzed by LC-MS/MS.

For each reaction, the concentration of compound in the incubation of human or rat liver microsomes was

plotted (expressed as a percentage) as a percentage relative to the zero time point to infer in vitro metabolic half-life and *in vivo* intrinsic hepatic clearance rate. The specific experimental results are shown in Table 5.

**2) Experimental results:**

Table 5 Experimental results of the stability of some compounds of the present invention in human and rat liver microsomes

| Example No. | Human | | Rat | |
|---|---|---|---|---|
| | Half-life $t_{1/2}$ (min) | Clearance rate (ml/min/kg) | Half-life $t_{1/2}$ (min) | Clearance rate (ml/min/kg) |
| Example 39 | 212 | 8.2 | 337 | 7.4 |
| Example 48 | 600 | 2.9 | Not metabolized | / |
| Example 91 | 416 | 4.2 | 142 | 38 |
| Example 117 | Not metabolized | / | 367 | 3.8 |
| Example 119 | 315 | 4.4 | 430 | 3.2 |

Conclusion: It can be seen from the results that the compound of the present invention has a long half-life and low clearance rate in human and rat liver microsomes, that is, the compound of the present invention has better stability in human and rat liver microsomes.

Finally, it should be noted that there are other ways of implementing the invention. Accordingly, the embodiments of the present invention will be described as examples, but are not limited to the content described in the present invention, and may also be modifications made within the scope of the present invention or equivalents added in the claims. All publications or patents cited herein are incorporated herein by reference.

## Claims

1. A compound having Formula (I), or a stereoisomer, a geometric isomer, a tautomer, an *N*-oxide, a hydrate, a solvate, a metabolite, an ester, a pharmaceutically acceptable salt or a prodrug thereof,

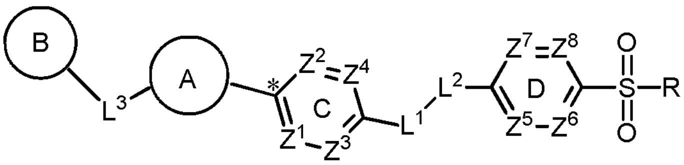

(I),

wherein:

R is $C_{1-6}$ alkyl, $C_{1-6}$ alkoxy, $C_{1-6}$ alkylamino, $C_{3-8}$ cycloalkylamino, -$C_{1-6}$ alkylene-$C_{3-8}$ cycloalkyl, $C_{3-8}$ cycloalkyl, $C_{1-6}$ haloalkyl or $C_{1-6}$ haloalkoxy;
each of $Z^1$, $Z^2$, $Z^3$, $Z^4$, $Z^5$, $Z^6$, $Z^7$ and $Z^8$ is independently $CR^1$ or N;
each $R^1$ is independently hydrogen, deuterium, cyano, fluorine, chlorine, bromine, iodine, $C_{1-6}$ alkyl, $C_{1-6}$ haloalkyl, hydroxy-substituted $C_{1-6}$ alkyl, -$C_{1-6}$ alkylene-$C_{1-6}$ alkoxy, $C_{1-6}$ alkoxy, $C_{3-8}$ cycloalkyl or 3- to 7-membered heterocyclyl;
ring A is 3- to 5-membered heterocyclyl or 7-membered heterocyclyl, and each of the 3- to 5-membered heterocyclyl and 7-membered heterocyclyl is independently and optionally substituted with 1, 2, 3, 4 or 5 $R^2$; wherein * represents the direction in which ring A is connected to ring C;
each $R^2$ is independently oxo, $R^a$, $C_{1-6}$ alkyl, -$C_{1-6}$ alkylene-$R^a$, -$OR^b$, -$C_{1-6}$ alkylene-$OR^b$, -(C=O)-$R^a$, -$C_{1-6}$ alkylene-(C=O)-$R^a$, -(C=O)-$NR^cR^d$ or -$C_{1-6}$ alkylene-(C=O)-$NR^cR^d$;
each $R^a$ is independently deuterium, fluorine, chlorine, bromine, iodine, hydroxy, amino, nitro, cyano, $C_{1-6}$ alkyl, $C_{1-6}$ alkoxy, $C_{1-6}$ haloalkyl, $C_{3-8}$ cycloalkyl, $C_{6-10}$ aryl, 5- to 10-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;

each $R^b$, $R^c$ and $R^d$ is independently hydrogen, deuterium, $C_{1-6}$ alkyl, $C_{1-6}$ haloalkyl, $C_{1-6}$ alkoxy, $C_{3-8}$ cycloalkyl, $C_{6-10}$ aryl, 5- to 10-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl;

wherein each $R^a$, $R^b$, $R^c$ and $R^d$ is independently and optionally substituted with 1, 2, 3, 4, 5 or 6 $R^g$;

each $R^g$ is independently deuterium, fluorine, chlorine, bromine, iodine, oxo, hydroxy, amino, nitro, cyano, $C_{1-6}$ alkyl, $C_{1-6}$ haloalkyl, $C_{1-6}$ alkoxy, $C_{1-6}$ haloalkoxy, $C_{3-8}$ cycloalkyl or 3- to 7-membered heterocyclyl;

ring B is $C_{6-10}$ aryl, $C_{3-8}$ cycloalkyl, 5- to 12-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3 or 4 $R^e$;

each $R^e$ is independently deuterium, fluorine, chlorine, bromine, iodine, cyano, nitro, hydroxy, amino, $C_{1-6}$ alkyl, $C_{1-6}$ alkoxy, $C_{3-8}$ cycloalkyl, 3- to 7-membered heterocyclyl, $C_{1-6}$ haloalkyl or $C_{1-6}$ haloalkoxy, wherein each of the $C_{1-6}$ alkyl, $C_{1-6}$ alkoxy, $C_{3-8}$ cycloalkyl, 3- to 7-membered heterocyclyl, $C_{1-6}$ haloalkyl and $C_{1-6}$ haloalkoxy is independently and optionally substituted with 1, 2, 3 or 4 substituents selected from cyano, nitro, hydroxy, amino or 3- to 7-membered heterocyclyl;

$L^1$ is $-S(O)_2-NH-$, $-NH-S(O)_2-$, -S(O)-NH-, -NH-S(O)-, -C(=O)NH- or -NHC(=O)-;

$L^2$ is a bond or $-CR^3R^4-$;

$L^3$ is -C(=O)-, -S-, -O-, $-NR^5-$ or $-CR^6R^7-$;

$R^3$ is hydrogen, deuterium, hydroxy-substituted $C_{1-6}$ alkyl, $-C_{1-6}$ alkylene-OC(=O)-$R^f$, $-C_{1-6}$ alkylene-C(=O)-O-$R^f$ or $-C_{1-6}$ alkylene-$R^f$;

$R^f$ is $C_{2-6}$ alkyl, amino-substituted $C_{1-6}$ alkyl, $C_{1-6}$ haloalkyl, $-C_{1-6}$ alkylene-C(=O)-$OR^h$ or -OP(=O)-($OR^m$)($OR^n$);

wherein each of $R^h$, $R^m$ and $R^n$ is independently hydrogen, deuterium, sodium, potassium, $C_{1-6}$ alkyl or $C_{1-6}$ haloalkyl;

$R^4$ is hydrogen, deuterium or hydroxy-substituted $C_{1-6}$ alkyl;

$R^5$ is hydrogen, deuterium, $C_{1-4}$ alkyl, $C_{1-4}$ haloalkyl, $C_{1-4}$ alkoxy, $C_{3-6}$ cycloalkyl or 3- to 6-membered heterocyclyl;

each of $R^6$ and $R^7$ is independently hydrogen, deuterium, $C_{1-4}$ alkyl, $C_{1-4}$ haloalkyl, $C_{1-4}$ haloalkoxy, $C_{1-4}$ alkoxy, $C_{3-6}$ cycloalkyl or 3- to 6-membered heterocyclyl.

**2.** The compound of claim 1, wherein R is $C_{1-4}$ alkyl, $C_{1-4}$ alkoxy, $C_{1-4}$ alkylamino, $C_{3-6}$ cycloalkylamino, $-C_{1-4}$ alkylene-$C_{3-6}$ cycloalkyl, $C_{3-6}$ cycloalkyl, $C_{1-4}$ haloalkyl or $C_{1-4}$ haloalkoxy;

each $R^1$ is independently hydrogen, deuterium, cyano, fluorine, chlorine, bromine, iodine, $C_{1-4}$ alkyl, $C_{1-4}$ haloalkyl, hydroxy-substituted $C_{1-4}$ alkyl, $-C_{1-4}$ alkylene-$C_{1-4}$ alkoxy, $C_{1-4}$ alkoxy, $C_{3-6}$ cycloalkyl or 3- to 7-membered heterocyclyl.

**3.** The compound of claim 1 or 2, wherein each $R^2$ is independently oxo, $R^a$, $C_{1-4}$ alkyl, $-C_{1-4}$ alkylene-$R^a$, $-OR^b$, $-C_{1-4}$ alkylene-$OR^b$, -(C=O)-$R^a$, $-C_{1-4}$ alkylene-(C=O)-$R^a$, -(C=O)-$NR^cR^d$ or $-C_{1-4}$ alkylene-(C=O)-$NR^cR^d$;

each $R^a$ is independently deuterium, fluorine, chlorine, bromine, iodine, hydroxy, amino, nitro, cyano, $C_{1-4}$ alkyl, $C_{1-4}$ alkoxy, $C_{1-4}$ haloalkyl, $C_{3-6}$ cycloalkyl, $C_{6-10}$ aryl, 5- to 7-membered heteroaryl, 3- to 7-membered heterocyclyl, 5- to 12-membered spirocyclyl, 4- to 12-membered fused cyclyl, 5- to 12-membered spiro heterocyclyl or 4- to 12-membered fused heterocyclyl;

each $R^b$, $R^c$ and $R^d$ is independently hydrogen, deuterium, $C_{1-4}$ alkyl, $C_{1-4}$ haloalkyl, $C_{1-4}$ alkoxy, $C_{3-6}$ cycloalkyl, $C_{6-10}$ aryl, 5- to 7-membered heteroaryl, 3- to 7-membered heterocyclyl, 5-to 12-membered spirocyclyl, 4- to 12-membered fused cyclyl, 5- to 12-membered spiro heterocyclyl or 4- to 12-membered fused heterocyclyl; or, $R^c$ and $R^d$ together with the N atom to which they are attached form 4- to 7-membered heterocyclyl;

wherein each $R^a$, $R^b$, $R^c$ and $R^d$ is independently and optionally substituted with 1, 2, 3, 4, 5 or 6 $R^g$;

each $R^g$ is independently deuterium, fluorine, chlorine, bromine, iodine, oxo, hydroxy, amino, nitro, cyano, $C_{1-4}$ alkyl, $C_{1-4}$ haloalkyl, $C_{1-4}$ alkoxy, $C_{1-4}$ haloalkoxy, $C_{3-6}$ cycloalkyl or 3- to 7-membered heterocyclyl.

**4.** The compound of any one of claims 1 to 3, wherein the ring B is $C_{6-10}$ aryl, $C_{3-6}$ cycloalkyl, 5-to 7-membered heteroaryl, 3- to 7-membered heterocyclyl, 4- to 12-membered fused heterocyclyl, 5- to 12-membered spiro heterocyclyl, 4- to 12-membered fused cyclyl or 5- to 12-membered spirocyclyl, wherein the ring B is optionally substituted with 1, 2, 3, 4, 5 or 6 $R^e$;

each $R^e$ is independently deuterium, fluorine, chlorine, bromine, iodine, cyano, nitro, hydroxy, amino, $C_{1-4}$ alkyl, $C_{1-4}$ alkoxy, $C_{3-6}$ cycloalkyl, 3- to 7-membered heterocyclyl, $C_{1-4}$ haloalkyl or $C_{1-4}$ haloalkoxy, wherein each of the

$C_{1-4}$ alkyl, $C_{1-4}$ alkoxy, $C_{3-6}$ cycloalkyl, 3- to 7-membered heterocyclyl, $C_{1-4}$ haloalkyl and $C_{1-4}$ haloalkoxy is independently and optionally substituted with 1, 2, 3 or 4 substituents selected from cyano, nitro, hydroxy, amino or 3- to 7-membered heterocyclyl.

**5.** The compound of any one of claims 1 to 4, wherein $R^3$ is hydrogen, deuterium, hydroxy-substituted $C_{1-4}$ alkyl, -$C_{1-4}$ alkylene-OC(=O)-$R^f$, -$C_{1-4}$ alkylene-C(=O)-O-$R^f$ or -$C_{1-4}$ alkylene-$R^f$;

$R^f$ is $C_{2-4}$ alkyl, amino-substituted $C_{1-4}$ alkyl, $C_{1-4}$ haloalkyl, -$C_{1-4}$ alkylene-C(=O)-$OR^h$ or -OP(=O)-($OR^m$)($OR^n$); wherein each $R^h$, $R^m$ and $R^n$ is independently hydrogen, deuterium, sodium, potassium, $C_{1-4}$ alkyl or $C_{1-4}$ haloalkyl;
$R^4$ is hydrogen, deuterium or hydroxy-substituted $C_{1-4}$ alkyl.

**6.** The compound of any one of claims 1 to 5, wherein R is methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, methylamino, ethylamino, dimethylamino, cyclopropylamino, cyclobutylamino, cyclopentylamino, cyclohexylamino, -methylene-cyclopropyl, -methylene-cyclobutyl, -methylene-cyclopentyl, -methylene-cyclohexyl, -ethylene-cyclopropyl, -ethylene-cyclobutyl, -ethylene-cyclopentyl, -ethylene-cyclohexyl, cyclopropyl, cyclobutyl, cyclopentyl or cyclohexyl.

**7.** The compound of any one of claims 1 to 6, wherein each $R^1$ is independently hydrogen, deuterium, cyano, fluorine, chlorine, bromine, iodine, methyl, ethyl, *n*-propyl, isopropyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, hydroxymethyl, 2-hydroxyethyl, -methylenemethoxy, -methyleneethoxy, -methylene-*n*-propoxy, -methyleneisopropoxy, cyclopropyl, cyclobutyl, cyclopentyl or cyclohexyl.

**8.** The compound of any one of claims 1 to 7, wherein ring A is

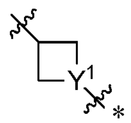, 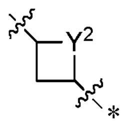, 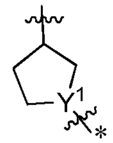,

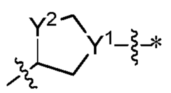, 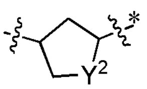, 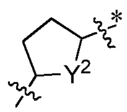, 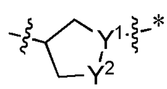, 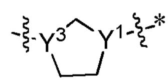, 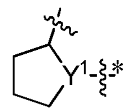, 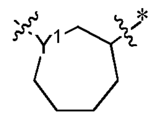,

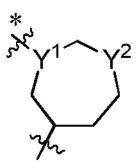, 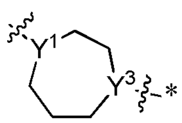 or 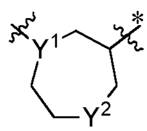;

wherein, each $Y^1$ and $Y^3$ is independently N or CH; $Y^2$ is O, S, NH or $CH_2$; * represents the direction in which ring A is connected to ring C; the ring A is optionally substituted with 1, 2, 3, 4 or 5 $R^2$.

**9.** The compound of any one of claims 1 to 8, wherein ring A is

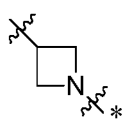, 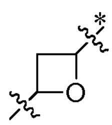,

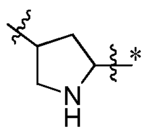, 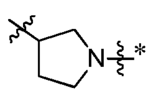, 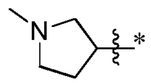, 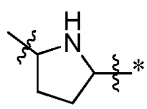, 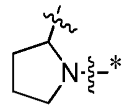, 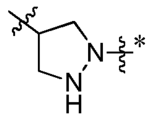, 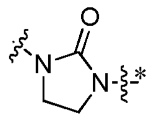,

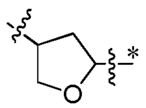, 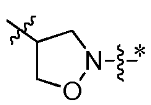, 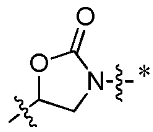, 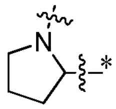, 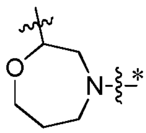, 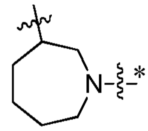, 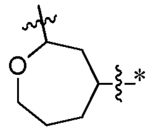,

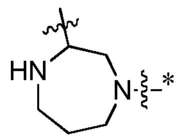, 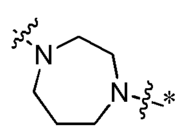 or 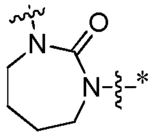;

wherein, * represents the direction in which ring A is connected to ring C; the ring A is optionally substituted with 1, 2, 3, 4 or 5 $R^2$.

**10.** The compound of any one of claims 1 to 9, wherein each $R^2$ is independently oxo, $R^a$, methyl, ethyl, *n*-propyl, isopropyl, *n*-butyl, isobutyl, sec-butyl, *tert*-butyl, $-CH_2R^a$, $-CH_2CH_2R^a$, $-CH_2CH_2CH_2R^a$, $-C(CH_3)_2R^a$, $-CH(CH_3)CH_2R^a$, $-OR^b$, $-CH_2OR^b$, $-CH_2CH_2OR^b$, $-CH_2CH_2CH_2OR^b$, $-C(CH_3)_2OR^b$, $-CH(CH_3)CH_2OR^b$, $-(C=O)-R^a$, $-CH_2(C=O)-R^a$, $-CH_2CH_2(C=O)-R^a$, $-CH_2CH_2CH_2(C=O)-R^a$, $-C(CH_3)_2(C=O)-R^a$, $-CH(CH_3)CH_2(C=O)-R^a$, $-(C=O)-NR^cR^d$, $-CH_2(C=O)-NR^cR^d$, $-CH_2CH_2(C=O)-NR^cR^d$, $-CH_2CH_2CH_2(C=O)-NR^cR^d$, $-C(CH_3)_2(C=O)-NR^cR^d$ or $-CH(CH_3)CH_2(C=O)-NR^cR^d$.

**11.** The compound of any one of claims 1 to10, wherein each $R^a$ is independently deuterium, fluorine, chlorine, bromine, iodine, hydroxy, amino, nitro, cyano, methyl, ethyl, *n*-propyl, isopropyl, *n*-butyl, isobutyl, *sec*-butyl, *tert*-butyl, methoxy, ethoxy, isopropoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, oxetanyl, azetidinyl, pyrrolidinyl, tetrahydrofuranyl, tetrahydropyranyl, morpholinyl, thiomorpholinyl, piperidinyl, piperazinyl, oxepanyl, cyclopropyl, cyclobutyl, cyclopentyl, cyclohexyl, phenyl, thiazolyl, pyrazolyl, imidazolyl, furanyl, oxazolyl, isoxazolyl, triazolyl, thienyl, pyrrolyl, pyridyl, pyrimidinyl,

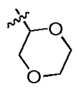, 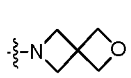, 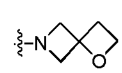, 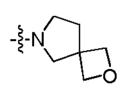, 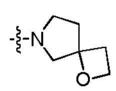, 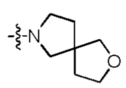,

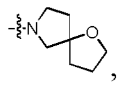, 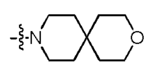, 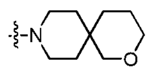 or 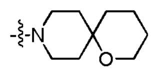;

each $R^b$, $R^c$ and $R^d$ is independently hydrogen, deuterium, methyl, ethyl, *n*-propyl, isopropyl, *n*-butyl, isobutyl, *sec*-butyl, *tert*-butyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, methoxy, ethoxy, *n*-propoxy, isopropoxy, *n*-butoxy, isobutoxy, *sec*-butoxy, *tert*-butoxy, cyclopropyl, cyclobutyl, cyclopentyl, cyclohexyl, phenyl, oxetanyl, azetidinyl, pyrrolidinyl, tetrahydrofuranyl, tetrahydropyranyl, morpholinyl, thiomorpholinyl, piperidinyl, piperazinyl, oxepanyl, thiazolyl, pyrazolyl, imidazolyl, furanyl, oxazolyl, isoxazolyl, triazolyl, thienyl, pyrrolyl, pyridyl, pyrimidinyl,

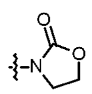, 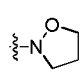, 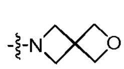, 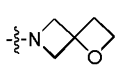, 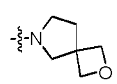, 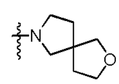, 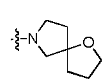, 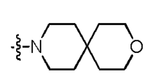,

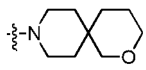 or 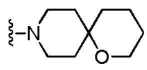;

or, $R^c$ and $R^d$ together with the N atom to which they are attached form

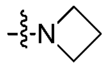, 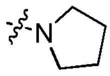, 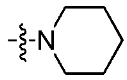 or 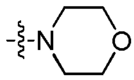;

wherein each $R^a$, $R^b$, $R^c$ and $R^d$ is independently and optionally substituted with 1, 2, 3, 4, 5 or 6 $R^g$.

**12.** The compound of any one of claims 1 to 11, wherein each $R^g$ is independently deuterium, fluorine, chlorine, bromine, iodine, oxo, hydroxy, amino, nitro, cyano, methyl, ethyl, *n*-propyl, isopropyl, *n*-butyl, isobutyl, sec-butyl, tert-butyl, methoxy, ethoxy, *n*-propoxy, *iso*-propoxy, *n*-butoxy, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$, $-OCF_2CF_3$, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl or oxepanyl.

**13.** The compound of any one of claims 1 to 12, wherein the ring B is phenyl, cyclopropyl, cyclobutyl, cyclopentyl, cyclohexyl, pyridyl, pyrimidinyl, pyrrolyl, pyrazolyl, thiazolyl, imidazolyl, furanyl, oxazolyl, isoxazolyl, thienyl, triazolyl, tetrazolyl, piperazinyl, piperidinyl, morpholinyl, thiomorpholinyl, tetrahydropyranyl, tetrahydrofuranyl, pyrrolidinyl, azetidinyl, oxetanyl,

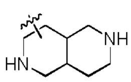, 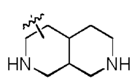, 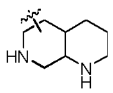, 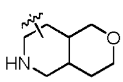, 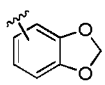, 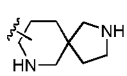, 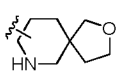, 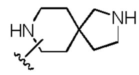

or

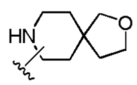;

wherein the ring B is optionally substituted with 1, 2, 3 or 4 $R^e$.

**14.** The compound of any one of claims 1 to 13, wherein each $R^e$ is independently deuterium, fluorine, chlorine, bromine, iodine, cyano, nitro, hydroxy, methyl, ethyl, *n*-propyl, isopropyl, methoxy, ethoxy, *n*-propoxy, isopropoxy, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CF_3$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-CF_2CF_3$, $-OCH_2F$, $-OCHF_2$, $-OCF_3$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$, $-OCF_2CHF_2$ or $-OCF_2CF_3$; wherein each of the methyl, ethyl, *n*-propyl, isopropyl, methoxy, ethoxy, *n*-propoxy, isopropoxy, cyclopropyl, cyclobutyl, cyclopentyl, cyclohexyl, oxetanyl, azetidinyl, pyrrolidinyl, tetrahydrofuranyl, tetrahydropyranyl, morpholinyl, thiomorpholinyl, piperidinyl, piperazinyl, oxepanyl, $-CH_2F$, $-CHF_2$, $-CHFCH_2F$, $-CF_2CH_3$, $-CH_2CHF_2$, $-CH_2CF_3$, $-CF_2CH_2CH_3$, $-CH_2CH_2CHF_2$, $-CF_2CH_2F$, $-CF_2CHF_2$, $-OCH_2F$, $-OCHF_2$, $-OCHFCH_2F$, $-OCF_2CH_3$, $-OCH_2CHF_2$, $-OCH_2CF_3$, $-OCF_2CH_2CH_3$, $-OCH_2CH_2CHF_2$, $-OCF_2CH_2F$ and $-OCF_2CHF_2$ is independently and optionally substituted with 1, 2, 3 or 4 substituents selected from cyano, nitro, hydroxy, amino, piperazinyl, piperidinyl, morpholinyl, thiomorpholinyl, tetrahydropyranyl, tetrahydrofuranyl, pyrrolidinyl, azetidinyl or oxetanyl.

**15.** The compound of any one of claims 1 to 14, wherein $R^3$ is hydrogen, deuterium, hydroxymethyl, 2-hydroxyethyl, 1-hydroxyethyl, 3-hydroxy-*n*-propyl, 2-hydroxy-1-methylethyl, $-CH_2OC(=O)R^f$, $-CH_2CH_2OC(=O)R^f$, $-CH_2CH_2CH_2OC(=O)R^f$, $-CH(CH_3)_2OC(=O)R^f$, $-CH_2C(=O)OR^f$, $-CH_2CH_2C(=O)OR^f$, $-CH_2CH_2CH_2C(=O)OR^f$, $-CH(CH_3)_2C(=O)OR^f$, $-CH_2R^f$, $-CH_2CH_2R^f$, $-CH_2CH_2CH_2R^f$ or $-CH(CH_3)_2R^f$;

$R^f$ is ethyl, *n*-propyl, isopropyl, $-CH_2NH_2$, $-CH_2CH_2NH_2$, $-CH_2CH_2CH_2NH_2$, $-CH(NH_2)CH(CH_3)_2$, $-CH_2F$, $-CHF_2$,

$-CF_3$, $-CHFCH_2F$, $-CF_2CH_3$, $-CH_2CHF_2$, $-CH_2CF_3$, $-CH_2C(=O)ONa$, $-CH_2CH_2C(=O)-ONa$, $-CH_2CH_2CH_2C(=O)ONa$, $-CH(CH_3)_2C(=O)ONa$ or $-O-P(=O)(ONa)_2$;
$R^4$ is hydrogen, deuterium, hydroxymethyl, 2-hydroxyethyl, 1-hydroxyethyl, 3-hydroxy-*n*-propyl or 2-hydroxy-1-methylethyl.

**16.** The compound of any one of claims 1 to 15, wherein $R^5$ is hydrogen, deuterium, methyl, ethyl, isopropyl, *n*-propyl, *n*-butyl, isobutyl, *sec*-butyl, *tert*-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl;
each of $R^6$ and $R^7$ is independently hydrogen, deuterium, methyl, ethyl, isopropyl, *n*-propyl, *n*-butyl, isobutyl, sec-butyl, *tert*-butyl, $-CH_2F$, $-CHF_2$, $-CF_3$, $-CHFCH_2F$, $-CF_2CH_3$, $-CH_2CHF_2$, $-CH_2CF_3$, methoxy, ethoxy, *n*-propoxy, isopropoxy, cyclopropyl, cyclobutyl or azetidinyl.

**17.** The compound of any one of claims 1 to 16 having one of the following structures:

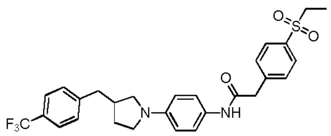 (1), 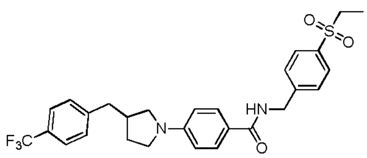 (2), 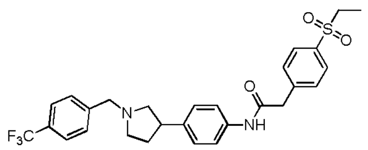 (3),

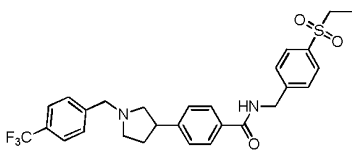 (4), 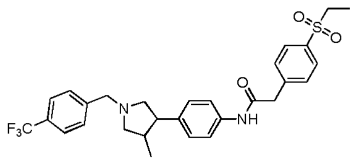 (5), 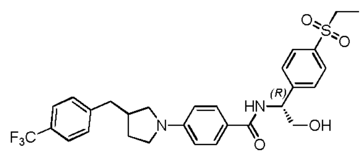 (6),

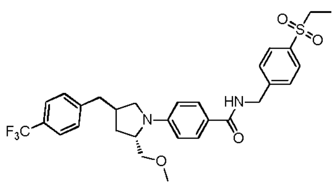 (7), 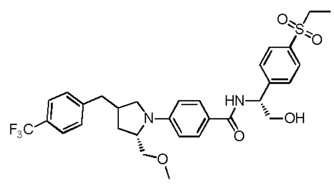 (8), 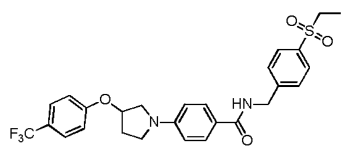 (9),

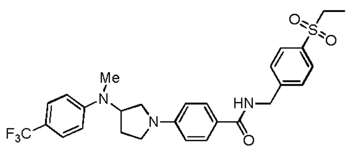 (10), 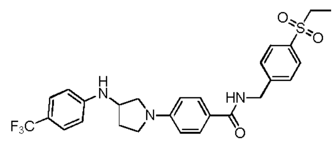 (11), 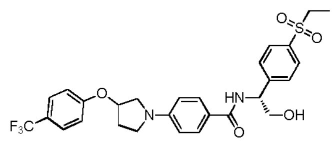 (12),

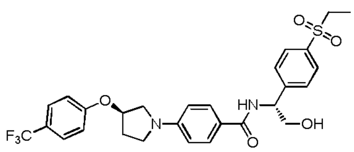 (13), 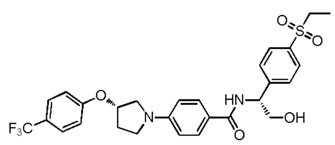 (14), 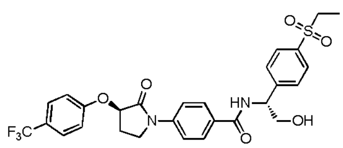 (15),

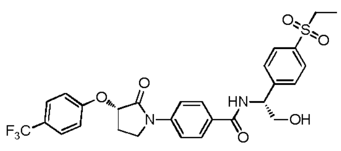 (16), 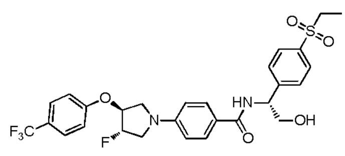 (17), 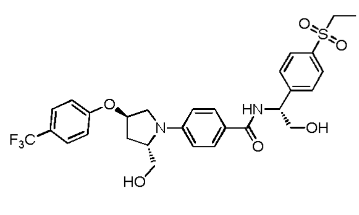 (18),

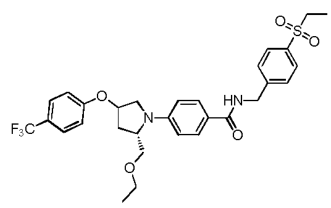
(19),

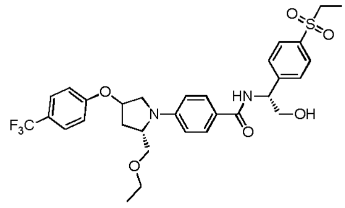
(20),

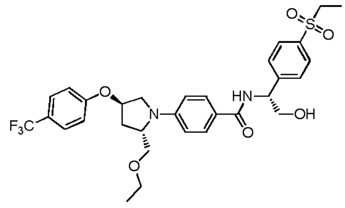
(21),

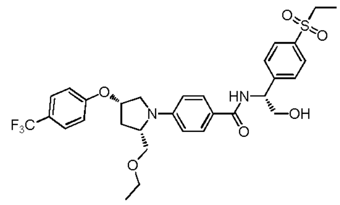
(22),

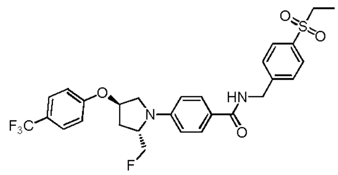
(23),

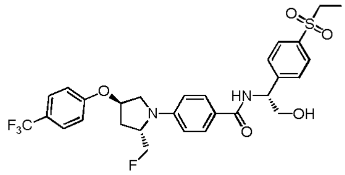
(24),

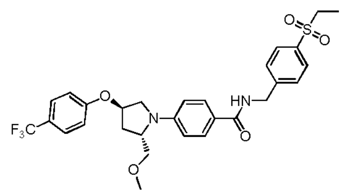
(25),

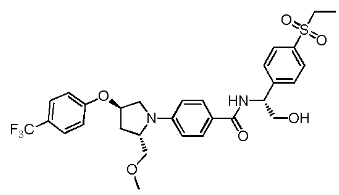
(26),

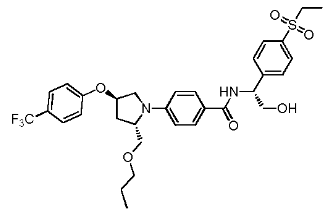
(27),

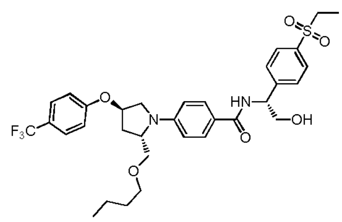
(28),

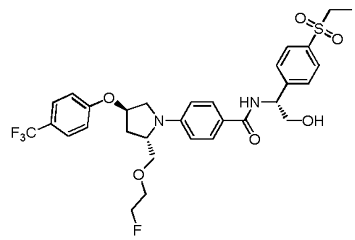
(29),

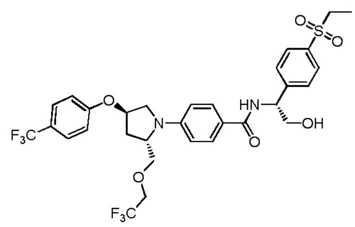
(30),

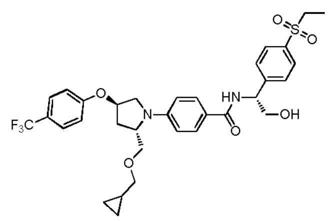
(31),

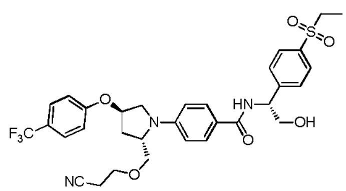
(32),

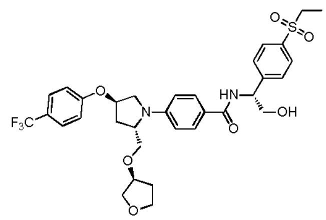
(33),

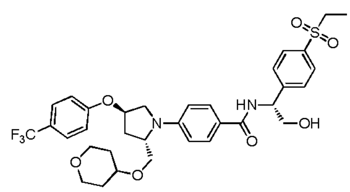
(34),

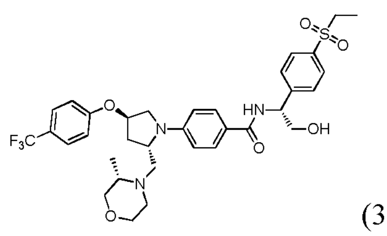
(35),

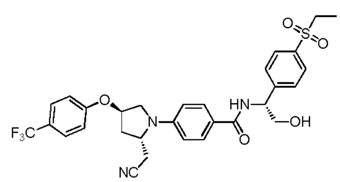
(36),

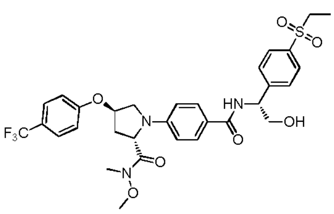 (37), 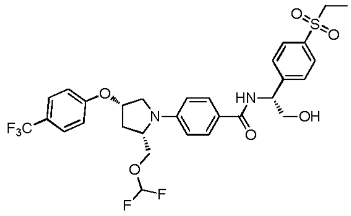 (38), 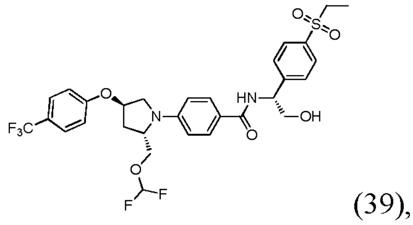 (39),

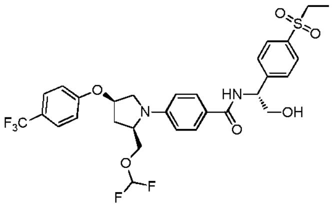 (40), 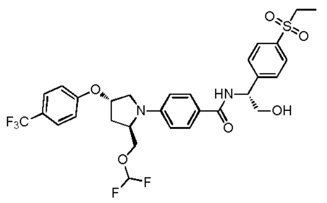 (41), 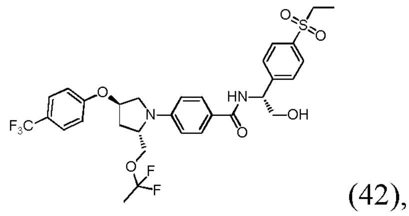 (42),

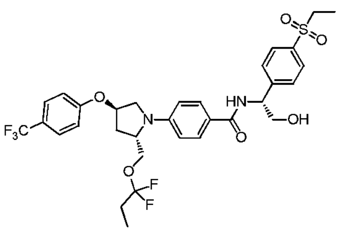 (43), 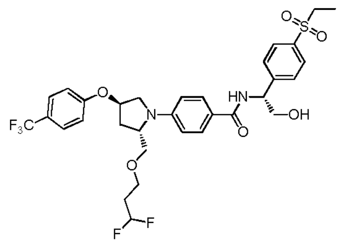 (44), 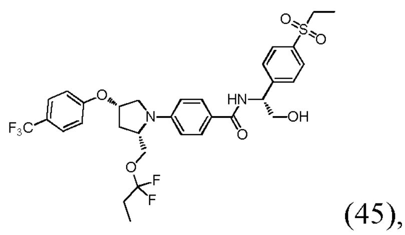 (45),

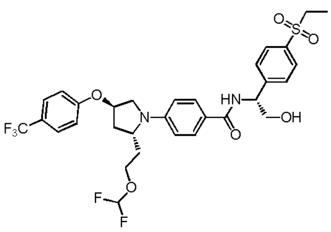 (46), 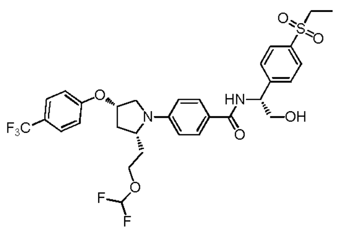 (47), 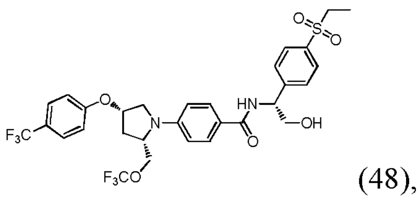 (48),

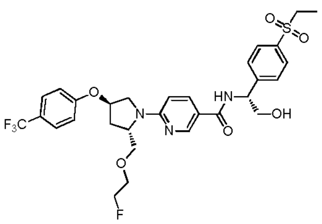 (49), 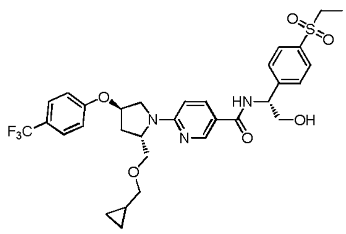 (50), 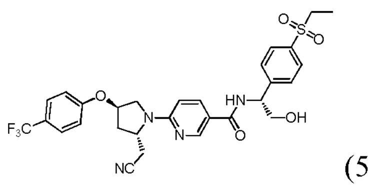 (51),

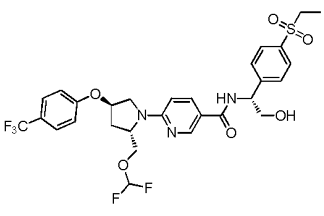 (52), 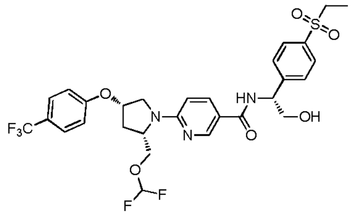 (53), 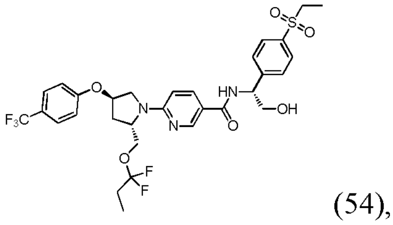 (54),

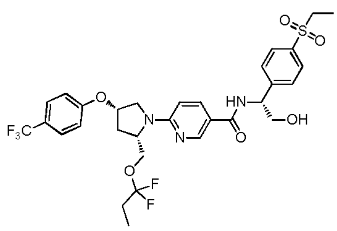 (55), 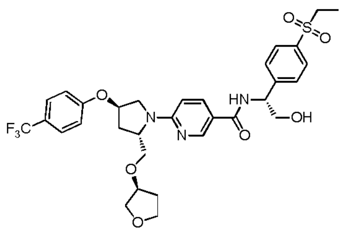 (56), 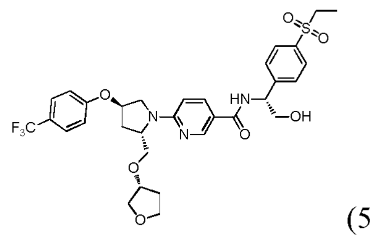 (57),

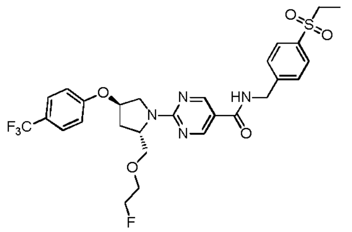 (58), 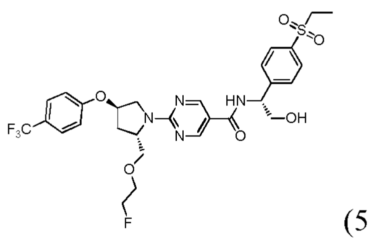 (59), 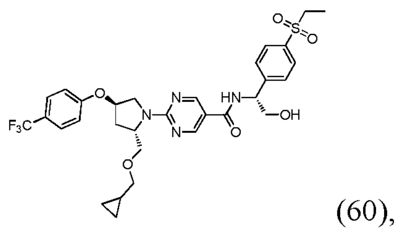 (60),

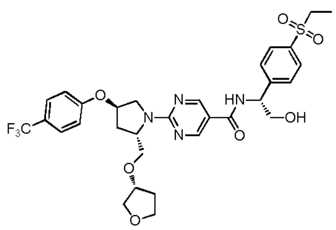 (61), 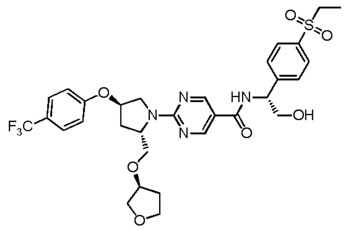 (62), 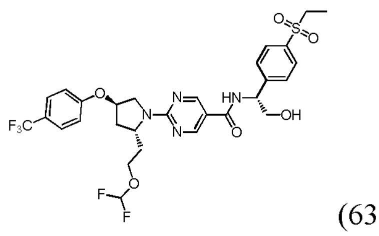 (63),

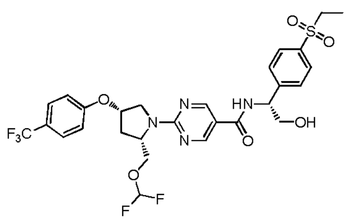 (64), 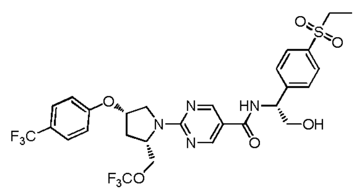 (65), 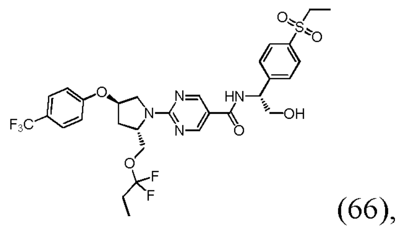 (66),

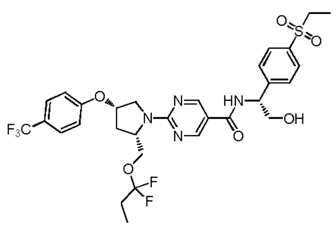 (67), 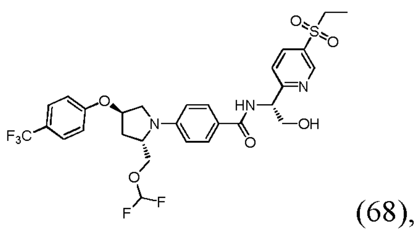 (68), 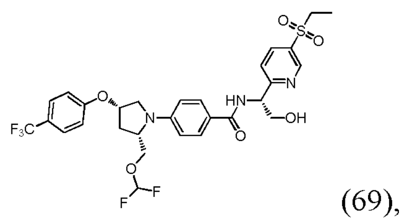 (69),

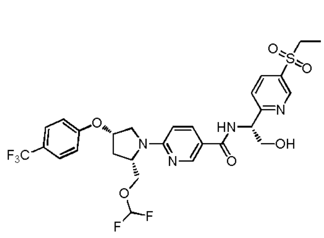 (70), 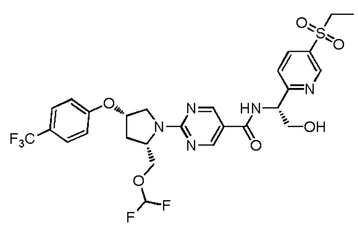 (71), 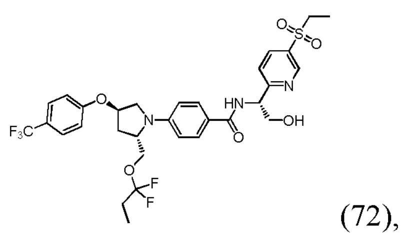 (72),

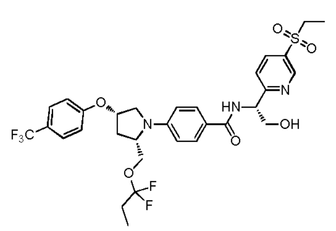 (73),

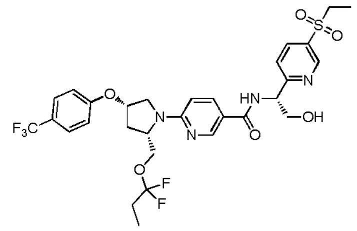 (74),

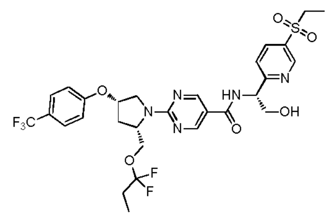 (75),

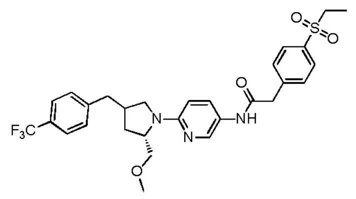 (76),

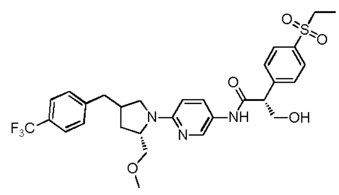 (77),

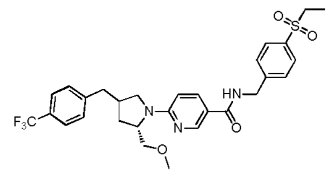 (78),

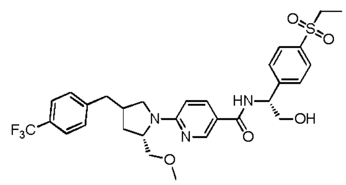 (79),

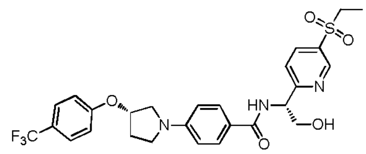 (80),

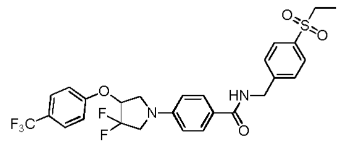 (81),

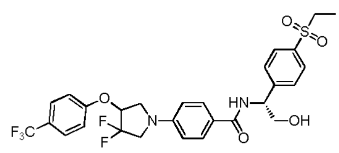 (82),

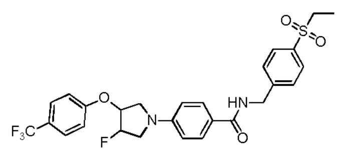 (83),

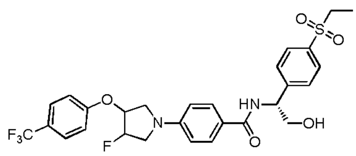 (84),

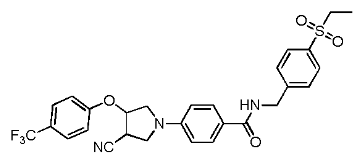 (85),

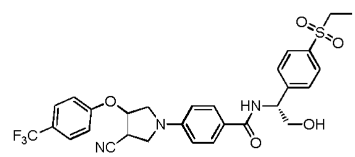 (86),

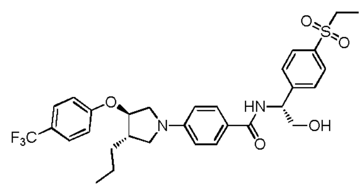 (87),

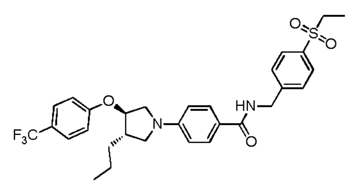 (88),

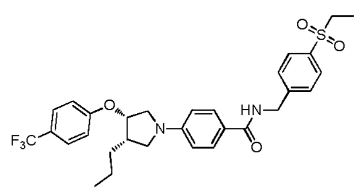 (89),

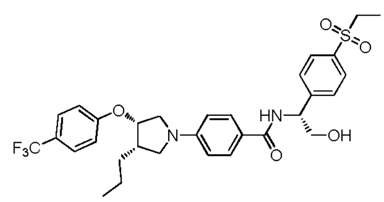 (90),

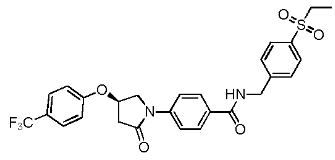 (91), 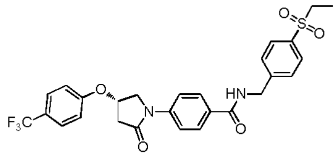 (92), 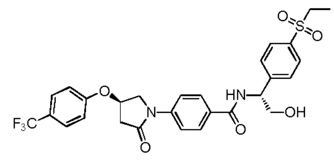 (93),

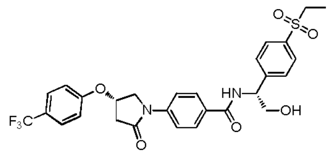 (94), 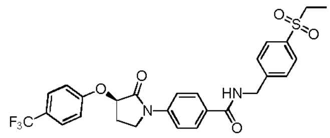 (95), 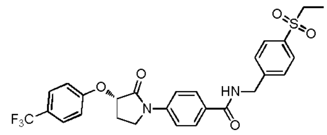 (96),

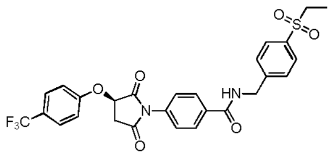 (97), 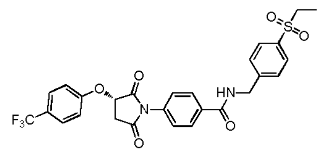 (98), 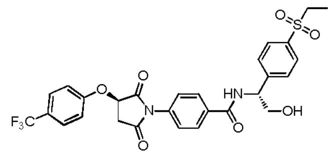 (99),

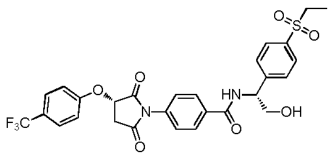 (100), 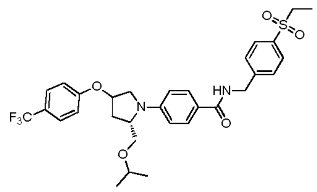 (101), 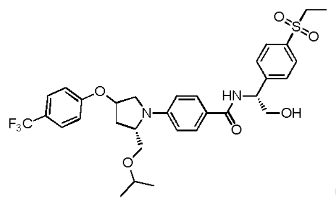 (102),

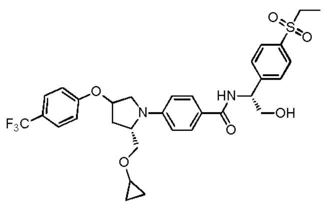 (103), 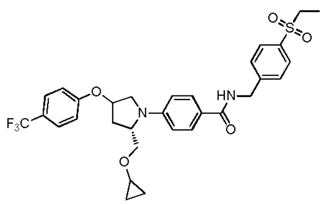 (104), 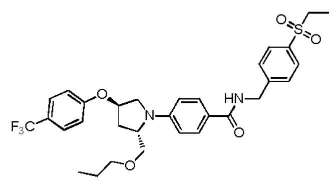 (105),

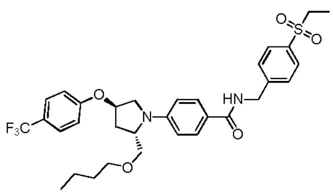 (106), 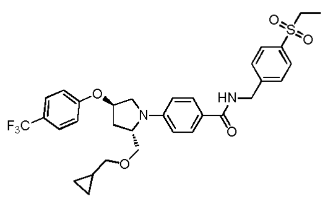 (107), 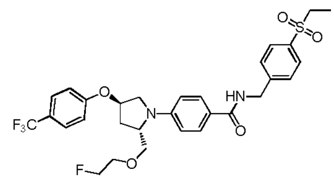 (108),

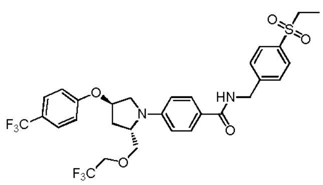 (109), 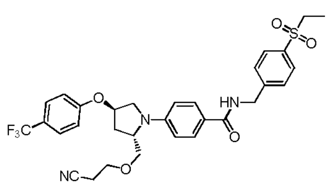 (110), 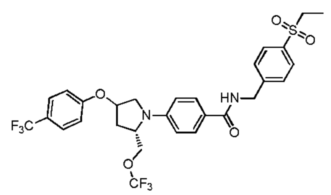 (111),

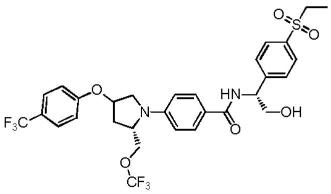 (112), 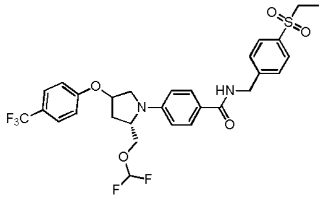 (113), 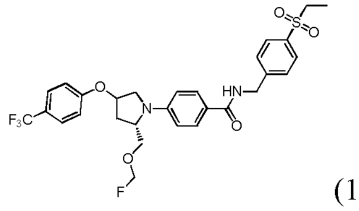 (114),

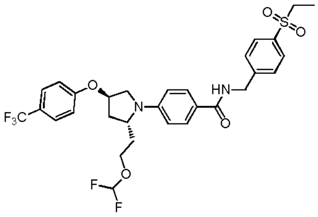 (115), 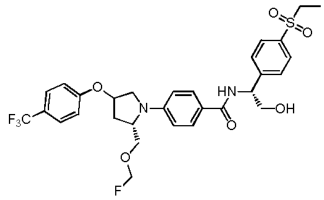 (116), 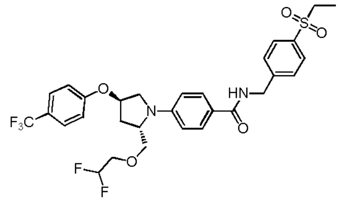 (117),

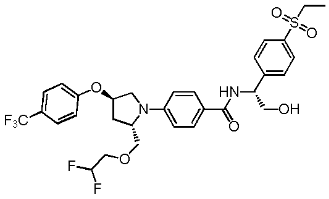 (118), 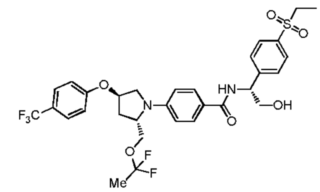 (119), 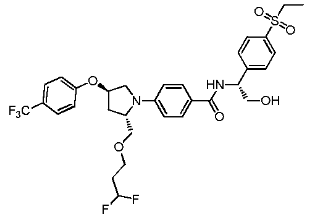 (120),

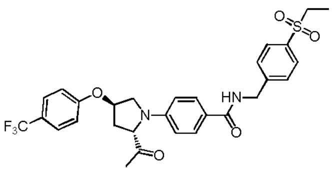 (121), 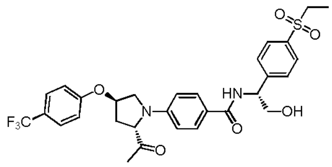 (122), 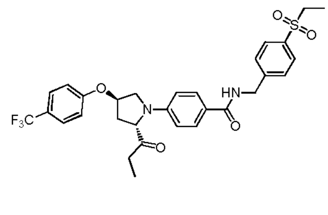 (123),

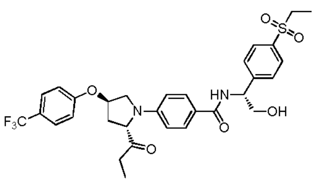 (124), 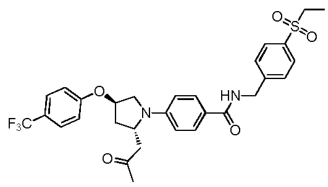 (125), 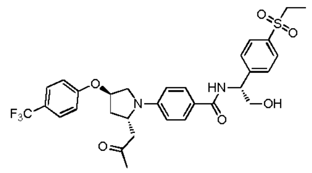 (126),

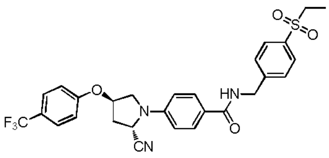 (127), 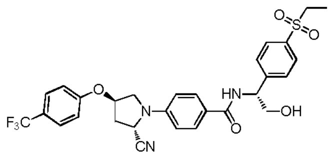 (128), 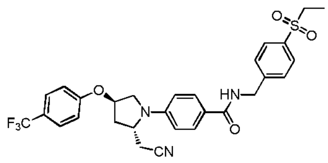 (129),

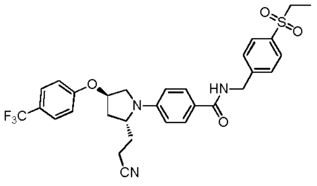 (130), 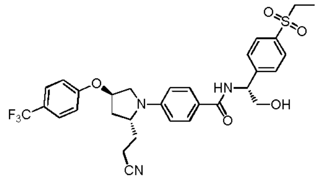 (131), 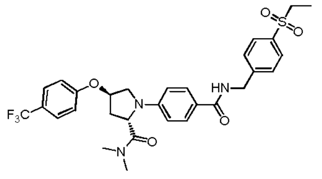 (132),

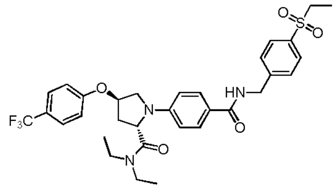 (133), 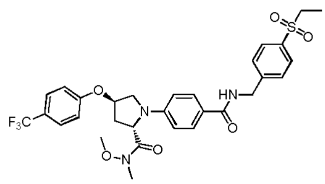 (134), 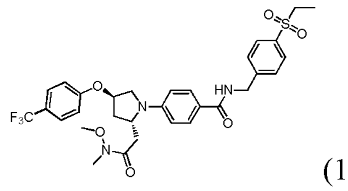 (135),

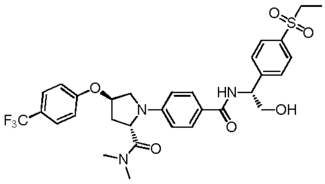 (136), 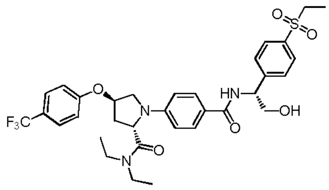 (137), 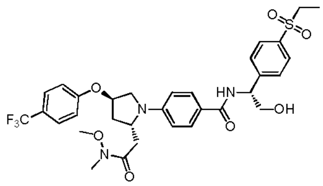 (138),

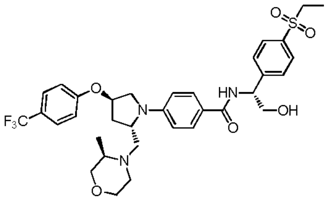 (139), 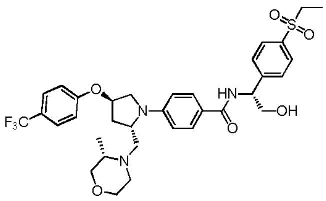 (140), 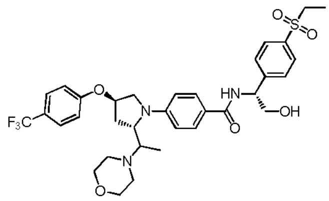 (141),

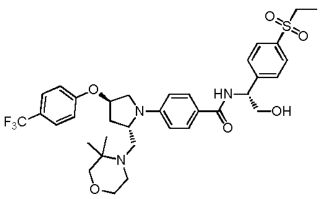 (142), 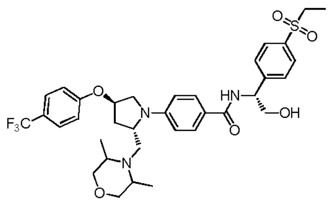 (143), 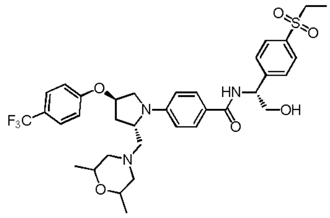 (144),

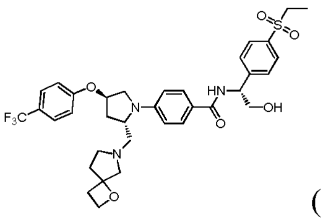 (145), 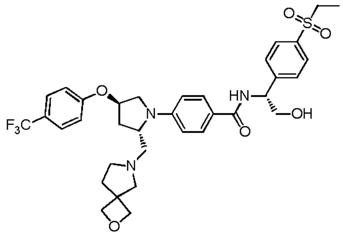 (146), 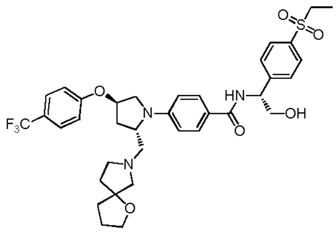 (147),

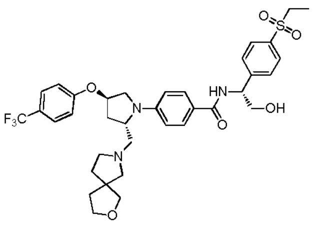 (148), 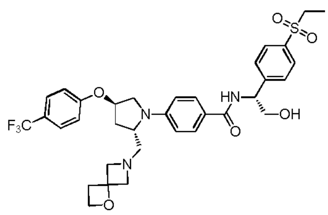 (149), 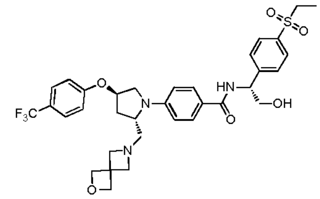 (150),

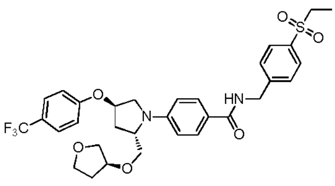 (151), 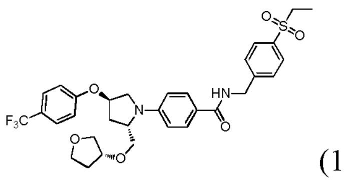 (152), 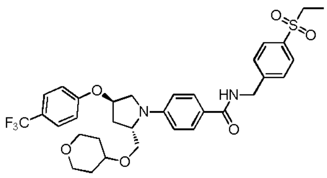 (153),

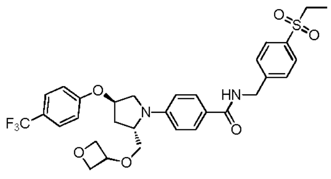 (154), 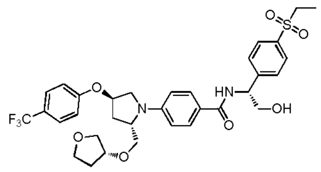 (155), 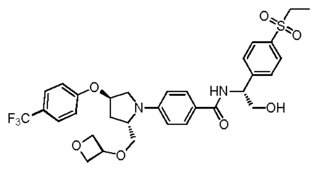 (156),

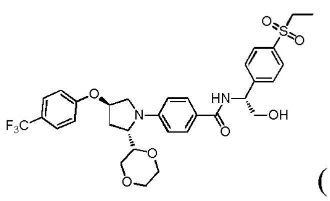 (157), 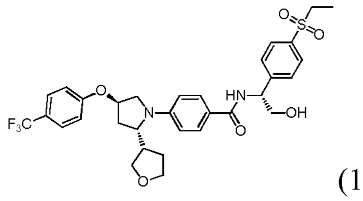 (158), 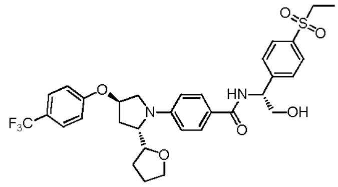 (159),

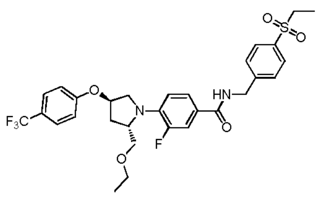 (160), 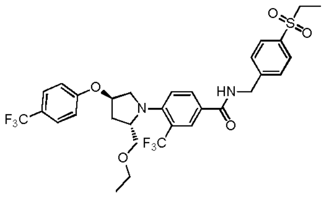 (161), 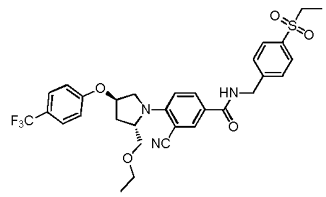 (162),

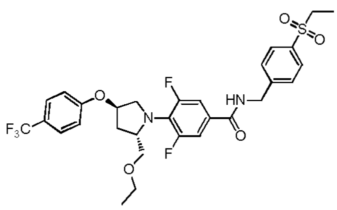 (163), 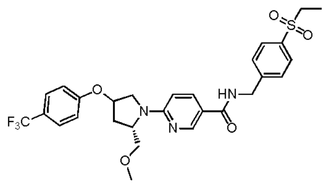 (164), 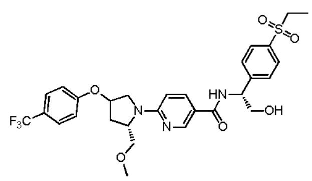 (165),

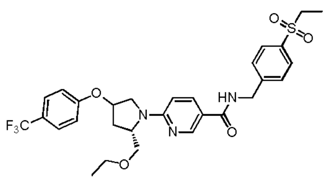 (166), 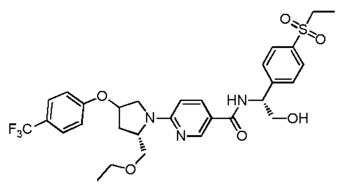 (167), 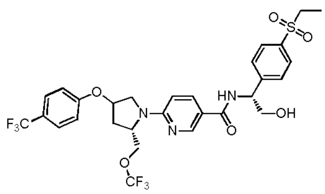 (168),

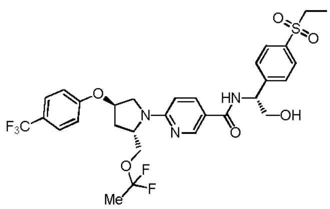 (169), 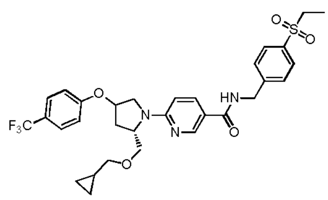 (170), 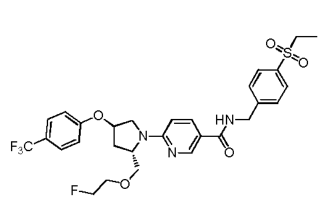 (171),

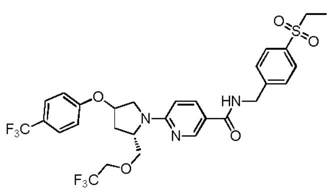 (172), 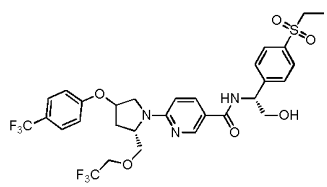 (173), 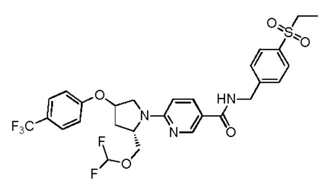 (174),

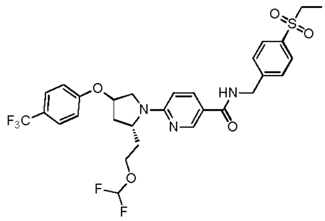

(175),

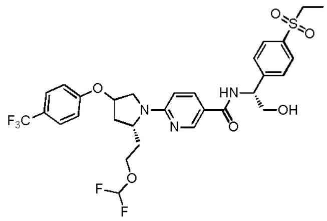

(176),

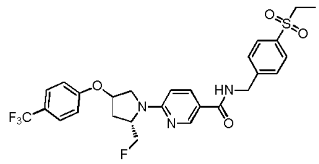

(177),

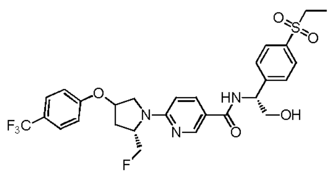

(178),

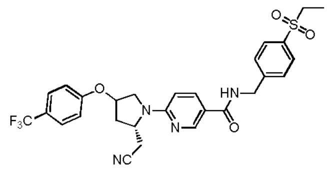

(179),

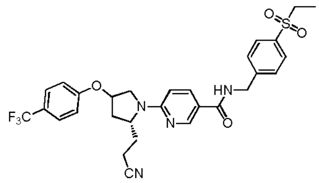

(180),

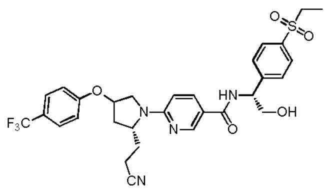

(181),

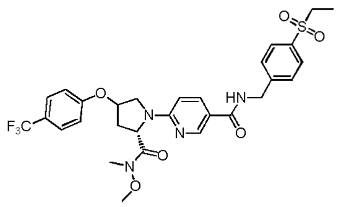

(182),

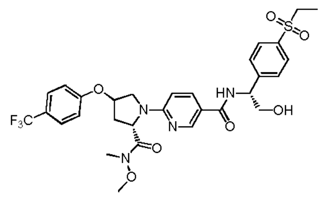

(183),

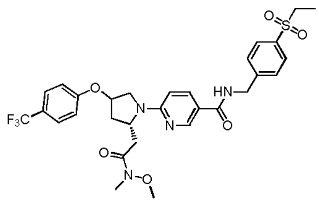

(184),

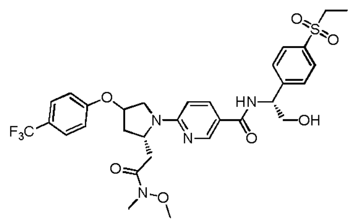

(185),

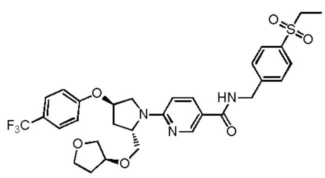

(186),

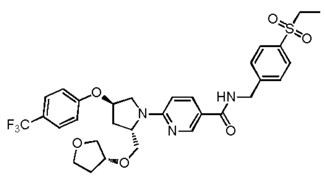

(187),

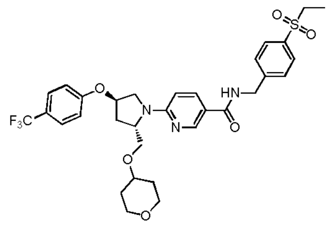

(188),

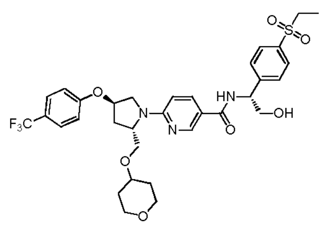

(189),

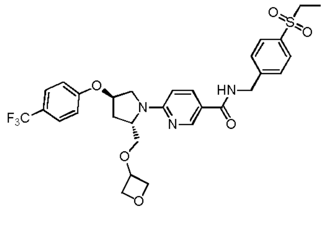

(190),

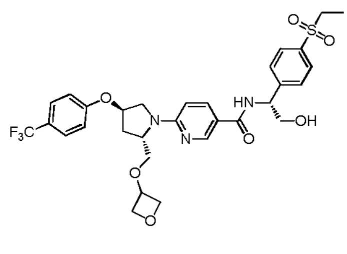

(191),

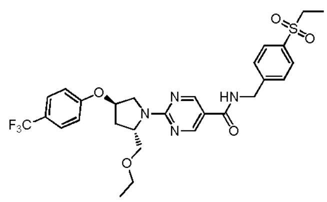

(192),

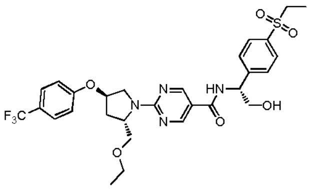

(193),

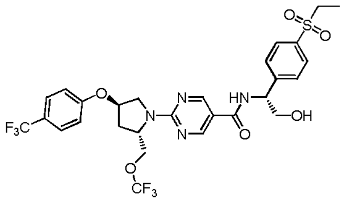

(194),

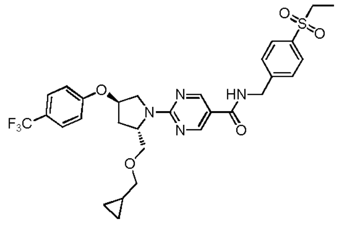

(195),

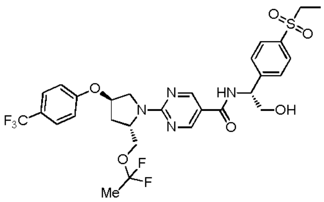

(196),

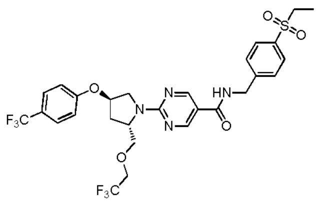

(197),

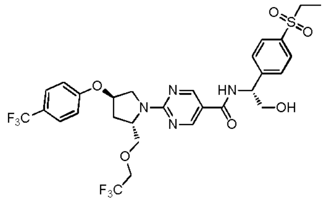

(198),

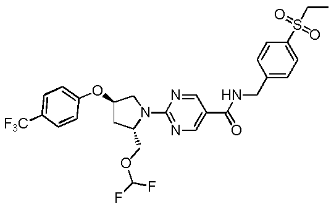

(199),

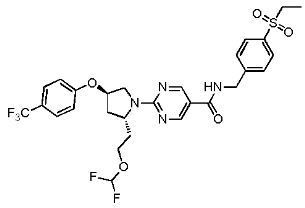

(200),

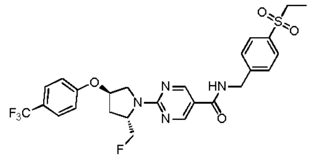

(201),

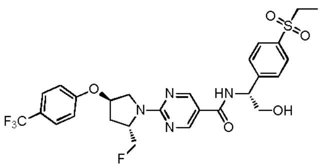

(202),

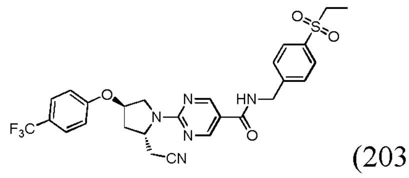

(203),

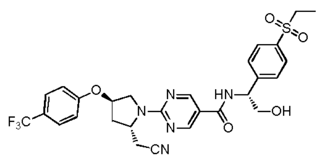

(204),

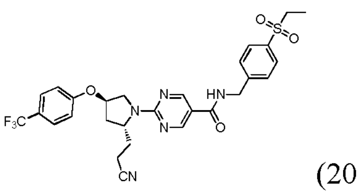

(205),

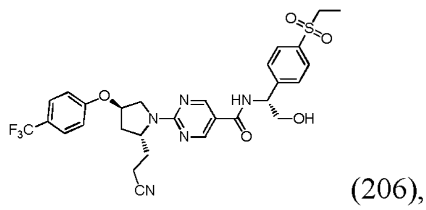

(206),

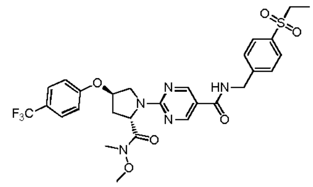

(207),

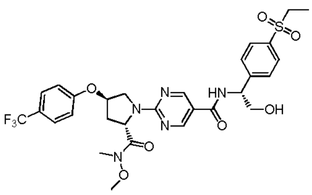

(208),

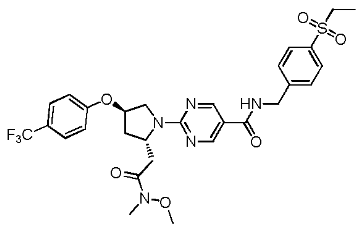

(209),

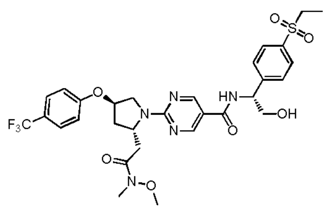

(210),

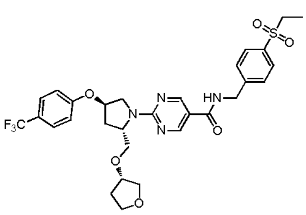

(211),

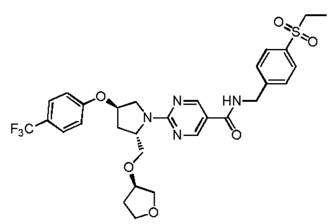

(212),

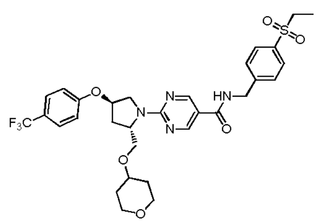

(213),

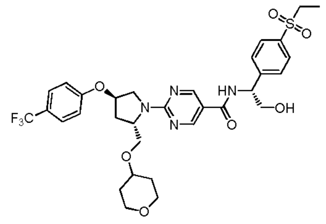 (214),

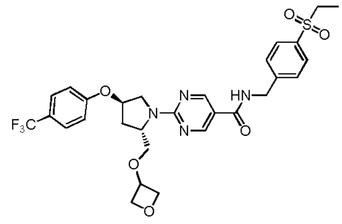 (215),

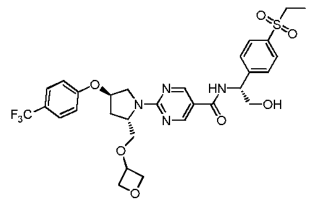 (216),

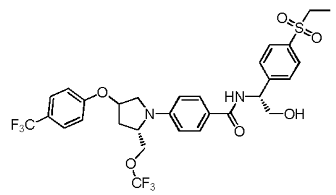 (217),

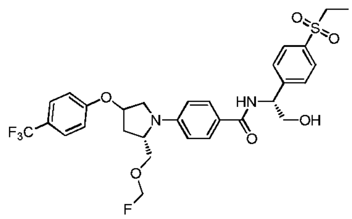 (218),

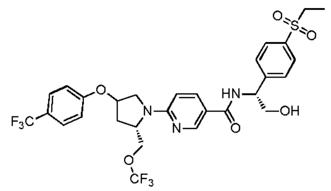 (219),

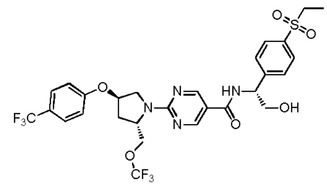 (220),

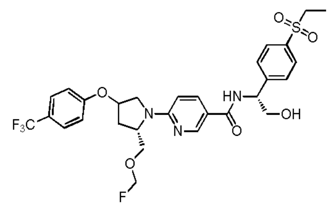 (221),

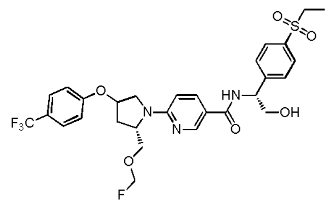 (222),

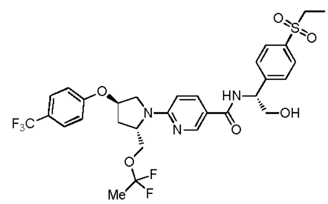 (223),

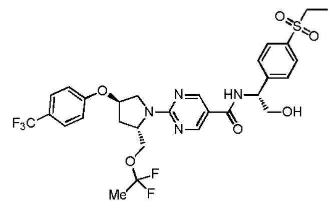 (224),

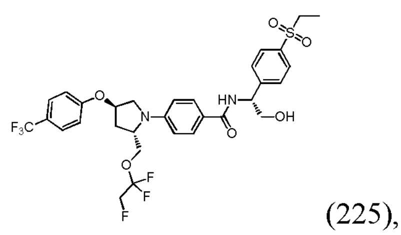 (225),

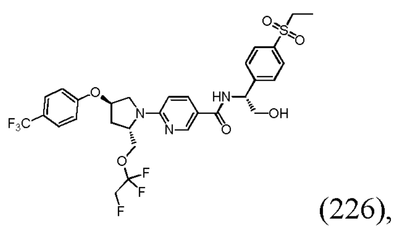 (226),

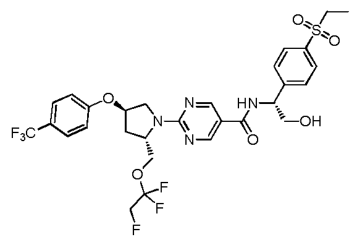 (227),

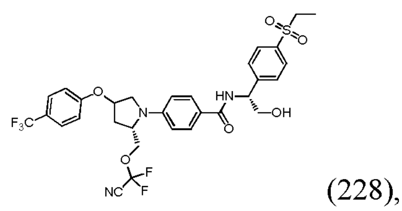 (228),

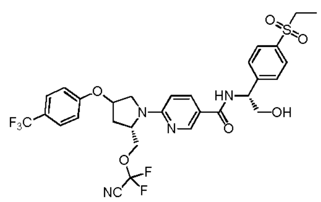 (229),

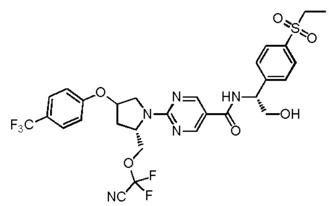 (230),

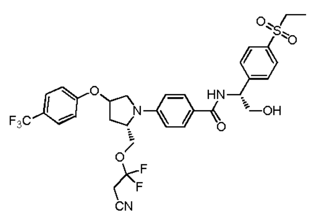 (231), 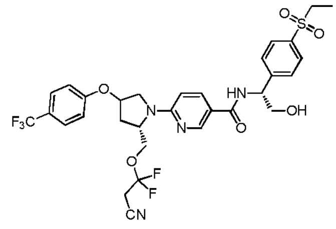 (232), 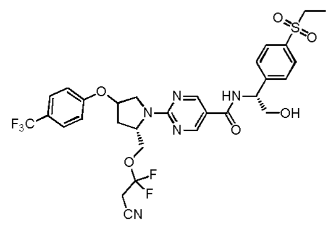 (233),

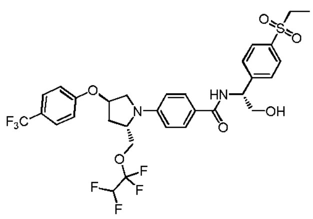 (234), 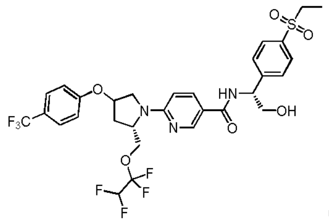 (235), 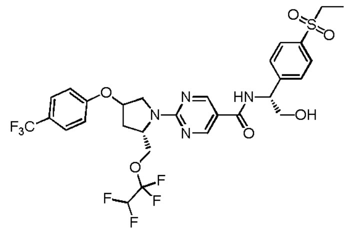 (236),

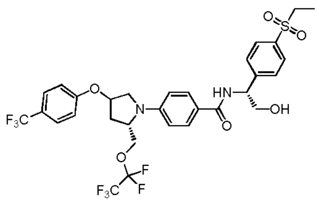 (237), 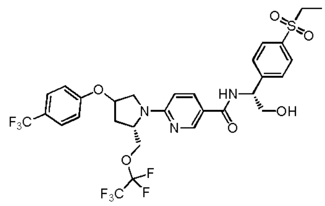 (238), 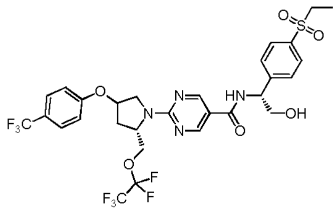 (239),

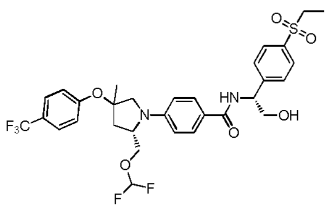 (240), 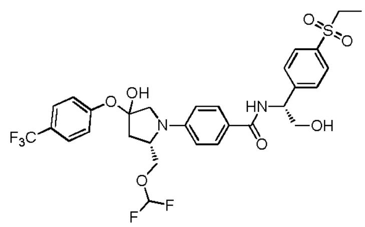 (241),

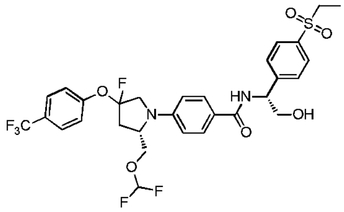 (242), 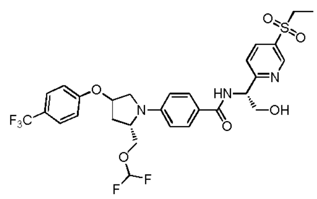 (243), 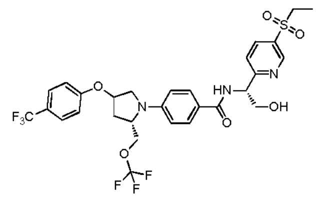 (244),

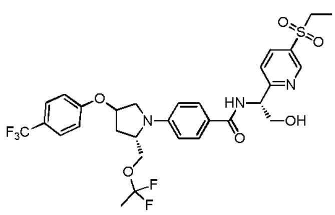 (245), 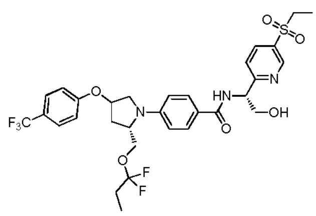 (246), 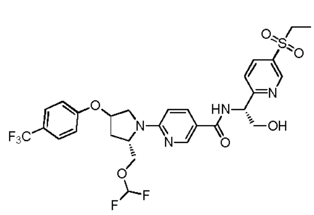 (247),

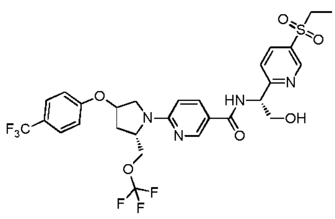 (248), 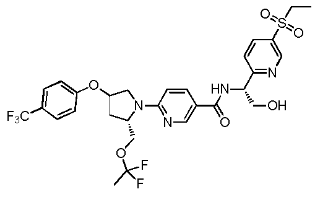 (249), 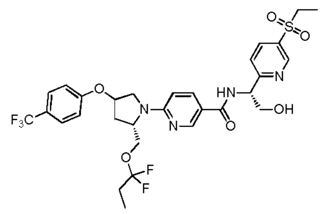 (250),

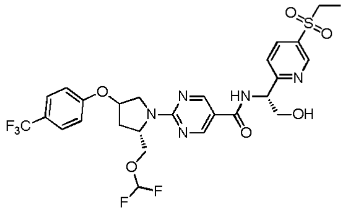 (251), 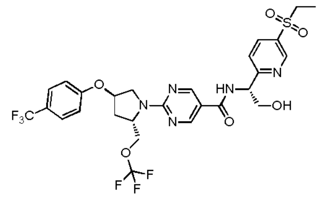 (252), 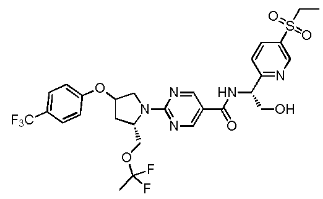 (253),

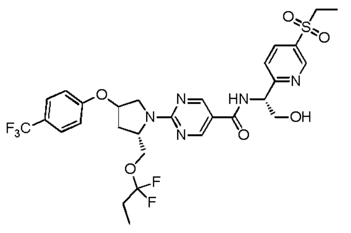 (254), 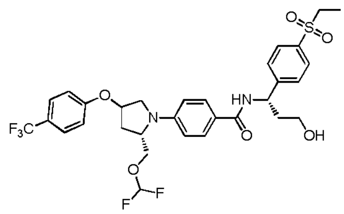 (255),

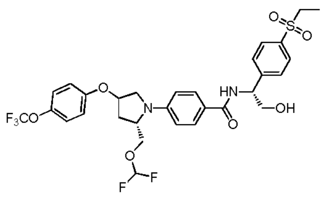 (256), 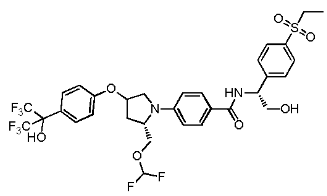 (257), 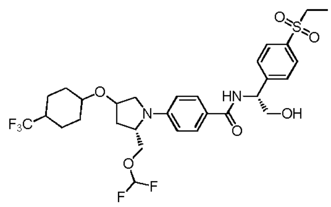 (258),

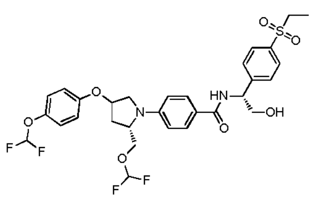 (259), 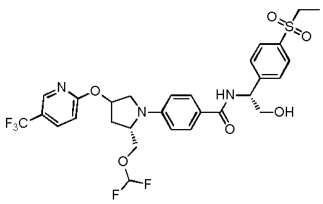 (260), 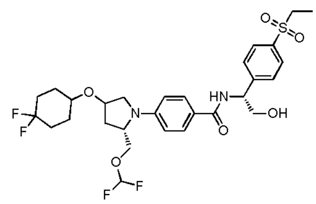 (261),

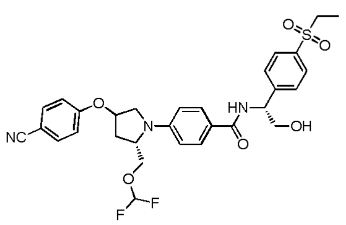 (262), 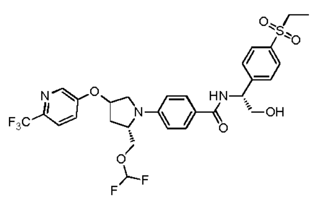 (263), 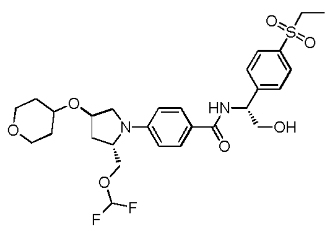 (264),

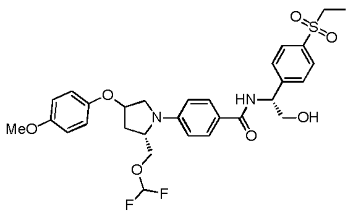 (265),
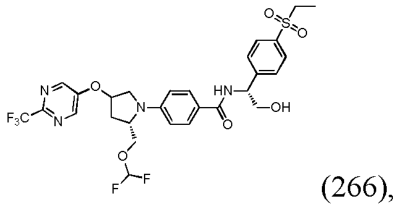 (266),
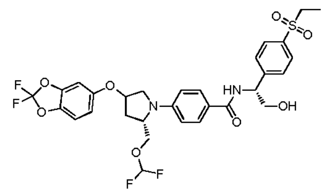 (267),

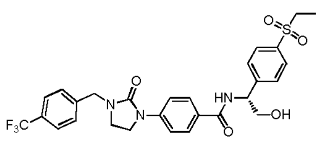 (268),
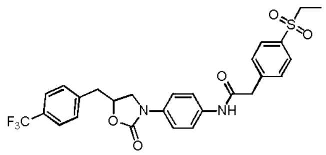 (269),
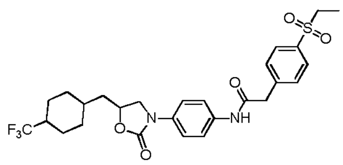 (270),

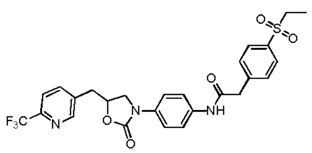 (271),
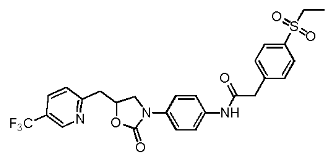 (272),
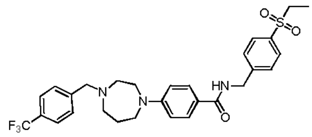 (273),

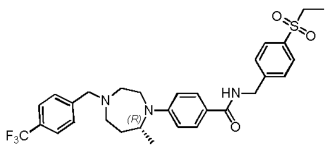 (274),
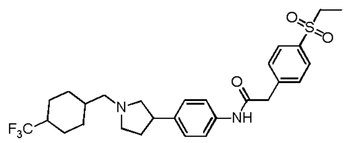 (275),
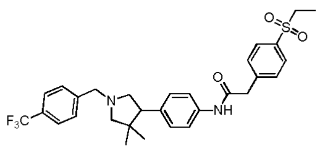 (276),

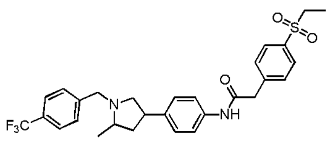 (277),
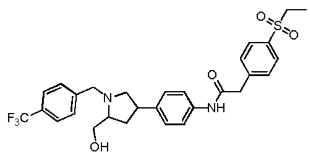 (278),
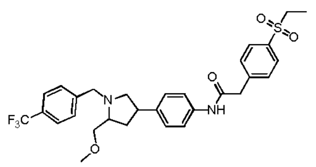 (279),

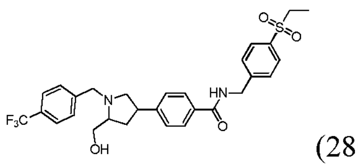 (280),
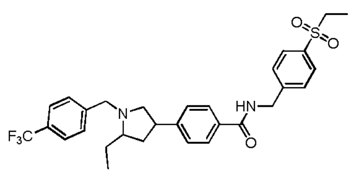 (281),
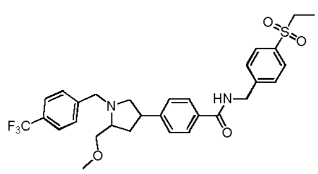 (282),

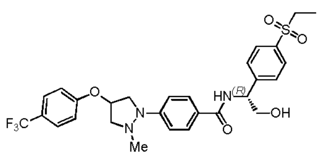 (283),
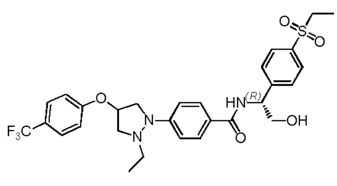 (284),
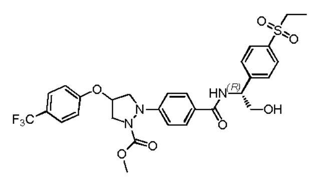 (285),

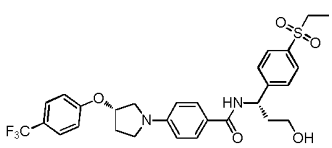 (286),
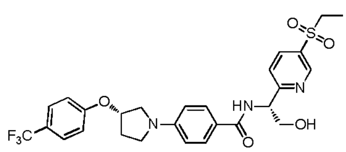 (287),

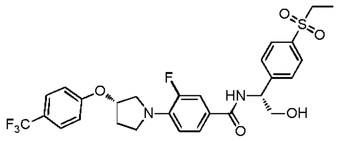 (288),

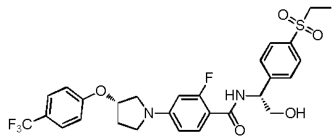 (289),

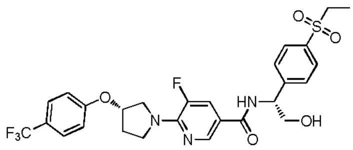 (290),

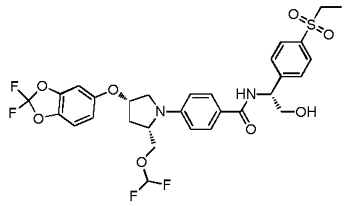 (291),

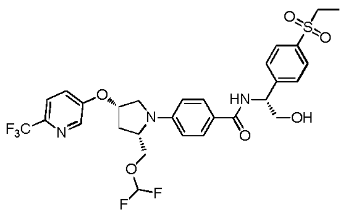 (292),

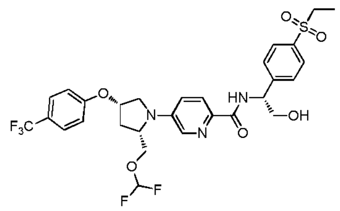 (293),

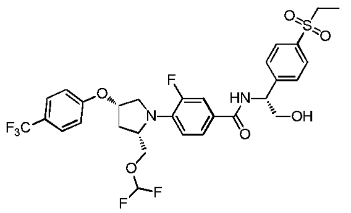 (294),

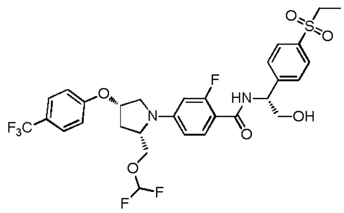 (295),

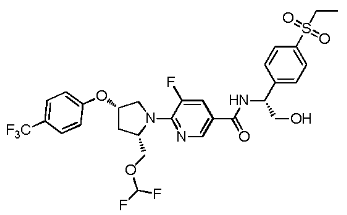 (296),

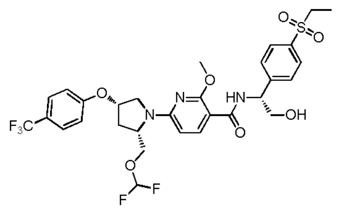 (297),

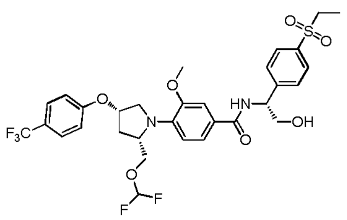 (298),

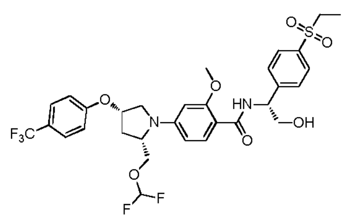 (299),

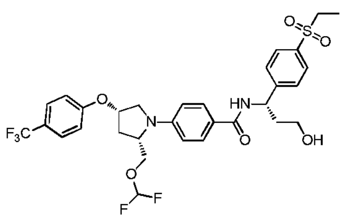 (300),

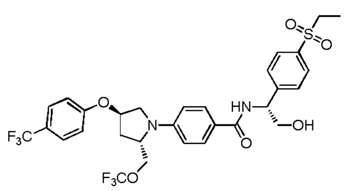 (301),

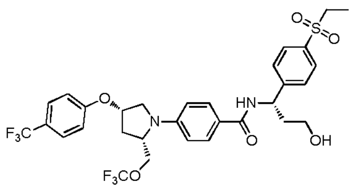 (302),

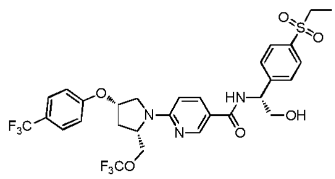 (303),

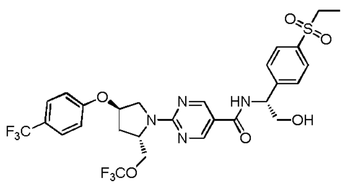 (304),

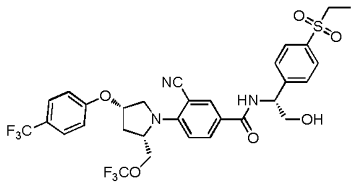 (305),

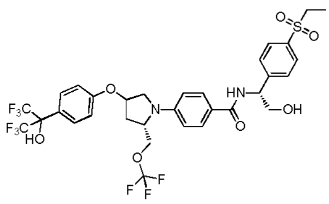 (306),

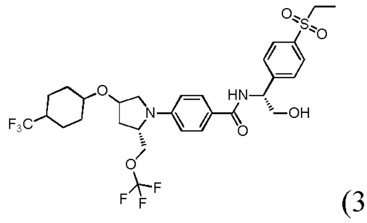 (307),

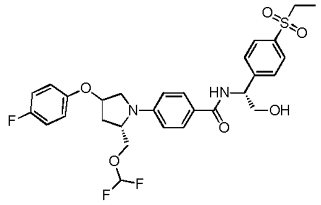 (308),

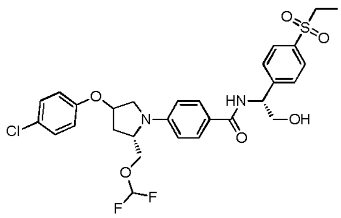 (309),

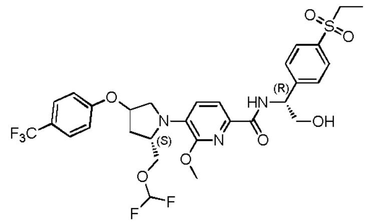 (310),

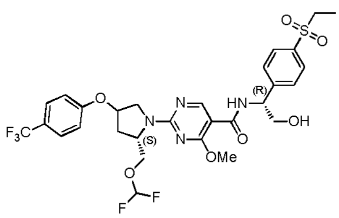 (311),

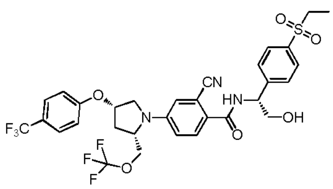 (312),

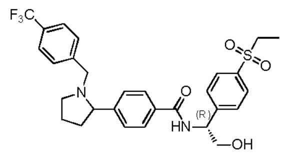 (313),

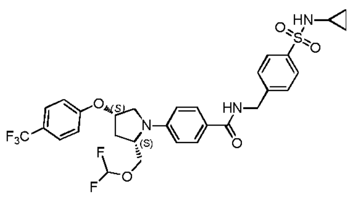 (314),

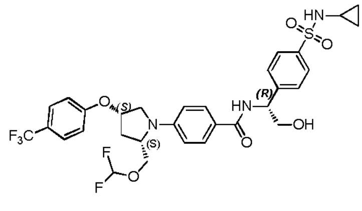 (315),

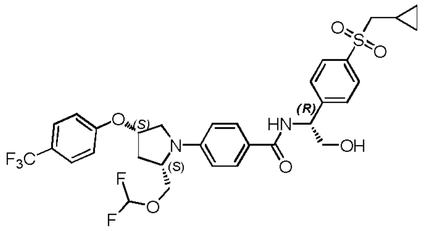

(316),

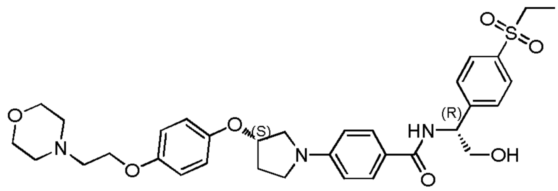

(317),

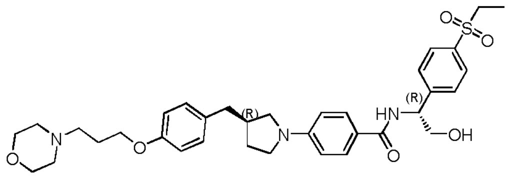

(318),

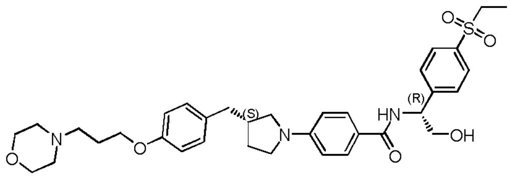

(319),

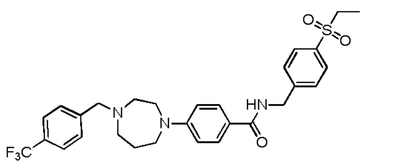

(320),

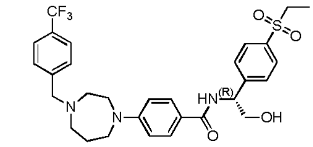

(321),

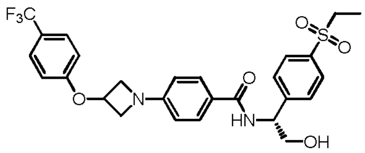

(322),

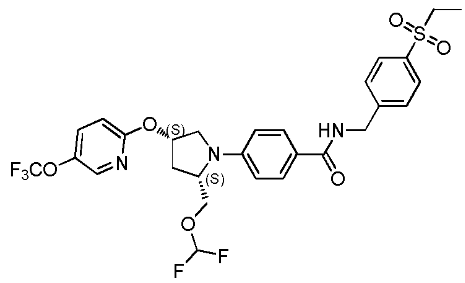

(323),

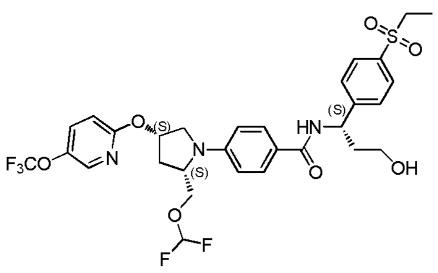

(324),

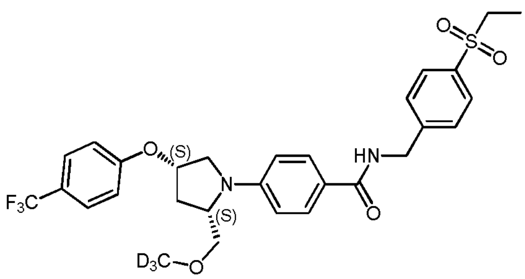

(325),

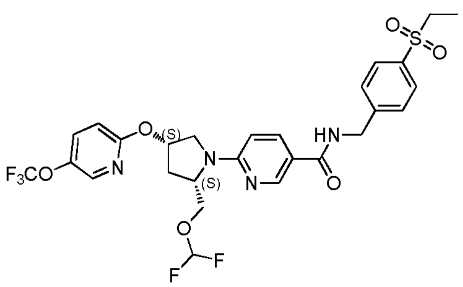

(326),

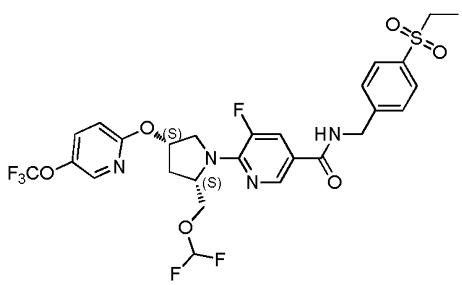

(327),

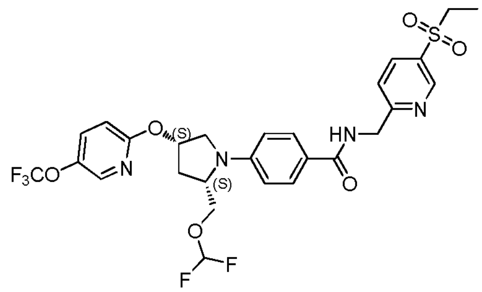
(328),

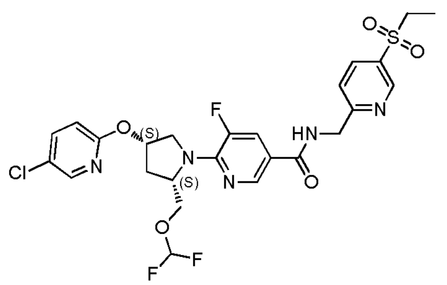
(329),

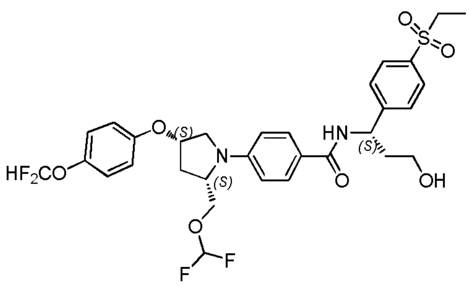
(330),

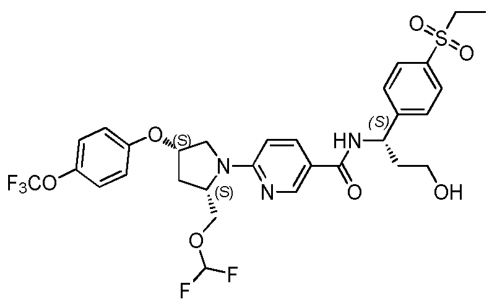
(331),

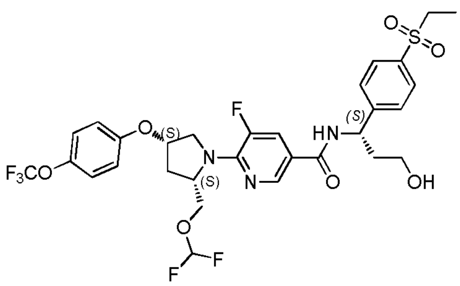
(332),

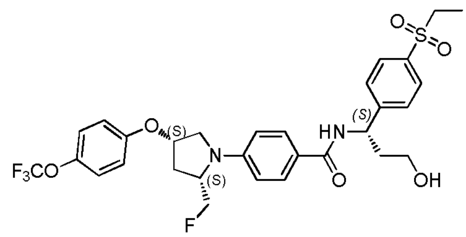
(333),

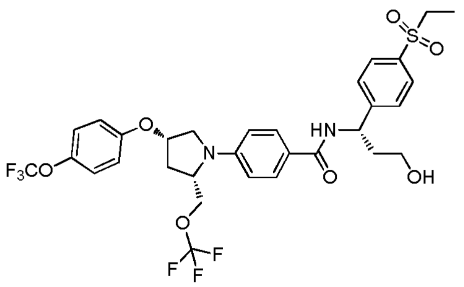
(334),

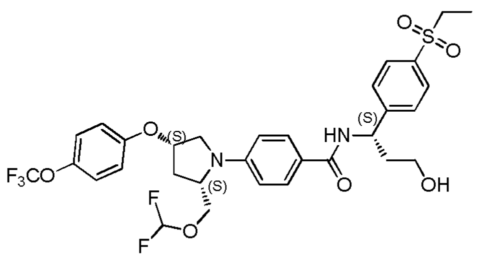
(335),

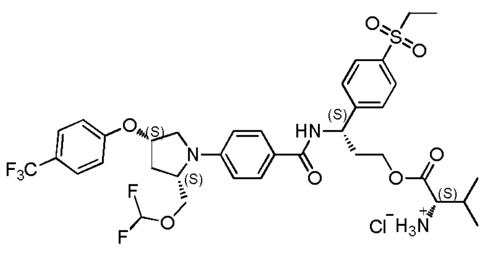
(336),

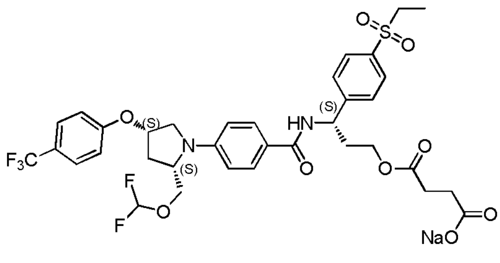
(337),

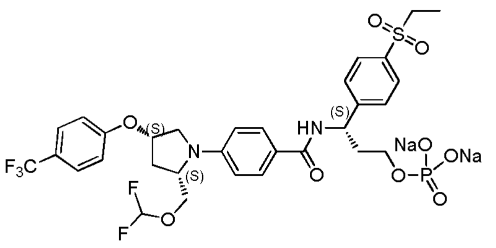

(338),

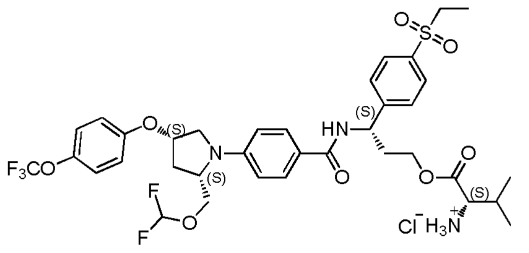

(339),

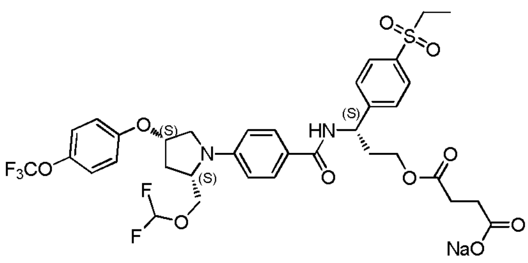

(340),

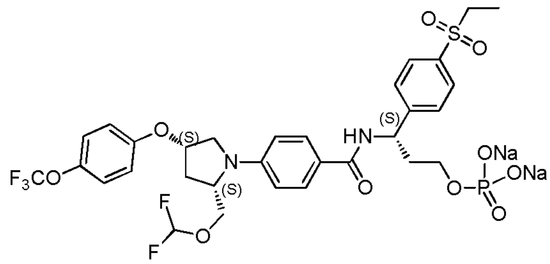

(341)

or

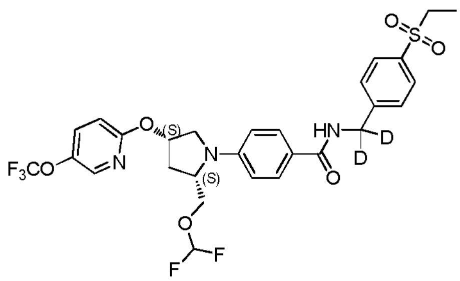

(342),

or a stereoisomer, a geometric isomer, a tautomer, an *N*-oxide, a hydrate, a solvate, a metabolite, an ester, a pharmaceutically acceptable salt or a prodrug thereof.

**18.** A pharmaceutical composition, comprising the compound of any one of claims 1 to 17 and a pharmaceutically acceptable excipient, carrier, adjuvant or a combination thereof.

**19.** The pharmaceutical composition of claim 18, wherein the pharmaceutical composition further comprises other active ingredients, and the other active ingredients are drugs for treating cancer, drugs for preventing or treating inflammatory syndrome, drugs for preventing or treating autoimmune diseases, or any combination thereof.

**20.** Use of the compound of any one of claims 1 to 17 or the pharmaceutical composition of any one of claims 18 to 19 in the manufacture of a medicament for preventing or treating diseases, disorders or syndromes mediated by RORγt in mammals.

**21.** The use of claim 20, wherein the disease, disorder or syndrome mediated by RORγt is cancer, inflammation or autoimmune disease.

**22.** The use of claim 21, wherein the disease, disorder or syndrome mediated by RORγt is cancer, psoriasis, systemic lupus erythematosus, multiple sclerosis, inflammatory bowel disease, colitis, ulcerative colitis, rheumatoid arthritis, autoimmune eye disease, ankylosing spondylitis, asthma, chronic obstructive pulmonary disease, osteoarthritis, allergic rhinitis, atopic dermatitis, Crohn's disease or Kawasaki disease.

**INTERNATIONAL SEARCH REPORT**

International application No.
**PCT/CN2020/142168**

**A. CLASSIFICATION OF SUBJECT MATTER**

C07D 295/155(2006.01)i; C07D 417/04(2006.01)i; C07D 413/04(2006.01)i; A61K 31/5377(2006.01)i; A61K 31/5375(2006.01)i; A61P 19/02(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 维甲酸相关孤核受体, 磺酰, 酰胺, 东阳光新药, retinoid related orphan receptor, ROR, sulfonyl, amide, HEC NEW DRUG, SUNSHINE LAKE

**C. DOCUMENTS CONSIDERED TO BE RELEVANT**

| Category* | Citation of document, with indication, where appropriate, of the relevant passages | Relevant to claim No. |
|---|---|---|
| PX | WO 2020011147 A1 (SUNSHINE LAKE PHARMA CO., LTD.) 16 January 2020 (2020-01-16)<br>claims 1-26 | 1-22 |
| X | WANG, Yonghui et al. . "From RORγt agonist to two types of RORγt inverse agonists." *ACS Med. Chem. Lett.*, Vol. 9, No. 2, 22 January 2018 (2018-01-22),<br>pp. 120-124 | 1-22 |
| A | WO 2013029338 A1 (GLAXO GROUP LTD.) 07 March 2013 (2013-03-07)<br>abstract ; claims 1-15 | 1-22 |
| A | WO 2017010399 A1 (SHIONOGI & CO., LTD.) 19 January 2017 (2017-01-19)<br>abstract; claims 1-24 | 1-22 |
| A | WO 2018009615 A1 (ACQUIST LLC) 11 January 2018 (2018-01-11)<br>entire document | 1-22 |
| A | WO 2018145653 A1 (FUDAN UNIVERSITY) 16 August 2018 (2018-08-16)<br>entire document | 1-22 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2021** | **26 March 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/142168**

**C. DOCUMENTS CONSIDERED TO BE RELEVANT**

| Category* | Citation of document, with indication, where appropriate, of the relevant passages | Relevant to claim No. |
|---|---|---|
| A | WO 2019213470 A1 (ETERNITY BIOSCIENCE INC.) 07 November 2019 (2019-11-07) entire document | 1-22 |

Form PCT/ISA/210 (second sheet) (January 2015)

**INTERNATIONAL SEARCH REPORT**
**Information on patent family members**

International application No.
**PCT/CN2020/142168**

| Patent document cited in search report | Publication date (day/month/year) | Patent family member(s) | Publication date (day/month/year) |
|---|---|---|---|
| WO 2020011147 A1 | 16 January 2020 | None | |
| WO 2013029338 A1 | 07 March 2013 | None | |
| WO 2017010399 A1 | 19 January 2017 | None | |
| WO 2018009615 A1 | 11 January 2018 | US 2020197393 A9 | 25 June 2020 |
| | | BR 112019000201 A2 | 09 July 2019 |
| | | MX 2018014718 A | 11 April 2019 |
| | | JP 2019520387 A | 18 July 2019 |
| | | EP 3481819 A1 | 15 May 2019 |
| | | US 2019117654 A1 | 25 April 2019 |
| | | EP 3481819 A4 | 19 February 2020 |
| | | US 10688095 B2 | 23 June 2020 |
| | | US 2020268757 A1 | 27 August 2020 |
| | | CA 3029883 A1 | 11 January 2018 |
| | | CN 109476642 A | 15 March 2019 |
| | | KR 20190025612 A | 11 March 2019 |
| | | WO 2018009615 A9 | 28 June 2018 |
| | | TW 201805282 A | 16 February 2018 |
| WO 2018145653 A1 | 16 August 2018 | EP 3581561 A4 | 09 December 2020 |
| | | US 2020002280 A1 | 02 January 2020 |
| | | JP 2020508981 A | 26 March 2020 |
| | | US 10844017 B2 | 24 November 2020 |
| | | EP 3581561 A1 | 18 December 2019 |
| WO 2019213470 A1 | 07 November 2019 | AU 2019262169 A1 | 29 October 2020 |
| | | CN 112135611 A | 25 December 2020 |
| | | CA 3097519 A1 | 07 November 2019 |
| | | TW 202014185 A | 16 April 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010008803
- CN 202010009133
- US 4328245 A
- US 4409239 A
- US 4410545 A
- US 6350458 B
- WO 2017132432 A


### Non-patent literature cited in the description


- **SUN et al.** *Science,* 2000, vol. 288, 2369-2372
- **EBERL et al.** *Nat Immunol.,* 2004, vol. 5, 64-73
- **IVANOV, II ; MCKENZIE BS ; ZHOU L ; TADOKORO CE ; LEPELLEY A ; LAFAILLE JJ et al.** *Cell,* 2006, vol. 126 (6), 1121-33
- **JETTEN et al.** *Nucl. Recept. Signal,* 2009, vol. 7, e003
- **MANEL et al.** *Nat. Immunol.,* 2008, vol. 9, 641-649
- **KORN et al.** *Annu. Rev. Immunol.,* 2009, vol. 27, 485-517
- **JETTEN et al.** *Adv. Dev.Biol,* 2006, vol. 16, 313-355
- **MEIER et al.** *Immunity,* 2007, vol. 26, 643-654
- **ALOISI et al.** *Nat. Rev. Immunol.,* 2006, vol. 6, 205-217
- **JAGER et al.** *J. Immunol.,* 2009, vol. 183, 7169-7177
- **BARNES et al.** *Nat. Rev. Immunol.,* 2008, vol. 8, 183-192
- Handbook of Chemistry and Physics. 1994
- Organic Chemistry. Thomas Sorrell. University Science Books, 1999
- **MICHAEL B. SMITH ; JERRY MARCH.** March's Advanced Organic Chemistry. John Wiley & Sons, 2007
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- **JACQUES et al.** Enantiomers, Racemates and Resolutions. Wiley Interscience, 1981
- **ROBERT E. GAWLEY ; JEFFREY AUBÉ.** Principles of Asymmetric Synthesis. Elsevier, 2012
- **ELIEL, E.L.** Stereochemistry of Carbon Compounds. McGraw-Hill, 1962
- **WILEN, S.H.** Tables of Resolving Agents and Optical Resolutions. Univ. of Notre Dame Press, 1972, 268
- Chiral Separation Techniques: A Practical Approach. Wiley-VCH Verlag GmbH & Co. KGaA, 2007
- **T. W. GREENE.** Protective Groups in Organic Synthesis. John Wiley & Sons, 1991
- **P. J. KOCIENSKI.** Protecting Groups, Thieme, Stuttgart. 2005
- Pro-drugs as Novel Delivery Systems. **T. HIGUCHI ; V. STELLA.** Bioreversible Carriers in Drug Design. American Pharmaceutical Association and Pergamon Press, 1987, vol. 14
- **J. RAUTIO et al.** Prodrugs: Design and Clinical Applications. *Nature Review Drug Discovery,* 2008, vol. 7, 255-270
- **S. J. HECKER et al.** Prodrugs of Phosphates and Phosphonates. *Journal of Medicinal Chemistry,* 2008, vol. 51, 2328-2345
- **S. M. BERGE et al.** describe pharmaceutically acceptable salts in detail. *J. Pharmaceutical Sciences,* 1977, vol. 66, 1-19
- **JERRY MARCH.** Advanced Organic Chemistiy. Wiley Interscience
- *Syn. Comm.,* 1977, vol. 7, 509-514
- Remington's Pharmaceutical Sciences. Mack Printing Company, 1990, 1289-1329
- Remington's Pharmaceutical Sciences. Mack Publishing Company, 1985
- **STAHL ; WERMUTH.** Handbook of Pharmaceutical Salts: Properties, Selection, and Use. Wiley-VCH, 2002
- Remington's Pharmaceutical Sciences. Mack Publishing Company
- The Handbook of Pharmaceutical Additives. Gower Publishing Limited
- The Handbook of Pharmaceutical Excipients. American Pharmaceutical Association and the Pharmaceutical Press
- Remington: The Science and Practice of Pharmacy. Lippincott Williams & Wilkins, 2005
- Encyclopedia of Pharmaceutical Technology. Marcel Dekker, 1988